(19) Europäisches Patentamt
European Patent Office
Office européen des brevets

(11) EP 2 357 173 A1

(12) EUROPÄISCHE PATENTANMELDUNG

(43) Veröffentlichungstag:
**17.08.2011 Patentblatt 2011/33**

(21) Anmeldenummer: **10179237.2**

(22) Anmeldetag: **25.10.2003**

(51) Int Cl.:
***C07D 213/30*** *(2006.01)* ***A61K 31/4375*** *(2006.01)*
***A61P 3/04*** *(2006.01)* ***C07D 401/12*** *(2006.01)*
***C07D 405/06*** *(2006.01)* ***C07D 401/06*** *(2006.01)*
***C07D 213/40*** *(2006.01)* ***C07D 401/10*** *(2006.01)*
***C07D 213/38*** *(2006.01)* ***C07D 401/14*** *(2006.01)*
***C07D 213/55*** *(2006.01)* ***C07D 213/56*** *(2006.01)*
***C07D 213/61*** *(2006.01)* ***C07D 213/53*** *(2006.01)*

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV**

(30) Priorität: **31.10.2002 DE 10250708**

(62) Dokumentnummer(n) der früheren Anmeldung(en) nach Art. 76 EPÜ:
**03809734.1 / 1 558 578**

(71) Anmelder: **Boehringer Ingelheim Pharma GmbH & Co. KG**
**55216 Ingelheim am Rhein (DE)**

(72) Erfinder:
- **Mueller, Stephan-Georg**
  **88447, WARTHAUSEN (DE)**
- **Stenkamp, Dirk**
  **88400, BIBERACH (DE)**
- **Arndt, Kirsten**
  **88214Ravensburg (DE)**
- **Roth, Gerald Juergen**
  **88400, BIBERACH (DE)**
- **Lotz, Ralf Richard Hermann**
  **88437, SCHEMMERHOFEN (DE)**
- **Lehmann-Lintz, Thorsten**
  **88416, OCHSENHAUSEN (DE)**
- **Lenter, Martin**
  **89231 Neu-Ulm (DE)**
- **Lustenberg, Philipp**
  **CH - 4056 Basel (CH)**
- **Rudolf, Klaus**
  **88447, WARTHAUSEN (DE)**

(74) Vertreter: **Hammann, Heinz et al**
**Boehringer Ingelheim GmbH**
**Corporate Patents**
**Binger Strasse 173**
**55216 Ingelheim am Rhein (DE)**

Bemerkungen:
Diese Anmeldung ist am 24-09-2010 als Teilanmeldung zu der unter INID-Code 62 erwähnten Anmeldung eingereicht worden.

# (54) Neue Alkin-Verbindungen mit MCH-antagonistischer Wirkung und diese Verbindungen enthaltende Arzneimittel

(57) Die vorliegende Erfindung betrifft Alkin-Verbindungen der allgemeinen Formel I

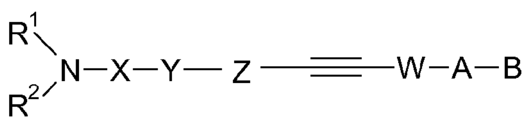

I

in der die Gruppen und Reste A, B, W, X, Y, Z, $R^1$ und $R^2$ die in Anspruch 1 angegebenen Bedeutungen aufweisen. Ferner betrifft die Erfindung Arzneimittel enthaltend mindestens ein erfindungsgemäßes Alkin. Auf Grund der MCH-Rezeptor antagonistischen Aktivität eignen sich die erfindungsgemäßen Arzneimittel zur Behandlung von metabolischen Störungen und/oder Essstörungen, insbesondere von Obesitas, Bulimie, Anorexie, Hyperphagia und Diabetes.

EP 2 357 173 A1

## Beschreibung

Gegenstand der vorliegenden Erfindung sind neue Alkin-Verbindungen, deren physiologisch verträglichen Salze und deren Verwendung als MCH-Antagonisten sowie deren Verwendung zur Herstellung eines Arzneimittels, welches zur Prophylaxe und/oder Behandlung von Erscheinungen und/oder Krankheiten, die durch MCH verursacht werden oder mit MCH in einem anderen kausalen Zusammenhang stehen, geeignet ist. Ein weiterer Gegenstand dieser Erfindung betrifft die Verwendung einer erfindungsgemäßen Verbindung zur Beeinflussung des Essverhaltens sowie zur Reduzierung des Körpergewichts und/ oder zum Verhindern einer Zunahme des Körpergewichts eines Säugetiers. Ferner sind Zusammensetzungen und Arzneimittel, jeweils enthaltend eine erfindungsgemäße Verbindung, sowie Verfahren zu deren Herstellung Gegenstand dieser Erfindung.

## Hintergrund der Erfindung

Die Aufnahme von Nahrung und deren Umsetzung im Körper spielt für alle Lebewesen eine existentielle Rolle im Leben. Daher führen Abweichungen bei der Aufnahme und Umsetzung der Nahrung in der Regel zu Störungen und auch Krankheiten. Die Veränderung der menschlichen Lebens- und Ernährungsgewohnheiten, insbesondere in Industrieländern, hat in den letzten Jahrzehnten Obesitas begünstigt. Obesitas führt bei den Betroffenen unmittelbar zu einer Einschränkung der Mobilität und einer Verminderung der Lebensqualität. Erschwerend kommt hinzu, dass Obesitas oft weitere Krankheiten zur Folge hat, wie beispielswiese Diabetes, Dyslipidaemie, Bluthochdruck, Arteriosklerose und koronare Herzerkrankungen. Darüber hinaus führt alleine das hohe Körpergewicht zu einer verstärkten Belastung des Stütz- und Bewegungsapparates, was zu chronischen Beschwerden und Krankheiten, wie Arthritis oder Osteoarthritis, führen kann. Somit stellt Obesitas ein schwerwiegendes gesundheitliches Problem für die Gesellschaft dar.

Der Begriff Obesitas bedeutet einen Überschuss an adipösem Gewebe. In diesem

Zusammenhang ist Obesitas grundsätzlich als jeglicher erhöhter Grad an Adipositas zu sehen, der zu einem gesundheitlichen Risiko führt. Eine Abgrenzung zwischen normalen und an Obesitas leidenden Individuen ist letztlich nicht exakt möglich, jedoch steigt das mit Obesitas einhergehende gesundheitliche Risiko wahrscheinlich kontinuierlich mit zunehmender Adipositát an. Aus Gründen der Vereinfachung werden im Zusammenhang mit der vorliegenden Erfindung vorzugsweise die Individuen mit einem Körpergewichtsindex (BMI = body mass index), der als das in Kilogramm gemessene Körpergewicht geteilt durch die Körpergröße (in Metern) im Quadrat definiert ist, oberhalb des Wertes 25, insbesondere oberhalb 30, als an Obesitas leidend betrachtet.

Abgesehen von körperlicher Aktivität und Ernährungsumstellung existiert derzeit keine überzeugende Behandlungsmöglichkeit zur effektiven Reduzierung des Körpergewichts. Da Obesitas jedoch einen hohen Risikofaktor bei der Entstehung ernsthafter und sogar lebensbedrohlicher Erkrankungen darstellt, ist es umso wichtiger, pharmazeutische Wirkstoffe zur Prophylaxe und/oder Behandlung von Obesitas bereit zu stellen. Ein in neuester Zeit vorgeschlagener Ansatz ist der therapeutische Einsatz von MCH-Antagonisten (u.a. WO 01/21577, WO 01/82925).

Melanin-konzentrierendes Hormon (melanin-concentrating hormone, MCH) ist ein zyklisches Neuropeptid bestehend aus 19 Aminosäuren. Es wird in Säugetieren vorwiegend im Hypothalamus synthetisiert und erreicht von dort weitere Gehirnregionen über die Projektionen hypothalamischer Neurone. Seine biologische Aktivität wird im Menschen über zwei unterschiedliche G-Protein-gekoppelte Rezeptoren (GPCRs) aus der Familie Rhodopsin-verwandter GPCRs vermittelt, die MCH-Rezeptoren 1 und 2 (MCH-1R, MCH-2R).

Untersuchungen der Funktion von MCH in Tiermodellen ergeben gute Anhaltspunkte für eine Rolle des Peptides bei der Regulation der Energiebilanz, d.h. Veränderung metabolischer Aktivität und Futteraufnahme [1, 2]. Beispielsweise wird nach intraventrikulärer Applikation von MCH bei Ratten die Futteraufnahme im Vergleich zu Kontrolltieren gesteigert. Daneben reagieren transgene Ratten, die mehr MCH produzieren als Kontrolltiere, nach Gabe einer fettreichen Diät mit einer deutlicheren

Gewichtssteigerung als Tiere mit nicht experimentell verändertem MCH-Spiegel. Auch konnte festgestellt werden, dass eine positive Korrelation zwischen Phasen gesteigerten Verlangens nach Futter und der Menge an MCH mRNA im Hypothalamus von Ratten besteht. Von besonderer Aussagekraft bezüglich der Funktion von MCH sind aber Experimente mit MCH knock out Mäusen. Ein Verlust des Neuropeptides führt zu mageren Tieren mit verminderter Fettmasse, die deutlich weniger Nahrung zu sich nehmen als Kontrolltiere.

Die anorektischen Effekte von MCH werden in Nagetieren über den $G_{\alpha s}$-gekoppelten MCH-1 R vermittelt [3-6]. Im Gegensatz zum Primaten, Frettchen und Hund, konnte bei Nagern bisher kein zweiter Rezeptor nachgewiesen werden. Nach Verlust des MCH-1 R besitzen knock out Mäuse weniger Fettmasse, einen erhöhten Energieumsatz und bei fettreicher Diät keine Gewichtssteigerung im Vergleich zu Kontrolltieren. Ein weiterer Hinweis für die Bedeutung des MCH-MCH-1 R Systems bei der Regulation der Energiebilanz stammt aus Experimenten mit einem Rezeptor-Antagonisten (SNAP-7941) [3]. In Langzeit-Versuchen verlieren die mit dem Antagonisten behandelten Tiere deutlich an Gewicht.

Neben seiner anorektischen Wirkung werden mit dem MCH-1 R-Antagonisten SNAP-7941 noch weitere anxiolytische und antidepressive Effekte in Verhaltensexperimenten mit Ratten erzielt [3]. Damit liegen deutliche Hinweise vor, dass das MCH-MCH-1 R-System nicht nur an der Regulation der Energiebilanz sondern auch der Affektivität beteiligt ist.

Literatur:

1. Qu, D., et al., A role for melanin-concentrating hormone in the central regulation of feeding behaviour. Nature, 1996. 380(6571): p. 243-7.
2. Shimada, M., et al., Mice lacking melanin-concentrating hormone are hypophagic and lean. Nature, 1998. 396 (6712): p. 670-4.
3. Borowsky, B., et al., Antidepressant, anxiolytic and anorectic effects of a melanin-concentrating hormone-1 receptor antagonist. Nat Med, 2002. 8(8): p. 825-30.
4. Chen, Y., et al., Targeted disruption of the melanin-concentrating hormone receptor-1 results in hyperphagia and resistance to diet-induced obesity. Endocrinology, 2002. 143(7): p. 2469-77.
5. Marsh, D.J., et al., Melanin-concentrating hormone 1 receptor-deficient mice are lean, hyperactive, an+d hyperphagic and have altered metabolism. Proc Natl Acad Sci U S A, 2002. 99(5): p. 3240-5.
6. Takekawa, S., et al., T-226296: a novel, orally active and selective melanin-concentrating hormone receptor antagonist. Eur J Pharmacol, 2002. 438(3): p. 129-35.


In der Patentliteratur werden bestimmte Amin-Verbindungen als MCH Antagonisten vorgeschlagen. So werden in der WO 01/21577 (Takeda) Verbindungen der Formel

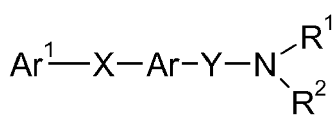

in der $Ar^1$ eine cyclische Gruppe , X einen Spacer, Y eine Bindung oder einen Spacer, Ar einen aromatischen Ring, der mit einem nicht-aromatischen Ring kondensiert sein kann, $R^1$ und $R^2$ unabhängig voneinander H oder eine Kohlenwasserstoff-Gruppe bedeuten, wobei $R^1$ und $R^2$ zusammen mit dem angrenzenden N-Atom einen N-haltigen Heteroring bilden können und $R^2$ mit Ar auch einen spirocyclischen Ring bilden kann, R zusammen mit dem angrenzenden N-Atom und Y einen N-haltigen Heteroring bilden kann, als MCH-Antagonisten zur Behandlung von u.a. Obesitas beschrieben.

Ferner werden in der WO 01/82925 (Takeda) ebenfalls Verbindungen der Formel

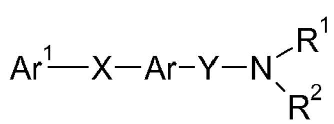

in der $Ar^1$ eine cyclische Gruppe , X und Y Spacer-Gruppen, Ar einen gegebenenfalls substituierten kondensierten polycyclischen aromatischen Ring, $R^1$ und $R^2$ unabhängig voneinander H oder eine Kohlenwasserstoff-Gruppe bedeuten, wobei $R^1$ und $R^2$ zusammen mit dem angrenzenden N-Atom einen N-haltigen heterocyclischen Ring bilden können und $R^2$ zusammen mit dem angrenzenden N-Atom und Y einen N-haltigen Heteroring bilden kann, als MCH-Antagonisten zur Behandlung von u.a. Obesitas beschrieben.

Aufgabe der Erfindung

Der vorliegenden Erfindung liegt die Aufgabe zugrunde, neue Alkin-Verbindungen aufzuzeigen, insbesondere solche, die eine Aktivität als MCH-Antagonisten besitzen. Ebenfalls ist es eine Aufgabe dieser Erfindung, neue Alkin-Verbindungen bereit zu stellen, die es erlauben, dass Essverhalten von Säugetieren zu beeinflussen und insbesondere bei Säugetieren eine Reduzierung des Körpergewichts zu erreichen und/oder eine Zunahme des Körpergewichts zu verhindern.

Ferner ist es eine Aufgabe der vorliegenden Erfindung, neue Arzneimittel bereit zu stellen, welche zur Prophylaxe und/oder Behandlung von Erscheinungen und/oder Krankheiten, die durch MCH verursacht werden oder mit MCH

in einem anderen kausalen Zusammenhang stehen, geeignet sind. Insbesondere liegt dieser Erfindung die Aufgabe zugrunde, Arzneimittel zur Behandlung von metabolischen Störungen, wie Obesitas und/oder Diabetes sowie von mit Obesitas und Diabetes einhergehenden Krankheiten und/oder Störungen, zur Verfügung zu stellen. Weitere Aufgaben der vorliegenden Erfindung beziehen sich auf das Aufzeigen von vorteilhaften Verwendungen der erfindungsgemäßen Verbindungen. Ebenfalls eine Aufgabe dieser Erfindung ist es, ein Verfahren zur Herstellung der erfindungsgemäßen Alkin-Verbindungen bereit zu stellen. Weitere Aufgaben der vorliegenden Erfindung ergeben sich für den Fachmann unmittelbar aus den vorhergehenden und nachfolgenden Ausführungen.

**Gegenstand der Erfindung**

Ein erster Gegenstand der vorliegenden Erfindung sind Alkin-Verbindungen der allgemeinen Formel

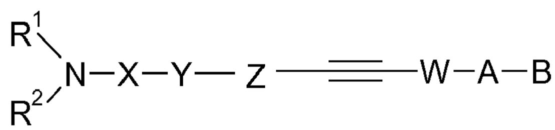

I

in der

$R^1$, $R^2$ unabhängig voneinander H, eine gegebenenfalls mit dem Rest $R^{11}$ substituierte $C_{1-8}$-Alkyl- oder $C_{3-7}$-Cycloalkyl-Gruppe, wobei eine -$CH_2$-Gruppe in Position 3 oder 4 einer 5, 6 oder 7-gliedrigen Cycloalkylgruppe durch -O-, -S- oder -$NR^{13}$- ersetzt sein kann, oder ein gegebenenfalls mit dem Rest $R^{12}$ ein- oder mehrfach und/oder mit Nitro einfach substituierter Phenyl- oder Pyridinylrest, oder
$R^1$ und $R^2$ bilden eine $C_2$-$_8$-Alkylen-Brücke, in der

- ein oder zwei -$CH_2$-Gruppen unabhängig voneinander durch -CH=N- oder -CH=CH- ersetzt sein können und/oder
- ein oder zwei -$CH_2$-Gruppen unabhängig voneinander durch -O-, -S-, -SO-, -($SO_2$)-, -C=N-$R^{18}$, -C=N-O-$R^{18}$, -CO-, -C(=$CH_2$)- oder -$NR^{13}$- derart ersetzt sein können, dass Heteroatome nicht unmittelbar miteinander verbunden sind,

wobei in der zuvor definierten Alkylen-Brücke ein oder mehrere H-Atome durch $R^{14}$ ersetzt sein können, und
wobei die zuvor definierte Alkylen-Brücke mit einer oder zwei gleichen oder verschiedenen carbo- oder heterocyclischen Gruppen Cy derart substituiert sein kann, dass die Bindung zwischen der Alkylenbrücke und der Gruppe Cy
- über eine Einfach- oder Doppelbindung,
- über ein gemeinsames C-Atom unter Ausbildung eines spirocyclischen Ringsystems,
- über zwei gemeinsame, benachbarte C- und/oder N-Atome unter Ausbildung eines kondensierten bicyclischen Ringsystems oder
- über drei oder mehrere C- und/oder N-Atome unter Ausbildung eines verbrückten Ringsystems erfolgt,

X eine Einfachbindung oder eine $C_{1-6}$-Alkylen-Brücke, in der

-eine -$CH_2$-Gruppe durch -CH=CH- oder -C≡C- ersetzt sein kann und/oder
-ein oder zwei -$CH_2$-Gruppen unabhängig voneinander durch -O-, -S-, -(SO)-, -($SO_2$)-, -CO- oder -$NR^4$- derart ersetzt sein können, dass jeweils zwei O-, S- oder N-Atome oder ein O- mit einem S-Atom nicht unmittelbar miteinander verbunden sind,

wobei die Brücke X mit $R^1$ unter Einschluss des mit $R^1$ und X verbundenen N-Atoms unter Ausbildung einer heterocyclischen Gruppe verbunden sein kann, wobei die Brücke X zusätzlich auch mit $R^2$ unter Einschluss des mit $R^2$ und X verbundenen N-Atoms unter Ausbildung einer heterocyclischen Gruppe verbunden sein kann, und
wobei zwei C-Atome oder ein C- und ein N-Atom der Alkylenbrücke durch eine zusätzliche $C_{1-4}$-Alkylen-Brücke miteinander verbunden sein können, und
wobei ein C-Atom mit $R^{10}$ und/oder ein oder zwei C-Atome jeweils mit einem oder zwei gleichen oder verschiedenen Substituenten ausgewählt aus $C_{1-6}$- Alkyl-, $C_{2-6}$-Alkenyl-, $C_{2-6}$-Alkinyl-, $C_{3-7}$-Cycioaikyi,

$C_{3-7}$-Cycloalkyl-$C_{1-3}$-alkyl, $C_{4-7}$-Cycloalkenyl und $C_{4-7}$-Cycloalkenyl-$C_{1-3}$-alkyl substituiert sein können, wobei zwei Alkyl- und/oder Alkenyl-Substituenten unter Ausbildung eines carbocyclischen Ringsystems miteinander verbunden sein können, und

W, Z unabhängig voneinander eine Einfachbindung oder eine $C_{1-4}$-Alkylen-Brücke,
wobei in der Gruppe W und/oder Z eine nicht mit der -C≡C-Gruppe benachbarte -$CH_2$-Gruppe durch -O- oder -$NR^5$- ersetzt sein kann, und
wobei zwei benachbarte C-Atome oder ein C-Atom und ein benachbartes N- Atom mit einer zusätzlichen $C_{1-4}$-Alkylen-Brücke miteinander verbunden sein können, und
wobei in der Alkylen-Brücke und/oder in der zusätzlichen Alkylen-Brücke ein C-Atom mit $R^{10}$ und/oder ein oder zwei C-Atome unabhängig voneinander mit einem oder zwei gleichen oder verschiedenen $C_{1-6}$-Alkyl-Resten substituiert sein können, wobei zwei Alkylreste unter Ausbildung eines carbocyclischen Rings miteinander verbunden sein können, und

Y eine der für Cy angegebenen Bedeutungen,
wobei $R^1$ mit Y unter Einschluss der Gruppe X und des mit $R^1$ und X verbundenen N-Atoms unter Ausbildung einer an Y kondensierten heterocyclischen Gruppe verbunden sein kann, und/oder
wobei X mit Y unter Ausbildung einer an Y kondensierten carbo- oder heterocyclischen Gruppe verbunden sein kann, und

A eine der für Cy angegebenen Bedeutungen und

B eine der für Cy angegebenen Bedeutungen oder
$C_{1-6}$-Alkyl, $C_{1-6}$-Alkenyl, $C_{1-6}$-Alkinyl, $C_{3-7}$-Cycloalkyl-$C_{1-3}$-alkyl-, $C_{3-7}$- Cycloalkenyl-$C_{1-3}$-alkyl-, $C_{3-7}$-Cycloalkyl-$C_{1-3}$-alkenyl- oder $C_{3-7}$-Cycloalkyl-$C_{1-3}$-alkinyl-, worin ein oder mehrere C-Atome ein- oder mehrfach mit Halogen und/ oder einfach mit Hydroxy oder Cyano und/ oder cyclische Gruppen ein- oder mehrfach mit $R^{20}$ substituiert sein können,

Cy eine carbo- oder heterocyclische Gruppe ausgewählt aus einer der folgenden Bedeutungen

-eine gesättigte 3- bis 7-gliedrige carbocyclische Gruppe,
-eine ungesättigte 4- bis 7-gliedrige carbocyclische Gruppe,
-eine Phenyl-Gruppe,
-eine gesättigte 4- bis 7-gliedrige oder ungesättigte 5- bis 7-gliedrige heterocyclische Gruppe mit einem N-, O- oder S-Atom als Heteroatom,
-eine gesättigte oder ungesättigte 5- bis 7-gliedrige heterocyclische Gruppe mit zwei oder mehreren N-Atomen oder mit einem oder zwei N-Atomen und einem O- oder S-Atom als Heteroatome,
-eine aromatische heterocyclische 5- oder 6-gliedrige Gruppe mit einem oder mehreren gleichen oder verschiedenen Heteroatomen ausgewählt aus N, O und/oder S,

wobei die zuvor angeführten 4-, 5-, 6- oder 7-gliedrigen Gruppen über zwei gemeinsame, benachbarte C-Atome mit einem Phenyl- oder Pyridin-Ring kondensiert verbunden sein können, und
wobei in den zuvor genannten 5-, 6- oder 7-gliedrigen Gruppen eine oder zwei nicht benachbarte -$CH_2$-Gruppen unabhängig voneinander durch eine -CO-, -C(=$CH_2$)-, -(SO)- oder -($SO_2$)-Gruppe ersetzt sein können, und
wobei die zuvor angeführten gesättigten 6- oder 7-gliedrigen Gruppen auch als verbrückte Ringsysteme mit einer Imino-, ($C_{1-4}$-alkyl)-imino-, Methylen-, ($C_{1-4}$-Alkyl)-methylen- oder Di-($C_{1-4}$-alkyl)-methylen-Brücke vorliegen können, und
wobei die zuvor genannten cyclischen Gruppen ein- oder mehrfach an ein oder mehreren C-Atomen mit $R^{20}$, im Falle einer Phenylgruppe auch zusätzlich einfach mit Nitro, und/oder ein oder mehrere NH-Gruppen mit $R^{21}$ substituiert sein können,

$R^4$, $R^5$ unabhängig voneinander eine der für $R^{17}$ angegebenen Bedeutungen,

$R^{10}$ Hydroxy, ω-Hydroxy-$C_{1-3}$-alkyl, $C_{1-4}$-Alkoxy, ω-($C_{1-4}$-Alkoxy)-$C_{1-3}$-alkyl-, Carboxy, $C_{1-4}$-Alkoxycarbonyl, Amino, $C_{1-4}$-Alkyl-amino, Di-($C_{1-4}$-alkyl)-amino, Cyclo-$C_{3-6}$-alkylenimino-, Amino-$C_{1-3}$-alkyl, $C_{1-4}$-Alkyl-amino-$C_{1-3}$-alkyl, Di-($C_{1-4}$-alkyl)-amino-$C_{1-3}$-alkyl, Cyclo-$C_{3-6}$-alkylenimino-$C_{1-3}$-alkyl, Amino-$C_{2-3}$- alkoxy, $C_{1-4}$-Alkyl-amino-$C_{2-3}$-alkoxy, Di-($C_{1-4}$-alkyl)-amino-$C_{2-3}$-alkoxy, Cyclo- $C_{3-6}$-alkylenimino-$C_{2-3}$-alkoxy, Ami-

nocarbonyl-, $C_{1-4}$-Alkyl-aminocarbonyl-, Di- ($C_{1-4}$-alkyl)-aminocarbonyl-,Cyclo-$C_{3-6}$-alkylenimino-carbonyl-,

$R^{11}$ $C_{2-6}$-Alkenyl, $C_{2-6}$-Alkinyl, $R^{15}$-O-, $R^{15}$-O-CO-, $R^{15}$-CO-O-, $R^{16}R^{17}$N-, $R^{18}R^{19}$N- CO- oder Cy-,

$R^{12}$ eine der für $R^{20}$ angegebenen Bedeutungen,

$R^{13}$ eine der für $R^{17}$ angegebenen Bedeutungen, ausgenommen Carboxy,

$R^{14}$ Halogen, $C_{1-6}$-Alkyl, $C_{2-6}$-Alkenyl, $C_{2-6}$-Alkinyl, $R^{15}$-O-, $R^{15}$-O-CO-, $R^{15}$-CO-, $R^{15}$-CO-O-, $R^{16}R^{17}$N-, $R^{18}R^{19}$N-CO-, $R^{15}$-O-$C_{1-3}$-alkyl, $R^{15}$-O-CO-$C_{1-3}$-alkyl, $R^{15}$-O-CO-NH-, $R^{15}$-$SO_2$-NH-, $R^{15}$-O-CO-NH-$C_{1-3}$-alkyl-, $R^{15}$-$SO_2$-NH-$C_{1-3}$- alkyl-, $R^{15}$-CO-$C_{1-3}$-alkyl, $R^{15}$-CO-O-$C_{1-3}$-alkyl, $R^{16}R^{17}$N-$C_{1-3}$-alkyl, $R^{18}R^{19}$N-CO-$C_{1-3}$-alkyl oder Cy-$C_{1-3}$-alkyl,

$R^{15}$ H, $C_{1-4}$-Alkyl, $C_{3-7}$-Cycloalkyl, $C_{3-7}$-Ccycloalkyl-$C_{1-3}$-alkyl, Phenyl, Phenyl-$C_{1-3}$- alkyl, Pyridinyl oder Pyridinyl-$C_{1-3}$-alkyl,

$R^{16}$ H, $C_{1-6}$-Alkyl, $C_{3-7}$-Cycloalkyl, $C_{3-7}$-Ccyloalkyl-$C_{1-3}$-alkyl, $C_{4-7}$-Cycloalkenyl, $C_{4- 7}$-Cycloalkenyl-$C_{1-3}$-alkyl, ω-Hydroxy-$C_{2-3}$-alkyl, ω-($C_{1-4}$-Alkoxy)-$C_{2-3}$-alkyl, Amino-$C_{2-6}$-alkyl, $C_{1-4}$-Alkyl-amino-$C_{2-6}$-alkyl, Di-($C_{1-4}$-alkyl)-amino-$C_{2-6}$-alkyl oder Cycol-$C_{3-6}$-alkylenimino-$C_{2-6}$-alkyl-,

$R^{17}$ eine der für $R^{16}$ angegebenen Bedeutungen oder Phenyl, Phenyl-$C_{1-3}$-alkyl, Pyridinyl, Dioxolan-2-yl, -CHO, $C_{1-4}$-Alkylcarbonyl, Carboxy, Hydroxycarbonyl-$C_{1-3}$-alkyl, $C_{1-4}$-Alkoxycarbonyl-, $C_{1-4}$-Alkoxycarbonyl-$C_{1-3}$-alkyl, $C_{1-4}$-Alkylcarbonylamino-$C_{2-3}$-alkyl, N-($C_{1- 4}$-Alkylcarbonyl)-N-($C_{1-4}$-Alkyl)-amino-$C_{2-3}$-alkyl, $C_{1-4}$-Alkylsulfonyl, $C_{1- 4}$-Alkylsulfonylamino-$C_{2-3}$-alkyl oder N-($C_{1-4}$-Alkylsulfonyl)-N(-$C_{1-4}$-Alkyl)- amino-$C_{2-3}$-alkyl

$R^{18}$, $R^{19}$ unabhängig voneinander H oder $C_{1-6}$-Alkyl,

$R^{20}$ Halogen, Hydroxy, Cyano, $C_{1-6}$-Alkyl, $C_{2-6}$-Alkenyl, $C_{2-6}$-Alkinyl, $C_{3-7}$- Cycloalkyl, $C_{3-7}$-Cycloalkyl- $C_{1-3}$-alkyl, Hydroxy-$C_{1-3}$-alkyl, $R^{22}$-$C_{1-3}$-alkyl oder eine der für $R^{22}$ angegebenen Bedeutungen,

$R^{21}$ $C_{1-4}$-Alkyl, ω-Hydroxy-$C_{2-6}$-alkyl, ω-$C_{1-4}$-Alkoxy-$C_{2-6}$-alkyl, ω-$C_{1-4}$-Alkyl-amino- $C_{2-6}$-alkyl, ω-Di-($C_{1-4}$-alkyl)-amino-$C_{2-6}$-alkyl, ω-Cyclo-$C_{3-6}$-alkylenimino-$C_{2-6}$- alkyl, Phenyl, Phenyl-$C_{1-3}$-alkyl, $C_{1-4}$-Alkyl-carbonyl, $C_{1-4}$-Alkoxy-carbonyl, $C_{1- 4}$-Alkylsulfonyl, Phenylcarbonyl oder Phenyl-$C_{1-3}$-alkyl-carbonyl,

$R^{22}$ Pyridinyl, Phenyl, Phenyl-$C_{1-3}$-alkoxy, OHC-, HO-N=HC-, $C_{1-4}$-Alkoxy-N=HC-, $C_{1-4}$-Alkoxy, $C_{1-4}$-Alkylthio, Carboxy, $C_{1-4}$-Alkylcarbonyl, $C_{1-4}$-Alkoxycarbonyl, Aminocarbonyl, $C_{1-4}$-Alkylaminocarbonyl, Di-($C_{1-4}$-alkyl)-aminocarbonyl, Cyclo-$C_{3-6}$-alkyl-amino-carbonyl-,Cyclo-$C_{3-6}$-alkylenimino-carbonyl, Cyclo-$C_{3- 6}$- alkylenimino-$C_{2-4}$-alkyl-aminocarbonyl, $C_{1-4}$-Alkyl-sulfonyl, $C_{1-4}$-Alkyl-sulfinyl, $C_{1-4}$-Alkyl-sulfonylamino, Amino, $C_{1-4}$-alkylamino, Di-($C_{1-4}$-alkyl)-amino, $C_{1-4}$- Alkyl-carbonyl-amino, Cyclo-$C_{3-6}$-alkylenimino, Phenyl-$C_{1-3}$-alkylamino, N-($C_{1- 4}$-Alkyl)-phenyl-$C_{1-3}$-alkylamino, Acetylamino-, Propionylamino, Phenylcarbonyl, Phenylcarbonylamino, Phenylcarbonylmethylamino, Hy- droxy-$C_{2-3}$-alkylaminocarbonyl, (4-Morpholinyl)carbonyl, (1-Pyrrolidinyl)- carbonyl, (1-Piperidinyl)carbonyl, (Hexahydro-1-azepinyl)carbonyl, (4-Methyl- 1-piperazinyl)carbonyl, Methylendioxy, Aminocarbonylamino oder Alkylaminocarbonylamino bedeuten,

wobei in den zuvor genannten Gruppen und Resten, insbesondere in A, B, W, X, Y, Z, $R^1$ bis $R^5$ und $R^{10}$ bis $R^{22}$, jeweils ein oder mehrere C-Atome zusätzlich ein- oder mehrfach mit F und/oderjeweils ein oder zwei C-Atome unabhängig voneinander zusätzlich einfach mit Cl oder Br und/oder jeweils ein oder mehrere Phenyl-Ringe unabhängig voneinander zusätzlich ein, zwei oder drei Substituenten ausgewählt aus der Gruppe F, Cl, Br, I, Cyano, $C_{1-4}$-alkyl, $C_{1-4}$-alkoxy-, Difluormethyl-, Trifluormethyl-, Hydroxy-, Amino-, $C_{1-3}$-alkylamino-, Di-($C_{1-3}$-alkyl)-amino-, Acetylamino-, Aminocarbonyl-, Difluormethoxy-, Trifluormethoxy-, Amino-$C_{1-3}$-alkyl-, $C_{1-3}$-alkylamino-$C_{1-3}$-alkyl- und Di-($C_{1-3}$-Alkyl)-amino-$C_{1-3}$-alkyl- aufweisen und/oder einfach mit Nitro substituiert sein können, und
das H-Atom einer vorhandenen Carboxygruppe oder ein an ein N-Atom gebundenes H-Atom jeweils durch einen in-vivo abspaltbaren Rest ersetzt sein kann,
deren Tautomere, deren Diastereomere, deren Enantiomere, deren Gemische und deren Salze.

Gegenstand der Erfindung sind auch die jeweiligen Verbindungen in Form der einzelnen optischen Isomeren, Mischungen der einzelnen Enantiomeren oder Racemate, in Form der Tautomere sowie in Form der freien Basen oder der entsprechenden Säureadditionssalze mit pharmakologisch unbedenklichen Säuren. Ebenfalls mit vom Gegenstand

dieser Erfindung umfasst sind die erfindungsgemäßen Verbindungen, einschließlich deren Salze, in denen ein oder mehrere Wasserstoffatome durch Deuterium ausgetauscht sind.

Ferner sind die physiologisch verträglichen Salze der vorstehend und nachfolgend beschriebenen erfindungsgemäßen Alkin-Verbindungen ebenfalls ein Gegenstand dieser Erfindung.

Ebenfalls eine Gegenstand dieser Erfindung sind Zusammensetzungen, enthaltend mindestens eine erfindungsgemäße Alkin-Verbindung und/ oder ein erfindungsgemäßes Salz neben gegebenenfalls einem oder mehreren physiologisch verträglichen Hilfsstoffen.

Weiterhin sind Arzneimittel, enthaltend mindestens eine erfindungsgemäße Alkin-Verbindung und/ oder ein erfindungsgemäßes Salz neben gegebenenfalls einem oder mehreren inerten Trägerstoffen und/oder Verdünnungsmitteln Gegenstand der vorliegenden Erfindung.

Ebenfalls ein Gegenstand dieser Erfindung ist die Verwendung mindestens einer erfindungsgemäßen Alkin-Verbindung und/ oder eines erfindungsgemäßen Salzes zur Beeinflussung des Essverhaltens eines Säugetiers.

Weiterhin ist die Verwendung mindestens einer erfindungsgemäßen Alkin-Verbindung und/ oder eines erfindungsgemäßen Salzes zur Reduzierung des Körpergewichts und/ oder zum Verhindern einer Zunahme des Körpergewichts eines Säugetiers ein Gegenstand dieser Erfindung.

Ebenfalls ein Gegenstand der vorliegenden Erfindung ist die Verwendung mindestens einer erfindungsgemäßen Alkin-Verbindung und/ oder eines erfindungsgemäßen Salzes zur Herstellung eines Arzneimittels mit MCH-Rezeptor antagonistischer Aktivität, insbesondere mit MCH-1 Rezeptor antagonistischer Aktivität.

Darüber hinaus ist ein Gegenstand dieser Erfindung die Verwendung mindestens einer erfindungsgemäßen Alkin-Verbindung und/ oder eines erfindungsgemäßen Salzes zur Herstellung eines Arzneimittels, welches zur Prophylaxe und/oder Behandlung von Erscheinungen und/oder Krankheiten, die durch MCH verursacht werden oder mit MCH in einem anderen kausalen Zusammenhang stehen, geeignet ist.

Ein weiterer Gegenstand dieser Erfindung ist die Verwendung mindestens einer erfindungsgemäßen Alkin-Verbindung und/ oder eines erfindungsgemäßen Salzes zur Herstellung eines Arzneimittels, welches zur Prophylaxe und/oder Behandlung von metabolischen Störungen und/oder Essstörungen, insbesondere von Obesitas, Bulimie, Bulimie nervosa, Cachexia, Anorexie, Anorexie nervosa und Hyperphagia, geeignet ist.

Ebenfalls ein Gegenstand dieser Erfindung liegt in der Verwendung mindestens einer erfindungsgemäßen Alkin-Verbindung und/ oder eines erfindungsgemäßen Salzes zur Herstellung eines Arzneimittels, welches zur Prophylaxe und/oder Behandlung von mit Obesitas einhergehenden Krankheiten und/oder Störungen, insbesondere von Diabetes, besonders Typ II Diabetes, diabetischen Komplikationen, einschließlich diabetischer Retinopathie, diabetischer Neuropathie, diabetischer Nephropathie, Insulin-Resistenz, pathologischer Glukosetoleranz, Encephalorrhagie, Herzinsuffizienz, Herzkreislauferkrankungen, insbesondere Arteriosklerose und Bluthochdruck, Arthritis und Gonitis geeignet ist.

Darüber hinaus hat die vorliegende Erfindung die Verwendung mindestens einer erfindungsgemäßen Alkin-Verbindung und/ oder eines erfindungsgemäßen Salzes zur Herstellung eines Arzneimittels, welches zur Prophylaxe und/oder Behandlung von Hyperlipidämie, Cellulitis, Fettakkumulation, maligne Mastocytose, systemische

Mastocytose, emotionalen Störungen, Affektivitätsstörungen, Depressionen, Angstzuständen, Schlafstörungen, Fortpflanzungsstörungen, sexuelle Störungen, Gedächtnisstörungen, Epilepsie, Formen der Dementia und hormonelle Störungen geeignet ist, zum Gegenstand.

Weiterhin ein Gegenstand dieser Erfindung ist die Verwendung mindestens einer erfindungsgemäßen Alkin-Verbindung und/ oder eines erfindungsgemäßen Salzes zur Herstellung eines Arzneimittels, welches zur Prophylaxe und/oder Behandlung von Miktionsstörungen, wie beispielsweise Harninkontinenz, überaktiver Harnblase, Harndrang, Nykturie und Enuresis, geeignet ist.

Darüber hinaus bezieht sich ein Gegenstand dieser Erfindung auf Verfahren zur Herstellung eines erfindungsgemäßen Arzneimittels, dadurch gekennzeichnet, dass auf nichtchemischem Wege mindestens eine erfindungsgemäße Alkin-Verbindung und/ oder ein erfindungsgemäßes Salz in einen oder mehrere inerte Trägerstoffe und/oder Verdünnungsmittel eingearbeitet wird.

Ein weiterer Gegenstand dieser Erfindung ist ein Arzneimittel, enthaltend einen ersten Wirkstoff, der aus den erfindungsgemäßen Alkin-Verbindungen und/ oder den entsprechenden Salzen ausgewählt ist, sowie einen zweiten Wirkstoff, der aus der Gruppe ausgewählt ist bestehend aus Wirkstoffen zur Behandlung von Diabetes, Wirkstoffen zur Behandlung diabetischer Komplikationen, Wirkstoffen zur Behandlung von Obesitas, vorzugsweise anderen als MCH-Antagonisten, Wirkstoffen zur Behandlung von Bluthochdruck, Wirkstoffen zur Behandlung von Hyperlipidemia, einschließlich Arteriosklerose, Wirkstoffen zur Behandlung von Arthritis, Wirkstoffen zur Behandlung von Angstzuständen und Wirkstoffen zur Behandlung von Depressionen, neben gegebenenfalls einem oder mehreren inerten Trägerstoffen und/oder Verdünnungsmitteln.

Des weiteren betrifft ein Gegenstand dieser Erfindung ein Verfahren zur Herstellung von Alkin-Verbindungen der Formel A.5

$R^1R^2N$-X-Y-C=C-W-A-B (A.5)

wobei in den Formeln A.1, A.2, A.3, A.4 und A.5 $R^1$, $R^2$, X, Y, W, A und B eine der in den zuvor und nachfolgend angegebenen Bedeutungen besitzen und Y Aryl- oder Heteroaryl bedeutet,
bei dem eine Halogenverbindung der Formel A.1

HO-X-Y-Hal (A.1)

worin Hal Chlor, Brom oder lod, vorzugsweise Brom oder lod, bedeutet,
mit einer Alkinverbindung der Formel A.2

H-C=C-W-A-B (A.2)

in Gegenwart eines geeigneten Palladium-Katalysators, einer geeigneten Base und Kupfer(I)iodid in einem geeigneten Lösungsmittel umgesetzt wird, und
die erhaltene Verbindung der Formel A.3

HO-X-Y-C=C-W-A-B (A.3)

mit Methansulfonsäurechlorid (MsCl) zum Methansulfonat-Derivat A.4 umgesetzt wird,

MsO-X-Y-C=C-W-A-B (A.4)

das mit einem Amin der Formel H-$NR^1R^2$ zu dem Endprodukt A.5 weiter umgesetzt wird.

Ein weiterer Gegenstand dieser Erfindung ist ein Verfahren zur Herstellung von Alkin-Verbindungen der Formel B.5

$R^1R^2N$-X-Y-Z-C=C-A-B (B.5)

wobei in den Formeln B.1, B.2, B.3, B.4 und B.5 $R^1$, $R^2$, X, Y, Z, A und B eine der zuvor und nachfolgend angegebenen Bedeutungen besitzen und A speziell Aryl- oder Heteroaryl bedeutet,
bei dem eine Halogenverbindung der Formel B.1

Hal-A-B (B.1)

worin Hal Chlor,Brom oder lod, vorzugsweise Brom oder lod, bedeutet,
mit einer Alkinverbindung der Formel B.2

HO-X-Y-Z-C=C-H (B.2)

in Gegenwart eines geeigneten Palladium-Katalysators, einer geeigneten Base und Kupfer(I)iodid in einem geeigneten Lösungsmittel umgesetzt wird, und
die erhaltene Verbindung der Formel B.3

HO-X-Y-Z-C=C-A-B (B.3)

mit Methansulfonsäurechlorid (MsCl) zum Methansulfonat-Derivat B.4 umgesetzt wird,

MsO-X-Y-Z-C=C-A-B (B.4)

das mit einem Amin der Formel H-$NR^1R^2$ zu dem Endprodukt B.5 weiter umgesetzt wird.

Ferner betrifft ein Gegenstand dieser Erfindung ein Verfahren zur Herstellung von Alkin-Verbindungen der Formel C.3

$R^1R^2N$-X-Y-C=C-W-A-B (C.3)

wobei in den Formeln C.1, C.2 und C.3 $R^1$, $R^2$, X, Y, W, A und B eine der zuvor und nachfolgend angegebenen Bedeutungen besitzen und Y Aryl- oder Heteroaryl bedeutet,

bei dem eine Halogenverbindung der Formel C.1

$R^1R^2N$-X-Y-Hal (C.1)

worin Hal Chlor, Brom oder Iod, vorzugsweise Brom oder Iod, bedeutet,
mit einer Alkinverbindung der Formel C.2

H-C≡C-W-A-B (C.2)

in Gegenwart eines geeigneten Palladium-Katalysators, einer geeigneten Base und Kupfer(I)iodid in einem geeigneten Lösungsmittel zu dem Endprodukt C.3 weiter umgesetzt wird.

Ein weiterer Gegenstand dieser Erfindung ist ein Verfahren zur Herstellung von Alkin-Verbindungen der Formel D.3

$R^1R^2N$-X-Y-Z-C≡C-A-B (D.3)

wobei in den Formeln D.1, D.2 und D.3 $R^1$, $R^2$, X, Y, Z, A und B eine der zuvor und nachfolgend angegebenen Bedeutungen besitzen und A speziell Aryl- oder Heteroaryl bedeutet,
bei dem eine Halogenverbindung der Formel D.2

Hal-A-B (D.2)

worin Hal Chlor, Brom oder Iod, vorzugsweise Brom oder Iod, bedeutet,
mit einer Alkinverbindung der Formel D.1

$R^1R^2N$-X-Y-Z-C≡C-H (D.1)

in Gegenwart eines geeigneten Palladium-Katalysators, einer geeigneten Base und Kupfer(I)iodid in einem geeigneten Lösungsmittel zu dem Endprodukt D.3 umgesetzt wird.

## Detailierte Beschreibung der Erfindung

Sofern nicht anders angegeben besitzen die Gruppen, Reste und Substituenten, insbesondere A, B, W, X, Y, Z, $R^1$ bis $R^5$ und $R^{10}$ bis $R^{22}$, die zuvor und nachfolgend angegebenen Bedeutungen.

Gemäß einer Ausführungsform der Erfindung besitzen die Gruppen $R^1$, $R^2$, X, W, Z, B, $R^{10}$ $R^{13}$, $R^{14}$, $R^{15}$, $R^{17}$, $R^{20}$, $R^{22}$ folgende Bedeutungen:

$R^1$, $R^2$ unabhängig voneinander H, eine gegebenenfalls mit dem Rest $R^{11}$ substituierte $C_{1-8}$-Alkyl- oder $C_{3-7}$-Cycloalkyl-Gruppe oder ein gegebenenfalls mit dem Rest $R^{12}$ ein- oder mehrfach und/oder mit Nitro einfach substituierter Phenylrest, oder
$R^1$ und $R^2$ bilden eine $C_{2-8}$-Alkylen-Brücke, in der

- ein oder zwei $-CH_2$-Gruppen unabhängig voneinander durch -CH=N- oder -CH=CH- ersetzt sein können und/oder
- ein oder zwei $-CH_2$-Gruppen unabhängig voneinander durch -O-, -S-, -CO-, $-C(=CH_2)$- oder $-NR^{13}$- derart ersetzt sein können, dass Heteroatome nicht unmittelbar miteinander verbunden sind,

wobei in der zuvor definierten Alkylen-Brücke ein oder mehrere H-Atome durch $R^{14}$ ersetzt sein können, und wobei die zuvor definierte Alkylen-Brücke mit einer oder zwei gleichen oder verschiedenen carbo- oder heterocyclischen Gruppen Cy derart substituiert sein kann, dass die Bindung zwischen der Alkylenbrücke und der Gruppe Cy

- über eine Einfach- oder Doppelbindung,
- über ein gemeinsames C-Atom unter Ausbildung eines spirocyclischen Ringsystems,
- über zwei gemeinsame, benachbarte C- und/oder N-Atome unter Ausbildung eines kondensierten bicyclischen Ringsystems oder
- über drei oder mehrere C- und/oder N-Atome unter Ausbildung eines verbrückten Ringsystems erfolgt,

X eine Einfachbindung oder eine $C_{1-6}$-Alkylen-Brücke, in der

-eine $-CH_2$-Gruppe durch -CH=CH- oder -C≡C- ersetzt sein kann und/oder
-ein oder zwei $-CH_2$-Gruppen unabhängig voneinander durch -O-, -S-, - (SO)-, $-(SO_2)$-, -CO- oder $-NR^4$- derart ersetzt sein können, dass jeweils zwei O-, S- oder N-Atome oder ein O- mit einem S-Atom nicht unmittelbar miteinander verbunden sind,

wobei die Brücke X mit $R^1$ unter Einschluss des mit $R^1$ und X verbundenen N-Atoms unter Ausbildung einer heterocyclischen Gruppe verbunden sein kann, und
wobei zwei C-Atome oder ein C- und ein N-Atom der Alkylenbrücke durch eine zusätzliche $C_{1-4}$-Alkylen-Brücke miteinander verbunden sein können, und
wobei ein C-Atom mit $R^{10}$ und/oder ein oder zwei C-Atome jeweils mit einem oder zwei gleichen oder verschiedenen $C_{1-6}$-Alkyl-Resten substituiert sein können, und

W, Z unabhängig voneinander eine Einfachbindung oder eine $C_{1-4}$-Alkylen- Brücke,
wobei in der Gruppe W und/oder Z eine nicht mit der -C≡C-Gruppe benachbarte $-CH_2$-Gruppe durch -O- oder $-NR^5$- ersetzt sein kann, und
wobei zwei benachbarte C-Atome oder ein C-Atom und ein benachbartes N-Atom mit einer zusätzlichen $C_{1-4}$-Alkylen-Brücke miteinander verbunden sein können, und
wobei in der Alkylen-Brücke und/oder in der zusätzlichen Alkylen-Brücke ein C-Atom mit $R^{10}$ und/oder ein oder zwei C-Atome unabhängig voneinander mit einem oder zwei gleichen oder verschiedenen $C_{1-6}$-Alkyl-Resten substituiert sein können, und

B eine der für Cy angegebenen Bedeutungen oder
$C_{1-6}$-Alkyl, $C_{1-6}$-Alkenyl, $C_{1-6}$-Alkinyl, $C_{3-7}$-Cycloalkyl-$C_{1-3}$-alkyl-, $C_{3-7}$- Cycloalkenyl-$C_{1-3}$-alkyl-, $C_{3-7}$-Cycloalkyl-$C_{1-3}$-alkenyl- oder $C_{3-7}$-Cycloalkyl- $C_{1-3}$-alkinyl-, worin ein oder mehrere C-Atome ein- oder mehrfach mit Fluor und cyclische Gruppen ein- oder mehrfach mit $R^{20}$ substituiert sein können,

$R^{10}$ Hydroxy, ω-Hydroxy-$C_{1-3}$-alkyl, $C_{1-4}$-Alkoxy, ω-($C_{1-4}$-Alkoxy)-$C_{1-3}$-alkyl-, Amino, $C_{1-4}$-Alkyl-amino, Di-($C_{1-4}$-alkyl)-amino, Cyclo-$C_{3-6}$-alkylenimino-, Amino-$C_{1-3}$-alkyl, $C_{1-4}$-Alkyl-amino-$C_{-3}$-alkyl, Di-($C_{1-4}$-alkyl)-amino-$C_{1-3}$- alkyl, Cyclo-$C_{3-6}$-alkylenimino-$C_{1-3}$-alkyl, Amino-$C_{2-3}$-alkoxy, $C_{1-4}$-Alkyl- amino-$C_{2-3}$-alkoxy, Di-($C_{1-4}$-alkyl)-amino-$C_{2-3}$-alkoxy oder Cyclo-$C_{3-6}$- alkylen imino-$C_{2-3}$-alkoxy,

$R^{13}$ eine der für $R^{17}$ angegebenen Bedeutungen,

$R^{14}$ Halogen, $C_{1-6}$-Alkyl, $R^{15}$-O-, $R^{15}$-O-CO-, $R^{15}$-CO-, $R^{15}$-CO-O-, $R^{16}R^{17}$N-, $R^{18}R^{19}$N-C0-, $R^{15}$-O-$C_{1-3}$-alkyl , $R^{15}$-O-CO-$C_{1-3}$-alkyl, $R^{15}$-CO-$C_{1-3}$-alkyl, $R^{15}$-CO-O-$C_{1-3}$-alkyl, $R^{16}R^{17}$N-$C_{1-3}$-alkyl, $R^{18}R^{19}$N-CO-$C_{1-3}$-alkyl oder Cy- $C_{1-3}$-alkyl,

$R^{15}$ H, $C_{1-4}$-Alkyl, $C_{3-7}$-Cycloalkyl, $C_{3-7}$-Cycloalkyl-$C_{1-3}$-alkyl, Phenyl oder Phenyl-$C_{1-3}$-alkyl,

$R^{17}$ eine der für $R^{16}$ angegebenen Bedeutungen oder Phenyl, Phenyl-$C_{1-3}$-alkyl, $C_{1-4}$-Alkylcarbonyl, Hydroxycarbonyl-$C_{1-3}$-alkyl, $C_{1-4}$-Alkylcarbonylamino-$C_{2-3}$-alkyl, N-($C_{1--4}$-Alkylcarbonyl)-N-($C_{1-4}$-Alkyl)- amino-$C_{2-3}$-alkyl, $C_{1-4}$-Alkylsulfonyl, $C_{1-4}$-Alkylsulfonylamino-$C_{2-3}$-alkyloderN-($C_{1-4}$-Alkylsulfonyl)-N(-$C_{1-4}$-Alkyl)-amino-$C_{2-3}$-alkyl

$R^{20}$ Halogen, Hydroxy, Cyano, $C_{1-6}$-Alkyl, $C_{3-7}$-Cycloalkyl, $C_{3-7}$-Cycloalkyl- $C_{1-3}$- alkyl, Hydroxy-$C_{1-3}$-alkyl, $R^{22}$-$C_{1-3}$-alkyl oder eine der für $R^{22}$ angegebenen Bedeutungen,

$R^{22}$ Phenyl, Phenyl-$C_{1-3}$-alkoxy, $C_{1-4}$-Alkoxy, $C_{1-4}$-Alkylthio, Carboxy, $C_{1-4}$-Alkylcarbonyl, $C_{1-4}$-Alkoxycarbonyl, Aminocarbonyl, $C_{1-4}$-Alkylamino- carbonyl,Di-($C_{1-4}$-alkyl)-aminocarbonyl,Cyclo-$C_{3-6}$-alkylenimino-carbonyl, $C_{1-4}$-Alkyl-sulfonyl, $C_{1-4}$-Alkyl-sulfinyl, $C_{1-4}$-Alkyl-sulfonylamino, Amino, $C_{1-4}$-alkylamino, Di-($C_{1-4}$-alkyl)-amino, Cyclo-$C_{3-6}$-alkylenimino, Phe- nyl-$C_{1-3}$-alkylamino, N-($C_{1-4}$-Alkyl)-phenyl-$C_{1-3}$-alkylamino, Acetylamino-, Propionylamino, Phenylcarbonyl, Phenylcarbonylamino, Phenylcarbonylmethylamino, Hydroxyalkylaminocarbonyl, (4- Morpholinyl)carbonyl, (1-Pyrrolidinyl)carbonyl, (1-Piperidinyl)carbonyl, (Hexahydro-1-azepinyl) carbonyl, (4-Methyl-1-piperazinyl)carbonyl, Methylendioxy, Aminocarbonylamino oder Alkylaminocarbonylamino bedeuten,

wobei $R^4$, $R^{11}$, $R^{12}$ , $R^{16}$, $R^{18}$ , $R^{19}$ und Cy die zuvor angegebene Bedeutung aufweisen.
Sind $R^1$ und $R^2$ nicht über eine Alkylenbrücke miteinander verbunden, so bedeuten $R^1$ und $R^2$ unabhängig voneinander vorzugsweise eine gegebenenfalls mit dem Rest $R^{11}$ substituierte $C_{1-8}$-Alkyl- oder $C_{3-7}$-Cycloalkyl-Gruppe, wobei eine $-CH_2$-Gruppe in Position 3 oder 4 einer 5, 6 oder 7-gliedrigen Cycloalkylgruppe durch -O-, -S- oder -NH-, -N($C_{1-4}$-Alkyl)- oder -N(CO-O-$C_{1-4}$-Alkyl)- ersetzt sein kann, oder ein gegebenenfalls mit dem Rest $R^{12}$ ein- oder mehrfach und/oder mit Nitro einfach substituierter Phenyl- oder Pyridinylrest, und wobei einer der Reste $R^1$ und $R^2$ auch H bedeuten kann.

Bevorzugt bedeuten die Reste $R^1$, $R^2$ unabhängig voneinander H, $C_{1-6}$-Alkyl, $C_{3-7}$-Cycloalkyl, $C_{3-7}$-Cycloalkyl-$C_{1-3}$-alkyl, ω-Hydroxy-$C_{2-3}$-alkyl, ω-($C_{1-4}$-Alkoxy)-$C_{2-3}$-alkyl, $C_{1-4}$-Alkoxy-carbonyl-$C_{1-4}$-alkyl, Carboxyl-$C_{1-4}$-alkyl, Amino-$C_{2-4}$-alkyl, $C_{1-4}$-Alkyl-amino-$C_{2-4}$-alkyl, Di-($C_{1-4}$-alkyl)-amino-$C_{1-4}$-alkyl, Cyclo-$C_{3-6}$-alkylenimino-$C_{2-4}$-alkyl, Pyrrolidin-3-yl, N-($C_{1-4}$-alkyl)-pyrrolidinyl, Pyrrolidinyl-$C_{1-3}$-alkyl, N-($C_{1-4}$-alkyl)-pyrrolidinyl-$C_{-3}$-alkyl, Piperidinyl, N-($C_{1-4}$-alkyl)-piperidinyl, Piperidinyl-$C_{1-3}$-alkyl, N-($C_{1-4}$-alkyl)-piperidinyl-$C_{1-3}$-alkyl, Phenyl, Phenyl-$C_{1-3}$-alkyl, Pyridyl oder Pyridyl-$C_{1-3}$-alkyl, wobei in den zuvor angegebenen Gruppen und Resten ein oder mehrere C-Atome ein- oder mehrfach mit F und/oder ein oder zwei C-Atome unabhängig voneinander einfach mit Cl oder Br substituiert sein können, und wobei der Phenyl- oder Pyridylrest ein- oder mehrfach mit dem zuvor definierten Rest $R^{12}$ und/oder einfach mit Nitro substituiert sein kann. Bevorzugte Substituenten der zuvor genannten Phenyl- oder Pyridylreste sind ausgewählt aus der Gruppe F, Cl, Br, I, Cyano, $C_{1-4}$-alkyl, $C_{1-4}$-alkoxy-, Difluormethyl-, Trifluormethyl-, Hydroxy-, Amino-, $C_{1-3}$-alkylamino-, Di-($C_{1-3}$-alkyl)-amino-, Acetylamino-, Aminocarbonyl-, Difluormethoxy-, Trifluormethoxy-, Amino-$C_{1-3}$-alkyl-, $C_{1-3}$-alkylamino-$C_{1-3}$-alkyl- und Di-($C_{1-3}$-Alkyl)-amino-$C_1$3-alkyl-, wobei ein Phenylrest auch einfach mit Nitro substituiert sein kann.

Besonders bevorzugt weist mindestens einer der Reste $R^1$, $R^2$ ganz besonders bevorzugt beide Reste, eine von H verschiedene Bedeutung auf.

Bilden $R^1$ und $R^2$ eine Alkylen-Brücke, so handelt es sich hierbei bevorzugt um eine $C_{3-7}$-Alkylen-Brücke, in der

- eine nicht mit dem N-Atom der $R^1R^2N$-Gruppe benachbarte $-CH_2$-Gruppe durch -CH=N- oder -CH=CH- ersetzt sein kann und/oder
- eine $-CH_2$-Gruppe, die vorzugsweise nicht mit dem N-Atom der $R^1R^2N$-Gruppe benachbart ist, durch -O-, -S-, -C(=N-$R^{18}$)-, -CO-, -C(=$CH_2$)- oder -$NR^{13}$- derart ersetzt sein kann, dass Heteroatome nicht unmittelbar miteinander verbunden sind,

wobei in der zuvor definierten Alkylen-Brücke ein oder mehrere H-Atome durch $R^{14}$ ersetzt sein können, und wobei die zuvor definierte Alkylen-Brücke mit einer carbo- oder heterocyclischen Gruppe Cy derart substituiert sein kann, dass die Bindung zwischen der Alkylenbrücke und der Gruppe Cy

- über eine Einfachbindung,
- über ein gemeinsames C-Atom unter Ausbildung eines spirocyclischen Ringsystems,
- über zwei gemeinsame, benachbarte C- und/oder N-Atome unter Ausbildung eines kondensierten bicyclischen Ringsystems oder
- über drei oder mehrere C- und/oder N-Atome unter Ausbildung eines verbrückten Ringsystems erfolgt,

Weiterhin bevorzugt bilden $R^1$ und $R^2$ derart eine Alkylen-Brücke, dass $R^1R^2N$- eine Gruppe ausgewählt aus Azetidin, Pyrrolidin, Piperidin, Azepan, 2,5-Dihydro-1H-pyrrol, 1,2,3,6-Tetrahydro-pyridin, 2,3,4,7-Tetrahydro-1H-azepin, 2,3,6,7-Tetrahydro-1H-azepin, Piperazin, worin die freie Imin-Funktion mit $R^{13}$ substituiert ist, Piperidin-4-on, Piperidin-4-on-oxim, Piperidin-4-on-O-$C_{1-4}$-alkyl-oxim, Morpholin und Thiomorpholin bedeutet, wobei gemäß der allgemeinen Definition von $R^1$ und $R^2$ ein- oder mehrere H-Atome durch $R^{14}$ ersetzt sein können, und/ oder die zuvor genannten Gruppen in einer gemäß der allgemeinen Definition von $R^1$ und $R^2$ angegebenen Weise mit einer oder zwei gleichen oder verschiedenen carbo- oder heterocyclischen Gruppen Cy substituiert sein kann. Hierbei besonders bevorzugte Gruppen Cy sind $C_{3-7}$-Cycloalkyl, Aza-$C_{4-7}$-cycloalkyl-, insbesondere Cyclo-$C_{3-6}$-alkylenimino-, sowie 1-$C_{1-4}$-Alkyl-aza-$C_{4-7}$-cycloalkyl-.

Die von $R^1$ und $R^2$ gebildete $C_{2-8}$-Alkylen-Brücke, in der wie angegeben $-CH_2$-Gruppen ersetzt sein können, kann, wie beschrieben, mit einer oder zwei gleichen oder verschiedenen carbo- oder heterocyclischen Gruppen Cy substituiert sein.

Für den Fall, dass die Alkylenbrücke mit einer Gruppe Cy über ein Einfachbindung verbunden ist, ist Cy bevorzugt ausgewählt aus der Gruppe bestehend aus $C_{3-7}$-Cycloalkyl, Cyclo-$C_{3-6}$-alkylenimino-, 1H-Imidazol, Thienyl und Phenyl.

Für den Fall, dass die Alkylenbrücke mit einer Gruppe Cy über ein gemeinsames C-Atom unter Ausbildung eines spirocyclischen Ringsystems verbunden ist, ist Cy bevorzugt ausgewählt aus der Gruppe bestehend aus $C_{3-7}$-Cycloalkyl, Aza-$C_{4-8}$-cycloalkyl-, Oxa-$C_{4-8}$-Cycloalkyl-, 2,3-Dihydro-1H-chinazolin-4-on.

Für den Fall, dass die Alkylenbrücke mit einer Gruppe Cy über zwei gemeinsame, benachbarte C- und/oder

N-Atome unter Ausbildung eines kondensierten bicyclischen Ringsystems verbunden ist, ist Cy bevorzugt ausgewählt aus der Gruppe bestehend aus $C_{4-7}$-Cycloalkyl, Phenyl, Thienyl.

Für den Fall, dass die Alkylenbrücke mit einer Gruppe Cy über drei oder mehrere C- und/oder N-Atome unter Ausbildung eines verbrückten Ringsystems verbunden ist, bedeutet Cy bevorzugt $C_{4-8}$-Cycloalkyl oder Aza-$C_{4-8}$-Cycloalkyl.

Besonders bevorzugt besitzt die Gruppe

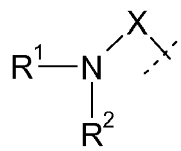

eine Bedeutung gemäß einer der folgenden Teilformeln

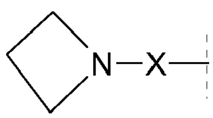 , 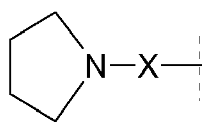 , 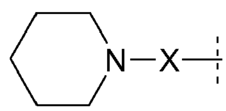 ,

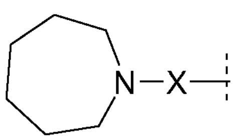 , 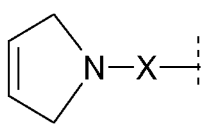 , 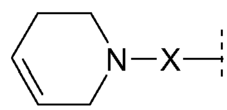 ,

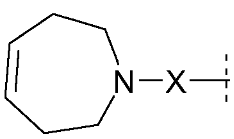 , 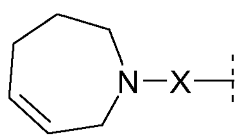 , 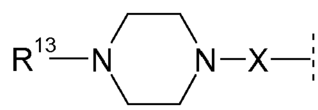

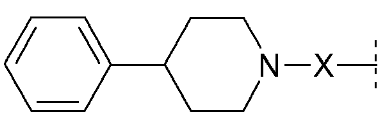 , 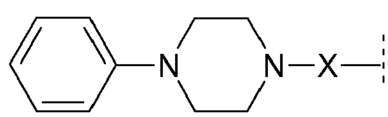 ,

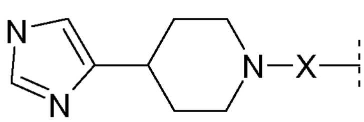 , 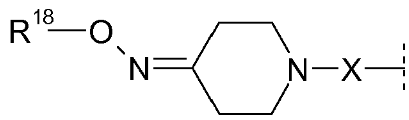 ,

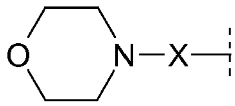 , 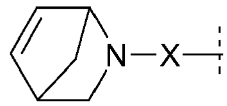 , 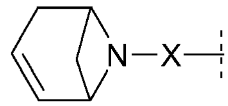 ,

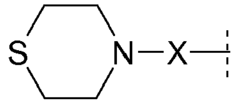 , 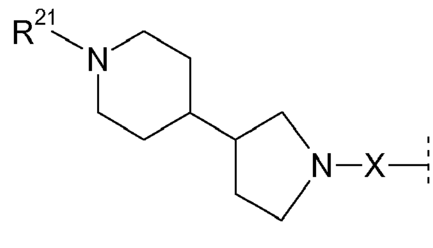 ,

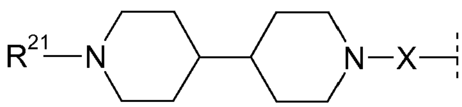 , 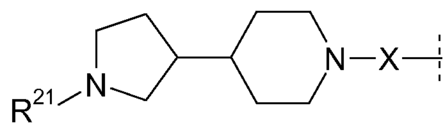 ,

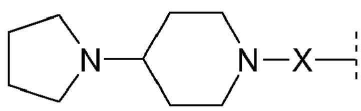 , 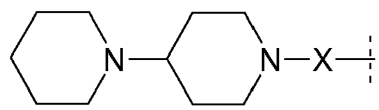 ,

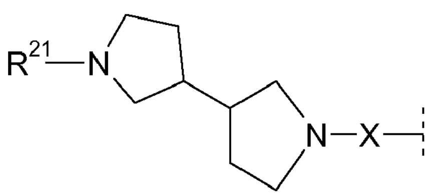 , 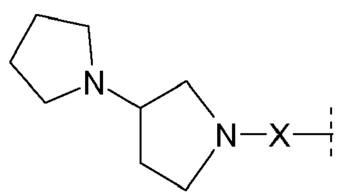 ,

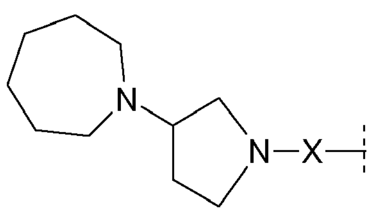 ,

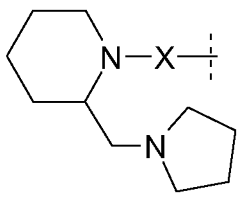 , 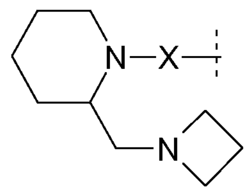 ,

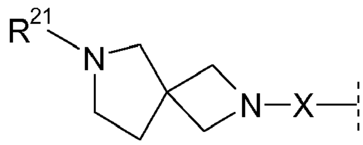 , ,

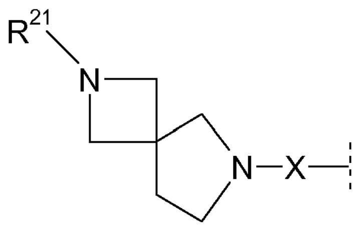 , 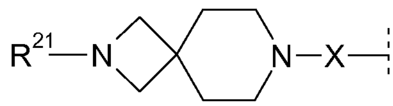 ,,

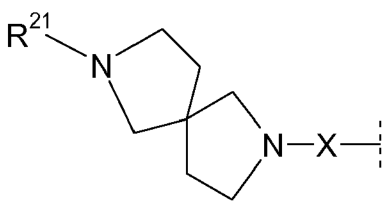 , 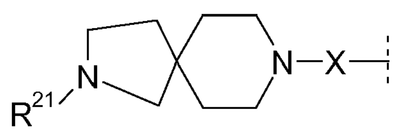 ,

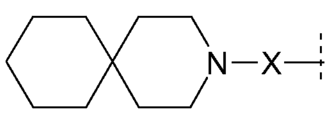 , 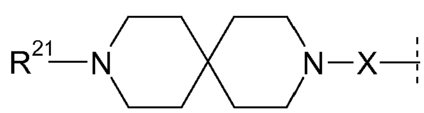 ,

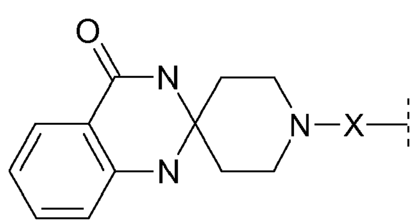 ,

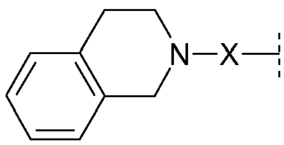 , 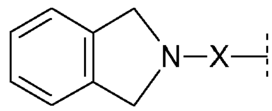 , 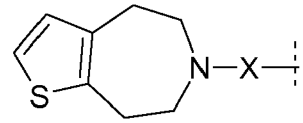 ,

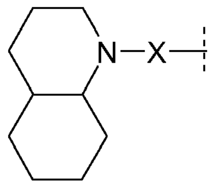 , 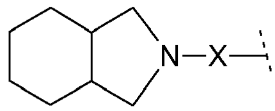 , 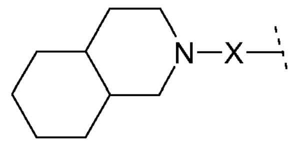

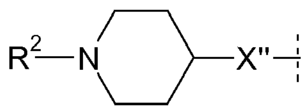 , 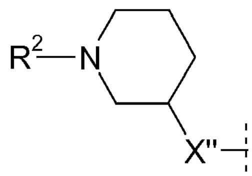 , 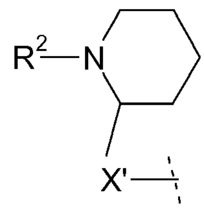 ,

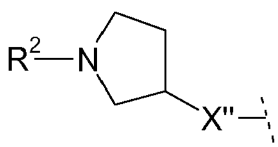 , 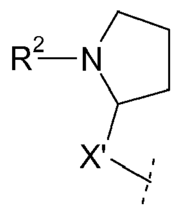 ,

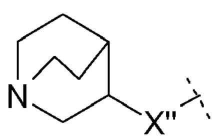 , 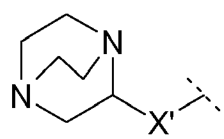 , 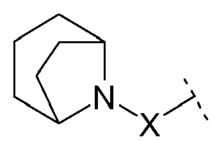 ,

worin ein- oder mehrere H-Atome des durch die Gruppe $R^1R^2N$- gebildeten

Heterocyclus durch $R^{14}$ ersetzt sein können und der mit dem durch die Gruppe $R^1R^2N$-gebildeten Heterocyclus verbundene Ring ein- oder mehrfach an einem oder mehreren C-Atomen mit $R^{20}$, im Falle eines Phenyl-Rings auch zusätzlich einfach mit Nitro substituiert sein kann und

X', X" unabhängig voneinander eine Einfachbindung oder $C_{1-3}$-Alkylen und
für den Fall, dass die Gruppe Y über ein C-Atom mit X' bzw. X" verbunden ist, auch -$C_{1-3}$-Alkylen-O-, -$C_{1-3}$-Alkylen-NH- oder -$C_{1-3}$-Alkylen-N($C_{1-3}$-alkyl)-, und

X" zusätzlich auch -O-$C_{1-3}$-Alkylen, -NH-$C_{1-3}$-Alkylen oder -N($C_{1-3}$-alkyl)-$C_{1-3}$-Alkylen und
für den Fall, dass die Gruppe Y über ein C-Atom mit X" verbunden ist, auch -NH-, -N($C_{1-3}$-alkyl)- oder -O- bedeutet,
wobei in den zuvor für X', X" genannten Bedeutungen jeweils ein C-Atom mit $R^{10}$, vorzugsweise mit einem

Hydroxy-, ω-Hydroxy-$C_{1-3}$-alkyl-, ω ($C_{1-4}$-Alkoxy)-$C_{1-3}$-alkyl- und/oder $C_{1-4}$-Alkoxy-Rest, und/oder ein oder zwei C-Atome jeweils mit einem oder zwei gleichen oder verschiedenen Substituenten ausgewählt aus $C_{1-6}$-Alkyl-, $C_{2-6}$-Alkenyl-, $C_{2-6}$-Alkinyl-, $C_{3-7}$- Cycloalkyl, $C_{3-7}$-Cycloalkyl-$C_{1-3}$-alkyl, $C_{4-7}$-Cycloalkenyl und $C_{4-7}$- Cycloalkenyl-$C_{1-3}$-alkyl substituiert sein können, wobei zwei Alkyl- und/oder Alkenyl-Substituenten unter Ausbildung eines carbocyclischen Ringsystems miteinander verbunden sein können, und
wobei in X', X" unabhängig voneinander jeweils ein oder mehrere C-Atome ein- oder mehrfach mit F und/oder jeweils ein oder zwei C-Atome unabhängig voneinander einfach mit Cl oder Br substituiert sein können und

worin $R^2$, $R^{10}$, $R^{13}$, $R^{14}$, $R^{20}$, $R^{21}$ und X die zuvor und nachstehend angegebenen Bedeutungen besitzen.

In den zuvor aufgeführten bevorzugten und besonders bevorzugten Bedeutungen von $R^1R^2N$ sind folgende Definitionen des Substituenten $R^{14}$ bevorzugt: $C_{1-4}$-Alkyl, $C_{2-4}$-Alkenyl, $C_{2-4}$-Alkinyl, $C_{3-7}$-Cycloalkyl, $C_{3-7}$-Cycloalkyl-$C_{1-3}$-alkyl, Hydroxy, ω-Hydroxy-$C_{1-3}$-alkyl, $C_{1-4}$-Alkoxy, ω-($C_{1-4}$-Alkoxy)-$C_{1-3}$-alkyl, $C_{1-4}$-Alkyl-carbonyl-, Carboxy, $C_{1-4}$-Alkoxycarbonyl-, Hydroxy-carbonyl-$C_{1-3}$-alkyl, $C_{1-4}$-Alkoxycarbonyl-$C_{1-3}$-alkyl, $C_{1-4}$-Alkoxy-carbonylamino-, $C_{1-4}$-Alkoxy-carbonylamino-$C_{1-3}$-alkyl,Amino-,$C_{1-4}$-Alkyl-amino-,$C_{3-7}$-Cycloalkyl-amino-,N-($C_{3-7}$-Cycloalkyl)-N-($C_{1-4}$-alkyl)-amino-, Di-($C_{1-4}$-alkyl)-amino-, Amino-$C_{1-3}$-alkyl-, $C_{1-4}$-Alkyl-amino-$C_{1-3}$-alkyl-, $C_{3-7}$-Cycloalkyl-amino-$C_{1-3}$-alkyl-, N-($C_{3-7}$-Cycloalkyl)-N-($C_{1-4}$-alkyl)-amino-$C_{1-3}$-alkyl-, Di-($C_{1-4}$-alkyl)-amino-$C_{1-3}$-alkyl-, Cyclo-$C_{3-6}$-alkylenimino-$C_{1-3}$-alkyl-,Aminocarbonyl-,$C_{1-4}$-Alkyl-aminocarbonyl-,$C_{3-7}$-Cycloalkyl-amino-carbonyl-,N-($C_{3-7}$-Cycloalkyl)-N-($C_{1-4}$-alkyl)-amino-carbonyl-, Di-($C_{1-4}$-alkyl)-amino-carbonyl-, Pyridinyl-oxy-, Pyridinyl-amino-, Pyridinyl-$C_{1-3}$-alkyl-amino-.

Ganz besonders bevorzugte Bedeutungen des Substituenten $R^{14}$ sind $C_{1-4}$-Alkyl, Hydroxy, ω-Hydroxy-$C_{1-3}$-alkyl, $C_{1-4}$-Alkoxy und ω-($C_{1-4}$-Alkoxy)-$C_{1-3}$-alkyl.

Vorzugsweise bedeutet X eine Einfachbindung oder eine $C_{1-4}$-Alkylen-Brücke, in der

- eine -$CH_2$-Gruppe durch -CH=CH- oder -C≡C- ersetzt sein kann und/oder
- eine -$CH_2$-Gruppe durch -O-, -S-, -CO- oder -$NR^4$- derart ersetzt sein kann, dass jeweils zwei O-, S- oder N-Atome oder ein O-mit einem S-Atom nicht unmittelbar miteinander verbunden sind,

wobei die Brücke X mit $R^1$ unter Einschluss des mit $R^1$ und X verbundenen N-Atoms unter Ausbildung einer heterocyclischen Gruppe verbunden sein kann, wobei die Brücke X zusätzlich auch mit $R^2$ unter Einschluss des mit $R^2$ und X verbundenen N-Atoms unter Ausbildung einer heterocyclischen Gruppe verbunden sein kann, und
wobei zwei C-Atome oder ein C- und ein N-Atom der Alkylenbrücke durch eine zusätzliche $C_{1-4}$-Alkylen-Brücke miteinander verbunden sein können, und
wobei ein C-Atom mit $R^{10}$ und/oder ein oder zwei C-Atome jeweils mit einem oder zwei gleichen oder verschiedenen Substituenten ausgewählt aus $C_{1-6}$-Alkyl-, $C_{2-6}$-Alkenyl-, $C_{2-6}$-Alkinyl-, $C_{3-7}$-Cycloalkyl, $C_{3-7}$-Cycloalkyl-$C_{1-3}$-alkyl, $C_{4-7}$-Cycloalkenyl und $C_{4-7}$-Cycloalkenyl-$C_{1-3}$-alkyl substituiert sein können, wobei zwei Alkyl- und/oder Alkenyl-Substituenten unter Ausbildung eines carbocyclischen Ringsystems, insbesondere einer Cyclopropyl-, Cyclobutyl- oder Cyclopentylgruppe, miteinander verbunden sein können.

Ist in der Gruppe X eine -$CH_2$-Gruppe der Alkylen-Brücke erfindungsgemäß ersetzt, so ist diese -$CH_2$-Gruppe vorzugsweise nicht mit einem Heteroatom, einer Doppel- oder Dreifachbindung unmittelbar verbunden.

Vorzugsweise weist die Alkylen-Brücke X, X' oder X" keine oder maximal eine Imino-Gruppe auf. Die Position der Imino-Gruppe innerhalb der Alkylenbrücke X, X' oder X" ist vorzugsweise derart gewählt, dass zusammen mit der Aminogruppe $NR^1R^2$ oder einer anderen benachbarten Aminogruppe keine Aminalfunktion gebildet wird oder zwei N-Atome nicht miteinander benachbart sind.

Bevorzugt bedeutet X eine Einfachbindung oder $C_{1-4}$-Alkylen und
für den Fall, dass die Gruppe Y über ein C-Atom mit X verbunden ist, auch -$CH_2$-CH=CH-, -$CH_2$-C≡C-, $C_{2-4}$-Alkylenoxy, C2_4-Alkylen-$NR^4$ $C_{2-4}$-Alkylen-$NR^4$-$C_{2-4}$-Alkylen-O-, 1,2- oder 1,3-Pyrrolidinylen oder 1,2-, 1,3- oder 1,4-Piperidinylen, wobei die Bindung der Pyrrolidinylen- und Piperidinylen-Gruppe an Y über die Imino-Gruppe erfolgt,
wobei die Brücke X mit $R^1$ unter Einschluss des mit $R^1$ und X verbunden N-Atoms unter Ausbildung einer heterocyclischen Gruppe verbunden sein kann, und wobei die Brücke X zusätzlich auch mit $R^2$ unter Einschluss des mit $R^2$ und X verbundenen N-Atoms unter Ausbildung einer heterocyclischen Gruppe verbunden sein kann, und
wobei in X ein C-Atom mit $R^{10}$ und/oder ein oder zwei C-Atome jeweils mit einem oder zwei gleichen oder verschiedenen Substituenten ausgewählt aus $C_{1-6}$-Alkyl-, $C_{2-6}$-Alkenyl-, $C_{2-6}$-Alkinyl-, $C_{3-7}$-Cycloalkyl, $C_{3-7}$-Cycloalkyl-$C_{1-3}$-alkyl, $C_{4-7}$-Cycloalkenyl und $C_{4-7}$-Cycloalkenyl-$C_{1-3}$-alkyl substituiert sein können, wobei zwei Alkyl- und/oder Alkenyl-Substituenten unter Ausbildung eines carbocyclischen Ringsystems miteinander verbunden sein können, und
wobei in den zuvor angegebenen Gruppen und Resten ein oder mehrere C-Atome ein- oder mehrfach mit F und/oder ein oder zwei C-Atome unabhängig voneinander einfach mit Cl oder Br substituiert sein können und
worin $R^1$, $R^4$ und $R^{10}$ wie zuvor und nachstehend definiert sind.

Besonders bevorzugt bedeutet X -$CH_2$-, -$CH_2$-$CH_2$- oder -$CH_2$-$CH_2$-$CH_2$- und für den Fall, dass die Gruppe Y

über ein C-Atom mit X verbundenen ist, auch $-CH_2-C\equiv C-$, $-CH_2-CH_2-O-$, .$-CH_2-CH_2-NR^4-$ oder 1,3-Pyrrolidinylen, wobei die Pyrrolidinylen-Gruppe über die Imino-Gruppe mit Y verbunden ist, und
wobei die Brücke X mit $R^1$ unter Einschluss des mit $R^1$ und X verbundenen N-Atoms unter Ausbildung einer heterocyclischen Gruppe verbunden sein kann, und wobei die Brücke X zusätzlich auch mit $R^2$ unter Einschluss des mit $R^2$ und X verbundenen N-Atoms unter Ausbildung einer heterocyclischen Gruppe verbunden sein kann, und
wobei in X ein C-Atom mit $R^{10}$, vorzugsweise einem Hydroxy-, ω-Hydroxy-$C_{1-3}$-alkyl-, ω-($C_{1-4}$-Alkoxy)-$C_{1-3}$-aikyi- und/oder $C_{1-4}$-Alkoxy-Rest, und/oder ein oder zwei C-Atome jeweils mit einem oder zwei gleichen oder verschiedenen Substituenten ausgewählt aus $C_{1-6}$-Alkyl-, $C_{2-6}$-Alkenyl-, $C_{2-6}$-Alkinyl-, $C_{3-7}$-Cycloalkyl, $C_{3-7}$-Cycloalkyl-$C_{1-3}$-alkyl, $C_{4-7}$-Cycloalkenyl und $C_{4-7}$-Cycloalkenyl-$C_{1-3}$-alkyl substituiert sein können, wobei zwei Alkyl- und/oder Alkenyl-Substituenten unter Ausbildung eines carbocyclischen Ringsystems miteinander verbunden sein können, und
wobei jeweils ein oder mehrere C-Atome ein- oder mehrfach mit F und/oder jeweils ein oder zwei C-Atome unabhängig voneinander einfach mit Cl oder Br substituiert sein können.

Sind in der Gruppe X, X' oder X" ein oder mehrere C-Atome mit einem Hydroxy- und/oder $C_1$-$_4$-Alkoxy-Rest substituiert, so ist das substituierte C-Atom vorzugsweise nicht unmittelbar mit einem weiteren Heteroatom benachbart.

Ist in X, X' oder X" ein C-Atom substituiert, so sind bevorzugte Substituenten ausgewählt aus der Gruppe der $C_1$-$_4$-Alkyl-, $C_2$-$_4$-Alkenyl-, $C_2$-$_4$-Alkinyl-, $C_3$-$_7$-Cycloalkyl, $C_3$-$_7$-Cycloalkyl-$C_1$-$_3$-a!kyl, Hydroxy-, ω-Hydroxy-$C_1$-$_3$-alkyl-, ω-($C_1$-$_4$-Alkoxy)-$C_1$-$_3$-alkyl- und $C_1$-$_4$-Alkoxy-Reste.Des weiteren können in X, X' oder X" ein C-Atom zweifach und/oder ein oder zwei C-Atome ein- oder zweifach substituiert sein, wobei bevorzugte Substituenten ausgewählt sind aus der Gruppe $C_1$-$_4$-Alkyl-,$C_2$-$_4$-Alkenyl-, $C_2$-$_4$-Alkinyl-, $C_3$-$_7$-Cycloalkylund $C_3$-$_7$-Cycloalkyl-$C_1$-$_3$-alkyl, und zwei $C_1$-$_4$-Alkyl-und/oder $C_{2-4}$-Alkenyl-Substituenten auch unter Ausbildung eines gesättigten oder einfach ungesättigten carbocyclischen Rings miteinander verbunden sein können.

Ganz besonders bevorzugte Substituenten ein oder zweier C-Atome in X, X' oder X" sind ausgewählt aus Methyl, Ethyl, n-Propyl, i-Propyl, Cyclopropyl, Cyclopropylmethyl, wobei zwei Alkylsubstituenten an einem C-Atom auch unter Ausbildung eines carbocyclischen Rings miteinander verbunden sein können.

Bedeutet X eine Alkylenbrücke, so ist vorzugsweise die mit der Gruppe $R^1R^2N$-benachbarte $-CH_2$-Gruppe nicht durch -O-, -S-, -(SO)-, $-(S0_2)-$, -CO- oder $-NR^4-$ ersetzt.

Ganz besonders bevorzugt bedeutet X $-CH_2-$, $-CH_2-CH_2-$ oder $-CH_2-CH_2-CH_2-$ und für den Fall, dass die Gruppe Y über ein C-Atom mit X verbundenen ist, auch $-CH_2-CH_2-O-$, $-CH(CH_3)-CH_2-O-$, $-CH_2-CH(CH_3)-0-$, $-CH_2-CH_2-NH-$, $-CH(CH_3)-CH_2-NH-$ oder $-CH_2-CH_2-N(CH_3)-$. Für den Fall, dass $R^1$und/oder $R^2$ eine Aminfunktion aufweisen, die auch substituiert sein kann, ist eine weitere besonders bevorzugte Bedeutung von X auch eine Einfachbindung.

Gemäß einer ersten bevorzugten erfindungsgemäßen Ausführungsform bedeutet Z eine Einfachbindung.

In einer zweiten bevorzugten erfindungsgemäßen Ausführungsform ist Z eine $C_1$-$_4$-Alkylen-Brücke, die substituiert und/ oder in der eine $-CH_2$-Gruppe wie angegeben ersetzt sein kann.

Bevorzugte Bedeutungen der Gruppen W und/oder Z, insbesondere der Gruppe Z, sind unabhängig voneinander eine Einfachbindung oder eine Brücke ausgewählt aus der Gruppe $-CH_2-$, $-CH_2-CH_2-$, $-CH_2-CH(CH_3)-$, $-CH_2-C(CH_3)_2-$, $-CH(CH_3)-CH_2-$, $-C(CH_3)_2-CH_2-$, Cyclopropylen, $-CH_2-CH(R^{10})-$ und $-CH(R^{10})-CH_2-$. Zusätzliche besonders bevorzugte Bedeutungen der Gruppe W sind auch $-CH_2-O-$ oder $-CH_2-NR^4-$. Zusätzliche besonders bevorzugte Bedeutungen der Gruppe Z sind auch $-O-CH_2-$ oder $-NR^4-CH_2-$.

Gemäß einer bevorzugten erfindungsgemäßen Ausführungsform bedeutet W eine Einfachbindung.

Bevorzugt bedeuten W und/oder Z unabhängig voneinander eine Einfachbindung, $-CH_2--CH_2-CH_2-$, $-CH_2-CH_2-CH_2-$, 1,1-Cyclopropylen oder 1,2-Cyclopropylen.

W kann zusätzlich bevorzugt auch $-CH_2-O-$, $-CH_2-CH_2-O-$, $-CH_2-NR^4-$ oder $-CH_2-CH_2-NR^4-$ bedeuten.

Über die zuvor genannten Bedeutungen kann Z zusätzlich auch bevorzugt $-O-CH_2-$, $-O-CH_2-CH_2-$, $-NR^4-CH_2-$ oder $-NR^4-CH_2-CH_2-$ bedeuten.

In den zuvor genannten Bedeutungen der Gruppen W und Z kann ein C-Atom mit $R^{10}$, vorzugsweise mit einem Hydroxy-, ω-Hydroxy-$C_1$-$_3$-alkyl-,ω-($C_1$-$_4$-Alkoxy)-$C_1$-$_3$-alkyl- und/oder $C_1$-$_4$-Alkoxy-Rest,und/oder ein oder zwei C-Atome unabhängig voneinander jeweils mit einem oder zwei gleichen oder verschiedenen $C_1$-$_4$-Alkyl-Restensubstituiert sein, wobei zwei Alkylreste unter Ausbildung einer carbocyclischen Gruppe, insbesondere einer Cyclopropyl-, Cyclobutyl- oder Cyclopentylgruppe, miteinander verbunden sein können. Ferner können jeweils ein oder mehrere C-Atome in den Gruppen W und Z ein- oder mehrfach mit F und/oder jeweils ein oder zwei C-Atome unabhängig voneinander einfach mit Cl oder Br substituiert sein.

Für den Fall, dass die Brücke X eine Carbonylgruppe aufweist, enthalten W und Z vorzugsweise keine -0-Brücke.

$R^4$ besitzt in den Definitionen der Gruppen W und/oder Z die zuvor angegebenen Bedeutungen, vorzugsweise -H, Methyl, Ethyl, Propyl oder iso-Propyl.

$R^{10}$ besitzt in den Definitionen der Gruppen W und/oder Z die zuvor angegebenen Bedeutungen, vorzugsweise -OH, N-Pyrrolidinyl, Amino-ethoxy-, $C_1$-$_4$-Alkyl-amino-ethoxy- oder Di-($C_1$-$_4$-Alkyl)-amino-ethoxy-.

In den zuvor genannten Definitionen der Gruppen W und/oder Z können jeweils ein oder mehrere C-Atome

ein- oder mehrfach mit F und/oder jeweils ein oder zwei C-Atome unabhängig voneinander einfach mit Cl oder Br substituiert sein.

Sind in der Gruppe W und/oder Z ein oder zwei C-Atome mit einem Hydroxy- und/oder $C_{1-3}$-Alkoxy-Rest substituiert, so ist das substituierte C-Atom vorzugsweise nicht unmittelbar mit einem weiteren Heteroatom benachbart.

Gemäß einer Ausführungsform weisen erfindungsgemäße Verbindungen der Formel I Brücken W und Z auf, wobei genau eine oder beide der Brücken W und Z eine Einfachbindung bedeuten.

Eine bevorzugte Bedeutung der Gruppe Y ist Aryl oder Heteroaryl.

Die Gruppe Y besitzt vorzugsweise eine Bedeutung, die ausgewählt ist aus der Gruppe der bivalenten cyclischen Gruppen Phenyl, Naphthyl, Thienyl, Benzothienyl, Tetrahydronaphthyl, Pyridinyl, Pyrimidinyl, Pyrazinyl, Pyridazinyl, Indolyl, Dihydroindolyl, Dihydroindolon-yl, Chinolinyl, Tetrahydrochinolinyl, Isochinolinyl, Tetrahydro-isochinolinyl, Indazolyl, Benzimidazolyl, Benzfuranyl oder Benzoxazolyl, wobei die zuvor genannten cyclischen Gruppen ein- oder mehrfach an ein oder mehreren C-Atomen mit $R^{20}$,im Falle einer Phenylgruppe auch zusätzlich einfach mit Nitro, und/ oder an ein oder mehreren N-Atomen mit $R^{21}$substituiert sein können. Hierbei kann $R^1$mit Y und/oder X mit Y wie zuvor angegeben verbunden sein.

Besonders bevorzugt ist eine Bedeutung der Gruppe Y ausgewählt aus der Gruppe der bivalenten cyclischen Gruppen

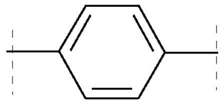 , 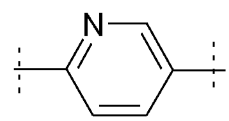 ,

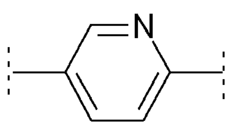 , 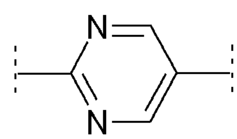 ,

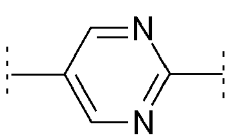 , 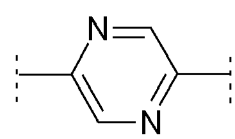 ,

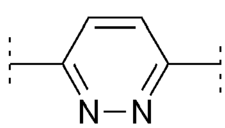 , 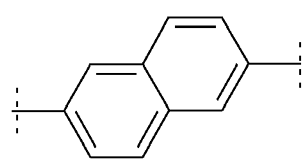 ,

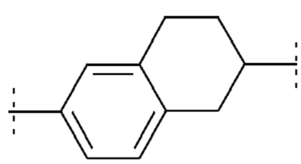 , 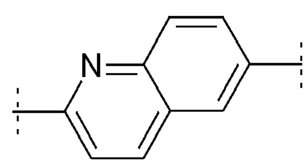 ,

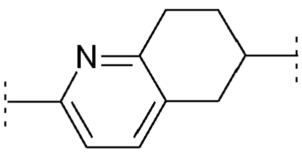 , 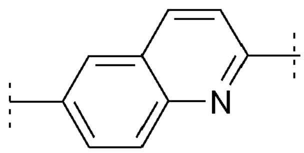 ,

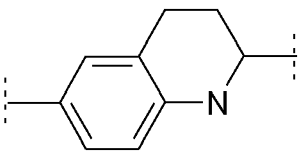 , 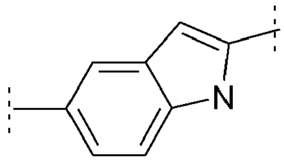 ,

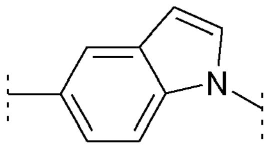 , 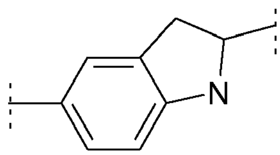 ,

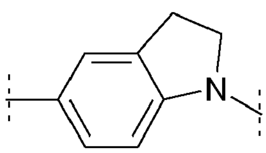 , 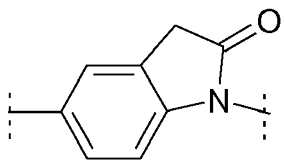 ,

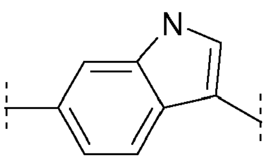 , 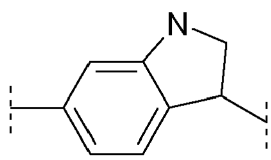 ,

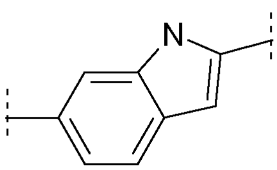 , 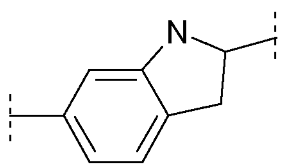 ,

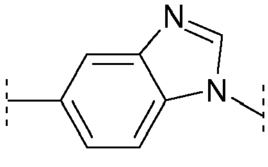 , 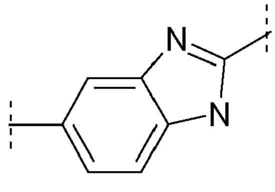 ,

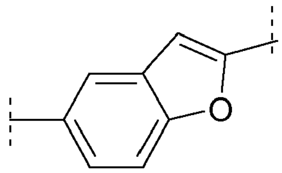 , 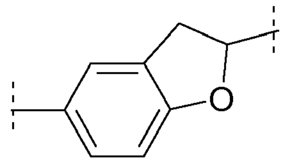 ,

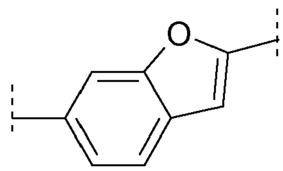 , 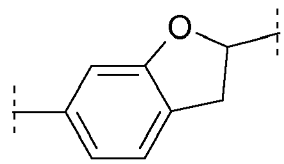 ,

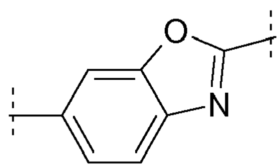 , 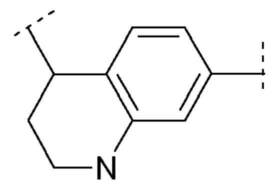 ,

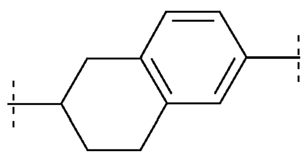 , 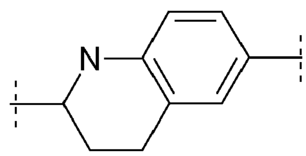 ,

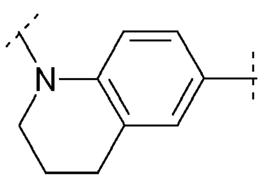 , 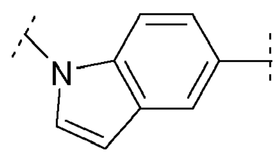 ,

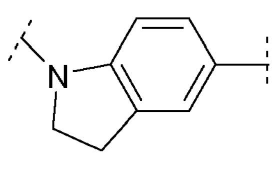 , 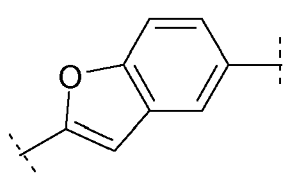 ,

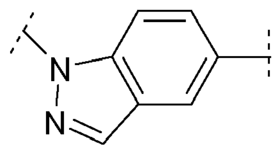 , 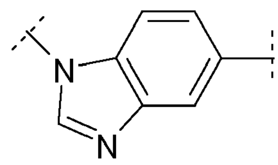 ,

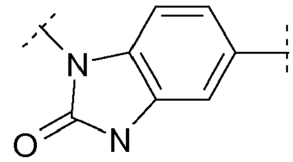 .

Die vorstehend aufgeführten cyclischen Gruppen können ein- oder mehrfach an ein oder mehreren C-Atomen mit $R^{20}$,im Falle einer Phenylgruppe auch zusätzlich einfach mit Nitro, und/oder eine oder mehrere NH-Gruppen mit $R^{21}$substituiert sein.

Ganz besonders bevorzugt ist Y eine der nachfolgend aufgeführten Gruppen

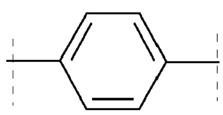 , 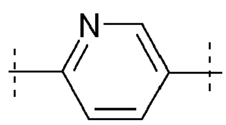 ,

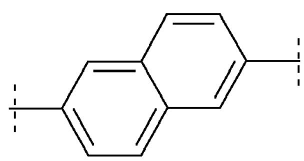 , 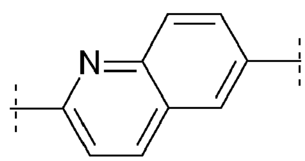 ,

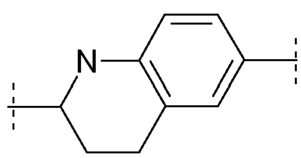 , 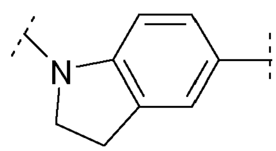 ,

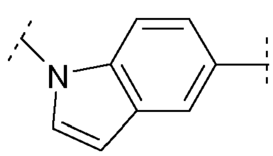 , 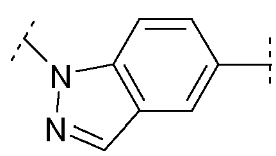 ,

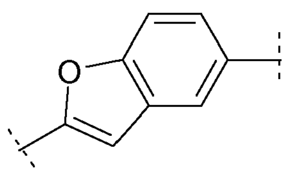 , 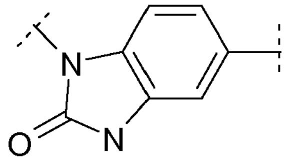 ,

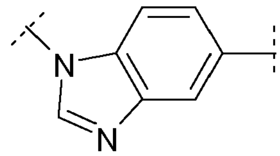 ,

insbesondere eine 1,4-Phenylen-Gruppe, wobei die aufgeführten Gruppen wie zuvor angegeben substituiert sein können.

Besonders bevorzugte Substituenten $R^{20}$der Gruppe Y sind ausgewählt aus der Gruppe bestehend aus Fluor, Chlor, Brom, $C_{1}$-$_{4}$-Alkyl,$C_{2}$-$_{6}$-Alkenyl,-CHO, Hydroxy, ω-Hydroxy-$C_{1}$-$_{3}$-alkyl,$C_{1}$-$_{4}$-Alkoxy, Trifluormethyl, Trifluormethoxy, $C_{2}$-$_{4}$-Alkinyl,Carboxy, $C_{1}$-$_{4}$-Alkoxycarbonyl-,ω-($C_{1}$-$_{4}$-Alkoxy)-$C_{1}$-$_{3}$-alkyl,$C_{1}$-$_{4}$-Alkoxy-carbonylamino-,Amino-, $C_{1}$-$_{4}$-Alkyl-amino-,Di-($C_{1}$-$_{4}$-alkyl)-amino-,Aminocarbonyl-,$C_{1}$-$_{4}$-Alkyl-amino-carbonyl-,Di-($C_{1}$-$_{4}$-alkyl)-amino-carbonyl-, -CH=N-OH und -CH=N-O-$C_{1}$-$_{4}$-alkyl.

Eine bevorzugte Bedeutung der Gruppe A ist Aryl oder Heteroaryl.

Vorzugsweise ist die Gruppe A ausgewählt aus der Gruppe der bivalenten cyclischen Gruppen Phenyl, Pyridinyl, Pyrimidinyl, Pyrazinyl oder Pyridazinyl, die ein- oder mehrfach an ein oder mehreren C-Atomen mit $R^{20}$,im Falle eines Phenylrings auch zusätzlich einfach mit Nitro.

Ganz besonders bevorzugt ist A eine der nachfolgend aufgeführten Gruppen

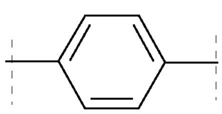 , 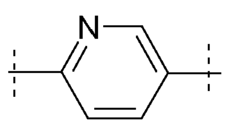 ,

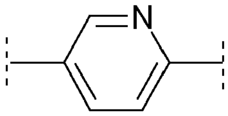 , 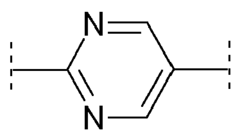 ,

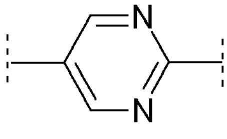 , 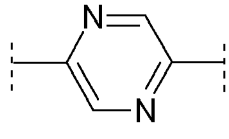 ,

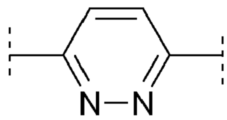

,

insbesondere

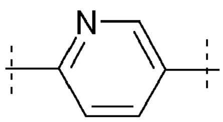

oder

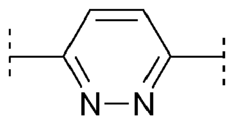

wobei die aufgeführten Gruppen, wie zuvor angegeben substituiert sein können.

Besonders bevorzugte Substituenten $R^{20}$ der Gruppe A sind Fluor, Chlor, Brom, Methoxy und $C_1$-$_3$-Alkyl.

Vorzugsweise sind die Gruppen A und/oder Y unsubstituiert oder mit $R^{20}$, wie angegeben, monosubstituiert. Die Gruppe A ist vorzugsweise unsubstituiert oder einfach fluoriert.

Die Bedeutung der Gruppe B ist gemäß einer ersten Ausführungsform vorzugsweise ausgewählt aus der Gruppe der ungesättigten Carbo- und Heterocyclen Phenyl, Thienyl und Furanyl. Besonders bevorzugt bedeutet die Gruppe B Phenyl. Die Gruppe B in den angegebenen Bedeutungen kann ein- oder mehrfach mit $R^{20}$,eine Phenylgruppe zusätzlich auch einfach mit Nitro substituiert sein. Vorzugsweise ist die Gruppe B ein-, zwei- oder dreifach, insbesondere ein- oder zweifach substituiert. Im Falle einer Einfachsubstitution ist der Substituent vorzugsweise in para-Position zur Gruppe A.

Besonders bevorzugte Substituenten $R^{20}$ der Gruppe B sind ausgewählt aus der Gruppe bestehend aus Fluor, Chlor, Brom, Cyano, Nitro, $C_1$-$_4$-Alkyl,Hydroxy, ω-Hydroxy-$C_1$-$_3$-alkyl,$C_1$-$_4$-Alkoxy,Difluormethyl, Trifluormethyl, Difluormethoxy, Trifluormethoxy, $C_2$-$_4$-Alkinyl,Carboxy, $C_1$-$_4$-Alkoxycarbonyl-,ω-($C_1$-$_4$-Alkoxy)-$C_1$-$_3$-alkyl, $C_l$-$_4$-Alkoxy-carbonylamino-,Amino-,$C_l$-$_4$-Alkyl-amino-,Di-($C_1$-$_4$-alkyl)-amino-,Aminocarbonyl-,$C_1$-$_4$-Alkyl-amino-carbonyl-und Di-($C_1$-$_4$-alkyl)-amino-carbonyl-, Ganz besonders bevorzugte Substituenten $R^{20}$ der Gruppe B sind ausgewählt aus der Gruppe bestehend aus Fluor, Chlor, Brom, $CF_3$, $C_1$-$_3$-Alkylund $C_1$-$_4$-Alkoxy.

Gemäß einer zweiten Ausführungsform ist die Bedeutung der Gruppe B vorzugsweise ausgewählt aus $C_1$-$_6$-Alkyl, $C_2$-$_6$-Alkenyl, $C_2$-$_6$-Alkinyl, $C_3$-$_7$-Cycloalkyl- $C_1$-$_3$-alkyl-, $C_3$-$_7$-Cycloalkenyl- $C_1$-$_3$-alkyl-, $C_3$-$_7$-Cycloalkyl- $C_1$-$_3$-alkenyl-, $C_3$-$_7$-Cycloalkyl-$C_7$-$_3$-alkinyl-, wobei ein oder mehrere C-Atome in den zuvor für B genannten Gruppen ein- oder mehrfach mit Fluor substituiert sein können.

In den cyclischen Gruppen gemäß der zuvor angeführten Ausführungsform können ein oder mehrere C-Atomen mit $R^{20}$ substituiert sein.

Besonders bevorzugt sind gemäß dieser Ausführungsform die Gruppen $C_3$-$_6$-Alkyl,$C_3$-$_6$-Alkenyl, $C_3$-$_6$-Alkinyl, Cyclopentyl, Cyclopentenyl, Cyclohexyl, Cyclohexenyl, Cycloheptyl, Cycloheptenyl, Cyclopentyl-$C_1$-$_3$-alkyl-,Cyclopentenyl-$C_1$-$_3$-alkyl-, Cyclohexyl-$C_1$-$_3$-alkyl-, Cyclohexenyl-$C_1$-$_3$-alkyl-, Cycloheptyl-$C_1$-$_3$-alkyl-,Cycloheptenyl-$C_1$-$_3$-alkyl-, wobei ein oder mehrere C-Atome in den zuvor für B genannten Gruppen ein- oder mehrfach mit Fluor substituiert sein können.

$R^4$ und/oder $R^5$ weisen eine der für $R^{17}$,vorzugsweise für $R^{16}$ angegebenen Bedeutungen auf. Besonders bevorzugte Bedeutungen von $R^4$ und/oder $R^5$ sind H, $C_{1-4}$-Alkyl, $C_3$-$_7$-Cycloalkylund $C_3$-$_7$-Cycloalkyl-$C_1$-$_3$-alkyl.

Ist $R^{11}$eine $C_2$-$_6$-Alkenyl- oder $C_2$-$_6$-Alkinyl-Gruppe,so sind die Bedeutungen $-CH=CH_2$, $-CH=CH(CH_3)$, $-CH=C(CH_3)_2$ sowie $-C≡CH$,$-C≡C-CH_3$bevorzugt.

Bevorzugte Bedeutungen der Gruppe $R^{20}$sind Halogen, Hydroxy, Cyano, $C_1$-$_4$-Alkyl, $C_1$-$_4$-Alkoxy, $C_3$-$_7$-Cycloalkylund $C_1$-$_4$-Alkoxy-$C_1$-$_3$-alkyl,wobei C-Atome ein- oder mehrfach mit Fluor und einfach mit Cl oder Br substituiert sein können. Besonders bevorzugt bedeutet $R^{20}$F, Cl, Br, I,OH, Cyano, Methyl, Difluormethyl, Trifluormethyl, Ethyl, n-Propyl, iso-Propyl, Methoxy, Difluormethoxy, Trifluormethoxy, Ethoxy, n-Propoxy oder iso-Propoxy.

Bevorzugte Bedeutungen der Gruppe $R^{21}$sind $C_1$-$_4$-Alkyl,$C_1$-$_4$-Alkylcarbonyl,$C_1$-$_4$-Alkylsulfonyl-, -$S0_2$-$NH_2$, -$S0_2$-NH-$C_1$-$_3$-alkyl,-$S0_2$-N($C_1$-$_3$-alkyl)$_2$und Cyclo-$C_{3-6}$-alkylenimino-sulfonyl-.

Cy bedeutet vorzugsweise eine $C_3$-$_7$-Cycloalkyl-,insbesondere eine $C_5$-$_7$-CycloalkylGruppe, eine $C_5$-$_7$-Cycloalkenyl-Gruppe,Aryl oder Heteroaryl, wobei Aryl oder Heteroaryl vorzugsweise ein monocyclisches oder kondensiert bicyclisches Ringsystem darstellt, und wobei die zuvor genannten cyclischen Gruppen ein- oder mehrfach an ein oder mehreren C-Atomen mit $R^{20}$,im Falle einer Phenylgruppe auch zusätzlich einfach mit Nitro, und/oder ein oder mehrere NH-Gruppen mit $R^{21}$substituiert sein können.

Diejenigen erfindungsgemäßen Verbindungen sind bevorzugt, in denen eine oder mehrere der Gruppen, Reste, Substituenten und/oder Indizes eine der zuvor als bevorzugt angegebenen Bedeutungen aufweisen.

Insbesondere sind diejenigen erfindungsgemäßen Verbindungen bevorzugt, in denen

Y eine der zuvor als bevorzugt genannten Bedeutungen aufweist, besonders bevorzugt eine Gruppe ausgewählt aus

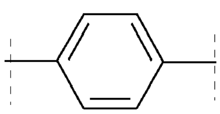 , 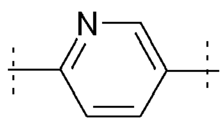 ,

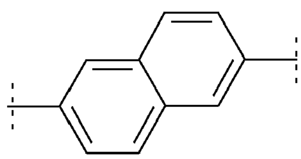 , 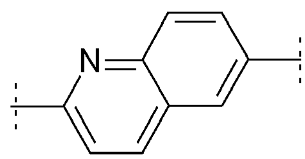 ,

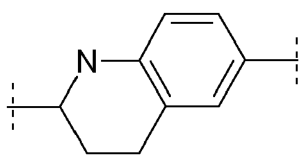 , 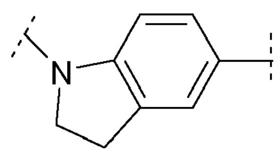 ,

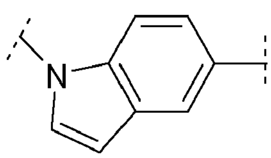 , 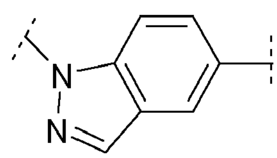 ,

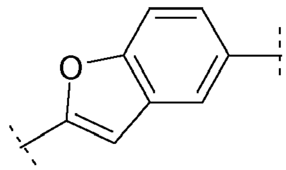 , 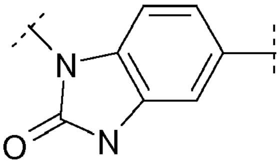 ,

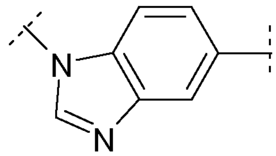 ,

bedeutet, und/oder,

A eine der zuvor als bevorzugt genannten Bedeutungen aufweist, besonders bevorzugt

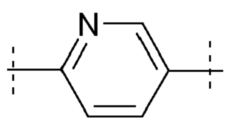

oder

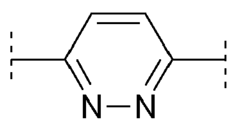 ,

bedeutet, und/oder

B eine der zuvor als bevorzugt genannten Bedeutungen, besonders bevorzugt Phenyl, aufweist,

wobei A, B und/oder Y ein- oder zweifach, B auch dreifach, an ein oder mehreren C-Atomen mit $R^{20}$,im Falle eines Phenyl-Rings auch zusätzlich einfach mit Nitro, substituiert sein können.

Ganz besonders sind diejenigen erfindungsgemäßen Verbindungen bevorzugt, in denen A, B, X, Y, Z, $R^1$, $R^2$ und W unabhängig voneinander die vorstehend genannten bevorzugten Bedeutungen aufweisen.

Besonders bevorzugte erfindungsgemäße Verbindungen werden daher mit einer der allgemeinen Formeln IIa bis IIL

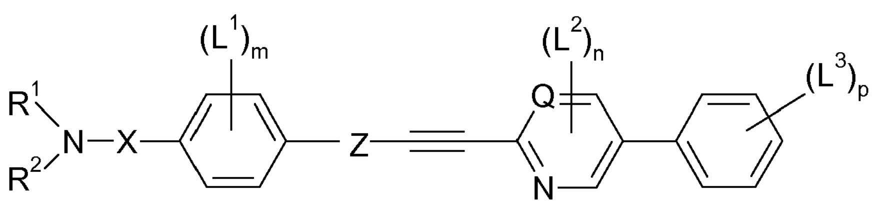

IIa

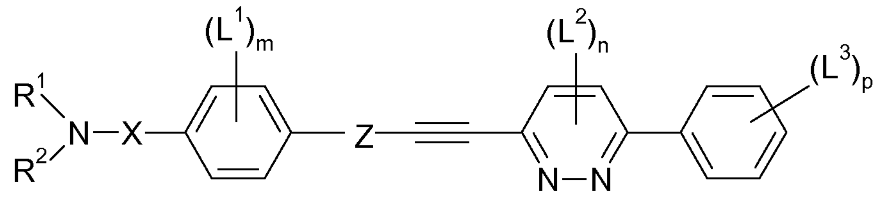

IIb

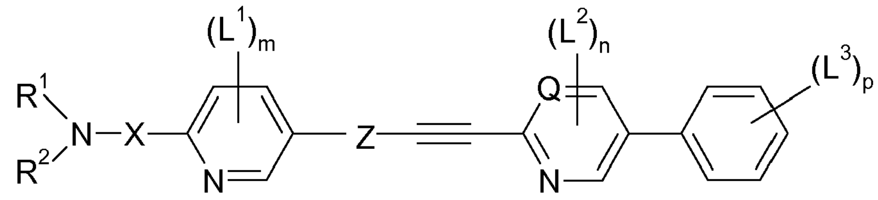

IIc

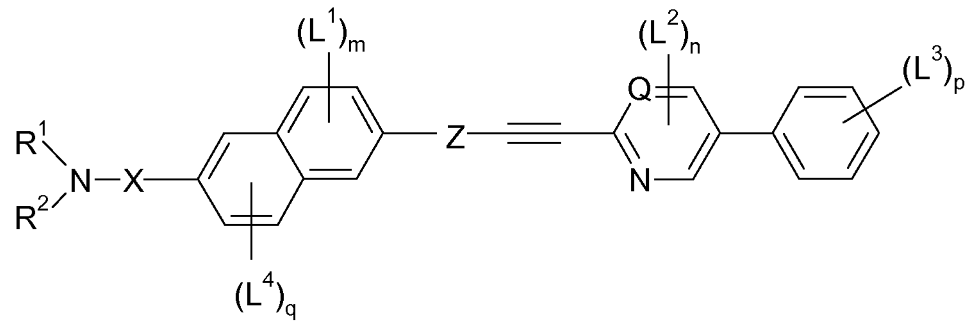

IId

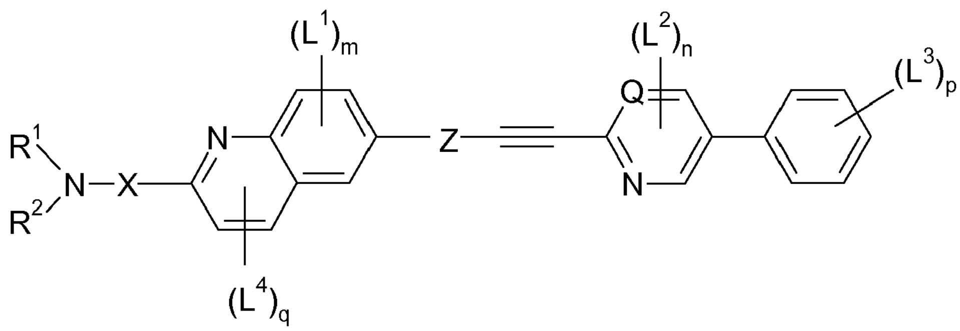

IIe

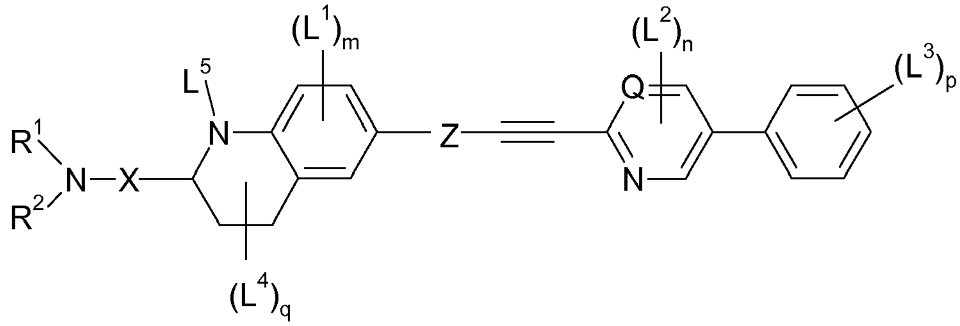

IIf

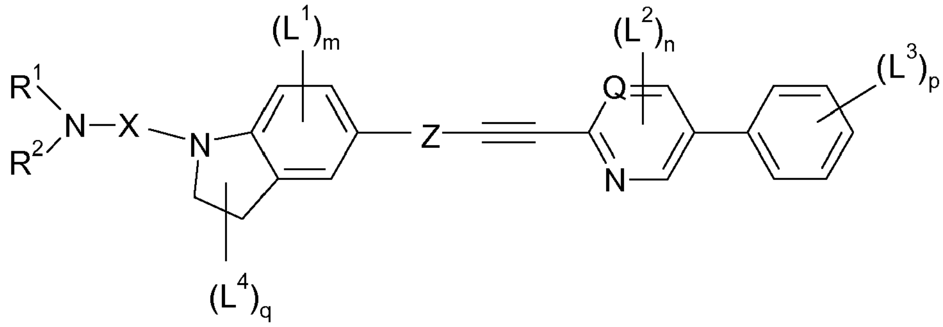

IIg

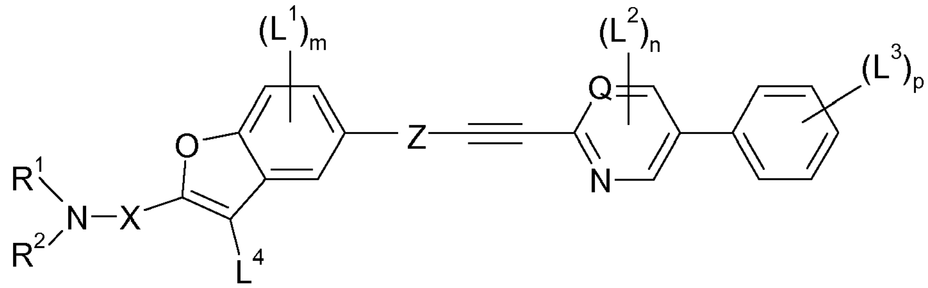

IIh

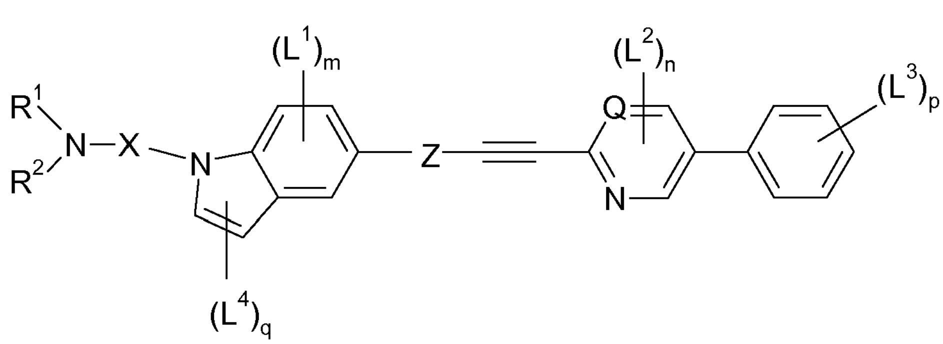

IIi

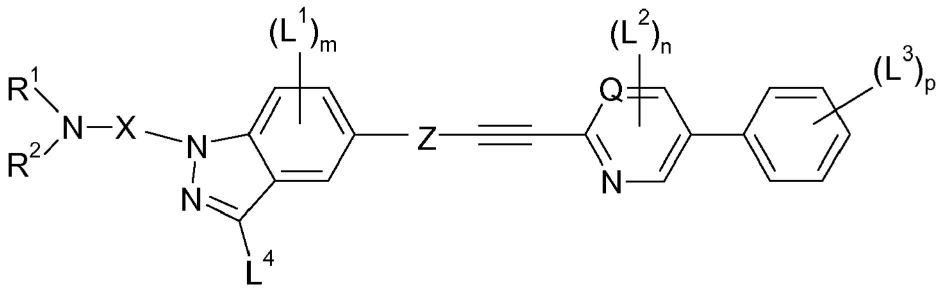

IIj

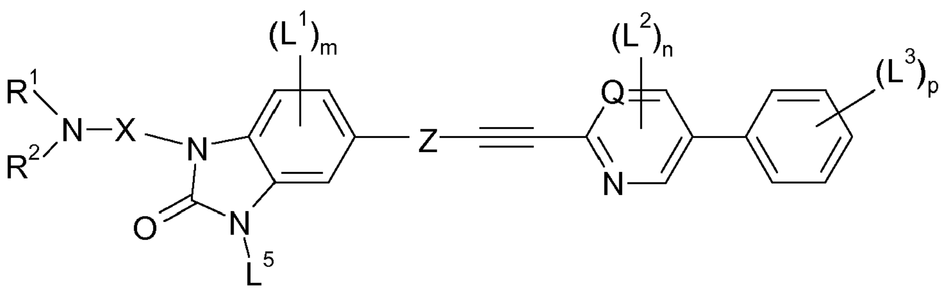

IIk

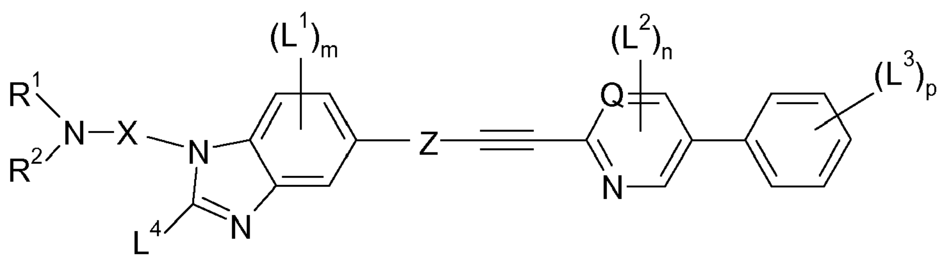

IIL

beschrieben werden, in der

| | |
|---|---|
| $R^1$, $R^2$, X und Z | die zuvor genannten Bedeutungen besitzen und |
| Q | -CH- oder N, vorzugsweise -CH-, bedeutet und |
| $L^1$ $L^2$, $L^3$,$L^4$ | H oder eine der für $R^{2'}$ angegebenen Bedeutungen besitzen, und |
| $L^5$ | H oder eine der für $R^{21}$ angegebenen Bedeutungen besitzt, und |
| m, n, p, q | unabhängig voneinander die Werte 0, 1 oder 2, p auch den Wert 3, bedeuten. |

Eine Gruppe ganz besonders bevorzugter Verbindungen kann durch die Formel IIa beschrieben werden, in der die Gruppe Q -CH- bedeutet.

Vorzugsweise, insbesondere in den Formeln IIa bis IIL,besitzen die zuvor genannten Gruppen folgende Bedeutungen:

X $-CH_2-$, $-CH_2-CH_2-$ oder $-CH_2-CH_2-CH_2-$,
in den Formeln IIa bis IIe auch $-CH_2-C{\equiv}C-$,$-CH_2-CH_2-O-$, $-CH_2-CH_2-NR^4-$ oder 1,3-Pyrrolidinylen,wobei die Pyrrolidinylen-Gruppeüber die Imino- Gruppe mit Y verbunden ist,
wobei in den zuvor angeführten Bedeutungen eine oder zwei $-CH_2$-Gruppen mit ein oder zwei Methyl-Gruppen substituiert sein können,
wobei die Brücke X mit $R^1$ unter Einschluss des mit $R^1$ und X verbundenen N-Atoms unter Ausbildung einer heterocyclischen Gruppe verbunden sein kann, und

Z eine Einfachbindung oder eine Brücke ausgewählt aus der Gruppe $-CH_2-$, $-CH_2-CH_2-$, $-CH_2-CH(CH_3)-$, $-CH_2-C(CH_3)_2-$, $-CH(CH_3)-CH_2-$, $-C(CH_3)_2-CH_2-$, Cyclopropylen, $-CH_2-CH(R^{10})-$ und $-CH(R^{10})-CH_2-$, $-O-CH_2-$ oder $-NR^4-CH_2-$.

In den zuvor angeführten Bedeutungen für X und Z kann jeweils ein C-Atom mit einem Hydroxy-, ω-Hydroxy-$C_{1-3}$-alkyl-,ω-($C_{1-4}$-Alkoxy)-$C_{1-3}$-alkyl-und/oder $C_{1-4}$-Alkoxy-Rest, und/oder ein oder zwei C-Atome unabhängig voneinander jeweils mit einem oder zwei gleichen oder verschiedenen $C_{1-4}$-Alkyl-Resten substituiert sein, wobei die Alkyl-Reste unter Ausbildung eines carbocyclischen Rings miteinander verbunden sein können. Darüber hinaus können in den Gruppen X und Z jeweils ein oder mehrere C-Atome ein- oder mehrfach mit F und/oder jeweils ein oder zwei C-Atome unabhängig voneinander einfach mit Cl oder Br substituiert sein.

$R^4$ besitzt in den Definitionen der Gruppen X und Z die zuvor angegebenen Bedeutungen, vorzugsweise -H, Methyl, Ethyl, Propyl oder iso-Propyl.

$R^{10}$besitzt in den Definitionen der Gruppen X und Z die zuvor angegebenen Bedeutungen, vorzugsweise -OH, N-Pyrrolidinyl, Amino-ethoxy, $C_{1-4}$-Alkyl-amino-ethoxy oder Di-($C_{1-4}$-Alkyl)-amino-ethoxy.

Ganz besonders bevorzugt bedeuten

X $-CH_2-$, $-CH_2-CH_2-$ oder $-CH_2-CH_2-CH_2-$, in den Formeln IIa bis IIe auch $-CH_2-CH=CH-$, $-CH_2-C\equiv C-$,$-CH_2-CH_2-O-$, $-CH(CH_3)-CH_2-O-$ oder $-CH_2-CH(CH_3)-0-$, und/oder

Z eine Einfachbindung, $-CH_2-$, $-CH_2-CH_2-$ oder $-O-CH_2-$, insbesondere eine Einfachbindung oder $-CH_2-CH_2-$ und/oder

$L^1$ L2, L $^3$, $L^4$, unabhängig voneinander F, Cl, Br, I, OH, Cyano, $C_{1-4}$-Alkyl,$C_{2-4}$-Alkinyl, $C_{1-4}$- Alkoxy-, Difluormethyl-, Trifluormethyl-, Amino-, $C_{l-4}$-Alkylamino-,Di- ($C_{1-4}$-alkyl)-amino-, Acetylamino-, Aminocarbonyl-, Difluormethoxy-, Trifluormethoxy-, Amino-$C_{1-3}$-alkyl-,$C_{1-4}$-Alkylamino-$C_{1-3}$-alkyl-oder Di-($C_{1-4}$-Alkyl)-amino-$C_{1-3}$-alkyl- oder Nitro, mit der Maßgabe, dass ein Phenyl nur einfach mit Nitro substituiert sein kann, und/oder

$L^1$ zusätzlich auch -CH=N-OH oder -CH=N-0-$C_{1-4}$-alkyl,

m, n, q 0 oder 1 und/oder

p 1, 2 oder 3, insbesondere 1 oder 2.

Besonders bevorzugt sind die folgenden Einzelverbindungen:

(1)

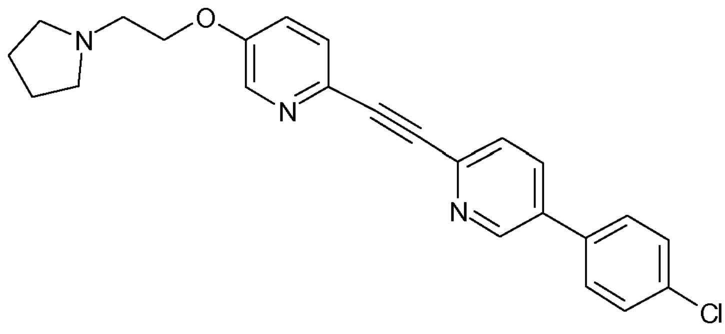

5-(4-Chlor-phenyl)-2-[5-(2-pyrrolidin-1-yl-ethoxy)- pyrid-2-yl-ethinyl]-pyridin

(2)

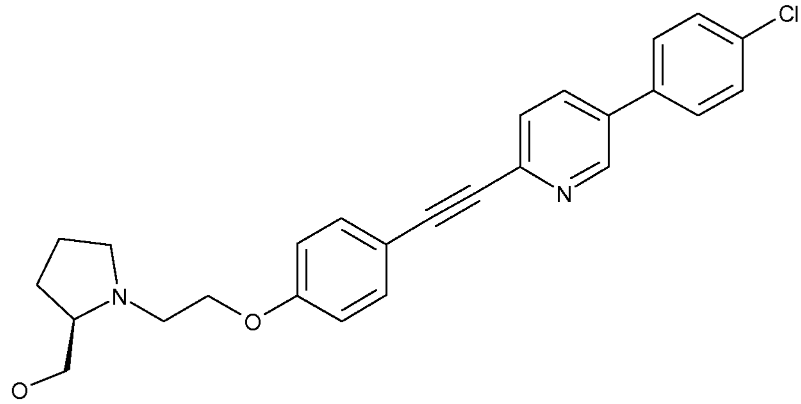

[(R)-1 -(2-{4-[5-(4-Chlor-phenyl)-pyridin-2-ylethinyl]-phenoxyl-ethyl)-pyrrolidin-2-yl]-methanol

(3)

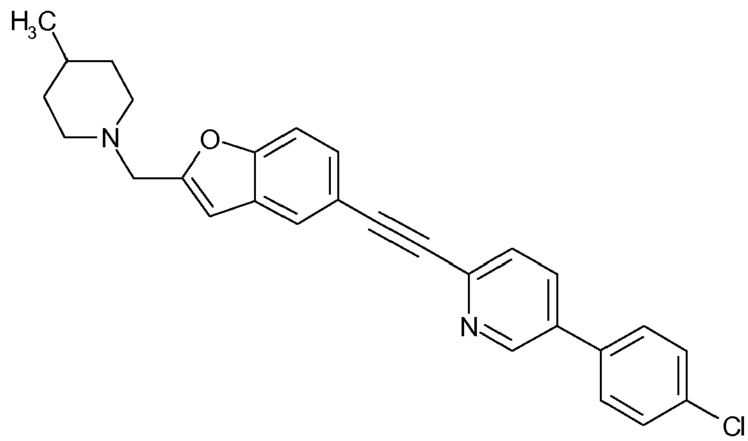

5-(4-Chlor-phenyl)-2-[2-(4-methyl-piperidin-1-ylmethyl)-benzofuran-5-ylethinyl]-pyridin

(4)

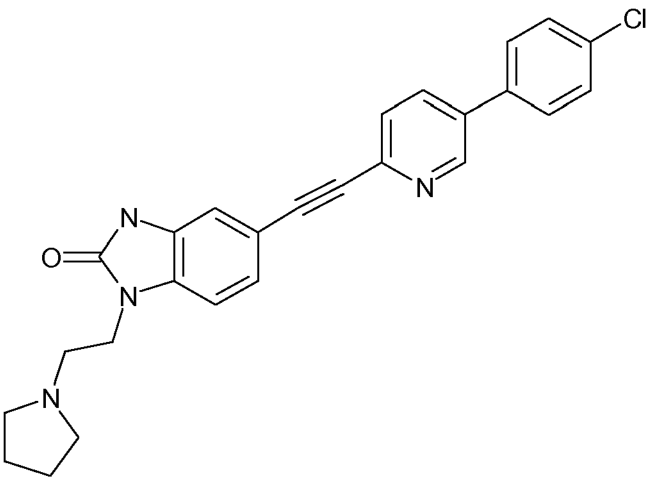

5-[5-(4-Chlor-phenyl)-pyridin-2-ylethinyl]-1-(2-pyrrol idin-1-yl-ethyl)-1,3-d ihyd ro-benzim idazol-2-on

(5)

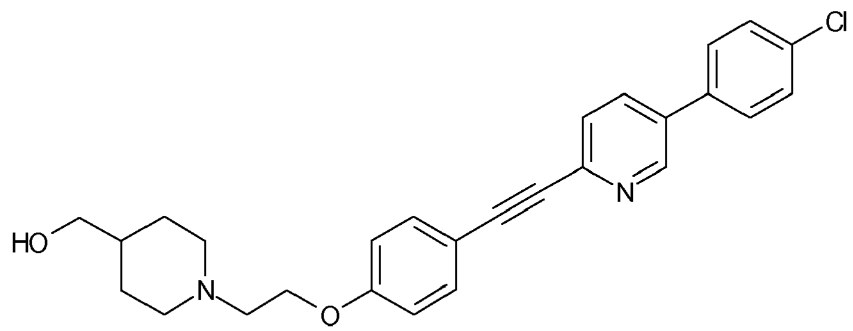

[1-(2-{4-[5-(4-Chlor-phenyl)-pyhdln-2-yleth!nyl]-phenoxy}-ethyl)-piperidin-4-yl]-methanol

(6)

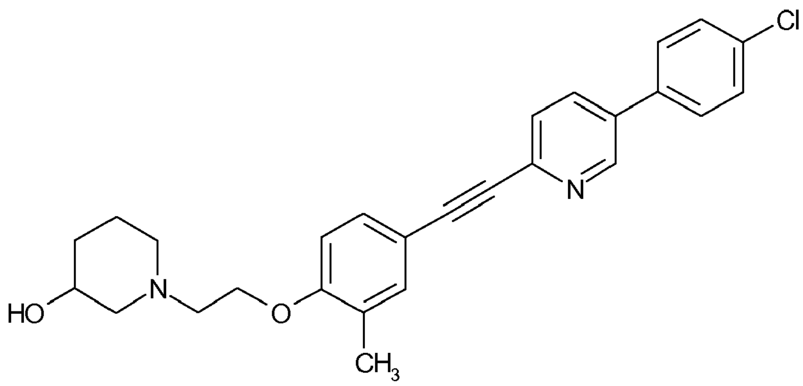

1-(2-{4-[5-(4-Chlor-phenyl)-pyridin-2-ylethinyl]-2-methyl-phenoxyl-ethyl)-piperidin-3-ol

(7)

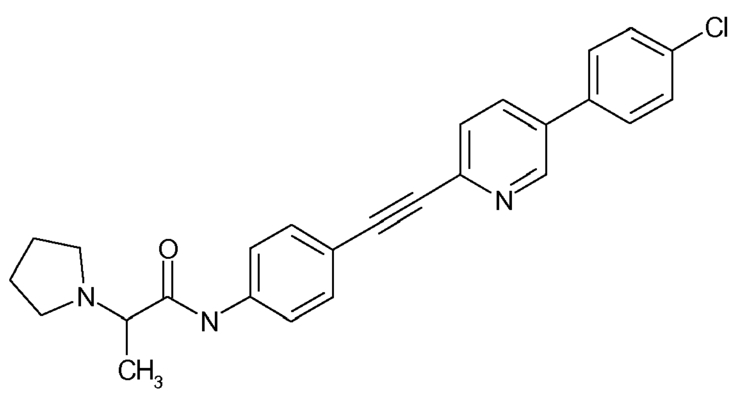

N-{4-[5-(4-Chlor-phenyl)-pyhdln-2-ylethlnyl]-phenyl}-2-pyrrolidin-1-yl-propionamid

(8)

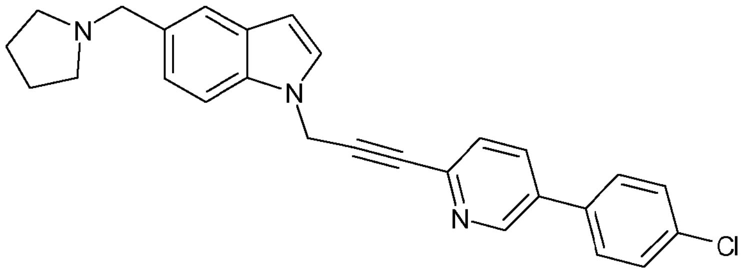

1-(3-[5-(4-Chlor-phenyl)-pyridin-2-yl]-prop-2-inyl)-5-pyrrolidin-1-ylmethyl-1H-indol

(9)

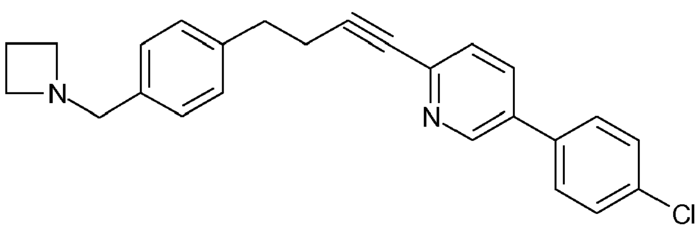

2-[4-(4-Azetidin-1-ylmethyl-phenyl)-but-1-inyl]-5-(4-chlor-phenyl)-pyridin

(10)

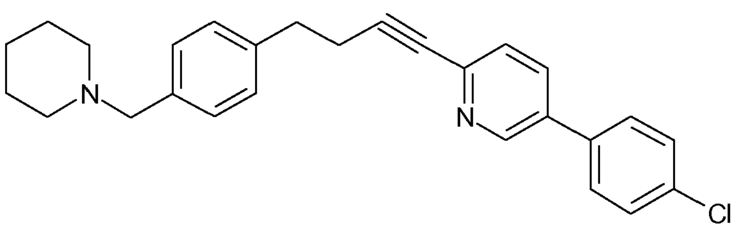

5-(4-Chlor-phenyl)-2-[4-(4-piperidin-1-ylmethyl-phenyl)-but-1-inyl]-pyridin

(11)

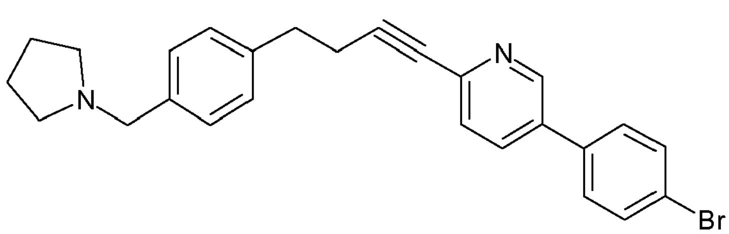

5-(4-Brom-phenyl)-2-[4-(4-pyrrolidin-1-ylmethyl-phenyl)-but-1-inyl]-pyridin

(12)

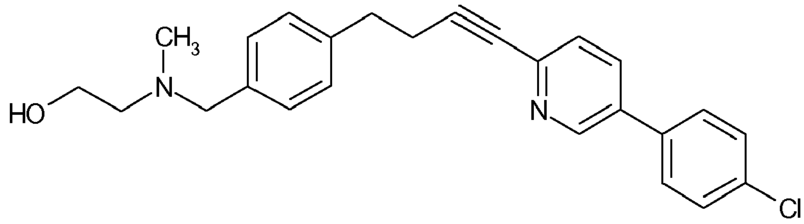

2-[(4-{4-[5-(4-Chlor-phenyl)-pyrid in-2-yl]-but-3-inyll-benzyl)-methyl - amino]-ethanol

(13)

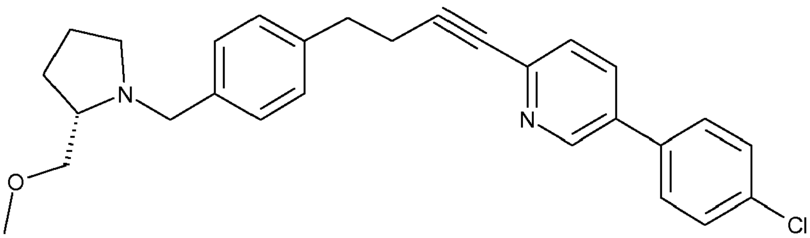

5-(4-Chlor-phenyl)-2-{4-[4-((S)-2-methoxymethyl-pyrrolidin-1-ylmethyl)-phenyl]-but-1-inyl)-pyridin

(14)

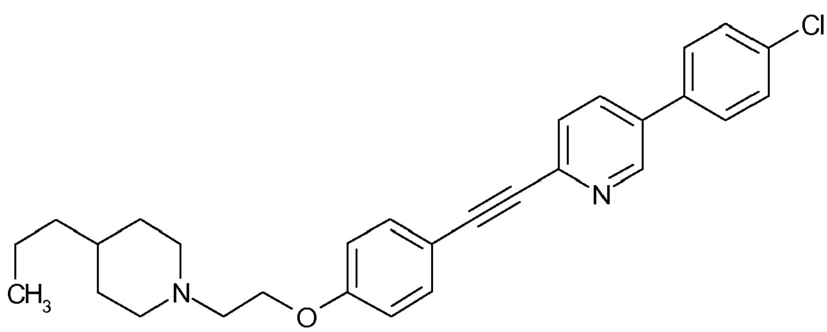

5-(4-Chlor-phenyl)-2-(4-[2-(4-propyl-piperidin-1-yl)-ethoxy]-phenylethinyl)-pyridin

(15)

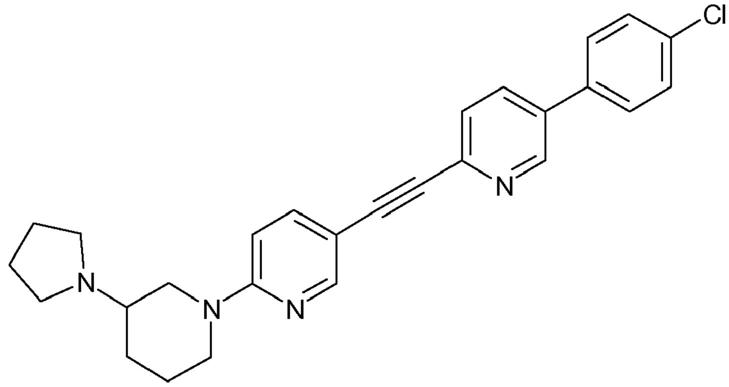

5'-[5-(4-Chlor-phenyl)-pyridin-2-ylethinyl]-3-pyrrolidin-1-yl-3,4,5,6-tetrahydro-2H-[1,2']bipyridinyl

(16)

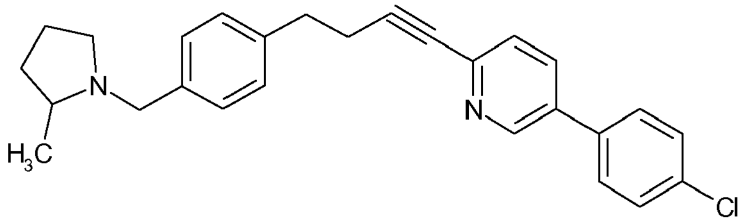

5-(4-Chlor-phenyl)-2-{4-[4-(2-methyl-pyrrolidin-1-ylmethyl)-phenyl]-but-1-inyl)-pyridin

(17)

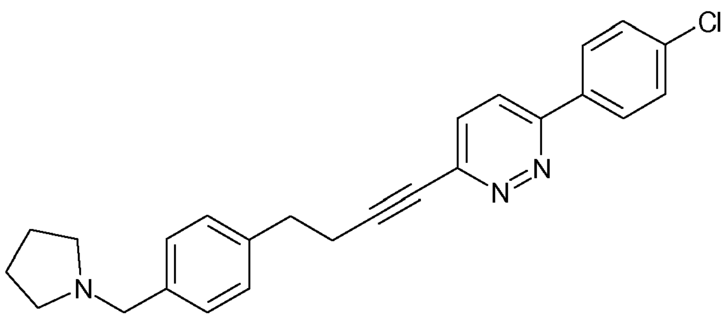

3-(4-Chlor-phenyl)-6-[4-(4-pyrrolidin-1-ylmethyl-phenyl)-but-1-inyl]-pyridazin

(18)

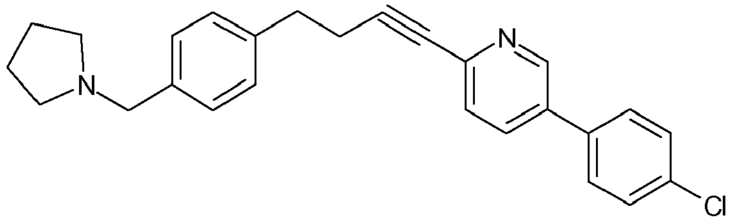

5-(4-Chlor-phenyl)-2-[4-(4-pyrrolidin-1-ylmethyl-phenyl)-but-1-inyl]-pyridin

(19)

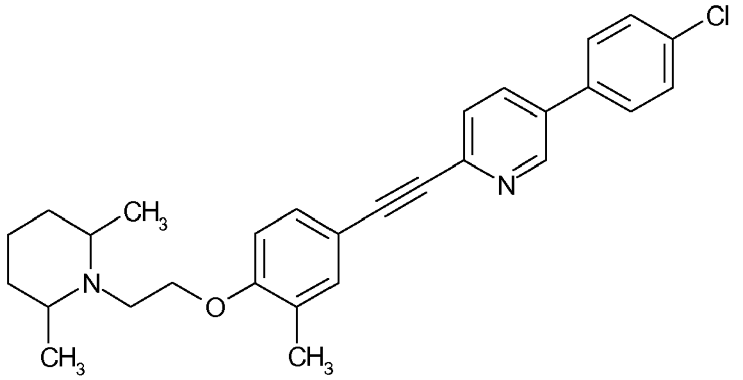

5-(4-Chlor-phenyl)-2-{4-[2-(2,6-dimethyl-piperidin-1-yl)-ethoxy]-3-methyl-phenylethinyl}-pyridin

(20)

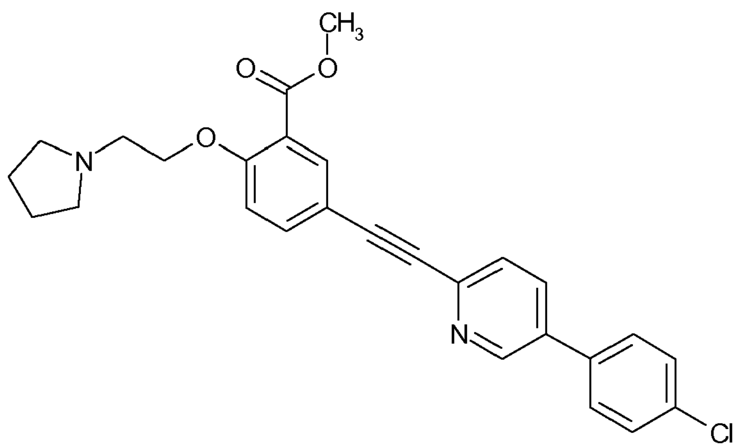

5-[5-(4-Chlor-phenyl)-pyridin-2-ylethinyl]-2-(2-pyrrolidin-1-yl-ethoxy)-benzoesäuremethylester

(21)

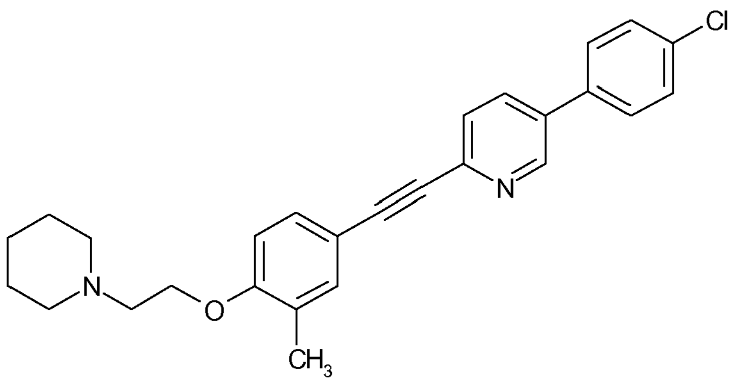

5-(4-Chlor-phenyl )-2-[3-methyl-4-(2-piperid in-1-yl-ethoxy)-phenyleth inyl]-pyridin

(22)

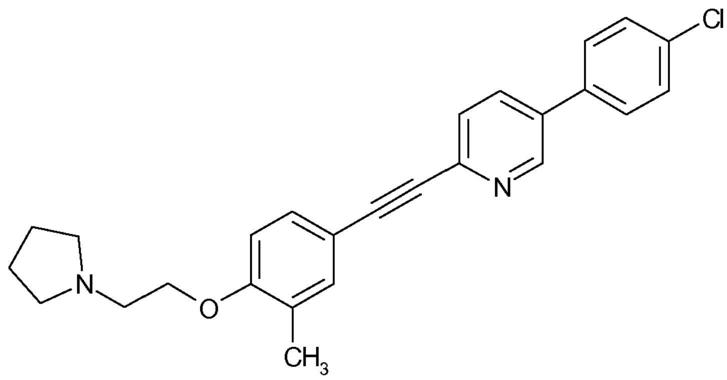

5-(4-Chlor-phenyl )-2-[3-methyl-4-(2-pyrrol id in-1-yl-ethoxy)-phenyleth inyl]-pyridin

(23)

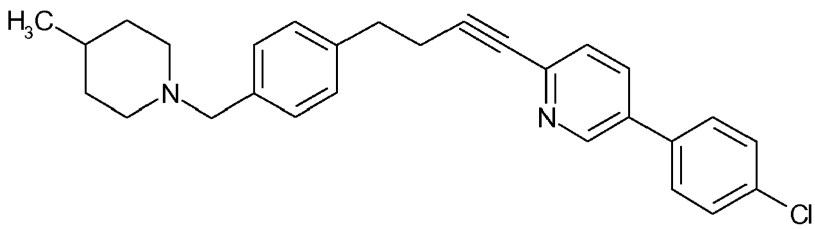

5-(4-Chlor-phenyl)-2-{4-[4-(4-methyl-piperidin-1-ylmethyl)-phenyl]-but-1-inyl}-pyridin

(24)

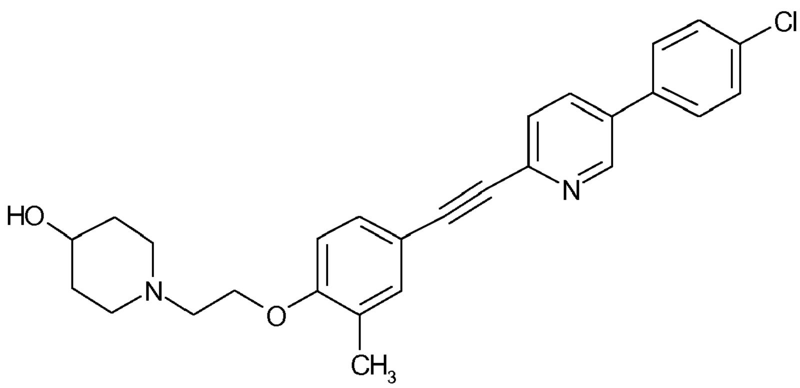

1-(2-{4-[5-(4-Chlor-phenyl)-pyridin-2-ylethinyl]-2-methyl-phenoxyl-ethyl)-piperidin-4-ol

(25)

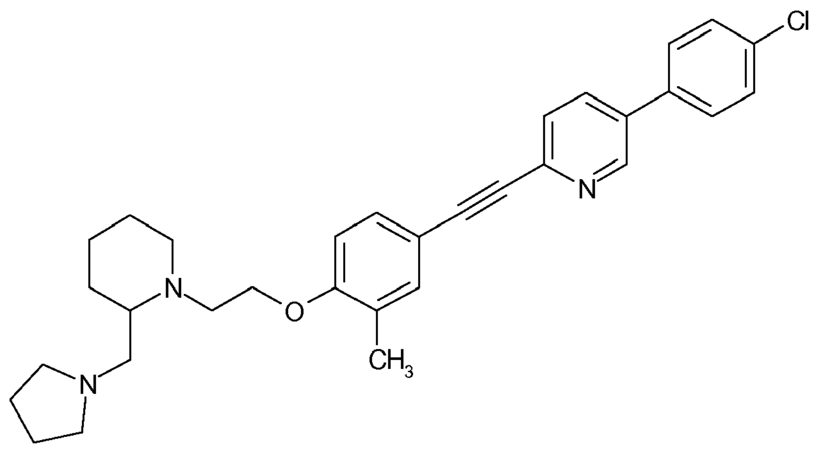

5-(4-Chlor-phenyl)-2-{3-methyl-4-[2-(2-pyrrolidin-1 -ylmethyl-piperidin-1 - yl)-ethoxy]-phenylethinyl)-pyridin

(26)

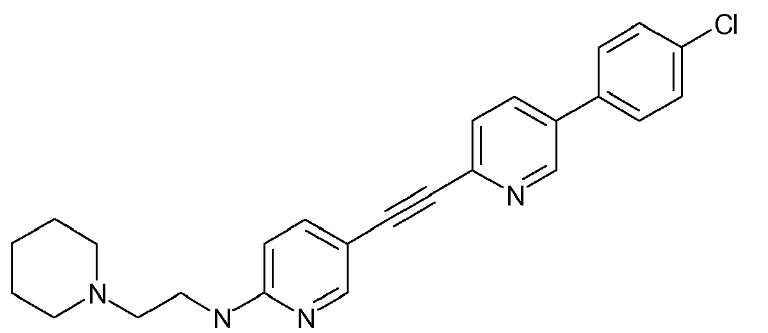

{5-[5-(4-Chlor-phenyl)-pyhdln-2-ylethlnyl]-pyhdln-2-yl}-(2-plpehdln-1-yl-ethyl)-amin

(27)

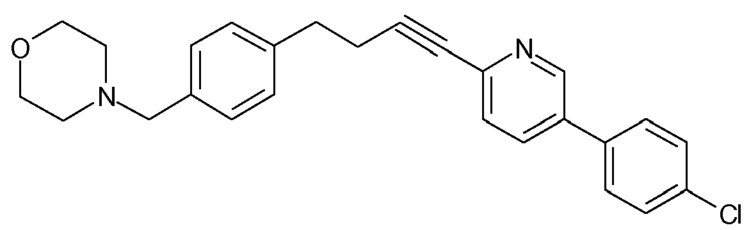

4-(4-{4-[5-(4-Chlor-phenyl)-pyridin-2-yl]-but-3-inyll-benzyl)-morpholin

(28)

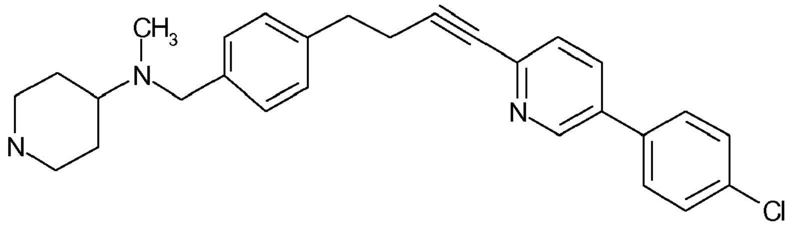

(4-{4-[5-(4-Chlor-phenyl)-pyridin-2-yl]-but-3-inyll-benzyl)-methyl-piperidin-4-yl-amin

(29)

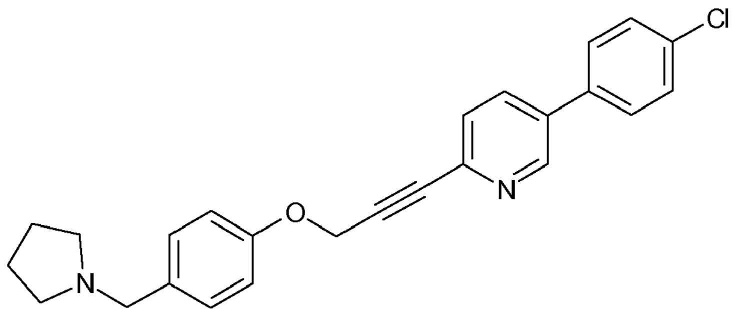

5-(4-Chlor-phenyl)-2-[3-(4-pyrrolidin-1-ylmethyl-phenoxy)-prop-1-inyl]-pyridin

(30)

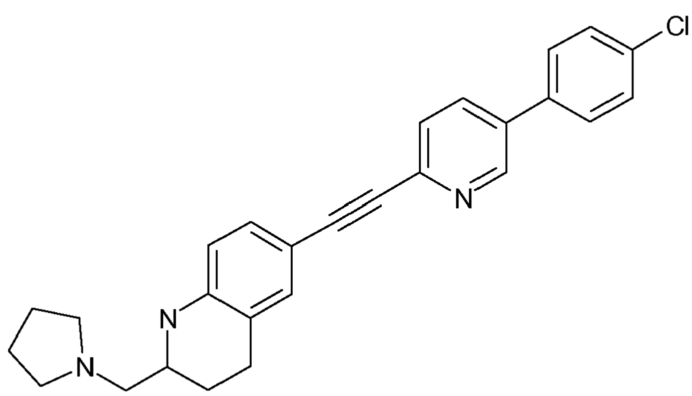

6-[5-(4-Chlor-phenyl )-pyrid in-2-yleth inyl]-2-pyrrol id in-1-yl methyl-1,2,3,4-tetrahydro-chinolin

(31)

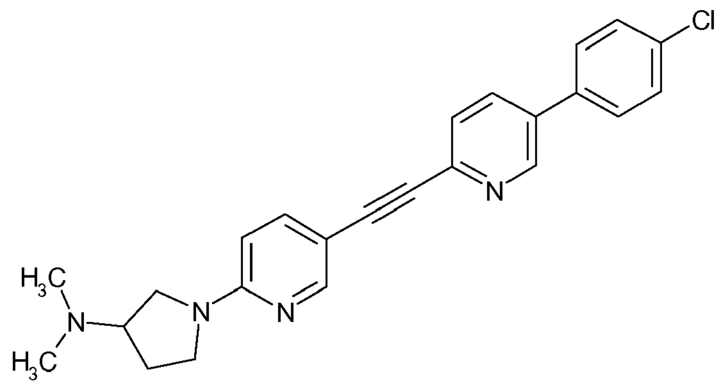

(1-{5-[5-(4-Chlor-phenyl)-pyridin-2-ylethinyl]-pyridin-2-yll-pyrrolidin-3-yl)-dimethyl-amin

(32)

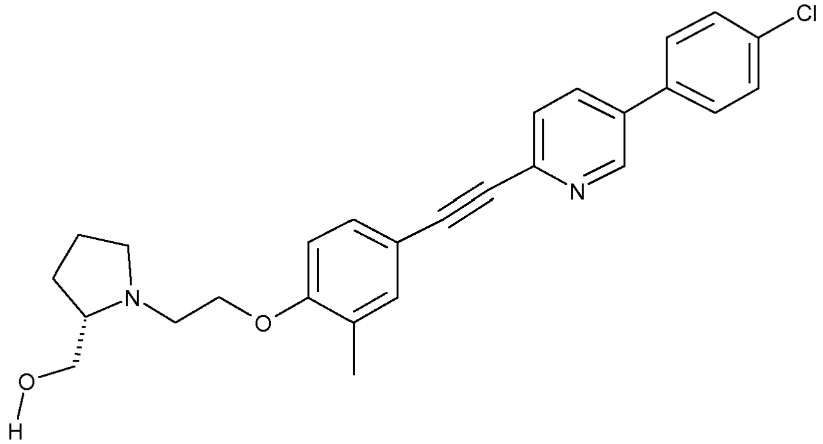

[(S)-1-(2-(4-[5-(4-Chlor-phenyl)-pyridin-2-ylethinyl]-2-methyl-phenoxy)-ethyl)-pyrrolidin-2-yl]-methanol

(33)

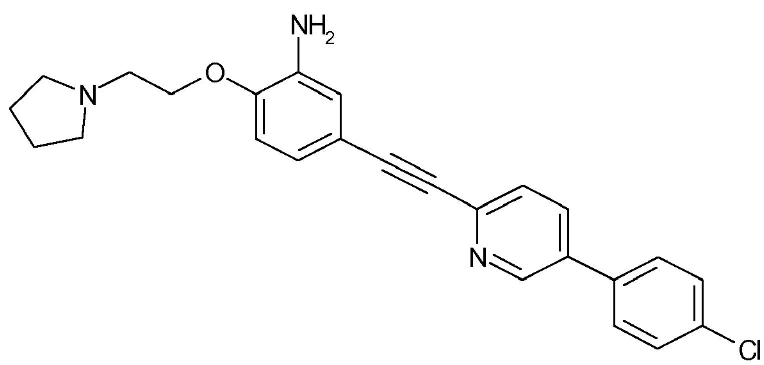

5-[5-(4-Chlor-phenyl)-pyridin-2-ylethinyl]-2-(2-pyrrolidin-1-yl-ethoxy)-phenylamin

(34)

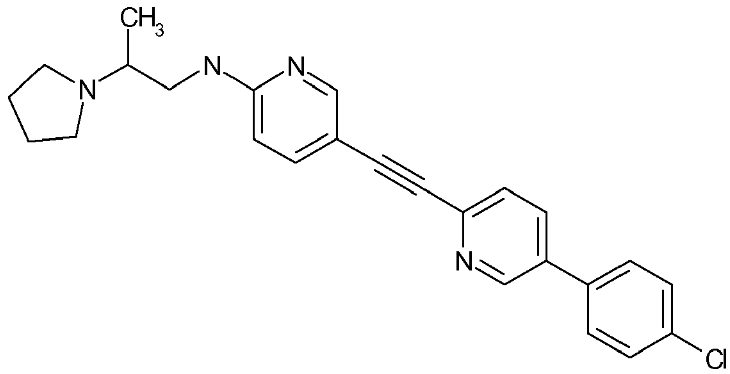

{5-[5-(4-Chlor-phenyl)-pyhdln-2-ylethlnyl]-pyhdin-2-yl}-(2-pyrrolidin-1-yl-propyl)-amin

(35)

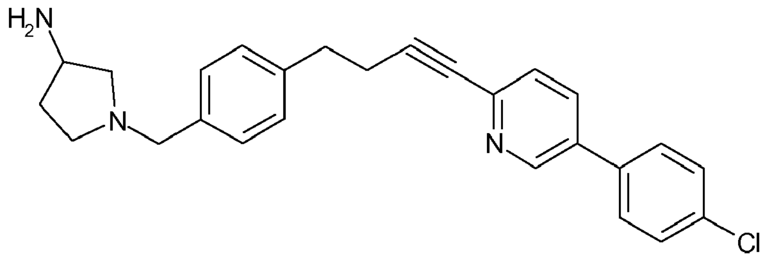

1-(4-(4-[5-(4-Chlor-phenyl)-pyridin-2-yl]-but-3-inyl)-benzyl)-pyrrolidin-3-ylamin

(36)

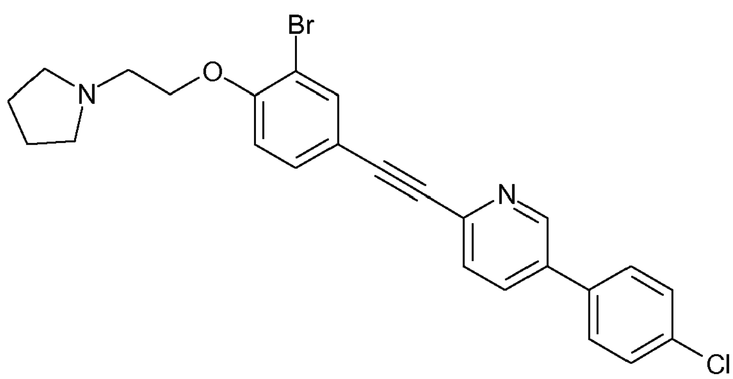

2-[3-Brom-4-(2-pyrrolidin-1-yl-ethoxy)-phenylethinyl]-5-(4-chlor-phenyl)-pyridin

(37)

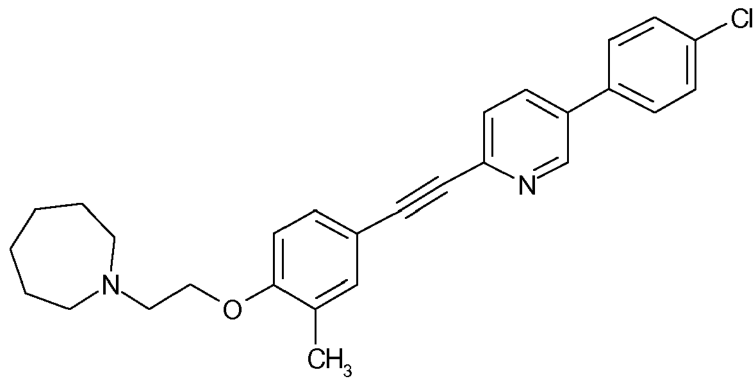

1-(2-{4-[5-(4-Chlor-phenyl)-pyridin-2-ylethinyl]-2-methyl-phenoxyl-ethyl)-azepan

(38)

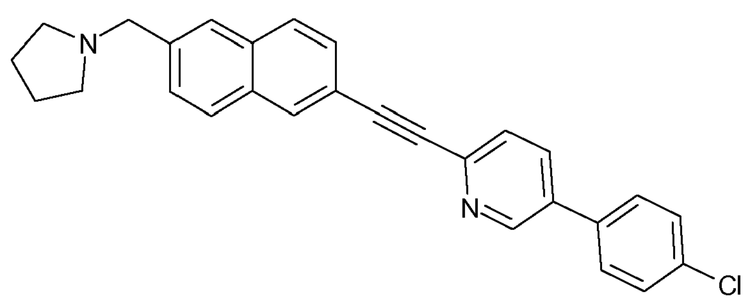

5-(4-Chlor-phenyl)-2-(6-pyrrolidin-1-ylmethyl-naphthalen-2-ylethinyl)-pyridin

(39)

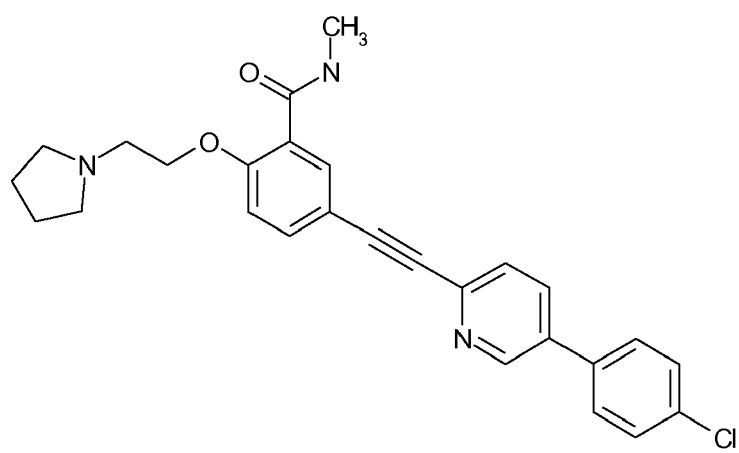

5-[5-(4-Chlor-phenyl)-pyridin-2-ylethinyl]-N-methyl-2-(2-pyrrolidin-1-yl-ethoxy)-benzamid

(40)

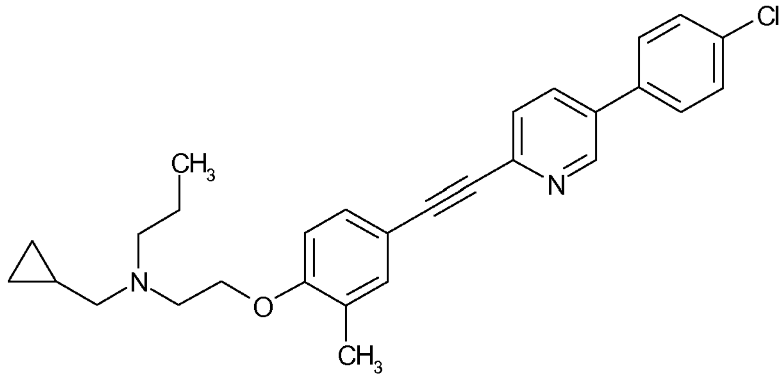

(2-{4-[5-(4-Chlor-phenyl)-pyrid in-2-ylethinyl]-2-methyl-phenoxyl-ethyl)-cyclopropylmethyl-propyl-amin

(41)

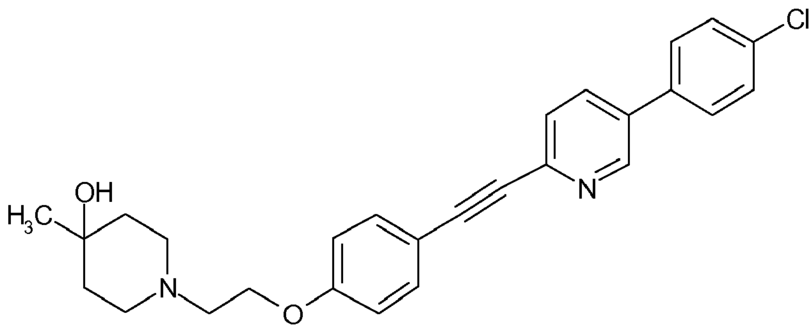

1-(2-{4-[5-(4-Chlor-phenyl)-pyridin-2-ylethinyl]-phenoxyl-ethyl)-4-methylpiperidin-4-ol

(42)

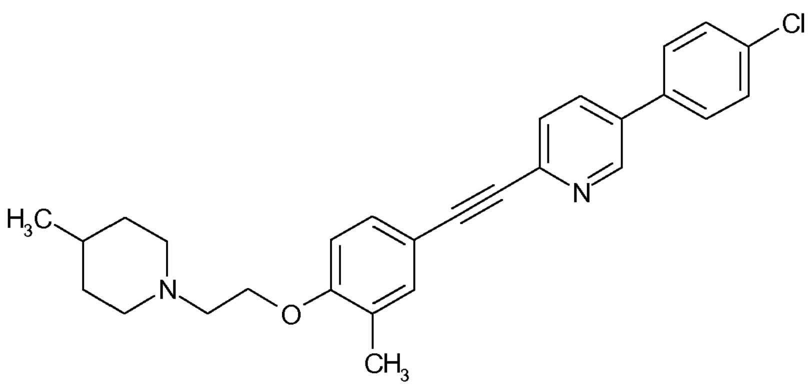

5-(4-Chlor-phenyl)-2-{3-methyl-4-[2-(4-methyl-piperidin-1-yl)-ethoxy]-phenylethinyl)-pyridin

(43)

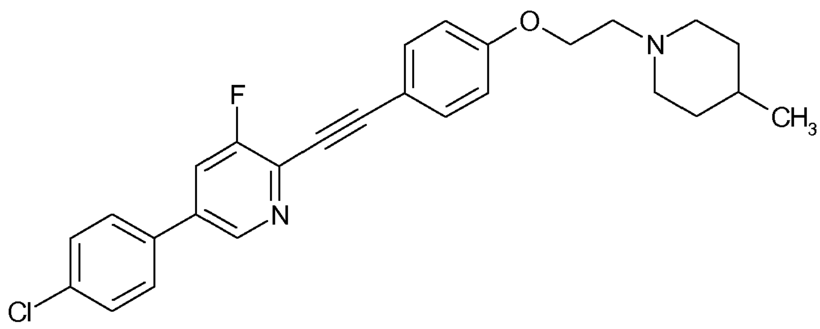

5-(4-Chlor-phenyl)-3-fluor-2-{4-[2-(4-methyl-piperidin-1-yl)-ethoxy]-phenylethinyl}-pyridin

(44)

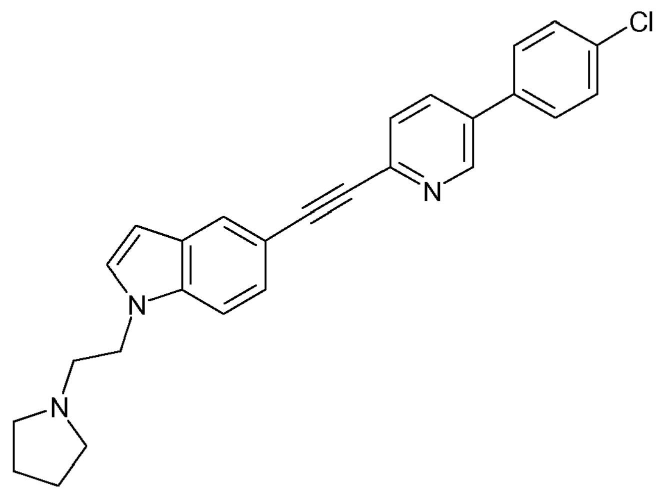

5-[5-(4-Chlor-phenyl)-pyridin-2-ylethinyl]-1-(2-pyrrolidin-1-yl-ethyl)-1H-indol

(45)

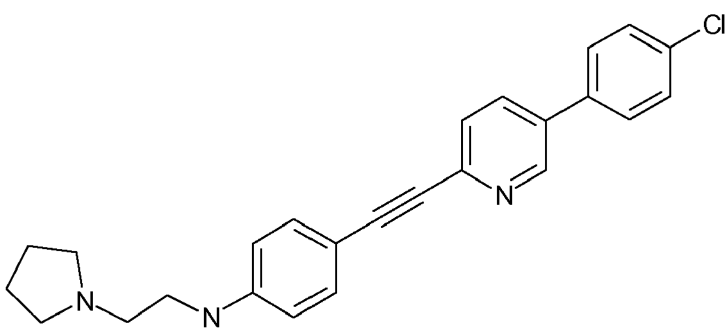

(4-[5-(4-Chlor-phenyl)-pyridin-2-ylethinyl]-phenyl)-(2-pyrrolidin-1-yl-ethyl)-amin

(46)

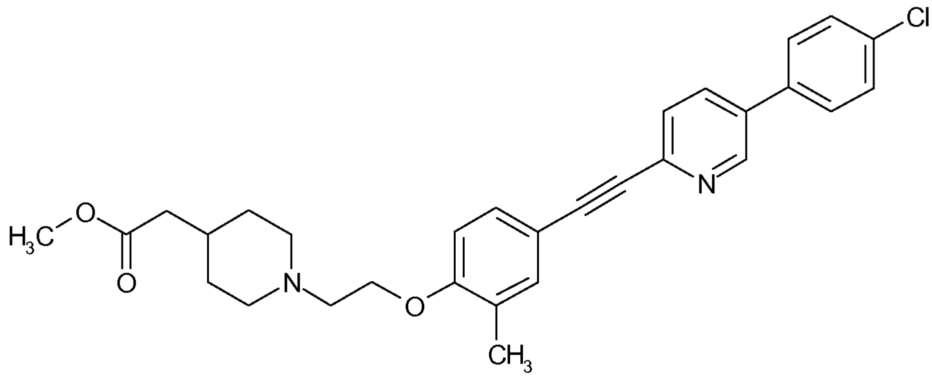

[1-(2-(4-[5-(4-Chlor-phenyl)-pyridin-2-ylethinyl]-2-methyl-phenoxy)-ethyl)-piperidin-4-yl]-essigsäuremethylester

(47)

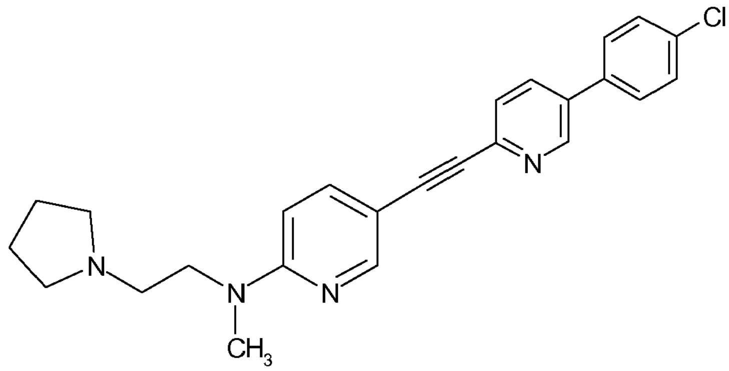

(5-[5-(4-Chlor-phenyl)-pyridin-2-ylethinyl]-pyridin-2-yl)-methyl-(2-pyrrolidin-1-yl-ethyl)-amin

(48)

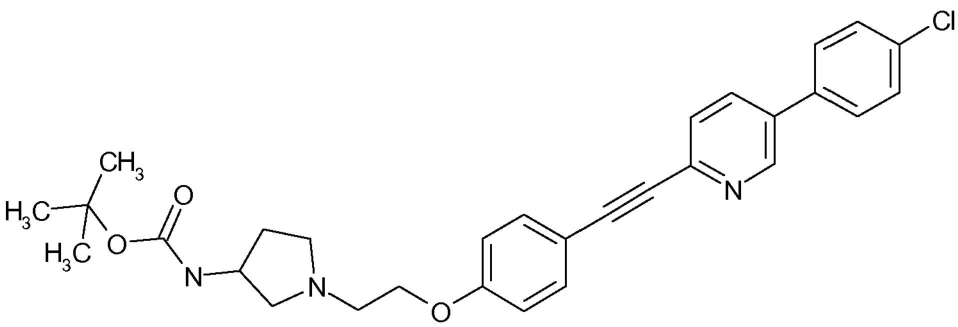

[1-(2-(4-[5-(4-Chlor-phenyl)-pyridin-2-ylethinyl]-phenoxy)-ethyl)-pyrrolidin-3-yl]-carbaminsäure-tert-butylester

(49)

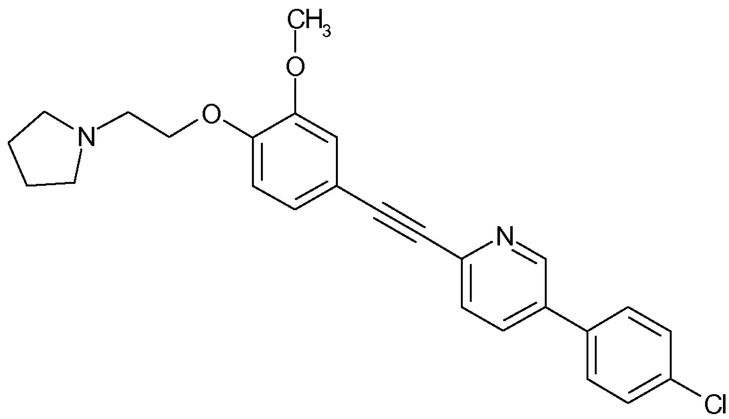

5-(4-Chlor-phenyl)-2-[3-methoxy-4-(2-pyrrolidin-1-yl-ethoxy)-phenylethinyl]-pyridin

(50)

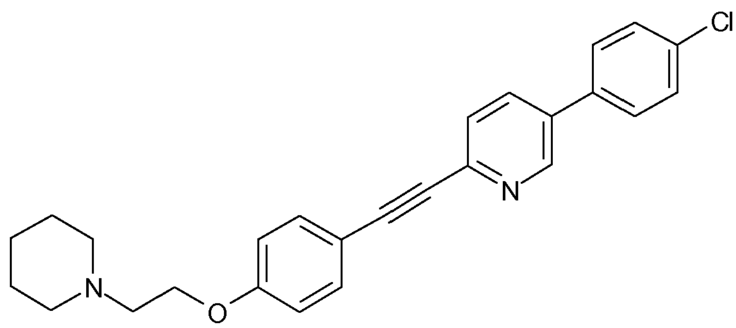

5-(4-Chlor-phenyl )-2-[4-(2-piperid in-1-yl-ethoxy)-phenyleth inyl]-pyrid in

(51)

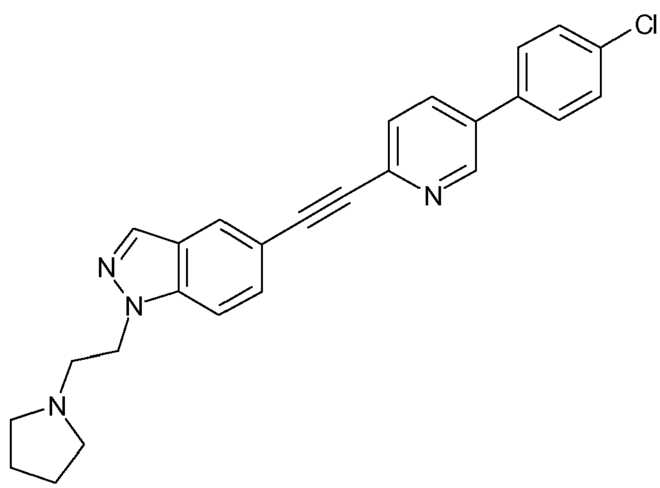

5-[5-(4-Chlor-phenyl)-pyridin-2-ylethinyl]-1-(2-pyrrolidin-1-yl-ethyl)-1H-indazol

(52)

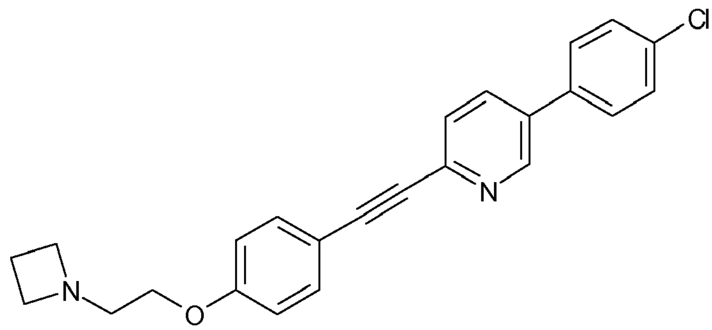

2-[4-(2-Azetid in-1-yl-ethoxy)-phenyleth inyl]-5-(4-ch lor-phenyl )-pyrid in

(53)

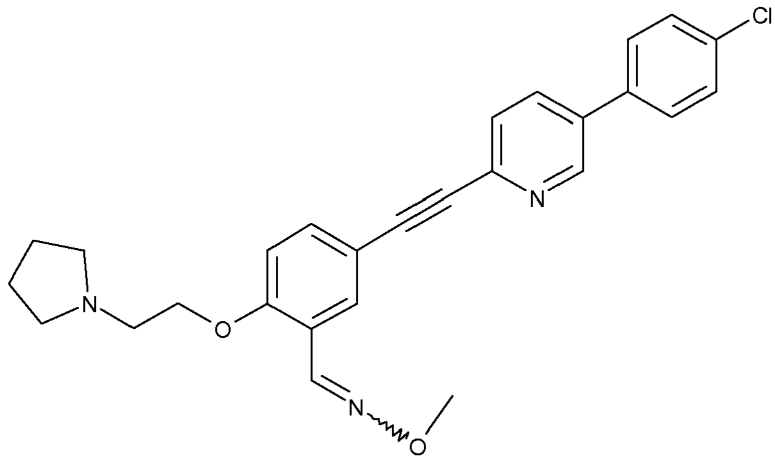

5-[5-(4-Chlor-phenyl)-pyridin-2-ylethinyl]-2-(2-pyrrolidin-1-yl-ethoxy)-benzaldehyd O-methyl-oxim

(54)

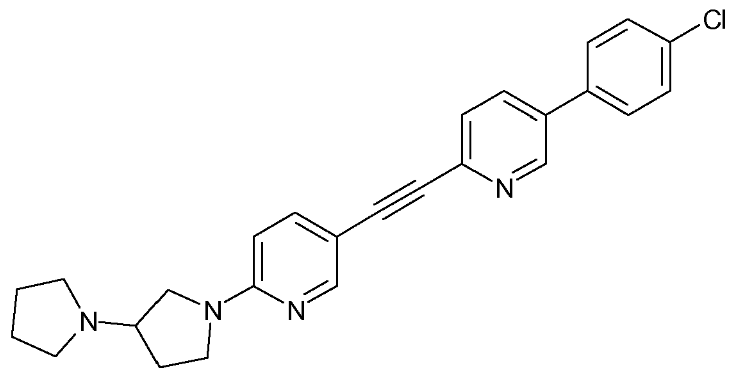

h1'-(5-[5-(4-Chlor-phenyl)-pyridin-2-ylethinyl]-pyridin-2-yl)-[1,3']bipyrrolidinyl

(55)

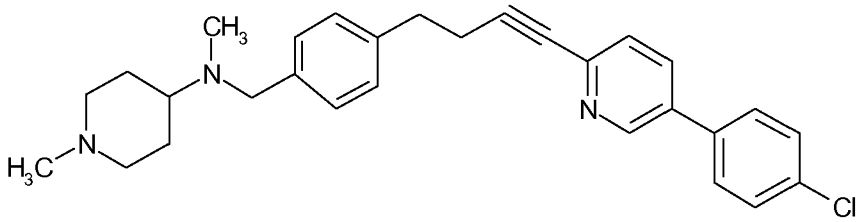

(4-{4-[5-(4-Chlor-phenyl)-pyhdin-2-yl]-but-3-inyl}-benzyl)-methyl-(1-methyl-piperidin-4-yl)-amin

(56)

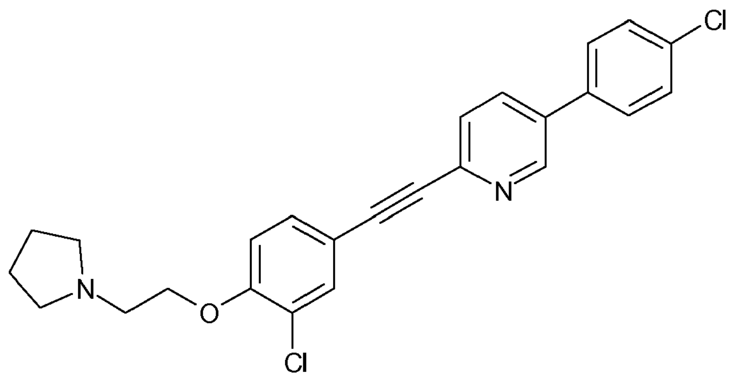

5-(4-Chlor-phenyl )-2-[3-chlor-4-(2-pyrrol id in-1-yl-ethoxy)-phenylethynyl]-pyridin

(57)

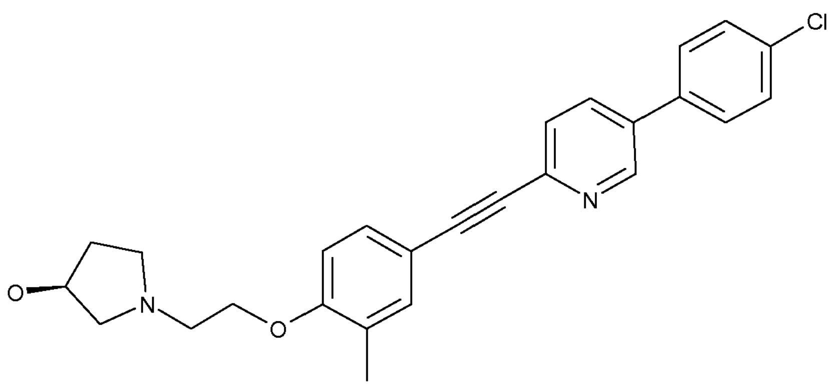

(S)-1-(2-(4-[5-(4-Chlor-phenyl)-pyridin-2-ylethinyl]-2-methyl-phenoxy)-ethyl)-pyrrolidin-3-ol

(58)

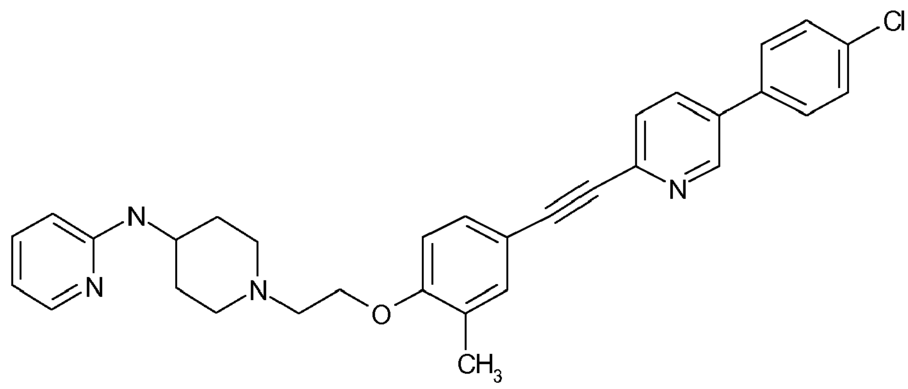

[1-(2-(4-[5-(4-Chlor-phenyl)-pyridin-2-ylethinyl]-2-methyl-phenoxy)-ethyl)-piperidin-4-yl]-pyridin-2-yl-amin

(59)

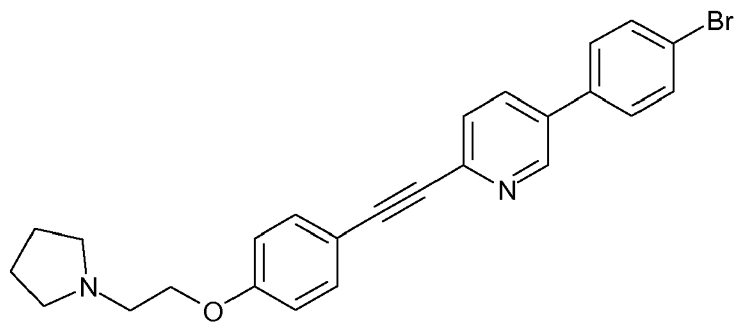

5-(4-Brom-phenyl )-2-[4-(2-pyrrol id in-1-yl-ethoxy)-phenyleth inyl]-pyrid in

(60)

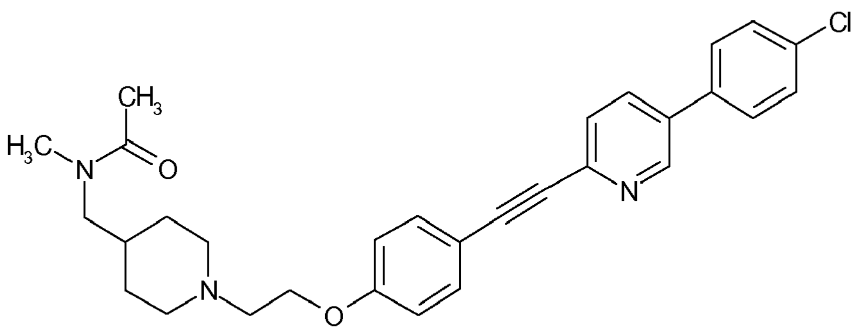

N-[1-(2-(4-[5-(4-Chlor-phenyl)-pyridin-2-ylethinyl]-phenoxy)-ethyl)-piperidin-4-ylmethyl]-N-methyl-acetamid

(61)

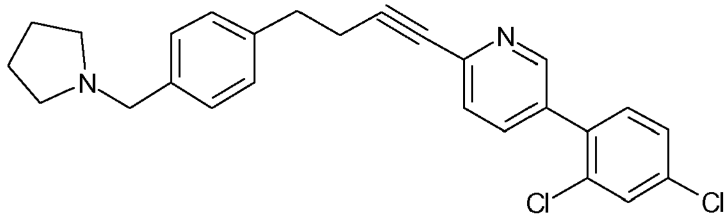

5-(2,4-Dichlor-phenyl)-2-[4-(4-pyrrolidin-1-ylmethyl-phenyl)-but-1-inyl]-pyridin

(62)

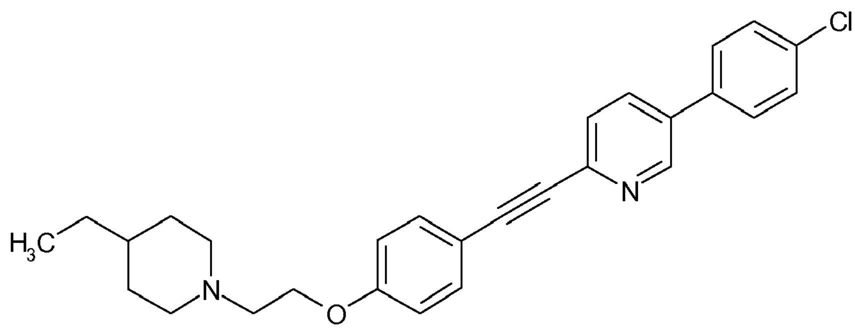

5-(4-Chlor-phenyl)-2-(4-[2-(4-ethyl-piperidin-1-yl)-ethoxy]-phenylethinyl)-pyridin

(63)

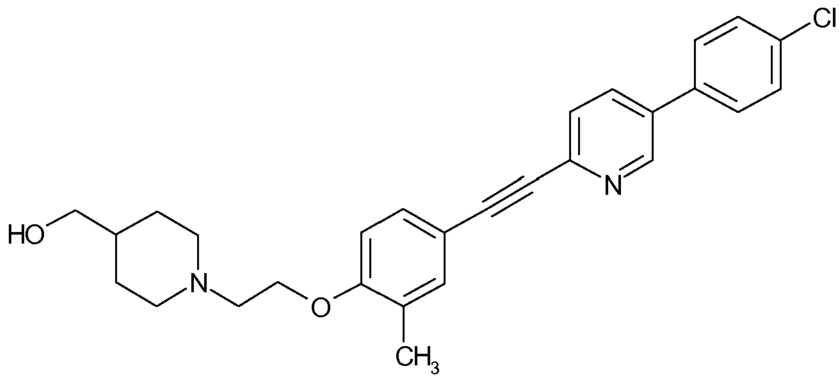

[1-(2-{4-[5-(4-Chlor-phenyl)-pyridin-2-ylethinyl]-2-methyl-phenoxyl-ethyl)-piperidin-4-yl]-methanol

(64)

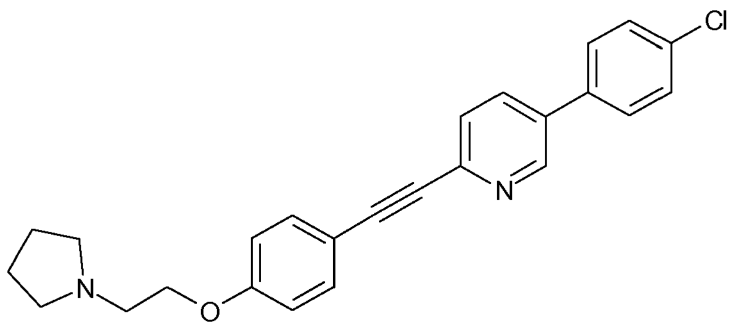

5-(4-Chlor-phenyl )-2-[4-(2-pyrrol id in-1-yl-ethoxy)-phenyleth inyl]-pyrid in

(65)

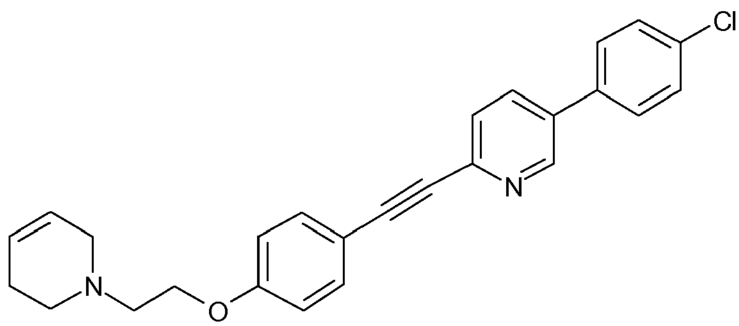

5-(4-Chlor-phenyl)-2-{4-[2-(3,6-dihydro-2H-pyridin-1 -yl)-ethoxy]-phenylethinyl)-pyridin

(66)

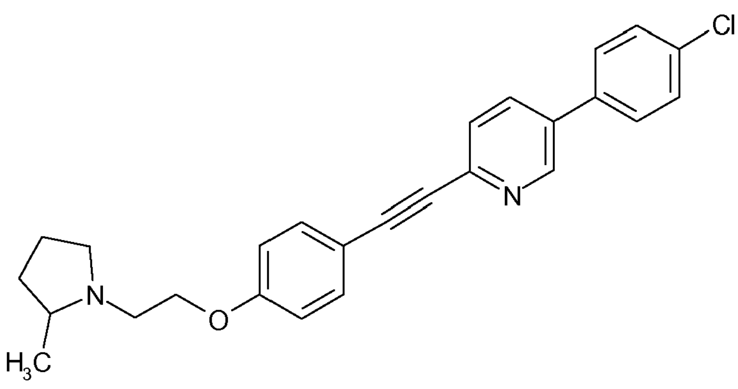

5-(4-Chlor-phenyl)-2-{4-[2-(2-methyl-pyrrolidin-1 -yl)-ethoxy]-phenylethinyl)-pyridin

(67)

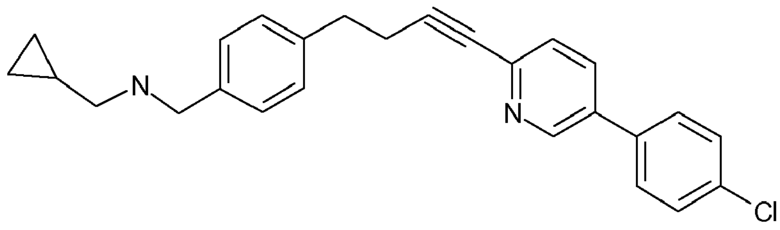

(4-{4-[5-(4-Chlor-phenyl)-pyridin-2-yl]-but-3-inyl}-benzyl)-cyclopropylmethyl-amin

(68)

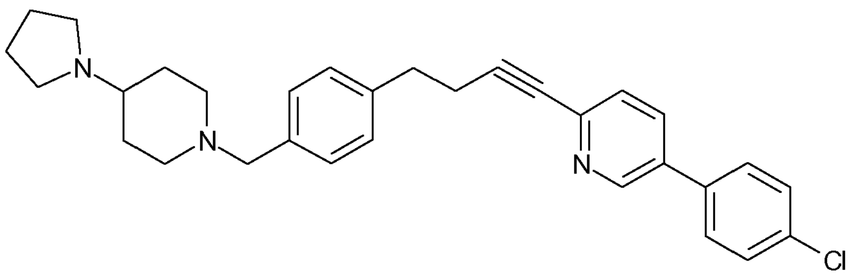

5-(4-Chlor-phenyl)-2-{4-[4-(4-pyrrolidin-1 -yl-piperidin-1 -ylmethyl)-phenyl]-but-1-inyl)-pyrid in

(69)

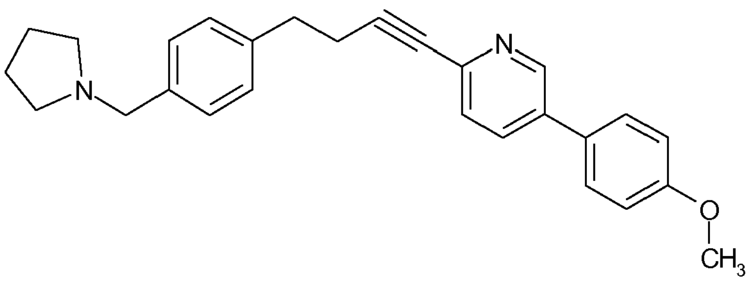

5-(4-Methoxy-phenyl)-2-[4-(4-pyrrolidin-1-ylmethyl-phenyl)-but-1-inyl]-pyridin

(70)

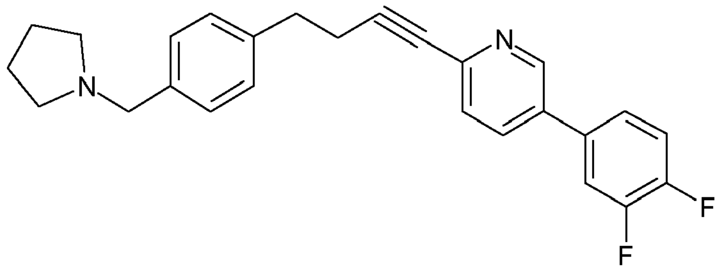

5-(3,4-Difluor-phenyl)-2-[4-(4-pyrrolidin-1-ylmethyl-phenyl)-but-1-inyl]-pyridin

(71)

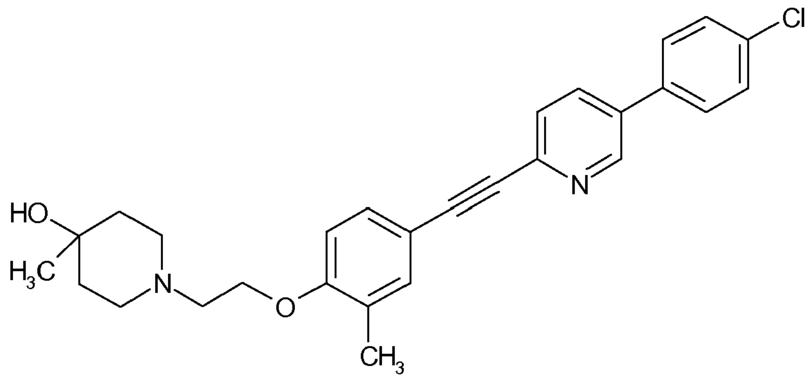

1-(2-{4-[5-(4-Chlor-phenyl)-pyridin-2-ylethinyl]-2-methyl-phenoxyl-ethyl)-4-methyl-piperidin-4-ol

(72)

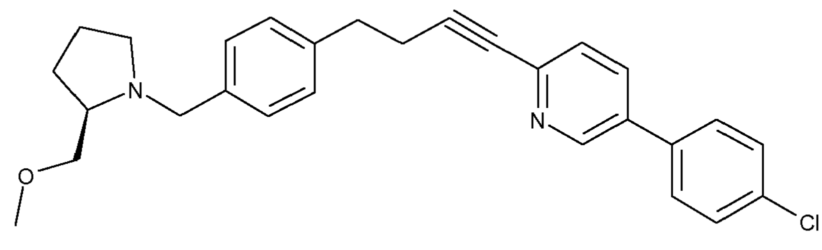

5-(4-Chlor-phenyl)-2-{4-[4-((R)-2-methoxymethyl-pyrrolidin-1-ylmethyl)-phenyl]-but-1-inyl)-pyridin

(73)

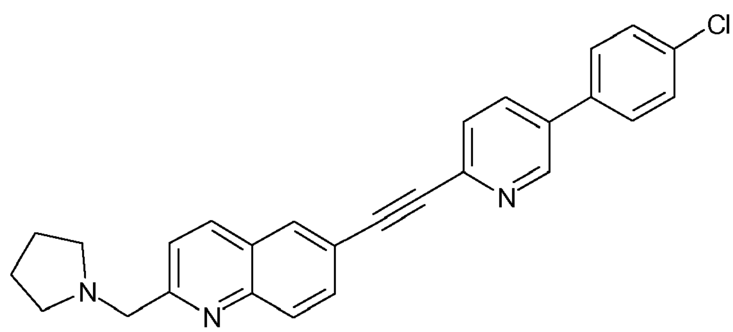

6-[5-(4-Chlor-phenyl )-pyrid in-2-yleth inyl]-2-pyrrol id in-1-yl methyl-ch inol in

(74)

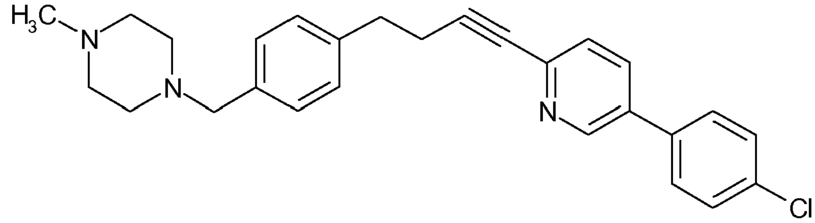

1-(4-{4-[5-(4-Ch lor-phenyl)-pyrid in-2-yl]-but-3-inyll-benzyl)-4-methylpiperazin

(75)

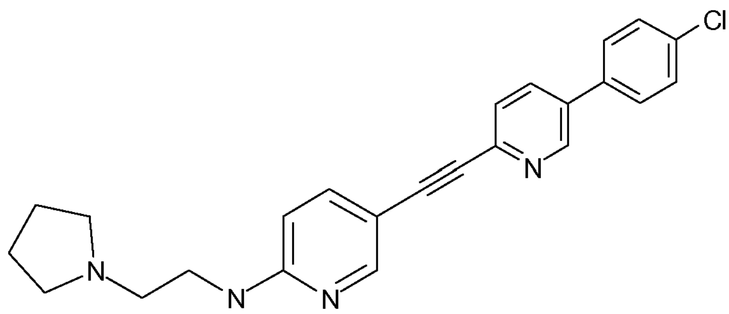

(5-[5-(4-Chlor-phenyl)-pyridin-2-ylethinyl]-pyridin-2-yl)-(2-pyrrolidin-1-yl-ethyl)-amin

(76)

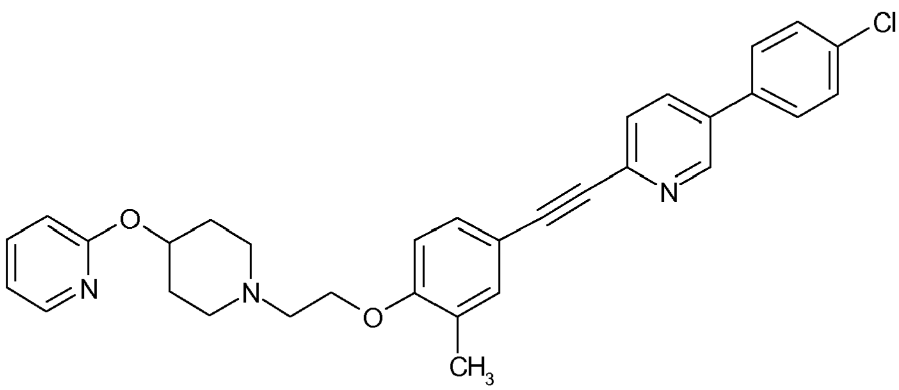

5-(4-Chlor-phenyl)-2-(3-methyl-4-{2-[4-(pyridin-2-yloxy)-piperidin-1 -yl]-ethoxy}-phenylethlnyl)-pyridin

(77)

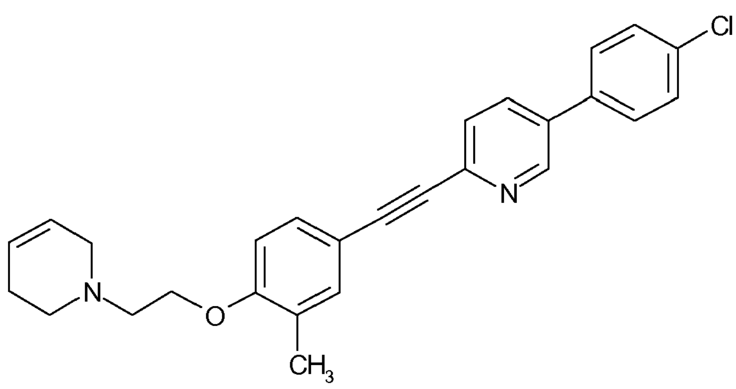

5-(4-Chlor-phenyl)-2-{4-[2-(3,6-dihydro-2H-pyridin-1-yl)-ethoxy]-3-methyl-phenylethinyl)-pyridin

(78)

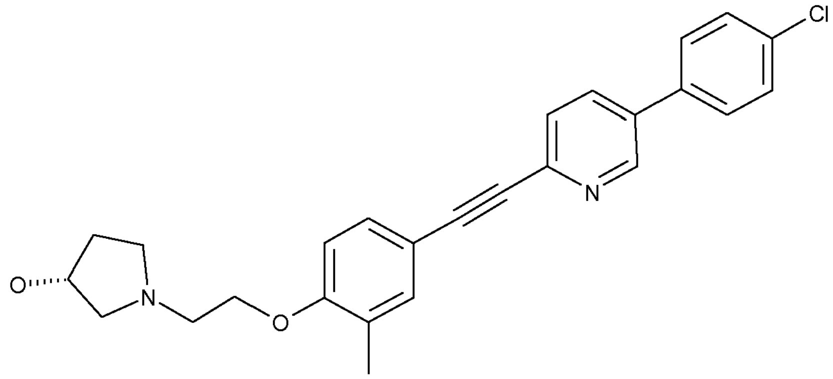

(R)-1-(2-{4-[5-(4-Chlor-phenyl)-pyridin-2-ylethinyl]-2-methyl-phenoxy}-ethyl)-pyrrolidin-3-ol

(79)

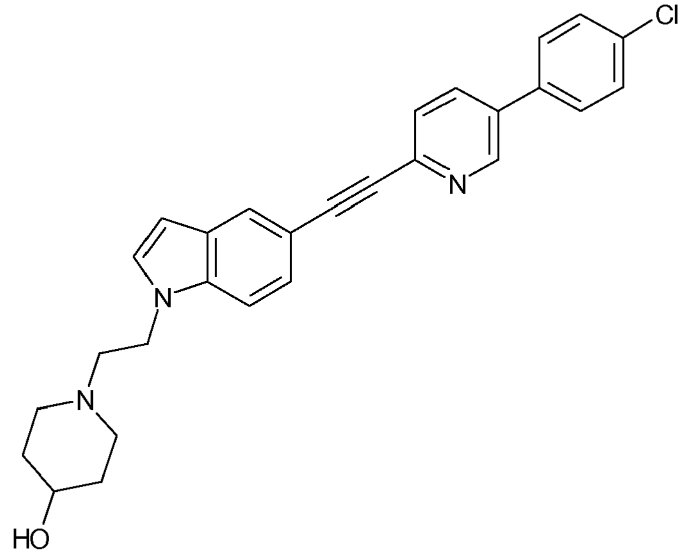

1-(2-{5-[5-(4-Chlor-phenyl)-pyridin-2-ylethinyl]-indol-1-yl)-ethyl)-piperidin-4-ol

(80)

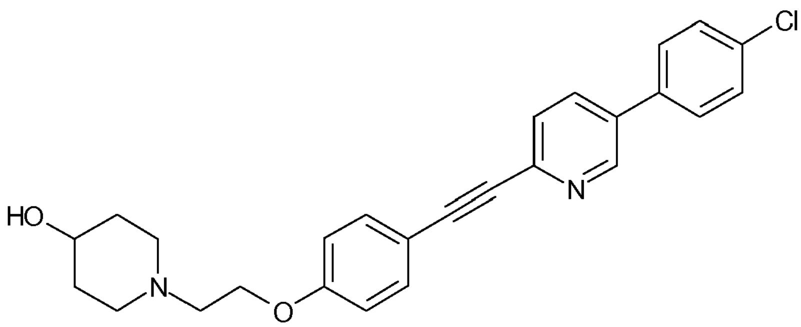

1-(2-(4-[5-(4-Chlor-phenyl)-pyridin-2-ylethinyl]-phenoxy)-ethyl)-piperidin-4-ol

(81)

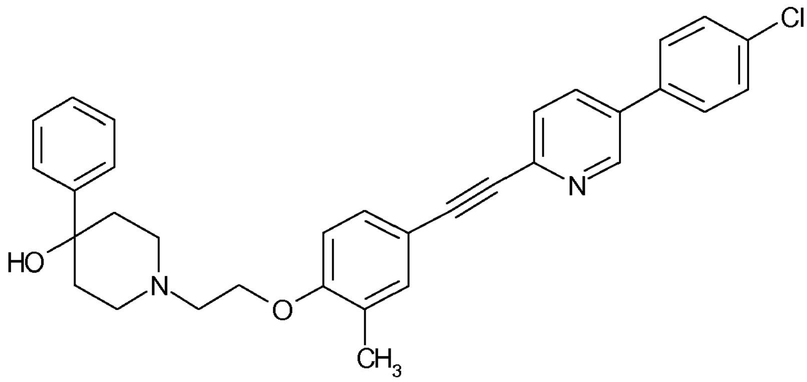

1-(2-(4-[5-(4-Chlor-phenyl)-pyridin-2-ylethinyl]-2-methyl-phenoxy)-ethyl)-4-phenyl-piperidin-4-ol

(82)

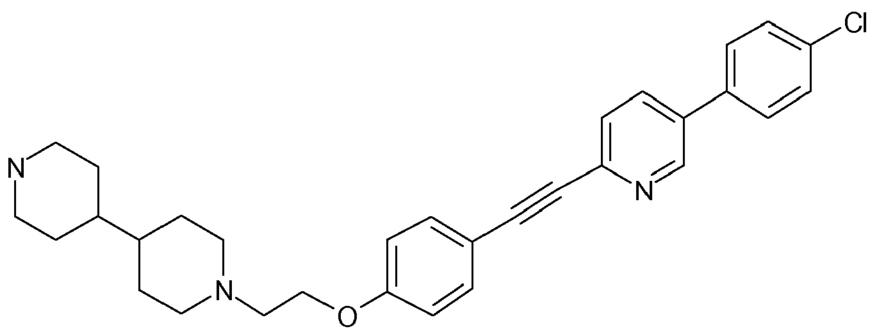

1-(2-{4-[5-(4-Chlor-phenyl)-pyridin-2-ylethinyl]-phenoxyl-ethyl)-[4,4']bipiperidinyl

(83)

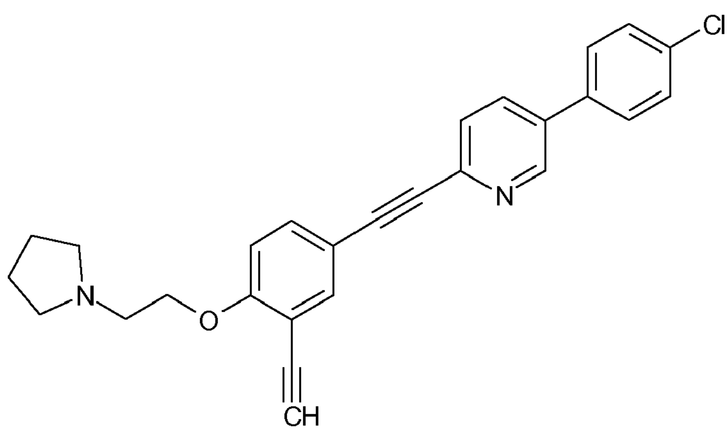

5-(4-Chlor-phenyl )-2-[3-eth inyl-4-(2-pyrrol id in-1-yl-ethoxy)-phenyleth inyl]-pyridin

(84)

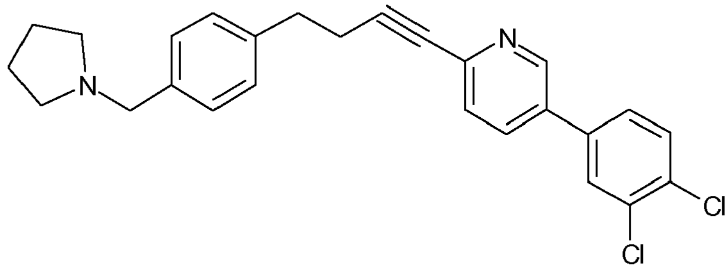

5-(3,4-Dichlor-phenyl)-2-[4-(4-pyrrolidin-1-ylmethyl-phenyl)-but-1-inyl]-pyridin

(85)

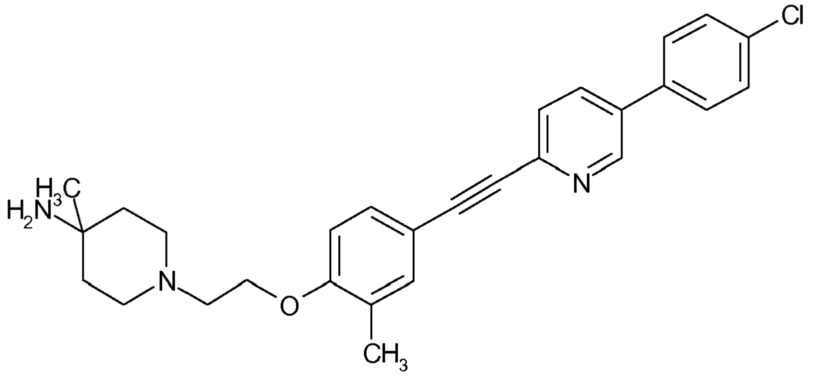

1-(2-{4-[5-(4-Chlor-phenyl)-pyridin-2-ylethinyl]-2-methyl-phenoxyl-ethyl)-4-methyl-piperidin-4-ylamin

(86)

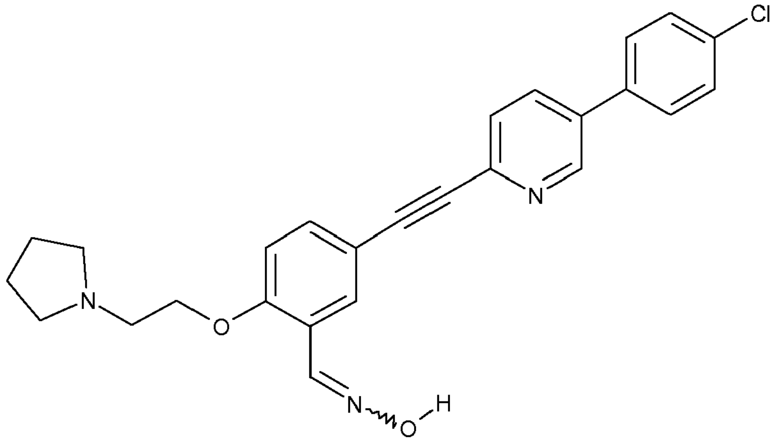

5-[5-(4-Chlor-phenyl)-pyridin-2-ylethinyl]-2-(2-pyrrolidin-1-yl-ethoxy)-benzaldehyd-oxim

(87)

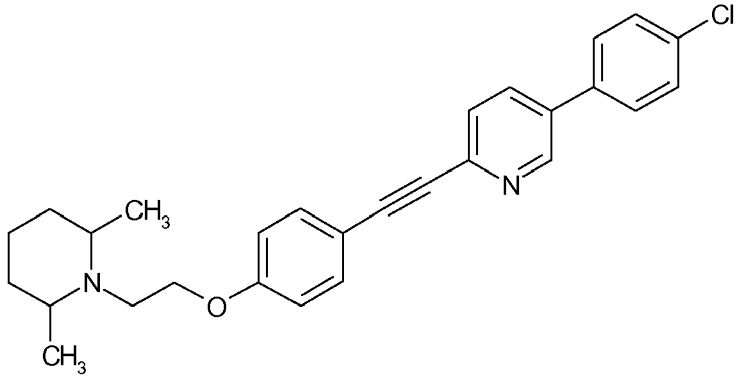

5-(4-Chlor-phenyl)-2-{4-[2-(2,6-dimethyl-piperidin-1 -yl)-ethoxy]-phenylethinyl)-pyridin

(88)

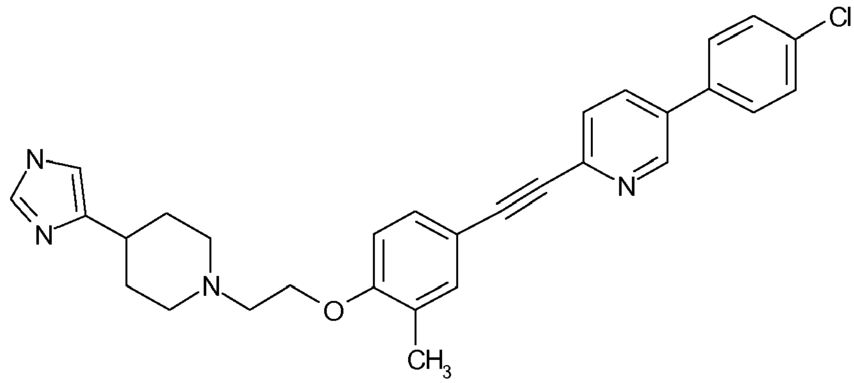

5-(4-Chlor-phenyl)-2-(4-{2-[4-(1H-imidazol-4-yl)-piperidin-1-yl]-ethoxy)-3-methyl-phenyleth inyl )-pyrid in

(89)

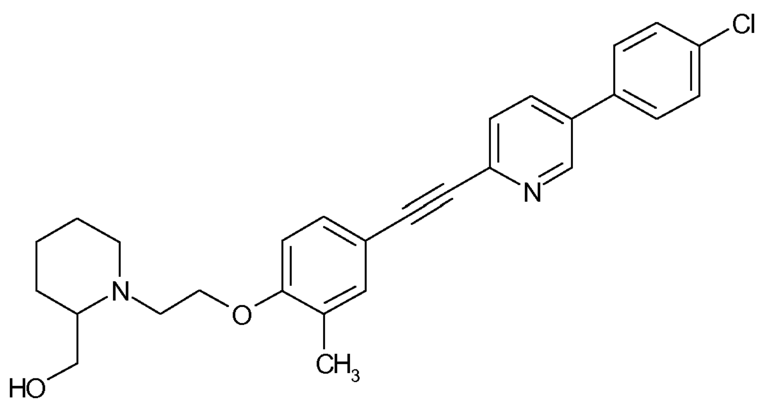

[1-(2-{4-[5-(4-Chlor-phenyl)-pyridin-2-ylethinyl]-2-methyl-phenoxy)-ethyl)-piperidin-2-yl]-methanol

(90)

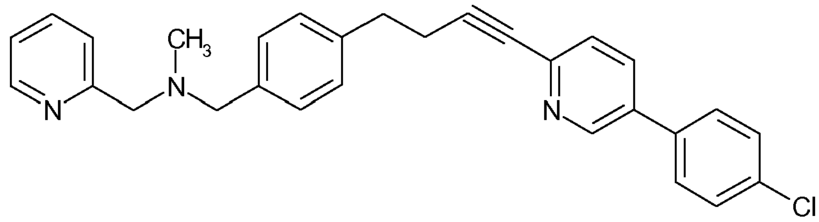

(4-{4-[5-(4-Chlor-phenyl)-pyrid in-2-yl]-but-3-inyll-benzyl)-methyl-pyridin-2-ylmethyl-amin

(91)

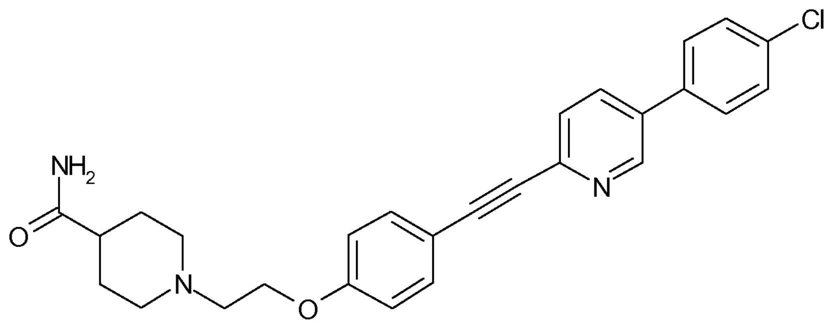

1 -(2-{4-[5-(4-Chlor-phenyl)-pyridin-2-ylethinyl]-phenoxyl-ethyl)-piperidin-4-carbonsäureamid

(92)

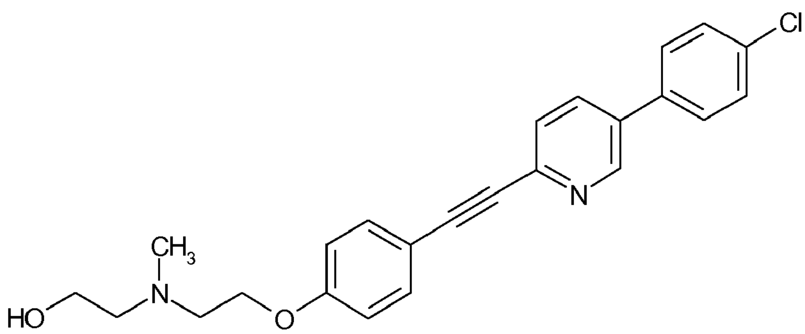

2-[(2-{4-[5-(4-Chlor-phenyl)-pyridin-2-ylethinyl]-phenoxyl-ethyl)-methyl-amino]-ethanol

(93)

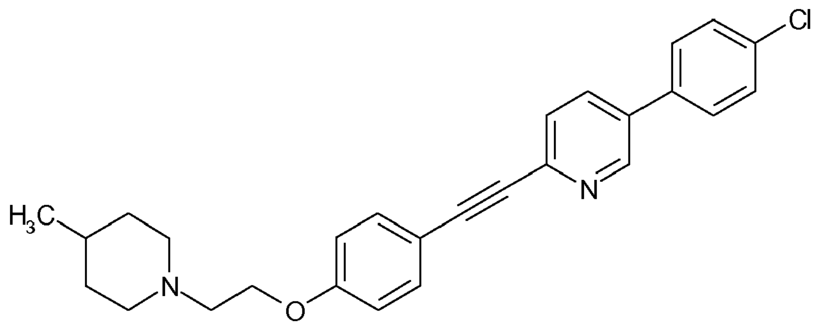

5-(4-Chlor-phenyl)-2-{4-[2-(4-methyl-piperidin-1-yl)-ethoxy]-phenylethinyl)-pyridin

(94)

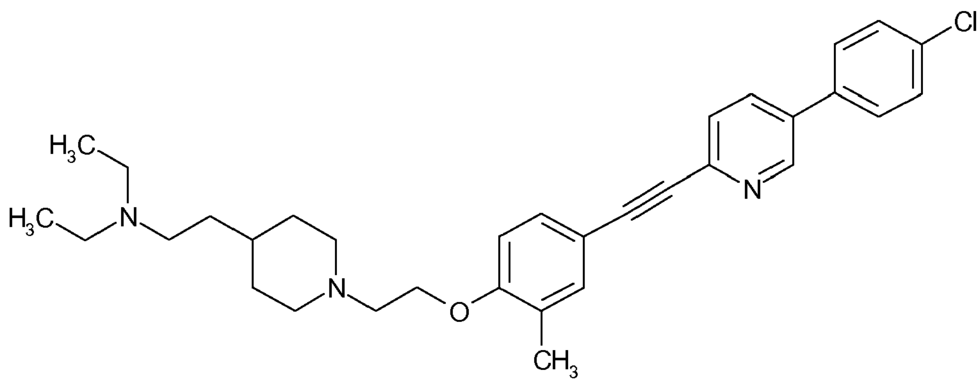

{2-[1-(2-{4-[5-(4-Chlor-phenyl)-pyridin-2-ylethinyl]-2-methyl-phenoxyl-ethyl)-piperidin-4-yl]-ethyl)-diethyl-amin

(95)

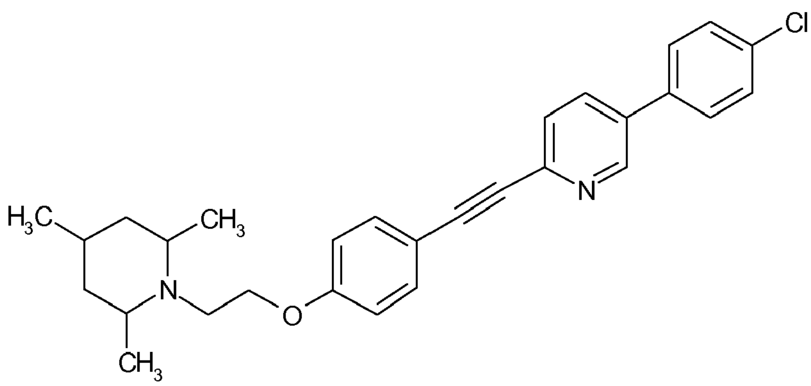

5-(4-Chlor-phenyl)-2-{4-[2-(2,4,6-trimethyl-piperidin-1-yl)-ethoxy]-phenylethinyl)-pyridin

(96)

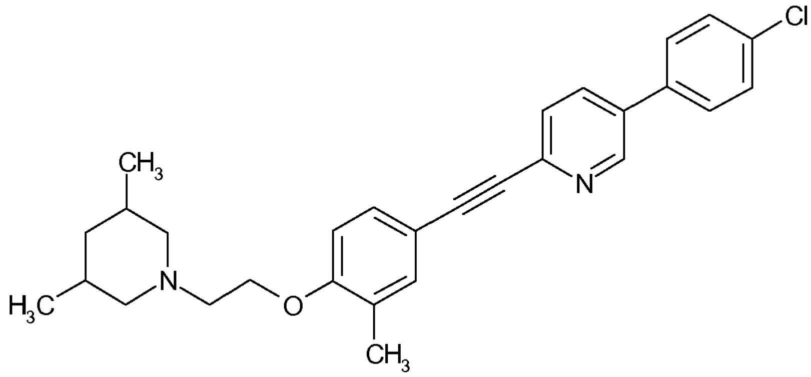

5-(4-Chlor-phenyl)-2-{4-[2-(3,5-dimethyl-piperidin-1-yl)-ethoxy]-3-methyl-phenylethlnyl}-pyridin

(97)

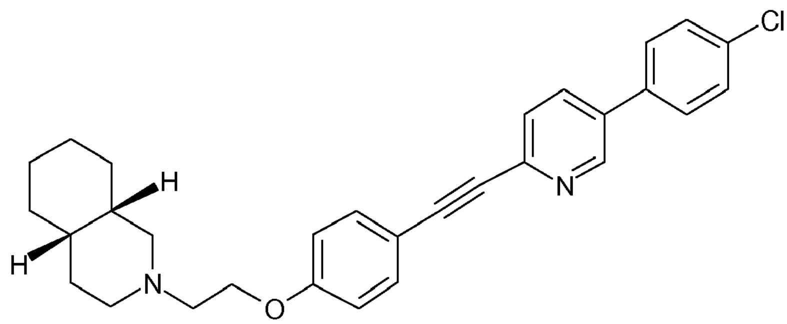

cis-2-(2-{4-[5-(4-Chlor-phenyl)-pyridin-2-ylethinyl]-phenoxyl-ethyl)-decahydro-isochinolin

(98)

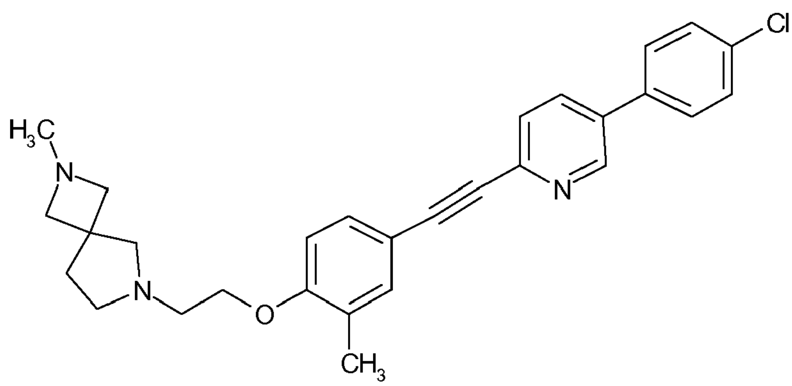

6-(2-{4-[5-(4-Chlor-pheny)-pyhdin-2-ylethinyl]-2-methyl-phenoxy}-ethyl)-2-methyl-2,6-diaza-spiro[3.4]octan

(99)

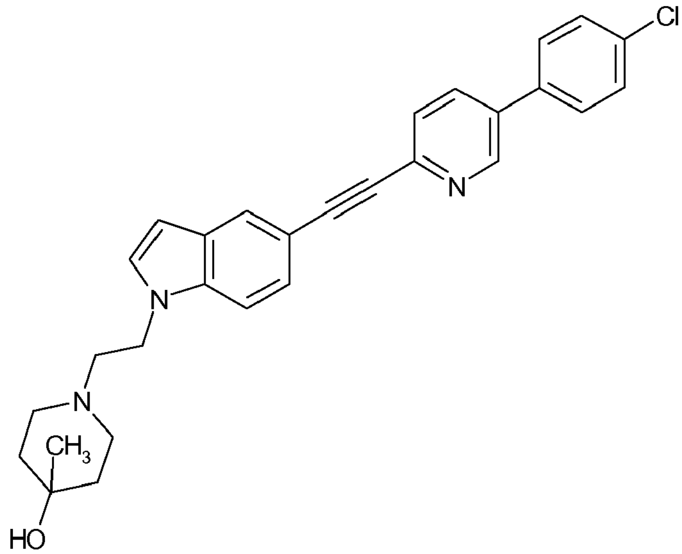

1-(2-(5-[5-(4-Chlor-phenyl)-pyridin-2-ylethinyl]-indol-1-yl)-ethyl)-4-methylpiperidin-4-ol

(100)

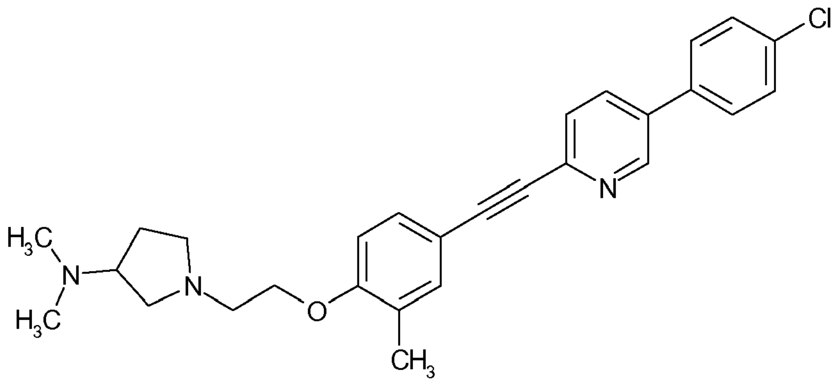

[1-(2-{4-[5-(4-Chlor-phenyl)-pyridin-2-ylethinyl]-2-methyl-phenoxyl-ethyl)-pyrrolidin-3-yl]-dimethyl-amin

(101)

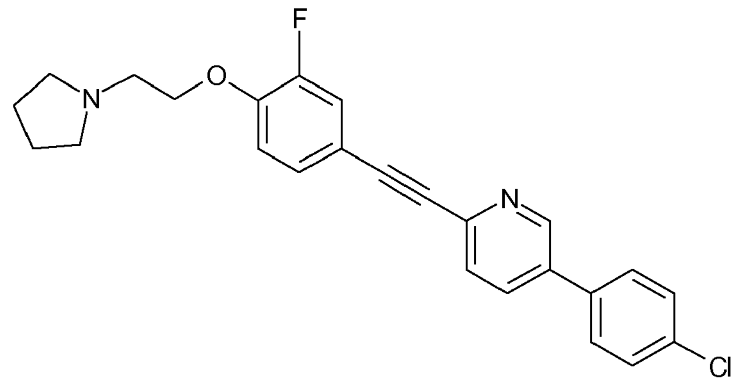

5-(4-Chlor-phenyl )-2-[3-fl uor-4-(2-pyrrol id in-1-yl-ethoxy)-phenyleth inyl]-pyridin

(102)

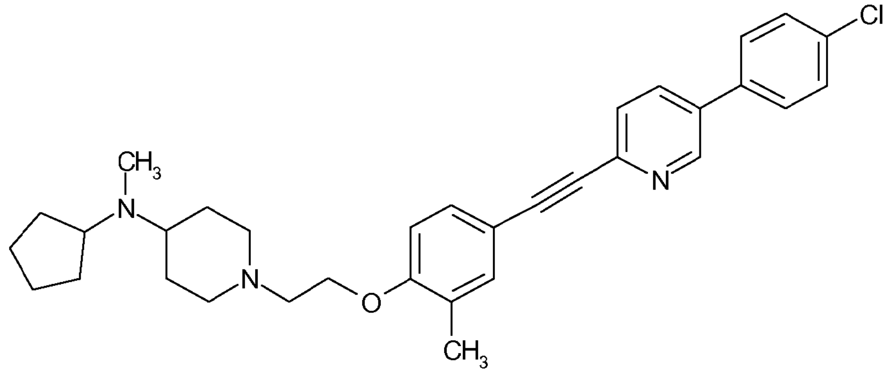

[1-(2-{4-[5-(4-Chlor-phenyl)-pyridin-2-ylethinyl]-2-methyl-phenoxyl-ethyl)-piperidin-4-yl]-cyclopentyl-methyl-amin

(103)

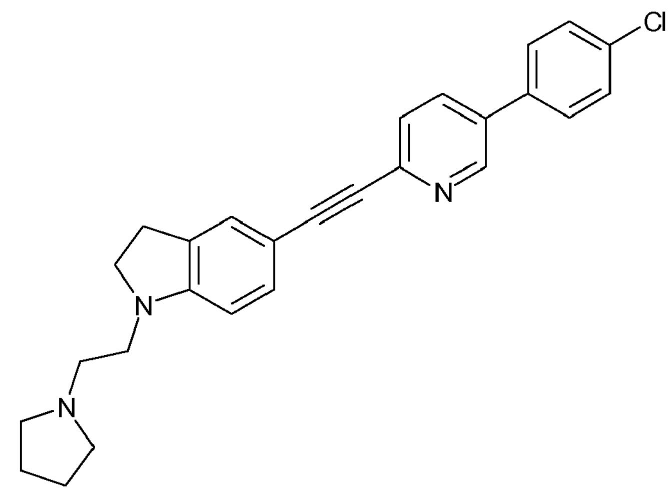

5-[5-(4-Chlor-phenyl)-pyridin-2-ylethinyl]-1-(2-pyrrol idin-1-yl-ethyl)-2,3-dihydro-1H-indol

(104)

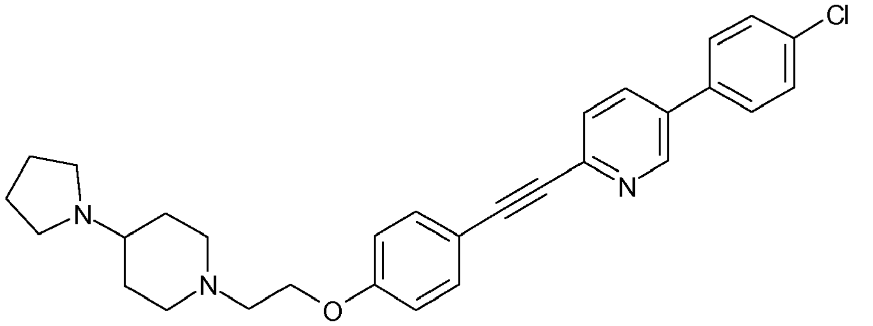

5-(4-Chlor-phenyl)-2-{4-[2-(4-pyrrolidin-1-yl-piperidin-1-yl)-ethoxy]-phenylethinyl)-pyridin

(105)

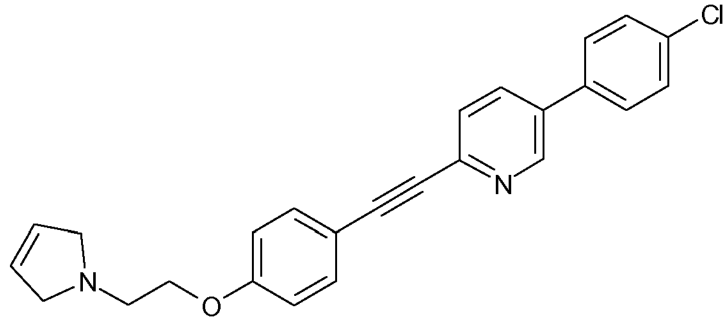

5-(4-Chlor-phenyl)-2-{4-[2-(2,5-dihydro-pyrrol-1-yl)-ethoxy]-phenylethinyl)-pyridin

(106)

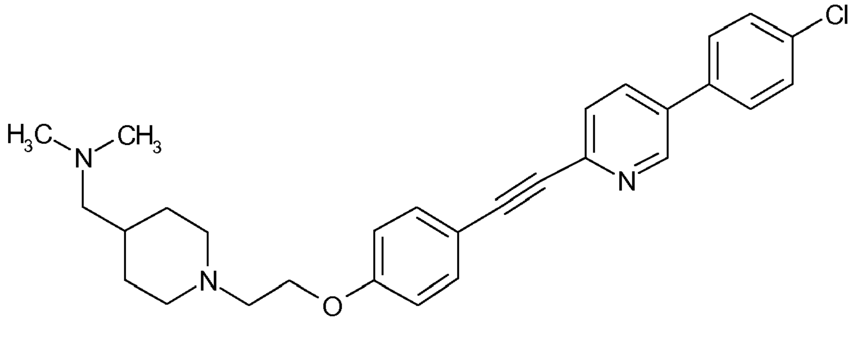

[1-(2-{4-[5-(4-Chlor-phenyl)-pyhdin-2-ylethinyl]-phenoxy}-ethyl)-pipehdin-4-ylmethyl]-dimethyl-amin

(107)

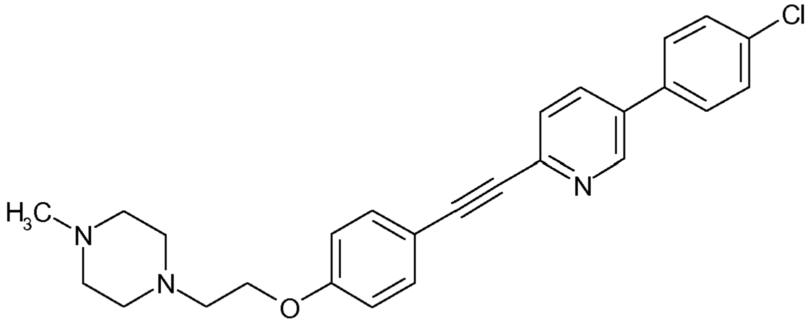

1-(2-{4-[5-(4-Chlor-phenyl)-pyridin-2-ylethinyl]-phenoxyl-ethyl)-4-methylpiperazin

(108)

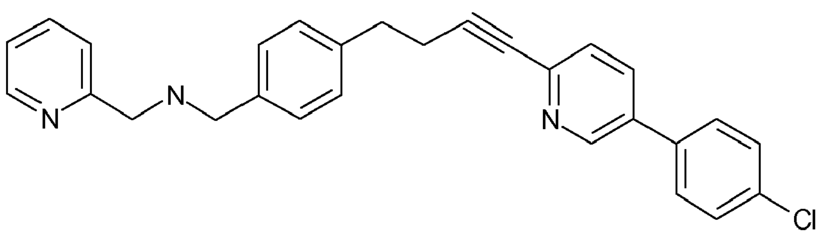

(4-{4-[5-(4-Chlor-phenyl)-pyrid in-2-yl]-but-3-inyll-benzyl)-pyrid in-2-ylmethyl-amin

(109)

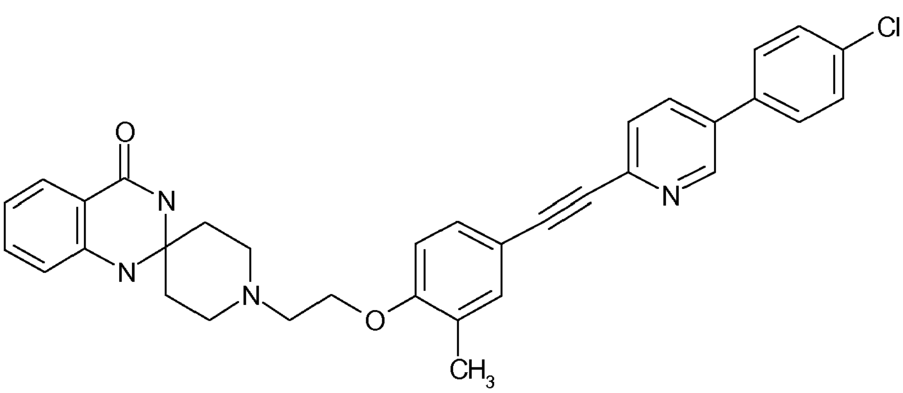

1-(2-{4-[5-(4-Chlor-phenyl)-pyridin-2-ylethinyl]-2-methyl-phenoxyl-ethyl)-spiro[piperidin-4,2'(1H')-chinazolin]-4'(3'H)on

(110)

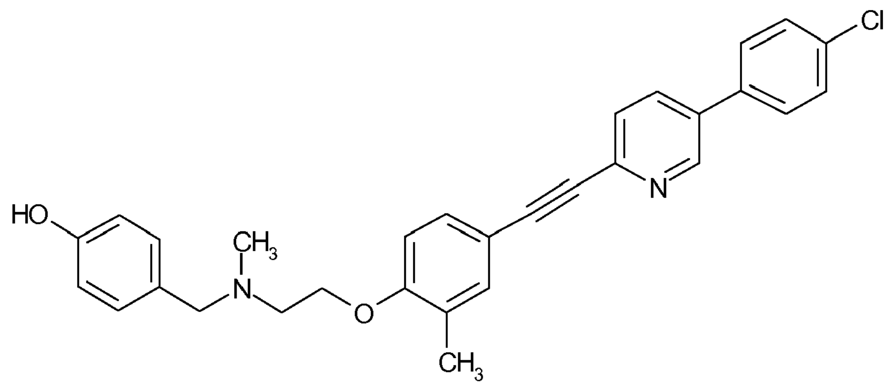

4-{[(2-{4-[5-(4-Chlor-phenyl)-pyrid in-2-yleth inyl]-2-methyl -phenoxyl-ethyl)-methyl-amino]-methyl)-phenol

(111)

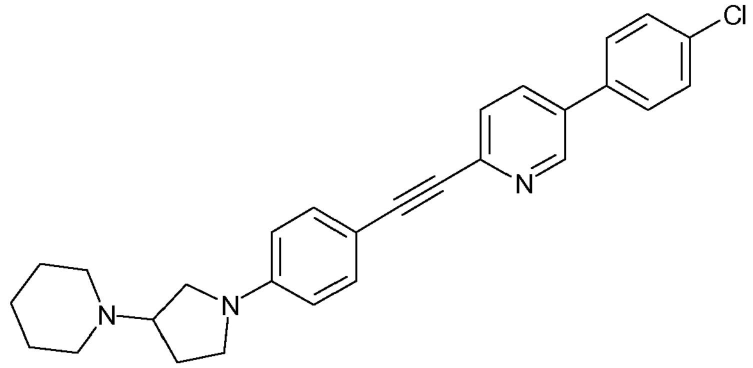

5-(4-Chlor-phenyl)-2-[4-(3-piperidin-1-yl-pyrrolidin-1-yl)-phenylethinyl]-pyridin

(112)

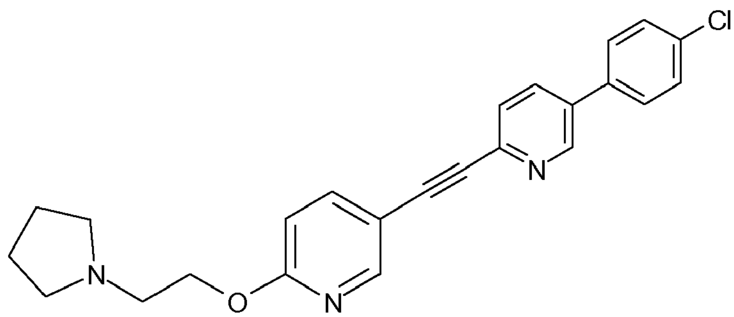

5-(4-Chlor-phenyl)-2-[2-(2-pyrrolidin-1-yl-ethoxy)-pyrid-5-yl-ethynyl]-pyridin

(113)

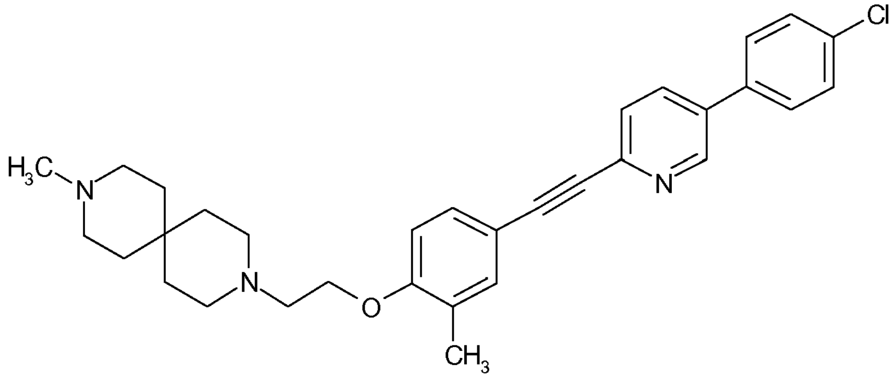

3-(2-{4-[5-(4-Chlor-phenyl)-pyridin-2-ylethinyl]-2-methyl-phenoxyl-ethyl)-9-methyl-3,9-diaza-spiro[5.5]undecan

(114)

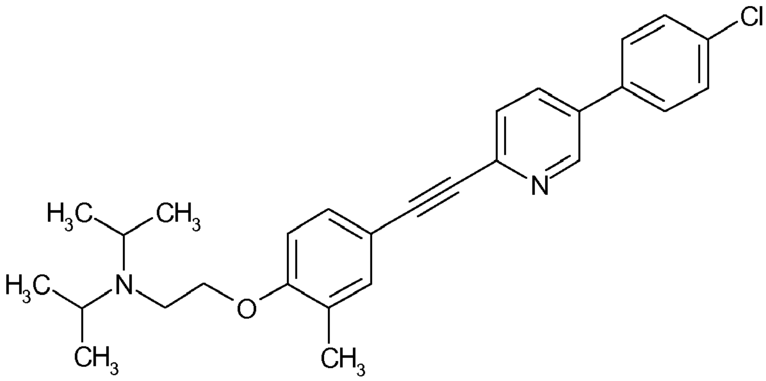

(2-{4-[5-(4-Chlor-phenyl)-pyridin-2-ylethinyl]-2-methyl-phenoxyl-ethyl)-diisopropyl-amin

(115)

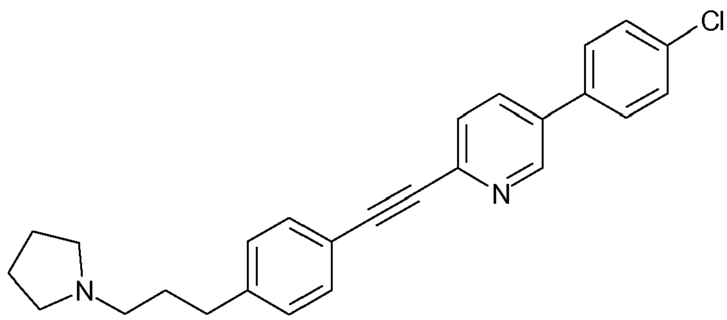

5-(4-Chlor-phenyl)-2-[4-(3-pyrrolidin-1-yl-propyl)-phenylethinyl]-pyridin

(116)

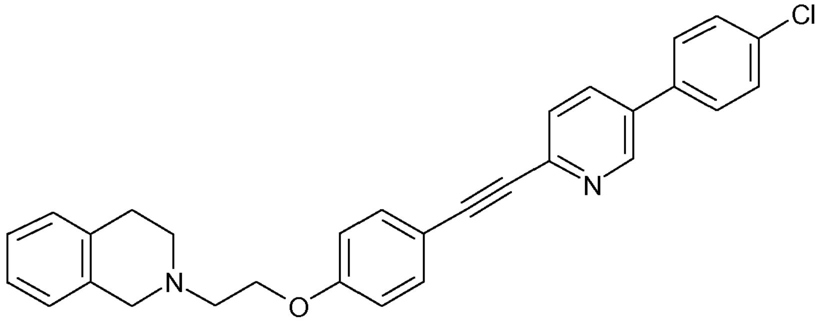

2-(2-{4-[5-(4-Chlor-phenyl)-pyridin-2-ylethinyl]-phenoxyl-ethyl)- 1,2,3,4-tetrahydro-isochinolin

(117)

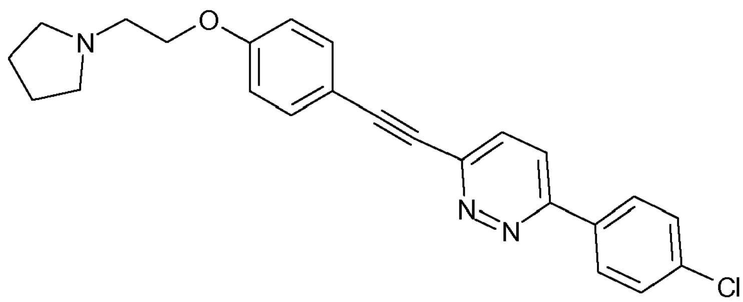

3-(4-Chlor-phenyl)-6-[4-(2-pyrrolidin-1-yl-ethoxy)-phenylethinyl]-pyridazin

(118)

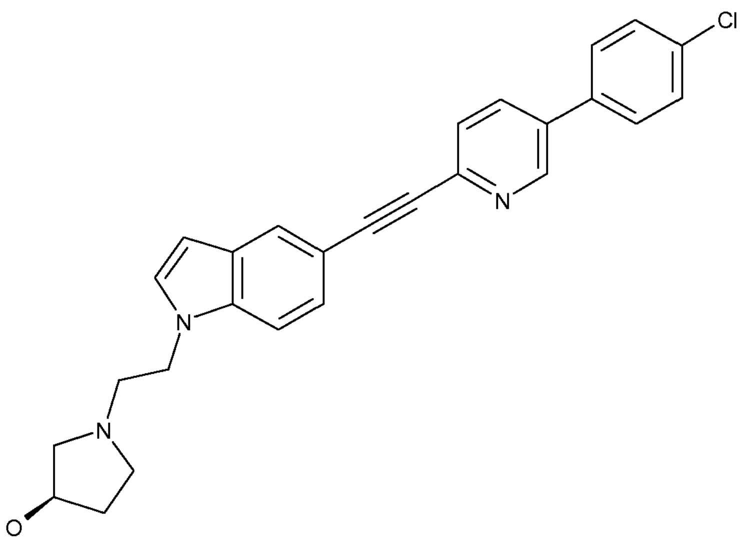

(R)-1-(2-{5-[5-(4-Chlor-phenyl)-pyridin-2-ylethinyl]-indol-1-yll-ethyl)-pyrrolidin-3-ol

(119)

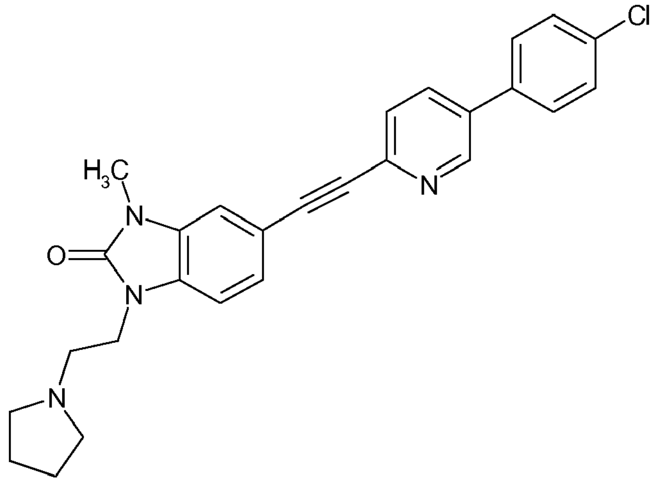

5-[5-(4-Chlor-phenyl)-pyridin-2-ylethinyl]-3-methyl-1-(2-pyrrolidin-1-yl-ethyl)-1,3-dihydro-benzimidazol-2-on

(120)

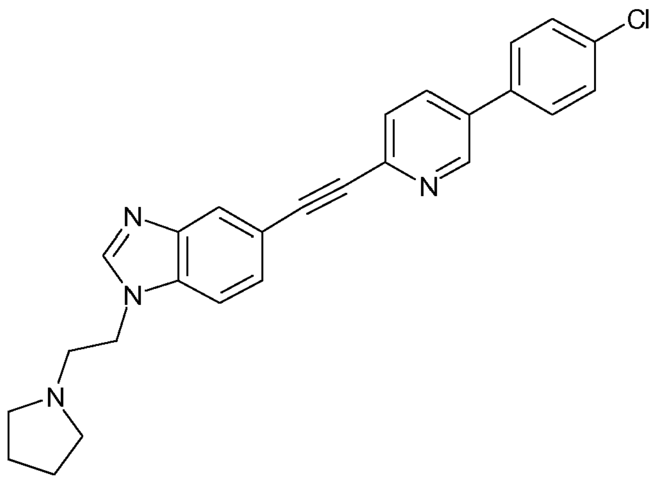

5-[5-(4-Chlor-phenyl)-pyridin-2-ylethinyl]-1-(2-pyrrolidin-1-yl-ethyl)-1H-benzimidazol

(121)

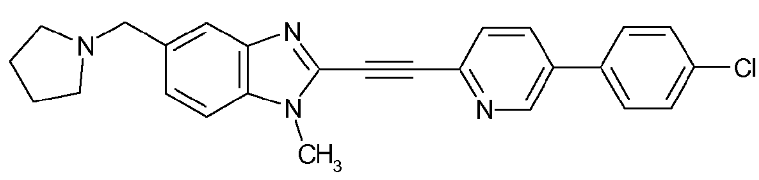

2-[5-(4-Chlor-phenyl)-pyridin-2-ylethinyl]-1-methyl-5-pyrrolidin-1-ylmethyl-**1H-benzimidazol**

(122)

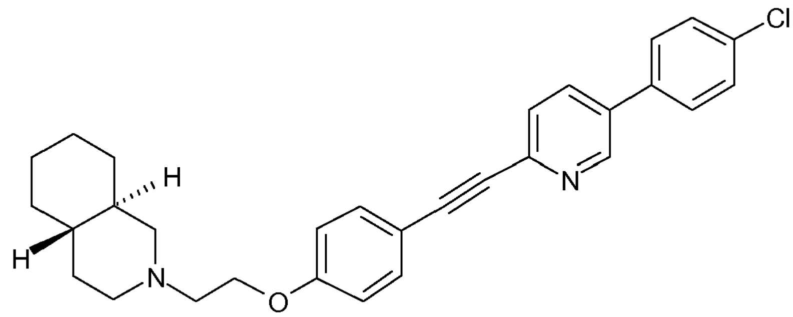

trans-2-(2-{4-[5-(4-Chlor-phenyl)-pyridin-2-ylethinyl]-phenoxyl-ethyl)-decahydro-isochinolin einschließlich deren Tautomere, deren Diastereomere, deren Enantiomere, deren Gemische und deren Salze.

Im folgenden werden Begriffe, die zuvor und nachfolgend zur Beschreibung der erfindungsgemäßen Verbindungen verwendet werden, näher definiert.

Die Bezeichnung Halogen bezeichnet ein Atom ausgewählt aus der Gruppe bestehend aus F, Cl, Br und 1, insbesondere F, Cl und Br.

Die Bezeichnung $C_1$-$_n$-Alkyl, wobei n einen Wert von 3 bis 8 besitzt, bedeutet eine gesättigte, verzweigte oder unverzweigte Kohlenwasserstoffgruppe mit 1 bis n C-Atomen. Beispiele solcher Gruppen umfassen Methyl, Ethyl, n-Propyl, iso-Propyl, Butyl, iso-Butyl, sec-Butyl, tert-Butyl, n-Pentyl, iso-Pentyl, neo-Pentyl, tert-Pentyl, n-Hexyl, isoHexyl, etc..

Die Bezeichnung $C_{1-n}$-Alkylen, wobei n einen Wert von 1 bis 8 besitzen kann, bedeutet eine gesättigte, verzweigte oder unverzweigte Kohlenwasserstoffbrücke mit 1 bis n C-Atomen. Beispiele solcher Gruppen umfassen Methylen ($-CH_2-$), Ethylen ($-CH_2-CH_2-$), 1-Methyl-ethylen ($-CH(CH_3)-CH_2-$), 1,1-Dimethyl-ethylen ($-C(CH_3)_2-CH_2-$), n-Prop-1,3-ylen ($-CH_2-CH_2-CH_2-$), 1-Methylprop-1,3-ylen ($-CH(CH_3)-CH_2-CH_2-$), 2-Methylprop-1,3-ylen ($-CH_2-CH(CH_3)-CH_2-$), etc., sowie die entsprechenden spiegelbildlichen Formen.

Der Begriff $C_{2-n}$-Alkenyl, wobei n einen Wert von 3 bis 6 besitzt, bezeichnet eine verzweigte oder unverzweigte Kohlenwasserstoffgruppe mit 2 bis n C-Atomen und einer C=C-Doppelbindung. Beispiele solcher Gruppen umfassen Vinyl, 1-Propenyl, 2-Propenyl, iso-Propenyl, 1-Butenyl, 2-Butenyl, 3-Butenyl, 2-Methyl-1-propenyl, 1-Pentenyl, 2-Pentenyl, 3-Pentenyl, 4-Pentenyl, 3-Methyl-2-butenyl, 1-Hexenyl, 2-Hexenyl, 3-Hexenyl, 4-Hexenyl-, 5-Hexenyl etc..

Der Begriff $C_{2-n}$-Alkinyl, wobei n einen Wert von 3 bis 6 besitzt, bezeichnet eine verzweigte oder unverzweigte Kohlenwasserstoffgruppe mit 2 bis n C-Atomen und einer C≡C-Dreifachbindung. Beispiele solcher Gruppen umfassen Ethinyl, 1-Propinyl, 2-Propinyl, iso-Propinyl, 1-Butinyl, 2-Butinyl, 3-Butinyl, 2-Methyl-1-propinyl, 1-Pentinyl, 2-Pentinyl, 3-Pentinyl, 4-Pentinyl, 3-Methyl-2-butinyl, 1-Hexinyl, 2-Hexinyl, 3-Hexinyl, 4-Hexinyl-, 5-Hexinyl etc..

Der Begriff $C_{1-n}$-Alkoxy bezeichnet eine $C_{1-n}$-Alkyl-0-Gruppe, worin $C_1$-$_n$-Alkyl wie oben definiert ist. Beispiele solcher Gruppen umfassen Methoxy, Ethoxy, n-Propoxy, iso-Propoxy, n-Butoxy, iso-Butoxy, sec-Butoxy, tert-Butoxy, n-Pentoxy, iso-Pentoxy, neo-Pentoxy, tert-Pentoxy, n-Hexoxy, iso-Hexoxy etc..

Der Begriff $C_{1-n}$-Alkylthio bezeichnet eine $C_{1-n}$-Alkyl-S-Gruppe, worin $C_1$-$_n$-Alkyl wie oben definiert ist. Beispiele solcher Gruppen umfassen Methylthio, Ethylthio, n-Propylthio, iso-Propylthio, n-Butylthio, iso-Butylthio, sec-Butylthio, tert-Butylthio, n-Pentylthio, iso-Pentylthio, neo-Pentylthio, tert-Pentylthio, n-Hexylthio, iso-Hexylthio, etc..

Der Begriff $C_{1-n}$-Alkylcarbonyl bezeichnet eine $C_{1-n}$-Alkyl-C(=0)-Gruppe, worin $C_1$-$_n$-Alkyl wie oben definiert ist. Beispiele solcher Gruppen umfassen Methylcarbonyl, Ethylcarbonyl, n-Propylcarbonyl, iso-Propylcarbonyl, n-Butylcarbonyl, iso-Butylcarbonyl, sec-Butylcarbonyl, tert-Butylcarbonyl, n-Pentylcarbonyl, iso-Pentylcarbonyl, neo-Pentylcarbonyl, tert-Pentylcarbonyl, n-Hexylcarbonyl, iso-Hexylcarbonyl, etc..

Der Begriff $C_{3-n}$-Cycloalkyl bezeichnet eine gesättigte mono-, bi-, tri- oder spirocarbocyclische Gruppe mit 3 bis n C-Atomen. Beispiele solcher Gruppen umfassen Cyclopropyl, Cyclobutyl, Cyclopentyl, Cyclohexyl, Cycloheptyl, Cyclooctyl, Cyclononyl, Cyclododecyl, Bicyclo[3.2.1.]octyl, Spiro[4.5]decyl, Norpinyl, Norbonyl, Norcaryl, Adamantyl, etc.. Vorzugsweise umfasst der Begriff $C_{3-7}$-Cycloalkyl gesättigte monocyclische Gruppen.

Der Begriff $C_{5-n}$-Cycloalkenyl bezeichnet eine einfach ungesättigte mono-, bi-, tri- oder spirocarbocyclische Gruppe mit 5 bis n C-Atomen. Beispiele solcher Gruppen umfassen Cyclopentenyl, Cyclohexenyl, Cycloheptenyl, Cyclooctenyl, Cyclononenyl, etc..

Der Begriff $C_{3-n}$-Cycloalkylcarbonylbezeichnet eine $C_{3-n}$-Cycloalkyl-C(=0)-Gruppe, worin $C_{3-n}$-Cycloalkyl wie oben definiert ist.

Der Begriff Aryl bezeichnet ein carbocyclisches, aromatisches Ringsystem, wie beispielsweise Phenyl, Biphenyl, Naphthyl, Anthracenyl, Phenanthrenyl, Fluorenyl, Indenyl, Pentalenyl, Azulenyl, Biphenylenyl, etc.. Eine besonders

bevorzugte Bedeutung von "Aryl" ist Phenyl

Der Begriff Cyclo-$C_{3-7}$-alkylenimino-bezeichnet einen 4- bis 7-gliedrigen Ring, der 3 bis 7 Methylen-Einheiten sowie eine Imino-Gruppe aufweist, wobei die Bindung zum Rest des Moleküls über die Imino-Gruppe erfolgt.

Der Begriff Cyclo-$C_{3-7}$-alkylenimino-carbonyl bezeichnet einen zuvor definierten Cyclo-$C_{3-7}$-alkylenimino-Ring, der über die Imino-Gruppe mit einer Carbonyl-Gruppe verbunden ist.

Der in dieser Anmeldung verwendete Begriff Heteroaryl bezeichnet ein heterocyclisches, aromatisches Ringsystem, das neben mindestens einem C-Atom ein oder mehrere Heteroatome ausgewählt aus N, 0 und/oder S umfasst. Beispiele solcher Gruppen sind Furanyl, Thiophenyl, Pyrrolyl, Oxazolyl, Thiazolyl, Imidazolyl, Isoxazolyl, Isothiazolyl, 1,2,3-Triazolyl, 1,3,5-Triazolyl, Pyranyl, Pyridyl, Pyridazinyl, Pyrimidinyl, Pyrazinyl, 1,2,3-Triazinyl, 1,2,4-Triazinyl, 1,3,5-Triazinyl, 1,2,3-Oxadiazolyl, 1,2,4-Oxadiazolyl, 1,2,5-Oxadiazolyl, 1,3,4-Oxadiazolyl, 1,2,3-Thiadiazolyl, 1,2,4-Thiadiazolyl, 1,2,5-Thiadiazolyl, 1,3,4-Thiadiazolyl, Tetrazolyl, Thiadiazinyl, Indolyl, Isoindolyl, Benzfuranyl, Benzthiophenyl (Thianaphthenyl), Indazolyl, Benzimidazolyl, Benzthiazolyl, Benzisothiazolyl, Benzoxazolyl, Benzisoxazolyl, Purinyl, Chinazolinyl, Chinozilinyl, Chinolinyl, Isochinolinyl, Chinoxalinyl, Naphthyridinyl, Pteridinyl, Carbazolyl, Azepinyl, Diazepinyl, Acridinyl, etc.. Der Begriff Heteroaryl umfasst auch die partiell hydrierten heterocyclischen, aromatischen Ringsysteme, insbesondere die oben aufgezählt sind. Beispiele solcher partiell hydrierten Ringsysteme sind 2,3-Dihydrobenzfuranyl, Pyrolinyl, Pyrazolinyl, Indolinyl, Oxazolidinyl, Oxazolinyl, Oxazepinyl, etc.. Besonders bevorzugt bedeutet Heteroaryl ein heteroaromatisches mono- oder bicyclisches Ringsystem.

Begriffe, wie Aryl-$C_{1-n}$-alkyl, Heteroaryl-$C_{1-n}$-alkyl, etc. bezeichnen $C_{1-n}$-Alkyl, wie oben definiert, das mit einer Aryl- oder Heteroaryl-Gruppe substituiert ist.

Manche der zuvor angeführten Begriffe können mehrfach in der Definition einer Formel oder Gruppe verwendet werden und besitzen jeweils unabhängig voneinander eine der angegebenen Bedeutungen.

Der Begriff "ungesättigte carbocyclische Gruppe" oder "ungesättigte heterocyclische Gruppe", wie er insbesondere in der Definition der Gruppe Cy verwendet wird, umfasst neben den vollständig ungesättigten Gruppen auch die entsprechenden, lediglich teilweise ungesättigten Gruppen, insbesondere ein- und zweifach ungesättigte Gruppen.

Der in dieser Anmeldung verwendete Begriff "gegebenenfalls substituiert" bedeutet, dass die so bezeichnete Gruppe entweder unsubstituiert oder ein- oder mehrfach mit den angegebenen Substituenten substituiert ist. Falls die betreffende Gruppe mehrfach substituiert ist, so können die Substituenten gleich oder verschieden sein.

Das H-Atom einer vorhandenen Carboxygruppe oder ein an ein N-Atom gebundenes H-Atom (Imino- oder Amino-Gruppe) kann jeweils durch einen in-vivo abspaltbaren Rest ersetzt sein. Unter einem von einem N-Atom in-vivo abspaltbaren Rest versteht man beispielsweise eine Hydroxygruppe, eine Acylgruppe wie die Benzoyl- oder Pyridinoylgruppe oder eine $C_{1-16}$-Alkanoylgruppe wie die Formyl-, Acetyl-, Propionyl-, Butanoyl-, Pentanoyl- oder Hexanoylgruppe, eine Allyloxycarbonylgruppe, eine $C_{1-16}$-Alkoxycarbonylgruppe wie die Methoxycarbonyl-, Ethoxycarbonyl-, Propoxycarbonyl-, Isopropoxycarbonyl-, Butoxycarbonyl-, tert.Butoxycarbonyl-, Pentoxycarbonyl-, Hexyloxycarbonyl-, Octyloxycarbonyl-, Nonyloxycarbonyl-, Decyloxycarbonyl-, Undecyloxycarbonyl-, Dodecyloxycarbonyl- oder Hexadecyloxycarbonylgruppe, eine Phenyl-$C_{1-6}$-alkoxycarbonylgruppe wie die Benzyloxycarbonyl-, Phenylethoxycarbonyl- oder Phenylpropoxycarbonylgruppe, eine $C_{1-3}$-Alkylsulfonyl-$C_{2-4}$-alkoxycarbonyl-, $C_{1-3}$-Alkoxy-$C_{2-4}$-alkoxy-$C_{2-4}$-alkoxycarbonyl- oder $R_eCO$-O-($R_fCRg$)-O-CO-Gruppe, in der

$R_e$ eine $C_{1-8}$-Alkyl-,$C_{5-7}$-$C_{YC}$loalkyl-, Phenyl- oder Phenyl- $C_{1-3}$-alkylgruppe,

$R_f$ ein Wasserstoffatom, eine $C_{1-3}$-Alkyl-,$C_{5-7}$-Cycloalkyl- oder Phenylgruppe und

Rg ein Wasserstoffatom, eine $C_{1-3}$-Alkyl- oder $R_eCO$-O-($R_fCR_g$)-O-Gruppe, in der $R_e$ bis Rg wie vorstehend erwähnt definiert sind, darstellen,

wobei zusätzlich für eine Aminogruppe die Phthalimidogruppe in Betracht kommt, wobei die vorstehend erwähnten Esterreste ebenfalls alsin-vivo in eine Carboxygruppe überführbare Gruppe verwendet werden können.

Die zuvor beschriebenen Reste und Substituenten können in der beschriebenen Weise ein- oder mehrfach mit Fluor substituiert sein. Bevorzugte fluorierte Alkylreste sind Fluormethyl, Difluormethyl und Trifluormethyl. Bevorzugte fluorierte Alkoxyreste sind Fluormethoxy, Difluormethoxy und Trifluormethoxy. Bevorzugte fluorierte Alkylsulfinyl- und Alkylsulfonylgruppen sind Trifluormethylsulfinyl und Trifluormethylsulfonyl.

Die erfindungsgemäßen Verbindungen der allgemeinen Formel 1 können Säuregruppen besitzen, hauptsächlich Carboxylgruppen, und/oder basische Gruppen wie z.B. Aminofunktionen. Verbindungen der allgemeinen Formel I können deshalb als innere Salze, als Salze mit pharmazeutisch verwendbaren anorganischen Säuren wie Salzsäure, Schwefelsäure, Phosphorsäure, Sulfonsäure oder organischen Säuren (wie beispielsweise Maleinsäure, Fumarsäure, Zitronensäure, Weinsäure oder Essigsäure) oder als Salze mit pharmazeutisch verwendbaren Basen wie Alkali- oder Erdalkalimetallhydroxiden oder Carbonaten, Zink- oder Ammoniumhydroxiden oder organischen Aminen wie z.B. Diethylamin, Triethylamin, Triethanolamin u.a. vorliegen.

Die erfindungsgemäßen Verbindungen sind unter Anwendung im Prinzip bekannter Syntheseverfahren erhältlich. Bevorzugt werden die Verbindungen nach den nachfolgend näher erläuterten erfindungsgemäßen Herstellungsverfahren erhalten.

In den beiden nachfolgenden Reaktionsschemata A und B wird die Synthese der erfindungsgemäßen Verbindungen A.5 und B.5 dargestellt, wobei $R^1$, $R^2$, X, Y, Z, W, A und B eine der zuvor beschriebenen Bedeutungen aufweisen. Im Reaktionsschema A besitzt die Gruppe Y die Bedeutung einer Aryl- oder Heteroaryl-Gruppe, während im Reaktionsschema B die Gruppe A eine Aryl- oder Heteroaryl-Gruppe bedeutet. Hal bedeutet Chlor, Brom oder lod, insbesondere Brom oder lod, besonders bevorzugt lod.

Gemäß Reaktionsschema A wird die Halogenverbindung A.1 mit der Alkinverbindung A.2 in einem molaren Verhältnis von etwa 1,5 : 1 bis 1 : 1,5 unter Schutzgasatmosphäre in Gegenwart eines geeigneten Palladium-Katalysators, einer geeigneten Base und Kupfer(I)iodid in einem geeigneten Lösungsmittel umgesetzt.

Eine hierbei bevorzugte Menge an Kupfer(I)iodid liegt im Bereich von 1 bis 15 mol%, insbesondere von 5 bis 10 mol% bezogen auf das Edukt A.1.

Geeignete Palladium-Katalysatoren sind beispielsweise $Pd(PPh_3)_4$, $Pd_2(dba)_3$, $Pd(OAc)_2$, $Pd(PPh_3)_2Cl_2$, Pd $(CH_3CN)_2Cl_2$, $Pd(d_{PP}f)Cl_2$. Der Palladium-Katalysator wird vorzugsweise in einer Menge von 1 bis 15 mol%, insbesondere 5 bis 10 mol% bezogen auf das Edukt A.1 eingesetzt.

Geeignete Basen sind insbesondere Amine, wie beispielsweise Triethylamin oder Ethyldiisopropylamin, sowie $C_{S2}C0_3$. Die Base wird vorzugsweise mindestens in equimolarer Menge bezogen auf das Edukt A.1, im Überschuss oder auch als Lösungsmittel eingesetzt. Des weiteren sind geeignete Lösungsmittel Dimethylformamid oder Ether, wie beispielsweise Tetrahydrofuran, einschließlich deren Gemische. Die Umsetzung erfolgt in einem Zeitraum von etwa 2 bis 24 Stunden in einem Temperaturbereich von etwa 20 bis 90˚C.

Die erhaltene Alkinverbindung A.3 wird direkt oder nach vorheriger Aufreinigung mit Methansulfonsäurechlorid zum Methansulfonat-Derivat A.4 umgesetzt. Die hierbei einzuhaltenden Reaktionsbedingungen sind dem Fachmann als solche bekannt. Vorteilhafte Lösungsmittel sind halogenierte Kohlenwasserstoffe, wie beispielsweise Dichlormethan. Geeignete Reaktionstemperaturen liegen üblicherweise in einem Bereich von 0 bis 30˚C.

Die das Methansulfonat-Derivat A.4 enthaltende Reaktionslösung oder das aufgereinigte Methansulfonat-Derviat A.4, gelöst in einem geeigneten Lösungsmittel, wird mit einem Amin H-$NR^1R^2$zu dem Endprodukt A.5 umgesetzt und anschließend gegebenenfalls aufgereinigt. Besitzt das Amin H-$NR^1R^2$eine weitere primäre oder sekundäre Aminfunktion, so wird diese vorteilhaft vorher mit einer Schutzgruppe versehen, die nach beendeter Reaktion unter Verwendung literaturbekannter Verfahren wieder abgespalten werden kann. Das so erhaltene Produkt kann beispielsweise durch Umsetzung mit einer entsprechenden Säure in die Salzform überführt werden. Ein hierbei bevorzugtes molares Verhältnis des Derivats A.4 zur Aminverbindung liegt im Bereich von 1,5 : 1 bis 1 : 1,5. Geeignete Lösungsmittel sind Dimethylformamid oder Ether, wie beispielsweise Tetrahydrofuran, einschließlich deren Gemische.

Die Umsetzung zum Produkt A.5 erfolgt vorteilhaft in einem Temperaturbereich von etwa 20 bis 90˚C.

Reaktionsschema A:

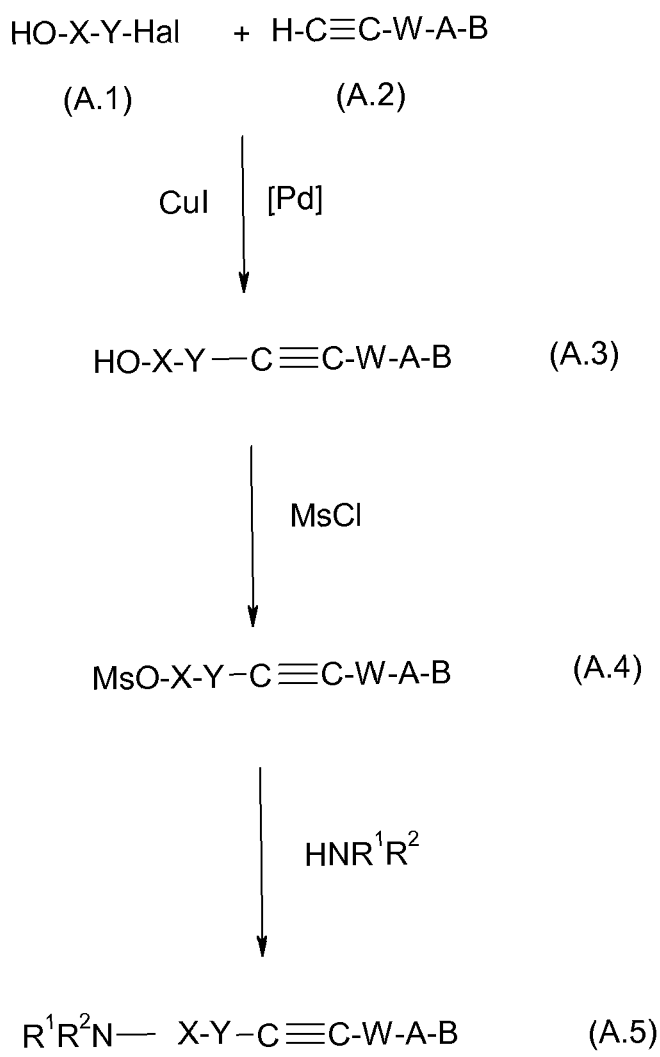


Gemäß Reaktionsschema B wird die Halogenverbindung B.2 mit der Alkinverbindung B.1 in einem molaren Verhältnis von etwa 1,5 : 1 bis 1 : 1,5 unter Schutzgasatmosphäre in Gegenwart eines geeigneten Palladium-Katalysators, einer geeigneten Base und Kupfer(I)iodid in einem geeigneten Lösungsmittel umgesetzt. Angaben zu geeigneten Reaktionsbedingungen, einschließlich Katalysatoren, Basen und Lösungsmitteln, können den Erläuterungen zu Reaktionsschema A entnommen werden.

Die erhaltene Alkinverbindung B.3 wird direkt oder nach vorheriger Aufreinigung mit Methansulfonsäurechlorid zum Methansulfonat-Derivat B.4 umgesetzt. Die hierbei einzuhaltenden Reaktionsbedingungen sind wiederum dem zu Schema A Gesagtem zu entnehmen.

Die das Methansulfonat-Derivat B.4 enthaltende Reaktionslösung oder das aufgereinigte Methansulfonat-Derivat B.4, gelöst in einem geeigneten Lösungsmittel, wird mit einem Amin H-$NR^1R^2$zu dem Endprodukt B.5 umgesetzt und anschließend gegebenenfalls aufgereinigt. Auch hier finden die Ausführungen zu Schema A Anwendung.

Reaktionschema B:

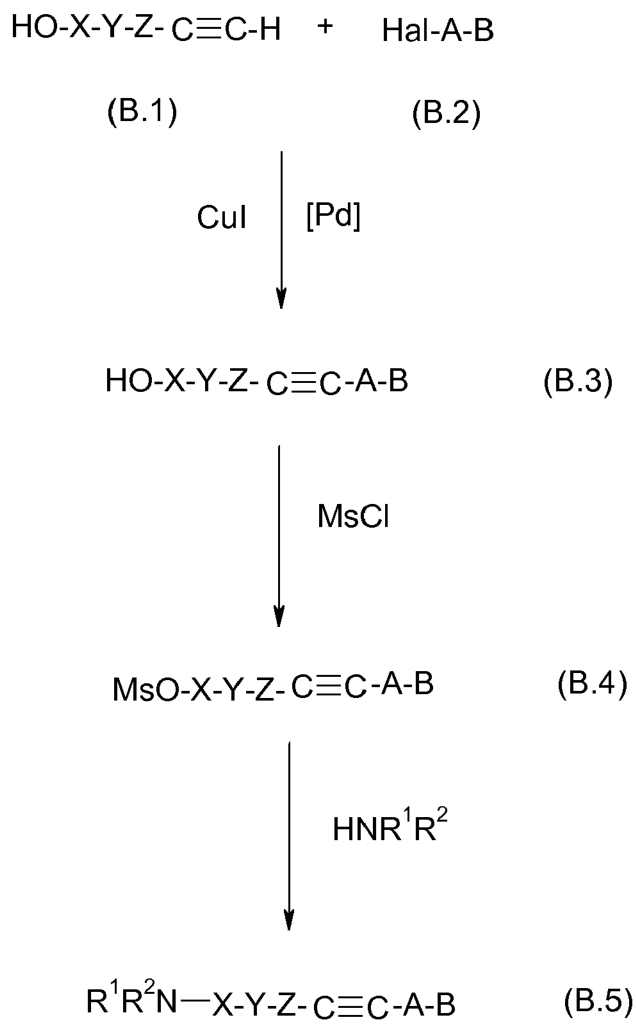


(B.1) (B.2) (B.3) (B.4) (B.5)

Gemäß dem weiteren Reaktionsschema C wird die Halogenverbindung C.1 mit der Alkinverbindung C.2 in einem molaren Verhältnis von etwa 1,5 : 1 bis 1 : 1,5 unter Schutzgasatmosphäre in Gegenwart eines geeigneten Palladium-Katalysators, einer geeigneten Base und Kupfer(I)iodidin einem geeigneten Lösungsmittel unmittelbar zu dem Produkt C.3 umgesetzt. Angaben zu geeigneten Reaktionsbedingungen, einschließlich Katalysatoren, Basen und Lösungsmitteln, können den Erläuterungen zu Reaktionsschema A entnommen werden.

Reaktionsschema C:

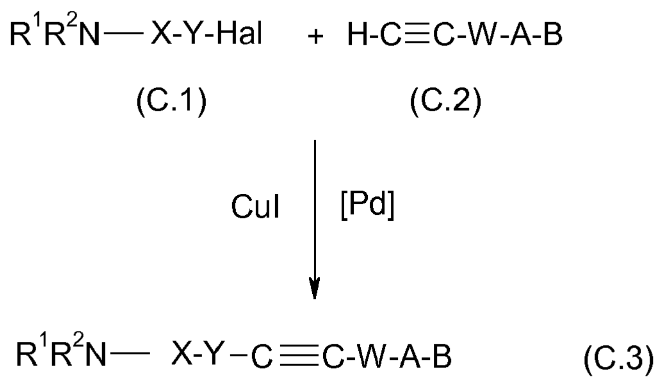


Eine hierzu alternative Synthese ist in dem Reaktionsschema D dargestellt. Hiernach wird die Halogenverbindung D.2 mit der Alkinverbindung D.1 in einem molaren Verhältnis von etwa 1,5 : 1 bis 1 : 1,5 unter Schutzgasatmosphäre in Gegenwart eines geeigneten Palladium-Katalysators, einer geeigneten Base und Kupfer(I)iodid in einem geeigneten Lösungsmittel unmittelbar zu dem Produkt D.3 umgesetzt. Auch hier sind die Angaben zu geeigneten Reaktionsbedingungen, einschließlich Katalysatoren, Basen und Lösungsmitteln, den Erläuterungen zu Reaktionsschema A zu entnehmen.

Reaktionsschema D:

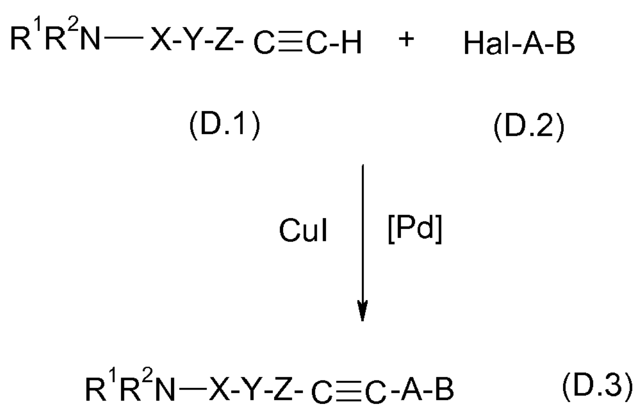


Die Umsetzungen gemäß der Schemata A, B, C und D sind besonders vorteilhaft mit den entspechenden Iod-Verbindungen A.1, B.2, C.1 bzw. D.2 durchzuführen. Für den Fall, dass Hal in den Verbindungen A.1, B.2, C.1 bzw. D. 2 Brom bedeutet, ist es vorteilhaft diese zuvor in die entsprechende Iodverbindung zu überführen. Ein hierbei besonders vorteilhaftes Verfahren ist die Aryl-Finkelstein-Reaktion (Klapars, Artis; Buchwald, Stephen L.. Copper-Catalyzed Halogen Exchange in Aryl Halides: An Aromatic Finkelstein Reaction. Journal of the American Chemical Society (2002), 124(50), 14844-14845). So kann beispielsweise die Halogenverbindung A.1, B.2, C.1 bzw. D.2 mit Natriumiodid in Gegenwart von *N,N'*-Dimethyi-ethylendiamin und Kupfer(I)iodid in einem geeigneten Lösungsmittel zur entsprechenden Iodverbindung umgesetzt werden. Ein hierbei vorteilhaftes molaren Verhältnis der Halogenverbindung zu Natriumiodid ist 1 : 1,8 bis 1 : 2,3. *N,N'*-Dimethyl-ethylendiamin wird vorteilhaft in einem molaren Verhältnis von 10 bis 30 mol% bezogen auf die Halogenverbindung A.1, B.2, C.1 bzw. D.2 eingesetzt. Bevorzugte Mengen an Kupfer(I)iodid liegen im Bereich von 5 bis 20 mol% bezogen auf die Halogenverbindung A.1, B.2, C.1 bzw. D.2. Ein hiebei geeignetes Lösungsmittel ist beispielsweise 1,4-Dioxan. Geeignete Reaktionstemperaturen liegen im Bereich von etwa 20 bis 110˚C. Die Umsetzung ist nach 2 bis 72 Stunden im wesentlichen beendet.

Die erfindungsgemäßen Verbindungen sind vorteilhaft auch nach den in den nachfolgenden Beispielen beschriebenen Verfahren zugänglich, wobei diese hierzu auch mit dem Fachmann beispielsweise aus der Literatur bekannten Verfahren kombiniert werden können.

Stereoisomere Verbindungen der Formel (I) lassen sich prinzipiell nach üblichen Methoden trennen. Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel (I)fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (—)-Weinsäure,(+)- oder (—+)-Diacetylweinsäure, (+)- oder (—)-Monomethyltartrat oder (+)-Camphersulfonsäure, bzw. einer optisch aktiven Base, beispielsweise mit (*R*)-(+)-1-Phenylethylamin, (*S*)-(—)-1-Phenylethylamin oder (S)-Brucin, entstehen.

Nach einem üblichen Verfahren zur Isomerentrennung wird das Racemat einer Verbindung der allgemeinen Formel (I) mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (R)- oder (S)-Enantiomer bzw. ein Gemisch zweier optisch aktiver, unter die allgemeine Formel (I) fallender diastereomerer Verbindungen wird auch dadurch erhalten, dass man die oben beschriebenen Synthesen mit jeweils einer geeigneten (R)- bzw. (S)-konfigurierten Reaktionskomponente durchführt.

Wie vorstehend genannt, können die Verbindungen der Formel (I) in ihre Salze, insbesondere für die pharmazeutische Anwendung, in ihre physiologisch und pharmakologisch verträglichen Salze überführt werden. Diese Salze können einerseits als physiologisch und pharmakologisch verträgliche Säureadditionssalze der Verbindungen der Formel (I) mit anorganischen oder organischen Säuren vorliegen. Andererseits kann die Verbindung der Formel (I) im Falle von acidisch gebundenem Wasserstoff durch Umsetzung mit anorganischen Basen auch in physiologisch und pharmakologisch verträgliche Salze mit Alkali- oder Erdalkalimetallkationen als Gegenion überführt werden. Zur Darstellung der Säureadditionssalze kommen beispielsweise Salzsäure, Bromwasserstoffsäure, Schwefelsäure, Phosphorsäure, Methansulfonsäure, Ethansulfonsäure, Toluolsulfonsäure, Benzolsulfonsäure, Essigsäure, Fumarsäure, Bernsteinsäure, Milchsäure, Zitronensäure, Weinsäure oder Maleinsäure in Betracht. Ferner können Mischungen der vorgenannten Säuren eingesetzt werden. Zur Darstellung der Alkali- und Erdalkalimetallsalze der Verbindung der Formel (I) mit acidisch gebundenem Wasserstoff kommen vorzugsweise die Alkali- und Erdalkalihydroxide und -hydride in Betracht, wobei die Hydroxide und Hydride der Alkalimetalle, insbesondere des Natriums und Kaliums bevorzugt, Natrium- und Kaliumhydroxid besonders bevorzugt sind.

Die Verbindungen gemäß der vorliegenden Erfindung, einschließlich der physiologisch verträglichen Salze, besitzen eine Wirkung als Antagonisten des MCH-Rezeptors, insbesondere des MCH-1 Rezeptors, und zeigen gute Affinitäten in MCH-Rezeptorbindungsstudien. Pharmakologische Testsysteme für MCH-antagonistische Eigenschaften werden im nachfolgenden experimentellen Teil beschrieben.

Als Antagonisten des MCH-Rezeptors sind die erfindungsgemäßen Verbindungen vorteilhaft als pharmazeutische Wirkstoffe zur Prophylaxe und/oder Behandlung von Erscheinungen und/oder Krankheiten geeignet, die durch MCH verursacht werden oder mit MCH in einem anderen kausalen Zusammenhang stehen. Generell weisen die erfindungsgemäßen Verbindungen eine geringe Toxizität, eine gute orale Absorbierbarkeit und intracerebrale Transitivität, insbesondere Hirngängigkeit, auf.

Daher sind MCH-Antagonisten, die mindestens eine erfindungsgemäße Verbindung aufweisen, besonders bei Säugetieren, wie beispielsweise Ratten, Mäusen, Meerschweinchen, Hasen, Hunden, Katzen, Schafen, Pferden, Schweinen, Rindern, Affen sowie Menschen, zur Behandlung und/oder Prophylaxe von Erscheinungen und/oder Krankheiten, die durch MCH verursacht werden oder mit MCH in einem anderen kausalen Zusammenhang stehen, geeignet.

Krankheiten, die durch MCH verursacht werden oder mit MCH in einem anderen kausalen Zusammenhang stehen, sind insbesondere metabolische Störungen, wie beispielsweise Obesitas, und Essstörungen, wie beispielsweise Bulimie, einschließlich

Bulimie nervosa. Die Indikation Obesitas umfasst vorallem exogener Obesitas, hyperinsulinärer Obesitas, hyperplasmischer Obesitas, hyperphysealer Adipositas, hypoplasmischer Obesitas, hypothyroider Obesitas, hypothalamischer Obesitas, symptomatischer Obesitas, infantiler Obesitas, Oberkörperobesitas, alimentärer Obesitas, hypogonadaler Obesitas, zentraler Obesitas. Des weiteren sind in diesem Indikationsumfeld auch Cachexia, Anorexie und Hyperphagia zu nennen.

Erfindungsgemäße Verbindungen können insbesondere geeignet sein, den Hunger zu reduzieren, Appetit zu zügeln, das Essverhalten zu kontrollieren und/oder ein Sättigungsgefühl hervorzurufen.

Darüber hinaus können zu den Krankheiten, die durch MCH verursacht werden oder mit MCH in einem anderen kausalen Zusammenhang stehen, auch Hyperlipidämie, Cellulitis, Fettakkumulation, maligne Mastocytose, systemische Mastocytose, emotionale Störungen, Affektivitätsstörungen, Depressionen, Angstzuständen, Schlafstörungen, Fortpflanzungsstörungen, sexuellen Störungen, Gedächtnisstörungen, Epilepsie, Formen der Dementia und hormonelle Störungen geeignet ist.

Erfindungsgemäße Verbindungen sind auch als Wirkstoffe zur Prophylaxe und/oder Behandlung weiterer Krankheiten und/oder Störungen, insbesondere solcher die mit Obesitas einhergehen, wie beispielsweise von Diabetes, Diabetes mellitus, insbesondere Typ II Diabetes, Hyperglykämie, insbesondere chronischer Hyperglykämie, diabetischen Komplikationen, einschließlich diabetischer Retinopathie, diabetischer Neuropathie, diabetischer Nephropathie, etc., Insulin-Resistenz, pathologischer Glukosetoleranz, Encephalorrhagie, Herzinsuffizienz, Herzkreislauferkrankungen, insbesondere Arteriosklerose und Bluthochdruck, Arthritis und Gonitis geeignet ist.

Erfindungsgemäße MCH Antagonisten und Formulierungen können vorteilhaft in Kombination mit einer alimentären Therapie, wie beispielsweise einer alimentären Diabetes-Therapie, und Übung eingesetzt werden.

Ein weiteres Indikationsgebiet, für das die erfindungsgemäßen Verbindungen vorteilhaft geeignet sind, ist die Prophylaxe und/oder Behandlung von Miktionsstörungen, wie beispielsweise Harninkontinenz, überaktiver Harnblase, Harndrang, Nykturie, Enuresis, wobei die überaktive Blase und der Harndrang mit oder nicht mit benigner Prostatahyperplasie in Verbindung zu stehen brauchen.

Die zur Erzielung einer entsprechenden Wirkung erforderliche Dosierung beträgt zweckmäßigerweise bei intravenöser oder subcutaner Gabe 0,001 bis 30 mg/kg Körpergewicht, vorzugsweise 0,01 bis 5 mg/kg Körpergewicht, und bei oraler, nasaler oder inhalativer Gabe 0,01 bis 50 mg/kg Körpergewicht, vorzugsweise 0,1 bis 30 mg/kg Körpergewicht, jeweils einmal bis dreimal täglich.

Hierzu lassen sich die erfindungsgemäß hergestellten Verbindungen der allgemeinen Formel I, gegebenenfalls in Kombination mit anderen Wirksubstanzen, wie sie nachfolgend näher beschrieben werden, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Oblaten, Pulver, Granulate, Lösungen, Emulsionen, Sirupe, Inhalationsaerosole, Salben, Suppositorien einarbeiten.

Neben Arzneimitteln umfasst die Erfindung auch Zusammensetzungen, enthaltend mindestens eine erfindungsgemäße Alkin-Verbindung und/ oder ein erfindungsgemäßes Salz neben gegebenenfalls einem oder mehreren physiologisch verträglichen Hilfsstoffen. Solche Zusammensetzungen können beispielsweise auch Lebensmittel, die fest oder flüssig sein können, sein, in die die erfindungsgemäße Verbindung eingearbeitet ist.

Für die oben erwähnten Kombinationen kommen als weitere Wirksubstanzen insbesondere solche in Betracht, die beispielsweise die therapeutische Wirksamkeit eines erfindungsgemäßen MCH-Antagonisten im Hinblick auf eine der genannten Indikationen verstärken und/oder die eine Reduzierung der Dosierung eines erfindungsgemäßen MCH-Antagonisten erlauben. Vorzugsweise sind ein oder mehrere weiteren Wirksubstanzen ausgewählt aus der Gruppe bestehend aus

- Wirkstoffe zur Behandlung von Diabetes,
- Wirkstoffe zur Behandlung diabetischer Komplikationen,
- Wirkstoffe zur Behandlung von Obesitas, vorzugsweise andere als MCH-Antagonisten,
- Wirkstoffe zur Behandlung von Bluthochdruck,
- Wirkstoffe zur Behandlung von Hyperlipidemia, einschließlich Arteriosklerose,
- Wirkstoffe zur Behandlung von Arthritis,
- Wirkstoffe zur Behandlung von Angstzuständen,
- Wirkstoffe zur Behandlung von Depressionen.

Nachfolgend werden die zuvor genannten Wirkstoffklassen anhand von Beispielen näher erläutert.

Beispiele von Wirkstoffen zur Behandlung von Diabetes sind Insulin Sensibilisatoren, Insulin Sekretionsbeschleuniger, Biguanide, Insuline, α-Glucosidase Inhibitoren, β3 Adreno Rezeptor Agonisten.

Insulin Sensibilisatoren umfassen Pioglitazone und seine Salze (vorzugsweise Hydrochloride), Troglitazone, Rosiglitazone und seine Salze (vorzugsweise Maleate), JTT-501, GI-262570, MCC-555, YM-440, DRF-2593, BM-13-1258, KRP-297, R-119702, GW-1929.

Insulin Sekretionsbeschleuniger umfassen Sulfonylharnstoffe, wie beispielsweise Tolbutamide, Chlorpropamide, Tolzamide, Acetohexamide, Glyclopyramide und seine Ammonium-Salze, Glibenclamide, Gliclazide, Glimepiride. Weitere Beispiele von Insulin Sektretionsbeschleunigern sind Repaglinide, Nateglinide, Mitiglinide (KAD-1229), JTT-608.

Biguanide umfassen Metformin, Buformin, Phenformin. Insuline umfassen aus Tieren, insbesondere Rindern oder Schweinen, gewonnene Insuline, halbsynthetische Human-Insuline, die enzymatisch aus tierisch gewonnenem Insulin synthetisiert werden, Human-Insulin, das gentechnologisch, beispielsweise aus Escherichia coli oder Hefen, erhalten wird. Ferner wird als Insulin Insulin-Zink (enthaltend 0,45 bis 0,9 Gewichtsprozent Zink) und Protamin-Insulin-Zink erhältlich aus Zinkchlorid, Protaminsulfat und Insulin, verstanden. Darüber hinaus kann Insulin aus Insulin-Fragmenten oder Derivaten (beispielsweise INS-1, etc.) erhalten werden.

Insulin kann auch unterschiedliche Arten umfassen, beispielsweise bezüglich der Eintrittszeit und Dauer der Wirkung ("ultra immediate action type", "immediate action type", "two phase type", "intermediate type", "prolonged action type", etc.), die in Abhängigkeit vom pathologischen Zustand der Patienten ausgewählt werden.

$\alpha$-Glucosidase Inhibitoren umfassen Acarbose, Voglibose, Miglitol, Emiglitate.

$\beta_3$ Adreno Rezeptor Agonisten umfassen AJ-9677, BMS-196085, SB-226552, AZ40140.

Andere als die zuvor genannten Wirkstoffe zur Behandlung von Diabetes umfassen Ergoset, Pramlintide, Leptin, BAY-27-9955 sowie Glykogen Phosphorylase Inhibitoren, Sorbitol Dehydrogenase Inhibitoren, Protein Tyrosin Phosphatase 1 B Inhibitoren, Dipeptidyl Protease Inhibitoren, Glipizid, Glyburide.

Wirkstoffe zur Behandlung diabetischer Komplikationen umfassen beispielsweise Aldose Reduktase Inhibitoren, Glykations Inhibitoren, Protein Kinase C Inhibitoren.

Aldose Reduktase Inhibitoren sind beispielsweise Tolrestat, Epalrestat, Imirestat, Zenarestat, SNK-860, Zopolrestat, ARI-50i, AS-3201.

Ein Beispiel eines Glykations Inhibitors ist Pimagedine.

Protein Kinase C Inhibitoren sind beispielsweise NGF, LY-333531.

Andere als die zuvor genannten Wirkstoffe zur Behandlung diabetischer Komplikationen umfassen Alprostadil, Thiapride Hydrochlorid, Cilostazol, Mexiletine Hydrochlorid, Ethyl eicosapentate, Memantine, Pimagedine (ALT-711).

Wirkstoffe zur Behandlung von Obesitas, vorzugsweise andere als MCH-Antagonisten, umfassen Lipase Inhibitoren und Anorektika.

Ein bevorzugtes Beispiel eines Lipase Inhibitors ist Orlistat.

Beispiele bevorzugter Anorektika sind Phentermin, Mazindol, Dexfenfluramine, Fluoxetine, Sibutramine, Baiamine, (S)-Sibutramine, SR-141716, NGD-95-1.

Andere als die zuvor genannten Wirkstoffe zur Behandlung von Obesitas umfassen Lipstatin.

Ferner werden für die Zwecke dieser Anmeldung zu der Wirkstoffgruppe der AntiObesitas-Wirkstoffe auch die Anorektika gezählt, wobei die $\beta_3$ Agonisten, thyromimetische Wirkstoffe und NPY Antagonisten hervorzuheben sind. Der Umfang der hierbei als bevorzugte Antiobesitas oder anorektische Wirkstoffe in Frage kommenden Substanzen wird durch folgende weitere Liste beispielhaft angegeben: Phenylpropanolamin, Ephedrin, Pseudoephedrin, Phentermin, ein Cholecystokinin-A (nachfolgend als CCK-A bezeichnet) Agonist, ein Monoamin Wiederaufnahme (reuptake)-Inhibitor (wie beispielsweise Sibutramine), ein sympathomimetischer Wirkstoff, ein serotonerger Wirkstoff (wie beispielsweise Dexfenfluramine oder Fenfluramine), ein Dopamin-Antagonist (wie beispielsweise Bromocriptine), ein Melanocyten-stimulierender Hormonrezeptor Agonist oder Mimetikum, ein Analog zum Melanocyten-stimulierenden Hormon, ein Cannabinoid-Rezeptor Antagonist, ein MCH Antagonist, das OB Protein (nachfolgend als Leptin bezeichnet), ein Leptin Analog, ein Leptin Rezeptor Agonist, ein Galanin Antagonist, ein GI Lipase Inhibitor oder Verminderer (wie beispielsweise Orlistat). Weitere Anorektika umfassen Bombesin Agonisten, Dehydroepiandrosteron oder seine Analoga, Glucocorticoid Rezeptor Agonisten und Antagonisten, Orexin Rezeptor Antagonisten, Urocortin Bindungsprotein Antagonisten, Agonisten des Glukagon ähnlichen Peptid-1 Rezeptors, wie beispielsweise Exendin und ciliäre neurotrophe Faktoren, wie beispielsweise Axokine.

Wirkstoffe zur Behandlung von Bluthochdruck umfassen Inhibitoren des Angiotensin umwandelnden Enzyms, Calcium Antagonisten, Kalium-Kanal Öffner, Angiotensin II Antagonisten.

Inhibitoren des Angiotensin umwandelnden Enzyms umfassen Captopril, Enalapril, Alacepril, Delapril (Hydrochloride), Lisinopril, Imidapril, Benazepril, Cilazapril, Temocapril, Trandolapril, Manidipine (Hydrochloride).

Beispiele von Calcium Antagonisten sind Nifedipine, Amlodipine, Efonidipine, Nicardipine.

Kalium-Kanal Öffner umfassen Levcromakalim, L-27152, AL0671, NIP-121.

Angiotensin II Antagonisten umfassen Telmisartan, Losartan, Candesartan Cilexetil, Valsartan, Irbesartan, CS-866, E4177.

Wirkstoffe zur Behandlung von Hyperlipidemia, einschließlich Arteriosklerose, umfassen HMG-CoA Reduktase Inhibitoren, Fibrat-Verbindungen.

HMG-CoA Reduktase Inhibitoren umfassen Pravastatin, Simvastatin, Lovastatin, Atorvastatin, Fluvastatin, Lipantil, Cerivastatin, Itavastatin, ZD-4522 und deren Salze.

Fibrat-Verbindungen umfassen Bezafibrate, Clinofibrate, Clofibrate, Simfibrate.

Wirkstoffe zur Behandlung von Arthritis umfassen Ibuprofen.

Wirkstoffe zur Behandlung von Angstzuständen umfassen Chlordiazepoxide,

Diazepam, Oxazolam, Medazepam, Cloxazolam, Bromazepam, Lorazepam, Alprazolam, Fludiazepam.

Wirkstoffe zur Behandlung von Depressionen umfassen Fluoxetine, Fluvoxamine, Imipramine, Paroxetine, Sertraline.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung.

In einer weiteren Ausführungsform betrifft die Erfindung auch die Verwendung mindestens einer erfindungsgemäßen Alkin-Verbindung und/ oder eines erfindungsgemäßen Salzes zur Beeinflussung des Essverhaltens eines Säugetiers. Diese Verwendung beruht insbesondere darauf, dass erfindungsgemäße Verbindungen geeignet sein können, den Hunger zu reduzieren, Appetit zu zügeln, das Essverhalten zu kontrollieren und/oder ein Sättigungsgefühl hervorzurufen. Das Essverhalten wird vorteilhaft dahingehend beeinflusst, dass die Nahrungsaufnahme reduziert wird. Daher finden die erfindungsgemäßen Verbindungen vorteilhaft Anwendung zur Reduzierung des Körpergewichts. Eine weitere erfindungsgemäße Verwendung ist das Verhindern einer Zunahme des Körpergewichts, beispielsweise in Menschen, die zuvor Maßnahmen zur Gewichtsreduzierung ergriffen hatten und anschließend an einer Beibehaltung des reduzierten Körpergewichts interessiert sind. Gemäß dieser Ausführungsform handelt es sich vorzugsweise um eine nicht-therapeutische Verwendung. Solch eine nicht-therapeutische Verwendung kann eine kosmetische Anwendung, beispielsweise zur Veränderung der äußeren Erscheinung, oder eine Anwendung zur Verbesserung der Allgemeinbefindens sein. Die erfindungsgemäßen Verbindungen werden vorzugsweise für Säugetiere, insbesondere Menschen, nichttherapeutisch verwendet, die keine diagnostizierten Störungen des Essverhaltens, keine diagnostizierte Obesitas, Bulimie, Diabetes und/ oder keine diagnostizierten Miktionsstörungen, insbesondere Harninkontinenz aufweisen. Bevorzugt sind die erfindungsgemäßen Verbindungen zur nicht-therapeutischen Verwendung für Menschen geeignet, deren Körpergewichtsindex (BMI = body mass index), der als das in Kilogramm gemessene Körpergewicht geteilt durch die Körpergröße (in Metern) im Quadrat definiert ist, unterhalb des Wertes 30, insbesondere unterhalb 25, liegt.

Die nachfolgenden Beispiele sollen die Erfindung näher erläutern:

Vorbemerkungen:

Für hergestellte Verbindungen liegen in der Regel IR-, $^1$H-NMR und/oder Massenspektren vor. Wenn nicht anders angegeben, werden $R_f$-Werte unter Verwendung von DC-Fertigplatten Kieselgel 60 $F_{254}$ (E. Merck, Darmstadt, Artikel-Nr. 1.05714) ohne Kammersättigung bestimmt. Die unter der Bezeichnung Alox ermittelten $R_f$-Werte werden unter Verwendung von DC-Fertigplatten Aluminiumoxid 60 $F_{254}$ (E. Merck, Darmstadt, Artikel-Nr. 1.05713) ohne Kammersättigung bestimmt. Die bei den Fliessmitteln angegebenen Verhältnisse beziehen sich auf Volumeneinheiten der jeweiligen Lösungsmittel. Die angegebenen Volumeneinheiten bei $NH_3$ beziehen sich auf eine konzentrierte Lösung von $NH_3$ in Wasser. Zu chromatographischen Reinigungen wird Kieselgel der Firma Millipore (MATREX™,35-70 my) verwendet. Zu chromatographischen Reinigungen wird Alox (E. Merck, Darmstadt, Aluminiumoxid 90 standardisiert, 63-200 μm, Artikel-Nr: 1.01097.9050) verwendet. Die angegebenen HPLC-Daten werden unter nachstehend angeführten Parametern gemessen:

Analytische Säulen: Zorbax-Säule (Agilent Technologies), SB (Stable Bond) - C18; 3.5 μm;4.6 x 75 mm; Säulentemperatur: 30°C; Fluss: 0.8 mL / min; Injektionsvolumen: 5 μL;Detektion bei 254 nm (Methoden A und B)
Symmetry 300 (Waters), 3.5 μm;4.6 x 75 mm; Säulentemperatur: 30°C; Fluss: 0.8 mL / min; Injektionsvolumen: 5 μL;Detektion bei 254 nm (Methode C)
Methode A: Wasser:Acetonitril:Ameisensäure 9:1:0.01 nach 1:9:0.01 über 9 min
Methode B: Wasser:Acetonitril:Ameisensäure 9:1:0.01 nach 1:9:0.01 über 4 min, dann 6 min 1:9:0.01
Methode C: Wasser:Acetonitril:Ameisensäure 9:1:0.01 nach 1:9:0.01 über 4 min, dann 6 min 1:9:0.01
Präparative Säule: Zorbax-Säule (Agilent Technologies), SB (Stable Bond) - C18; 3.5 μm;30 x 100 mm; Säulentemperatur: Raumtemperatur; Fluss: 30 mL / min; Detektion bei 254 nm.

Bei präparativen HPLC-Reinigungen werden in der Regel die gleichen Gradienten verwendet, die bei der Erhebung der analytischen HPLC-Daten benutzt wurden.

Die Sammlung der Produkte erfolgt massengesteuert, die Produkt enthaltenden Fraktionen werden vereinigt und gefriergetrocknet.

Falls nähere Angaben zur Konfiguration fehlen, bleibt offen, ob es sich um reine Enantiomere handelt oder ob partielle oder gar völlige Racemisierung eingetreten ist.

Vorstehend und nachfolgend werden die folgenden Abkürzungen verwendet:

CDI Carbonyldiimidazol
Cyc Cyclohexan
DCM Dichlormethan
DMF Dimethylformamid
DMSO Dimethylsulfoxid
dppf 1,1 '-bis(diphenylphosphino)ferrocen
EtOAc Essigsäureethylester
EtOH Ethanol
Fp Schmelzpunkt
i.vac. im Vakuum
MeOH Methanol
PE Petrolether
$PPh_3$ Triphenylphosphan
RT Raumtemperatur
TBAF Tetrabutylammoniumfluorid Trihydrat
THF Tetrahydrofuran

**Allgemeine Versuchsvorschrift I (Sonogashira Kupplungen)**

Unter Argonatmospäre werden zu einer Lösung des Aryl- oder Heteroaryliodids oderbromids (1.0 eq) und des Alkins (1.05 eq) in THF oder DMF nacheinander ein geeigneter Palladium-Katalysator (z.B. $Pd(PPh_3)_4$ (5 mol%), Pd $(PPh_3)_2Cl_2$ (5 mol%), $Pd(CH_3CN)Cl_2$ (5 mol%) oder Pd(dppf)$C1_2$ (5 bzw. 10 mol%)), eine geeignete Base (z.B. Cäsiumcarbonat (1.5 eq) oder Triethylamin (1.5 eq.)) und CuI (5 bzw. 10 mol%) gegeben. Die Reaktionslösung wird zwischen 2-24 h bei RT bis 90˚C gerührt, filtriert und das Lösungsmittel i.vac. entfernt. Die weitere Reinigung erfolgt durch Säulenchromatographie oder durch Aufreinigung mittels HPLC-MS.

Allgemeine Versuchsvorschrift II (Brom-Iod Austausch)

Zu einer Lösung des Aryl- oder Heteroarylbromids (1.0 eq.) in 1,4-Dioxan unter Argon werden nacheinander NaI (2.0 eq), N,N'-Dimethyl-ethylendiamin (0.2 eq.) und CuI (0.1 eq.) gegeben. Die Reaktion wird 2-72 h bei RT bis 110˚C gerührt und dann mit $NH_3$ verdünnt. Die wässrige Phase wird mit DCM extrahiert, die organische Phase über $MgS0_4$ getrocknet und das Lösungsmittel i.vac. entfernt. Falls notwendig erfolgt die weitere Reinigung durch Säulenchromatographie.

Beispiel 1

Diethyl-(2-{4-[5-(4-methoxy-phenyl)-pyridin-2-ylethinyl]-phenoxy}-ethyl)-amin

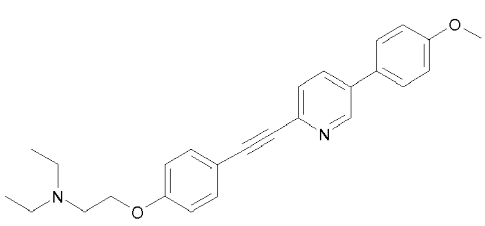

1 a [2-(4-Brom-phenoxy)-ethyl]-diethyl-amin

Eine Suspension von 31.4 g (178 mmol) 4-Bromphenol, 30.6 g (178 mmol) (2-Chlorethyl)-diethyl-amin (eingesetzt als Hydrochlorid) und 61.5 g (445 mmol) $K_2C0_3$ in 300 mL DMF wird 8 h auf 80˚C erhitzt. Das Lösungsmittel wird i.vac. eingeengt, der Rückstand mit Wasser versetzt, die wässrige Phase erschöpfend mit EtOAc extrahiert, die vereinigten organischen Phasen nochmals mit Wasser gewaschen und über $MgS0_4$ getrocknet. Nach Entfernen des Trocken- und Lösungsmittels wird der Rückstand chromatographisch (Kieselgel, EtOAc/Me0H/$NH_3$ 90:10:1) gereinigt.
Ausbeute: 28.0 g (58% d. Theorie)
$C_{12}H_{18}BrN0$ (M= 272,187)

ber.: Molpeak $(M+H)^+$: 272/274 gef.: Molpeak $(M+H)^+$: 272/274
$R_f$-Wert: 0.25 (Kieselgel, EtOAc/Me0H/$NH_3$ 90:10:1)

1 b Diethyl-[2-(4-trimethylsilanylethinyl-phenoxy)-ethyl]-amin

Unter Stickstoffatmosphäre wird eine Mischung aus 5.44 g (20 mmol) [2-(4-Bromphenoxy)-ethyl]-diethyl-amin, 3.11 mL (22 mmol) Ethinyl-trimethyl-silan, 462 mg (0.4 mmol) Tetrakis-triphenylphosphan-Palladium, 76 mg (0.4 mmol) Cul in 50 mL Piperidin 21 h bei 70˚C erwärmt. Das Lösungsmittel wird i.vac. abdestilliert, der Rückstand in Wasser aufgenommen, erschöpfend mit EtOAc extrahiert und über $Na_2S0_4$ getrocknet.
Nach Entfernen des Trocken- und Lösungsmittels wird der Rückstand an Kieselgel
(EtOAc/Me0H/$NH_3$ 95:5:0.5) gereinigt.
Ausbeute: 1.4 g (24% d. Theorie)
$C_{17}H_{27}NOSi$ (M= 289,497)
ber.: Molpeak $(M+H)^+$: 290 gef.: Molpeak $(M+H)^+$: 290
$R_f$-Wert: 0.67 (Kieselgel, EtOAc/Me0H/$NH_3$ 95:5:0.5)

1 c Diethyl-[2-(4-ethinyl-phenoxy)-ethyl]-amin

Unter Stickstoffatmosphäre wird eine Lösung von 1.4 g (4.8 mmol) Diethyl-[2-(4-trimethylsilanylethinyl-phenoxy)-ethyl]-amin in 50 mL THF mit 1.68 g (5.3 mmol) TBAF versetzt und über Nacht bei RT gerührt. Das Lösungsmittel wird i.vac. abdestilliert, der Rückstand in Wasser aufgenommen, erschöpfend mit EtOAc extrahiert und über $Na_2S0_4$ getrocknet. Nach Entfernen des Trocken- und Lösungsmittels wird der Rückstand an Kieselgel (EtOAc/MeOH 95:5) gereinigt.
Ausbeute: 0.5 g (47% d. Theorie)
$C_{14}H19NO$ (M= 217,314)
ber.: Molpeak $(M+H)^+$: 218 gef.: Molpeak $(M+H)^+$: 218
$R_f$-Wert: 0.46 (Kieselgel, EtOAc/Me0H/$NH_3$ 95:5:0.5)

1d {2-[4-(5-Brom-pyridin-2-ylethinyl)-phenoxy]-ethyl}-diethyl-amin

Eine Mischung aus 500 mg (2.30 mmol) Diethyl-[2-(4-ethinyl-phenoxy)-ethyl]-amin, 545 mg (2.30 mmol) 2,5-Dibrompyridin, 161 mg (0.23 mmol) Tetrakis-triphenylphosphan-Palladium, 13 mg (0.07 mmol) Cul, 2 mL Ethyldiisopropylamin und 2 mL Diisopropylamin in 50 mL DMF wird 20 h bei 100˚C unter Stickstoffatmosphäre erwärmt. Das Lösungsmittel wird i.vac. abdestilliert, der Rückstand in Wasser aufgenommen, erschöpfend mit EtOAc extrahiert und über $Na_2S0_4$ getrocknet. Nach Entfernen des Trocken- und Lösungsmittels wird der Rückstand an Kieselgel (EtOAc/Me0H/$NH_3$ 95:5:0.5) gereinigt.
Ausbeute: 200 mg (23% d. Theorie)
$C19H_{21}BrN_20$ (M= 373,296)
ber.: Molpeak $(M+H)^+$: 373/375 gef.: Molpeak $(M+H)^+$: 373/375
$R_f$-Wert: 0.50 (Kieselgel, EtOAc/Me0H/$NH_3$ 90:10:1)

1e Diethyl-(2-{4-[5-(4-methoxy-phenyl)-pyridin-2-ylethinyl]-phenoxyl-ethyl)-amin

Eine Mischung aus 200 mg (0.54 mmol) {2-[4-(5-Brom-pyridin-2-ylethinyl)-phenoxy]-ethyl}-diethyl-amin, 163 mg (1.07 mmol) 4-Methoxy-phenylboronsäure, 31 mg (0.03 mmol) Tetrakis-triphenylphosphan-Palladium und 0.27 mL einer 2 M wässrigen $Na_2C0_3$-Lösung in 5 mL 1,4-Dioxan wird 20 h bei 110˚C unter Stickstoffatmosphäre erwärmt. Das Lösungsmittel wird i.vac. abdestilliert, der Rückstand in Wasser aufgenommen, erschöpfend mit EtOAc extrahiert und über $Na_2S0_4$ getrocknet. Nach Entfernen des Trocken- und Lösungsmittels wird der Rückstand an Kieselgel (EtOAc/Me0H/$NH_3$ 95:5:0.5) gereinigt. Die Produktfraktionen werden eingeengt, der Rückstand mit Diethylether verrieben, abgesaugt und mit Diisopropylether nachgewaschen.
Ausbeute: 30 mg (14% d. Theorie)
$C_{26}H_{28}N_20_2$ (M= 400,525)
ber.: Molpeak $(M+H)^+$: 401 gef.: Molpeak $(M+H)^+$: 401
$R_f$-Wert: 0.46 (Kieselgel, EtOAc/Me0H/$NH_3$ 95:5:0.5)

Beispiel 1.1

Diethyl-(2-{4-[5-(2-methoxy-phenyl)-pyridin-2-ylethinyl]-phenoxy}-ethyl)-amin

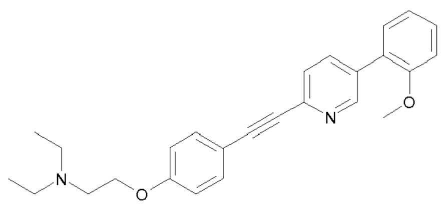

Analog Beispiel 1 e wird aus 200 mg (0.54 mmol) {2-[4-(5-Brom-pyridin-2-ylethinyl)-phenoxy]-ethyll-diethyl-amin und 163 mg (1.07 mmol) 2-Methoxy-phenylboronsäure das Produkt erhalten.
Ausbeute: 40 mg (14% d. Theorie)
$C_{26}H_{28}N_20_2$ (M= 400,525)
ber.: Molpeak $(M+H)^+$: 401 gef.: Molpeak $(M+H)^+$: 401
$R_f$-Wert: 0.23 (Kieselgel, EtOAc/Me0H/$NH_3$ 95:5:0.5)

Beispiel 1.2

(2-{4-[5-(4-Ethoxy-phenyl)-pyrid in-2-ylethinyl]-phenoxyl-ethyl)-diethyl-amin

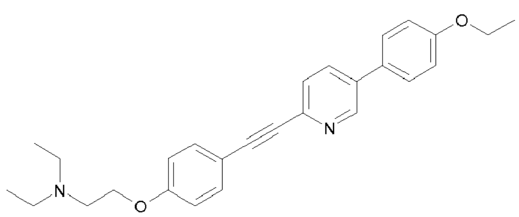

Analog Beispiel 1 e wird aus 200 mg (0.54 mmol) {2-[4-(5-Brom-pyridin-2-ylethinyl)-phenoxy]-ethyll-diethyl-aminund 178 mg (1.07 mmol) 4-Ethoxy-phenylboronsäuredas Produkt erhalten.
Ausbeute: 83 mg (37% d. Theorie)
$C_{27}H_{30}N_2O_2$(M= 414,552)
ber.: Molpeak $(M+H)^+$: 414 gef.: Molpeak $(M+H)^+$: 414
$R_f$-Wert: 0.26 (Kieselgel, EtOAc/Me0H/$NH_3$ 95:5:0.5)

Beispiel 1.3

(2-{4-[5-(3,4-Difluor-phenyl)-pyridin-2-ylethinyl]-phenoxyl-ethyl)-diethyl-amin

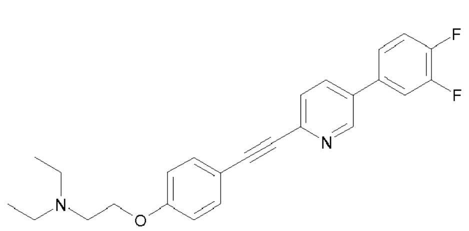

Analog Beispiel 1 e wird aus 200 mg (0.54 mmol) {2-[4-(5-Brom-pyridin-2-ylethinyl)-phenoxy]-ethyl}-dlethyl-amlnund 169 mg (1.07 mmol) 3,4-Difluor-phenylboronsäure das Produkt erhalten.
Ausbeute: 35 mg (16% d. Theorie)
$C_{25}H_{24}F_2N_20$ (M= 406,480)
ber.: Molpeak $(M+H)^+$: 407 gef.: Molpeak $(M+H)^+$: 407
$R_f$-Wert: 0.34 (Kieselgel, EtOAc/Me0H/$NH_3$ 95:5:0.5)

Beispiel 1.4

(2-{4-[5-(4-Chlor-phenyl)-pyrdin-2-ylethunyl]-phenoxy}-ethyl)-diethyl-amin

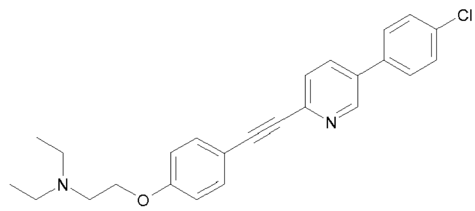

Analog Beispiel 1 e wird aus 200 mg (0.54 mmol) {2-[4-(5-Brom-pyridin-2-ylethinyl)-phenoxy]-ethyll-diethyl-amin und 167 mg (1.07 mmol) 4-Chlor-phenylboronsäure das Produkt erhalten.
Ausbeute: 51 mg (24% d. Theorie)
$C_{25}H_{25}C1N_20$ (M= 404,944)
ber.: Molpeak $(M+H)^+$: 405/407 gef.: Molpeak $(M+H)^+$: 405/407
$R_f$-Wert: 0.26 (Kieselgel, EtOAc/Me0H/$NH_3$ 95:5:0.5)

Beispiel 1.5

Diethyl-(2-(4-[5-(4-methoxy-phenyl)-pyrimidin-2-ylethinyl]-phenoxy)-ethyl)-amin

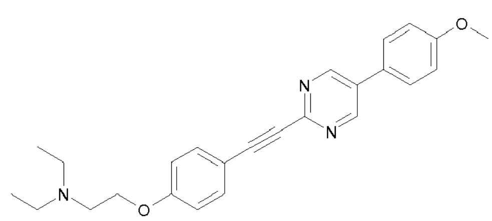

Analog Beispiel 1d wird aus 434 mg (2.0 mmol) Diethyl-[2-(4-ethinyl-phenoxy)-ethyl]-amin und 441 mg (2.0 mmol) 2-Chlor-5-(4-methoxy-phenyl)-pyrimidin das Produkt erhalten.
Ausbeute: 100 mg (13% d. Theorie)
$C_{25}H_{27}N_30_2$ (M= 401,513)
ber.: Molpeak $(M+H)^+$: 402 gef.: Molpeak $(M+H)^+$: 402
$R_f$-Wert: 0.65 (Kieselgel, EtOAc/Me0H/$NH_3$ 90:10:1 )

Beispiel 1.6

5-(4-Chlor-phenyl)-2-[4-(2-pyrrolidin-1-yl-ethoxy)-phenylethinyl]-pyridin

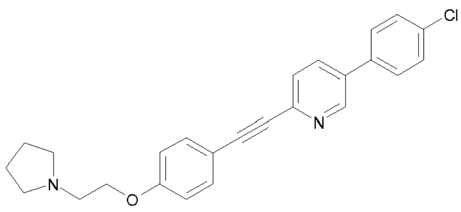

1.6a 1-[2-(4-lod-phenoxy)-ethyl]-pyrrolidin

Eine Suspension von 22 g (100 mmol) 4-lodphenol, 17 g (100 mmol) 1-(2-Chloro-ethyl)-pyrrolidin (eingesetzt als Hydrochlorid) und 55.3 g (400 mmol) $K_2C0_3$ in 400 mL DMF wird 48 h bei RT gerührt. Das Lösungsmittel wird i.vac. eingeengt, der Rückstand mit Wasser versetzt, die wässrige Phase erschöpfend mit EtOAc extrahiert, die vereinigten organischen Phasen mit gesättigter, wässriger NaCl-Lösung gewaschen und über $Na_2S0_4$ getrocknet. Nach Entfernen des Trocken- und Lösungsmittels wird der Rückstand an Kieselgel (EtOAc/Me0H/$NH_3$ 85:15:1.5) gereinigt.
Ausbeute: 18.0 g (57% d. Theorie)
$C_{12}H_{16}1N0$ (M= 317,172)
ber.: Molpeak $(M+H)^+$: 318 gef.: Molpeak $(M+H)^+$: 318
$R_f$-Wert: 0.59 (Kieselgel, EtOAc/Me0H/$NH_3$ 80:20:2)

1.6b 1-[2-(4-Trimethylsilanylethinyl-phenoxy)-ethyl]-pyrrolidin

Unter Stickstoffatmosphäre wird zu einer Mischung aus 14.3 g (45 mmol) 1-[2-(4-lodphenoxy)-ethyl]-pyrrolidin, 1.04 g (0.9 mmol) Tetrakis-triphenylphosphan-Palladium und 171 mg (0.4 mmol) Cul in 140 mL Piperidin langsam 7.0 mL (49.5 mmol) Ethinyl-trimethyl-silan gegeben (exotherme Reaktion) und 30 Minuten nachgerührt. Das Lösungsmittel wird i.vac. abdestilliert, der Rückstand in Wasser aufgenommen, erschöpfend mit EtOAc extrahiert und über $Na_2S0_4$ getrocknet. Nach Entfernen des Trocken- und Lösungsmittels wird der Rückstand an Kieselgel (EtOAc/Me0H/$NH_3$ 95: 5:0.5) gereinigt.
Ausbeute: 12.8 g (99% d. Theorie)
$C_{17}H_{25}NOSi$ (M= 287,481)
ber.: Molpeak $(M+H)^+$: 288 gef.: Molpeak $(M+H)^+$: 288
$R_f$-Wert: 0.42 (Kieselgel, EtOAc/Me0H/$NH_3$ 90:10:1 )

1.6c 1-[2-(4-Ethinyl-phenoxy)-ethyl]-pyrrolidin

Unter Stickstoffatmosphäre wird eine Lösung von 12.8 g (44.5 mmol) 1-[2-(4-Trimethylsilanylethinyl-phenoxy)-ethyl]-pyrrolidin in 200 mL THF mit 15.5 g (49.0 mmol) TBAF versetzt und 3 h bei RT gerührt. Das Lösungsmittel wird i.vac. abdestilliert, der Rückstand in EtOAc aufgenommen, die organische Phase mit gesättigter, wässriger NaCl-Lösung gewaschen und über $Na_2S0_4$ getrocknet. Nach Entfernen des Trocken- und Lösungsmittels wird das Produkt ohne Reinigung weiter umgesetzt.
Ausbeute: 9.6 g (100% d. Theorie)
$C_{14}H_{17}N0$ (M= 215,298)
ber.: Molpeak $(M+H)^+$: 216 gef.: Molpeak $(M+H)^+$: 216
$R_f$-Wert: 0.76 (Kieselgel, EtOAc/Me0H/$NH_3$ 80:20:2)

1.6d 5-Brom-2-[4-(2-pyrrolidin-1-yl-ethoxy)-phenylethinyl]-pyridin

Eine Mischung aus 9.6 g (44.6 mmol) 1-[2-(4-Ethinyl-phenoxy)-ethyl]-pyrrolidin, 10.6 g (44.6 mmol) 2,5-Dibrompyridin, 626 mg (0.9 mmol) Tetrakis-triphenylphosphan-Palladium, 170 mg (0.9 mmol) CuCl und 12.6 mL Diisopropylamin in 500 mL THF wird 3 h bei 40˚C unter Argonatmosphäre erwärmt. Das Lösungsmittel wird i.vac. abdestilliert, der Rückstand in EtOAc aufgenommen, die organische Phase mit Wasser und gesättigter, wässriger NaCl-Lösung gewaschen und über $Na_2S0_4$ getrocknet. Nach Entfernen des Trocken- und Lösungsmittels wird der Rückstand an Kieselgel (EtOAc/Me0H/$NH_3$ 90:10:1 ) gereinigt.
Ausbeute: 8.9 g (54% d. Theorie)
$C_{19}H_{19}BrN_20$ (M= 371,280)
ber.: Molpeak $(M+H)^+$: 371/373 gef.: Molpeak $(M+H)^+$: 371/373

$R_f$-Wert: 0.47 (Kieselgel, EtOAc/Me0H/$NH_3$ 90:10:1 )

1.6e 5-(4-Chlor-phenyl)-2-[4-(2-pyrrolidin-1-yl-ethoxy)-phenylethinyl]-pyridin

Eine Mischung aus 2.97 g (8.0 mmol) 5-Brom-2-[4-(2-pyrrolidin-1-yl-ethoxy)-phenylethinyl]-pyridin, 2.50 g (16.0 mmol) 4-Chlor-phenylboronsäure, 462 mg (0.4 mmol) Tetrakis-triphenylphosphan-Palladium und 8.0 mL einer 2M wässrigen $Na_2C0_3$-Lösung in 100 mL 1,4-Dioxan wird 4 h bei 100˚C unter Argonatmosphäre erwärmt. Das Lösungsmittel wird i.vac. abdestilliert, der Rückstand mit Wasser/EtOAc (1/1,v/v) verrührt, über einen Glasfaserfilter abgesaugt, die organische Phase mit gesättigter, wässriger NaCI-Lösung gewaschen und über $Na_2S0_4$ getrocknet. Nach Entfernen des

Trocken- und Lösungsmittels wird der Rückstand chromatographisch (Alox, Cyc/EtOAc 2:1) gereinigt. Die Produktfraktionen werden eingeengt, der Rückstand mit Diethylether verrieben, abgesaugt und mit Diethylether nachgewaschen.
Ausbeute: 1.95 g (60% d. Theorie)
$C_{25}H_{23}C1N_20$ (M= 402,928)
ber.: Molpeak $(M+H)^+$: 403/405 gef.: Molpeak $(M+H)^+$: 403/405
$R_f$-Wert: 0.47 (Alox, Cyc/EtOAc 2:1)

**Beispiel 1.7**

5-(4-Fluor-phenyl)-2-[4-(2-pyrrolidin-1-yl-ethoxy)-phenylethinyl]-pyridin

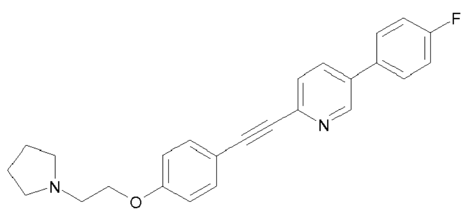

Analog Beispiel 1.6ewird aus 297 mg (0.8 mmol) 5-Brom-2-[4-(2-pyrrolidin-1-yl-ethoxy)-phenylethinyl]-pyridin und 224 mg (1.6 mmol) 4-Fluor-phenylboronsäure das Produkt erhalten.
Ausbeute: 37 mg (12% d. Theorie)
$C_{25}H_{23}FN_20$ (M= 386,473)
ber.: Molpeak $(M+H)^+$: 387 gef.: Molpeak $(M+H)^+$: 387
$R_f$-Wert: 0.41 (Alox, Cyc/EtOAc 2:1)

**Beispiel 1.8**

5-(4-Brom-phenyl)-2-[4-(2-pyrrolidin-1-yl-ethoxy)-phenylethinyl]-pyridin

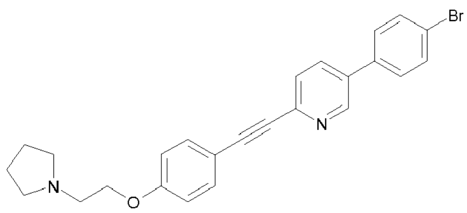

Analog Beispiel 1.6e wird aus 297 mg (0.8 mmol) 5-Brom-2-[4-(2-pyrrolidin-1-yl-ethoxy)-phenylethinyl]-pyridin und 321 mg (1.6 mmol) 4-Brom-phenylboronsäure das Produkt erhalten. Die Reinigung erfolgt über neutralem Alox (Merck Aluminiumoxid 90 standardisiert, 63-200my; Cyc/EtOAc 4:1 ). Das so erhaltene Produkt wird aus EtOH umkristallisiert.

Ausbeute: 40 mg (11 % d. Theorie)
$C_{25}H_{23}BrN_20$ (M= 447,379)
ber.: Molpeak $(M+H)^+$: 447/449 gef.: Molpeak $(M+H)^+$: 447/449
$R_f$-Wert: 0.45 (Alox, Cyc/EtOAc 2:1)

Beispiel 1.9

2-[4-(2-Pyrrolidin-1-yl-ethoxy)-phenylethinyl]-5-(4-trifluormethoxy-phenyl)-pyridin

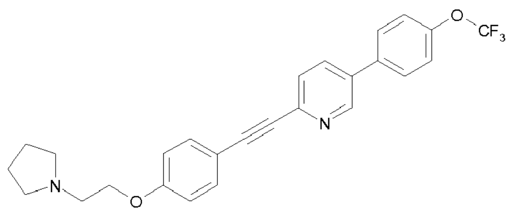

Analog Beispiel 1.6e wird aus 297 mg (0.8 mmol) 5-Brom-2-[4-(2-pyrrolidin-1-yl-ethoxy)-phenylethinyl]-pyridin und 329 mg (1.6 mmol) 4-Trifluormethoxy-phenylboronsäure das Produkt erhalten. Die Reinigung erfolgt über neutralem Alox (Merck Aluminiumoxid 90 standardisiert, 63-200my; Cyc/EtOAc 4:1). Das so erhaltene Produkt wird mit n-Hexan verrührt und abgesaugt.
Ausbeute: 190 mg (53% d. Theorie)
$C_{26}H_{23}F_3N_20_2$ (M= 452,481)
ber.: Molpeak $(M+H)^+$: 453 gef.: Molpeak $(M+H)^+$: 453
$R_f$-Wert: 0.46 (Alox, Cyc/EtOAc 2:1)

Beispiel 1.10

2-[4-(2-Pyrrolidin-1-yl-ethoxy)-phenylethinyl]-5-(4-methoxy-phenyl)-pyridin

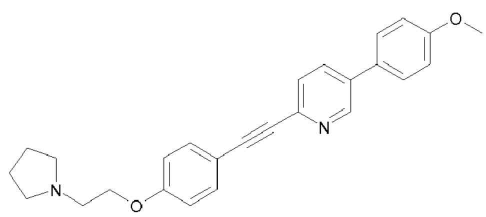

Analog Beispiel 1.9 wird aus 297 mg (0.8 mmol) 5-Brom-2-[4-(2-pyrrolidin-1-yl-ethoxy)-phenylethinyl]-pyridin und 243 mg (1.6 mmol) 4-Methoxy-phenylboronsäure das Produkt erhalten.
Ausbeute: 115 mg (53% d. Theorie)
$C2_6H_{26}N_20_2$ (M= 398,509)
ber.: Molpeak $(M+H)^+$: 399 gef.: Molpeak $(M+H)^+$: 399
$R_f$-Wert: 0.30 (Alox, Cyc/EtOAc 2:1)

**Beispiel 1.11**

2-[4-(2-Pyrrolidin-1-yl-ethoxy)-phenylethinyl]-5-(4-trifluormethyl-phenyl)-pyridin

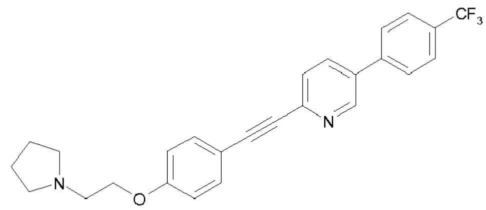

Analog Beispiel 1.9 wird aus 297 mg (0.8 mmol) 5-Brom-2-[4-(2-pyrrolidin-1-yl-ethoxy)-phenylethinyl]-pyridin und 304 mg (1.6 mmol) 4-Trifluormethyl-phenylboronsäure das Produkt erhalten.
Ausbeute: 150 mg (43% d. Theorie)
$C_{26}H_{23}F_3N_20$ (M= 436,481)
ber.: Molpeak $(M+H)^+$: 437 gef.: Molpeak $(M+H)^+$: 437
$R_f$-Wert: 0.45 (Alox, Cyc/EtOAc 2:1)

**Beispiel 2**

5-(4-Chlor-phenyl)-2-[4-(4-pyrrolidin-1-ylmethyl-phenyl)-but-1-inyl]-pyridin

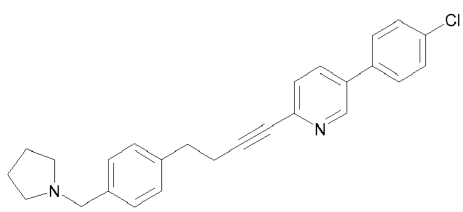

2a 1-(4-Brom-benzyl)-pyrrolidin

Zu einer Lösung von 4.52 mL (55 mmol) Pyrrolidin und 10.3 mL (60 mmol) Ethyldiisopropylamin in 100 mL THF wird eine Lösung von 12.5 g (50 mmol) 4-Brombenzylbromid langsam zugetropft und über Nacht bei RT nachgerührt. Der Niederschlag wird abfiltriert und das Lösungsmittel i.vac. entfernt. Man erhielt das Produkt als hellbraune Flüssigkeit, das ohne Reinigung weiter umgesetzt wird.
Ausbeute: 9.0 g (75% d. Theorie)
$C_{11}H_1 4BrN$ (M= 240,145)
ber.: Molpeak $(M+H)^+$: 241/243 gef.: Molpeak $(M+H)^+$: 241/243
$R_f$-Wert: 0.74 (Kieselgel, EtOAc/Me0H/$NH_3$ 90:10:1)

2b 3-(4-Pyrrolidin-1-ylmethyl-phenyl)-prop-2-in-1-ol

Eine Mischung aus 4.8 g (20.0 mmol) 1-(4-Brom-benzyl)-pyrrolidin, 1.75 mL (30.0 mmol) Propargylalkohol, 2.31 g (2.0 mmol) Tetrakis-triphenylphosphan-Palladium, 381 mg (2.0 mmol) CuI und 7.07 mL Diisopropylamin in 100 mL Acetonitril wird 14 h bei 60˚C unter Argonatmosphäre erwärmt. Das Lösungsmittel wird i.vac. abdestilliert, der Rückstand in Wasser aufgenommen, mit EtOAc erschöpfend extrahiert und die organische Phase über $Na_2S0_4$ getrocknet. Nach Entfernen des Trocken- und Lösungsmittels wird der Rückstand an Kieselgel (EtOAc/Me0H/$NH_3$ 95:5:0.5) gereinigt.
Ausbeute: 1.55 g (36% d. Theorie)
$C_{14}H_{17}N0$ (M= 215,298)
ber.: Molpeak $(M+H)^+$: 216 gef.: Molpeak $(M+H)^+$: 216
$R_f$-Wert: 0.48 (Kieselgel, EtOAc/Me0H/$NH_3$ 90:10:1)

2c 3-(4-Pyrrolidin-1-ylmethyl-phenyl)-propan-1-ol

Eine Lösung von 1.65 g (7.66 mmol) 3-(4-Pyrrolidin-1-ylmethyl-phenyl)-prop-2-in-1-ol in 20 mL EtOH wird mit 200 mg 10% Pd/C versetzt und im Autoklaven bei RT und 30 psi $H_2$ bis zur theoretischen Aufnahme an Wasserstoff hydriert. Der Katalysator wird abgesaugt, das Filtrat eingedampft und der Rückstand an Kieselgel (EtOAc/Me0H/$NH_3$ 90:10:1) gereinigt.
Ausbeute: 0.81 g (48% d. Theorie)
$C_{14}H_{21}$ NO (M= 219,330)
ber.: Molpeak $(M+H)^+$: 220 gef.: Molpeak $(M+H)^+$: 220
$R_f$-Wert: 0.2 (Kieselgel, EtOAc/Me0H/$NH_3$ 90:10:1)

2d 3-(4-Pyrrolidin-1 -ylmethyl-phenyl)-propionaldehyd

Zu einer Lösung von 780 mg (3.56 mmol) 3-(4-Pyrrolidin-1-ylmethyl-phenyl)-propan-1-ol in 30 mL DCM werden 2.87 mL (35.56 mmol) Pyridin und 2.11 g (4.98 mmol) Dess-Martin Periodinan gegeben. Die Reaktionsmischung wird 4 h bei RT gerührt, dann auf 100 mL gesättigte, wässrige $NaHCO_3$-Lösung gegeben, erschöpfend mit *tert*-Butylmethylether extrahiert, die organische Phase mit gesättigter, wässriger NaCI-Lösung gewaschen und über $Na_2S0_4$ getrocknet. Nach Entfernen des Trocken- und Lösungsmittels wird das Rohprodukt ohne Reinigung weiter umgesetzt.
Ausbeute: 750 mg (97% d. Theorie)

2e 1-(4-But-3-inyl-benzyl)-pyrrolidin

Zu einer Mischung aus 760 mg (3.5 mmol) 3-(4-Pyrrolidin-1-ylmethyl-phenyl)-propionaldehyd und 970 mg (7.0 mmol) $K_2C0_3$ in 100 mL trockenem MeOH wird unter Argonatmosphäre 815 mg (4.2 mmol) (1-Diazo-2-oxo-propyl)-phosphonsäure-dimethylester gegeben und über Nacht bei RT gerührt. Die Reaktionsmischung wird mit Diethylether verdünnt, die organische Phase mit gesättigter, wässriger $NaHC0_3$-Lösung gewaschen und über $Na_2S0_4$ getrocknet. Nach Entfernen des Trocken- und Lösungsmittels wird der Rückstand an Kieselgel (EtOAc/Me0H/$NH_3$ 95:5:0.5) gereinigt.
Ausbeute: 200 mg (27% d. Theorie)
$C_{15}H_{19}N$ (M= 213,325)
ber.: Molpeak $(M+H)^+$: 214 gef.: Molpeak $(M+H)^+$: 214
$R_f$-Wert: 0.74 (Kieselgel, EtOAc/Me0H/$NH_3$ 95:5:0.5)

2f 5-Brom-2-[4-(4-pyrrolidin-1-ylmethyl-phenyl)-but-1-inyl]-pyridin

Eine Mischung aus 200 mg (0.94 mmol) 1-(4-But-3-inyl-benzyl)-pyrrolidin, 222 mg (0.94 mmol) 2,5-Dibrompyridin, 13.2 mg (0.02 mmol) Tetrakis-triphenylphosphan-Palladium, 3.6 mg (0.02 mmol) Cul und 0.27 mL Diisopropylamin in 10 mL THF wird 4 h bei 40˚C unter Argonatmosphäre erwärmt. Das Reaktionsgemisch wird mit Wasser verdünnt, erschöpfend mit EtOAc extrahiert, die organische Phase mit gesättigter, wässriger NaCI-Lösung gewaschen und über $Na_2SO_4$ getrocknet. Nach Entfernen des Trocken- und Lösungsmittels wird der Rückstand an Kieselgel (EtOAc/Me0H/$NH_3$ 95:5:0.5) gereinigt.
Ausbeute: 110 mg (32% d. Theorie)
$C_{20}H_{21}$ $BrN_2$ (M= 369,308)
ber.: Molpeak $(M+H)^+$: 369/371 gef.: Molpeak $(M+H)^+$: 369/371
$R_f$-Wert: 0.44 (Kieselgel, EtOAc/Me0H/$NH_3$ 95:5:0.5)

2g 5-(4-Chlor-phenyl)-2-[4-(4-pyrrolidin-1 -ylmethyl-phenyl)-but-1 -inyl]-pyridin

Eine Mischung aus 100 mg (0.27 mmol) 5-Brom-2-[4-(4-pyrrolidin-1-ylmethyl-phenyl)-but-1-inyl]-pyridin, 85 mg (0.54 mmol) 4-Chlor-phenylboronsäure, 15.7 mg (0.014 mmol) Tetrakis-triphenylphosphan-Palladium, 0.28 mL einer 2 M wässrigen $Na_2C0_3$-Lösung in 10 mL 1,4-Dioxan wird 8 h bei 100˚C unter Argonatmosphäre erwärmt. Das Lösungsmittel wird i.vac. abdestilliert, der Rückstand in Wasser aufgenommen, erschöpfend mit EtOAc extrahiert und über $Na_2SO_4$ getrocknet. Nach Entfernen des Trocken- und Lösungsmittels wird der Rückstand über neutralem Alox (ICN Alumina N + 5% $H_2O$; Cyc/EtOAc 7:3) gereinigt. Die Produktfraktionen werden eingeengt, der Rückstand mit PE verrieben und abgesaugt.
Ausbeute: 12 mg (11% d. Theorie)
$C_{26}H_{25}C1N_2$ (M= 400,956)
ber.: Molpeak $(M+H)^+$: 401/403 gef.: Molpeak $(M+H)^+$: 401/403
$R_f$-Wert: 0.41 (Alox, Cyc/EtOAc 7:3)

**Beispiel 2.1**

5-(4-Chlor-phenyl)-2-[4-(4-piperidin-1-ylmethyl-phenyl)-but-1-inyl]-pyridin

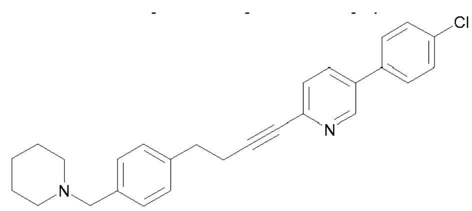

2.1 a 3-(4-Hydroxymethyl-phenyl)-propionaldehyd

Zu einer Lösung von 15.0 g (62.2 mmol) 4-lodbenzylalkohol in 100 mL DMF wird bei RT unter $N_2$-Atmosphäre 10.5 mL (152.8 mmol) Allylalkohol, 18.8 g (62.2 mmol) Tetrabutylammoniumchlorid Monohydrat, 12.8 g (152.8 mmol) $NaHC0_3$ und 0.75 g (3.1 mmol) $Pd(OAc)_2$ gegeben und die Reaktionslösung 3 h auf 60˚C erwärmt. Das Lösungsmittel wird i.vac. entfernt, der Rückstand mit 250 mL EtOAc und 80 mL Wasser versetzt und über einen Glasfaserfilter abgesaugt. Zum Filtrat werden 80 mL NaC1-Lösung gegeben, die Phasen getrennt und die organische Phase über $MgS0_4$ getrocknet. Nach Entfernen des Trocken- und Lösungsmittels wird der Rückstand durch Säulenchromatographie an Kieselgel (Gradient: Cyc/EtOAc 3:1 nach Cyc/EtOAc 1:1) gereinigt.
Ausbeute: 7.43 g (72.7 % d. Theorie)
$C_{10}H_{12}O_2$ (M= 164,206)
ber.: Molpeak $(M+H-H_2O)^+$: 147 gef.: Molpeak $(M+H-H_2O)^+$: 147
Retentionszeit HPLC: 5.26 min (Methode A)

2.1 b (4-But-3-inyl-phenyl)-methanol

Zu einer Lösung von 5.0 g (30.4 mmol) 3-(4-Hydroxymethyl-phenyl)-propionaldehyd in 100 mL MeOH werden 8.5 g (61.5 mmol) $K_2C0_3$ gegeben und dann eine Lösung von 7.0 g (36.4 mmol) (1-Diazo-2-oxo-propyl)-phosphonsäuredimethylester in 50 mL MeOH zugetropft und bei RT 3 h gerührt. Das Reaktionsgemisch wird mit 200 mL EtOAc verdünnt, mit 80 mL gesättigter $NaHCO_3$-Lösung gewaschen, die wässrige Phase mit 100 mL EtOAc extrahiert und die vereinigten organischen Phasen über $Na_2S0_4$ getrocknet. Nach Entfernen des Trocken- und Lösungsmittels wird der Rückstand chromatographisch an Kieselgel (Cyc/EtOAc 3:1) gereinigt.
Ausbeute: 3.42 g (70.1 % d. Theorie)
$C_{11}H_{12}O$ (M= 160,218)
ber.: Molpeak $(M+H-H_2O)^+$: 143 gef.: Molpeak $(M+H)^+$: $(M+H-H_2O)^+$: 143
$R_f$-Wert: 0.36 (Kieselgel, Cyc/EtOAc 2:1)

2.1 c (4-{4-[5-(4-Chlor-phenyl)-pyridin-2-yl]-but-3-inyl}-phenyl)-methanol

Unter Stickstoff-Atmosphäre werden zu einer Lösung von 1.27 g (7.92 mmol) (4-But-3-inyl-phenyl)-methanol und 2.5 g (7.92 mmol) 5-(4-Chlor-phenyl)-2-iod-pyridin in 40 mL Triethylamin und 20 mL DMF 76 mg (0.4 mmol) Cul und 281 mg (0.4 mmol) $Pd(PPh_3)_2Cl_2$ gegeben und das Reaktionsgemisch 2 h bei 65˚C gerührt. Das Lösungsmittel wird i.vac. abgezogen, der Rückstand in wenig EtOAc und MeOH gelöst und chromatographisch an Kieselgel (Gradient: Cyc/EtOAc 3:1 nach Cyc/EtOAc 1:1) gereinigt.
Ausbeute: 1.48 g (53.6 % d. Theorie)
$C_{22}H_{18}$ CINO (M= 347,848)
ber.: Molpeak $(M+H)^+$: 348/350 gef.: Molpeak $(M+H)^+$: 348/350
$R_f$-Wert: 0.23 (Kieselgel, Cyc/EtOAc 2:1)

2.1d 5-(4-Chlor-phenyl)-2-[4-(4-piperidin-1-ylmethyl-phenyl)-but-1-inyl]-pyridin

Zu einer auf 0˚C gekühlten Lösung von 75 mg (0.22 mmol) 4-{4-[5-(4-Chlor-phenyl)-pyridin-2-yl]-but-3-inyl}-phenyl)-methanol in 5 mL DCM werden 20 μL (0.26 mmol) Methansulfonsäurechlorid und 45 μL (0.26 mmol) Ethyldiiso-

propylamin zugegeben und bei dieser Temperatur 30 min gerührt. Anschliessend werden 108 μL (1.09 mmol) Piperidin zugegeben und das Reaktionsgemisch 72 h bei RT gerührt. Die Reaktionslösung wird i.vac. eingeengt und der Rückstand via HPLC gereinigt.
Ausbeute: 9.3 mg (53.6 % d. Theorie)
$C_{27}H_{27}$ $ClN_2$ (M= 414,983)
ber.: Molpeak $(M+H)^+$: 415/417 gef.: Molpeak $(M+H)^+$: 415/417
Retentionszeit HPLC: 7.62 min (Methode A)

Die folgenden Verbindungen werden wie in Beispiel 2.1 d beschrieben hergestellt:

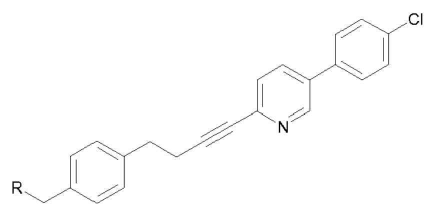

| Beispiel | R | Ausbeute (%) | Summenformel | Massenspektrum | Retentionszeit HPLC in min (Methode) |
|---|---|---|---|---|---|
| 2.2 | 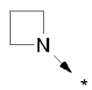 | 19.3 | $C_{25}H_{23}ClN_2$ | 387/389 LM+HI$^+$ | 7.04 (A) |
| 2.3 | 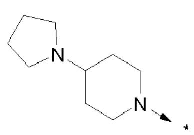 | 24.4 | $C_{31}H_{34}C1N_3$ | 484/486 LM+HI$^+$ | 5.96 (A) |
| 2.4 | 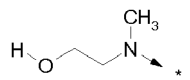 | 13.1 | $C_{25}H_{25}ClN_20$ | 405/407 $[M+H]^+$ | 6.95 (A) |
| 2.5 | 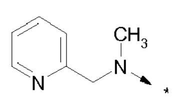 | 22.4 | $C_{29}H_{26}ClN_3$ | 452/454 $[M+H]^+$ | 7.71 (A) |
| 2.6 | 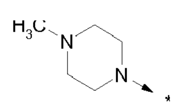 | 11.4 | $C_{27}H_{28}ClN_3$ | 430/432 $[M+H]^+$ | 6.87 (A) |
| 2.7 | 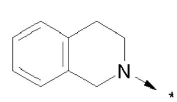 | 25.9 | $C_{31}H_{27}ClN_2$ | 463/465 $[M+H]^+$ | 8.26 (A) |

(fortgesetzt)

| Beispiel | R | Ausbeute (%) | Summenformel | Massenspektrum | Retentionszeit HPLC in min (Methode) |
|---|---|---|---|---|---|
| 2.8 | 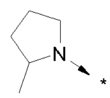 | 24.7 | $C_{27}H_{27}ClN_2$ | 415/417 $[M+H]^+$ | 7.53 (A) |
| 2.9 | 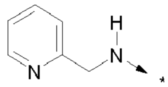 | 28.1 | $C_{28}H_{24}ClN_3$ | 438/440 $[M+H]^+$ | 7.47 (A) |
| 2.10 | 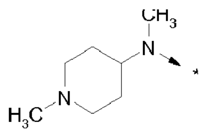 | 15.7 | $C_{29}H_{32}ClN_3$ | 458/460 $[M+H]^+$ | 5.82 (A) |
| 2.11 | 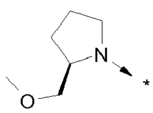 | 19.7 | $C_{28}H_{29}ClN_2O$ | 445/447 $[M+H]^+$ | 7.81 (A) |
| 2.12 | 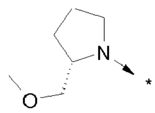 | 10.1 | $C_{28}H_{29}ClN_2O$ | 445/447 $[M+H]^+$ | 7.83 (A) |
| 2.13 | 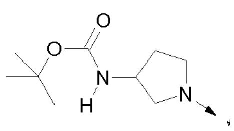 | 21.4 | $C_{31}H_{34}ClN_3O_2$ | 516/518 $[M+H]^+$ | 8.18 (A) |
| 2.14 | 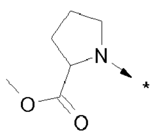 | 25.1 | $C_{28}H_{27}ClN_2O_2$ | 459/461 $[M+H]^+$ | 7.56 (A) |
| 2.15 | 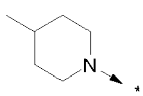 | 23.8 | $C_{28}H_{29}ClN_2$ | 429/431 $[M+H]^+$ | 8.18 (A) |

**Beispiel 2.16**

4-[(4-{4-[5-(4-Chlor-phenyl)-pyridin-2-yl]-but-3-inyl}-benzyl)-methyl-amino]-piperidin-1 - carbonsäure-*tert*-butylester

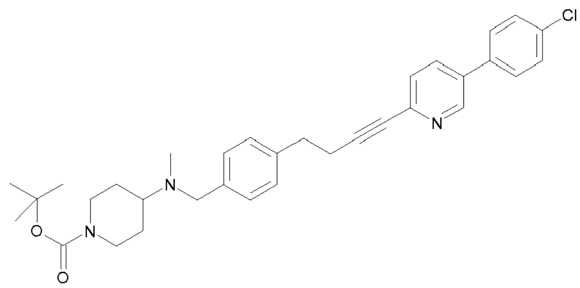

Hergestellt analog zu Beispiel 2.1 d aus 75 mg (0.22 mmol) 4-{4-[5-(4-Chlor-phenyl)-pyridin-2-yl]-but-3-inyl}-phenyl)-methanol und 20 µL (1.09 mmol) 4-Methylaminopiperidin-1-carbonsäure-tert-butylester, wobei 7 Tage bei RT gerührt wird. Zur Vervollständigung der Reaktion wird erneut die beschriebene Reaktionssequenz mit der gleichen Menge an eingesetzten Reagentien wiederholt und nach 24 h Reaktionsdauer aufgearbeitet.
Ausbeute: 8.5 mg (7.2 % d. Theorie)
$C_{33}H_{33}$ $ClN_3O_2$ (M= 544,143)
ber.: Molpeak $(M+H)^+$: 544/546 gef.: Molpeak $(M+H)^+$: 544/546
Retentionszeit HPLC: 8.46 min (Methode A)

**Beispiel 2.17**

(4-{4-[5-(4-Chlor-phenyl)-pyridin-2-yl]-but-3-inyl}-benzyl)-methyl-piperidin-4-yl-amin

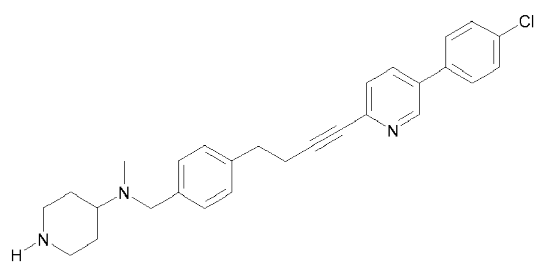

Zu einer Lösung von 35 mg (0.06 mmol) 4-[(4-{4-[5-(4-Chlor-phenyl)-pyridin-2-yl]-but-3-inyl}-benzyl)-methyl-amino]-piperidin-1-carbonsäure-*tert*-butylester in 3 mL DCM werden 0.5 mL Trifluoressigsäure gegeben und das Reaktionsgemisch bei RT 3 h gerührt. Man engt i.vac. ein, versetzt den Rückstand mit 10 mL $NaHCO_3$-Lösung, extrahiert mit 20 mL DCM und trocknet die organische Phase über $Na_2SO_4$. Nach Entfernen des Trocken- und Lösungsmittels erhält man das gewünschte Produkt.
Ausbeute: 8.0 mg (28.2 % d. Theorie)
$C_{28}H_{30}$ $ClN_3$ (M= 444,024)
ber.: Molpeak $(M+H)^+$: 444/446 gef.: Molpeak $(M+H)^+$: 444/446
Retentionszeit HPLC: 5.83 min (Methode A)

**Beispiel 2.18**

1-(4-{4-[5-(4-Chlor-phenyl)-pyridin-2-yl]-but-3-inyl}-benzyl)-pyrrolidin-3-ylamin

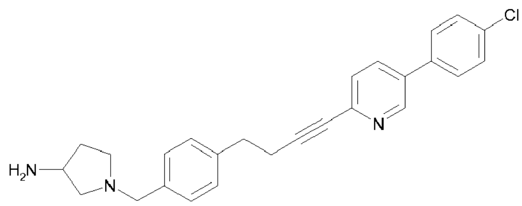

Hergestellt analog Beispiel 2.17 aus 17 mg (0.03 mmol) [1-(4-{4-[5-(4-Chlor-phenyl)-pyridin-2-yl]-but-3-inyl)-benzyl)-pyrrolidin-3-yl]-carbaminsäure-tert-butylester (Beispiel 2.13).
Ausbeute: 12.0 mg (87.4 % d. Theorie)
$C_{26}H_{26}$ $ClN_3$ (M= 415,970)
ber.: Molpeak $(M+H)^+$: 416/418 gef.: Molpeak $(M+H)^+$: 416/418
Retentionszeit HPLC: 5.83 min (Methode A)

**Beispiel 2.19**

1-(4-{4-[5-(4-Chlor-phenyl)-pyridin-2-yl]-but-3-inyl}-benzyl)-pyrrolidin-2-carbonsäure

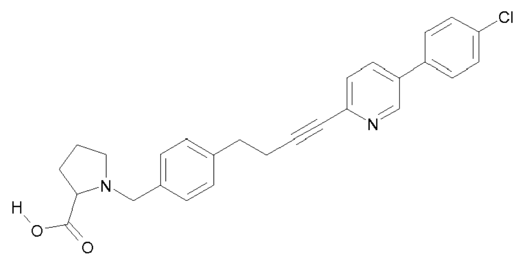

Zu einer Lösung von 33 mg (0.07 mmol) 1-(4-{4-[5-(4-Chlor-phenyl)-pyridin-2-yl]-but-3-inyl}-benzyl)-pyrrolidin-2-carbonsäuremethylester (Beispiel 2.14) in 5 mL MeOH werden 0.5 mL 1 M NaOH-Lösung zugegeben und das Reaktionsgemisch 4 h bei RT gerührt. Man engt i.vac. ein, versetzt mit 5 mL Wasser, extrahiert mit 10 mL EtOAc und sättigt die wässrige Phase mit NaCl, wobei das Produkt ausfällt. Man engt erneut i.vac. ein, versetzt den Rückstand mit EtOH, filtriert und entfernt das Lösungsmittel.
Ausbeute: 30.0 mg (93.6 % d. Theorie)
$C_{27}H_{25}$ $ClN_2O_2$ (M= 444,966)
ber.: Molpeak $(M+H)^+$: 445/447 gef.: Molpeak $(M+H)^+$: 445/447
Retentionszeit HPLC: 7.28 min (Methode A)

Beispiel 2.20

5-(2,4-Dichlor-phenyl)-2-[4-(4-pyrrolidin-1-ylmethyl-phenyl)-but-1-inyl]-pyridin

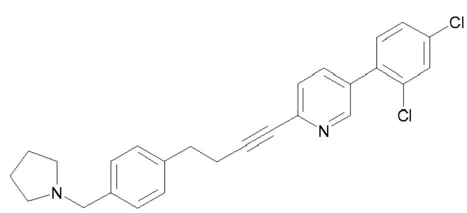

2.20a {4-[4-(5-Brom-pyridin-2-yl)-but-3-inyl]-phenyl}-methanol

Unter $N_2$-Atmosphäre werden zu einer Lösung von 2.0 g (12.48 mmol) (4-But-3-inyl-phenyl)-methanol und 3.2 g (13.1 mmol) 2,5-Dibrompyridin in 80 mL Triethylamin 130 mg (0.67 mmol) CuI und 300 mg (0.42 mmol) $Pd(PPh_3)_2Cl_2$ gegeben und das Reaktionsgemisch 1.5 h bei 50°C gerührt. Das Lösungsmittel wird i.vac. abgezogen, der Rückstand in wenig DCM gelöst und chromatographisch an Kieselgel (Gradient: Cyc/EtOAc 4:1 nach Cyc/EtOAc 3:1) gereinigt.
Ausbeute: 2.76 g (66.6 % d. Theorie)
$C_{16}H_{14}$ BrNO (M= 316,20)
ber.: Molpeak $(M+H)^+$: 316/318 gef.: Molpeak $(M+H)^+$: 316/318
$R_f$-Wert: 0.28 (Kieselgel, Cyc/EtOAc 2:1)

2.20b 5-Brom-2-[4-(4-pyrrolidin-1-ylmethyl-phenyl)-but-1-inyl]-pyridin

Zu einer auf 0°C gekühlten Lösung von 800 mg (2.53 mmol) {4-[4-(5-Brom-pyridin-2-yl)-but-3-inyl]-phenyl}-methanol in 17 mL DCM werden 0.24 mL (3.04 mmol) Methansulfonsäurechlorid zugegeben und dann eine Lösung von 0.52 mL Ethyldiisopropylamin in 3 mL DCM zugetropft. Man rührt weitere 30 min bei 0°C, gibt 0.51 mL (6.08 mmol) Pyrrolidin zu, erwärmt auf RT und hält das Reaktionsgemisch 5 h bei dieser Temperatur. Zur Vervollständigung der Reaktion werden nochmals 0.26 mL (3 mmol) Pyrrolidin zugegeben und 1 h bei RT gerührt. Man engt i.vac. ein, versetzt mit 10 mL Wasser und 20 ml EtOAc, säuert mit 1 M HCl an und trennt die organische Phase ab. Die wässrige Phase wird mit 2 M $Na_2C0_3$-Lösung alkalisch gestellt, mit 20 mL EtOAc extrahiert, die organische Phase abgetrennt und über $Na_2SO_4$ getrocknet. Nach Entfernen des Trocken- und Lösungsmittels erhält man das gewünschte Produkt.
Ausbeute: 630.0 mg (67.4 % d. Theorie)
$C_{20}H_{21}$ $BrN_2$ (M= 369,308)
ber.: Molpeak $(M+H)^+$: 369/371 gef.: Molpeak $(M+H)^+$: 369/371
Retentionszeit HPLC: 6.08 min (Methode A)

2.20c 5-(2,4-Dichlor-phenyl)-2-[4-(4-pyrrolidin-1-ylmethyl-phenyl)-but-1-inyl]-pyridin

Zu einer Suspension von 60 mg (0.16 mmol) 5-Brom-2-[4-(4-pyrrolidin-1-ylmethyl-phenyl)-but-1-inyl]-pyridin und 63 mg (0.32 mmol) 2,4-Dichlorphenylboronsäure in 4 mL 1,4-Dioxan und 1 mL 2 M $Na_2C0_3$-Lösung werden 10 mg (0.01 mmol) Tetrakis-triphenylphosphan-Palladium gegeben und das Reaktionsgemisch 1 h bei 110°C gerührt. Man engt i.vac. ein und extrahiert den Rückstand zweimal mit jeweils 15 mL EtOH. Das Lösungsmittel wird entfernt und der Rückstand via HPLC gereinigt.
Ausbeute: 22.7 mg (32.2 % d. Theorie)
$C_{26}H_{24}$ $C1_2N_2$ (M= 435,401)
ber.: Molpeak $(M+H)^+$: 435/437/439 gef.: Molpeak $(M+H)^+$: 435/437/439
Retentionszeit HPLC: 5.53 min (Methode C)

Die folgenden Verbindungen werden wie in Beispiel 2.20c beschrieben hergestellt:

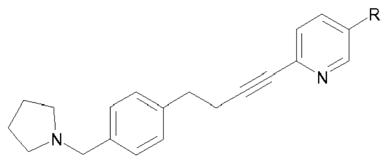

| Beispiel | R | Ausbeute (%) | Summenformel | Massenspektrum | Retentionszeit HPLC in min (Methode) |
|---|---|---|---|---|---|
| 2.21 | 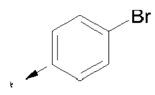 | 13.0 | $C_{26}H_{25}BrN_2$ | 445/447 $[M+H]^+$ | 5.53 (C) |

(fortgesetzt)

| Beispiel | R | Ausbeute (%) | Summenformel | Massenspektrum | Retentionszeit HPLC in min (Methode) |
|---|---|---|---|---|---|
| 2.22 | 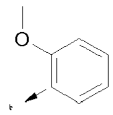 | 41.4 | $C_{27}H_{28}N_2O$ | 397 $[M+H]^+$ | 3.39 (C) |
| 2.23 | 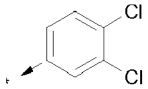 | 30.8 | $C_{26}H_{24}Cl_2N_2$ | 435/437/43 9 $[M+H]^+$ | 3.70 (A) |
| 2.24 | 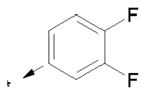 | 21.8 | $C_{26}H_{24}F_2N_2$ | 403 $[M+H]^+$ | 7.21 (A) |
| 2.25 | 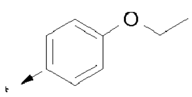 | 7.5 | $C_{28}H_{30}N_2O$ | 411 $[M+H]^+$ | 7.30 (A) |

**Beispiel 2.26**

5-(4-Methoxy-phenyl)-2-[4-(4-pyrrolidin-1-ylmethyl-phenyl)-but-1-inyl]-pyridin

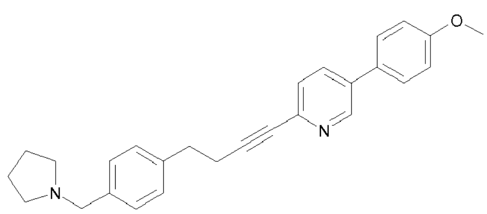

Hergestellt analog zu Beispiel 2.20c, nach beendeter Umsetzung wird das Reaktionsgemisch mit 10 mL Wasser und 20 mL EtOAc versetzt, über einen Glasfaserfilter filtriert, die organische Phase abgetrennt und über $Na_2SO_4$ getrocknet. Das Lösungsmittel wird entfernt und der Rückstand via HPLC gereinigt.
Ausbeute: 17.4 mg (23.1 % d. Theorie)
$C_{27}H_{28}N_2O_2$ (M= 396,537)
ber.: Molpeak $(M+H)^+$: 397 gef.: Molpeak $(M+H)^+$: 397
Retentionszeit HPLC: 8.15 min (Methode A)

**Beispiel 2.27**

4-(4-{4-[5-(4-Chlor-phenyl)-pyridin-2-yl]-but-3-inyl}-benzyl)-morpholin

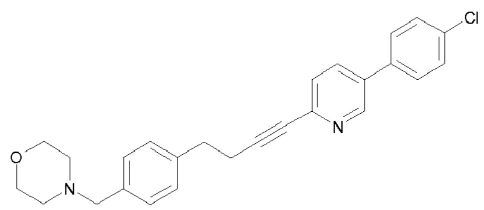

2.27a 4-{4-[4-(5-Brom-pyridin-2-yl)-but-3-inyl]-benzyl}-morpholin

Zu einer auf 0°C gekühlten Lösung von 120 mg (0.38 mmol) {4-[4-(5-Brom-pyridin-2-yl)-but-3-inyl]-phenyl}-methanol (Beispiel 2.20a) in 5 mL DCM werden 36 μL (0.46 mmol) Methansulfonsäurechlorid gegeben. Man tropft langsam eine Lösung von 78 μL (0.46 mmol) Ethyldiisopropylamin in 1 mL DCM zu, rührt weitere 30 min bei 0°C, gibt dann 80 μL (0.92 mmol) Morpholin zu, lässt auf RT erwärmen und hält das Reaktionsgemisch 2 h bei RT. Man engt i.vac. ein, versetzt den Rückstand mit 20 mL EtOAc und 10 mL Wasser, säuert mit 1 M HCl an und trennt die Phasen. Die wässrige Phase wird mit 2 M $Na_2C0_3$-Lösung versetzt, mit 20 mL EtOAc extrahiert und die organische Phase über $Na_2S0_4$ getrocknet. Nach Entfernen des Trocken- und Lösungsmittels erhält man das Produkt.
Ausbeute: 146 mg (100 % d. Theorie)
$C_{20}H_{21}$ $BrN_2O$ (M= 385,307)
ber.: Molpeak $(M+H)^+$: 385/387 gef.: Molpeak $(M+H)^+$: 385/387
Retentionszeit HPLC: 5.92 min (Methode A)

2.27b 4-(4-{4-[5-(4-Chlor-phenyl)-pyridin-2-yl]-but-3-inyl)-benzyl)-morpholin

Hergestellt analog Beispiel 2.20c aus 90 mg (0.23 mmol) 4-{4-[4-(5-Brom-pyridin-2-yl)-but-3-inyl]-benzyl}-morpholin und 73 mg (0.47 mmol) 4-Chlorphenylboronsäure.
Ausbeute: 17.5 mg (17.9 % d. Theorie)
$C_{26}H_{25}ClN_2O$ (M= 416,955)
ber.: Molpeak $(M+H)^+$: 417/419 gef.: Molpeak $(M+H)^+$: 417/419
Retentionszeit HPLC: 7.51 min (Methode A)

**Beispiel 2.28**

(4-{4-[5-(4-Chlor-phenyl)-pyridin-2-yl]-but-3-inyl}-benzyl)-cyclopropylmethyl-amin

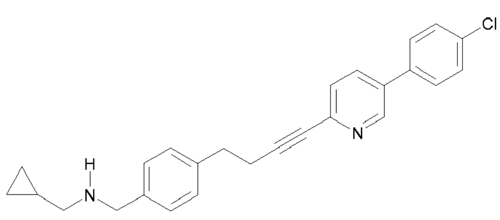

2.28a (4-{4-[4-(5-Brom-pyrid in-2-yl)-but -3-inyl]-benzyl)-cyclopropylmethyl-amin

Zu einer auf 0°C gekühlten Lösung von 120 mg (0.38 mmol) {4-[4-(5-Brom-pyridin-2-yl)-but-3-inyl]-phenyll-methanol (Beispiel 2.20a) in 5 mL DCM werden 36 μL (0.46 mmol) Methansulfonsäurechlorid gegeben. Man tropft langsam eine Lösung von 78 μL (0.46 mmol) Ethyldiisopropylamin in 1 mL DCM zu, rührt weitere 30 min bei 0°C, gibt dann 70 μL (0.92 mmol) C-Cyclopropyl-methylamin zu, lässt auf RT erwärmen und hält das Reaktionsgemisch 21 h bei RT. Zur Vervollständigung der Reaktion werden erneut 78 μL C-Cyclopropyl-methylamin zugegeben und weiter 5.5 h bei RT gerührt. Das Reaktionsgemisch wird i.vac. eingeengt und der Rückstand chromatographisch an Kieselgel (Gradient: Cyc/EtOAc 2:1 nach Cyc/EtOAc 1:1) gereinigt.

Ausbeute: 70.0 mg (49.9 % d. Theorie)
$C_{20}H_{21}$ $BrN_2$ (M= 369,308)
ber.: Molpeak $(M+H)^+$: 369/371 gef.: Molpeak $(M+H)^+$: 369/371
Retentionszeit HPLC: 6.55 min (Methode A)

2.28b (4-{4-[5-(4-Chlor-phenyl)-pyridin-2-yl]-but-3-inyl)-benzyl)-cydopropylmethyl-amin

Hergestellt analog Beispiel 2.20c aus 65 mg (0.18 mmol) (4-{4-[4-(5-Brom-pyridin-2-yl)-but-3-inyl]-benzyl)-cyclopropylmethyl-amin und 55 mg (0.35 mmol)) 4-Chlor-phenylboronsäure.
Ausbeute: 15.4 mg (21.8 % d. Theorie)
$C_{26}H_{25}ClN_2$ (M= 400,956)
ber.: Molpeak $(M+H)^+$: 401/403 gef.: Molpeak $(M+H)^+$: 401/403
Retentionszeit HPLC: 7.63 min (Methode A)

**Beispiel 2.29**

3-(4-Chlor-phenyl)-6-[4-(4-pyrrolidin-1-ylmethyl-phenyl)-but-1-inyl]-pyridazin

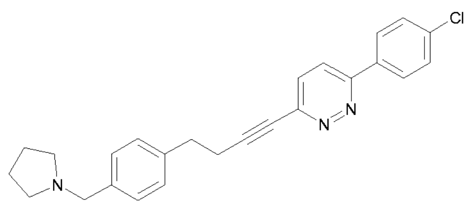

2.29a 3-Chlor-6-(4-chlor-phenyl)-pyridazin

Unter Argon-Atmosphäre wird eine Lösung von 1.08 g (7.05 mmol) 3,6-Dichlor-pyridazin, 10 mL 2 M $Na_2CO_3$-Lösung und 80 mg (0.14 mmol) Chlor(di-2-norbornylphosphino)(2'-dimethylamino-1-1'-biphenyl-2-yl)palladium(ll) in 150 mL 1,4-Dioxan auf 110˚C erhitzt. Bei dieser Temperatur wird eine Lösung von 1.13 g (7.05 mmol) 4-Chlorphenylboronsäure in 50 mL 1,4-Dioxan innerhalb von 2 h zugetropft und das Reaktionsgemisch eine weitere Stunde erhitzt. Nach dem Abkühlen wird mit 100 mL Wasser versetzt, mit 100 mL EtOAc extrahiert und die organische Phase über $Na_2SO_4$ getrocknet. Nach Entfernen des Trocken- und Lösungsmittels wird der Rückstand chromatographisch (Kieselgel, Cyc/EtOAc 8:2) gereinigt.
Ausbeute: 567 mg (35.7 % d. Theorie)
$C_{10}H_6Cl_2N_2$ (M= 225,079)
ber.: Molpeak $(M+H)^+$: 225/227 gef.: Molpeak $(M+H)^+$: 225/227
$R_f$-Wert: 0.29 (Kieselgel, Cyc/EtOAc 8:2)

2.29b 3-(4-Chlor-phenyl)-6-[4-(4-pyrrolidin-1-ylmethyl-phenyl)-but-1-inyl]-pyridazin

Unter Argon-Atmosphäre werden zu einer Lösung von 77 mg (0.34 mmol) 3-Chlor-6-(4-chlor-phenyl)-pyridazin und 73 mg (0.34 mmol) 1-(4-But-3-inyl-benzyl)-pyrrolidin (Beispiel 2e) in 4 mL DMF 0.1 mL (0.72 mmol) Triethylamin, 3 mg (0.02 mmol) Cul und 8 mg (0.01 mmol) Bis-triphenylphosphan-palladium(ll)-chlorid gegeben und das Reaktionsgemisch 20 min bei 100˚C und 300 W in der Mikrowelle gerührt. Das Reaktionsgemisch wird mit 10 mL Wasser versetzt, mit 10 mL EtOAc extrahiert und die organische Phase mit Wasser gewaschen und über $Na_2SO_4$ getrocknet. Nach Entfernen des Trocken- und Lösungsmittels wird der Rückstand chromatographisch (Kieselgel, EtOAc/MeOH/$NH_3$ 90:10:1) gereinigt.
Ausbeute: 6 mg (4.4 % d. Theorie)
$C_{25}H_{24}BrN_3$ (M= 401,943)
ber.: Molpeak $(M+H)^+$: 402/404 gef.: Molpeak $(M+H)^+$: 402/404
$R_f$-Wert: 0.66 (Kieselgel, EtOAc/Me0H/$NH_3$ 90:10:1)

**Beispiel 2.30**

5-(4-Chlor-phenyl)-2-[4-(4-pyrrolid in-1-ylmethyl-phenyl )-but-1-inyl]-nicotinnitril

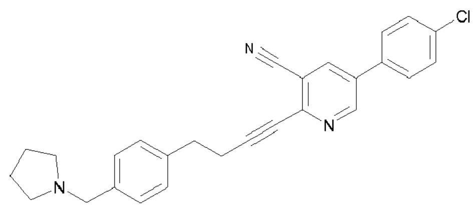

Unter Argon-Atmosphäre werden zu einer Lösung von 50 mg (0.2 mmol) 2-Chlor-5-(4-chlor-phenyl)-nicotinnitril und 43 mg (0.2 mmol) 1-(4-But-3-inyl-benzyl)-pyrrolidin in 2 mL DMF und 5 mL (20 mmol) Triethylamin 1.9 mg (0.1 mmol) CuI und 7 mg (0.1 mmol) $Pd(PPh_3)_2Cl_2$ gegeben und das Reaktionsgemisch 18 h bei 50˚C gerührt. Man engt i.vac. ein, nimmt den Rückstand in Wasser auf, extrahiert erschöpfend mit EtOAc und trocknet die organische Phase über $Na_2SO_4$. Nach Entfernen des Trocken- und Lösungsmittels wird der Rückstand via HPLC gereinigt.
Ausbeute: 6.5 mg (7.6 % d. Theorie)
$C_{27}H_{24}BrN_3$ (M= 425,965)
ber.: Molpeak $(M+H)^+$: 425 gef.: Molpeak $(M+H)^+$: 425
Retentionszeit HPLC: 6.80 min (Methode A)

**Beispiel 3.1:**

5-(4-Chlor-phenyl)-2-[3-chlor-4-(2-pyrrolid in-1-yl-ethoxy)-phenylethinyl]-pyridin

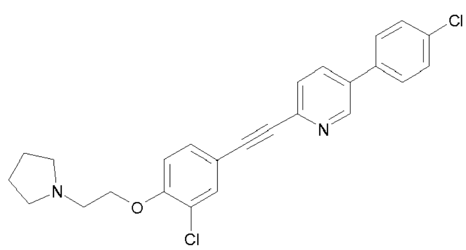

3.1 a 5-(4-Chlor-phenyl)-pyridin-2-ylamin

Zu einer Lösung aus 53.5 g (300 mmol) 2-Amino-5-brompyridin und 50.0 g (313 mmol)

4-Chlorphenylboronsäure in 1.0 L 1,4-Dioxan und 250 mL Methanol unter Argon werden nacheinander 300 mL (600 mmol) einer 2 M $Na_2CO_3$-Lösung und 3.45 g (3.0 mmol) Tetrakis-triphenylphosphan-palladium gegeben. Die Reaktionsmischung wird 2.5 h bei 110˚C gerührt. Das Lösungsmittel wird i.vac. entfernt, der Rückstand in EtOAc und Wasser aufgenommen. Die organische Phase wird über $Na_2SO_4$ getrocknet und das Lösungsmittel i.vac. entfernt. Weitere Reinigung erfolgt durch Säulenchromatographie an Kieselgel (Gradient: DCM nach DCM/MeOH 20:1).
Ausbeute: 47 g (76.5 % d. Theorie)
$C_{11}H_9ClN_2$ (M= 204,661)
ber.: Molpeak $(M+H)^+$: 205/207 gef.: Molpeak $(M+H)^+$: 205/207
Retentionszeit HPLC: 5.15 min (Methode A)

3.1 b 5-(4-Chlor-phenyl)-2-iod-pyridin

Zu einer Lösung von 38 g (190 mmol) 5-(4-Chlor-phenyl)-pyridin-2-ylamin in 400 mL Tetrachlorkohlenstoff in einem gegen Lichteinfluss geschützten Kolben werden 40.5 mL (33 mmol) *tert*-Butylnitrit und 54 g (210 mmol) Iod gegeben und die Mischung wird 72 h bei RT gerührt. Es werden weitere 40.5 mL (33 mmol) *tert*-Butylnitrit, 54 g (210 mmol) Iod und 100 mL DCM zugegeben. Die Reaktionslösung wird weitere 24 h bei RT gerührt. Das Lösungsmittel wird i.vac. entfernt und der Rückstand in 125 mL EtOAc und 50 mL Wasser aufgenommen. Die wässrige Phase wird einmal mit EtOAc extrahiert. Die organische Phase wird über $Na_2SO_4$ getrocknet und eine Nacht über Aktivkohle gerührt. Nach Filtration wird das Lösungsmittel i.vac. entfernt. Weitere Reinigung erfolgt durch Säulenchromatographie an Kieselgel (PE/EtOAc 9:1).
Ausbeute: 35 g (58.4 % d. Theorie)
$C_{11}H_7ClIN$ (M= 315,543)
ber.: Molpeak $(M+H)^+$: 316/318 gef.: Molpeak $(M+H)^+$: 316/318
$R_f$-Wert: 0.87 (Kieselgel, PE/EtOAc 6:4)

3.1 c 5-(4-Chlor-phenyl)-2-trimethylsilanylethinyl-pyridin

Zu einer Lösung von 34 g (110 mmol) 5-(4-Chlor-phenyl)-2-iod-pyridin in 300 mL Acetonitril und 150 mL THF unter einer Argon-Atmosphäre werden nacheinander 34.9 mL (250 mmol) Triethylamin und 20.8 mL (150.0 mmol) Ethinyl-trimethyl-silan gegeben.
Dann werden 803 mg (1.10 mmol) Pd(dppf)Cl und 209 mg (1.10 mmol) CuI zugegeben. Die Reaktionslösung wird über Nacht bei RT gerührt. Das Lösungsmittel wird i.vac. entfernt und die weitere Reinigung erfolgt durch Säulenchromatographie an Kieselgel (PE/EtOAc 8:2).
Ausbeute: 15.3 g (48.7 % d. Theorie)
$C_{16}H_{16}ClNSi$ (M= 285,852)
ber.: Molpeak $(M+H)^+$: 286/288 gef.: Molpeak $(M+H)^+$: 286/288
Retentionszeit HPLC: 7.10 min (Methode A)

3.1d 5-(4-Chlor-phenyl)-2-ethinyl-pyridin

Unter Argonatmosphäre werden zu einer Lösung von 5.8 g (20.3 mmol) 5-(4-Chlor-phenyl)-2-trimethylsilanylethinyl-pyridin in 200 mL DCM bei 0˚C 6.6 g (21.0 mmol) TBAF zugegeben. Die Reaktionslösung wird 3 h gerührt, wobei die Reaktionstemperatur langsam auf RT ansteigt. Es wird auf 50 mL Wasser gegeben und die organische Phase wird viermal mit je 50 mL Wasser extrahiert, über $MgSO_4$ getrocknet und über Aktivkohle filtriert. Das Lösungsmittel wird i.vac. entfernt und die weitere Reinigung erfolgt durch Säulenchromatographie an Kieselgel (PE/EtOAc 1:1).
Ausbeute: 3.9 g (90.0 % d. Theorie)
$C_{13}H_8ClN$ (M= 213,668)
ber.: Molpeak $(M+H)^+$: 214/216 gef.: Molpeak $(M+H)^+$: 214/216
$R_f$-Wert: 0.87 (Kieselgel, Cyc/EtOAc 8:2)

3.1 e 1-[2-(4-Brom-2-chlor-phenoxy)-ethyl]-pyrrolidin

Zu einer Lösung von 207 mg (1.00 mmol) 4-Brom-2-chlor-phenol in 5 mL DMF wird 415 mg (3.00 mmol) $K_2CO_3$ und 170 mg (1.00 mmol) N-(2-Chlorethyl)-pyrrolidin-hydrochlorid gegeben und das Gemisch 24 h bei RT gerührt. Das Reaktionsgemisch wird mit 50 mL EtOAc verdünnt, einmal mit 30 mL Wasser und zweimal mit je 30 mL halbgesättigter $NaHCO_3$-Lösung extrahiert. Die organische Phase wird über $MgSO_4$ getrocknet und das Lösungsmittel i.vac. entfernt. Die weitere Reinigung erfolgt durch Säulenchromatographie an Kieselgel (Gradient: DCM nach DCM/MeOH 9:1).
Ausbeute: 100 mg (32.8 % d. Theorie)
$C_{12}H_{15}BrClNO$ (M= 304,616)
ber.: Molpeak $(M+H)^+$: 304/306/308 gef.: Molpeak $(M+H)^+$: 304/306/308
Retentionszeit HPLC: 5.59 min (Methode A)

3.1f f 5-(4-Chlor-phenyl)-2-[3-chlor-4-(2-pyrrolidin-1-yl-ethoxy)-phenylethinyl]-pyridin

Unter Argonatmosphäre werden zu einer Lösung von 71 mg (0.33 mmol) 5-(4-Chlor-phenyl)-2-ethinyl-pyridin und 100 mg (0.33 mmol) 1-[2-(4-Brom-2-chlor-phenoxy)-ethyl]-pyrrolidin in 3.0 mL DMF nacheinander 0.14 mL (1.00 mmol) Triethylamin, 11 mg (0.02 mmol) $Pd(PPh_3)_2Cl_2$ und 2.9 mg (0.015 mmol) CuI zugegeben. Die Mischung wird 10 min bei 100 ˚C und 200 Watt in der Mikrowelle gerührt. Die Reaktionslösung wird mit 30 mL EtOAc verdünnt, zweimal

mit halbgesättigter NaCl-Lösung gewaschen und die organische Phase über $MgSO_4$ getrocknet. Das Lösungsmittel wird i.vac. entfernt und die weitere Reinigung erfolgt durch Säulenchromatographie mit HPLC-MS.
Ausbeute: 12 mg (8.3 % d. Theorie)
$C_{25}H_{22}Cl_2N_2O$ (M= 437,373)
ber.: Molpeak $(M+H)^+$: 437/439/441 gef.: Molpeak $(M+H)^+$: 437/439/441
$R_f$-Wert: 0.28 (Kieselgel, DCM/MeOH 9:1)

**Beispiel 3.2**

5-(4-Chlor-phenyl )-2-[3,5-dimethyl-4-(2-pyrrolid in-1-yl-ethoxy)-phenylethinyl]-pyridin

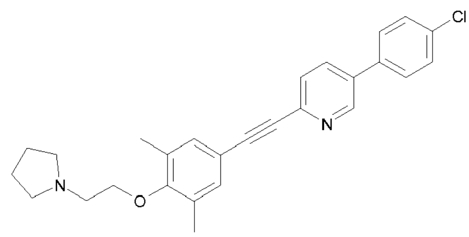

3.2a 1-[2-(4-Brom-2,6-dimethyl-phenoxy)-ethyl]-pyrrolidin

Analog Beispiel 3.1e wird aus 201 mg (1.00 mmol) 4-Brom-2,6-dimethyl-phenol und 170 mg (1.00 mmol) N-(2-Chlorethyl)-pyrrolidin-hydrochlorid das Produkt erhalten.
Ausbeute: 200 mg (67.1 % d. Theorie)
$C_{14}H_{20}BrNO$ (M= 298,226)
ber.: Molpeak $(M+H)^+$: 298/300 gef.: Molpeak $(M+H)^+$: 298/300
Retentionszeit HPLC: 5.76 min (Methode A)

3.2b 5-(4-Chlor-phenyl)-2-[3,5-dimethyl-4-(2-pyrrolidin-1-yl-ethoxy)-phenylethinyl]-pyridin

Analog Beispiel 3.1f wird aus 200 mg (0.67 mmol) 1-[2-(4-Brom-2,6-dimethyl-phenoxy)-ethyl]-pyrrolidin und 143 mg (0.67 mmol) 5-(4-Chlor-phenyl)-2-ethinyl-pyridin das Produkt erhalten.
Ausbeute: 5 mg (1.7 % d. Theorie)
$C_{27}H_{27}ClN_2O$ (M= 430,982)
ber.: Molpeak $(M+H)^+$: 431/433 gef.: Molpeak $(M+H)^+$: 431/433
$R_f$-Wert: 0.29 (Kieselgel, DCM/MeOH 9:1)

**Beispiel 3.3**

5-(4-Chlor-phenyl)-2-[3-fluor-4-(2-pyrrolid in-1-yl-ethoxy)-phenyleth inyl]-pyridin

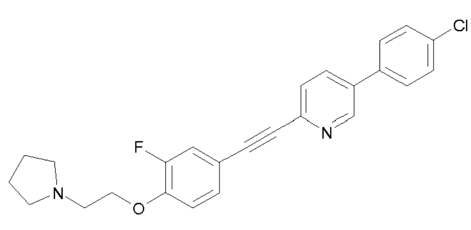

3.3a 1-[2-(4-Brom-2-fluor-phenoxy)-ethyl]-pyrrolidin

Analog Beispiel 3.1 e (Acetonitril statt DMF) werden aus 0.57 mL (5.24 mmol) 4-Brom-2-fluor-phenol und 1.02 g (6.00 mmol) N-(2-Chlorethyl)-pyrrolidin-hydrochlorid das Produkt erhalten.
Ausbeute: 1.16 g (76.6 % d. Theorie)
$C_{12}H_{15}BrFNO$ (M= 288,162)
ber.: Molpeak $(M+H)^+$: 288/290 gef.: Molpeak $(M+H)^+$: 288/290
$R_f$-Wert: 0.21 (Kieselgel, EtOAc/MeOH 9:1).

3.3b 1-[2-(2-Fluor-4-iod-phenoxy)-ethyl]-pyrrolidin

Hergestellt nach der allgemeinen Arbeitsvorschrift II aus 1-[2-(4-Brom-2-fluor-phenoxy)-ethyl]-pyrrolidin (1.10 g, 3.82 mmol).
Ausbeute: 1.13 g (88.3 % d. Theorie)
$C_{12}H_{15}FIN0$ (M= 335,162)
ber.: Molpeak $(M+H)^+$: 336 gef.: Molpeak $(M+H)^+$: 336
Retentionszeit HPLC: 4.79 min (Methode A)

3.3c 5-(4-Chlor-phenyl)-2-[3-fluor-4-(2-pyrrolidin-1-yl-ethoxy)-phenylethinyl]-pyridin

Hergestellt nach der allgemeinen Arbeitsvorschrift I aus 1-[2-(2-Fluor-4-iod-phenoxy)-ethyl]-pyrrolidin (300 mg, 0.90 mmol) und 5-(4-Chlor-phenyl)-2-ethinyl-pyridin (201 mg, 0.94 mmol).
Ausbeute: 150 mg (39.8 % d. Theorie)
$C_{25}H_{22}ClFIN_2O$ (M= 420,918)
ber.: Molpeak $(M+H)^+$: 421/423 gef.: Molpeak $(M+H)^+$: 421/423
Retentionszeit HPLC: 7.18 min (Methode A)

**Beispiel 3.4**

5-[5-(4-Chlor-phenyl)-pyridin-2-ylethinyl]-2-(2-pyrrolidin-1-yl-ethoxy)-benzoesäure-methylester

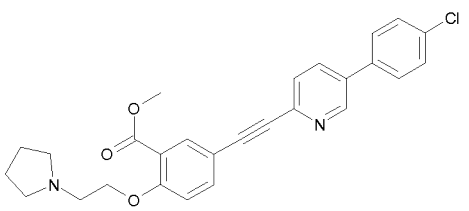

3.4a 5-Iod-2-(2-pyrrolidin-1-yl-ethoxy)-benzoesäuremethylester

Analog Beispiel 3.1.e (Acetonitril statt DMF) wird aus 10.0 g (36.0 mmol) 5-Iodsalicylsäuremethylester und 6.12 g (36.0 mmol) N-(2-Chlorethyl)-pyrrolidin-hydrochlorid das Produkt erhalten.
Ausbeute: 12.0 g (88.8 % d. Theorie)
$C_{14}H_{18}1NO_3$ (M= 375,209)
ber.: Molpeak $(M+H)^+$: 376 gef.: Molpeak $(M+H)^+$: 376
$R_f$-Wert: 0.40 (Kieselgel, DCM/MeOH/$NH_3$ 9:1:0.1)

3.4b 5-[5-(4-Chlor-phenyl)-pyridin-2-ylethinyl]-2-(2-pyrrolidin-1-yl-ethoxy)-benzoesäuremethylester

Hergestellt nach der allgemeinen Arbeitsvorschrift 1 aus 5-Iod-2-(2-pyrrolidin-1-yl-ethoxy)-benzoesäuremethylester (3.0 g, 8.0 mmol) und 5-(4-Chlor-phenyl)-2-ethinyl-pyridin (1.76 g, 8.24 mmol).
Ausbeute: 1.02 g (26.9 % d. Theorie)
$C_{27}H_{25}ClN_2O_3$ (M= 460,965)
ber.: Molpeak $(M+H)^+$: 461/463 gef.: Molpeak $(M+H)^+$: 461/463

Retentionszeit HPLC: 7.49 min (Methode A)

**Beispiel 3.5**

5-(4-Chlor-phenyl)-2-[3-methoxy-4-(2-pyrrolidin-1-yl-ethoxy)-phenylethinyl]-pyridin

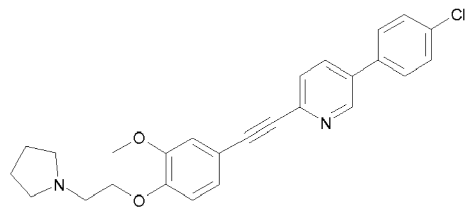

3.5a 1-[2-(4-Brom-2-methoxy-phenoxy)-ethyl]-pyrrolidin

Analog Beispiel 3.1 e (Acetonitril statt DMF) wird aus 6.0 g (29.6 mmol) 4-Brom-2-methoxy-phenol und 5.63 g (33.1 mmol) N-(2-Chlorethyl)-pyrrolidin-hydrochlorid das Produkt erhalten.
Ausbeute: 3.96 g (44.6 % d. Theorie)
$C_{13}H_{18}BrNO_2$ (M= 300,198)
ber.: Molpeak $(M+H)^+$: 300/302 gef.: Molpeak $(M+H)^+$: 300/302
Retentionszeit HPLC: 4.67 min (Methode A)

3.5b 1-[2-(4-lod-2-methoxy-phenoxy)-ethyl]-pyrrolidin

Hergestellt nach der allgemeinen Arbeitsvorschrift II aus 1-[2-(4-Brom-2-methoxy-phenoxy)-ethyl]-pyrrolidin (3.90 g, 13.0 mmol).
Ausbeute: 4.19 g (92.9 % d. Theorie)
$C_{13}H_{18}INO_2$ (M= 347,198)
ber.: Molpeak $(M+H)^+$: 348 gef.: Molpeak $(M+H)^+$: 348
Retentionszeit HPLC: 4.65 min (Methode A)

3.5c 5-(4-Chlor-phenyl)-2-[3-methoxy-4-(2-pyrrolidin-1-yl-ethoxy)-phenylethinyl]-pyridin

Hergestellt nach der allgemeinen Arbeitsvorschrift 1 aus 1-[2-(4-lod-2-methoxy-phenoxy)-ethyl]-pyrrolidin (300 mg, 0.86 mmol) und 5-(4-Chlor-phenyl)-2-ethinyl-pyridin (194 mg, 0.91 mmol).
Ausbeute: 106 mg (28.3 % d. Theorie)
$C_{26}H_{25}ClN_2O_2$ (M= 432,954)
ber.: Molpeak $(M+H)^+$: 433/435 gef.: Molpeak $(M+H)^+$: 433/435
Retentionszeit HPLC: 7.44 min (Methode A)

**Beispiel 3.6**

5-(4-Chlor-phenyl)-2-[4-(2-pyrrolid in-1-yl-ethoxy)-3-trifluormethoxy-phenylethinyl]-pyridin

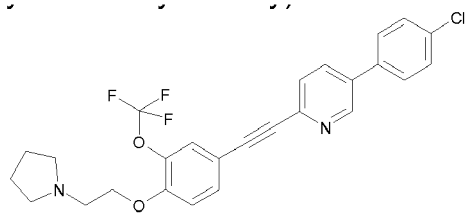

3.6a 4-Brom-2-trifluormethoxy-phenol

Zu einer Lösung von 5.0 g (28.1 mmol) 2-Trifluormethoxy-phenol in 70 mL DCM wird bei -78°C 1.55 mL (30.3 mmol) Brom in 50 mL DCM tropfenweise zugegeben. Die Reaktionslösung wird auf RT erwärmt und weitere 48 h gerührt. Dann werden 70 mL $Na_2SO_3$-Lösung zugegeben und so lange gerührt bis die orange Farbe verschwunden ist. Die Lösung wird mit DCM verdünnt, die organische Phase mit NaCl-Lösung gewaschen, über $MgSO_4$ getrocknet und das Lösungsmittel i.vac. entfernt. Die Reinigung erfolgt durch Säulenchromatographie an Kieselgel (Gradient: PE nach PE: EtOAc 4:1).
Ausbeute: 5.36 g (74.3 % d. Theorie)
$C_7H_4BrF_3O_2$ (M= 257,008)
ber.: Molpeak $(M-H)^-$: 255/257 gef.: Molpeak (M-H)-: 255/257
Retentionszeit HPLC: 8.18 min (Methode A)

3.6b 1-[2-(4-Brom-2-trifluormethoxy-phenoxy)-ethyl]-pyrrolidin

Analog Beispiel 3.1 e (Acetonitril statt DMF) wird aus 2.0 g (7.78 mmol) 4-Brom-2-trifluormethoxy-phenol und 1.53 g (33.1 mmol) N-(2-Chlorethyl)-pyrrolidin-hydrochlorid das Produkt erhalten.
Ausbeute: 0.49 g (17.8 % d. Theorie)
$C_{13}H_{15}$ $BrF_3NO_2$ (M= 354,169)
ber.: Molpeak $(M+H)^+$: 354/356 gef.: Molpeak $(M+H)^+$: 354/356
Retentionszeit HPLC: 5.82 min (Methode A)

3.6c 1-[2-(4-lod-2-trifluormethoxy-phenoxy)-ethyl]-pyrrolidin

Hergestellt nach der allgemeinen Arbeitsvorschrift II aus 1-[2-(4-Brom-2-trifluormethoxy-phenoxy)-ethyl]-pyrrolidin (476 mg, 1.34 mmol).
Ausbeute: 540 mg (100.0 % d. Theorie)
$C_{13}H_{15}$ $F_{3I}NO_2$ (M= 401,170)
ber.: Molpeak $(M+H)^+$: 402 gef.: Molpeak $(M+H)^+$: 402
Retentionszeit HPLC: 6.07 min (Methode A)

3.6d 5-(4-Chlor-phenyl)-2-[4-(2-pyrrolidin-1-yl-ethoxy)-3-trifluormethoxy-phenylethinyl]-pyridin

Hergestellt nach der allgemeinen Arbeitsvorschrift I aus 1-[2-(4-lod-2-trifluormethoxy-phenoxy)-ethyl]-pyrrolidin (250 mg, 0.62 mmol) und 5-(4-Chlor-phenyl)-2-ethinyl-pyridin (140 mg, 0.65 mmol).
Ausbeute: 116 mg (38.5 % d. Theorie)
$C_{26}H_{22}ClF_3N_2O_2$ (M= 486,926)
ber.: Molpeak $(M+H)^+$: 487/489 gef.: Molpeak $(M+H)^+$: 487/489
Retentionszeit HPLC: 8.09 min (Methode A)

Beispiel 3.7

2-[3-Brom-4-(2-pyrrolid in-1-yl-ethoxy)-phenylethinyl]-5-(4-chlor-phenyl)-pyridin

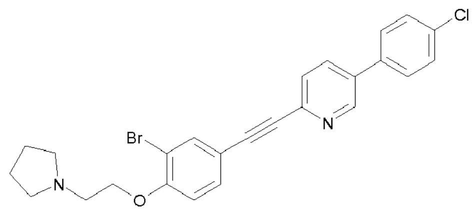

3.7a 2-Brom-4-iod-phenol

Zu einer Lösung von 4.0 g (18.2 mmol) 4-lod-phenol in 35 mL EtOAc wird bei RT 1.55 mL (30.3 mmol) Brom in 15 mL EtOAc tropfenweise zugegeben. Die Reaktionslösung wird 2 h bei RT gerührt. Dann wird 75 mL $Na_2SO_3$-Lösung zugegeben und so lange gerührt bis die orange Farbe verschwunden ist. Die Lösung wird mit DCM verdünnt, die organische Phase mit NaCl-Lösung gewaschen, über $MgSO_4$ getrocknet und das Lösungsmittel i.vac. entfernt. Es wird eine 3.4:1.4:1.0 Mischung an 2-Brom-4-iod-phenol : 2,4-Dibrom-phenol : 4-Brom-phenol erhalten, die ohne weitere Reinigung weiter umgesetzt wird.
Ausbeute an 2-Brom-4-iod-phenol: 2.60 g (47.9 % d. Theorie)
$CgH_4BrIO$ (M= 298,907)
ber.: Molpeak $(M-H)^-$: 297/299 gef.: Molpeak (M-H)-: 297/299
$R_f$-Wert: 0.40 (Kieselgel, EtOAc/MeOH 9:1)

3.7b 1-[2-(2-Brom-4-iod-phenoxy)-ethyl]-pyrrolidin

Analog Beispiel 3.1 e wird aus 1.0 g (1.15 mmol, 59%ig) 2-Brom-4-iod-phenol und 626 mg (3.68 mmol) N-(2-Chlorethyl)-pyrrolidin-hydrochlorid das Produkt erhalten. Es wird eine 4.7:1.0:1.0 Mischung an 1-[2-(2-Brom-4-iod-phenoxy)-ethyl]-pyrrolidin : 1-[2-(2,4-Dibrom-phenoxy)-ethyl]-pyrrolidin : 1-[2-(2-Brom-phenoxy)-ethyl]-pyrrolidin erhalten, die ohne weitere Reinigung weiter umgesetzt wird.
Ausbeute 1-[2-(2-Brom-4-iod-phenoxy)-ethyl]-pyrrolidin: 0.37 g (47.7 % d. Theorie) $C_{12}H_{15}$ BrINO (M= 396,068)
ber.: Molpeak $(M+H)^+$: 397/399 gef.: Molpeak $(M+H)^+$: 397/399
$R_f$-Wert: 0.25 (Kieselgel, EtOAc/MeOH 9:1)

3.7c 2-[3-Brom-4-(2-pyrrolidin-1-yl-ethoxy)-phenylethinyl]-5-(4-chlor-phenyl)-pyridin

Hergestellt nach der allgemeinen Arbeitsvorschrift 1 aus 1-[2-(2-Brom-4-iod-phenoxy)-ethyl]-pyrrolidin (278 mg, 0.34 mmol, 70%) und 5-(4-Chlor-phenyl)-2-ethinyl-pyridin (110 mg, 0.52 mmol).
Ausbeute: 152 mg (64.3 % d. Theorie)
$C_{25}H_{22}BrClN_2O$ (M= 481,824)
ber.: Molpeak $(M+H)^+$: 481/483/485 gef.: Molpeak $(M+H)^+$: 481/483/485
$R_f$-Wert: 0.25 (Kieselgel, DCM/MeOH/$NH_3$ 9:1:0.1)

**Beispiel 3.8**

5-(4-Chlor-phenyl)-2-[4-(2-pyrrolidin-1-yl-ethoxy)-3-trifluormethyl-phenylethinyl]-pyridin

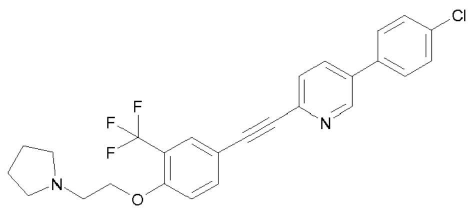

3.8a 4-Brom-2-trifluormethyl-phenol

Eine Lösung von 3.0 g (11.8 mmol) 4-Brom-1-methoxy-2-trifluormethyl-benzol in 20 mL 1 M HBr in Eisessig wird 60 h bei 90°C gerührt. Die Reaktionslösung wird mit 300 mL Wasser verdünnt, mit $K_2CO_s$ auf pH 7 eingestellt. Die wässrige Phase wird mit EtOAc extrahiert, die vereinigten organischen Extrakte mit 40 mL viertel-gesättigter $NaHCO_3$-Lösung gewaschen und über $MgSO_4$ getrocknet. Das Lösungsmittel wird i.vac. entfernt und das Produkt ohne weitere Reinigung weiter umgesetzt.
Ausbeute: 1.20 g (42.3 % d. Theorie)
$C_7H_4BrF_3O$ (M= 241,009)
ber.: Molpeak (M-H)-: 239/241 gef.: Molpeak (M-H)-: 239/241
Retentionszeit HPLC: 8.37 min (Methode A)

3.8b 1-[2-(4-Brom-2-trifluormethyl-phenoxy)-ethyl]-pyrrolidin

Analog Beispiel 3.1 e wird aus 1.20 g (4.98 mmol) 4-Brom-2-trifluormethyl-phenol und 850 mg (5.00 mmol) N-(2-Chlorethyl)-pyrrolidin-hydrochlorid das Produkt erhalten.
Ausbeute: 400 mg (23.8 % d. Theorie)
$C_{13}H_{15}Br\ F_3NO$ (M= 338,170)
ber.: Molpeak $(M+H)^+$: 338/340 gef.: Molpeak $(M+H)^+$: 338/340
Retentionszeit HPLC: 5.91 min (Methode A)

3.8c 1-[2-(4-Iod-2-trifluormethyl-phenoxy)-ethyl]-pyrrolidin

Hergestellt nach der allgemeinen Arbeitsvorschrift II aus 1-[2-(4-Brom-2-trifluormethyl-phenoxy)-ethyl]-pyrrolidin (400 mg, 1.18 mmol).
Ausbeute: 350 mg (76.8 % d. Theorie)
$C_{13}H_{15}\ F_3IN0$ (M= 385,170)
ber.: Molpeak $(M+H)^+$: 386 gef.: Molpeak $(M+H)^+$: 386
Retentionszeit HPLC: 6.01 min (Methode A)

3.8d 5-(4-Chlor-phenyl)-2-[4-(2-pyrrolidin-1-yl-ethoxy)-3-trifluormethyl-phenylethinyl]-pyridin

Hergestellt nach der allgemeinen Arbeitsvorschrift I aus 1-[2-(4-Iod-2-trifluormethyl-phenoxy)-ethyl]-pyrrolidin (180 mg, 0.47 mmol) und 5-(4-Chlor-phenyl)-2-ethinyl-pyridin (83 mg, 0.39 mmol).
Ausbeute: 35 mg (19.1 % d. Theorie)
$C_{26}H_{22}Cl\ F_3N_2O$ (M= 470,926)
ber.: Molpeak $(M+H)^+$: 471/473 gef.: Molpeak $(M+H)^+$: 471/473
Retentionszeit HPLC: 8.23 min (Methode A)

**Beispiel 3.9**

5-(4-Chlor-phenyl)-2-[2-methyl-4-(2-pyrrolidin-1-yl-ethoxy)-phenyleth inyl]-pyridin

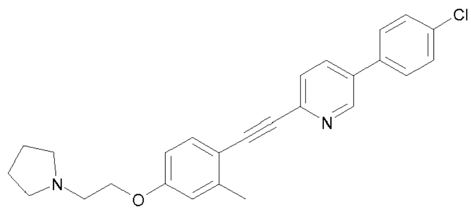

3.9a 1-[2-(4-Brom-3-methyl-phenoxy)-ethyl]-pyrrolidin

Analog Beispiel 3.1 e (Acetonitril statt DMF) wird aus 1.0 g (5.35 mmol) 4-Brom-3-methyl-phenol und 909 mg (5.35 mmol) N-(2-Chlorethyl)-pyrrolidin-hydrochlorid das Produkt erhalten.
Ausbeute: 1.20 g (79.0 % d. Theorie)
$C_{13}H_{18}BrNO$ (M= 284,199)
ber.: Molpeak $(M+H)^+$: 284/286 gef.: Molpeak $(M+H)^+$: 284/286
Retentionszeit HPLC: 3.64 min (Methode B)

3.9b 5-(4-Chlor-phenyl)-2-[2-methyl-4-(2-pyrrolidin-1-yl-ethoxy)-phenylethinyl]-pyridin

Zu einer Lösung von 80 mg (0.37 mmol) 5-(4-Chlor-phenyl)-2-ethinyl-pyridin und 106 mg (0.37 mmol) 1-[2-(4-Brom-3-methyl-phenoxy)-ethyl]-pyrrolidin in 3.0 mL DMF in einer Argonatmosphäre werden nacheinander 0.13 mL (1.00 mmol) Triethylamin, 22 mg (0.02 mmol) Tetrakis-triphenylphosphan-palladium und 3.7 mg (0.02 mmol) CuI gegeben. Die Mischung wird 15 min bei 100 ˚C und 200 Watt in der Mikrowelle gerührt. Die Reaktionslösung wird mit 30 mL EtOAc

verdünnt, mit halbgesättigter $NaHCO_3$-Lösung gewaschen und die organische Phase über $MgSO_4$ getrocknet. Das Lösungsmittel wird i.vac. entfernt und der Rückstand mit *tert*-Butylmethylether verrieben. Das Lösungsmittel wird i.vac. entfernt und die weitere Reinigung mittels HPLC-MS.
Ausbeute: 5.0 mg (3.2 % d. Theorie)
$C_{26}H_{25}ClN_2O$ (M= 416,955)
ber.: Molpeak $(M+H)^+$: 417/419 gef.: Molpeak $(M+H)^+$: 417/419
$R_f$-Wert: 0.38 (Kieselgel, DCM/MeOH/$NH_3$ 9:1:0.1)

**Beispiel 3.10**

5-(4-Chlor-phenyl)-2-[2-chlor-4-(2-pyrrolidin-1-yl-ethoxy)-phenylethinyl]-pyridin

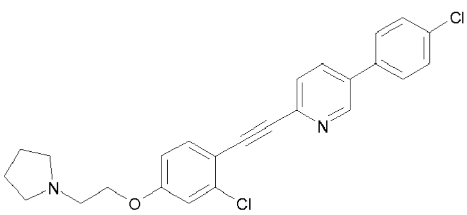

3.10a 1-[2-(4-Brom-3-chlor-phenoxy)-ethyl]-pyrrolidin

Analog Beispiel 3.1 e (Acetonitril statt DMF) wird aus 820 mg (4.82 mmol) 4-Brom-3-chlor-phenol und 1.0 g (4.82 mmol) N-(2-Chlorethyl)-pyrrolidin-hydrochlorid das Produkt erhalten.
Ausbeute: 1.20 g (81.7 % d. Theorie)
$C_{12}H_{15}BrClNO$ (M= 304,616)
ber.: Molpeak $(M+H)^+$: 304/306/308 gef.: Molpeak $(M+H)^+$: 304/306/308
Retentionszeit HPLC: 3.69 min (Methode B)

3.10b 5-(4-Chlor-phenyl)-2-[2-chlor-4-(2-pyrrolidin-1-yl-ethoxy)-phenylethinyl]-pyridin

Unter Argonatmosphäre werden zu einer Lösung von 80 mg (0.37 mmol) 5-(4-Chlor-phenyl)-2-ethinyl-pyridin und 114 mg (0.37 mmol) 1-[2-(4-Brom-3-chlor-phenoxy)-ethyl]-pyrrolidin in 3.0 mL DMF nacheinander 0.13 mL (1.00 mmol) Triethylamin, 22 mg (0.02 mmol) Tetrakis-triphenylphosphan-palladium und 3.7 mg (0.02 mmol) CuI gegeben. Die Mischung wird 15 min bei 100 ˚C und 200 W in der Mikrowelle gerührt. Die Reaktionslösung wird mit 40 mL EtOAc verdünnt, zweimal mit halbgesättigter $NaHC0_3$-Lösung gewaschen und die organische Phase über $MgSO_4$ getrocknet. Das Lösungsmittel wird i.vac. entfernt und der Rückstand mit *tert*-Butylmethylether verrieben. Das Lösungsmittel wird i.vac. entfernt und die weitere Reinigung erfolgt durch Säulenchromatographie mit HPLC-MS.
Ausbeute: 12.0 mg (7.3 % d. Theorie)
$C_{25}H_{22}C1_2N_20$ (M= 437,373)
ber.: Molpeak $(M+H)^+$: 437/439/441 gef.: Molpeak $(M+H)^+$: 437/439/441
Retentionszeit HPLC: 4.91 min (Methode B)

**Beispiel 3.11**

5-(4-Chlor-phenyl)-2-[3-(2-pyrrolidin-1-yl-ethoxy)-phenylethinyl]-pyridin

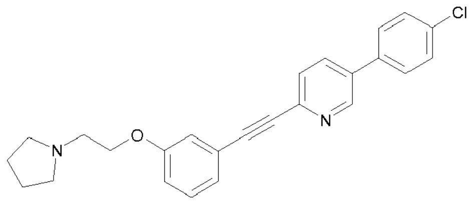

3.11 a 1-[2-(3-Iod-phenoxy)-ethyl]-pyrrolidin

Analog Beispiel 3.1 e (Acetonitril statt DMF) wird aus 1.06 g (4.82 mmol) 3-Iod- phenol und 820 mg (4.82 mmol) N-(2-Chlorethyl)-pyrrolidin-hydrochlorid das Produkt erhalten.
Ausbeute: 1.20 g (78.5 % d. Theorie)
$C_{12}H_{16}INO$ (M= 317,172)
ber.: Molpeak $(M+H)^+$: 318 gef.: Molpeak $(M+H)^+$: 318
Retentionszeit HPLC: 5.01 min (Methode A)

3.11b 5-(4-Chlor-phenyl)-2-[3-(2-pyrrolidin-1-yl-ethoxy)-phenylethinyl]-pyridin

Hergestellt nach der allgemeinen Arbeitsvorschrift I aus 1-[2-(3-Iod-phenoxy)-ethyl]-pyrrolidin (119 mg, 0.37 mmol) und 5-(4-Chlor-phenyl)-2-ethinyl-pyridin (80 mg, 0.37 mmol).
Ausbeute: 14 mg (9.3 % d. Theorie)
$C_{25}H_{23}ClN_2O$ (M= 402,928)
ber.: Molpeak $(M+H)^+$: 403/405 gef.: Molpeak $(M+H)^+$: 403/405
Retentionszeit HPLC: 4.07 min (Methode A)

**Beispiel 3.12**

5-(4-Chlor-phenyl)-2-[3-(3-pyrrolidin-1-yl-propoxy)-phenylethinyl]-pyridin

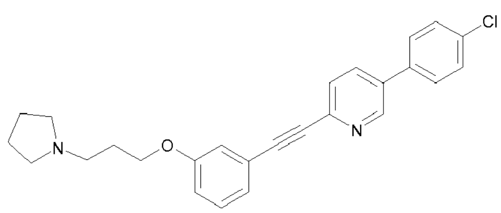

3.12a 1-[3-(3-Iod-phenoxy)-propyl]-pyrrolidin

Analog Beispiel 3.1 e (Acetonitril statt DMF) wird aus 2.7 g (12.2 mmol) 3-Iod-phenol und 1.80 mg (12.2 mmol) N-(3-Chlorpropyl)-pyrrolidin das Produkt erhalten.
Ausbeute: 3.60 g (89.2 % d. Theorie)
$C_{13}H_{18}INO$ (M= 331,199)
ber.: Molpeak $(M+H)^+$: 332 gef.: Molpeak $(M+H)^+$: 332
Retentionszeit HPLC: 5.42 min (Methode A)

3.12b 5-(4-Chlor-phenyl)-2-[3-(3-pyrrolidin-1-yl-propoxy)-phenylethinyl]-pyridin

Hergestellt nach der allgemeinen Arbeitsvorschrift 1 aus 1-[3-(3-Iod-phenoxy)-propyl]-pyrrolidin (124 mg, 0.37 mmol) und 5-(4-Chlor-phenyl)-2-ethinyl-pyridin (80 mg, 0.37 mmol).
Ausbeute: 54 mg (34.6 % d. Theorie)

$C_{26}H_{25}ClN_2O$ (M= 416,955)
ber.: Molpeak $(M+H)^+$: 416/418 gef.: Molpeak $(M+H)^+$: 416/418
Retentionszeit HPLC: 4.99 min (Methode B)

**Beispiel 3.13**

5-(4-Chlor-phenyl)-2-[3-nitro-4-(2-pyrrolid in-1-yl-ethoxy)-phenylethinyl]-pyridin

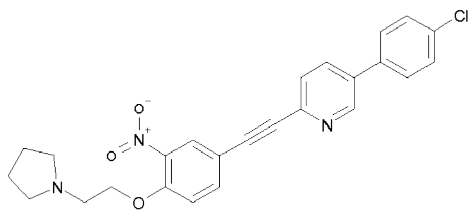

3.13a 1-[2-(4-Brom-2-nitro-phenoxy)-ethyl]-pyrrolidin

Analog Beispiel 3.1 e (Acetonitril statt DMF) wird aus 10.5 g (48.2 mmol) 4-Brom-3-nitrophenol und 8.2 mg (48.2 mmol) N-(2-Chlorethyl)-pyrrolidin-hydrochlorid das Produkt erhalten.
Ausbeute: 1.0 g (6.6 % d. Theorie)
$C_{12}H_{15}BrN_2O_3$ (M= 315,17)
ber.: Molpeak $(M+H)^+$: 315/317 gef.: Molpeak $(M+H)^+$: 315/317
$R_f$-Wert: 0.30 (Kieselgel, DCM/MeOH/$NH_3$ 9:1:0.1)

3.13b 1-[2-(4-lod-2-nitro-phenoxy)-ethyl]-pyrrolidin

Hergestellt nach der allgemeinen Arbeitsvorschrift II aus 1-[2-(4-Brom-2-nitro-phenoxy)-ethyl]-pyrrolidin (1.0 g, 2.22 mmol).
Ausbeute: 600 mg (74.6 % d. Theorie)
$C_{12}H_{15}IN_20_3$ (M= 362,17)
ber.: Molpeak $(M+H)^+$: 363 gef.: Molpeak $(M+H)^+$: 363
$R_f$-Wert: 0.35 (Kieselgel, DCM/MeOH/$NH_3$ 9:1:0.1)

3.13c 5-(4-Chlor-phenyl)-2-[3-nitro-4-(2-pyrrolidin-1-yl-ethoxy)-phenylethinyl]-pyridin

Hergestellt nach der allgemeinen Arbeitsvorschrift I aus 1-[2-(4-lod-2-nitro-phenoxy)-ethyl]-pyrrolidin (600 mg, 1.66 mmol) und 5-(4-Chlor-phenyl)-2-ethinyl-pyridin (363 mg, 1.70 mmol).
Ausbeute: 100 mg (13.1 % d. Theorie)
$C_{25}H_{22}ClN_3O_3$ (M= 447,93)
ber.: Molpeak $(M+H)^+$: 448/450 gef.: Molpeak $(M+H)^+$: 448/450
$R_f$-Wert: 0.35 (Kieselgel, DCM/MeOH/$NH_3$ 9:1:0.1)

Beispiel 3.14

5-[5-(4-Chlor-phenyl)-pyridin-2-ylethinyl]-3-methyl-2-(2-pyrrolidin-1-yl-ethoxy)-benzoesäuremethylester

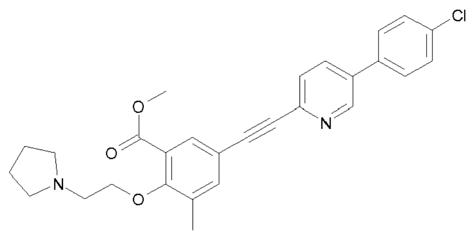

3.14a 2-Hydroxy-5-iod-3-methyl-benzoesäure

Zu einer Lösung von 4.0 g (24.1 mmol) 2-Hydroxy-3-methyl-benzoesäuremethylester, 3.6 g (24.1 mmol) NaI, 0.96 g (24.1 mmol) NaOH in 100 mL MeOH wird bei -5˚C über 40 min 14.9 mL (24.1 mmol) Natriumhypochlorit-Lösung (10 Gewichtsprozent in Wasser) zugetropft. Die Reaktion wird 30 min bei -5˚C und 5 Tage bei RT gerührt. Das Lösungsmittel wird i.vac. entfernt und der Rückstand wird in 80 mL Wasser und 50 mL DCM aufgenommen. Nach Sättigung der organischen Phase mit NaCl wird diese zweimal mit DCM extrahiert. Die vereinigten organischen Extrakte werden filtriert und das Lösungsmittel i.vac. entfernt. Das Produkt wird ohne weitere Reinigung weiter umgesetzt.
Ausbeute: 7.25 g (108 % d. Theorie)
$C_8H_7IO_3$ (M= 278,048)
ber.: Molpeak $(M+H)^+$: 279 gef.: Molpeak $(M+H)^+$: 279
Retentionszeit HPLC: 8.41 min (Methode A)

3.14b 2-Hydroxy-5-iod-3-methyl-benzoesäuremethylester

Eine Lösung von 2.0 g (7.19 mmol) 2-Hydroxy-5-iod-3-methyl-benzoesäure in 5.0 mL Thionylchlorid (69.0 mmol) wird 20 min bei 80˚C gerührt. Thionylchlorid wird i.vac. entfernt und der Rückstand mit 20 mL MeOH versetzt und 20 min bei RT gerührt. Das Produkt fällt aus der Reaktion aus. MeOH wird bis auf 5 mL i.vac. entfernt und der Rückstand wird abgesaugt. Das Produkt wird ohne weitere Reinigung weiter umgesetzt.
Ausbeute: 1.14 g (54.3 % d. Theorie)
$C_9H_9IO_3$ (M= 292,075)
ber.: Molpeak (M-H)-: 291 gef.: Molpeak (M-H)-: 291
$R_f$-Wert: 0.96 (Kieselgel, EtOAc)

3.14c 5-Iod-3-methyl-2-(2-pyrrolidin-1-yl-ethoxy)-benzoesäuremethylester

Analog Beispiel 3.1 e wird aus 1.1 g (3.77 mmol) 2-Hydroxy-5-iod-3-methyl-benzoesäure-methylester und 641 mg (3.77 mmol) N-(2-Chlorethyl)-pyrrolidin-hydrochlorid das Produkt erhalten.
Ausbeute: 347 mg (23.7 % d. Theorie)
$C_{15}H_{20}INO_3$ (M= 389,236)
ber.: Molpeak $(M+H)^+$: 390 gef.: Molpeak $(M+H)^+$: 390
Retentionszeit HPLC: 6.20 min (Methode A)

3.14d 5-[5-(4-Chlor-phenyl)-pyridin-2-ylethinyl]-3-methyl-2-(2-pyrrolidin-1-yl-ethoxy)-benzoesäuremethylester

Hergestellt nach der allgemeinen Arbeitsvorschrift I aus 5-Iod-3-methyl-2-(2-pyrrolidin-1-yl-ethoxy)-benzoesäuremethylester (150 mg, 0.39 mmol) und 5-(4-Chlor-phenyl)-2-ethinyl-pyridin (112 mg, 0.53 mmol).
Ausbeute: 31 mg (17.1 % d. Theorie)
$C_{28}H_{27}ClN_2O_3$ (M= 474,992)
ber.: Molpeak $(M+H)^+$: 475/477 gef.: Molpeak $(M+H)^+$: 475/477
Retentionszeit HPLC: 8.11 min (Methode A)

Beispiel 3.15

5-(4-Chlor-phenyl)-2-[2-(2-pyrrolidin-1-yl-ethoxy)-pyrid-5-yl-ethinyl]-pyridin

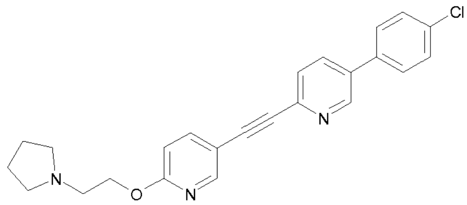

3.15a 5-Brom-2-(2-pyrrolidin-1-yl-ethoxy)-pyridin

Zu einer Lösung von 0.76 mL (6.14 mmol) N-(2-Hydroxyethyl)pyrrolidin in 20 mL DMF werden bei RT 280 mg (7.00 mmol, 60%) NaH gegeben. Die Reaktionslösung wird 45 min bei RT gerührt und dann 1.35 g (5,53 mmol) 2,5-Dibrompyridin zugegeben. Die Lösung wird 16 h bei 70˚C gerührt und das Lösungsmittel i.vac. entfernt. Der Rückstand wird in 100 mL EtOAc und 50 mL Wasser aufgenommen und die organische Phase wird mit 40 mL gesättigter NaCl-Lösung extrahiert. Die organische Phase wird über $Na_2S0_4$ getrocknet und das Lösungsmittel i.vac. entfernt. Die weitere Reinigung erfolgt durch Säulenchromatographie an Kieselgel (Gradient: Cyc/EtOAc 1:1 nach EtOAc).
Ausbeute: 926 mg (61.8 % d. Theorie)
$C_{11}H_{15}BrN_2O$ (M= 271,159)
ber.: Molpeak $(M+H)^+$: 271/273 gef.: Molpeak $(M+H)^+$: 271/273
$R_f$-Wert: 0.05 (Kieselgel, Cyc/EtOAc 2:1)

3.15b 5-(4-Chlor-phenyl)-2-[2-(2-pyrrolidin-1-yl-ethoxy)-pyrid-5-yl-ethinyl]-pyridin

Hergestellt nach der allgemeinen Arbeitsvorschrift I aus 5-Brom-2-(2-pyrrolidin-1-yl-ethoxy)-pyridin (90 mg, 0.33 mmol) und 5-(4-Chlor-phenyl)-2-ethinyl-pyridin (80 mg, 0.37 mmol).
Ausbeute: 19 mg (13.8 % d. Theorie)
$C_{24}H_{22}ClN_3O$ (M= 403,915)
ber.: Molpeak $(M+H)^+$: 404/406 gef.: Molpeak $(M+H)^+$: 404/406
$R_f$-Wert: 0.38 (Kieselgel, DCM/MeOH/$NH_3$ 9:1:0.1)

**Beispiel 3.16**

5-[5-(4-Chlor-phenyl)-pyridin-2-ylethinyl]-2-(2-pyrrolidin-1-yl-ethoxy)-pyrimidin

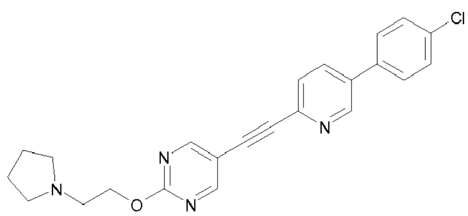

3.16a 5-Brom-2-(2-pyrrolidin-1-yl-ethoxy)-pyrimidin

Zu einer Lösung von 0.17 mL (1.38 mmol) N-(2-Hydroxyethyl)pyrrolidin in 10 mL THF werden bei RT 50 mg (1.15 mmol, 60%) NaH gegeben. Die Reaktionslösung wird 15 min bei RT gerührt und dann werden 200 mg (1.03 mmol) 5-Brom-2-chlorpyrimidin zugegeben. Die Lösung wird 16 h bei RT gerührt. 10 mL Wasser werden zugegeben und die wässrige Phase mit 20 mL EtOAc extrahiert. Die organische Phase wird über $Na_2S0_4$ getrocknet und das Lösungsmittel i.vac. entfernt. Die weitere Reinigung erfolgt durch Säulenchromatographie an Kieselgel (DCM/MeOH/$NH_3$ 9:1:0.1).
Ausbeute: 200 mg (71.1 % d. Theorie)
$C_{10}H_{14}BrN_3O$ (M= 272,147)
ber.: Molpeak $(M+H)^+$: 272/274 gef.: Molpeak $(M+H)^+$: 272/274

$R_f$-Wert: 0.47 (Kieselgel, DCM/MeOH/$NH_3$ 9:1:0.1)

3.16b 5-[5-(4-Chlor-phenyl)-pyridin-2-ylethinyl]-2-(2-pyrrolidin-1-yl-ethoxy)-pyrimidin

Unter Argonatmosphäre werden zu einer Lösung von 56 mg (0.26 mmol) 5-(4-Chlor-phenyl)-2-ethinyl-pyridin und 71 mg (0.26 mmol) 5-Brom-2-(2-pyrrolidin-1-yl-ethoxy)-pyrimidin in 3.0 mL DMF nacheinander 0.11 mL (0.79 mmol) Triethylamin, 7 mg (0.01 mmol) Tetrakis-triphenylphosphan-palladium und 1.3 mg (0.01 mmol) CuI gegeben. Die Mischung wird 20 min bei 100 ˚C und 300 Watt in der Mikrowelle gerührt. Die Reaktionslösung wird mit 10 mL Wasser verdünnt und die wässrige Phase mit 20 mL

EtOAc extrahiert. Die organische Phase wird mit gesättigter NaCl-Lösung extrahiert und über $Na_2SO_4$ getrocknet. Das Lösungsmittel i.vac. entfernt und die weitere Reinigung erfolgt durch Säulenchromatographie mit HPLC-MS.
Ausbeute: 7 mg (6.6 % d. Theorie)
$C_{23}H_21ClN_4O$ (M= 404,903)
ber.: Molpeak $(M+H)^+$: 405/407 gef.: Molpeak $(M+H)^+$: 405/407
$R_f$-Wert: 0.39 (Kieselgel, DCM/MeOH/$NH_3$ 9:1:0.1)

**Beispiel 3.17**

3-[5-(4-Chlor-phenyl)-pyridin-2-ylethinyl]-6-(2-pyrrolidin-1-yl-ethoxy)-pyridazin

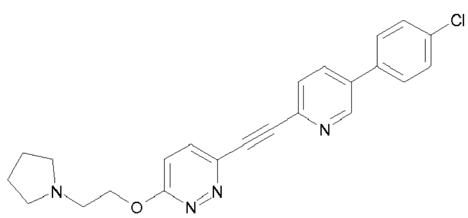

3.17a 3-Chlor-6-(2-pyrrolidin-1-yl-ethoxy)-pyridazin

Zu einer Lösung von 0.50 mL (4.04 mmol) N-(2-Hydroxyethyl)pyrrolidin in 50 mL THF werden bei 0˚C 175 mg (4.01 mmol, 55%) NaH gegeben. Die Reaktionslösung wird 60 min gerührt und dabei auf RT erwärmt. 500 mg (3.26 mmol) 3,6-Dichlor-pyridazin werden zugegeben. Die Lösung wird 5 h bei RT gerührt. 50 mL Wasser werden zugegeben und die wässrige Phase mit 100 mL EtOAc extrahiert. Die organische Phase wird einmal mit gesättigter NaCl-Lösung extrahiert und über $Na_2SO_4$ getrocknet. Das Lösungsmittel wird i.vac. entfernt und die weitere Reinigung erfolgt durch Säulenchromatographie an Kieselgel (Gradient: EtOAc nach EtOAc/Me0H/$NH_3$ 9:1:0.1).
Ausbeute: 652 mg (87.9 % d. Theorie)
$C_{10}H_{14}ClN_3O$ (M= 227,696)
ber.: Molpeak $(M+H)^+$: 228/230 gef.: Molpeak $(M+H)^+$: 228/230
$R_f$-Wert: 0.45 (Kieselgel, EtOAc/Me0H/$NH_3$ 9:1:0.1)

3.17b 3-[5-(4-Chlor-phenyl)-pyridin-2-ylethinyl]-6-(2-pyrrolidin-1-yl-ethoxy)-pyridazin

Unter Argonatmosphäre werden zu einer Lösung von 57 mg (0.27 mmol) 5-(4-Chlor-phenyl)-2-ethinyl-pyridin und 61 mg (0.26 mmol) 3-Chlor-6-(2-pyrrolidin-1-yl-ethoxy)-pyridazin in 3.0 mL DMF nacheinander 0.11 mL (0.79 mmol) Triethylamin, 4 mg (0.01 mmol) Chlor(di-2-norbornylphosphino)(2'-dimethylamino-1-1 '-biphenyl-2-yl)palladium(II) und 1.2 mg (0.01 mmol) CuI gegeben. Die Mischung wird 20 min bei 100 ˚C und 300 Watt in der Mikrowelle gerührt. Die Reaktionslösung wird mit 10 mL Wasser verdünnt und die wässrige Phase mit 20 mL EtOAc extrahiert. Die organische Phase wird mit gesättigter NaCl-Lösung extrahiert und über $Na_2SO_4$ getrocknet. Das Lösungsmittel wird i.vac. entfernt und die weitere Reinigung erfolgt durch Säulenchromatographie mit HPLC-MS.
Ausbeute: 3 mg (2.9 % d. Theorie)
$C_{23}H_{21}ClN_4O$ (M= 404,903)
ber.: Molpeak $(M+H)^+$: 405/407 gef.: Molpeak $(M+H)^+$: 405/407
Retentionszeit HPLC: 6.39 min (Methode A)

**Beispiel 3.18**

5-(4-Chlor-phenyl)-2-[4-(1-ethyl-piperidin-3-yloxy)-phenylethinyl]-pyridin

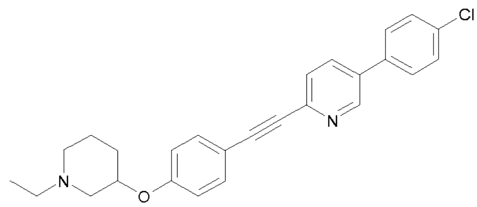

3.18a 3-(4-Brom-phenoxy)-1-ethyl-piperidin

Zu einer Lösung von 289 mg (1.00 mmol) 1-Brom-4-iodbenzol und 0.27 mL (2.00 mmol) N-Ethyl-3-hydroxypiperidin in 1.0 mL Toluol werden 652 mg (2.00 mmol) Cäsiumcarbonat, 36 mg (0.20 mmol) 1,10-Phenanthrolin und 19 mg (0.10 mmol) CuI gegeben. Die Reaktionsmischung wird 36 h bei 110°C gerührt und dann mit 10 mL Wasser und 10 mL EtOAc versetzt. Nach Filtration wird die wässrige Phase mit 10 mL EtOAc extrahiert und die vereinigten organischen Extrakte werden mit gesättigter NaCl-Lösung gewaschen und über $Na_2SO_4$ getrocknet. Das Lösungsmittel wird i.vac. entfernt und die weitere Reinigung erfolgt durch Säulenchromatographie an Kieselgel (EtOAc).
Ausbeute: 100 mg (35.2 % d. Theorie)
$C_{13}H_{18}BrNO$ (M= 284,199)
ber.: Molpeak $(M+H)^+$: 284/286 gef.: Molpeak $(M+H)^+$: 284/286
$R_f$-Wert: 0.50 (Kieselgel, EtOAc)

3.18b 5-(4-Chlor-phenyl)-2-[4-(1-ethyl-piperidin-3-yloxy)-phenylethinyl]-pyridin

Hergestellt nach der allgemeinen Arbeitsvorschrift I aus 3-(4-Brom-phenoxy)-1-ethylpiperidin (90 mg, 0.32 mmol) und 5-(4-Chlor-phenyl)-2-ethinyl-pyridin (68 mg, 0.32 mmol).
Ausbeute: 24 mg (18.1 % d. Theorie)
$C_{26}H_{25}ClN_2O$ (M= 416,955)
ber.: Molpeak $(M+H)^+$: 417/419 gef.: Molpeak $(M+H)^+$: 417/419
$R_f$-Wert: 0.69 (Kieselgel, EtOAc/Me0H/$NH_3$ 9:1:0.1)

**Beispiel 3.19**

(S)-3-{4-[5-(4-Chlor-phenyl)-pyridin-2-ylethinyl]-phenoxyl-l-aza-bicyclo[2.2.2]octan

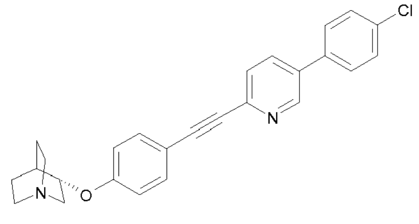

3.19a (S)-3-(4-Brom-phenoxy)-1-aza-bicyclo[2.2.2]octan

Analog Beispiel 3.18a wird aus 577 mg (2.00 mmol) 1-Brom-4-iodbenzol und 254 mg (2.00 mmol) (S)-(+)-3-Hydroxychinuclidin das Produkt erhalten.
Ausbeute: 170 mg (30.1 % d. Theorie)
$C_{13}H_{16}BrNO$ (M= 282,183)
ber.: Molpeak $(M+H)^+$: 282/284 gef.: Molpeak $(M+H)^+$: 282/284
$R_f$-Wert: 0.28 (Kieselgel, EtOAc/MeOH/$NH_3$ 9:1:0.1)

3.19b (*S*)-3-{4-[5-(4-Chlor-phenyl)-pyridin-2-ylethinyl]-phenoxy}-1-aza-bicyclo[2.2.2]octan

Hergestellt nach der allgemeinen Arbeitsvorschrift I aus (*S*)-3-(4-Brom-phenoxy)-1-aza-bicyclo[2.2.2]octan (170 mg, 0.62 mmol) und 5-(4-Chlor-phenyl)-2-ethinyl-pyridin (100 mg, 0.47 mmol).
Ausbeute: 3.4 mg (1.8 % d. Theorie)
$C_{26}H_{23}ClN_2O$ (M= 414,939)
ber.: Molpeak $(M+H)^+$: 415/417 gef.: Molpeak $(M+H)^+$: 415/417
$R_f$-Wert: 0.11 (Kieselgel, EtOAc/Me0H/$NH_3$ 9:1:0.1)

**Beispiel 3.20**

(2-{4-[5-(4-Chlor-phenyl)-pyridin-2-ylethinyl]-phenoxy}-ethyl)-pyridin-4-yl-amin

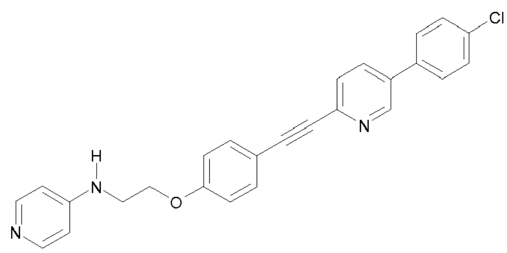

3.20a Pyridin-4-yl-[2-(tetrahydro-pyran-2-yloxy)-ethyl]-carbaminsäure-*tert*-butylester

Zu einer Lösung von 1.50 g (7.72 mmol) Pyridin-4-yl-carbaminsäure-tert-butylester in 80 mL DMF wird bei 0˚C 309 mg (7.72 mmol, 60%) NaH gegeben. Die Reaktion wird 1 h gerührt und dabei auf RT erwärmt. 2.09 g (10.00 mmol) 2-(2-Bromethoxy)tetrahydro-2H-pyran in 20 mL DMF wird innerhalb von 10 min zugegeben. Die Reaktionsmischung wird 16 h bei RT gerührt und mit 50 mL Wasser und 100 mL EtOAc versetzt. Die organische Phase wird über $Na_2SO_4$ getrocknet und das Lösungsmittel i.vac. entfernt. Die weitere Reinigung erfolgt durch Säulenchromatographie an Kieselgel (Cyc/EtOAc 7:3).
Ausbeute: 1.08 g (43.4 % d. Theorie)
$C_{17}H_{26}BrN_2O_4$ (M= 322,408)
$R_f$-Wert: 0.25 (Kieselgel, EtOAc/Cyc 8:2)

3.20b 2-(Pyridin-4-ylamino)-ethanol

Zu einer Lösung von 1.08 g (3.35 mmol) Pyridin-4-yl-[2-(tetrahydro-pyran-2-yloxy)-ethyl]-carbaminsäure-tert-butylester in DCM wird bei 0˚C Trifluoressigsäure zugegeben, auf RT erwärmt und 16 h gerührt. Die Reaktionsmischung wird auf 0˚C abgekühlt und mit gesättigter $K_2C0_3$-Lösung alkalisch gestellt. Die wässrige Phase wird mit 50 mL EtOAc extrahiert, die organische Phase über $Na_2SO_4$ getrocknet und das Lösungsmittel i.vac. entfernt. Die weitere Reinigung erfolgt durch Säulenchromatographie an Kieselgel (EtOAc/MeOH/$NH_3$ 9:1:0.1).
Ausbeute: 120 mg (25.9 % d. Theorie)
$C_7H_{10}N_2O$ (M= 138,171)
ber.: Molpeak $(M+H)^+$: 139 gef.: Molpeak $(M+H)^+$: 139
$R_f$-Wert: 0.18 (Kieselgel, EtOAc/Me0H/$NH_3$ 9:1:0.1)

3.20c [2-(4-Brom-phenoxy)-ethyl]-pyridin-4-yl-amin

Analog Beispiel 3.18a wird aus 251 mg (0.87 mmol) 1-Brom-4-iodbenzol und 120 mg (0.86 mmol) 2-(Pyridin-4-ylamino)-ethanol das Produkt erhalten.
Ausbeute: 90 mg (35,4 % d. Theorie)
$C_{13}H_{13}BrN_2O$ (M= 293,165)
ber.: Molpeak $(M+H)^+$: 293/295 gef.: Molpeak $(M+H)^+$: 293/295
$R_f$-Wert: 0.50 (Kieselgel, EtOAc/Me0H/$NH_3$ 9:1:0.1)

3.20d [2-(4-lod-phenoxy)-ethyl]-pyridin-4-yl-amin

Hergestellt nach der allgemeinen Arbeitsvorschrift II aus [2-(4-Brom-phenoxy)-ethyl]-pyridin-4-yl-amin (90 mg, 0.31 mmol).
Ausbeute: 95 mg (91.0 % d. Theorie)
$C_{13}H_{13}1N_2O$ (M= 340,166)
Retentionszeit HPLC: 5.86 min (Methode A)

3.20e (2-{4-[5-(4-Chlor-phenyl)-pyridin-2-ylethinyl]-phenoxy}-ethyl)-pyridin-4-yl-amin

Hergestellt nach der allgemeinen Arbeitsvorschrift I aus [2-(4-lod-phenoxy)-ethyl]-pyridin-4-yl-amin (95 mg, 0.28 mmol) und 5-(4-Chlor-phenyl)-2-ethynyl-pyridin (70 mg, 0.33 mmol).
Ausbeute: 30 mg (25.2 % d. Theorie)
$C_{26}H_{20}ClN_3O$ (M= 425,922)
ber.: Molpeak $(M+H)^+$: 426/428 gef.: Molpeak $(M+H)^+$: 426/428
$R_f$-Wert: 0.38 (Kieselgel, EtOAc/MeOH/$NH_3$ 9:1:0.1)

**Beispiel 3.21**

5-(4-Chlor-phenyl)-2-{4-[2-(2,2,6,6-tetramethyl-piperidin-1-yl)-ethoxy]-phenylethinyl}-pyridin

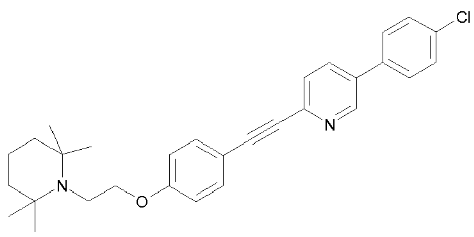

3.21a 1-[2-(4-lod-phenoxy)-ethyl]-2,2,6,6-tetramethyl-piperidin

Analog Beispiel 3.1 e wird aus 500 mg (2.27 mmol) 4-lod-phenol und 500 mg (2.08 mmol) 1-(2-Chlor-ethyl)-2,2,6,6-tetramethyl-piperidin das Produkt erhalten.
Ausbeute: 673 mg (83.5 % d. Theorie)
$C_{17}H_{26}INO$ (M= 387,307)
ber.: Molpeak $(M+H)^+$: 388 gef.: Molpeak $(M+H)^+$: 388
$R_f$-Wert: 0.79 (Kieselgel, Cyc/EtOAc 4:1)

3.21 b 5-(4-Chlor-phenyl)-2-{4-[2-(2,2,6,6-tetramethyl-piperidin-1-yl)-ethoxy]-phenylethinyl}-pyridin

Hergestellt nach der allgemeinen Arbeitsvorschrift I aus 1-[2-(4-lod-phenoxy)-ethyl]-2,2,6,6-tetramethyl-piperidin (260 mg, 0.67 mmol) und 5-(4-Chlor-phenyl)-2-ethinyl-pyridin (155 mg, 0.73 mmol).
Ausbeute: 31 mg (9.8 % d. Theorie)
$C_{30}H_{33}ClN_2O$ (M= 473,063)
ber.: Molpeak $(M+H)^+$: 473/475 gef.: Molpeak $(M+H)^+$: 473/475
$R_f$-Wert: 0.21 (Kieselgel, Cyc/EtOAc 3:1)

**Beispiel 3.22**

5-(4-Chlor-phenyl)-2-[4-(3-pyrrolidin-1 -yl-propyl)-phenylethinyl]-pyridin

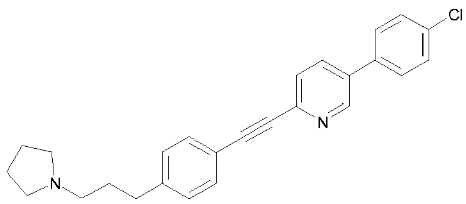

3.22a 3-(4-Brom-phenyl)-propionaldehyd

Zu einer Lösung von 5.0 g (17,32 mmol) 4-Brom-iod-benzol und 3.0 mL (43.67 mmol) Allylalkohol in 30 mL DMF werden 210 mg (0.86 mmol) $Pd(OAc)_2$, 5.23 g (17.32 mmol) Tetra-n-butylammoniumchlorid und 3.6 g $NaHCO_3$ gegeben. Die Reaktionslösung wird 2 h bei 60˚C gerührt und mit 50 mL Wasser verdünnt. Die wässrige Phase wird mit 50 mL EtOAc extrahiert und die vereinigten organischen Extrakte mit 50 mL gesättigter NaCl-Lösung gewaschen. Die organische Phase wird über $Na_2SO_4$ getrocknet und das Lösungsmittel i.vac. entfernt. Die weitere Reinigung erfolgt durch Säulenchromatographie an Kieselgel (Cyc/EtOAc 3:1 ).
Ausbeute: 2.48 g (67.2 % d. Theorie)
$C_9H_9BrO$ (M= 213,075)
ber.: Molpeak $(M-H)^-$: 211/213 gef.: Molpeak (M-H)-: 211/213
$R_f$-Wert: 0.43 (Kieselgel, Cyc/EtOAc 4:1)

3.22b 1-[3-(4-Brom-phenyl)-propyl]-pyrrolidin

Eine Lösung von 1.03 g (4.82 mmol) 3-(4-Brom-phenyl)-propionaldehyd und 0.41 mL (4.82 mmol) Pyrrolidin in 50 mL MeOH wird mit Eisessig auf pH 4-5 eingestellt. Dann werden 400 mg (6.05 mmol) $NaBH_3CN$ portionsweise zugegeben und die Reaktion 3 Tage bei RT gerührt. Die Reaktionslösung wird mit 30 mL Wasser verdünnt und die wässrige Phase mit 50 mL EtOAc extrahiert. Die organische Phase wird über $Na_2SO_4$ getrocknet und das Lösungsmittel i.vac. entfernt. Die weitere Reinigung erfolgt durch Säulenchromatographie an Kieselgel (EtOAc/Me0H/$NH_3$ 9:1:0.1).
Ausbeute: 1.06 g (82.1 % d. Theorie)
$C_{13}H_{18}BrN$ (M= 268,199)
ber.: Molpeak $(M+H)^+$: 268/270 gef.: Molpeak $(M+H)^+$: 268/270
$R_f$-Wert: 0.50 (Kieselgel, EtOAc/Me0H/$NH_3$ 9:1:0.1)

3.22c 5-(4-Chlor-phenyl)-2-[4-(3-pyrrolidin-1-yl-propyl)-phenylethinyl]-pyridin

Analog Beispiel 3.16b ($Pd(PPh_3)_2Cl_2$ anstatt Tetrakis-triphenylphosphan-palladium) wird aus 72 mg (0.27 mmol) 1-[3-(4-Brom-phenyl)-propyl]-pyrrolidin und 57 mg (0.27 mmol) 5-(4-Chlor-phenyl)-2-ethinyl-pyridin das Produkt erhalten.
Ausbeute: 10 mg (9,6 % d. Theorie)
$C_{26}H_{25}ClN_2$ (M= 400,956)
ber.: Molpeak $(M+H)^+$: 401/403 gef.: Molpeak $(M+H)^+$: 401/403
Retentionszeit HPLC: 6.94 min (Methode A)

Beispiel 3.23

5-(4-Chlor-phenyl)-2-[4-(2-pyrrolidin-1-yl-ethyl)-phenylethinyl]-pyridin

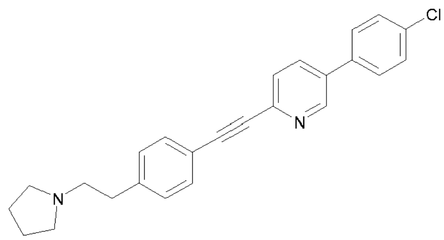

3.23a 1-[2-(4-Brom-phenyl)-ethyl]-pyrrolidin

Zu einer Lösung von 1.0 g (4.23 mmol) 4-Brom-phenethylamin Hydrochlorid, 1.8 g (13.0 mmol) $K_2C0_3$ und 200 mg (1.20 mmol) KI in 100 mL Acetonitril wird bei 75˚C langsam 0.51 mL (4.23 mmol) 1,4-Dibrombutan in 20 mL Acetonitril zugegeben und die Reaktionsmischung weitere 4 h bei 75˚C gerührt. Die Reaktionslösung wird mit 100 mL Wasser verdünnt und die wässrige Phase mit 100 mL EtOAc extrahiert. Die vereinigten organischen Extrakte werden mit gesättigter NaCl-Lösung gewaschen, die organische Phase über $Na_2SO_4$ getrocknet und das Lösungsmittel i.vac. entfernt. Die weitere Reinigung erfolgt durch Säulenchromatographie an Kieselgel (EtOAc/MeOH/$NH_3$ 9:1:0.1)
Ausbeute: 540 mg (50.2 % d. Theorie)
$C_{12}H_{16}BrN$ (M= 254,172)
ber.: Molpeak $(M+H)^+$: 254/256 gef.: Molpeak $(M+H)^+$: 254/256
$R_f$-Wert: 0.54 (Kieselgel, EtOAc/MeOH/$NH_3$ 9:1:0.1)

3.23b 5-(4-Chlor-phenyl)-2-[4-(2-pyrrolidin-1-yl-ethyl)-phenylethinyl]-pyridin

Analog Beispiel 3.16b wird aus 60 mg (0.23 mmol) 1-[2-(4-Brom-phenyl)-ethyl]-pyrrolidin und 50 mg (0.23 mmol) 5-(4-Chlor-phenyl)-2-ethinyl-pyridin das Produkt erhalten.
Ausbeute: 8 mg (8.7 % d. Theorie)
$C_{25}H_{23}ClN_2$ (M= 386,928)
ber.: Molpeak $(M+H)^+$: 387/389 gef.: Molpeak $(M+H)^+$: 387/389
Retentionszeit HPLC: 6.45 min (Methode A)

**Beispiel 3.24**

1 -{5-[5-(4-Chlor-phenyl)-pyridin-2-ylethinyl]-pyridin-2-yl}-4-methyl-[1,4]diazepan

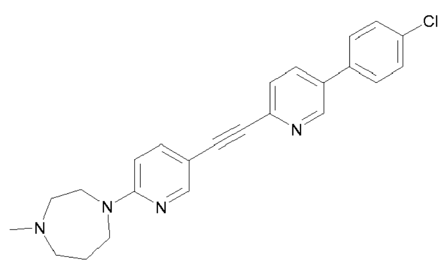

3.24a 1-(5-Brom-pyridin-2-yl)-4-methyl-[1,4]diazepan

1.5 g (5.29 mmol) 5-Brom-2-iodbenzol und 1.5 mL (11.7 mmol) 1-Methylhomopiperazin werden 1.5 h auf 170˚C erwärmt. Nach Abkühlung des Reaktionsgemisches werden 40 mL halbgesättigte $NaHCO_3$-Lösung und 100 mL EtOAc zugegeben. Die organische Phase wird über $Na_2SO_4$ getrocknet und das Lösungsmittel i.vac. entfernt. Die weitere Reinigung erfolgt durch Säulenchromatographie an Kieselgel (EtOAc/MeOH/$NH_3$ 85:15:1).
Ausbeute: 1.10 g (77.1 % d. Theorie)
$C_{11}H_{16}BrN_3$ (M= 270,174)
ber.: Molpeak $(M+H)^+$: 270/272 gef.: Molpeak $(M+H)^+$: 270/272

$R_f$-Wert: 0.57 (Kieselgel, EtOAc/MeOH/$NH_3$ 8:2:0.2)

3.24b 1-(5-Iod-pyridin-2-yl)-4-methyl-[1,4]diazepan

Hergestellt nach der allgemeinen Arbeitsvorschrift II aus 1-(5-Brom-pyridin-2-yl)-4-methyl-[1,4]diazepan (472 mg, 1.75 mmol).
Ausbeute: 546 mg (98.5 % d. Theorie)
$C_{11}H_{16}IN_3$ (M= 317,175)
Retentionszeit HPLC: 4.56 min (Methode A)

3.24c 1-{5-[5-(4-Chlor-phenyl)-pyridin-2-ylethinyl]-pyridin-2-yl-4-methyl-[1,4]diazepan

Hergestellt nach der allgemeinen Arbeitsvorschrift I aus 1-(5-Iod-pyridin-2-yl)-4-methyl-[1,4]diazepan (237 mg, 0.75 mmol) und 5-(4-Chlor-phenyl)-2-ethinyl-pyridin (161 mg, 0.75 mmol).
Ausbeute: 54 mg (17.9 % d. Theorie)
$C_{24}H_{23}ClN_4$ (M= 402,931)
ber.: Molpeak $(M+H)^+$: 403/405 gef.: Molpeak $(M+H)^+$: 403/405
Retentionszeit HPLC: 6.79 min (Methode A)

**Beispiel 3.25**

1-{5-[5-(4-Chlor-phenyl)-pyridin-2-ylethinyl]-pyridin-2-yl}-4-methyl-piperazin

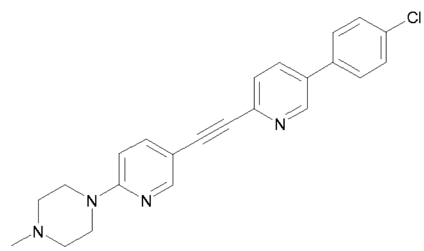

3.25a 1-(5-Brom-pyridin-2-yl)-4-methyl-piperazin

Analog Beispiel 3.24a wird aus 1.5 g (5.28 mmol) 5-Brom-2-iodpyridin und 1.3 mL (11.7 mmol) N-Methylpiperazin das Produkt erhalten.
Ausbeute: 1.15 g (85.1 % d. Theorie)
$C_{10}H_{14}BrN_3$ (M= 256,147)
ber.: Molpeak $(M+H)^+$: 256/258 gef.: Molpeak $(M+H)^+$: 256/258
$R_f$-Wert: 0.50 (Kieselgel, EtOAc/MeOH/$NH_3$ 9:1:0.1)

3.25b 1-(5-Iod-pyridin-2-yl)-4-methyl-piperazin

Hergestellt nach der allgemeinen Arbeitsvorschrift II aus 1-(5-Brom-pyridin-2-yl)-4-methyl-piperazin (500 mg, 1.95 mmol).
Ausbeute: 532 mg (89.9 % d. Theorie)
$C_{10}H_{14}IN_3$ (M= 303,148)
Retentionszeit HPLC: 4.59 min (Methode A)

3.25c 1-{5-[5-(4-Chlor-phenyl)-pyridin-2-ylethinyl]-pyridin-2-yl}-4-methyl-piperazin

Hergestellt nach der allgemeinen Arbeitsvorschrift I aus 1-(5-Iod-pyridin-2-yl)-4-methylpiperazin (235 mg, 0.78 mmol) und 5-(4-Chlor-phenyl)-2-ethinyl-pyridin (167 mg, 0.78 mmol).
Ausbeute: 15 mg (5.0 % d. Theorie)
$C_{23}H_{21}ClN_4$ (M= 388,904)

ber.: Molpeak $(M+H)^+$: 389/391 gef.: Molpeak $(M+H)^+$: 389/391
Retentionszeit HPLC: 6.79 min (Methode A)

**Beispiel 3.26**

(*1S*, *4S*)-2-{5-[5-(4-Chlor-phenyl)-pyridin-2-ylethinyl]-pyridin-2-yl}-6-methyl-2,6-diazabicyclo[2.2.1 ]heptan

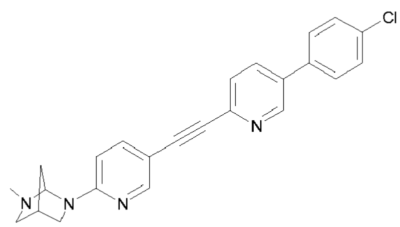

3.26a (*1S, 4S*)-2-(5-Brom-pyridin-2-yl)-6-methyl-2,6-diaza-bicyclo[2.2.1]heptan

Eine Lösung von 300 mg (1.10 mmol) (*1S, 4S*)-2-Methyl-2,5-diazabicylo[2.2.1]heptan dihydrobromid, 0.75 mL (4.40 mmol) Ethyldiisopropylamin und 270 mg (1.11 mmol) 2,5-Dibrompyridin in 1.5 mL *n*-Butanol werden 18 h bei 115˚C gerührt. Das Lösungsmittel wird i.vac. entfernt, der Rückstand mit 10 mL EtOAc versetzt und mit 1 M HCl sauer gestellt. Die wässrige Phase wird zweimal mit 2 M $K_2CO_3$-Lösung alkalisch gestellt und mit 30 mL EtOAc extrahiert. Die vereinigten organischen Extrakte werden über $Na_2SO_4$ getrocknet und das Lösungsmittel i.vac. entfernt.
Ausbeute: 70 mg (23.8 % d. Theorie)
$C_{11}H_{14}BrN_3$ (M= 268,158)
Retentionszeit HPLC: 4.07 min (Methode A)
$R_f$-Wert: 0.05 (Kieselgel, EtOAc/MeOH/$NH_3$ 9:1:0.1)

3.26b (*1S, 4S*)-2-(5-Iod-pyridin-2-yl)-6-methyl-2,6-diaza-bicyclo[2.2.1]heptan

Hergestellt nach der allgemeinen Arbeitsvorschrift II aus (*1S, 4S*)-2-(5-Brom-pyridin-2-yl)-6-methyl-2,6-diaza-bicyclo[2.2.1]heptan (70 mg, 0.26 mmol).
Ausbeute: 45 mg (54.7 % d. Theorie)
$C_{11}H_{14}IN_3$ (M= 315,159)
Retentionszeit HPLC: 4.18 min (Methode A)
$R_f$-Wert: 0.06 (Kieselgel, EtOAc/MeOH/$NH_3$ 9:1:0.1)

3.26c (*1S, 4S*)-2-{5-[5-(4-Chlor-phenyl)-pyridin-2-ylethinyl]-pyridin-2-yl}-6-methyl-2,6-diaza-bicyclo[2.2.1]heptan

Hergestellt nach der allgemeinen Arbeitsvorschrift I aus (*1S, 4S*)-2-(5-Iod-pyridin-2-yl)-6-methyl-2,6-diaza-bicyclo[2.2.1]heptan (45 mg, 0.14 mmol) und 5-(4-Chlor-phenyl)-2-ethinyl-pyridin (51 mg, 0.24 mmol).
Ausbeute: 6 mg (10.3 % d. Theorie)
$C_{24}H_{21}ClN_4$ (M= 400,915)
ber.: Molpeak $(M+H)^+$: 401/403 gef.: Molpeak $(M+H)^+$: 401/403
Retentionszeit HPLC: 6.44 min (Methode A)

**Beispiel 3.27**

{5-[5-(4-Chlor-phenyl)-pyridin-2-ylethinyl]-pyridin-2-yl}-methyl-(2-pyrrolidin-1-yl-ethyl)-amin

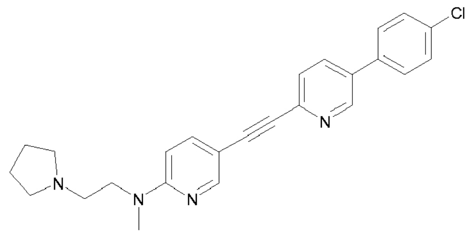

3.27a (5-Brom-pyridin-2-yl)-methyl-(2-pyrrolidin-1-yl-ethyl)-amin

Analog Beispiel 3.24a (Reaktionszeit 2.5 h) wird aus 11.5 g (40.5 mmol) 5-Brom-2-iodpyridin und 6.3 mL (11.7 mmol) Methyl-(2-pyrrolidin-1-yl-ethyl)-amin das Produkt erhalten.
Ausbeute: 4.0 g (34.8 % d. Theorie)
$C_{12}H_{18}BrN_3$ (M= 284,201)
ber.: Molpeak $(M+H)^+$: 284/286 gef.: Molpeak $(M+H)^+$: 284/286
$R_f$-Wert: 0.37 (Kieselgel, DCM/MeOH/$NH_3$ 9:1:0.1)
Retentionszeit HPLC: 5.09 min (Methode A)

3.27b (5-Iod-pyridin-2-yl)-methyl-(2-pyrrolidin-1-yl-ethyl)-amin

Hergestellt nach der allgemeinen Arbeitsvorschrift II aus (5-Brom-pyridin-2-yl)-methyl-(2-pyrrolidin-1-yl-ethyl)-amin (1.1 g, 3.87 mmol).
Ausbeute: 1.0 g (81.1 % d. Theorie)
$C_{12}H_{18}IN_3$ (M= 331,202)
ber.: Molpeak $(M+H)^+$: 332 gef.: Molpeak $(M+H)^+$: 332
Retentionszeit HPLC: 5.19 min (Methode A)

3.27c (5-[5-(4-Chlor-phenyl)-pyridin-2-ylethinyl]-pyridin-2-yl)-methyl-(2-pyrrolidin-1-yl-ethyl)-amin

Hergestellt nach der allgemeinen Arbeitsvorschrift I aus (5-Iod-pyridin-2-yl)-methyl-(2-pyrrolidin-1-yl-ethyl)-amin (100 mg, 0.30 mmol) und 5-(4-Chlor-phenyl)-2-ethinyl-pyridin (65 mg, 0.30 mmol).
Ausbeute: 43 mg (34.2 % d. Theorie)
$C_{25}H_{25}ClN_4$ (M= 416,958)
ber.: Molpeak $(M+H)^+$: 417/419 gef.: Molpeak $(M+H)^+$: 417/419
$R_f$-Wert: 0.25 (Alox, Cyc/EtOAc 2:1).
Retentionszeit HPLC: 7.57 min (Methode A)

**Beispiel 3.28**

(1-{5-[5-(4-Chlor-phenyl)-pyridin-2-ylethinyl]-pyridin-2-yl}-pyrrolidin-3-yl)-dimethyl-amin

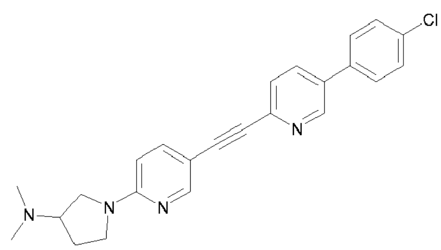

3.28a [1-(5-Brom-pyridin-2-yl)-pyrrolidin-3-yl]-dimethyl-amin

Eine Lösung vom 215 mg (0.88 mmol) 2,5-Dibrombenzol, 100 mg (0.88 mmol) 3-(Dimethylamino)-pyrrolidin

und 0.60 mL (3.51 mmol) Ethyldiisopropylamin in 0.5 mL n-Butanol werden 30 min in der Mikrowelle bei 150°C gerührt. Das Lösungsmittel wird i.vac. entfernt und der Rückstand in 20 mL EtOAc und 10 mL Wasser aufgenommen. Die wässrige Phase wird mit 1 M HCl sauer gestellt. Die Phasen werden getrennt und dann wird die wässrige Phase mit 2 M $Na_2CO_3$-Lösung alkalisch gestellt und mit 40 mL EtOAc extrahiert. Die organische Phase wird über $Na_2SO_4$ getrocknet und das Lösungsmittel i.vac. entfernt.
Ausbeute: 179 mg (75.6 % d. Theorie)
$C_{11}H_{16}BrN_3$ (M= 270,174)
ber.: Molpeak $(M+H)^+$: 270/272 gef.: Molpeak $(M+H)^+$: 270/272
$R_f$-Wert: 0.30 (Kieselgel, EtOAc/MeOH/$NH_3$ 9:1:0.1)

3.28b [1-(5-Iod-pyridin-2-yl)-pyrrolidin-3-yl]-dimethyl-amin

Hergestellt nach der allgemeinen Arbeitsvorschrift II aus [1-(5-Brom-pyridin-2-yl)-pyrrolidin-3-yl]-dimethyl-amin (160 mg, 0.59 mmol).
Ausbeute: 168 mg (89.5 % d. Theorie)
$C_{11}H_{16}IN_3$ (M= 317,175)
ber.: Molpeak $(M+H)^+$: 318 gef.: Molpeak $(M+H)^+$: 318
Retentionszeit HPLC: 3.56 min (Methode A)

3.28c (1-{5-[5-(4-Chlor-phenyl)-pyridin-2-ylethinyl]-pyridin-2-yl}-pyrrolidin-3-yl)-dimethyl-amin

Hergestellt nach der allgemeinen Arbeitsvorschrift I aus [1-(5-Iod-pyridin-2-yl)-pyrrolidin-3-yl]-dimethyl-amin (160 mg, 0.50 mmol) und 5-(4-Chlor-phenyl)-2-ethinyl-pyridin (108 mg, 0.50 mmol).
Ausbeute: 8 mg (3.9 % d. Theorie)
$C_{24}H_{23}ClN_4$ (M= 402,931)
ber.: Molpeak $(M+H)^+$: 403/405 gef.: Molpeak $(M+H)^+$: 403/405
Retentionszeit HPLC: 6.37 min (Methode A)

**Beispiel 3.29**

{5-[5-(4-Chlor-phenyl)-pyridin-2-ylethinyl]-pyridin-2-yl}-(2-pyrrolidin-1-yl-ethyl)-amin

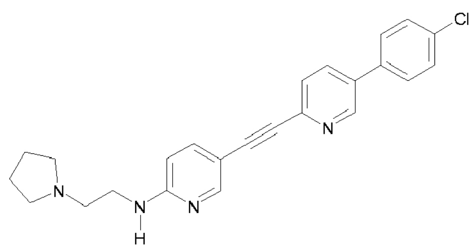

3.29a (5-Brom-pyridin-2-yl)-(2-pyrrolidin-1-yl-ethyl)-amin

Zu einer Lösung von 1.5 g (6.33 mmol) 2,5-Dibrompyridin und 0.98 mL (7.60 mmol) 1-(2-Aminoethyl)-pyrrolidin in 60 mL Toluol werden nacheinander 13.8 g (100.0 mmol) $K_2CO_3$, 79 mg (0.12 mmol) 2,2'-Bis(diphenylphosphino)-1,1'-binaphthyl und 28 mg (0.12 mmol) $Pd(OAc)_2$ gegeben. Die Reaktion wird 40 h am Rückfluß gekocht. Das Lösungsmittel wird i.vac. entfernt und der Rückstand wird in 150 mL EtOAc und 100 mL Wasser aufgenommen. Die organische Phase wird über $Na_2SO_4$ getrocknet und das Lösungsmittel i.vac. entfernt. Eine Reinigung erfolgt durch Säulenchromatographie an Kieselgel (Gradient: EtOAc/MeOH/$NH_3$ 19:1:0.1 nach EtOAc/MeOH/$NH_3$ 9:1:0.1).
Ausbeute: 145 mg (8.5 % d. Theorie)
$C_{11}H_{16}BrN_3$ (M= 270,174)
ber.: Molpeak $(M+H)^+$: 270/272 gef.: Molpeak $(M+H)^+$: 270/272
$R_f$-Wert: 0.05 (Kieselgel, EtOAc/MeOH/$NH_3$ 9:1:0.1)

3.29b {5-[5-(4-Chlor-phenyl)-pyridin-2-ylethinyl]-pyridin-2-yl}-(2-pyrrolidin-1-yl-ethyl)-amin

Analog Beispiel 3.16b wird aus 90 mg (0.33 mmol) (5-Brom-pyridin-2-yl)-(2-pyrrolidin-1-yl-ethyl)-amin und 88 mg (0.41 mmol) 5-(4-Chlor-phenyl)-2-ethinyl-pyridin das Produkt erhalten.
Ausbeute: 4 mg (5.8 % d. Theorie)
$C_{24}H_{23}ClN_4$ (M= 402,931)
ber.: Molpeak $(M+H)^+$: 403/405 gef.: Molpeak $(M+H)^+$: 403/405
Retentionszeit HPLC: 6.71 min (Methode A)

Beispiel 3.30

*N*-{5-[5-(4-Chlor-phenyl)-pyridin-2-ylethinyl]-pyridin-2-yl}-N-(2-pyrrolidin-1-yl-ethyl)-acetamid

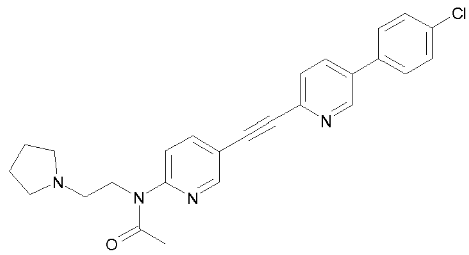

Zu einer Lösung von 89 mg (0.22 mmol) {5-[5-(4-Chlor-phenyl)-pyridin-2-ylethinyl]-pyridin-2-yl}-(2-pyrrolidin-1-yl-ethyl)-amin in 2 mL DCM wird 45 μL (0.48 mmol) Acetanhydrid gegeben. Die Reaktionslösung wird 16 h bei RT gerührt. Das Lösungsmittel wird i.vac. entfernt und die weitere Reinigung erfolgt mittels HPLC-MS.
Ausbeute: 62 mg (63.0 % d. Theorie)
$C_{26}H_{25}ClN_4O$ (M= 444,968)
ber.: Molpeak $(M+H)^+$: 445/447 gef.: Molpeak $(M+H)^+$: 445/447
$R_f$-Wert: 0.38 (Alox, Cyc/EtOAc 1:1).

**Beispiel 3.31**

{5-[5-(4-Chlor-phenyl)-pyridin-2-ylethinyl]-pyridin-2-yl}-(2-piperidin-1-yl-ethyl)-amin

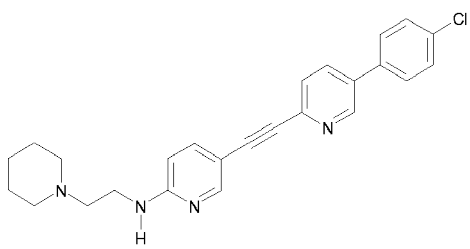

3.31a (5-Brom-pyridin-2-yl)-(2-piperidin-1-yl-ethyl)-amin

800 mg (3.38 mmol) 2,5-Dibrombenzol und 1.0 g (7.80 mmol) N-(2-Aminoethyl)piperidin werden 45 min auf 170°C erwärmt. Nach Abkühlen des Reaktionsgemisches werden 80 mL EtOAc zugegeben und filtriert. Das Filtrat wird zweimal mit je 40 mL gesättigter $NaHCO_3$-Lösung gewaschen und über $MgSO_4$ getrocknet. Nach Entfernen des Trocken- und Lösungsmittels wird der Rückstand chromatographisch (Kieselgel, EtOAc/MeOH/$NH_3$ 85:15:2) gereinigt.
Ausbeute: 720 mg (75.0 % d. Theorie)
$C_{12}H_{18}BrN_3$ (M= 284,201)
ber.: Molpeak $(M+H)^+$: 284/286 gef.: Molpeak $(M+H)^+$: 284/286

$R_f$-Wert: 0.30 (Kieselgel, EtOAc/MeOH/$NH_3$ 9:1:0.1)

3.31b (5-Iod-pyridin-2-yl)-(2-piperidin-1-yl-ethyl)-amin

Hergestellt nach der allgemeinen Arbeitsvorschrift II aus (5-Brom-pyridin-2-yl)-(2-piperidin-1-yl-ethyl)-amin (720 mg, 2.53 mmol).
Ausbeute: 750 mg (89.4 % d. Theorie)
$C_{12}H_{18}IN_3$ (M= 331,202)
ber.: Molpeak $(M+H)^+$: 332 gef.: Molpeak $(M+H)^+$: 332
Retentionszeit HPLC: 4.32 min (Methode A)

3.31c {5-[5-(4-Chlor-phenyl)-pyridin-2-ylethinyl]-pyridin-2-yl}-(2-piperidin-1-yl-ethyl)-amin

Hergestellt nach der allgemeinen Arbeitsvorschrift I aus (5-Iod-pyridin-2-yl)-(2-piperidin-1-yl-ethyl)-amin (397 mg, 1.20 mmol) und 5-(4-Chlor-phenyl)-2-ethinyl-pyridin (256 mg, 1.20 mmol).
Ausbeute: 230 mg (46.0 % d. Theorie)
$C_{25}H_{25}ClN_4$ (M= 416,958)
ber.: Molpeak $(M+H)^+$: 417/419 gef.: Molpeak $(M+H)^+$: 417/419
$R_f$-Wert: 0.55 (Kieselgel, EtOAc/MeOH/$NH_3$ 9:1:0.1)
Retentionszeit HPLC: 7.26 min (Methode A)

**Beispiel 3.32**

5'-[5-(4-Chlor-phenyl)-pyridin-2-ylethinyl]-3-pyrrolidin-1-yl-3,4,5,6-tetrahydro-*2H*-[1,2']bipyridinyl

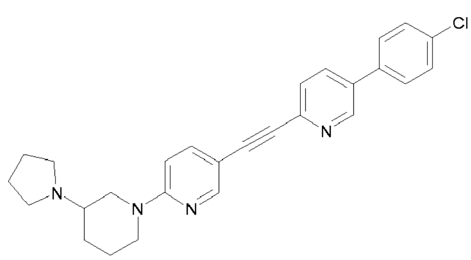

3.32a 5'-Brom-3-pyrrolidin-1-yl-3,4,5,6-tetrahydro-2H-[1,2']bipyridinyl

Analog Beispiel 3.31 a (Reaktionszeit: 35 min bei 160˚C) wird aus 2,37 g (10.0 mmol) 2,5-Dibrompyridin und 1.6 g (10.4 mmol) 3-Pyrrolidin-1-yl-piperidin das Produkt erhalten.
Ausbeute: 700 mg (21.8 % d. Theorie)
$C_{14}H_{20}BrN_3$ (M= 310,240)
ber.: Molpeak $(M+H)^+$: 310/312 gef.: Molpeak $(M+H)^+$: 310/312
Retentionszeit HPLC: 5.06 min (Methode B)

3.32b 5'-Iod-3-pyrrolidin-1-yl-3,4,5,6-tetrahydro-*2H*-[1,2']bipyridinyl

Hergestellt nach der allgemeinen Arbeitsvorschrift II aus 5'-Brom-3-pyrrolidin-1-yl-3,4,5,6-tetrahydro-*2H*-[1,2'] bipyridinyl (700 mg, 2.26 mmol).
Ausbeute: 700 mg (86.9 % d. Theorie)
$C_{14}H_{2O}IN_3$ (M= 357,240)
ber.: Molpeak $(M+H)^+$: 358 gef.: Molpeak $(M+H)^+$: 358
Retentionszeit HPLC: 5.20 min (Methode A)

3.32c 5'-[5-(4-Chlor-phenyl)-pyridin-2-ylethinyl]-3-pyrrolidin-1-yl-3,4,5,6-tetrahydro-*2H*-[1,2']bipyridinyl

Hergestellt nach der allgemeinen Arbeitsvorschrift I aus 5'-Iod-3-pyrrolidin-1-yl-3,4,5,6-tetrahydro-*2H*-[1,2']bipyridinyl (179 mg, 0.50 mmol) und 5-(4-Chlor-phenyl)-2-ethinyl-pyridin (107 mg, 0.50 mmol).
Ausbeute: 120 mg (54.2 % d. Theorie)
$C_{27}H_{27}ClN_4$ (M= 442,996)
ber.: Molpeak $(M+H)^+$: 443/445 gef.: Molpeak $(M+H)^+$: 443/445
$R_f$-Wert: 0.38 (Kieselgel, DCM/MeOH/$NH_3$ 9:1:0.1)
Retentionszeit HPLC: 7.40 min (Methode A)

Beispiel 3.33

1’-{5-[5-(4-Chtor-phenyt)-pyridin-2-yiethinyt]-pyridin-2-yl}-[1,3’]bipyrrotidinyt

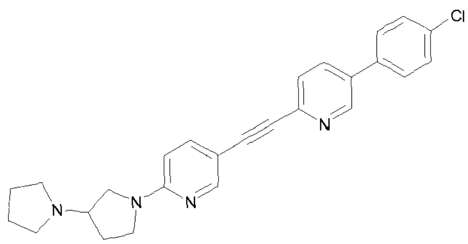

3.33a 1’-Benzyl-[1,3’]bipyrrolidinyl

Zu einer Lösung von 1.23 mL (15.0 mmol) Pyrrolidin und 2.41 mL (15.0 mmol) N-Benzylpyrrolidinon in 100 mL THF werden 3.82 g (18.0 mmol) $NaBH(OAc)_3$ gegeben und mit 2 mL Essigsäure sauer gestellt. Die Reaktion wird über Nacht bei RT gerührt. Die Reaktionslösung wird mit 200 mL gesättigter $NaHCO_3$-Lösung versetzt und zweimal mit je 200 mL EtOAc extrahiert. Die organische Phase wird über $MgSO_4$ getrocknet und das Lösungsmittel i.vac. entfernt. Die Reinigung erfolgt durch Säulenchromatographie an Kieselgel (EtOAc/MeOH/$NH_3$ 8:2:0.2).
Ausbeute: 1.80 g (52.1 % d. Theorie)
$C_{15}H_{22}N_2$ (M= 230,356)
ber.: Molpeak $(M+H)^+$: 231 gef.: Molpeak $(M+H)^+$: 231
$R_f$-Wert: 0.05 (Kieselgel, EtOAc/MeOH/$NH_3$ 9:1:0.1)

3.33b [1,3’]Bipyrrolidinyl

Zu einer Lösung von 1.80 g (7.42 mmol) 1’-Benzyl-[1,3’]bipyrrolidinyl in 80 mL MeOH werden 180 mg 10% Pd/C gegeben. Die Reaktionslösung wird 5 h bei RT und 3 bar $H_2$ gerührt. Weitere 180 mg 10% Pd/C werden zugegeben und nach 4 h 100 mg Palladiumhydroxid. Die Reaktion wird weitere 6 h bei RT und 3 bar $H_2$ gerührt. Der Katalysator wird abgesaugt und das Lösungsmittel i.vac. entfernt.
Ausbeute: 900 mg (86.5 % d. Theorie)
$C_8H_{16}N_2$ (M= 140,230)
$R_f$-Wert: 0.05 (Kieselgel, EtOAc/MeOH/$NH_3$ 8:2:0.2)

3.33c 1’-(5-Brom-pyridin-2-yl)-[1,3’]bipyrrolidinyl

Analog Beispiel 3.31 a (Reaktionszeit: 60 min bei 170˚C) wird aus 1.52 g (6.40 mmol) 2,5-Dibrompyridin und 0.90 g (6.42 mmol) [1,3’]Bipyrrolidinyl das Produkt erhalten.
Ausbeute: 700 mg (36.8 % d. Theorie)
$C_{13}H_{18}BrN_3$ (M= 296,213)
ber.: Molpeak $(M+H)^+$: 296/298 gef.: Molpeak $(M+H)^+$: 296/298
$R_f$-Wert: 0.42 (Kieselgel, EtOAc/MeOH/$NH_3$ 9:1:0.1)

3.33d 1’-(5-Iod-pyridin-2-yl)-[1,3’]bipyrrolidinyl

Hergestellt nach der allgemeinen Arbeitsvorschrift II aus 1’-(5-Brom-pyridin-2-yl)-[1,3’]bipyrrolidinyl (700 mg, 2.36 mmol).

Ausbeute: 650 mg (80.1 % d. Theorie)
$C_{13}H_{18}IN_3$ (M= 343,213)
ber.: Molpeak $(M+H)^+$: 344 gef.: Molpeak $(M+H)^+$: 344
Retentionszeit HPLC: 3.95 min (Methode A)

3.33e 1'-{5-[5-(4-Chlor-phenyl)-pyridin-2-ylethinyl]-pyridin-2-yl}-[1,3']bipyrrolidinyl

Hergestellt nach der allgemeinen Arbeitsvorschrift I aus 1'-(5-lod-pyridin-2-yl)-[1,3']bipyrrolidinyl (172 mg, 0.50 mmol) und 5-(4-Chlor-phenyl)-2-ethinyl-pyridin (107 mg, 0.50 mmol).
Ausbeute: 65 mg (30.3 % d. Theorie)
$C_{26}H_{25}ClN_4$ (M= 428,969)
ber.: Molpeak $(M+H)^+$: 429/431 gef.: Molpeak $(M+H)^+$: 429/431
$R_f$-Wert: 0.50 (Kieselgel, EtOAc/MeOH/$NH_3$ 9:1:0.1)
Retentionszeit HPLC: 6.71 min (Methode A)

**Beispiel 3.34**

{5-[5-(4-Chlor-phenyl)-pyridin-2-ylethinyl]-pyridin-2-yl}-(2-pyrrolidin-1-yl-propyl)-amin

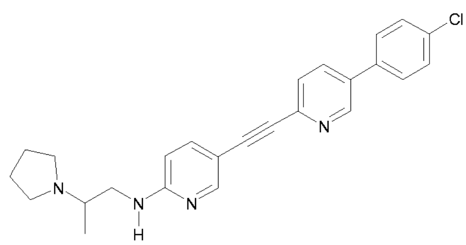

3.34a *N*-(5-Brom-pyridin-2-yl)-2-chlor-propionamid

Zu einer Lösung von 3.46 g (20.0 mmol) 2-Amino-5-brompyridin und 6.12 mL (44.0 mmol) Triethylamin in 80 mL DCM wird bei 0°C 2.14 mL (22.0 mmol) 2-Chlorpropionsäurechlorid in 5 mL DCM zugetropft. Das Eisbad wird entfernt und die Reaktionslösung wird weitere 1.5 h bei RT gerührt. Nochmals wird 0.40 mL (4.12 mmol) 2-Chlorpropionsäurechlorid zugegeben und die Lösung eine weitere Stunde bei RT gerührt. Das Reaktionsgemisch wird mit 80 mL Wasser versetzt, einmal mit 80 mL gesättigter NaCl-Lösung gewaschen und über $MgSO_4$ getrocknet. Das Lösungsmittel wird i.vac. entfernt und der Rückstand mit wenig EtOAc verrieben, abgesaugt und getrocknet.
Ausbeute: 3.50 g (66.4 % d. Theorie)
$C_8H_8BrClN_20$ (M= 263,523)
ber.: Molpeak $(M+H)^+$: 263/265/267 gef.: Molpeak $(M+H)^+$: 263/265/267
$R_f$-Wert: 0.85 (Kieselgel, PE/EtOAc 6:4)

3.34b N-(5-Brom-pyridin-2-yl)-2-pyrrolidin-1-yl-propionamid

Zu einer Lösung von 3.5 g (13.3 mmol) N-(5-Brom-pyridin-2-yl)-2-chlor-propionamid in 50 mL DMF werden nacheinander 4.01 g (29.0 mmol) $K_2CO_3$ und 1.19 mL (14.5 mmol) Pyrrolidin gegeben. Die Reaktion wird 3 Tage bei RT gerührt und mit mit 150 mL Wasser versetzt. Die wässrige Phase wird zweimal mit EtOAc extrahiert und die organische Phase über $MgSO_4$ getrocknet. Nach Entfernen des Trocken- und Lösungsmittels wird der Rückstand chromatographisch (Kieselgel, Gradient: PE/EtOAc 4:6 nach EtOAc) gereinigt.
Ausbeute: 1.80 g (45,4 % d. Theorie)
$C_{12}H_{16}BrN_3O_3$ (M= 298,185)
ber.: Molpeak $(M+H)^+$: 298/300 gef.: Molpeak $(M+H)^+$: 298/300
Retentionszeit HPLC: 4.21 min (Methode A)

3.34c (5-Brom-pyridin-2-yl)-(2-pyrrolidin-1-yl-propyl)-amin

Unter Stickstoffatmospäre werden zu einer auf 0˚C gekühlten Lösung von 1.8 g (6.04 mmol) N-(5-Brom-pyridin-2-yl)-2-pyrrolidin-1-yl-propionamid in 30 mL THF 6.00 mL (6.00 mmol) 1 M Lithiumaluminiumhydrid-Lösung in THF so zugegeben, dass die Innentemperatur 4˚C nicht übersteigt. Die Reaktionslösung wird weitere 20 min bei 0˚C gerührt. EtOAc wird vorsichtig zugegeben, der Aluminiumkomplex wird mit 0.2 mL Wasser, dann mit 0.2 mL 15% Natronlauge-Lösung und schliesslich mit 0.6 mL Wasser zersetzt. Der entstandene Niederschlag wird abgesaugt und das Filtrat mit 50 mL EtOAc verdünnt. Die organische Phase wird mit 30 mL gesättigter $NaHCO_3$-Lösung gewaschen und über $MgSO_4$ getrocknet. Das Lösungsmittel wird i.vac. entfernt und die weitere Reinigung erfolgt durch Säulenchromatographie an Kieselgel (EtOAc/MeOH/$NH_3$ 9:1:0.1).
Ausbeute: 700 mg (40.8 % d. Theorie)
$C_{12}H_{18}BrN_3$ (M= 284,201)
ber.: Molpeak $(M+H)^+$: 284/286 gef.: Molpeak $(M+H)^+$: 284/286
$R_f$-Wert: 0.32 (Kieselgel, EtOAc/MeOH/$NH_3$ 9:1:0.1)
Retentionszeit HPLC: 4.63 min (Methode A)

3.34d (5-Iod-pyridin-2-yl)-(2-pyrrolidin-1-yl-propyl)-amin

Hergestellt nach der allgemeinen Arbeitsvorschrift II aus (5-Brom-pyridin-2-yl)-(2-pyrrolidin-1-yl-propyl)-amin (600 mg, 2.11 mmol).
Ausbeute: 560 mg (80.1 % d. Theorie)

3.34e {5-[5-(4-Chlor-phenyl)-pyridin-2-ylethinyl]-pyridin-2-yl}-(2-pyrrolidin-1-yl-propyl)-amin

Hergestellt nach der allgemeinen Arbeitsvorschrift I aus (5-Iod-pyridin-2-yl)-(2-pyrrolidin-1-yl-propyl)-amin (250 mg, 0.76 mmol) und 5-(4-Chlor-phenyl)-2-ethinyl-pyridin (165 mg, 0.77 mmol).
Ausbeute: 95 mg (29.5 % d. Theorie)
$C_{25}H_{25}ClN_4$ (M= 416,958)
ber.: Molpeak $(M+H)^+$: 417/419 gef.: Molpeak $(M+H)^+$: 417/419
Retentionszeit HPLC: 7.19 min (Methode A)

**Beispiel 3.35**

N-{4-[5-(4-Chlor-phenyl)-pyridin-2-ylethinyl]-phenyl}-2-pyrrolidin-1-yl-propionamid

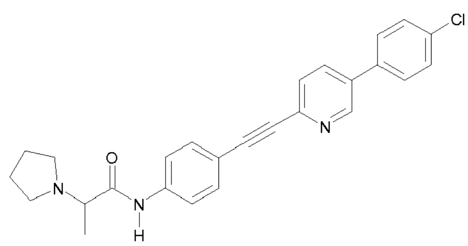

3.35a 4-[5-(4-Chlor-phenyl)-pyridin-2-ylethinyl]-phenylamin

Hergestellt nach der allgemeinen Arbeitsvorschrift I aus 4-Iodanilin (732 mg, 3.28 mmol) und 5-(4-Chlor-phenyl)-2-ethinyl-pyridin (700 mg, 3.28 mmol).
Ausbeute: 440 mg (44.1 % d. Theorie)
$C0_{19}H_{13}ClN_2$ (M= 304,782)
ber.: Molpeak $(M+H)^+$: 305/307 gef.: Molpeak $(M+H)^+$: 305/307
Retentionszeit HPLC: 5.70 min (Methode A)

3.35b N-{4-[5-(4-Chlor-phenyl)-pyridin-2-ylethinyl]-phenyl}-2-pyrrolidin-1-yl-propionamid

Zu einer Lösung von 100 mg (0.70 mmol) 2-Pyrrolidin-1-yl-propionsäure in 10 mL THF werden nacheinander 0.18 mL (1.31 mmol) Triethylamin und 269 mg (0.84 mmol) TBTU gegeben. Die Lösung wird 1 h bei RT gerührt und dann werden 200 mg (0.66 mmol) 4-[5-(4-Chlor-phenyl)-pyridin-2-ylethinyl]-phenylamin zugegeben. Die Reaktionslösung wird über Nacht bei RT gerührt. Die Umsetzung ist nicht vollständig. Deshalb wird zum Reaktionsgemisch 100 mg (0.70 mmol) 2-Pyrrolidin-1-yl-propionsäure in 10 mL THF gegeben (1 h aktiviert durch Rühren mit 0.18 mL (1.31 mmol) Triethylamin und 269 mg (0.84 mmol) TBTU). Die Reaktionslösung wird weitere 16 h gerührt und mit $NaHCO_3$-Lösung verdünnt. Die wässrige Phase wird mit EtOAc extrahiert und die organische Phase über $MgSO_4$ getrocknet. Das Lösungsmittel wird i.vac. entfernt und die weitere Reinigung erfolgt durch Säulenchromatographie an Kieselgel (EtOAc/MeOH/$NH_3$ 8:2:0.2).
Ausbeute: 40 mg (14.2 % d. Theorie)
$C_{26}H_{24}ClN_3O$ (M= 429,954)
ber.: Molpeak $(M+H)^+$: 430/432 gef.: Molpeak $(M+H)^+$: 430/432
Retentionszeit HPLC: 7.29 min (Methode A)

**Beispiel 3.36**

N-{4-[5-(4-Chlor-phenyl)-pyridin-2-ylethinyl]-phenyl}-2-pyrrolidin-1 -yl-acetamid

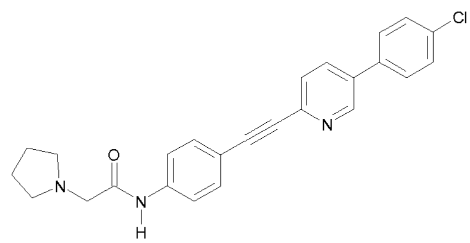

Analog Beispiel 3.35b wird aus 200 mg (0.66 mmol) 4-[5-(4-Chlor-phenyl)-pyridin-2-ylethinyl]-phenylamin und 100 mg (0.77 mmol) Pyrrolidin-1-yl-essigsäure das Produkt erhalten.
Ausbeute: 5 mg (1.8 % d. Theorie)
$C_{25}H_{22}ClN_3O$ (M= 415,927)
ber.: Molpeak $(M+H)^+$: 416/418 gef.: Molpeak $(M+H)^+$: 416/418
Retentionszeit HPLC: 6.75 min (Methode B)

**Beispiel 3.37**

5-[5-(4-Chlor-phenyl)-pyridin-2-ylethinyl]-1-(2-pyrrolidin-1-yl-ethyl)-1,3-dihydrobenzimidazol-2-on

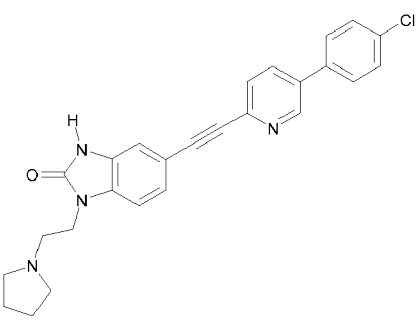

3.37a (4-Brom-2-nitro-phenyl)-(2-pyrrolidin-1-yl-ethyl)-amin

Zu einer Lösung von 5.00 g (22.7 mmol) 2-Brom-5-fluornitrobenzol und 2.59 g (22.7 mmol) 1-(2-Amino)-pyrrolidin in 20 mL Acetonitril werden 4.42 g (32.0 mmol) $K_2C0_3$ gegeben. Die Reaktionslösung wird über Nacht bei RT gerührt. Die Lösung wird filtriert und das Lösungsmittel i.vac. entfernt. Die Reinigung erfolgt durch Säulenchromatographie an Kieselgel (Gradient: DCM nach DCM/MeOH 9:1).
Ausbeute: 5.90 g (82.6 % d. Theorie)
$C_{12}H_{16}BrN_3O_2$ (M= 314,184)
ber.: Molpeak $(M+H)^+$: 314/316 gef.: Molpeak $(M+H)^+$: 314/316
$R_f$-Wert: 0.40 (Kieselgel, DCM/MeOH 9:1)

3.37b 4-Brom-$N^1$-(2-pyrrolidin-1-yl-ethyl)-benzol-1,2-diamin

Zu einer Lösung von 1.00 g (3.18 mmol) (4-Brom-2-nitro-phenyl)-(2-pyrrolidin-1-yl-ethyl)-amin in 100 mL MeOH werden 100 mg Raney Nickel gegeben. Die Reaktionslösung wird bei 3 bar $H_2$ und RT 15 min gerührt. Nach Filtration wird das Lösungsmittel i.vac. entfernt und das Produkt wird ohne Reinigung weiter umgesetzt.
Ausbeute: 850 mg (94.0 % d. Theorie)
$C_{12}H_{18}BrN_3$ (M= 284,201)
ber.: Molpeak $(M+H)^+$: 284/286 gef.: Molpeak $(M+H)^+$: 284/286
Retentionszeit HPLC: 4.56 min (Methode A)

3.37c 5-Brom-1-(2-pyrrolidin-1-yl-ethyl)-1,3-dihydro-benzimidazol-2-on

Zu einer Lösung von 853 mg (3.00 mmol) 4-Brom-$N^1$-(2-pyrrolidin-1-yl-ethyl)-benzol-1,2-diamin in 20 mL THF werden bei RT 600 mg (3.70 mmol) CDI gegeben. Die Reaktionslösung wird auf 40˚C erwärmt und 30 min bei dieser Temperatur gerührt. Weitere 600 mg (3.70 mmol) CDI werden zugegeben und die Reaktion wird weitere 30 min bei 40˚C gerührt. Die Lösung wird mit halbgesättigter $NaHCO_3$-Lösung verdünnt und die wässrige Phase wird zweimal mit EtOAc extrahiert. Die organische Phase wird über $MgSO_4$ getrocknet und das Lösungsmittel i.vac. entfernt. Der Rückstand wird mit Acetonitril verrieben, der Niederschlag filtriert und an der Luft getrocknet.
Ausbeute: 500 mg (53.7 % d. Theorie)
$C_{13}H_{16}BrN_3O$ (M= 310,196)
ber.: Molpeak $(M+H)^+$: 310/312 gef.: Molpeak $(M+H)^+$: 310/312
Retentionszeit HPLC: 4.30 min (Methode A)

3.37d 5-lod-1-(2-pyrrolidin-1-yl-ethyl)-1,3-dihydro-benzimidazol-2-on

Hergestellt nach der allgemeinen Arbeitsvorschrift II aus 5-Brom-1-(2-pyrrolidin-1-yl-ethyl)-1,3-dihydro-benzimidazol-2-on (150 mg, 0.48 mmol).
Ausbeute: 140 mg (81.0 % d. Theorie)
$C_{13}H_{16}IN_3O$ (M= 357,196)
ber.: Molpeak $(M+H)^+$: 358 gef.: Molpeak $(M+H)^+$: 358
Retentionszeit HPLC: 4.53 min (Methode A)

3.37e 5-[5-(4-Chlor-phenyl)-pyridin-2-ylethinyl]-1-(2-pyrrolidin-1-yl-ethyl)-1,3-dihydro-benzimidazol-2-on

Hergestellt nach der allgemeinen Arbeitsvorschrift 1 aus 5-lod-1-(2-pyrrolidin-1-yl-ethyl)-1,3-dihydro-benzimidazol-2-on (140 mg, 0.39 mmol) und 5-(4-Chlor-phenyl)-2-ethinyl-pyridin (83 mg, 0.39 mmol).
Ausbeute: 7 mg (3.7 % d. Theorie)
$C_{26}H_{23}ClN_4O$ (M= 442,952)
ber.: Molpeak $(M+H)^+$: 443/445 gef.: Molpeak $(M+H)^+$: 443/445
Retentionszeit HPLC: 6.78 min (Methode A)

**Beispiel 3.38**

5-[5-(4-Chlor-phenyl)-pyridin-2-ylethinyl]-3-methyl-1-(2-pyrrolidin-1-yl-ethyl)-1,3-dihydrobenzoimidazol-2-on

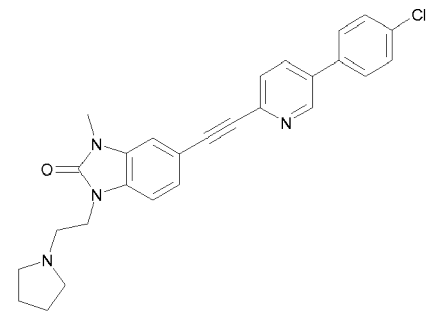

3.38a 5-Brom-3-methyl-1-(2-pyrrolidin-1-yl-ethyl)-1,3-dihydro-benzimidazol-2-on

Zu einer Lösung von 200 mg (0.65 mmol) 5-Brom-1-(2-pyrrolidin-1-yl-ethyl)-1,3-dihydrobenzimidazol-2-on in 4 mL DMSO werden bei RT 73 mg (0.65 mmol) Kalium-*tert*-butylat gegeben. Die Reaktionslösung wird 30 min gerührt und dann werden 40 μL (0.65 mmol) Iodmethan zugegeben und weitere 30 min gerührt. Das Gemisch wird mit halbgesättigter $NaHCO_3$-Lösung versetzt und die wässrige Phase zweimal mit je 30 mL EtOAc extrahiert. Die organische Phase wird über $MgSO_4$ getrocknet und das Lösungsmittel i.vac. entfernt.
Ausbeute: 180 mg (86.1 % d. Theorie)
$C_{14}H_{18}BrN_3O$ (M= 324,223)
ber.: Molpeak $(M+H)^+$: 324/326 gef.: Molpeak $(M+H)^+$: 324/326
Retentionszeit HPLC: 4.69 min (Methode B)

3.38b 5-Iod-3-methyl-1-(2-pyrrolidin-1-yl-ethyl)-1,3-dihydro-benzimidazol-2-on

Hergestellt nach der allgemeinen Arbeitsvorschrift II aus 5-Brom-3-methyl-1-(2-pyrrolidin-1-yl-ethyl)-1,3-dihydro-benzimidazol-2-on (160 mg, 0.49 mmol).
Ausbeute: 120 mg (65.6 % d. Theorie)
$C_{14}H_{18}IN_3O$ (M= 371,223)
ber.: Molpeak $(M+H)^+$: 372 gef.: Molpeak $(M+H)^+$: 372
Retentionszeit HPLC: 5.02 min (Methode A)

3.38c 5-[5-(4-Chlor-phenyl)-pyridin-2-ylethinyl]-3-methyl-1-(2-pyrrolidin-1-yl-ethyl)-1,3-dihydro-benzimidazol-2-on

Hergestellt nach der allgemeinen Arbeitsvorschrift I aus 5-Iod-3-methyl-1-(2-pyrrolidin-1-yl-ethyl)-1,3-dihydro-benzimidazol-2-on (120 mg, 0.32 mmol) und 5-(4-Chlor-phenyl)-2-ethinyl-pyridin (68 mg, 0.32 mmol).
Ausbeute: 15 mg (9.8 % d. Theorie)
$C_{27}H_{25}ClN_4O$ (M= 456,980)
ber.: Molpeak $(M+H)^+$: 457/459 gef.: Molpeak $(M+H)^+$: 457/459
Retentionszeit HPLC: 7.11 min (Methode A)

**Beispiel 3.39**

6-[5-(4-Chlor-phenyl)-pyridin-2-ylethinyl]-3-(2-pyrrolidin-1-yl-ethyl)-3*H*-imidazo[4,5-*b*]pyridin

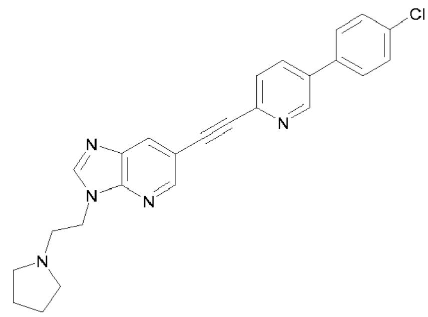

3.39a (5-Brom-3-nitro-pyridin-2-yl)-(2-pyrrolidin-1-yl-ethyl)-amin

Zu einer Lösung von 600 mg (2.53 mmol) 5-Brom-2-chlor-3-nitropyridin und 0.32 mL (2.53 mmol) 1-(2-Aminoethyl)-pyrrolidin in 3 mL *n*-Butanol werden 0.86 mL (5.05 mmol) Ethyldiiopropylamin gegeben. Die Reaktion wird auf 50˚C erwärmt und eine Stunde bei dieser Temperatur gerührt. Das Lösungsmittel wird i.vac. entfernt und der Rückstand mit 40 mL Wasser versetzt und mit 1 M HCl sauer gestellt. Die wässrige Phase wird mit 20 mL EtOAc extrahiert und die wässrige Phase danach mit gesättigter $K_2CO_3$-Lösung alkalisch gestellt. Die wässrige Phase wird mit 40 mL EtOAc extrahiert. Die organische Phase wird über $Na_2SO_4$ getrocknet und das Lösungsmittel i.vac. entfernt.
Ausbeute: 692 mg (86.9 % d. Theorie)
$C_{11}H_{15}BrN_4O_2$ (M= 315,172)
ber.: Molpeak $(M+H)^+$: 315/317 gef.: Molpeak $(M+H)^+$: 315/317
Retentionszeit HPLC: 5.00 min (Methode A)
$R_f$-Wert: 0.08 (Kieselgel, Cyc/EtOAc 2:1)

3.39b 5-Brom-$N^2$-(2-pyrrolidin-1-yl-ethyl)-pyridin-2,3-diamin

Zu einer Lösung von 680 mg (2.16 mmol) (5-Brom-3-nitro-pyridin-2-yl)-(2-pyrrolidin-1-yl-ethyl)-amin in 40 mL EtOAc werden bei RT 2.44 g (10.8 mmol) Zinn(II)chlorid Dihydrat und 2.20 g (26.2 mmol) $NaHCO_3$ gegeben. Die Reaktion wird 1.5 h unter Rückfluß erhitzt und dann mit 20 mL Wasser verdünnt. Die wässrige Phase wird mit 1 M HCl sauer gestellt und von der organischen Phase abgetrennt. Die wässrige Phase wird mit gesättigter $K_2CO_3$-Lösung alkalisch gestellt und zweimal mit je 40 mL EtOAc extrahiert. Die organische Phase wird über $Na_2SO_4$ getrocknet und das Lösungsmittel i.vac. entfernt.
Ausbeute: 479 mg (77.8 % d. Theorie)
$C_{11}H_{17}BrN_4$ (M= 285,189)
ber.: Molpeak $(M+H)^+$: 285/287 gef.: Molpeak $(M+H)^+$: 285/287
Retentionszeit HPLC: 3.9 min (Methode A)

3.39c 6-Brom-3-(2-pyrrolidin-1-yl-ethyl)-3H-imidazo[4,5-b]pyridin

Eine Lösung von 470 mg (1.65 mmol) 5-Brom-$N^2$-(2-pyrrolidin-1-yl-ethyl)-pyridin-2,3-diamin in 10 mL Ameisensäure wird 1.5 h unter Rückfluß erhitzt. Mit gesättigter $K_2CO_3$-Lösung stellt man alkalisch und extrahiert mit 40 mL EtOAc. Die organische Phase wird über $Na_2SO_4$ getrocknet und das Lösungsmittel i.vac. entfernt.
Ausbeute: 466 mg (95.8 % d. Theorie)
$C_{12}H_{15}BrN_4$ (M= 295,184)
ber.: Molpeak $(M+H)^+$: 295/297 gef.: Molpeak $(M+H)^+$: 295/297
Retentionszeit HPLC: 4.0 min (Methode A)

3.39d 6-Iod-3-(2-pyrrolidin-1-yl-ethyl)-3*H*-imidazo[4,5-*b*]pyridin

Hergestellt nach der allgemeinen Arbeitsvorschrift II aus 6-Brom-3-(2-pyrrolidin-1-yl-ethyl)-3*H*-imidazo[4,5-b]pyridin (450 mg, 1.52 mmol).
Ausbeute: 510 mg (97.8 % d. Theorie)
$C_{12}H_{15}IN_4$ (M= 342,185)
ber.: Molpeak $(M+H)^+$: 343 gef.: Molpeak $(M+H)^+$: 343
Retentionszeit HPLC: 4.08 min (Methode A)
$R_f$-Wert: 0.09 (Kieselgel, EtOAc/MeOH/$NH_3$ 9:1:0.1)

3.39e 6-[5-(4-Chlor-phenyl)-pyridin-2-ylethinyl]-3-(2-pyrrolidin-1-yl-ethyl)-3*H*-imidazo[4,5-*b*]pyridin

Hergestellt nach der allgemeinen Arbeitsvorschrift I aus 6-Iod-3-(2-pyrrolidin-1-yl-ethyl)-3*H*-imidazo[4,5-*b*]pyridin (300 mg, 0.88 mmol) und 5-(4-Chlor-phenyl)-2-ethinyl-pyridin (187 mg, 0.88 mmol).
Ausbeute: 67 mg (17.9 % d. Theorie)
$C_{25}H_{22}ClN_5$ (M= 427,941)
ber.: Molpeak $(M+H)^+$: 428/430 gef.: Molpeak $(M+H)^+$: 428/430
$R_f$-Wert: 0.41 (Kieselgel, DCM/MeOH/$NH_3$ 9:1:0.1)
Retentionszeit HPLC: 6.52 min (Methode A)

**Beispiel 3.40**

5-[5-(4-Chlor-phenyl)-pyridin-2-ylethinyl]-2-pyrrolidin-1-ylmethyl-1H-benzim idazol

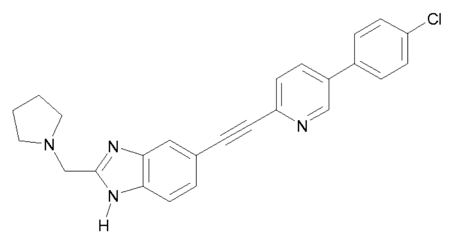

3.40a (5-Nitro-1*H*-benzimidazol-2-yl)-methanol

Zu einer Lösung von 6.24 g (40.8 mmol) 4-Nitro-o-phenylendiamin in 80 mL halbkonzentrierter HCl wird 6.2 g (81.5 mmol) Glycolsäure gegeben. Die Reaktionslösung wird 4 h am Rückfluß erhitzt und das Lösungsmittel i.vac. entfernt. Der Rückstand wird in Wasser aufgenommen und mit 2 N NaOH basisch gestellt. Das Produkt fällt aus und es wird noch 1 Stunde im Eisbad nachgerührt. Der Niederschlag wird abgesaugt und nacheinander mit Wasser und PE gewaschen. Das Produkt wird bei 40˚C getrocknet. Dieses enthält noch 40% 4-Nitro-o-phenylendiamin. Es wird nochmals in halbkonzentrierter HCl aufgenommen und nach Zugabe von 6.5 mL Glycolsäure (57% in Wasser) 3 h am Rückfluß gekocht und weitere 12 h bei 80˚C. Das Lösungsmittel wird i.vac. entfernt und der Rückstand wird in Wasser gelöst und mit 6 N NaOH alkalisch gestellt, wobei das Produkt ausfällt. Der Niederschlag wird abgesaugt und nacheinander mit Wasser und PE gewaschen. Das Produkt wird bei 50˚C im Umlufttrockenschrank getrocknet.
Ausbeute: 6.40 g (81.3 % d. Theorie)
$C_8H_7N_3O_3$ (M= 193,163)
ber.: Molpeak $(M+H)^+$: 194 gef.: Molpeak $(M+H)^+$: 194
$R_f$-Wert: 0.13 (Kieselgel, DCM/MeOH/$NH_3$ 9:1:0.1)

3.40b 2-Chlormethyl-5-nitro-1*H*-benzimidazol

Zu einer Lösung von 6.4 g (33.1 mmol) (5-Nitro-1H-benzimidazol-2-yl)-methanol in 100 mL DCM werden bei 10˚C langsam 20 mL (275 mmol) Thionylchlorid zugegeben. Die Reaktion wird 1 h bei RT gerührt und das Lösungsmittel i.vac. entfernt. Der Rückstand wird mit DCM verrieben, abgesaugt, mit DCM und Ether gewaschen und im Umlufttrokkenschrank bei 35˚C getrocknet.
Ausbeute: 7.01 g (100 % d. Theorie)
$C_8HeClN_3O_3$(M= 211,609)
ber.: Molpeak $(M+H)^+$: 212/214 gef.: Molpeak $(M+H)^+$: 212/214
Retentionszeit HPLC: 4.1 min (Methode B)

3.40c 5-Nitro-2-pyrrolidin-1-ylmethyl-1*H*-benzimidazol

Zu einer Lösung von 6.00 g (28.4 mmol) 2-Chlormethyl-5-nitro-1H-benzimidazol in 100 mL DCM werden 9.47 mL (113 mmol) Pyrrolidin gegeben. Die Reaktion wird über Nacht bei RT gerührt. Die Reaktionslösung wird viermal mit Wasser gewaschen. Die organische Phase wird über $MgSO_4$ getrocknet und das Lösungsmittel i.vac. entfernt.
Ausbeute: 5.50 g (78.8 % d. Theorie)

$C_{12}H_{14}N_4O_2$ (M= 246,271)
ber.: Molpeak $(M+H)^+$: 247 gef.: Molpeak $(M+H)^+$: 247
$R_f$-Wert: 0.22 (Kieselgel, EtOAc/MeOH 9:1)

3.40d 2-Pyrrolidin-1-ylmethyl-1*H*-benzimidazol-5-ylamin

Zu einer Lösung von 5.50 g (22.3 mmol) 5-Nitro-2-pyrrolidin-1-ylmethyl-1*H*-benzimidazol in 50 mL MeOH werden 1.00 g Raney Nickel gegeben. Die Reaktionslösung wird bei 3 bar $H_2$ und RT 30 h gerührt. Nach Filtration wird das Lösungsmittel i.vac. entfernt und die weitere Reinigung erfolgt durch Säulenchromatographie an Kieselgel (EtOAc/MeOH/$NH_3$ 8:2:0.2).
Ausbeute: 3.10 g (64.2 % d. Theorie)
$C_{12}H_{16}N_4$ (M= 216,288)
ber.: Molpeak $(M+H)^+$: 217 gef.: Molpeak $(M+H)^+$: 217

3.40e 5-Brom-2-pyrrolidin-1-ylmethyl-1H-benzimidazol

3.10 g (14.3 mmol) 2-Pyrrolidin-1-ylmethyl-1*H*-benzimidazol-5-ylamin wird in 32.2 mL 48% Bromwasserstoffsäure und 32.2 mL Wasser suspendiert und die Lösung wird auf 0˚C abgekühlt. 2.5 M Natriumnitrit-Lösung (1.68 g in 9.7 mL Wasser) wird langsam zugetropft, so dass die Innentemperatur 5˚C nicht überschreitet. Die Reaktion wird 10 min bei 0˚C gerührt und dann wird 3.50 g (24.37 mmol) CuBr in 11.3 mL 48% Bromwasserstoffsäure zugetropft. Die Reaktion wird auf 60˚C erwärmt und eine Stunde bei dieser Temperatur gerührt. Das Lösungsmittel wird i.vac. entfernt und der Rückstand mit Isopropanol verrieben. Der Niederschlag wird abgesaugt und mit Isopropanol gewaschen. Die Reinigung erfolgt durch Säulenchromatographie an Kieselgel (MeOH/$NH_3$ 9:1).
Ausbeute: 2.20 g (54.8 % d. Theorie)
$C_{12}H_{14}BrN_3$ (M= 280,169)
ber.: Molpeak $(M+H)^+$: 280/282 gef.: Molpeak $(M+H)^+$: 280/282
Retentionszeit HPLC: 4.47 min (Methode A)

3.40f 5-Iod-2-pyrrolidin-1-ylmethyl-1*H*-benzimidazol

Hergestellt nach der allgemeinen Arbeitsvorschrift II aus 5-Brom-2-pyrrolidin-1-ylmethyl-1*H*-benzimidazol (700 mg, 2.50 mmol).
Ausbeute: 200 mg (24.5 % d. Theorie)
$C_{12}H_{14}IN_3$ (M= 327,170)
ber.: Molpeak $(M+H)^+$: 328 gef.: Molpeak $(M+H)^+$: 328
Retentionszeit HPLC: 4.55 min (Methode A)

3.40g 5-[5-(4-Chlor-phenyl)-pyridin-2-ylethinyl]-2-pyrrolidin-1-ylmethyl-1*H*-benzimidazol

Hergestellt nach der allgemeinen Arbeitsvorschrift I aus 5-Iod-2-pyrrolidin-1-ylmethyl-1*H*-benzimidazol (200 mg, 0.61 mmol) und 5-(4-Chlor-phenyl)-2-ethinyl-pyridin (131 mg, 0.61 mmol).
Ausbeute: 5 mg (2.0 % d. Theorie)
$C_{25}H_{21}ClN_4$ (M= 412,926)
ber.: Molpeak (M-H)-: 411/413 gef.: Molpeak (M-H)-: 411/413
Retentionszeit HPLC: 3.94 min (Methode A)

**Beispiel 3.41**

5-[5-(4-Chlor-phenyl)-pyridin-2-ylethinyl]-1-(2-pyrrolidin-1-yl-ethyl)-1H-benzim idazol

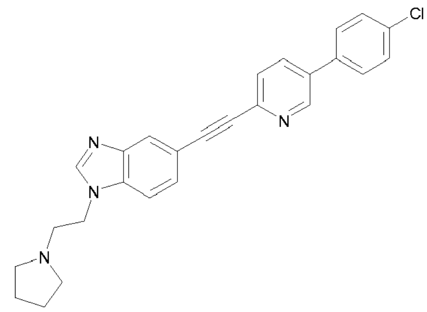

3.41a 5-Brom-1-(2-pyrrolidin-1-yl-ethyl)-1*H*-benzimidazol

Eine Lösung von 904 mg (3.18 mmol) 4-Brom-$N^1$-(2-pyrrolidin-1-yl-ethyl)-benzol-1,2-diamin in 5 mL Ameisensäure wird 1.5 h unter Rückfluß erhitzt. Mit halbgesättigter $NaHCO_3$-Lösung stellt man alkalisch und extrahiert zweimal mit je 70 mL EtOAc. Die organische Phase wird über $MgSO_4$ getrocknet und das Lösungsmittel i.vac. entfernt.
Ausbeute: 750 mg (80.2 % d. Theorie)
$C_{13}H_{16}BrN_3$ (M= 294,197)
ber.: Molpeak $(M+H)^+$: 294/296 gef.: Molpeak $(M+H)^+$: 294/296
Retentionszeit HPLC: 3.78 min (Methode A)

3.41b 5-Iod-1-(2-pyrrolidin-1-yl-ethyl)-1*H*-benzimidazol

Hergestellt nach der allgemeinen Arbeitsvorschrift II aus 5-Brom-1-(2-pyrrolidin-1-yl-ethyl)-1*H*-benzimidazol (750 mg, 2.55 mmol).
Ausbeute: 680 mg (78.2 % d. Theorie)
$C_{13}H_{16}IN_3$ (M= 341,197)
ber.: Molpeak $(M+H)^+$: 342 gef.: Molpeak $(M+H)^+$: 342
Retentionszeit HPLC: 4.04 min (Methode A)

3.41c 5-[5-(4-Chlor-phenyl)-pyridin-2-ylethinyl]-1-(2-pyrrolidin-1-yl-ethyl)-1*H*-benzimidazol

Hergestellt nach der allgemeinen Arbeitsvorschrift I aus 5-Iod-1-(2-pyrrolidin-1-yl-ethyl)-1*H*-benzimidazol (150 mg, 0.44 mmol) und 5-(4-Chlor-phenyl)-2-ethinyl-pyridin (94 mg, 0.44 mmol).
Ausbeute: 26 mg (13.7 % d. Theorie)
$C_{26}H_{23}ClN_4$ (M= 426,953)
ber.: Molpeak $(M+H)^+$: 427/429 gef.: Molpeak $(M+H)^+$: 427/429
Retentionszeit HPLC: 6.51 min (Methode A)

**Beispiel 3.42**

2-[5-(4-Chlor-phenyl)-pyridin-2-ylethinyl]-1-methyl-5-pyrrolidin-1-ylmethyl-1H-benzimidazol

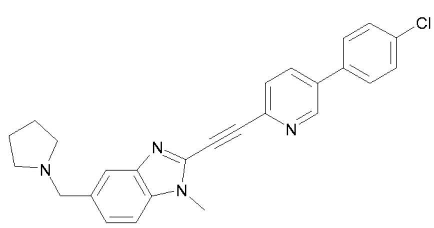

3.42a Methyl-(2-nitro-4-pyrrolidin-1-ylmethyl-phenyl)-amin

Zu einer Lösung von 4.70 g (26.1 mmol) 4-Methylamino-3-nitro-benzaldehyd in 100 mL THF werden 5.55 g

(78.0 mmol) Pyrrolidin gegeben und das Reaktionsgemisch mit Eisessig sauer gestellt. 6.36 g (30.0 mmol) $NaBH(OAc)_3$ werden zugegeben und das Reaktionsgemisch bei RT über Nacht gerührt. Das Gemisch wird mit gesättigter $NaHCO_3$Lösung versetzt und die wässrige Phase wird zweimal mit EtOAc extrahiert. Die vereinigten organischen Extrakte werden mit 200 mL halbgesättigter $NaHCO_3$-Lösung gewaschen und über $MgSO_4$ getrocknet. Das Lösungsmittel wird i.vac. entfernt und die weitere Reinigung erfolgt durch Säulenchromatographie an Kieselgel (Gradient: DCM nach DCM/MeOH 9:1).
Ausbeute: 2.00 g (32.6 % d. Theorie)
$C_{12}H_{17}N_3O_2$ (M= 235,288)
ber.: Molpeak $(M+H)^+$: 236 gef.: Molpeak $(M+H)^+$: 236
$R_f$-Wert: 0.15 (Kieselgel, DCM/MeOH 9:1)

3.42b $N^1$-Methyl-4-pyrrolidin-1-ylmethyl-benzol-1,2-diamin

Zu einer Lösung von 1.00 g (4.25 mmol) Methyl-(2-nitro-4-pyrrolidin-1-ylmethyl-phenyl)-amin in 60 mL EtOAc werden bei RT 4.85 g (21.5 mmol) Zinn(II)chlorid Dihydrat und 4.45 g (53.0 mmol) $NaHCO_3$ zugegeben. Die Reaktion wird 2 h unter Rückfluß erhitzt und dann mit 100 mL 1 M $KHSO_4$-Lösung und etwas Wasser verdünnt. Das Gemisch wird filtriert. Die wässrige Phase wird mit $K_2CO_3$ versetzt und zweimal mit je 80 mL EtOAc extrahiert. Die organische Phase wird über $MgS0_4$ getrocknet und das Lösungsmittel i.vac. entfernt.
Ausbeute: 850 mg (97.4 % d. Theorie)
$C_{12}H_{19}N_3$ (M= 205,305)
ber.: Molpeak $(M+H)^+$: 206 gef.: Molpeak $(M+H)^+$: 206
$R_f$-Wert: 0.15 (Kieselgel, DCM/MeOH/$NH_3$ 9:1:0.1)

3.42c 1-Methyl-5-pyrrolidin-1-ylmethyl-1*H*-benzimidazol

Eine Lösung von 850 mg (4.14 mmol) $N^1$-Methyl-4-pyrrolidin-1-ylmethyl-benzol-1,2-diamin in 4 mL Ameisensäure wird 1.5 h unter Rückfluß erhitzt. Mit 250 mL halbgesättigter $NaHCO_3$-Lösung stellt man alkalisch und extrahiert zweimal mit je 70 mL EtOAc. Die organische Phase wird über $MgSO_4$ getrocknet und das Lösungsmittel i.vac. entfernt.
Ausbeute: 650 mg (72.9 % d. Theorie)
$C_{13}H_{17}N_3$ (M= 215,301)
ber.: Molpeak $(M+H)^+$: 216 gef.: Molpeak $(M+H)^+$: 216
$R_f$-Wert: 0.25 (Kieselgel, DCM/MeOH/$NH_3$ 9:1:0.1)

3.42d 2-Iod-1-methyl-5-pyrrolidin-1-ylmethyl-1*H*-benzimidazol

Zu einer auf -75˚C gekühlten Lösung von 250 mg (1.16 mmol) 1-Methyl-5-pyrrolidin-1-ylmethyl-1H-benzimidazol in 8 mL THF werden 0.80 mL (1.28 mmol) 1.6 M n-Butyllithium-Lösung in Hexan gegeben. Das Reaktionsgemisch wird 10 min bei dieser Temperatur gerührt und dann werden 288 mg (1.28 mmol) N-Iodsuccinimid in 5 mL THF zugegeben. Das Kühlbad wird entfernt und die Reaktion 1 h bei RT gerührt. 12 mL 0.1 M HCl werden zugegeben und die wässrige Phase mit EtOAc extrahiert. Die organische Phase wird über $MgSO_4$ getrocknet und das Lösungsmittel i.vac. entfernt. Die Reinigung erfolgt durch Säulenchromatographie an Kieselgel (Gradient: DCM nach DCM/MeOH/$NH_3$ 9:1:0.1).
Ausbeute: 140 mg (22.2 % d. Theorie)
$C_{13}H_{16}IN_3$ (M= 341,197)
ber.: Molpeak $(M+H)^+$: 342 gef.: Molpeak $(M+H)^+$: 342
$R_f$-Wert: 0.20 (Kieselgel, DCM/MeOH/$NH_3$ 9:1:0.1)
Retentionszeit HPLC: 3.89 min (Methode A)

3.42e 2-[5-(4-Chlor-phenyl)-pyridin-2-ylethinyl]-1-methyl-5-pyrrolidin-1-ylmethyl-1H-benzimidazol

Hergestellt nach der allgemeinen Arbeitsvorschrift I aus 2-Iod-1-methyl-5-pyrrolidin-1-ylmethyl-1*H*-benzimidazol (100 mg, 0.29 mmol) und 5-(4-Chlor-phenyl)-2-ethinyl-pyridin (68 mg, 0.32 mmol).
Ausbeute: 9 mg (7.2 % d. Theorie)
$C_{26}H_{23}ClN_4$ (M= 426,953)
ber.: Molpeak $(M+H)^+$: 427/429 gef.: Molpeak $(M+H)^+$: 427/429
$R_f$-Wert: 0.20 (Kieselgel, DCM/MeOH/$NH_3$ 9:1:0.1)
Retentionszeit HPLC: 6.69 min (Methode A)

Beispiel 3.43

5-(4-Chlor-phenyl)-2-[2-fluor-4-(2-pyrrolidin-1-yl-ethoxy)-phenylethinyl]-pyridin

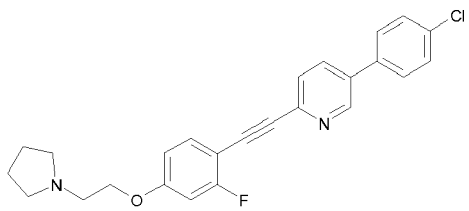

3.43a 1-[2-(3-Fluor-4-iod-phenoxy)-ethyl]-pyrrolidin

Analog Beispiel 3.1 e wird aus 13.6 g (57.0 mmol) 3-Fluor-4-iod-phenol und 9.69 g (57.0 mmol) N-(2-Chlorethyl)-pyrrolidin Hydrochlorid das Produkt erhalten.
Ausbeute: 17.1 g (89.6 % d. Theorie)
$C_{12}H_{15}FINO$ (M= 335,162)
ber.: Molpeak $(M+H)^+$: 336 gef.: Molpeak $(M+H)^+$: 336
$R_f$-Wert: 0.57 (Kieselgel, EtOAc/MeOH/$NH_3$ 95:5:0.5).

3.43b 5-(4-Chlor-phenyl)-2-[2-fluor-4-(2-pyrrolidin-1-yl-ethoxy)-phenylethinyl]-pyridin

Hergestellt nach der allgemeinen Arbeitsvorschrift I aus 1-[2-(3-Fluor-4-iod-phenoxy)-ethyl]-pyrrolidin (500 mg, 0.75 mmol) und 5-(4-Chlor-phenyl)-2-ethinyl-pyridin (159 mg, 0.75 mmol).
Ausbeute: 48 mg (15.4 % d. Theorie)
$C_{25}H_{22}ClFN_2O$ (M= 420,918)
ber.: Molpeak $(M+H)^+$: 421/423 gef.: Molpeak $(M+H)^+$: 421/423
$R_f$-Wert: 0.65 (Kieselgel, EtOAc/MeOH/$NH_3$ 9:1:0.1)
Retentionszeit HPLC: 7.74 min (Methode A)

**Beispiel 3.44**

5-[5-(4-Chlor-phenyl)-pyridin-2-ylethinyl]-8-(2-pyrrolidin-1-yl-ethoxy)-chinolin

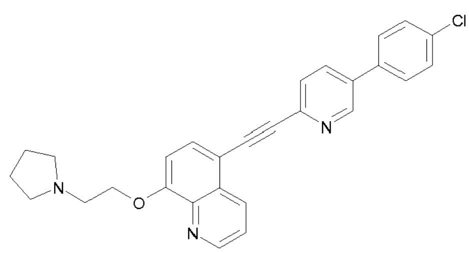

3.44a 5-Iod-8-(2-pyrrolidin-1-yl-ethoxy)-chinolin

Analog Beispiel 3.1 e wird aus 700 mg (2.58 mmol) 5-Iod-chinolin-8-ol und 450 mg (2.59 mmol) N-(2-Chlorethyl)-pyrrolidin Hydrochlorid das Produkt erhalten.
Ausbeute: 829 mg (87.2 % d. Theorie)
$C_{15}H_{17}1N_2O$ (M=368,220)
ber.: Molpeak $(M+H)^+$: 369 gef.: Molpeak $(M+H)^+$: 369
Retentionszeit HPLC: 5.56 min (Methode B)

3.44b 5-[5-(4-Chlor-phenyl)-pyridin-2-ylethinyl]-8-(2-pyrrolidin-1-yl-ethoxy)-chinolin

Hergestellt nach der allgemeinen Arbeitsvorschrift I aus 5-Iod-8-(2-pyrrolidin-1-yl-ethoxy)-chinolin (200 mg, 0.54 mmol) und 5-(4-Chlor-phenyl)-2-ethinyl-pyridin (116 mg, 0.54 mmol).
Ausbeute: 23 mg (9.4 % d. Theorie)
$C_{28}H_{24}ClN_3O$ (M= 453,976)
ber.: Molpeak $(M+H)^+$: 454/456 gef.: Molpeak $(M+H)^+$: 454/456
Retentionszeit HPLC: 7.40 min (Methode A)

**Beispiel 3.45**

6-[5-(4-Chlor-phenyl)-pyridin-2-ylethinyl]-2-pyrrolidin-1-ylmethyl-chinolin

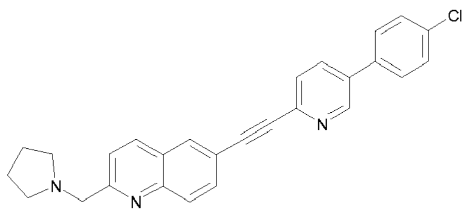

3.45a 6-Brom-2-brommethyl-chinolin

Zu einer Lösung von 10.0 g (45.0 mmol) 6-Brom-2-methyl-chinolin in 60 mL Tetrachlorkohlenstoff werden nacheinander 148 mg (1.00 mmol) $\alpha,\alpha$-Azoisobutyronitril und 8.01 g (45.0 mmol) N-Bromsuccinimid gegeben. Das Reaktionsgemisch wird 8 h unter Rückfluß erhitzt. Es wird filtriert und das Filtrat zweimal mit Wasser gewaschen. Die organische Phase wird über $MgSO_4$ getrocknet und das Lösungsmittel i.vac. entfernt. Die Reinigung erfolgt durch Säulenchromatographie an Kieselgel (PE/EtOAc 4:1).
Ausbeute: 5.10 g (37.7 % d. Theorie)
$C_{10}H_7Br_2N$ (M= 300,982)
ber.: Molpeak $(M+H)^+$: 300/302/304 gef.: Molpeak $(M+H)^+$: 300/302/304
Retentionszeit HPLC: 5.75 min (Methode B)

3.45b 6-Brom-2-pyrrolidin-1-ylmethyl-chinolin

Zu einer Lösung von 1.40 mL (16.8 mmol) Pyrrolidin und 6.34 g (45.9 mmol) $K_2C0_3$ in 50 mL Acetonitril werden 4.60 g (15.28 mmol) 6-Brom-2-brommethyl-chinolin gegeben. Die Reaktion wird über Nacht bei RT gerührt und dann werden die anorganischen Salze durch Filtration entfernt. Die organische Phase wird mit Wasser gewaschen und die wässrige Phase mit EtOAc extrahiert. Die vereinigten organischen Extrakte werden über $MgS0_4$ getrocknet und das Lösungsmittel i.vac. entfernt.
Ausbeute: 4.45 g (100 % d. Theorie)
$C_{14}H_{15}BrN_2$ (M= 291,193)
ber.: Molpeak $(M+H)^+$: 291/293 gef.: Molpeak $(M+H)^+$: 291/293
$R_f$-Wert: 0.27 (Kieselgel, DCM/MeOH 9:1)

3.45c 6-Iod-2-pyrrolidin-1-ylmethyl-chinolin

Hergestellt nach der allgemeinen Arbeitsvorschrift II aus 6-Brom-2-pyrrolidin-1-ylmethyl-chinolin (500 mg, 1.72 mmol).
Ausbeute: 400 mg (59.9 % d. Theorie)
$C_{14}H_{15}IN_2$ (M= 338,193)
ber.: Molpeak $(M+H)^+$: 339 gef.: Molpeak $(M+H)^+$: 339
Retentionszeit HPLC: 5.16 min (Methode A)

3.45d 6-[5-(4-Chlor-phenyl)-pyridin-2-ylethinyl]-2-pyrrolidin-1-ylmethyl-chinolin

Hergestellt nach der allgemeinen Arbeitsvorschrift I aus 6-Iod-2-pyrrolidin-1-ylmethyl-chinolin (151 mg, 0.45 mmol) und 5-(4-Chlor-phenyl)-2-ethinyl-pyridin (80 mg, 0.37 mmol).
Ausbeute: 18 mg (11.4 % d. Theorie)
$C_{27}H_{22}ClN_3$ (M= 423,949)
ber.: Molpeak $(M+H)^+$: 424/426 gef.: Molpeak $(M+H)^+$: 424/426
Retentionszeit HPLC: 4.78 min (Methode B)

**Beispiel 3.46**

6-[5-(4-Chlor-phenyl)-pyridin-2-ylethinyl]-2-pyrrolidin-1-ylmethyl-1,2,3,4-tetrahydrochinolin

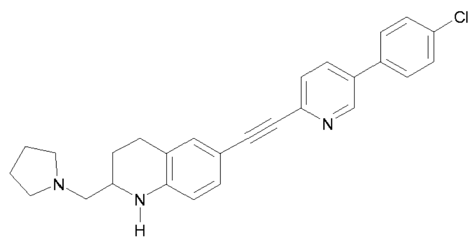

3.46a 6-Brom-2-pyrrolidin-1-ylmethyl-1,2,3,4-tetrahydro-chinolin

Unter Argonatmosphäre werden zu einer Lösung von 500 mg (1.72 mmol) 6-Brom-2-pyrrolidin-1-ylmethyl-chinolin (siehe 3.45b) in 10 mL Essigsäure bei RT 0.69 mL (6.87 mmol) Boran-Pyridin-Komplex zugegeben. Das Gemisch wird 7 h bei RT gerührt, erneut mit 0.35 mL (3.46 mmol) Boran-Pyridin-Komplex versetzt und eine weitere Stunde bei RT gerührt. Es wird auf 0˚C abgekühlt und die Lösung mit 8% NaOH-Lösung basisch gestellt. Die wässrige Phase wird mit EtOAc extrahiert und das Lösungsmittel i.vac. entfernt. Der Rückstand wird mit Wasser aufgenommen und mit 12% HCl angesäuert. Die wässrige Phase wird mit EtOAc extrahiert und dann unter Eiskühlung mit 20% NaOH-Lösung basisch gestellt. Die wässrige Phase wird mit EtOAc extrahiert. Die organische Phase wird über $MgSO_4$ getrocknet und das Lösungsmittel i.vac. entfernt.
Ausbeute: 420 mg (82.9 % d. Theorie)
$C_{14}H_{19}BrN_2$ (M= 295,25)
ber.: Molpeak $(M+H)^+$: 295/297 gef.: Molpeak $(M+H)^+$: 295/297
Retentionszeit HPLC: 5.01 min (Methode B)

3.46b 6-Iod-2-pyrrolidin-1-ylmethyl-1,2,3,4-tetrahydro-chinolin

Hergestellt nach der allgemeinen Arbeitsvorschrift II aus 6-Brom-2-pyrrolidin-1-ylmethyl-1,2,3,4-tetrahydro-chinolin (280 mg, 0.95 mmol).
Ausbeute: 260 mg (80.1 % d. Theorie)
$C_{14}H_{19}IN_2$ (M= 342,225)
ber.: Molpeak $(M+H)^+$: 343 gef.: Molpeak $(M+H)^+$: 343
Retentionszeit HPLC: 5.34 min (Methode A)

3.46c 6-[5-(4-Chlor-phenyl)-pyridin-2-ylethinyl]-2-pyrrolidin-1-ylmethyl-1,2,3,4-tetrahydro-chinolin

Hergestellt nach der allgemeinen Arbeitsvorschrift I aus 6-Iod-2-pyrrolidin-1-ylmethyl-1,2,3,4-tetrahydro-chinolin (260 mg, 0.76 mmol) und 5-(4-Chlor-phenyl)-2-ethinyl-pyridin (162 mg, 0.76 mmol).
Ausbeute: 72 mg (22.1 % d. Theorie)
$C_{27}H_{26}ClN_3$ (M= 427,981)
ber.: Molpeak $(M+H)^+$: 428 gef.: Molpeak $(M+H)^+$: 428
Retentionszeit HPLC: 4.66 min (Methode B)

**Beispiel 3.47**

5-(4-Chlor-phenyl)-2-(6-pyrrolidin-1-ylmethyl-naphthalen-2-ylethinyl)-pyridin

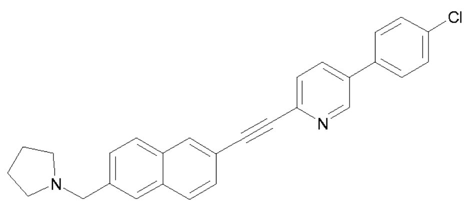

3.47a (6-Iod-naphthalen-2-yl)-methanol

Hergestellt nach der allgemeinen Arbeitsvorschrift II aus (6-Brom-naphthalen-2-yl)-methanol (500 mg, 2.11 mmol).
Ausbeute: 450 mg (75.1 % d. Theorie)
$C_{11}H_9IO$ (M= 284,10)
ber.: Molpeak $(M+H)^+$: 284 gef.: Molpeak $(M+H)^+$: 284
Retentionszeit HPLC: 8.30 min (Methode A)

3.47b {6-[5-(4-Chlor-phenyl)-pyridin-2-ylethinyl]-naphthalen-2-yl}-methanol

Hergestellt nach der allgemeinen Arbeitsvorschrift I aus (6-Iod-naphthalen-2-yl)-methanol (450 mg, 1.58 mmol) und 5-(4-Chlor-phenyl)-2-ethinyl-pyridin (342 mg, 1.60 mmol).
Ausbeute: 250 mg (42.8 % d. Theorie)
$C_{24}H_{16}ClN0$ (M= 369,85)
ber.: Molpeak $(M+H)^+$: 370/372 gef.: Molpeak $(M+H)^+$: 370/372
$R_f$-Wert: 0.25 (Kieselgel, DCM/MeOH 19:1)

3.47c 5-(4-Chlor-phenyl)-2-(6-pyrrolidin-1-ylmethyl-naphthalen-2-ylethinyl)-pyridin

Zu einer Lösung von 148 mg (0.40 mmol) {6-[5-(4-Chlor-phenyl)-pyridin-2-ylethinyl]-naphthalen-2-yl}-methanol in 5 mL DCM werden 58 µL (0.80 mmol) Thionylchlorid bei 0°C zugegeben. Die Lösung wird auf RT erwärmt und 1 h bei dieser Temperatur gerührt. Das Reaktionsgemisch wird mit 30 mL DCM verdünnt, mit Eiswasser versetzt, mit gesättigter $NaHCO_3$-Lösung alkalisch gestellt und die organische Phase mit Wasser gewaschen. Die organische Phase wird über $MgSO_4$ getrocknet und filtriert. Zum Filtrat werden 0.10 mL (1.20 mmol) Pyrrolidin gegeben, 2 h bei RT und 1 h bei 40°C gerührt. Das Lösungsmittel wird i.vac. entfernt und die Reinigung erfolgt durch Säulenchromatographie an Kieselgel (Gradient: DCM nach DCM/MeOH/$NH_3$ 5:1:0.1).
Ausbeute: 40 mg (23.6 % d. Theorie)
$C_{28}H_{23}ClN_2$ (M= 422,96)
ber.: Molpeak $(M+H)^+$: 423/425 gef.: Molpeak $(M+H)^+$: 423/425
$R_f$-Wert: 0.10 (Kieselgel, DCM/MeOH/$NH_3$ 19:1:0.1)

**Beispiel 3.48**

5-[5-(4-Chlor-phenyl)-pyridin-2-ylethinyl]-1-(2-pyrrolidin-l-yl-ethyl)-2,3-dihydro-1*H*-indol

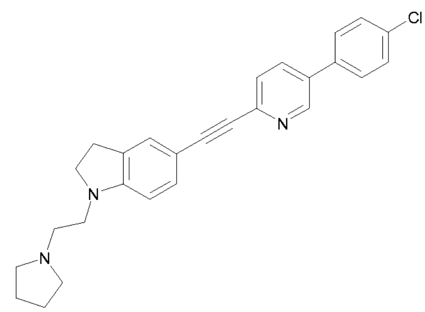

3.48a 5-Brom-1-(2-pyrrolidin-1-yl-ethyl)-2,3-dihydro-1*H*-indol

Unter Stickstoffatmosphäre werden zu einer Lösung von 722 mg (4.16 mmol) N-(2-Chlorethyl)-pyrrolidin Hydrochlorid und 1.19 mL (6.93 mmol) Ethyldiisopropylamin in 10 mL DMF 700 mg (3.46 mmol) 5-Bromindolin zugegeben. Die Reaktionslösung wird 21 h bei RT gerührt und erneut mit N-(2-Chlorethyl)-pyrrolidin Hydrochlorid versetzt. Die Reaktionslösung wird auf 70˚C erwärmt und 4 h bei dieser Temperatur gerührt. Das Lösungsmittel wird i.vac. entfernt und der Rückstand in 50 mL halbgesättigter NaCl-Lösung und 50 mL EtOAc aufgenommen. Die wässrige Phase wird zweimal mit 50 mL DCM extrahiert, die vereinigten organischen Extrakte über $Na_2SO_4$ getrocknet und das Lösungsmittel i.vac. entfernt.
Ausbeute: 226 mg (22.1 % d. Theorie)
$C_{14}H_{19}BrN_2$ (M= 295,225)
ber.: Molpeak $(M+H)^+$: 295/297 gef.: Molpeak $(M+H)^+$: 295/297
Retentionszeit HPLC: 5.93 min (Methode A)

3.48b 5-Iod-1-(2-pyrrolidin-1-yl-ethyl)-2,3-dihydro-1*H*-indol

Hergestellt nach der allgemeinen Arbeitsvorschrift II aus 5-Brom-1-(2-pyrrolidin-1-yl-ethyl)-2,3-dihydro-1*H*-indol (226 mg, 0.77 mmol).
Ausbeute: 142 mg (54.2 % d. Theorie)
$C_{14}H_1gIN_2$ (M= 342,225)
Retentionszeit HPLC: 6.10 min (Methode A)

3.48c 5-[5-(4-Chlor-phenyl)-pyridin-2-ylethinyl]-1-(2-pyrrolidin-1-yl-ethyl)-2,3-dihydro-1*H*-indol

Hergestellt nach der allgemeinen Arbeitsvorschrift I aus 5-Iod-1-(2-pyrrolidin-1-yl-ethyl)-2,3-dihydro-1*H*-indol (142 mg, 0.42 mmol) und 5-(4-Chlor-phenyl)-2-ethinyl-pyridin (89 mg, 0.42 mmol).
Ausbeute: 39 mg (22.1 % d. Theorie)
$C_{27}H_{26}ClN_3$ (M= 427,981)
ber.: Molpeak $(M+H)^+$: 428/430 gef.: Molpeak $(M+H)^+$: 428/430
$R_f$-Wert: 0.55 (Alox, Cyc/EtOAc 2:1)
Retentionszeit HPLC: 7.98 min (Methode A)

**Beispiel 3.49**

5-(4-Chlor-phenyl)-2-[4-(1-methyl-2-piperidin-1-yl-ethoxy)-phenylethinyl]-pyridin

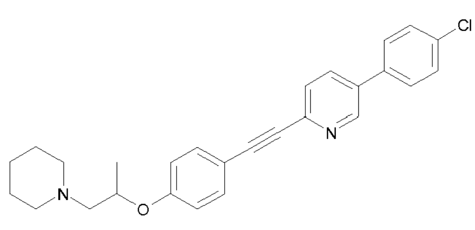

3.49a 1-[2-(4-Iod-phenoxy)-propyl]-piperidin

Zu einer Lösung von 1.00 g (6.98 mmol) 1-Piperidin-1-yl-propan-2-ol in 20 mL DCM werden nacheinander 1.54 g (7.00 mmol) Iodbenzol und 2.75 g (10.5 mmol) Triphenylphosphan gegeben. 2.19 mL (10.5 mmol, 95%) Azodicarbonsäurediisopropylester wird bei RT zugetropft und die Reaktion 2 h bei RT gerührt. Es wird mit Wasser verdünnt, die organische Phase mit Wasser gewaschen und über $MgSO_4$ getrocknet. Nach Entfernen des Trocken- und Lösungsmittels wird der Rückstand chromatographisch (Kieselgel, DCM/MeOH 9:1) gereinigt. Der ölige Rückstand wird mit Diisopropylether verrieben, vom unlöslichen Rückstand abfiltriert und das Filtrat wird i.vac. zur Trockene eingedampft.
Ausbeute: 500 mg (20.7 % d. Theorie)
$C_{14}H_{20}INO$ (M= 345,226)
ber.: Molpeak $(M+H)^+$: 346 gef.: Molpeak $(M+H)^+$: 346
$R_f$-Wert: 0.32 (Kieselgel, DCM/MeOH 9:1)

3.49b 5-(4-Chlor-phenyl)-2-[4-(1-methyl-2-piperidin-1-yl-ethoxy)-phenylethinyl]-pyridin

Hergestellt nach der allgemeinen Arbeitsvorschrift I aus 1-[2-(4-Iod-phenoxy)-propyl]-piperidin (173 mg, 0.50 mmol) und 5-(4-Chlor-phenyl)-2-ethinyl-pyridin (107 mg, 0.50 mmol).
Ausbeute: 80 mg (37.1 % d. Theorie)
$C_{27}H_{27}ClN_2O$ (M= 430,982)
ber.: Molpeak $(M+H)^+$: 431/433 gef.: Molpeak $(M+H)^+$: 431/433
$R_f$-Wert: 0.25 (Kieselgel, EtOAc/MeOH 9:1)
Retentionszeit HPLC: 5.03 min (Methode A)

**Beispiel 3.50**

5-(4-Chlor-phenyl)-2-[4-(3-piperidin-1-yl-pyrrolidin-1-yl)-phenylethinyl]-pyridin

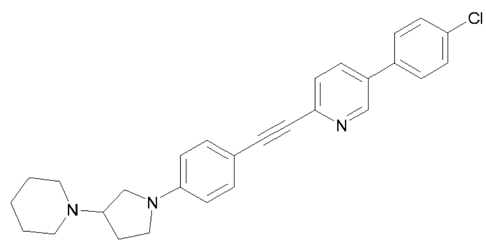

3.50a 1-(1-Benzyl-pyrrolidin-3-yl)-piperidin

Zu einer Lösung von 4.94 mL (50.0 mmol) Piperidin und 8.03 mL (50.0 mmol) N-Benzylpyrrolidinon in 200 mL THF werden 12.7 g (60.0 mmol) $NaBH(OAc)_3$ und 2.3 mL Essigsäure gegeben. Die Reaktion wird über Nacht bei RT gerührt. Die Reaktionslösung wird mit 200 mL gesättigter $NaHCO_3$-Lösung versetzt und zweimal mit je 200 mL EtOAc extrahiert. Die organische Phase wird über $MgSO_4$ getrocknet und das Lösungsmittel i.vac. entfernt. Die Reinigung erfolgt durch Säulenchromatographie an Kieselgel (EtOAc/MeOH/$NH_3$ 8:2:0.2).
Ausbeute: 5.50 g (45.0 % d. Theorie)
$C_{16}H_{24}N_2$ (M= 244,383)
ber.: Molpeak $(M+H)^+$: 245 gef.: Molpeak $(M+H)^+$: 245
$R_f$-Wert: 0.25 (Kieselgel, EtOAc/MeOH/$NH_3$ 9:1:0.1)

3.50b 1-Pyrrolidin-3-yl-piperidin

Zu einer Lösung von 5.50 g (22.5 mmol) 1-(1-Benzyl-pyrrolidin-3-yl)-piperidin in 200 mL MeOH werden 550 mg 10% Pd/C gegeben. Die Reaktionslösung wird 5 h bei RT und 3 bar $H_2$ gerührt. 550 mg Palladiumhydroxid werden zugegeben und die Reaktion weitere 6 h bei RT und 3 bar $H_2$ gerührt. Der Katalysator wird abgesaugt und das Lösungsmittel i.vac. entfernt.
Ausbeute: 900 mg (86.5 % d. Theorie)

$C_9H_{18}N_2$ (M= 154,257)
ber.: Molpeak $(M+H)^+$: 155 gef.: Molpeak $(M+H)^+$: 155
$R_f$-Wert: 0.05 (Kieselgel, EtOAc/MeOH/$NH_3$ 8:2:0.2)

3.50c 1-[1-(4-Brom-phenyl)-pyrrolidin-3-yl]-piperidin

283 mg (1.00 mmol) 4-Brom-iodbenzol, 10 mg (0.05 mmol) CuI, 124 mg (2.00 mmol) Ethylenglykol und 424 mg (2.00 mmol) Kaliumphosphat werden in ein Reaktionsgefäß gegeben, das mehrmals evakuiert und mit Argon gespült wird.
Dann werden 154 mg (1.00 mmol) 1-Pyrrolidin-3-yl-piperidin in 1 mL Isopropanol zugegeben und die Reaktion 15 h bei 80°C geschüttelt. Die Reaktionslösung wird mit EtOAc verdünnt und zweimal mit 5% Ammoniaklösung extrahiert. Die organische Phase wird über $MgSO_4$ getrocknet und das Lösungsmittel i.vac. entfernt.
Ausbeute: 230 mg (74.4 % d. Theorie)
$C_{15}H_{21}BrN_2$ (M= 309,252)
ber.: Molpeak $(M+H)^+$: 309/311 gef.: Molpeak $(M+H)^+$: 309/311
$R_f$-Wert: 0.73 (Kieselgel, DCM/MeOH/$NH_3$ 9:1:0.1)

3.50d 1-[1-(4-Iod-phenyl)-pyrrolidin-3-yl]-piperidin

Hergestellt nach der allgemeinen Arbeitsvorschrift II aus 1-[1-(4-Brom-phenyl)-pyrrolidin-3-yl]-piperidin (200 mg, 0.65 mmol).
Ausbeute: 120 mg (52.1 % d. Theorie)
$C_{15}H_{21}IN_2$ (M= 356,252)
ber.: Molpeak $(M+H)^+$: 357 gef.: Molpeak $(M+H)^+$: 357
Retentionszeit HPLC: 6.13 min (Methode A)

3.50e 5-(4-Chlor-phenyl)-2-[4-(3-piperidin-1-yl-pyrrolidin-1-yl)-phenylethinyl]-pyridin

Hergestellt nach der allgemeinen Arbeitsvorschrift I aus 1-[1-(4-Iod-phenyl)-pyrrolidin-3-yl]-piperidin (120 mg, 0.34 mmol) und 5-(4-Chlor-phenyl)-2-ethinyl-pyridin (73 mg, 0.34 mmol).
Ausbeute: 75 mg (50.4 % d. Theorie)
$C_{28}H_{28}ClN_3$ (M= 442,008)
ber.: Molpeak $(M+H)^+$: 442/444 gef.: Molpeak $(M+H)^+$: 442/444
$R_f$-Wert: 0.30 (Kieselgel, DCM/MeOH/$NH_3$ 9:1:0.1)
Retentionszeit HPLC: 4.94 min (Methode B)

**Beispiel 3.51**

5-(4-Chlor-phenyl)-2-[5-(2-pyrrolidin-1-yl-ethoxy)- pyrid-2-yl-ethinyl]-pyridin

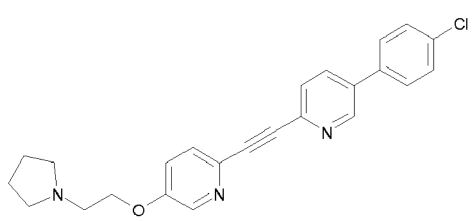

3.51a 2-Brom-5-(2-pyrrolidin-1-yl-ethoxy)-pyridin

Analog Beispiel 3.1 e (1:1 Mischung Aceton:Acetonitril statt DMF) wird aus 3.90 g (22.4 mmol) 6-Brom-pyridin-3-ol und 4.25 g (25.0 mmol) *N*-(2-Chlorethyl)-pyrrolidin Hydrochlorid das Produkt erhalten.
Ausbeute: 4.70 g (69.3 % d. Theorie)
$C_{11}H_{15}BrN_2O$ (M= 271,159)
ber.: Molpeak $(M+H)^+$: 271/273 gef.: Molpeak $(M+H)^+$: 271/273

$R_f$-Wert: 0.27 (Kieselgel, EtOAc/MeOH/$NH_3$ 9:1:0.1)

3.51b 5-(4-Chlor-phenyl)-2-[5-(2-pyrrolidin-1-yl-ethoxy)- pyrid-2-yl-ethinyl]-pyridin

Hergestellt nach der allgemeinen Arbeitsvorschrift I aus 2-Brom-5-(2-pyrrolidin-1-yl-ethoxy)-pyridin (271 mg, 0.50 mmol) und 5-(4-Chlor-phenyl)-2-ethinyl-pyridin (106 mg, 0.50 mmol).
Ausbeute: 22 mg (10.9 % d. Theorie)
$C_{24}H_{22}ClN_3O$ (M= 403,915)
ber.: Molpeak $(M+H)^+$: 404/406 gef.: Molpeak $(M+H)^+$: 404/406
$R_f$-Wert: 0.20 (Kieselgel, EtOAc/MeOH/$NH_3$ 9:1:0.1)

**Beispiel 3.52**

5-[5-(4-Chlor-phenyl)-pyridin-2-ylethinyl]-2-(2-pyrrolidin-1-yl-ethoxy)-benzonitril

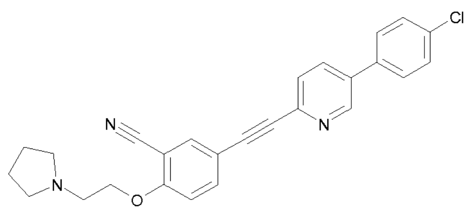

3.52a 5-Brom-2-(2-pyrrolidin-1-yl-ethoxy)-benzonitril

Analog Beispiel 3.1e (Acetonitril statt DMF) wird aus 2.00 g (10.1 mmol) 5-Brom-2-hydroxy-benzonitril und 2.00 g (11.8 mmol) N-(2-Chlorethyl)-pyrrolidin Hydrochlorid das Produkt erhalten.
Ausbeute: 1.32 g (44.3 % d. Theorie)
$C_{13}H_{15}BrN_2O$ (M= 295,181)
ber.: Molpeak $(M+H)^+$: 295/297 gef.: Molpeak $(M+H)^+$: 295/297
Retentionszeit HPLC: 4.91 min (Methode A)

3.52b 5-Iod-2-(2-pyrrolidin-1-yl-ethoxy)-benzonitril

Hergestellt nach der allgemeinen Arbeitsvorschrift II aus 5-Brom-2-(2-pyrrolidin-1-yl-ethoxy)-benzonitril (350 mg, 1.19 mmol).
Ausbeute: 324 mg (79.8 % d. Theorie)
$C_{13}H_{15}IN_2O$ (M= 342,182)
ber.: Molpeak $(M+H)^+$: 343 gef.: Molpeak $(M+H)^+$: 343
Retentionszeit HPLC: 5.14 min (Methode A)

3.52c 5-[5-(4-Chlor-phenyl)-pyridin-2-ylethinyl]-2-(2-pyrrolidin-1-yl-ethoxy)-benzonitril

Hergestellt nach der allgemeinen Arbeitsvorschrift I aus 5-Iod-2-(2-pyrrolidin-1-yl-ethoxy)-benzonitril (300 mg, 0.88 mmol) und 5-(4-Chlor-phenyl)-2-ethinyl-pyridin (206 mg, 0.97 mmol).
Ausbeute: 76 mg (20.3 % d. Theorie)
$C_{26}H_{22}ClN_3O$ (M= 427,938)
ber.: Molpeak $(M+H)^+$: 428/430 gef.: Molpeak $(M+H)^+$: 428/430
Retentionszeit HPLC: 7.31 min (Methode A)

**Beispiel 3.53**

5-(4-Chlor-phenyl)-2-[2-(4-methyl-piperidin-1-ylmethyl)-benzofuran-5-ylethinyl]-pyridin

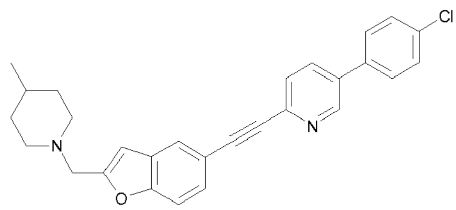

3.53a 5-Brom-benzofuran-2-carbonsäureethylester

Zu einer Lösung von 4.02 g (20.0 mmol) 5-Brom-salicylaldehyd und 2.26 mL (20.0 mmol, 98%) Bromessigester in 50 mL DMF werden 13.8 g (100 mmol) $Na_2CO_3$ gegeben. Das Reaktionsgemisch wird auf 80˚C erhitzt und 2 h bei dieser Temperatur gerührt. Es wird mit 200 mL Wasser verdünnt, die wässrige Phase dreimal mit je 100 mL *tert*-Butylmethylether extrahiert und die vereinigten organischen Extrakte zweimal mit je 50 mL Wasser gewaschen. Die organische Phase wird über $MgSO_4$ getrocknet, über Aktivkohle filtriert und das Lösungsmittel i.vac. entfernt.
Ausbeute: 3.80 g (70.6 % d. Theorie)
$C_{11}H_9BrO_3$ (M= 269,097)
ber.: Molpeak $(M+H)^+$: 269/271 gef.: Molpeak $(M+H)^+$: 269/271
$R_f$-Wert: 0.75 (Kieselgel, PE/EtOAc 8:2)

3.53b (5-Brom-benzofuran-2-yl)-methanol

Zu einer Lösung von 3.70 g (13.8 mmol) 5-Brom-benzofuran-2-carbonsäureethylester in 50 mL THF wird bei -5˚C langsam 7.0 mL (7.00 mmol) 1 M Lithiumaluminium-hydrid-Lösung in THF zugetropft. Die Reaktionslösung wird auf RT erwärmt und dann nochmals auf 10˚C abgekühlt. Weitere 0.7 mL (0.70 mmol) 1 M Lithiumaluminiumhydrid-Lösung in THF werden zugetropft und die Reaktion 1 h bei RT gerührt. Zum Reaktionsgemisch werden nacheinander 1.0 mL Wasser, 1.0 mL 15% NaOH und schließlich 3.0 mL Wasser gegeben und vom unlöslichen Niederschlag abfiltriert. Die organische Phase wird über $MgSO_4$ getrocknet, über Aktivkohle filtriert und das Lösungsmittel i.vac. entfernt.
Ausbeute: 2.10 g (67.3 % d. Theorie)
$C_9H_7BrO_2$ (M= 227,059)
ber.: Molpeak $(M)^+$: 226/228 gef.: Molpeak $(M)^+$: 226/228
$R_f$-Wert: 0.15 (Kieselgel, PE/EtOAc 8:2)

3.53c (5-Iod-benzofuran-2-yl)-methanol

Hergestellt nach der allgemeinen Arbeitsvorschrift II aus (5-Brom-benzofuran-2-yl)-methanol (2.10 g, 9.25 mmol).
Ausbeute: 2.53 g (100 % d. Theorie)
$C_9H_7IO_2$ (M= 274,059)
ber.: Molpeak $(M)^+$: 274 gef.: Molpeak $(M)^+$: 274
$R_f$-Wert: 0.26 (Kieselgel, PE/EtOAc 8:2)

3.53d {5-[5-(4-Chlor-phenyl)-pyridin-2-ylethinyl]-benzofuran-2-yl}-methanol

Hergestellt nach der allgemeinen Arbeitsvorschrift I aus (5-Iod-benzofuran-2-yl)-methanol (685 mg, 2.50 mmol) und 5-(4-Chlor-phenyl)-2-ethinyl-pyridin (534 mg, 2.50 mmol).
Ausbeute: 400 mg (44.5 % d. Theorie)
$C_{22}H_{14}ClNO_2$ (M= 359,815)
ber.: Molpeak $(M+H)^+$: 360/362 gef.: Molpeak $(M+H)^+$: 360/362
$R_f$-Wert: 0.58 (Kieselgel, DCM/MeOH/$NH_3$ 9:1:0.1 )

3.53e 5-(4-Chlor-phenyl)-2-[2-(4-methyl-piperidin-1-ylmethyl)-benzofuran-5-ylethinyl]-pyridin

Zu einer Lösung von 100 mg (0.28 mmol) {5-[5-(4-Chlor-phenyl)-pyridin-2-ylethinyl]-benzofuran-2-yl}-methanol und 69 μL (0.50 mmol) Triethylamin in 5 mL DCM werden bei 0°C 32 μL (0.40 mmol) Methansulfonsäurechlorid gegeben und die Reaktion 1 h bei dieser Temperatur gerührt. Nochmals werden 70 μL (0.89 mmol) Methansulfonsäurechlorid zugegeben und die Reaktion über Nacht bei RT gerührt. Dann wird 0,24 ml (2,00 mmol) 4-Methylpiperidin zugegeben und die Reaktion 2 Stunden bei RT gerührt. Die Reaktionslösung wird mit Wasser verdünnt und die wässrige Phase zweimal mit DCM extrahiert. Die organische Phase wird über $MgSO_4$ getrocknet und das Lösungsmittel i.vac. entfernt. Die Reinigung erfolgt durch Säulenchromatographie an Kieselgel (DCM/Methanol 9:1).
Ausbeute: 10 mg (8.1 % d. Theorie)
$C_{28}H_{25}ClN_2O$ (M= 440,977)
ber.: Molpeak $(M+H)^+$: 441/443 gef.: Molpeak $(M+H)^+$: 441/443
$R_f$-Wert: 0.27 (Kieselgel, DCM/MeOH 9:1 )

Beispiel 3.54

{4-[5-(4-Chlor-phenyl)-pyridin-2-ylethinyl]-phenyl}-(2-pyrrolidin-1 -yl-ethyl)-amin

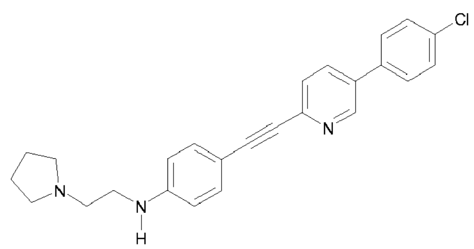

3.54a 2-Chlor-N-(4-iod-phenyl)-acetamid

Zu einer Lösung von 5.00 g (22.83 mmol) 4-Iod-phenylamin und 7.0 mL (50.2 mmol) Triethylamin in 100 mL DCM werden bei 0°C 2.0 mL (25.1 mmol) Chlor-acetylchlorid in 5 mL DCM zugegeben. Das Eisbad wird entfernt und die Reaktion wird weitere 1.5 h bei RT gerührt. Die Reaktionslösung wird mit 80 mL Wasser verdünnt und die organische Phase mit gesättigter NaCl-Lösung gewaschen. Die organische Phase wird über $MgS0_4$ getrocknet und das Lösungsmittel i.vac. entfernt. Der Rückstand wird mit EtOAc verrieben, abgesaugt und an der Luft getrocknet.
Ausbeute: 2.25 g (33.4 % d. Theorie)
$C_8H_7ClINO$ (M= 295,508)
ber.: Molpeak $(M+H)^+$: 296/298 gef.: Molpeak $(M+H)^+$: 296/298
Retentionszeit HPLC: 7.91 min (Methode A)

3.54b *N*-(4-Iod-phenyl)-2-pyrrolidin-1-yl-acetamid

Zu einer Lösung von 2.20 g (7.45 mmol) 2-Chlor-*N*-(4-iod-phenyl)-acetamid in 50 mL DCM werden 1.53 mL (18.6 mmol) Pyrrolidin gegeben. Die Reaktionslösung wird bei RT über Nacht gerührt. Die Mischung wird filtriert, das Filtrat über $MgSO_4$ getrocknet und das Lösungsmittel i.vac. entfernt.
Ausbeute: 1.65 g (67.1 % d. Theorie)
$C_{12}H_{15}IN_2O$ (M= 330,171)
ber.: Molpeak $(M+H)^+$: 331 gef.: Molpeak $(M+H)^+$: 331
Retentionszeit HPLC: 5.10 min (Methode A)

3.54c (4-Iod-phenyl)-(2-pyrrolidin-1-yl-ethyl)-amin

Zu einer Lösung von 500 mg (1.51 mmol) N-(4-Iod-phenyl)-2-pyrrolidin-1-yl-acetamid in 10 mL THF werden bei 0°C 2.25 mL (2.25 mmol) 1 M Lithiumaluminiumhydrid-Lösung gegeben und die Reaktion 20 min bei dieser Temperatur gerührt. Es wird EtOAc zugegeben und dann mit 85 μL Wasser, 85 μL 15% NaOH-Lösung und schließlich 256 μL Wasser versetzt. Es wird vom Niederschlag abgesaugt und das Filtrat mit 50 mL EtOAc verdünnt. Die organische

Phase wird mit 30 mL gesättigter $NaHCO_3$-Lösung gewaschen. Die organische Phase wird über $MgSO_4$ getrocknet und das Lösungsmittel i.vac. entfernt.
Ausbeute: 450 mg (94.0 % d. Theorie)
$C_{12}H_{17}1N_2$ (M= 316,187)
ber.: Molpeak $(M+H)^+$: 317 gef.: Molpeak $(M+H)^+$: 317
$R_f$-Wert: 0.17 (Kieselgel, DCM/MeOH/$NH_3$ 9:1:0.1 )

3.54d {4-[5-(4-Chlor-phenyl)-pyridin-2-ylethinyl]-phenyl}-(2-pyrrolidin-1-yl-ethyl)-amin

Hergestellt nach der allgemeinen Arbeitsvorschrift I aus (4-Iod-phenyl)-(2-pyrrolidin-1-yl-ethyl)-amin (450 mg, 1.42 mmol) und 5-(4-Chlor-phenyl)-2-ethinyl-pyridin (450 mg, 2.11 1 mmol).
Ausbeute: 98 mg (17.1 % d. Theorie)
$C_{25}H_{24}ClN_3$ (M= 401,943)
ber.: Molpeak $(M+H)^+$: 402/404 gef.: Molpeak $(M+H)^+$: 402/404
Retentionszeit HPLC: 7.08 min (Methode A)

Beispiel 3.55

5-[5-(4-Chlor-phenyl)-pyridin-2-ylethinyl]-2-(2-pyrrolidin-1-yl-ethoxy)-benzaldehyd

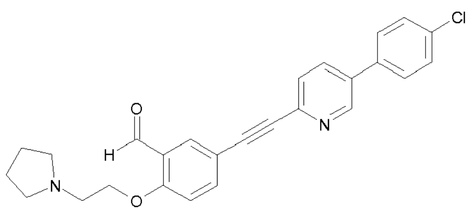

3.55a 5-Iod-2-(2-pyrrolidin-1-yl-ethoxy)-benzaldehyd

Analog Beispiel 3.1 e (Acetonitril statt DMF) wird aus 8.93 g (36.0 mmol) 2-Hydroxy-5-iod-benzaldehyd und 7.14 g (42.0 mmol) N-(2-Chlorethyl)-pyrrolidin Hydrochlorid das Produkt erhalten.
Ausbeute: 4.80 g (38.6 % d. Theorie)
$C_{13}H_{16}INO_2$ (M= 345,182)
ber.: Molpeak $(M+H)^+$: 346 gef.: Molpeak $(M+H)^+$: 346
Retentionszeit HPLC: 5.27 min (Methode A)

3.55b 5-[5-(4-Chlor-phenyl)-pyridin-2-ylethinyl]-2-(2-pyrrolidin-1-yl-ethoxy)-benzaldehyd

Hergestellt nach der allgemeinen Arbeitsvorschrift I aus 5-Iod-2-(2-pyrrolidin-1-yl-ethoxy)-benzaldehyd (1.50 g, 4.35 mmol) und 5-(4-Chlor-phenyl)-2-ethinyl-pyridin (214 mg, 5.00 mmol).
Ausbeute: 320 mg (17.1 % d. Theorie)
$C_{26}H_{23}ClN_2O_2$ (M= 430,938)
ber.: Molpeak $(M+H)^+$: 431/433 gef.: Molpeak $(M+H)^+$: 431/433
Retentionszeit HPLC: 7.31 min (Methode A)

Beispiel 3.56

5-[5-(4-Chlor-phenyl)-pyridin-2-ylethinyl]-2-(2-pyrrolidin-1-yl-ethoxy)-benzaldehyd-oxim

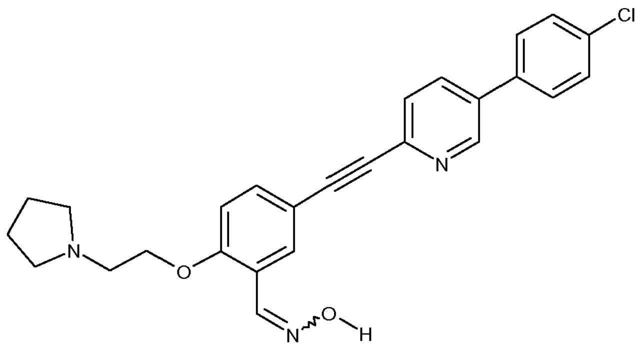

Zu einer Lösung von 200 mg (0.35 mmol) 5-[5-(4-Chlor-phenyl)-pyridin-2-ylethinyl]-2-(2-pyrrolidin-1-yl-ethoxy)-benzaldehyd (Beispiel 3.55b) in 2 mL einer 1:1 Mischung von Acetonitril und MeOH werden 27 mg (0.38 mmol) Hydroxylamin und 53 μL (0.38 mmol) Triethylamin gegeben. Die Reaktionslösung wird auf 85°C erwärmt. Nach vollständigem Umsatz wird mit Wasser und gesättigter $NaHCO_3$-Lösung verdünnt und die organische Phase mit DCM extrahiert. Die organische Phase wird über $MgSO_4$ getrocknet und das Lösungsmittel i.vac. entfernt. Die Reinigung erfolgt durch Säulenchromatographie via HPLC-MS.
Ausbeute: 5 mg (3.2 % d. Theorie)
$C_{26}H_{24}ClN_3O_2$ (M= 445,953)
ber.: Molpeak $(M+H)^+$: 446/448 gef.: Molpeak $(M+H)^+$: 446/448
Retentionszeit HPLC: 5.25 min (Methode A)

Beispiel 3.57

5-[5-(4-Chlor-phenyl)-pyridin-2-ylethinyl]-2-(2-pyrrolidin-1-yl-ethoxy)-benzaldehyd O-methyl-oxim

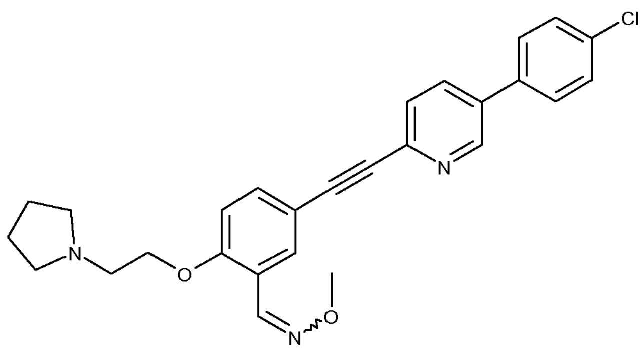

Analog Beispiel 3.56a wird aus 250 mg (0.44 mmol) 5-[5-(4-Chlor-phenyl)-pyridin-2-ylethinyl]-2-(2-pyrrolidin-1-yl-ethoxy)-benzaldehyd (Beispiel 3.55b) und 50 mg (0.60 mmol) 0-Methyl-hydroxylamin das Produkt erhalten.
Ausbeute: 40 mg (20.1 % d. Theorie)
$C_{27}H_{26}ClN_3O_2$ (M= 459,980)
ber.: Molpeak $(M+H)^+$: 460/462 gef.: Molpeak $(M+H)^+$: 460/462
Retentionszeit HPLC: 8.11 min (Methode A)

Beispiel 3.58

5-(4-Chlor-phenyl)-2-[3-ethinyl-4-(2-pyrrolidin-1-yl-ethoxy)-phenylethinyl]-pyridin

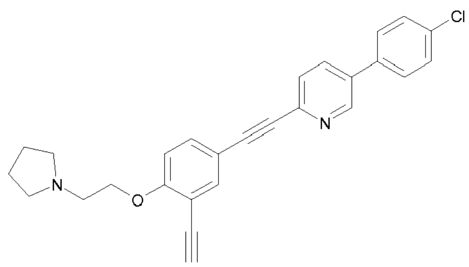

Zu einer Lösung von 300 mg (0.66 mmol) 5-[5-(4-Chlor-phenyl)-pyridin-2-ylethinyl]-2-(2-pyrrolidin-1-yl-ethoxy)-benzaldehyd (Beispiel 3.55b) und 183 mg (1.32 mmol) $K_2CO_3$ in 9 mL MeOH werden 152 mg (0.79 mmol) (1-Diazo-2-oxo-propyl)-phosphorsäuredimethylester in 2 mL MeOH gegeben. Die Reaktionslösung wird 3 h bei RT gerührt und mit 20 mL DCM verdünnt. Die organische Phase wird zweimal mit gesättigter $NaHCO_3$-Lösung extrahiert. Die organische Phase wird über $MgSO_4$ getrocknet und das Lösungsmittel i.vac. entfernt. Die Reinigung erfolgt via HPLC-MS.
Ausbeute: 104 mg (37.0 % d. Theorie)
$C_{27}H_{23}ClN_2O$ (M= 426,950)
ber.: Molpeak $(M+H)^+$: 427/429 gef.: Molpeak $(M+H)^+$: 427/429
Retentionszeit HPLC: 7.69 min (Methode A)

Beispiel 3.59

5-[5-(4-Chlor-phenyl)-pyridin-2-ylethinyl]-2,3-dihydro-indol-1-carbonsäure-2-pyrrolidin-1-yl-ethylester

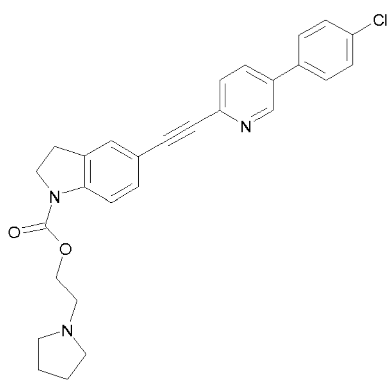

3.59a 5-Brom-2,3-dihydro-indol-1-carbonsäure-2-pyrrolidin-1-yl-ethylester

Zu einer Lösung von 868 mg (5.00 mmol) *N*-(2-Chlorethyl)-pyrrolidin Hydrochlorid und 1.70 g (12.2 mmol) $K_2CO_3$ in 15 mL DMF wird 1.00 g (4.95 mmol) 5-Bromindolin zugegeben. Die Reaktionslösung wird 4 h bei 70˚C gerührt und nochmals N-(2-Chlorethyl)-pyrrolidin Hydrochlorid zugegeben. Die Reaktionslösung wird weitere 3 h bei 70˚C gerührt und dann mit 25 mL Wasser verdünnt. Die wässrige Phase wird zweimal mit 30 mL EtOAc extrahiert. Die organische Phase wird mit gesättigter NaCl-Lösung gewaschen, über $Na_2SO_4$ getrocknet und das Lösungsmittel i.vac. entfernt. Die Reinigung erfolgt durch Säulenchromatographie an Kieselgel (Gradient: EtOAc/MeOH 9:1 nach EtOAc/MeOH 4:1 ).
Ausbeute: 687 mg (47.0 % d. Theorie)
$C_{15}H_{19}BrN_2O_2$ (M= 339,235)
ber.: Molpeak $(M+H)^+$: 339/341 gef.: Molpeak $(M+H)^+$: 339/341
$R_f$-Wert: 0.62 (Kieselgel, EtOAc/MeOH/$NH_3$ 9:1:0.1 )

3.59b 5-Iod-2,3-dihydro-indol-1-carbonsäure-2-pyrrolidin-1-yl-ethylester

Hergestellt nach der allgemeinen Arbeitsvorschrift II aus 5-Brom-2,3-dihydro-indol-1-carbonsäure-2-pyrrolidin-1-yl-ethylester (700 mg, 2.37 mmol).

Ausbeute: 590 mg (64.4 % d. Theorie)
$C_{15}H_{19}IN_2O_2$ (M= 386,235)
ber.: Molpeak $(M+H)^+$: 387 gef.: Molpeak $(M+H)^+$: 387
$R_f$-Wert: 0.37 (Kieselgel, EtOAc/MeOH/$NH_3$ 9:1:0.1)

3.59c 5-[5-(4-Chlor-phenyl)-pyridin-2-ylethinyl]-2,3-dihydro-indol-1-carbonsäure-2-pyrrolidin-1-yl-ethylester

Hergestellt nach der allgemeinen Arbeitsvorschrift I aus 5-lod-2,3-dihydro-indol-1-carbonsäure-2-pyrrolidin-1-yl-ethylester (120 mg, 0.31 mmol) und 5-(4-Chlor-phenyl)-2-ethinyl-pyridin (80 mg, 0.37 mmol).
Ausbeute: 48 mg (32.8 % d. Theorie)
$C_{28}H_{26}ClN_3O_2$ (M= 471,991)
ber.: Molpeak $(M+H)^+$: 472/474 gef.: Molpeak $(M+H)^+$: 472/474
Retentionszeit HPLC: 7.66 min (Methode A)

Beispiel 3.60

3-[5-(4-Chlor-phenyl)-pyridin-2-ylethinyl]-6,7,8,9-tetrahydro-5*H*-10-thia-7-azabenzo[a]azulen

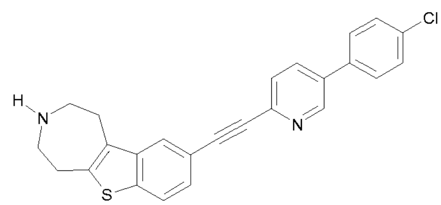

3.60a 4-Brom-5-oxo-azepan-1-carbonsäureethylester

Zu einer Lösung von 92.7 g (500 mmol) 4-Oxo-azepan-1-carbonsäureethylester in 350 mL Chloroform werden 79.9 g (500 mmol) Brom gegeben und die Reaktion über Nacht gerührt. Die Reaktionslösung wird dreimal mit gesättigter $NaHCO_3$-Lösung gewaschen, die organische Phase über $Na_2SO_4$ getrocknet und das Lösungsmittel i.vac. entfernt. Das Produkt wird ohne Reinigung weiter umgesetzt.
Ausbeute: 118 g (89.3 % d. Theorie)
$C_9H_{14}BrNO_3$ (M= 264,135)

3.60 b 4-(4-Brom-phenylsulfanyl)-5-oxo-azepan-1-carbonsäureethylester

Zu einer Lösung von 84.5 g (320 mmol) 4-Brom-5-oxo-azepan-1-carbonsäure-ethylester und 32.4 g (320 mmol) Triethylamin in 80 mL Chloroform werden über 45 min 60.5 g (320 mmol) 4-Bromthiophenol in 300 mL Chloroform gegeben, so dass die Innentemperatur 40˚C nicht überschreitet. Die Reaktionslösung wird 1.5 h bei RT gerührt. Das Reaktionsgemisch wird zweimal mit verdünnter Ammoniak-Lösung und zweimal mit Wasser gewaschen. Die organische Phase wird über $Na_2SO_4$ und $K_2CO_3$ getrocknet und das Lösungsmittel i.vac. entfernt. Die Reinigung erfolgt durch mehrere Säulenchromatographien an Kieselgel.
Ausbeute: 44.4 g (37.2 % d. Theorie)
$C_{15}H_{18}BrNO_3S$ (M= 372,30)
$R_f$-Wert: 0.33 (Kieselgel, Chloroform/Aceton 19:1 )

3.60c 3-Brom-5,6,8,9-tetrahydro-10-thia-7-aza-benzo[a]azulen-7-carbonsäure-ethylester

Eine Lösung von 44.3 g (119 mmol) 4-(4-Brom-phenylsulfanyl)-5-oxo-azepan-1-carbonsäureethylester in 443 g Polyphosphorsäure wird 45 min auf 80˚C erwärmt und dann mit 1000 mL Wasser verdünnt. Die wässrige Phase wird dreimal mit Chloroform extrahiert. Die organische Phase wird mit Wasser gewaschen, über $Na_2SO_4$ getrocknet und das Lösungsmittel i.vac. entfernt. Die Reinigung erfolgt durch mehrere Säulenchromatographien an Kieselgel (Chloroform/EtOAc 19:1) und durch Umkristallisation aus MeOH/Aceton.
Ausbeute: 22.4 g (52.8 % d. Theorie)

$C_{15}H_{16}BrNO_2S$ (M= 354,28)
Schmelzpunkt: 109˚C

3.60d 3-Brom-6,7,8,9-tetrahydro-5*H*-10-thia-7-aza-benzo[a]azulen

Zu einer Lösung von 19.0 g (53.5 mmol) 3-Brom-5,6,8,9-tetrahydro-10-thia-7-azabenzo[a]azulen-7-carbonsäureethylester werden 30.0 g (53.5 mmol) KOH in 700 mL EtOH gegeben. Bei Normaldruck wird EtOH durch Destillation entfernt und der Rückstand in Wasser aufgenommen. Mit HCl wird die Lösung sauer gestellt. Anschließend stellt man mit NaOH basisch und die wässrige Phase wird viermal mit Chloroform extrahiert. Die organische Phase wird über $Na_2SO_4$ und $K_2CO_3$ getrocknet und das Lösungsmittel i.vac. entfernt. Die Reinigung erfolgt durch mehrere Säulenchromatographien an Kieselgel.
Ausbeute: 12.6 g (83.0 % d. Theorie)
$C_{12}H_{12}BrNS$ (M= 282,22)
Schmelzpunkt: 89˚C

3.60e 3-Iod-6,7,8,9-tetrahydro-5H-10-thia-7-aza-benzo[a]azulen

Hergestellt nach der allgemeinen Arbeitsvorschrift II aus 3-Brom-6,7,8,9-tetrahydro-5*H*-10-thia-7-aza-benzo [a]azulen (1.80 g, 6.38 mmol).
Ausbeute: 1.80 g (85.7 % d. Theorie)
$C_{12}H_{12}INS$ (M= 329,205)
ber.: Molpeak $(M+H)^+$: 330 gef.: Molpeak $(M+H)^+$: 330
Retentionszeit HPLC: 5.45 min (Methode A)

3.60f 3-[5-(4-Chlor-phenyl)-pyridin-2-ylethinyl]-6,7,8,9-tetrahydro-5*H*-10-thia-7-azabenzo[a]azulen

Hergestellt nach der allgemeinen Arbeitsvorschrift 1 aus 3-Iod-6,7,8,9-tetrahydro-5*H*-10-thia-7-aza-benzo[a] azulen (770 mg, 2.34 mmol) und 5-(4-Chlor-phenyl)-2-ethinyl-pyridin (500 mg, 2.34 mmol).
Ausbeute: 350 mg (36.0 % d. Theorie)
$C_{25}H_19ClN_2S$ (M= 414,961 )
ber.: Molpeak $(M+H)^+$: 415/417 gef.: Molpeak $(M+H)^+$: 415/417
Retentionszeit HPLC: 7.41 min (Methode A)

Beispiel 3.61

3-[5-(4-Chlor-phenyl)-pyridin-2-ylethinyl]-7-methyl-6,7,8,9-tetrahydro-5*H*-10-thia-7-azabenzo[a]azulen

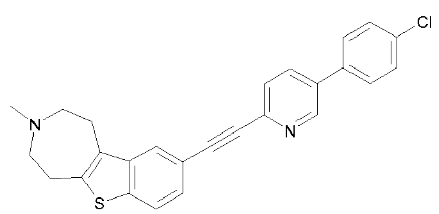

Zu einer Lösung von 100 mg (0.24 mmol) 3-[5-(4-Chlor-phenyl)-pyridin-2-ylethinyl]-6,7,8,9-tetrahydro-5H-10-thia-7-aza-benzo[a]azulen (siehe 3.60f) in 5 mL Acetonitril werden 0.18 mL (2.41 mmol) 37% Formalin-Lösung in Wasser gegeben. Dann werden 60 mg (0.96 mmol) $NaBH_3CN$ und 56 μL (0.96 mmol) Essigsäure zugegeben und das Reaktionsgemisch über Nacht gerührt. Die Lösung wird mit 2 M NaOH versetzt und mit EtOAc extrahiert. Die organische Phase wird über $MgSO_4$ getrocknet und das Lösungsmittel i.vac. entfernt. Die Reinigung erfolgt durch Säulenchromatographie via HPLC-MS.
Ausbeute: 3 mg (2.9 % d. Theorie)
$C_{26}H_{21}ClN_2S$ (M= 428,988)
ber.: Molpeak $(M+H)^+$: 429/431 gef.: Molpeak $(M+H)^+$: 429/431
Retentionszeit HPLC: 4.97 min (Methode B)

Beispiel 3.62

5-[5-(4-Chlor-phenyl)-pyridin-2-ylethinyl]-1-(2-pyrrolidin-1-yl-ethyl)-1 H-indazol

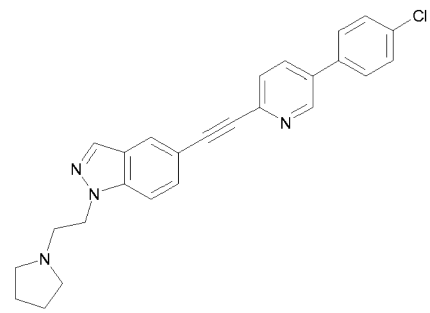

3.62a 5-Nitro-1-(2-pyrrolidin-1-yl-ethyl)-1H-indazol und 5-Nitro-2-(2-pyrrolidin-1-yl-ethyl)-2H-indazol

Zu einer Lösung von 5.00 g (31.0 mmol) 5-Nitroindazol in 100 mL Acetonitril werden nacheinander 10.5 g (62.0 mmol) 1-(2-Chlorethyl)-pyrrolidin Hydrochlorid und 12.9 g (93.0 mmol) $K_2CO_3$ zugegeben. Die Reaktionslösung wird 2 h bei RT gerührt und weitere 5 h unter Rückfluß erhitzt. Nach Abkühlung der Lösung wird von unlöslichen Salzen abfiltriert und das Lösungsmittel i.vac. entfernt. Der Rückstand wird in EtOAc und Wasser aufgenommen. Die organische Phase wird über $MgSO_4$ getrocknet und das Lösungsmittel i.vac. entfernt. Es liegt ein 4:1 Gemisch von 5-Nitro-1-(2-pyrrolidin-1-yl-ethyl)-1H-indazol und 5-Nitro-2-(2-pyrrolidin-1-yl-ethyl)-2H-indazol vor. Die Reinigung erfolgt durch Säulenchromatographie an Alox (PE/EtOAc 3:2).
5-Nitro-1-(2-pyrrolidin-1-yl-ethyl)-1*H*-indazol
Ausbeute: 4.00 g (49.6 % d. Theorie)
$C_{13}H_{16}N_4O_2$ (M= 260,298)
ber.: Molpeak $(M+H)^+$: 261 gef.: Molpeak $(M+H)^+$: 261
$R_f$-Wert: 0.78 (Alox, PE/EtOAc 1:1)
5-Nitro-2-(2-pyrrolid in-1-yl-ethyl )-2*H*-indazol
Ausbeute: 1.00 g (12.4 % d. Theorie)
$C_{13}H_{16}N_4O_2$ (M= 260,298)
ber.: Molpeak $(M+H)^+$: 261 gef.: Molpeak $(M+H)^+$: 261
$R_f$-Wert: 0.61 (Alox, PE/EtOAc 1:1)

3.62b 1-(2-Pyrrolidin-1-yl-ethyl)-1H-indazol-5-ylamin

Zu einer Lösung von 3.50 g (13.4 mmol) 5-Nitro-1-(2-pyrrolidin-1-yl-ethyl)-1H-indazol in 50 mL EtOAc werden 0.50 g Raney-Nickel gegeben und die Reaktionsmischung bei 1.4 bar $H_2$ 20 h bei RT gerührt. Nach Filtration wird das Lösungsmittel i.vac. entfernt. Das Produkt wird ohne weitere Reinigung weiter umgesetzt.
Ausbeute: 2.90 g (93.6 % d. Theorie)
$C_1gH_{18}N_4$ (M= 230,315)
ber.: Molpeak $(M+H)^+$: 231 gef.: Molpeak $(M+H)^+$: 231

3.62c 5-Brom-1-(2-pyrrolidin-1-yl-ethyl)-1*H*-indazol

1.00 g (4.34 mmol) 1-(2-Pyrrolidin-1-yl-ethyl)-1H-indazol-5-ylamin wird in 9.76 mL 48% Bromwasserstoffsäure und 9.76 mL Wasser gelöst und die Lösung wird auf 0˚C abgekühlt. 2.5 M Natriumnitrit-Lösung (300 mg in 1.74 mL Wasser) wird langsam zugetropft. Die Reaktion wird 10 min bei 0˚C gerührt und dann eine Lösung von 935 mg (6.51 mmol) CuBr in 3.42 mL 48% Bromwasserstoffsäure zugetropft. Die Reaktion wird auf 60˚C erwärmt und eine Stunde bei dieser Temperatur gerührt. Das Gemisch wird mit Wasser verdünnt und die wässrige Phase mit EtOAc extrahiert. Die organische Phase wird verworfen und die wässrige Phase mit gesättigter $NaHCO_3$-Lösung alkalisch gestellt. Die wässrige Phase wird mit EtOAc extrahiert und die organische Phase mit Wasser gewaschen. Die organische Phase wird über $MgSO_4$ getrocknet und das Lösungsmittel i.vac. entfernt.
Ausbeute: 500 mg (39.1 % d. Theorie)

$C_{13}H_{16}BrN_3$ (M= 294,197)
ber.: Molpeak $(M+H)^+$: 294/296 gef.: Molpeak $(M+H)^+$: 294/296
$R_f$-Wert: 0.59 (Kieselgel, EtOAc/MeOH/$NH_3$ 9:1:0.1)

3.62d 5-Iod-1-(2-pyrrolidin-1-yl-ethyl)-1H-indazol

Hergestellt nach der allgemeinen Arbeitsvorschrift II aus 5-Brom-1-(2-pyrrolidin-1-yl-ethyl)-1*H*-indazol (500 mg, 1.70 mmol).
Ausbeute: 230 mg (39.7 % d. Theorie)
$C_{13}H_{16}1N_3$ (M= 341,197)
ber.: Molpeak $(M+H)^+$: 342 gef.: Molpeak $(M+H)^+$: 342
$R_f$-Wert: 0.55 (Kieselgel, DCM/MeOH 4:1)

3.62e 5-[5-(4-Chlor-phenyl)-pyridin-2-ylethinyl]-1-(2-pyrrolidin-1-yl-ethyl)-1H-indazol Hydroiodid

Hergestellt nach der allgemeinen Arbeitsvorschrift I aus 5-Iod-1-(2-pyrrolidin-1-yl-ethyl)-1*H*-indazol (230 mg, 0.67 mmol) und 5-(4-Chlor-phenyl)-2-ethinyl-pyridin (144 mg, 0.67 mmol).
Ausbeute: 90 mg (24.1 % d. Theorie)
$C_{26}H_{23}C1N_4{}^*HI$ (M= 554,865)
ber.: Molpeak $(M+H)^+$: 427/429 gef.: Molpeak $(M+H)^+$: 427/429
Retentionszeit HPLC: 4.59 min (Methode B)

Beispiel 3.63

5-[5-(4-Chlor-phenyl)-pyridin-2-ylethinyl]-2-(2-pyrrolidin-1-yl-ethyl)-2H-indazol

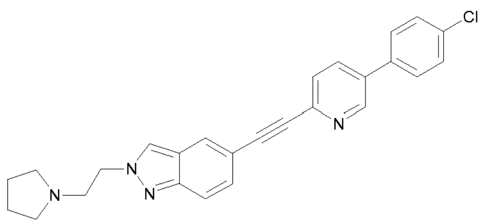

3.63a 2-(2-Pyrrolidin-1-yl-ethyl)-2H-indazol-5-ylamin

Analog Beispiel 3.62b wird aus 1.0 g (3.84 mmol) 5-Nitro-2-(2-pyrrolidin-1-yl-ethyl)-2H-indazol (siehe 3.62a) das Produkt erhalten.
Ausbeute: 840 mg (94.9 % d. Theorie)
$C1_3H_{18}N_4$ (M= 230,315)
ber.: Molpeak $(M+H)^+$: 231 gef.: Molpeak $(M+H)^+$: 231

3.63b 5-Brom-2-(2-pyrrolidin-1-yl-ethyl)-2H-indazol

Analog Beispiel 3.62c wird aus 840 mg (3.65 mmol) 2-(2-Pyrrolidin-1-yl-ethyl)-2H-indazol-5-ylamin das Produkt erhalten.
Ausbeute: 440 mg (41.0 % d. Theorie)
$C_{13}H_{16}BrN_3$ (M= 294,197)
ber.: Molpeak $(M+H)^+$: 294/296 gef.: Molpeak $(M+H)^+$: 294/296
Retentionszeit HPLC: 5.04 min (Methode A)

3.63c 5-Iod-2-(2-pyrrolidin-1-yl-ethyl)-2H-indazol

Hergestellt nach der allgemeinen Arbeitsvorschrift 11 aus 5-Brom-2-(2-pyrrolidin-1-yl-ethyl)-2H-indazol (440 mg, 1.50 mmol).

Ausbeute: 170 mg (33.3 % d. Theorie)
$C_{13}H_{16}1N_3$ (M= 341,197)
ber.: Molpeak $(M+H)^+$: 342 gef.: Molpeak $(M+H)^+$: 342
$R_f$-Wert: 0.40 (Kieselgel, DCM/MeOH 4:1)

3.63d 5-[5-(4-Chlor-phenyl)-pyridin-2-ylethinyl]-2-(2-pyrrolidin-1-yl-ethyl)-2H-indazol

Hergestellt nach der allgemeinen Arbeitsvorschrift 1 aus 5-Iod-2-(2-pyrrolidin-1-yl-ethyl)-2H-indazol (170 mg, 0.50 mmol) und 5-(4-Chlor-phenyl)-2-ethinyl-pyridin (106 mg, 0.50 mmol).
Ausbeute: 100 mg (42.3 % d. Theorie)
$C_{26}H_{23}C1N_4$ (M= 426,953)
ber.: Molpeak $(M+H)^+$: 427/429 gef.: Molpeak $(M+H)^+$: 427/429
Retentionszeit HPLC: 4.61 min (Methode B)

Beispiel 3.64

3-(4-Chlor-phenyl)-6-[4-(2-pyrrolidin-1-yl-ethoxy)-phenylethinyl]-pyridazin

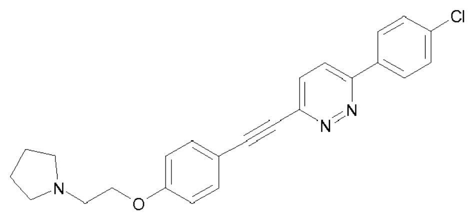

3.64a 3-Chlor-6-(4-chlor-phenyl)-pyridazin

Unter Argonatmosphäre werden zu einer Lösung aus 10.8 g (70.5 mmol) 3,6-Dichlorpyridazin, 10 mL (20 mmol) einer 2 M $Na_2CO_3$-Lösung und 600 mg (0.73 mmol) Pd(dppf)$C1_2$ in 150 mL 1,4-Dioxan bei 110˚C über 2 h eine Lösung von 11.3 g (70.5 mmol) 4-Chlorphenylboronsäure in 50 mL 1,4-Dioxan zugegeben. Die Reaktionsmischung wird 1 h bei 110˚C gerührt. 100 mL Wasser werden zugegeben und die wässrige Phase mit 100 mL EtOAc extrahiert. Die organische Phase wird über $Na_2SO_4$ getrocknet und das Lösungsmittel i.vac. entfernt. Eine Reinigung erfolgt durch Säulenchromatographie an Kieselgel (Cyc/EtOAc 4:1).
Ausbeute: 8.00 g (50.4 % d. Theorie)
$C_1$ pHgC$1_2N_2$ (M= 225,079)
ber.: Molpeak $(M+H)^+$: 225/227/229 gef.: Molpeak $(M+H)^+$: 225/227/229
Retentionszeit HPLC: 5.20 min (Methode A)

3.64b 3-(4-Chlor-phenyl)-6-trimethylsilanylethinyl-pyridazin

Unter Argon-Atmosphäre werden zu einer Lösung von 2.25 g (10.0 mmol) 3-Chlor-6-(4-chlor-phenyl)-pyridazin in 50 mL Acetonitril und 20 mL THF nacheinander 3.48 mL (25.0 mmol) Triethylamin und 2.08 mL (15.0 mmol) Ethinyltrimethyl-silan gegeben. Dann werden 292 mg (0.40 mmol) Pd(dppf)$Cl_2$ und 76 mg (0.40 mmol) CuI gegeben. Die Reaktionslösung wird über Nacht bei RT gerührt. Das Lösungsmittel wird i.vac. entfernt und die weitere Reinigung erfolgt durch Säulenchromatographie an Kieselgel (PE/EtOAc 1:1).
Ausbeute: 1.00 g (34.9 % d. Theorie)
$C_{15}H_{15}ClN_2Si$ (M= 286,839)
ber.: Molpeak $(M+H)^+$: 287/289 gef.: Molpeak $(M+H)^+$: 287/289
$R_f$-Wert: 0.45 (Kieselgel, DCM)

3.64c 3-(4-Chlor-phenyl)-6-ethinyl-pyridazin

Zu einer Lösung von 1.00 g (3.49 mmol) 3-(4-Chlor-phenyl)-6-trimethylsilanylethinylpyridazin in 10 mL DCM werden bei 0˚C 1.10 g (3.49 mmol) TBAF gegeben. Das Eisbad wird entfernt und die Reaktionslösung 30 min gerührt.

Es wird Wasser zugegeben und die wässrige Phase mit EtOAc extrahiert. Die organische Phase wird über $MgSO_4$ getrocknet und das Lösungsmittel i.vac. entfernt. Das Produkt wird ohne weitere Reinigung umgesetzt.
Ausbeute: 700 mg (93.5 % d. Theorie)
$C_{12}H_7ClN_2$ (M= 214.656)
ber.: Molpeak $(M+H)^+$: 215/217 gef.: Molpeak $(M+H)^+$: 215/217
Retentionszeit HPLC: 5.16 min (Methode B)

3.64d 3-(4-Chlor-phenyl)-6-[4-(2-pyrrolidin-1-yl-ethoxy)-phenylethinyl]-pyridazin

Hergestellt nach der allgemeinen Arbeitsvorschrift 1 aus 1-[2-(4-Iod-phenoxy)-ethyl]-pyrrolidin (200 mg, 0.63 mmol) und 3-(4-Chlor-phenyl)-6-ethinyl-pyridazin (135 mg, 0.63 mmol).
Ausbeute: 15 mg (5.9 % d. Theorie)
$C_{24}H_{22}ClN_3O$ (M= 403,915)
ber.: Molpeak $(M+H)^+$: 404/406 gef.: Molpeak $(M+H)^+$: 404/406
Retentionszeit HPLC: 5.01 min (Methode A)

Beispiel 3.65

5-(4-Chlor-phenyl)-3-fluor-2-(4-[2-(4-methyl-piperidin-1 -yl)-ethoxy]-phenylethinyl)-pyridin

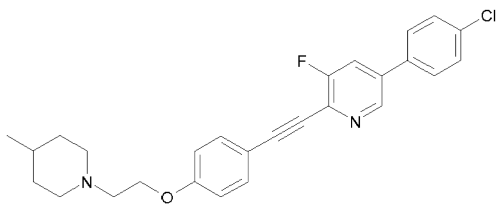

3.65a 1-[2-(4-Iod-phenoxy)-ethyl]-4-methyl-piperidin

Analog Beispiel 3.1 e wird aus 5.72 g (26.0 mmol) 4-Iod-phenol und 4.20 g (26.0 mmol) 1-(2-Chlor-ethyl)-4-methyl-piperidin das Produkt erhalten.
Ausbeute: 2.60 g (29.0 % d. Theorie)
$C_{14}H_{20}INO$ (M= 345,226)
ber.: Molpeak $(M+H)^+$: 346 gef.: Molpeak $(M+H)^+$: 346
Retentionszeit HPLC: 5.70 min (Methode A)

3.65b 5-(4-Chlor-phenyl)-3-nitro-pyridin-2-ol

Zu einer Lösung aus 22.1 g (101 mmol) 5-Brom-3-nitro-pyridin-2-ol, 200 mL (400 mmol) einer 2 M $Na_2CO_3$-Lösung und 731 mg (1.00 mmol) Pd(dppf)$C1_2$ in 400 mL Aceton und 80 mL Wasser werden unter Argon 23.5 g (150 mmol) 4-Chlorphenylboronsäure gegeben. Die Reaktionsmischung wird 18 h bei 60˚C gerührt. Aceton wird i.vac. entfernt und der Rückstand mit 160 mL 1 M Citronensäure auf pH 7 eingestellt. Die wässrige Phase wird dreimal mit EtOAc und einmal mit MeOH extrahiert. Die organische Phase wird über $MgSO_4$ getrocknet und das Lösungsmittel i.vac. entfernt. Der Rückstand wird mit EtOAc verrieben.
Ausbeute: 9.70 g (22.0 % d. Theorie)
$C_{11}H_7ClN_2O_3$ (M= 250,643)
ber.: Molpeak (M-H)-: 249/251 gef.: Molpeak (M-H)-: 249/251
Retentionszeit HPLC: 6.83 min (Methode A)

3.65c 2-Brom-5-(4-chlor-phenyl)-3-nitro-pyridin

Zu einer Lösung von 9.70 g (38.7 mmol) 5-(4-Chlor-phenyl)-3-nitro-pyridin-2-ol und 14.5 mmol (45.0 mmol) Tetrabutylammoniumbromid in 100 mL Toluol werden 13.2 g (93.0 mmol) Phosphorpentoxid gegeben. Das Reaktionsgemisch wird 1.5 h bei 95˚C gerührt. Nach Abkühlung wird die Toluolphase durch Dekantieren entfernt und der Rückstand

zweimal mit Tolouol versetzt und abdekantiert. Die vereinigten organischen Phasen werden mit gesättigter $NaHCO_3$-Lösung gewaschen. Die organische Phase wird über $MgSO_4$ getrocknet und das Lösungsmittel i.vac. entfernt. Das Produkt wird ohne weitere Reinigung weiter umgesetzt.
Ausbeute: 4.90 g (40.4 % d. Theorie)
$C_{11}H_6BrClN_2)O_2$ (M= 313,540)
ber.: Molpeak $(M+H)^+$: 313/315/317 gef.: Molpeak $(M+H)^+$: 313/315/317
Retentionszeit HPLC: 6.01 min (Methode B)

3.65d 2-Brom-5-(4-chlor-phenyl)-pyridin-3-ylamin

Eine Lösung von 5.60 g (17.9 mmol) 2-Brom-5-(4-chlor-phenyl)-3-nitro-pyridin, 20.3 g (90.0 mmol) Zinn(II)-chlorid und 18.9 g (225 mmol) $NaHCO_3$ in 300 mL EtOAc wird 30 h unter Rückfluß erhitzt. Nach Filtration wird das Lösungsmittel i.vac. entfernt. Der Rückstand wird mit DCM verrieben und nach Filtration wird der Filterrückstand an der Luft getrocknet.
Ausbeute: 3.50 g (69.1 % d. Theorie)
$C_{11}H_8BrClN_2$ (M= 283,557)
ber.: Molpeak $(M+H)^+$: 283/285/287 gef.: Molpeak $(M+H)^+$: 283/285/287
Retentionszeit HPLC: 5.45 min (Methode B)

3.65e 2-Brom-5-(4-chlor-phenyl)-3-fluor-pyridin

Zu einer Lösung von 1.00 g (3.53 mmol) 2-Brom-5-(4-chlor-phenyl)-pyridin-3-ylamin in 2 mL Wasser und 2.04 mL konzentrierter HCl werden bei -5˚C 243 mg (3.53 mmol) Natriumnitrit in 0.5 mL Wasser zugetropft. Dann werden bei 0˚C 1.56 mL (10.6 mmol) 60% Hexafluorphosphorsäure in Wasser zugegeben und die Reaktion eine weitere Stunde bei 0˚C gerührt. Das Diazoniumsalz wird abgesaugt, mit kaltem Wasser, Isopropanol und Ether gewaschen und im Exsikkator bei RT und 7 mbar über Nacht getrocknet. Dieses wird dann portionenweise bei 90˚C in 50 mL PE (Siedepunkt 100-140˚C) eingetragen. Nach Abkühlen der Reaktionslösung wird das Gemisch mit gesättigter $Na_2CO_3$-Lösung alkalisch gestellt. Die wässrige Phase wird mit EtOAc extrahiert und die organische Phase nacheinander mit gesättigter $Na_2CO_3$-Lösung und Wasser gewaschen. Die organische Phase wird über $MgSO_4$ getrocknet und das Lösungsmittel i.vac. entfernt.
Die Reinigung erfolgt durch Säulenchromatographie an Kieselgel (PE).
Ausbeute: 460 mg (45.5 % d. Theorie)
$C_{11}H_6BrClFN$ (M= 286,533)
ber.: Molpeak $(M+H)^+$: 286/288/290 gef.: Molpeak $(M+H)^+$: 286/288/290
Retentionszeit HPLC: 6.24 min (Methode B)

3.65f 5-(4-Chlor-phenyl)-3-fluor-2-trimethylsilanylethinyl-pyridin

Analog Beispiel 3.64b wird aus 460 mg (1.61 mmol) 2-Brom-5-(4-chlor-phenyl)-3-fluor-pyridin und 0.33 mL (2.41 mmol) Ethinyl-trimethyl-silan das Produkt erhalten.
Ausbeute: 490 mg (100 % d. Theorie)
$C_{16}H_{15}ClFNSi$ (M= 303,842)
ber.: Molpeak $(M+H)^+$: 304/306 gef.: Molpeak $(M+H)^+$: 304/306

3.65g 5-(4-Chlor-phenyl)-2-ethinyl-3-fluor-pyridin

Analog Beispiel 3.64c wird aus 490 mg (1.61 mmol) 5-(4-Chlor-phenyl)-3-fluor-2-trimethylsilanylethinyl-pyridin das Produkt erhalten.
Ausbeute: 300 mg (57.4 % d. Theorie)
$C_{13}H_7ClFN$ (M= 231,659)
ber.: Molpeak $(M+H)^+$: 232/234 gef.: Molpeak $(M+H)^+$: 232/234

3.65h 5-(4-Chlor-phenyl)-3-fluor-2-{4-[2-(4-methyl-piperidin-1-yl)-ethoxy]-phenylethinyl)-pyridin

Hergestellt nach der allgemeinen Arbeitsvorschrift I aus 1-[2-(4-Iod-phenoxy)-ethyl]-4-methyl-piperidin (164 mg, 0.48 mmol) und 5-(4-Chlor-phenyl)-2-ethinyl-3-fluor-pyridin (110 mg, 0.48 mmol).
Ausbeute: 14 mg (6.6 % d. Theorie)
$C_{27}H_{26}ClFN_2O$ (M= 448.972)

ber.: Molpeak $(M+H)^+$: 449/451 gef.: Molpeak $(M+H)^+$: 449/451
Retentionszeit HPLC: 5.16 min (Methode B)

Beispiel 3.66

6-[5-(4-Chlor-phenyl)-pyridin-2-ylethinyl]-1-methansulfonyl-2-pyrrolidin-1-ylmethyl-1,2,3,4-tetrahydro-chinolin

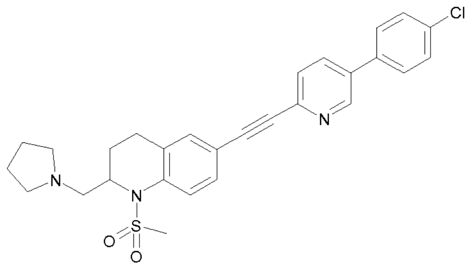

Zu einer Lösung von 200 mg (0.47 mmol) 6-[5-(4-Chlor-phenyl)-pyridin-2-ylethinyl]-2-pyrrolidin-1-ylmethyl-1,2,3,4-tetrahydro-chinolin (siehe Beispiel 3.46) in 5 mL DCM werden bei 0˚C nacheinander 0.13 mL (0.93 mmol) Triethylamin und 36 μL (0.47 mmol) Methansulfonsäurechlorid gegeben. Das Reaktiongemisch wird auf RT erwärmt und eine weitere Stunde bei dieser Temperatur gerührt. Es werden nochmals 36 μL (0.47 mmol) Methansulfonsäurechlorid zugegeben und eine weitere Stunde bei RT gerührt.

Das Reaktionsgemisch wird auf Wasser gegossen und erschöpfend mit DCM extrahiert. Die organische Phase wird über $MgSO_4$ getrocknet und das Lösungsmittel i.vac. entfernt. Die Reinigung erfolgt durch Säulenchromatographie mittels HPLC-MS.
Ausbeute: 9 mg (3.8 % d. Theorie)
$C_{28}H_{28}ClN_3O_2S$ (M= 506,071)
ber.: Molpeak $(M+H)^+$: 506/508 gef.: Molpeak $(M+H)^+$: 506/508
Retentionszeit HPLC: 5.26 min (Säule aus Methode A; Isokratisch: 30% Acetonitril)

Beispiel 3.67

1 -{6-[5-(4-Chlor-phenyl)-pyridin-2-ylethinyl]-2-pyrrolidin-1 -ylmethyl-3,4-dihydro-2H-chinolin-1-yl}-ethanon

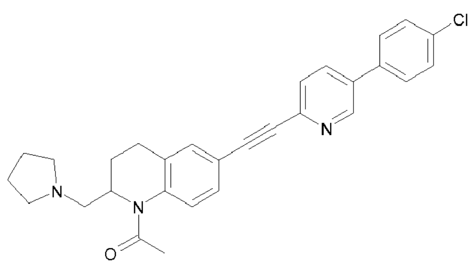

Zu einer Lösung von 220 mg (0.51 mmol) 6-[5-(4-Chlor-phenyl)-pyridin-2-ylethinyl]-2-pyrrolidin-1-ylmethyl-1,2,3,4-tetrahydro-chinolin (siehe Beispiel 3.46) in 5 mL DCM werden 74 μL (0.77 mmol) Acetanhydrid gegeben und die Mischung 2 h bei RT gerührt. Weitere 0.37 mL (3.85 mmol) Acetanhydrid werden zugegeben und die Reaktion weitere 4 Tage bei RT gerührt. Das Lösungsmittel wird i.vac. entfernt. Die Reinigung erfolgt durch Säulenchromatographie mittels HPLC-MS.
Ausbeute: 105 mg (43.5 % d. Theorie)
$C_{29}H_{28}ClN_3O$ (M= 470,019)
ber.: Molpeak $(M+H)^+$: 470/472 gef.: Molpeak $(M+H)^+$: 470/472
Retentionszeit HPLC: 7.08 min (Methode A)

Beispiel 3.68

5-(4-Chlor-phenyl)-2-[3-pyridin-2-yl-4-(2-pyrrolidin-1-yl-ethoxy)-phenyleth inyl]-pyridin

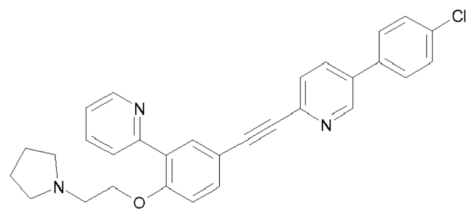

Zu einer Lösung aus 115 mg (0.24 mmol) 2-[3-Brom-4-(2-pyrrolidin-1-yl-ethoxy)-phenylethinyl]-5-(4-chlor-phenyl)-pyridin (siehe Beispiel 3.7), 0.5 mL (1.00 mmol) einer 2 M $Na_2CO_3$-Lösung und 15 mg (0.24 mmol) Tetrakistriphenylphosphan-palladium in 1 mL 1,4-Dioxan und 0.3 mL Methanol werden 30 mg (0.24 mmol) Pyridin-3-boronsäure gegeben. Die Reaktionsmischung wird 6 h unter Rückfluß erhitzt. Nach Filtration wird das Lösungsmittel i.vac. entfernt. Die Reinigung erfolgt durch Säulenchromatographie an Kieselgel (Gradient: DCM nach DCM/MeOH/$NH_3$ 1:1:0.1).
Ausbeute: 1.8 mg (1.6 % d. Theorie)
$C_{30}H_{26}ClN_3O$ (M= 480,01)
ber.: Molpeak (M-H)-: 480/482 gef.: Molpeak (M-H)-: 480/482
Retentionszeit HPLC: 6.50 min (Methode A)

Beispiel 3.69

5-(4-Chlor-phenyl)-2-{4-[2-(4-methyl-piperidin-1-yl)-ethoxy]-naphthyl-1-ylethinyll-pyridin

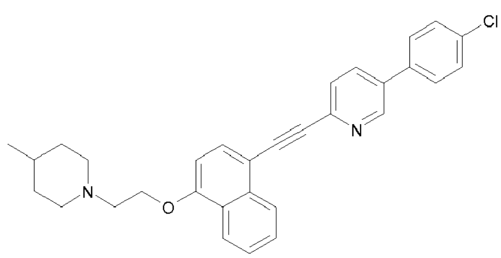

3.69a 1-[2-(4-Brom-naphthyl-1-yloxy)-ethyl]-4-methyl-piperidin

Analog Beispiel 3.1 e wird aus 1.0 g (5.35 mmol) 4-Brom-naphthyl-1-ol und 323 mg (2.00 mmol) 1-(2-Chlorethyl)-4-methyl-piperidin das Produkt erhalten.
Ausbeute: 530 mg (97.0 % d. Theorie)
$C_{18}H_{22}BrNO$ (M= 348,286)
ber.: Molpeak $(M+H)^+$: 348/350 gef.: Molpeak $(M+H)^+$: 348/350
Retentionszeit HPLC: 7.10 min (Methode A)

3.69b 1-[2-(4-lod-naphthyl-1-yloxy)-ethyl]-4-methyl-piperidin

Hergestellt nach der allgemeinen Arbeitsvorschrift ll aus 1-[2-(4-Brom-naphthyl-1-yloxy)-ethyl]-4-methyl-piperidin (530 mg, 1.52 mmol).
Ausbeute: 500 mg (83.1 % d. Theorie)
$C_{18}H_{22}IN0$ (M= 395,287)
ber.: Molpeak $(M+H)^+$: 396 gef.: Molpeak $(M+H)^+$: 396

Retentionszeit HPLC: 6.74 min (Methode A)

3.69c 5-(4-Chlor-phenyl)-2-(4-[2-(4-methyl-piperidin-1-yl)-ethoxy]-naphthyl-1-ylethinyl)-pyridin

Hergestellt nach der allgemeinen Arbeitsvorschrift 1 aus 1-[2-(4-Iod-naphthyl-1-yloxy)-ethyl]-4-methyl-piperidin (277 mg, 0.70 mmol) und 5-(4-Chlor-phenyl)-2-ethinyl-pyridin (150 mg, 0.70 mmol).
Ausbeute: 66 mg (19.6 % d. Theorie)
$C_{31}H_{29}ClN_2O$ (M= 481,043)
ber.: Molpeak $(M+H)^+$: 481/483 gef.: Molpeak $(M+H)^+$: 481/483
$R_f$-Wert: 0.60 (Kieselgel, EtOAc/MeOH/$NH_3$ 95:5:0.5)

Beispiel 3.70

2-{4-[2-(4-Methyl-piperidin-1-yl)-ethoxy]-phenylethinyl}-5-phenyl-pyridin

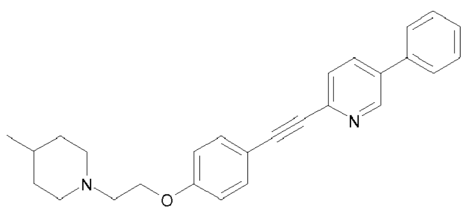

3.70a 5-Brom-2-{4-[2-(4-methyl-piperidin-1-yl)-ethoxy]-phenylethinyl)-pyridin

Hergestellt nach der allgemeinen Arbeitsvorschrift 1 aus 1-[2-(4-Iod-phenoxy)-ethyl]-4-methyl-piperidin (345 mg, 1.00 mmol) und 5-Brom-2-ethinyl-pyridin (83 mg, 0.39 mmol).
Ausbeute: 100 mg (25.0 % d. Theorie)
$C_{21}H_{23}BrN_2O$ (M= 399,334)
ber.: Molpeak $(M+H)^+$: 399/401 gef.: Molpeak $(M+H)^+$: 399/401
$R_f$-Wert: 0.83 (Kieselgel, DCM/MeOH/$NH_3$ 95:5:0.5)

3.70.b 2-{4-[2-(4-Methyl-piperidin-1 -yl)-ethoxy]-phenylethinyl}-5-phenyl-pyridin

Unter Argonatmosphäre werden zu einer Lösung von 100 mg (0.25 mmol) 5-Brom-2-{4-[2-(4-methyl-piperidin-1-yl)-ethoxy]-phenylethinyl)-pyridin, 0.25 mL (0.50 mmol) einer 2 M $Na_2CO_3$-Lösung und 4 mg (0.01 mmol) Pd(dppf) $C1_2$ in 5 mL 1,4-Dioxan und 2 mL MeOH 30 mg (0.25 mmol) Phenylboronsäure gegeben. Die Reaktionsmischung wird 3 Tage bei 90˚C gerührt. Die Reaktionsmischung wird mit EtOAc verdünnt und die organische Phase mit 40 mL Wasser und schließlich mit gesättigter NaCl-Lösung gewaschen. Die organische Phase wird über $MgSO_4$ getrocknet und das Lösungsmittel i.vac. entfernt. Die Reinigung erfolgt mittels HPLC-MS und durch Säulenchromatographie an Kieselgel (Gradient: DCM/MeOH/$NH_3$ 95:5:0.5 nach DCM/MeOH/$NH_3$ 9:1:0.1).
Ausbeute: 27 mg (27.2 % d. Theorie)
$C_{27}H_{28}N_2O$ (M= 396,537)
ber.: Molpeak $(M+H)^+$: 397 gef.: Molpeak $(M+H)^+$: 397
Retentionszeit HPLC: 7.61 min (Methode A)

Beispiel 3.71

5-(4-Chlor-phenyl)-2-{4-[2-(4-methyl-piperid in-1-yl)-propoxy]-phenylethinyl}-pyridin

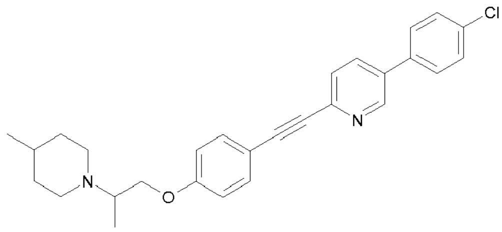

3.71a 1-(4-Iod-phenoxy)-propan-2-ol

Analog Beispiel 3.1e werden aus 1.39 g (10.0 mmol) 1-Brom-2-propanol und 2.20 g (10.0 mmol) 4-Iodphenol das Produkt erhalten.
Ausbeute: 2.00 g (71.9 % d. Theorie)
$C_9H_{11}10_2$ (M= 278,091)
ber.: Molpeak $(M+Na)^+$: 301 gef.: Molpeak $(M+Na)^+$: 301
$R_f$-Wert: 0.20 (Kieselgel, PE/EtOAc 4:1)

3.71 b 1-{4-[5-(4-Chlor-phenyl)-pyridin-2-ylethinyl]-phenoxy}-propan-2-ol

Hergestellt nach der allgemeinen Arbeitsvorschrift I aus 1-(4-Iod-phenoxy)-propan-2-ol (2.00 g, 7.19 mmol) und 5-(4-Chlor-phenyl)-2-ethinyl-pyridin (1.54 g, 7.20 mmol).
Ausbeute: 1.50 g (57.3 % d. Theorie)
$C_{22}H_{18}ClNO_2$ (M= 363,847)
ber.: Molpeak $(M+H)^+$: 364/366 gef.: Molpeak $(M+H)^+$: 364/366
$R_f$-Wert: 0.25 (Kieselgel, PE/EtOAc/DCM 1:1:8)

3.71c Methansulfonsäure-2-{4-[5-(4-chlor-phenyl)-pyridin-2-ylethinyl]-phenoxy}-1-methyl-ethylester

Zu einer Lösung von 1.50 g (4.12 mmol) 1-{4-[5-(4-Chlor-phenyl)-pyridin-2-ylethinyl]-phenoxy}-propan-2-ol und 1.14 mL (8.20 mmol) Triethylamin in 80 mL THF werden bei RT 0.35 mL (4.50 mmol) Methansulfonsäurechlorid gegeben und die Reaktion 3 h bei dieser Temperatur gerührt. Das Lösungsmittel wird i.vac. entfernt und der Rückstand mit 40 mL tert-Butylmethylether und 60 mL Wasser versetzt. Der Niederschlag wird abgesaugt und die weitere Reinigung erfolgt durch Säulenchromatographie an Kieselgel (EtOAc).
Ausbeute: 1.00 g (54.9 % d. Theorie)
$C_{23}H_{20}ClNO_4S$ (M= 441,937)
ber.: Molpeak $(M+H)^+$: 442/444 gef.: Molpeak $(M+H)^+$: 442/444
$R_f$-Wert: 0.78 (Kieselgel, PE/EtOAc/DCM 1:1:8)

3.71d 5-(4-Chlor-phenyl)-2-(4-[2-(4-methyl-piperidin-1-yl)-propoxy]-phenylethinyl}-pyridin

Zu einer Lösung von 133 mg (0.30 mmol) Methansulfonsäure-2-{4-[5-(4-chlor-phenyl)-pyridin-2-ylethinyl]-phenoxy}-1-methyl-ethylester in 2 mL DMF werden 0.21 mL (1.80 mmol) 4-Methylpiperidin gegeben und das Gemisch 16 h bei 60˚C und 6 h bei 80˚C gerührt. Das Lösungsmittel wird i.vac. entfernt, der Rückstand mit Isopropanol verrieben, abgesaugt und im Umlufttrockenschrank bei 30˚C getrocknet.
Ausbeute: 65 mg (48.7 % d. Theorie)
$C_{28}H_{29}C1N_20$ (M= 445,009)
ber.: Molpeak $(M+H)^+$: 445/447 gef.: Molpeak $(M+H)^+$: 445/447
Retentionszeit HPLC: 5.37 min (Methode B)

Beispiel 3.72

(1-{5-[5-(4-Chlor-phenyl)-pyridin-2-ylethinyl]-pyridin-2-yl}-pyrrolidin-3-yl)-4-methylpiperidin

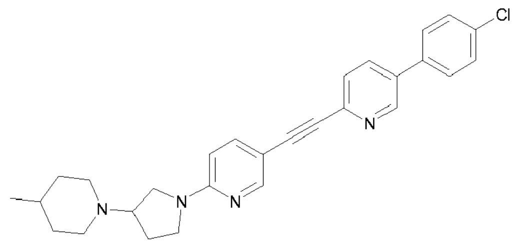

3.72a (R)-1-(5-Brom-pyridin-2-yl)-pyrrolidin-3-ol

Analog Beispiel 3.31 a (Reaktionszeit: 60 min bei 140˚C) wird aus 2.72 g (11.5 mmol) 2,5-Dibrompyridin und 1.00 g (11.5 mmol) (R)-3-Pyrrolidinol das Produkt erhalten.
Ausbeute: 1.20 g (43.0 % d. Theorie)
$CgH_{11}BrN_2O$ (M= 243,105)
ber.: Molpeak $(M+H)^+$: 242/244 gef.: Molpeak $(M+H)^+$: 242/244
Retentionszeit HPLC: 3.43 min (Methode A)

3.72b (R)-1-(5-lod-pyridin-2-yl)-pyrrolidin-3-ol

Hergestellt nach der allgemeinen Arbeitsvorschrift II aus (R)-1-(5-Brom-pyridin-2-yl)-pyrrolidin-3-ol (1.20 g, 4.94 mmol).
Ausbeute: 1.30 g (90.8 % d. Theorie)
$C_9H_{11}IN_2O$ (M= 290,105)
ber.: Molpeak $(M+H)^+$: 291 gef.: Molpeak $(M+H)^+$: 291
Retentionszeit HPLC: 3.48 min (Methode A)

3.72c (R)-1-{5-[5-(4-Chlor-phenyl)-pyridin-2-ylethinyl]-pyridin-2-yll-pyrrolidin-3-ol

Hergestellt nach der allgemeinen Arbeitsvorschrift I aus (R)-1-(5-lod-pyridin-2-yl)-pyrrolidin-3-ol (1.30 g, 4.48 mmol) und 5-(4-Chlor-phenyl)-2-ethinyl-pyridin (957 mg, 4.48 mmol).
Ausbeute: 1.36 g (80.7 % d. Theorie)
$C_{22}H_{18}ClN_3O$ (M= 375,861)
ber.: Molpeak $(M+H)^+$: 376/378 gef.: Molpeak $(M+H)^+$: 376/378
Retentionszeit HPLC: 6.76 min (Methode A)

3.72d 1-{5-[5-(4-Chlor-phenyl)-pyridin-2-ylethinyl]-pyridin-2-yl)-pyrrolidin-3-on

Zu einer Lösung von 200 mg (0.53 mmol) (R)-1-{5-[5-(4-Chlor-phenyl)-pyridin-2-ylethinyl]-pyridin-2-yl}-pyrrolidin-3-ol in 10 mL DCM werden 0.43 mL (5.32 mmol) Pyridin und 2.26 g (0.80 mmol, 15 Gewichtsprozent) Dess-Martin-Periodinan in DCM gegeben. Das Reaktionsgemisch wird 3 h bei RT gerührt und zu einer Lösung von halbgesättigter $NaHCO_3$-Lösung und *tert*-Butylmethylether gegeben. Die wässrige Phase wird zweimal mit EtOAc extrahiert. Die organische Phase wird über $MgS0_4$ getrocknet und das Lösungsmittel i.vac. entfernt. Das Produkt wird ohne Reinigung weiter umgesetzt.
Ausbeute: 100 mg (35.2 % d. Theorie)
$C_{22}H_{16}ClN_3O$ (M= 373,845)
ber.: Molpeak $(M+H)^+$: 374/376 gef.: Molpeak $(M+H)^+$: 374/376

3.72e (1-{5-[5-(4-Chlor-phenyl)-pyridin-2-ylethinyl]-pyridin-2-yl)-pyrrolidin-3-yl)-4-methylpiperidin

Zu einer Lösung von 100 mg (0.19 mmol, 70% Reinheit) 1-{5-[5-(4-Chlor-phenyl)-pyridin-2-ylethinyl]-pyridin-2-yl}-pyrrolidin-3-on und 22 μL 4-Methylpiperidin (0.19 mmol) in 5 mL THF werden 48 mg (0.22 mmol) $NaBH(OAc)_3$ und 27 μL (0.47 mmol) Essigsäure gegeben. Die Reaktionsmischung wird über Nacht gerührt und mit gesättigter $NaHCO_3$-Lösung versetzt. Die organische Phase wird zweimal mit EtOAc extrahiert. Die organische Phase wird über $MgSO_4$ getrocknet und das Lösungsmittel i.vac. entfernt. Die weitere Reinigung erfolgt durch Säulenchromatographie

mittels HPLC-MS.
Ausbeute: 11 mg (12.9 % d. Theorie)
$C_{28}H_{29}ClN_4$ (M= 457,023)
ber.: Molpeak $(M+H)^+$: 457/459 gef.: Molpeak $(M+H)^+$: 457/459
Retentionszeit HPLC: 5.19 min (Methode A)

Beispiel 3.73

5-(4-Chlor-phenyl)-2-[4-(3-pyrrolid in-1-yl-prop-1-inyl)-phenylethinyl]-pyridin

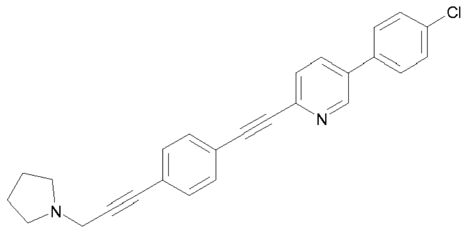

3.73a 1-[3-(4-Brom-phenyl)-prop-2-inyl]-pyrrolidin

Hergestellt nach der allgemeinen Arbeitsvorschrift I aus 4-Brom-iodbenzol (10.9 g, 38.5 mmol) und 1-Prop-2-inyl-pyrrolidin (4.20 g, 71 % Reinheit, 27.3 mmol).
Ausbeute: 6.40 g (88.7 % d. Theorie)
$C_{13}H_{14}BrN$ (M= 264,167)
ber.: Molpeak $(M+H)^+$: 264/266 gef.: Molpeak $(M+H)^+$: 264/266

3.73b 1-[3-(4-Iod-phenyl)-prop-2-inyl]-pyrrolidin

Hergestellt nach der allgemeinen Arbeitsvorschrift II aus 1-[3-(4-Brom-phenyl)-prop-2-inyl]-pyrrolidin (3.2 g, 12.1 mmol).
Ausbeute: 230 mg (4.6 % d. Theorie)
$C_{13}H_{14}IN$ (M= 311,168)
ber.: Molpeak $(M+H)^+$: 312 gef.: Molpeak $(M+H)^+$: 312

3.73c 5-(4-Chlor-phenyl)-2-[4-(3-pyrrolidin-1-yl-prop-1-inyl)-phenylethinyl]-pyridin

Hergestellt nach der allgemeinen Arbeitsvorschrift 1 aus 1-[3-(4-Iod-phenyl)-prop-2-inyl]-pyrrolidin (230 mg, 75%, 0.55 mmol) und 5-(4-Chlor-phenyl)-2-ethinyl-pyridin (118 mg, 0.55 mmol).
Ausbeute: 96 mg (43.7 % d. Theorie)
$C_{26}H_{21}ClN_2$ (M= 396,924)
ber.: Molpeak $(M+H)^+$: 397/399 gef.: Molpeak $(M+H)^+$: 397/399
Retentionszeit HPLC: 5.03 min (Methode B)

Beispiel 3.74

6-[5-(4-Chlor-phenyl)-pyrid in-2-ylethinyl]-1-methyl-2-pyrrolid in-1-ylmethyl-1,2,3,4-tetrahydro-chinolin

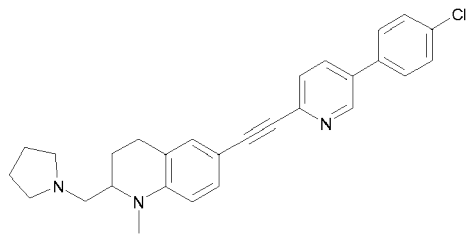

Zu einer Lösung von 350 mg (0.82 mmol) 6-[5-(4-Chlor-phenyl)-pyridin-2-ylethinyl]-2-pyrrolidin-1-ylmethyl-1,2,3,4-tetrahydro-chinolin (siehe Beispiel 3.46) in 1.6 mL THF werden 37 mg (1.23 mmol) Paraformaldehyd in 1.8 mL THF gegeben. Zu diesem Gemisch werden 0.24 mL Essigsäure und 1.2 mL THF gegeben. Schließlich werden 1.00 g (2.05 mmol) Cyanoborhydrid-Resin (Makroporöses Polystyren, Beladung: 2.04 mmol/g) zugegeben und das Gemisch 16 h bei RT gerührt. Nach Filtration wird das Filtrat mit 1.50 g (2.15 mmol) Toluolsulfonsäure-Resin (Makroporöses Polystyren, Beladung: 1.43 mmol/g) versetzt, 30 min geschüttelt und abgesaugt. Das Lösungsmittel wird i.vac. entfernt und die Reinigung erfolgt durch Säulenchromatographie mittels HPLC-MS.
Ausbeute: 33 mg (9.1 % d. Theorie)
$C_{28}H_{28}ClN_3$ (M= 442,008)
ber.: Molpeak $(M+H)^+$: 442/444 gef.: Molpeak $(M+H)^+$: 442/444
Retentionszeit HPLC: 5.22 min (Methode B)

**Beispiel 4**

5-(4-Chior-phenyl)-2-{4-[2-(2,5-dihydro-pyrrol-1-yl)-ethoxy]-phenylthinyl}-pyridin

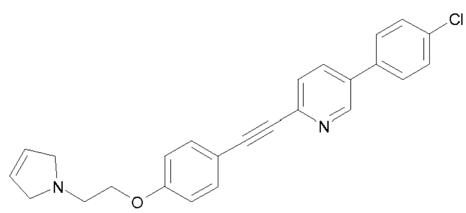

4a 2-(4-lod-phenoxy)-ethanol

Eine Suspension von 11 g (50 mmol) 4-lodphenol, 3.88 mL (55 mmol) 2-Bromethanol und 8.3 g (60 mmol) $K_2C0_3$ in 60 mL Aceton wird 24 h unter Rückfluss erhitzt. Das Lösungsmittel wird i.vac. entfernt, der Rückstand mit Wasser versetzt, erschöpfend mit EtOAc extrahiert und die organische Phase über $Na_2S0_4$ getrocknet. Nach Entfernen des Trocken- und Lösungsmittels wird der Rückstand chromatographisch an Kieselgel (Cyc/EtOAc 7:3) gereinigt.
Ausbeute: 2.9 g (22.0 % d. Theorie)
$C_8H_910_2$ (M= 264,064)
ber.: Molpeak $(M+H)^+$: 264 gef.: Molpeak $(M+H)^+$: 264
$R_f$-Wert: 0.24 (Kieselgel, Cyc/EtOAc 2:1 )

4b 2-(4-[5-(4-Chlor-phenyl)-pyridin-2-ylethinyl]-phenoxy}-ethanol

Unter Argonatmosphäre werden zu einer Lösung von 2.9 g (11 mmol) 2-(4-lodphenoxy)-ethanol und 2.35 g (11 mmol) 5-(4-Chlor-phenyl)-2-ethinyl-pyridin in 50 mL Piperidin 253 mg (0.22 mmol) Tetrakis-triphenylphosphan-Palladium und 42 mg (0.22 mmol) Cul gegeben und das Reaktionsgemisch 30 min bei RT gerührt. Das Lösungsmittel wird i.vac. entfernt, der Rückstand mit Wasser versetzt und mit EtOAc verrührt. Das ausgefallene Produkt wird abgesaugt und getrocknet.
Ausbeute: 2.1 g (54.7 % d. Theorie)
$C_{21}H_{16}ClN0_2$ (M= 349,820)
ber.: Molpeak $(M+H)^+$: 350 gef.: Molpeak $(M+H)^+$: 350

$R_f$-Wert: 0.42 (Kieselgel, Cyc/EtOAc 1:1 )

4c 5-(4-Chlor-phenyl)-2-{4-[2-(2,5-dihydro-pyrrol-1 -yl)-ethoxy]-phenylethiny}-pyridin

Zu einer auf 0˚C gekühlten Lösung von 85 mg (0.24 mmol) 2-{4-[5-(4-Chlor-phenyl)-pyridin-2-ylethinyl]-phenoxy}-ethanol und 41 μL (0.29 mmol) Triethylamin in 10 mL DCM werden 23 μL (0.29 mmol) Methansulfonsäurechlorid zugetropft und das Reaktionsgemisch 1 h bei dieser Temperatur nachgerührt. Man tropft 46 μL (0.58 mmol) 2,5-Dihydro-1*H*-pyrrol zu, erwärmt auf RT und rührt über Nacht. Man gibt 1 mL DMF zu und erwärmt für 8 h auf 70˚C. Man engt i.vac. ein, versetzt den Rückstand mit Wasser, extrahiert erschöpfend mit EtOAc und trocknet die organische Phase über $Na_2S0_4$. Nach Entfernen des Trocken- und Lösungsmittels wird der Rückstand chromatographisch an Kieselgel ($EtOAc/MeOH/NH_3$ 95:5:0.5) gereinigt.
Ausbeute: 16 mg (16.4 % d. Theorie)
$C_{25}H_21ClN_2O$ (M= 400,912)
ber.: Molpeak $(M+H)^+$: 401/403 gef.: Molpeak $(M+H)^+$: 401/403
$R_f$-Wert: 0.16 (Kieselgel, DCM/MeOH 95:5)

Beispiel 4.1

5-(4-Chlor-phenyl )-2-[4-(2-piperid in-1-yl-ethoxy)-phenylethinyl]-pyridin

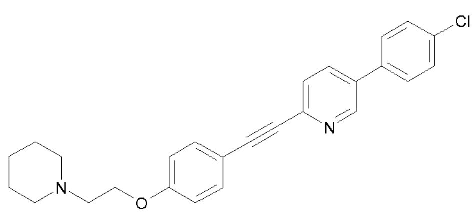

4.1 a Methansulfonsäure-2-{4-[5-(4-chlor-phenyl)-pyridin-2-ylethinyl]-phenoxy}-ethylester

Zu einer auf 0˚C gekühlten Lösung von 2.2 g (6.29 mmol) 2-{4-[5-(4-Chlor-phenyl)-pyridin-2-ylethinyl]-phenoxy}-ethanol und 1.74 mL (12.58 mmol) Triethylamin in 25 mL THF werden 0.59 mL (7.55 mmol) Methansulfonsäurechlorid zugetropft. Das Reaktionsgemisch wird auf RT erwärmt und 2 h gerührt. Zur Vervollständigung der Reaktion werden 5 mL Pyridin zugegeben und weitere 18 h bei RT gehalten. Das Lösungsmittel wird i.vac. entfernt, der Rückstand mit Wasser versetzt und mit Diethylether verrieben. Das ausgefallene Produkt wird abgesaugt und getrocknet.
Ausbeute: 2.4 g (89.2 % d. Theorie)
$C_{22}H_{18}ClN0_4S$ (M= 427,910)
ber.: Molpeak $(M+H)^+$: 428/430 gef.: Molpeak $(M+H)^+$: 428/430
$R_f$-Wert: 0.42 (Kieselgel, Cyc/EtOAc 1:1 )

4.1 b 5-(4-Chlor-phenyl)-2-[4-(2-piperidin-1-yl-ethoxy)-phenylethinyl]-pyridin

Zu einer Lösung von 85.6 mg (0.2 mmol) Methansulfonsäure-2-{4-[5-(4-chlor-phenyl)-pyridin-2-ylethinyl]-phenoxy}-ethyl-ester in 2 mL DMF werden 99 μL (1.0 mmol) Piperidin gegeben und das Reaktionsgemisch 18 h bei RT gerührt. Das Lösungsmittel wird i.vac. abdestilliert, der Rückstand mit 5 mL Wasser und 40 mL DCM verrührt, die organische Phase wird abgetrennt und mit $Na_2SO_4$ getrocknet. Nach Entfernen des Trocken- und Lösungsmittels wird der Rückstand mit 20 mL Diethylether verrieben und abgesaugt.
Ausbeute: 62 mg (74.3 % d. Theorie)
$C_{26}H_{25}ClN_20$ (M= 416,955)
ber.: Molpeak $(M+H)^+$: 417/419 gef.: Molpeak $(M+H)^+$: 417/419
Retentionszeit HPLC: 6.51 min (Methode A)
Die folgenden Verbindungen werden wie in Beispiel 4.1 b beschrieben hergestellt:

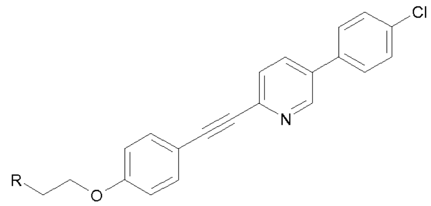

| Beispiel | R | Ausbeute (%) | Summenformel | Massenspektrum | Retentionszeit HPLC in min (Methode) |
|---|---|---|---|---|---|
| 4.2 | 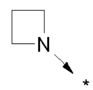 | 48.9 | $C_{24}H_{21}ClN_{2}O$ | 389/391 $[M+H]^+$ | 6.15 (A) |
| 4.3 | 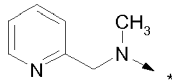 | 27.5 | $C_{28}H_{24}ClN_{3}O$ | 454/456 $[M+H]^+$ | 7.25 (A) |
| 4.4 | 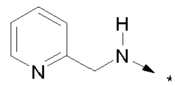 | 28.4 | $C_{27}H_{22}ClN_{3}O$ | 440/442 $[M+H]^+$ | 7.46 (A) |
| 4.5 | 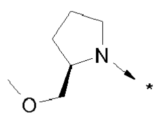 | 50.3 | $C_{27}H_{27}ClN_{2}O_{2}$ | 447/449 $[M+H]^+$ | 7.46 (A) |
| 4.6 | 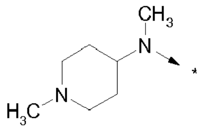 | 27.2 | $C_{28}H_{30}ClN_{3}0$ | 460/462 $[M+H]^+$ | 5.86 (A) |
| 4.7 | 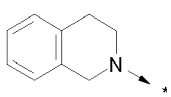 | 69.9 | $C_{30}H_{25}ClN_{2}0$ | 465/467 $[M+H]^+$ | 7.98 (A) |
| 4.8 | 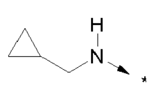 | 55.8 | $C_{25}H_{23}ClN_{2}0$ | 403/405 $[M+H]^+$ | 6.24 (A) |
| 4.9 | 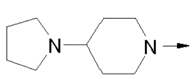 | 72.0 | $C_{30}H_{32}ClN_{3}0$ | 486/488 $[M+H]^+$ | 5.54 (A) |
| 4.10 | 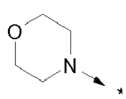 | 65.6 | $C_{25}H_{23}ClN_{2}O_{2}$ | 418/420 $[M+H]^+$ | 6.38 (A) |

(fortgesetzt)

| Beispiel | R | Ausbeute (%) | Summenformel | Massenspektrum | Retentionszeit HPLC in min (Methode) |
|---|---|---|---|---|---|
| 4.11 | 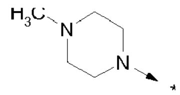 | 48.6 | $C_{26}H_{26}ClN_{3}0$ | 432/434 $[M+H]^+$ | 5.78 (A) |
| 4.12 | 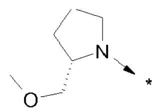 | 50.3 | $C_{27}H_{27}ClN_2O_2$ | 447/449 $[M+H]^+$ | 0.80 (EtOAc/ MeOH/$NH_3$ 90:10:1 ) |
| 4.13 | 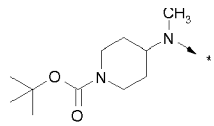 | 33.0 | $C_{32}H_{36}ClN_3O_3$ | 546/548 $[M+H]^+$ | 0.75 (EtOAc/ MeOH/$NH_3$ 90:10:1) |
| 4.14 | 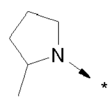 | 54.0 | $C_{26}H_{25}ClN_2O$ | 417/419 $[M+H]^+$ | 0.78 (EtOAc/ MeOH/$NH_3$ 90:10:1) |

Die folgenden Verbindungen werden wie in Beispiel 4.1 b beschrieben hergestellt, wobei man das Reaktionsgemisch nach Entfernen des Lösungsmittels mit 5 mL gesättigter $NaHCO_s$-Lösung versetzt, mit 40 mL DCM extrahiert und nach Abtrennen der organischen Phase diese mit $Na_2SO_4$ trocknet. Nach Entfernen des Trocken- und Lösungsmittels wird der Rückstand chromatographisch an Kieselgel gereinigt.

| Beispiel | R | Ausbeute (%) | Summenformel | Massenspektrum | Retentionszeit HPLC in in min (Methode) |
|---|---|---|---|---|---|
| 4.15 | 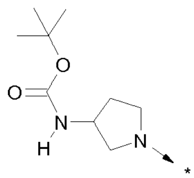 | 57.9 | $C_{30}H_{32}ClN_3O_3$ | 518/520 $[M+H]^+$ | 7.94 (A) |
| 4.16 | 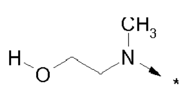 | 73.7 | $C_{24}H_{23}ClN_2O_2$ | 407/409 $[M+H]^+$ | 6.29 (A) |
| 4.17 | 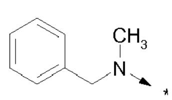 | 61.8 | $C_{29}H_{25}ClN_2O$ | 453/455 $[M+H]^+$ | 7.81 (A) |

Zu einer Lösung von 1 eq. Methansulfonsäure-2-{4-[5-(4-chlor-phenyl)-pyridin-2-ylethinyl]-phenoxy}-ethylester in DMF (2 mL/0.25 mmol) werden 3 eq. des entsprechenden Amins gegeben und das Reaktionsgemisch 16-72 h bei 60-70°C gerührt. Die Aufarbeitung erfolgt in 2 Varianten:

Variante A: Das Reaktionsgemisch wird direkt via HPLC gereinigt.

Variante B: Nach Abkühlen des Reaktionsgemisches wird der entstandene Niederschlag mit 1.5 mL Isopropanol

versetzt, abgesaugt, mit wenig Isopropanol nach gewaschen und im Umlufttrockenschrank bei 30˚C über Nacht getrocknet.

Die folgenden Verbindungen werden mit dieser Vorschrift erhalten:

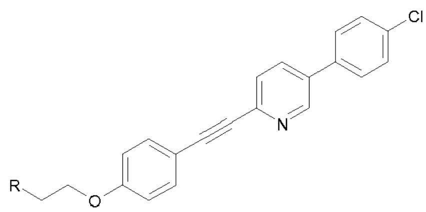

| Beispiel | R | Ausbeute (%) (Variante) | Summenformel | Massenspektrum | Retentionszeit HPLC in min (Methode) |
|---|---|---|---|---|---|
| 4.18 | 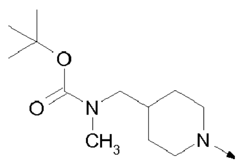 | 53.6 (A) | $C_{33}H_{38}ClN_3O$ | 560/562 $[M+H]^+$ | 5.44 (B) |
| 4.19 | 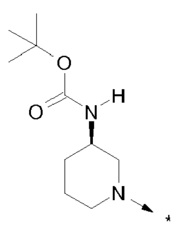 | 37.6 (A) | $C_{31}H_{34}ClN_3O$ | 532/534 $[M+H]^+$ | 5.24(B) |
| 4.20 | 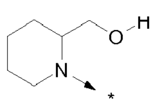 | 42.1 (A) | $C_{27}H_{27}ClN_2O$ | 447/449 $[M+H]^+$ | 7.19 (A) |
| 4.21 | 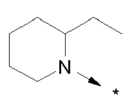 | 41.3 (A) | $C_{28}H_{29}ClN_2O$ | 445/447 $[M+H]^+$ | 7.95 (A) |
| 4.22 | 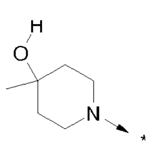 | 40.3 (A) | $C_{27}H_{27}ClN_2O_2$ | 447/449 $[M+H]^+$ | 4.68 (B) |
| 4.23 | 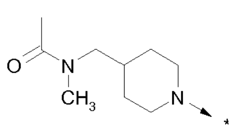 | 69.3 (A) | $C_{30}H_{32}ClN_3O_2$ | 502/504 $[M+H]^+$ | 6.86 (A) |

(fortgesetzt)

| Beispiel | R | Ausbeute (%) (Variante) | Summenformel | Massenspektrum | Retentionszeit HPLC in min (Methode) |
|---|---|---|---|---|---|
| 4.24 | 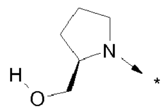 | 70.2 (A) | $C_{26}H_{25}ClN_2O_2$ | 433/435 $[M+H]^+$ | 4.68 (A) |
| 4.25 | 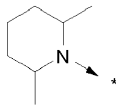 | 27.0 (A) | $C_{28}H_{29}ClN_2O$ | 445/447 $[M+H]^+$ | 5.30 (B) |
| 4.26 | 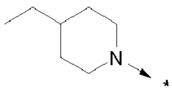 | 23.4 (A) | $C_{28}H_{29}ClN_2O$ | 445/447 $[M+H]^+$ | 8.09 (A) |
| 4.27 | 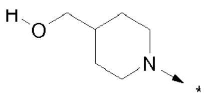 | 70.7 (A) | $C_{27}H_{27}ClN_2O_2$ | 447/449 $[M+H]^+$ | 6.79 (A) |
| 4.28 | 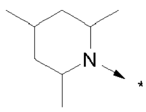 | 20.0 (B) | $C_{29}H_{31}ClN_2O$ | 459/461 $[M+H]^+$ | 5.49 (B) |
| 4.29 | 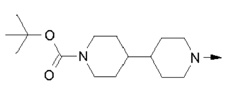 | 66.6 (B) | $C_{36}H_{42}ClN_3O_3$ | 600/602 $[M+H]^+$ | 5.70 (B) |
| 4.30 | 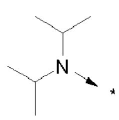 | 9.2 (A) | $C_{27}H_{29}ClN_2O$ | 433/435 $[M+H]^+$ | 5.20 (B) |
| 4.31 | 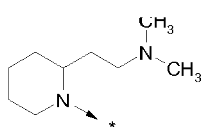 | 22.9 (A) | $C_{30}H_{34}ClN_3O$ | 488/490 $[M+H]^+$ | 5.60 (A) |
| 4.32 | 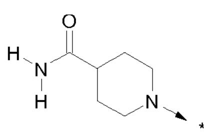 | 87.0 (B) | $C_{27}H_{26}ClN_3O_2$ | 460/462 $[M+H]^+$ | 0.12 (DCM/ MeOH/$NH_3$ 95: 5:0.5) |
| 4.33 | 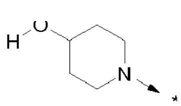 | 48.0 (B) | $C_{26}H_{25}ClN_2O_2$ | 433/435 $[M+H]^+$ | 0.13 (DCM/ MeOH/$NH_3$ 95: 5:0.5) |

(fortgesetzt)

| Beispiel | R | Ausbeute (%) (Variante) | Summenformel | Massenspektrum | Retentionszeit HPLC in min (Methode) |
|---|---|---|---|---|---|
| 4.34 | 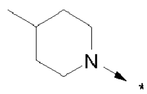 | 39.0 (B) | $C_{27}H_{27}ClN_2O$ | 431/433 $[M+H]^+$ | 0.28 (DCM/ MeOH/$NH_3$ 95: 5:0.5) |
| 4.35 | 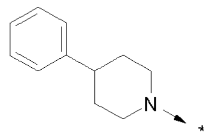 | 61.7 (B) | $C_{31}H_{29}ClN_2O$ | 493/495 $[M+H]^+$ | 0.35 (DCM/ MeOH/$NH_3$ 95: 5:0.5) |
| 4.36 | 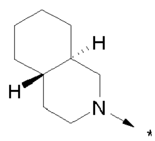 | 20.4 (B) | $C_{30}H_{31}ClN_2O$ | 471/473 $[M+H]^+$ | 0.25 (DCM/ MeOH/$NH_3$ 95: 5:0.5) |
| 4.37 | 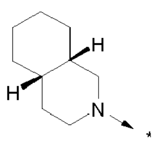 | 75.6 (B) | $C_{30}H_{31}ClN_2O$ | 471/473 $[M+H]^+$ | 0.23 (DCM/ MeOH/$NH_3$ 95: 5:0.5) |
| 4.38 | 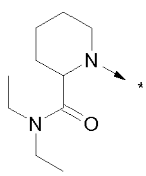 | 69.0 (B) | $C_{31}H_{34}ClN_3O_2$ | 516/518 $[M+H]^+$ | 0.20 (DCM/ MeOH/$NH_3$ 95: 5:0.5) |
| 4.39 | 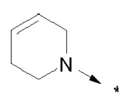 | 40.5 (B) | $C_{26}H_{23}ClN_2O$ | 415/417 $[M+H]^+$ | 0.22 (DCM/ MeOH/$NH_3$ 95: 5:0.5) |
| 4.40 | 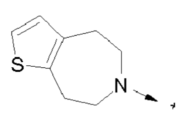 | 63.5 (B) | $C_{29}H_{25}ClN_2OS$ | 485/487 $[M+H]^+$ | 0.18 (DCM/ MeOH/$NH_3$ 9:1: 0.1) |
| 4.41 | 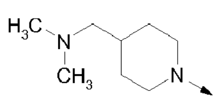 | 38.8 (B) | $C_{29}H_{32}ClN_3O$ | 474/476 $[M+H]^+$ | 0.09 (DCM/ MeOH/$NH_3$ 95: 5:0.5) |

(fortgesetzt)

| Beispiel | R | Ausbeute (%) (Variante) | Summenformel | Massenspektrum | Retentionszeit HPLC in min (Methode) |
|---|---|---|---|---|---|
| 4.42 | 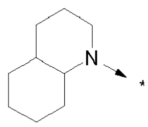 | 41.7 (B) | $C_{30}H_{31}ClN_2O$ | 471/473 $[M+H]^+$ | 0.30 (DCM/ MeOH/$NH_3$ 95: 5:0.5) |
| 4.43 | 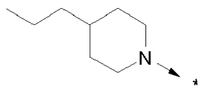 | 55.8 (B) | $C_{29}H_{31}ClN_2O$ | 459/461 $[M+H]^+$ | 0.23 (DCM/ MeOH/$NH_3$ 95: 5:0.5) |
| 4.44 | 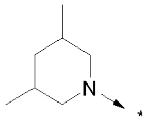 | 29.7 (B) | $C_{28}H_{29}ClN_2O$ | 445/447 $[M+H]^+$ | 0.32 (DCM/ MeOH/$NH_3$ 95: 5:0.5) |

**Beispiel 4.45**

1-(2-{4-[5-(4-Chlor-phenyl)-pyridin-2-ylethinyl]-phenoxyl-ethyl)-[4,4']bipiperidin

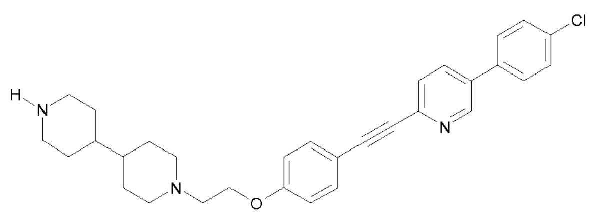

Zu einer Lösung von 200 mg (0.33 mmol) 1'-(2-{4-[5-(4-Chlor-phenyl)-pyridin-2-ylethinyl]-phenoxy}-ethyl)-[4,4'] bipiperidinyl-1-carbonsäure-*tert*-butylester (Beispiel 4.29) in 5 mL DCM werden 3 mL einer 5 N HCl-Lösung in Isopropanol zugegeben und das Reaktionsgemisch 4 h bei RT gerührt. Man verdünnt mit 30 mL DCM, neutralisiert mit gesättigter $NaHCO_s$-Lösung, versetzt mit 30 mL Wasser, extrahiert die wässrige Phase erschöpfend mit DCM und trocknet die vereinigten organischen Phasen über $MgSO_4$. Nach Entfernen des Trocken- und Lösungsmittels erhält man das gewünschte Produkt.
Ausbeute: 127 mg (76.3 % d. Theorie)
$C_{31}H_{34}ClN_3O$ (M= 500,089)
ber.: Molpeak $(M+H)^+$: 500/502 gef.: Molpeak $(M+H)^+$: 500/502
$R_f$-Wert: 0.10 (Kieselgel, DCM/MeOH/$NH_3$ 9:1:0.1 )

**Beispiel 4.46**

(R)-1-(2-{4-[5-(4-Chlor-phenyl)-pyridin-2-ylethinyl]-phenoxy)-ethyl)-piperidin-3-ylamin

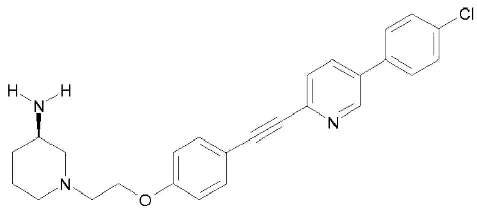

Zu einer Lösung von 110 mg (0.21 mmol) [(R)-1 -(2-{4-[5-(4-Chlor-phenyl)-pyridin-2-ylethinyl]-phenoxy)-ethyl)-piperidin-3-yl]-carbaminsäure-*tert*-butylester (Beispiel 4.19) in 5 mL DCM werden 1.5 mL einer 5 N HCl-Lösung in Isopropanol zugegeben und das Reaktionsgemisch 4 h bei RT gerührt. Der entstandene Niederschlag wird mit wenig *tert*-Butylmethylether versetzt, filtriert, mit *tert*-Butylmethylether gewaschen und bei 30˚C getrocknet.
Ausbeute: 104 mg (99.5 % d. Theorie)
$C_{26}H_{26}C1N_3O$*2HCl (M= 504,892)
ber.: Molpeak $(M+H)^+$: 432/434 gef.: Molpeak $(M+H)^+$: 432/434
$R_f$-Wert: 0.27 (Kieselgel, DCM/MeOH/$NH_3$ 9:1:0.1)

**Beispiel 4.47**

[1 -(2-(4-[5-(4-Chlor-phenyl)-pyridin-2-ylethinyl]-phenoxy)-ethyl)-piperidin-4-ylmethyl]-methyl-amin

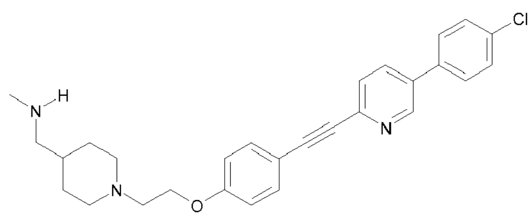

Hergestellt analog Beispiel 4.46 aus 160 mg (0.29 mmol) [1-(2-{4-[5-(4-Chlor-phenyl)-pyrid in-2-y1lethinyl]-phenoxy}-ethyl)-piperid in-4-ylmethyl]-methyl-carbaminsäure-*tert*-butylester (Beispiel 4.18).
Ausbeute: 156 mg (100 % d. Theorie)
$C_{28}H_{30}C1N_3O^*$2HCl (M= 532,946)
ber.: Molpeak $(M+H)^+$: 460/462 gef.: Molpeak $(M+H)^+$: 460/462
$R_f$-Wert: 0.13 (Kieselgel, DCM/MeOH/$NH_3$ 9:1:0.1)

**Beispiel 4.48**

1 -(2-{4-[5-(4-Chlor-phenyl)-pyridin-2-ylethinyl]-phenoxy}-ethyl)-pyrrolidin-3-ylamin

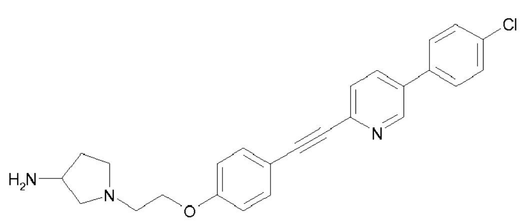

Zu einer Lösung von 45 mg (0.09 mmol) [1-(2-{4-[5-(4-Chlor-phenyl)-pyridin-2-ylethinyl]-phenoxy}-ethyl)-pyrrolidin-3-yl]-carbaminsäure-*tert*-butylester (Beispiel 4.15) in 5 mL DCM werden 1 mL Trifluoressigsäure gegeben und das Reaktionsgemisch für 24 h bei RT gerührt. Man engt i.vac. ein, versetzt den Rückstand mit 20 mL DCM, wäscht die organische Phase mit gesättigter $NaHCO_s$-Lösung und trocknet über $Na_2SO_4$. Nach Entfernen des Trocken- und Lösungsmittels erhält man das gewünschte Produkt.
Ausbeute: 15 mg (41.3 % d. Theorie)
$C_{25}H_{24}ClN_3O$ (M= 417,943)
ber.: Molpeak $(M+H)^+$: 418/420 gef.: Molpeak $(M+H)^+$: 418/420
Retentionszeit HPLC: 5.86 min (Methode A)

**Beispiel 4.49**

(2-{4-[5-(4-Chlor-phenyl)-pyridin-2-ylethinyl]-phenoxy}-ethyl)-methyl-piperidin-4-yl-amin

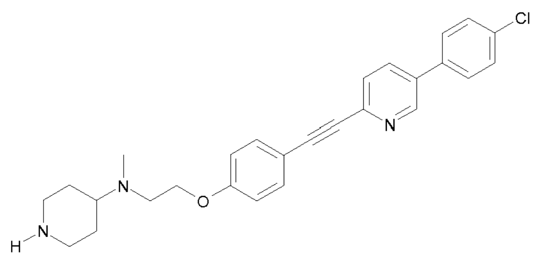

Zu einer Lösung von 22 mg (0.04 mmol) 4-[(2-{4-[5-(4-Chlor-phenyl)-pyridin-2-ylethinyl]-phenoxy}-ethyl)-methyl-amino]-piperidin-1-carbonsäure-*tert*-butylester in 5 mL DCM werden 60 μL (0.8 mmol) Trifluoressigsäure gegeben und das Reaktionsgemisch 24 h bei RT gerührt. Man engt i.vac. ein und verrührt den Rückstand mit Diethylether. Der Niederschlag wird abgesaugt, mit Diethylether gewaschen und getrocknet.
Ausbeute: 12 mg (67.3 % d. Theorie)
$C_{27}H_{28}C1N_3O*CF_3COOH$ (M= 560,017)
ber.: Molpeak $(M+H)^+$: 445/447 gef.: Molpeak $(M+H)^+$: 445/447
$R_f$-Wert: 0.07 (Kieselgel, EtOAc/MeOH/$NH_3$80:20:2)

Beispiel 4.50

1 -(2-{4-[5-(4-Chlor-phenyl)-pyridin-2-ylethinyl]-phenoxy)-ethyl)-pyrrolidin-2-carbonsäuremethylester

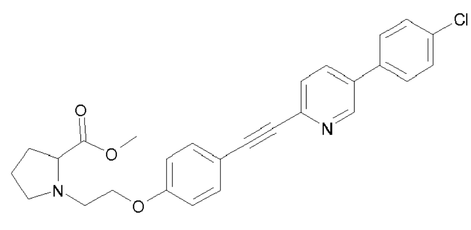

Zu einer Lösung von 171 mg (0.4 mmol) Methansulfonsäure-2-{4-[5-(4-chlor-phenyl)-pyridin-2-ylethinyl]-phenoxy}-ethylester in 2 mL DMF werden 0.15 mL Ethyldiisopropylamin und 73 mg (0.44 mmol) Prolin-methylester (eingesetzt als Hydrochlorid) gegeben und das Reaktionsgemisch 18 h bei RT gerührt. Man engt i.vac. ein und reinigt den Rückstand via HPLC.
Ausbeute: 10 mg (5.4 % d. Theorie)
$C_{27}H_{25}ClN_2O_3$ (M= 460,965)
ber.: Molpeak $(M+H)^+$: 461/463 gef.: Molpeak $(M+H)^+$: 461/463
$R_f$-Wert: 0.79 (Kieselgel, Cyc/EtOAc 1:1)

Mit beschriebenen Verfahren können folgende Verbindungen hergestellt werden:

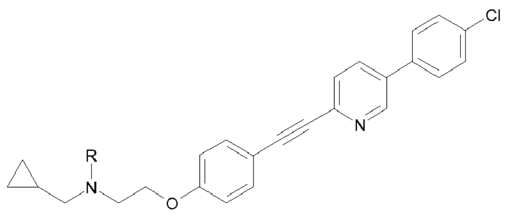

| Beispiel | R |
|---|---|
| 4.51 | 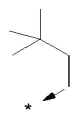 |
| 4.52 | 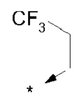 |
| 4.53 | 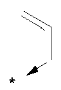 |
| 4.54 | 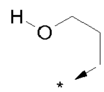 |
| 4.55 | 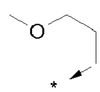 |
| 4.56 | 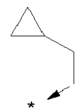 |
| 4.57 | 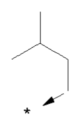 |

**Beispiel 4.58**

[1 -(2-{4-[5-(4-Chlor-phenyl)-pyridin-2-ylethinyl]-phenoxy}-ethyl)-piperidin-4-ylmethyl]-dimethyl-amin Hydrochlorid

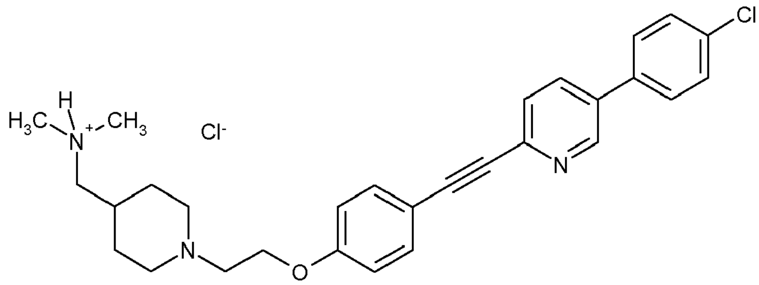

Eine Lösung von 15 mg (0.03 mmol) [1-(2-{4-[5-(4-Chlor-phenyl)-pyridin-2-ylethinyl]-phenoxy}-ethyl)-piperidin-4-ylmethyl]-dimethyl-amin (Beispiel 4.41) in 6 mL DCM und 4 mL Aceton wird solange mit gesättigter etherischer HCl-Lösung versetzt bis sich bei der Zugabe kein Niederschlag mehr bildet. Das entstandene Salz wird im Stickstoffstrom abgesaugt und getrocknet.
Ausbeute: 10 mg (61.2 % d. Theorie)
$C_{29}H_{32}C1N_3O$*HCl (M= 510,512)
ber.: Molpeak $(M+H)^+$: 474/476 gef.: Molpeak $(M+H)^+$: 474/476
Fp: >250˚C

Beispiel 5

5-(4-Chlor-phenyl )-2-[3-methyl-4-(2-pyrrol id in-1-yl-ethoxy)-phenyleth i nyl]-pyrid in

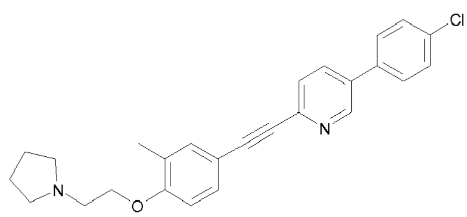

5a 2-(4-lod-2-methyl-phenoxy)-ethanol

Unter $N_2$-Atmosphäre werden zu einer auf 0˚C gekühlten Suspension von 0.48 g (11 mmol) NaH in 50 mL THF 2.34 g (10 mmol) 4-lod-2-methyl-phenol portionenweise zugegeben und weitere 30 min bei dieser Temperatur nachgerührt. Dann werden 0.85 mL (12 mmol) 2-Bromethanol, gelöst in 5 mL THF, zugetropft und 18 h bei RT gerührt. Man versetzt mit 5 mL DMF und erhitzt das Reaktionsgemisch für 8 h auf 70˚C. Man engt i.vac. ein, nimmt den Rückstand in Wasser auf, extrahiert erschöpfend mit EtOAc und trocknet mit $Na_2SO_4$. Nach Entfernen des Trocken- und Lösungsmittels wird der Rückstand chromatographisch an Kieselgel (Cyc/EtOAc 7:3) gereinigt.
Ausbeute: 0.39 g (14.0 % d. Theorie)
$C_9H_{11}IO_2$ (M= 278,091)
ber.: Molpeak $(M+H)^+$: 279 gef.: Molpeak $(M+H)^+$: 279
$R_f$-Wert: 0.28 (Kieselgel, Cyc/EtOAc 2:1)

5b 2-{4-[5-(4-Chlor-phenyl)-pyridin-2-ylethinyl]-2-methyl-phenoxy}-ethanol

Hergestellt analog Beispiel 4b aus 380 mg (1.37 mmol) 2-(4-lod-2-methyl-phenoxy)-ethanol und 292 mg (1.37 mmol) 5-(4-Chlor-phenyl)-2-ethinyl-pyridin in 38 mL Piperidin.
Ausbeute: 340 mg (68.4 % d. Theorie)
$C_{22}H_{18}ClNO_2$ (M= 363,847)
ber.: Molpeak $(M+H)^+$: 364 gef.: Molpeak $(M+H)^+$: 364
$R_f$-Wert: 0.26 (Kieselgel, Cyc/EtOAc 1:1)

5c Methansulfonsäure 2-{4-[5-(4-chlor-phenyl)-pyridin-2-yiethynyl]-2-methyl-phenoxy}-ethyl-ester

Hergestellt analog Beispiel 4.1 a aus 310 mg (0.93 mmol) 2-{4-[5-(4-Chlor-phenyl)-pyridin-2-ylethinyl]-2-methyl-phenoxy}-ethanol und 88 μL (1.12 mmol) Methansulfonsäurechlorid.
Ausbeute: 300 mg (72.7 % d. Theorie)
$C_{23}H_{20}ClNO_4S$ (M= 441,937)
ber.: Molpeak $(M+H)^+$: 442/444 gef.: Molpeak $(M+H)^+$: 442/444
$R_f$-Wert: 0.35 (Kieselgel, Cyc/EtOAc 1:1)

5d 5-(4-Chlor-phenyl)-2-[3-methyl-4-(2-pyrrolidin-1-yl-ethoxy)-phenylethinyl]-pyridin

Eine Lösung von 110 mg (0.25 mmol) Methansulfonsäure-2-{4-[5-(4-chlor-phenyl)-pyridin-2-ylethinyl]-2-methyl-phenoxy}-ethyl-ester in 2.11 mL (25 mmol) Pyrrolidin wird 3 h auf 70˚C erwärmt. Man engt i.vac. ein, versetzt den Rückstand mit Wasser, extrahiert erschöpfend mit DCM und trocknet die organische Phase über $Na_2SO_4$. Nach Entfernen des Trocken- und Lösungsmittels und Umkristallisation aus EtOH erhält man das gewünschte Produkt.
Ausbeute: 55 mg (52.8 % d. Theorie)
$C_{26}H_{25}ClN_2O$ (M= 416,955)
ber.: Molpeak $(M+H)^+$: 417/419 gef.: Molpeak $(M+H)^+$: 417/419
Retentionszeit HPLC: 7.19 min (Methode A)

**Beispiel 5.1**

5-(4-Chlor-phenyl)-2-{4-[2-(2,5-dihydro-pyrrol-1 -yl)-ethoxy]-3-methyl-phenylethinyl}-pyridin

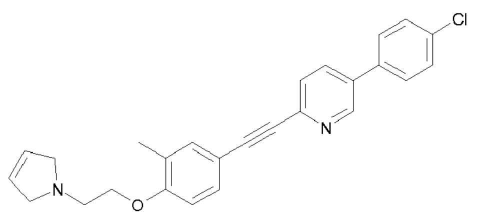

Hergestellt analog Beispiel 5d aus 110 mg (0.25 mmol) Methansulfonsäure-2-{4-[5-(4-chlor-phenyl)-pyridin-2-ylethinyl]-2-methyl-phenoxy}-ethyl-ester und 1.92 mL (25 mmol) 2,5-Dihydro-1*H*-pyrrol.
Ausbeute: 10 mg (9.6 % d. Theorie)
$C_{26}H_{23}ClN_2O$ (M= 414,939)
ber.: Molpeak $(M+H)^+$: 415/417 gef.: Molpeak $(M+H)^+$: 415/417
$R_f$-Wert: 0.50 (Kieselgel, EtOAc/MeOH/$NH_3$95:5:0.5)

Beispiel 5.2

5-(4-Chlor-phenyl)-2-{4-[2-(4-isopropyl-piperidin-1-yl)-ethoxy]-3-methyl-phenylethinyl}-pyridin

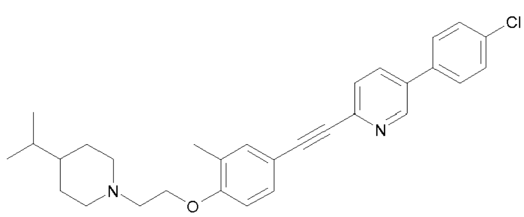

Zu einer Lösung von 88 mg (0.2 mmol) Methansulfonsäure-2-{4-[5-(4-chlor-phenyl)-pyridin-2-ylethinyl]-2-methyl-phenoxy}-ethyl-ester und 0.34 mL (2 mmol) Ethyldiisopropylamin in 1.8 mL DMF werden 164 mg (1.0 mmol) 4-Isopropyl-piperidin (eingesetzt als Hydrochlorid) gegeben und das Reaktionsgemisch 24 h bei RT gerührt. Man filtriert durch einen Spritzenfilter und reinigt den Reaktionsansatz via HPLC.
Ausbeute: 18 mg (19.4 % d. Theorie)
$C_{30}H_{33}ClN_2O$ (M= 473,063)
ber.: Molpeak $(M+H)^+$: 473/475 gef.: Molpeak $(M+H)^+$: 473/475
Retentionszeit HPLC: 5.70 min (Methode B)

Die folgenden Verbindungen werden wie in Beispiel 5.2 beschrieben hergestellt:

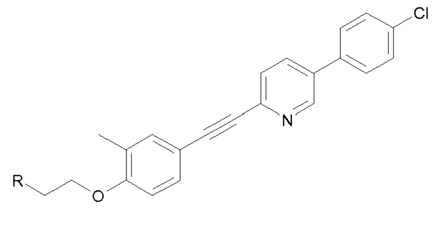

| Beispiel | R | Ausbeute (%) | Summenformel | Massen-spektrum | Retentionszeit HPLC in min (Methode) |
|---|---|---|---|---|---|
| 5.3 | 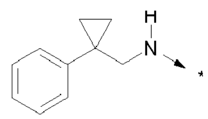 | 18.3 | $C_{32}H_{29}ClN_2O$ | 493/495 $[M+H]^+$ | 5.70 (B) |
| 5.4 | 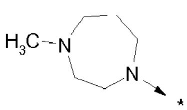 | 48.9 | $C_{28}H_{30}ClN_3O$ | 460/462 $[M+H]^+$ | 4.22 (B) |
| 5.5 | 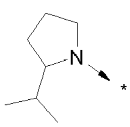 | 10.9 | $C_{29}H_{31}ClN_2O$ | 459/461 $[M+H]^+$ | 5.49 (B) |
| 5.6 | 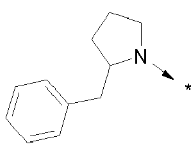 | 25.6 | $C_{33}H_{31}ClN_2O$ | 507/509 $[M+H]^+$ | 5.73 (B) |
| 5.7 | 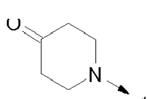 | 20.2 | $C_{27}H_{25}ClN_2O_2$ | 445/447 $[M+H]^+$ | 4.82 (B) |
| 5.8 | 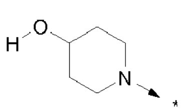 | 24.6 | $C_{27}H_{27}C1N_2O_2$ | 447/449 $[M+H]^+$ | 4.69 (B) |

(fortgesetzt)

| Beispiel | R | Ausbeute (%) | Summenformel | Massen-spektrum | Retentionszeit HPLC in min (Methode) |
|---|---|---|---|---|---|
| 5.9 | 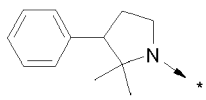 | 17.3 | $C_{34}H_{33}ClN_2O$ | 521/523 $[M+H]^+$ | 5.83 (B) |
| 5.10 | 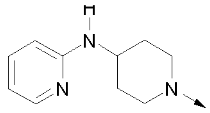 | 40.1 | $C_{32}H_{31}ClN_4O$ | 523/525 $[M+H]^+$ | 4.25 (B) |
| 5.11 | 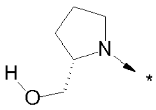 | 12.3 | $C_{27}H_{27}ClN_2O_2$ | 447/449 $[M+H]^+$ | 4.89 (B) |
| 5.12 | 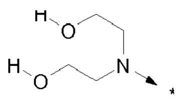 | 31.0 | $C_{26}H_{27}ClN_2O_3$ | 451/453 $[M+H]^+$ | 4.62 (B) |
| 5.13 | 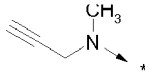 | 17.7 | $C_{26}H_{23}ClN_2O$ | 415/417 $[M+H]^+$ | 5.03 (B) |
| 5.14 | 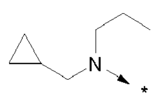 | 29.8 | $C_{29}H_{31}ClN_2O$ | 459/461 $[M+H]^+$ | 5.46 (B) |
| 5.15 | 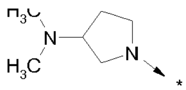 | 52.9 | $C_{28}H_{30}ClN_3O$ | 460/462 $[M+H]^+$ | 4.15 (B) |
| 5.16 | 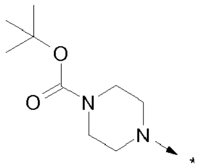 | 21.4 | $C_{31}H_{34}ClN_3O_3$ | 532/534 $[M+H]^+$ | 5.43 (B) |
| 5.17 | 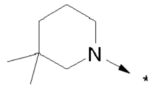 | 19.8 | $C_{29}H_{31}ClN_2O$ | 459/461 $[M+H]^+$ | 5.43 (B) |

(fortgesetzt)

| Beispiel | R | Ausbeute (%) | Summenformel | Massen-spektrum | Retentionszeit HPLC in min (Methode) |
|---|---|---|---|---|---|
| 5.18 | 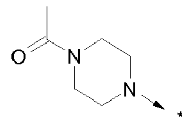 | 28.1 | $C_{28}H_{28}ClN_3O_2$ | 474/476 $[M+H]^+$ | 4.66 (B) |
| 5.19 | 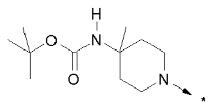 | 11.4 | $C_{33}H_{38}ClN_3O_3$ | 560/562 $[M+H]^+$ | 5.60 (B) |
| 5.20 | 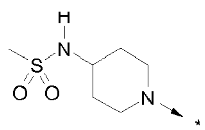 | 7.4 | $C_{28}H_{30}ClN_3O_3S$ | 524/526 $[M+H]^+$ | 4.79 (B) |
| 5.21 | 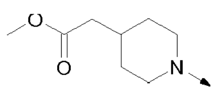 | 13.5 | $C_{30}H_{31}C1N_2O_3$ | 503/505 $[M+H]^+$ | 5.16 (B) |
| 5.22 | 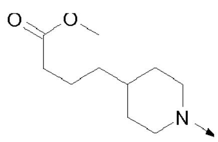 | 14.0 | $C_{32}H_{35}ClN_2O_3$ | 531/533 $[M+H]^+$ | 5.39 (B) |
| 5.23 | 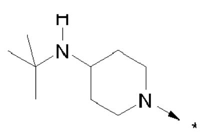 | 16.6 | $C_{31}H_{36}ClN_3O$ | 502/504 $[M+H]^+$ | 4.15 (B) |
| 5.24 | 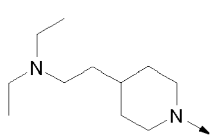 | 24.5 | $C_{33}H_{40}ClN_3O$ | 530/532 $[M+H]^+$ | 4.19 (B) |
| 5.25 | 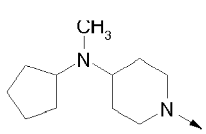 | 4.8 | $C_{33}H_{38}ClN_3O$ | 528/530 $[M+H]^+$ | 4.22 (B) |

(fortgesetzt)

| Beispiel | R | Ausbeute (%) | Summenformel | Massen-spektrum | Retentionszeit HPLC in min (Methode) |
|---|---|---|---|---|---|
| 5.26 | 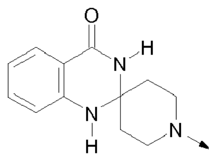 | 24.0 | $C_{34}H_{31}ClN_4O_2$ | 563/565 $[M+H]^+$ | 4.92 (B) |
| 5.27 | 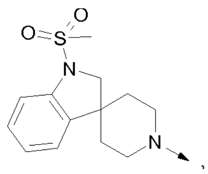 | 36.8 | $C_{35}H_{34}ClN_3O_3S$ | 612/614 $[M+H]^+$ | 5.43 (B) |

Die folgenden Verbindungen werden wie in Beispiel 5.2 beschrieben hergestellt, wobei man nach beendeter Reaktion das Reaktionsgemisch i.vac. eingeengt, den Rückstand mit Wasser versetzt, die wässrige Phase erschöpfend mit DCM extrahiert und die organische Phase über $Na_2SO_4$ trocknet. Nach Entfernen des Trocken- und Lösungsmittels wird der Rückstand chromatographisch an Kieselgel (DCM/MeOH 95:5 oder 8:2) gereinigt.

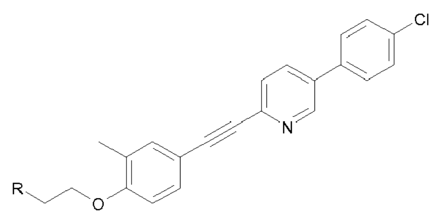

| Beispiel | R | Ausbeute (%) | Summenformel | Massen-spektrum | Retentionszeit HPLC in min (Methode) |
|---|---|---|---|---|---|
| 5.28 | 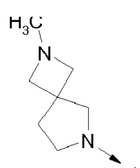 | 37.6 | $C_{29}H_{30}ClN_3O$ | 472/474 $[M+H]^+$ | 4.05 (B) |
| 5.29 | 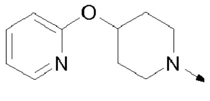 | 35.8 | $C_{32}H_{30}ClN_3O_2$ | 524/526 $[M+H]^+$ | 5.43 (B) |

(fortgesetzt)

| Beispiel | R | Ausbeute (%) | Summenformel | Massen-spektrum | Retentionszeit HPLC in min (Methode) |
|---|---|---|---|---|---|
| 5.30 | 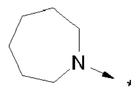 | 60.7 | $C_{28}H_{29}ClN_2O$ | 472/474 $[M+H]^+$ | 5.26 (B) |
| 5.31 | 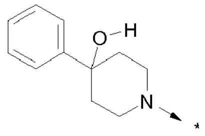 | 37.1 | $C_{33}H_{31}ClN_2O_2$ | 523/525 $[M+H]^+$ | 5.33 (B) |
| 5.32 | 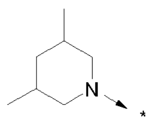 | 53.2 | $C_{29}H_{31}ClN_2O$ | 459/461 $[M+H]^+$ | 5.53 (B) |
| 5.33 | 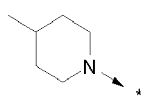 | 48.8 | $C_{28}H_{29}ClN_2O$ | 445/447 $[M+H]^+$ | 5.26 (B) |
| 5.34 | 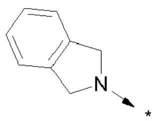 | 46.1 | $C_{30}H_{25}ClN_2O$ | 465/467 $[M+H]^+$ | 5 |

Die folgenden Verbindungen werden wie in Beispiel 5.2 beschrieben hergestellt, wobei das Reaktionsgemisch je nach Bedarf zwischen 4 und 18 h auf 60˚C erwärmt wird. Man engt nach beendeter Reaktion das Reaktionsgemisch i.vac. ein, versetzt den Rückstand mit Wasser, extrahiert die wässrige Phase erschöpfend mit DCM und trocknet die organische Phase über $Na_2S0_4$. Nach Entfernen des Trocken- und Lösungsmittels wird der Rückstand chromatographisch an Alox gereinigt.

| Beispiel | R | Ausbeute (%) | Summenformel | Massen-spektrum | $R_f$-Wert auf Alox (Fliessmittel) |
|---|---|---|---|---|---|
| 5.35 | 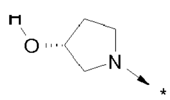 | 64.7 | $C_{26}H_{25}ClN_2O_2$ | 433/435 $[M+H]^+$ | 0.52 (EtOAc) |
| 5.36 | 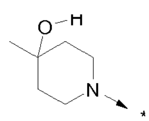 | 74.8 | $C_{28}H_{29}ClN_2O_2$ | 461 /463 $[M+H]^+$ | 0.31 (Cyc/ EtOAc1:1) |
| 5.37 | 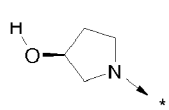 | 48.5 | $C_{26}H_{25}ClN_2O_2$ | 433/435 $[M+H]^+$ | 0.52 (Cyc/EtOAc 1:2) |

(fortgesetzt)

| Beispiel | R | Ausbeute (%) | Summenformel | Massen-spektrum | $R_f$-Wert auf Alox (Fliessmittel) |
|---|---|---|---|---|---|
| 5.38 | 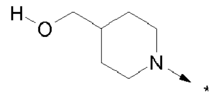 | 78.1 | $C_{28}H_{29}C1\ _{N}2_{0}2$ | 461 /463 [M+H]+ | 0.38 (Cyc/EtOAc 1:3) |
| 5.39 | 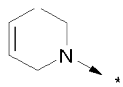 | 65.3 | $C_{27}H_{25}ClN_2O$ | 429/431 [M+H]+ | 0.70 (Cyc/EtOAc 2:1) |
| 5.40 | 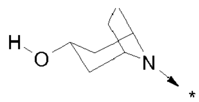 | 49.7 | $C_2sH_{29}ClN_2O_2$ | 473/475 [M+H]+ | 0.34 (Cyc/EtOAc 1:1) |
| 5.41 | 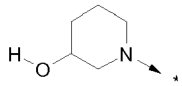 | 52.6 | $C_{27}H_{27}ClN_2O_2$ | 447/449 [M+H]+ | 0.21 (Cyc/EtOAc 1:1) |
| 5.42 | 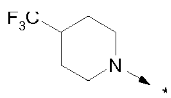 | 81.8 | $C_{28}H_{26}ClF_3N_2O_2$ | 499/501 [M+H]+ | 0.57 (Cyc/EtOAc 3:1) |
| 5.43 | 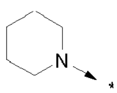 | 47.0 | $C_{27}H_{27}ClN_2O$ | 431/433 [M+H]+ | 0.72 (Cyc/EtOAc 2:1) |
| 5.44 | 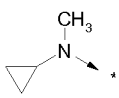 | 12.0 | $C_{26}H_{25}ClN_2O$ | 417/419 [M+H]+ | 0.52 (Cyc/EtOAc 4:1) |
| 5.45 | 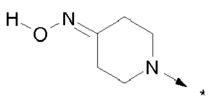 | 32.6 | $C_{27}H_{26}ClN_3O_2$ | 461/463 [M+H]+ | 0.27 (Cyc/EtOAc 1:1) |
| 5.46 | 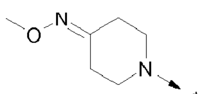 | 44.3 | $C_{28}H_{28}ClN_3O_2$ | 474/476 [M+H]+ | 0.25 (Cyc/EtOAc 4:1) |

Die folgenden Verbindungen werden wie in Beispiel 5.2 beschrieben hergestellt, wobei das Reaktionsgemisch 18 h auf 60°C erwärmt wird.

| Beispiel | R | Ausbeute (%) | Summenformel | Massen-spektrum | $R_f$-Wert auf Alox (Fliessmittel) |
|---|---|---|---|---|---|
| 5.47 | 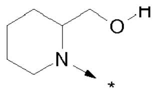 | 28.2 | $C_{28}H_{29}ClN2O_2$ | 461/463 $[M+H]^+$ | 0.40 (Cyc/EtOAc 1:1) |
| 5.48 | 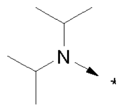 | 6.7 | $C_{28}H_{31}ClN_2O$ | 447/449 [M+H]+ | 0.63 (Cyc/EtOAc 4:1) |

Die folgenden Verbindungen werden wie in Beispiel 5.2 beschrieben hergestellt, wobei das Reaktionsgemisch je nach Bedarf zwischen 6 und 14 h auf 60˚C erwärmt wird. Man engt nach beendeter Reaktion das Reaktionsgemisch i.vac. ein, versetzt den Rückstand mit gesättigter $K_2CO_3$-Lösung, extrahiert die wässrige Phase erschöpfend mit DCM und trocknet die organische Phase über $Na_2SO_4$. Nach Entfernen des Trocken- und Lösungsmittels wird der Rückstand chromatographisch an Alox gereinigt.

| Beispiel | R | Ausbeute (%) | Summenformel | Massen-spektrum | $R_f$-Wert auf Alox (Fliessmittel) oder Retentionszeit HPLC in min (Methode) |
|---|---|---|---|---|---|
| 5.49 | 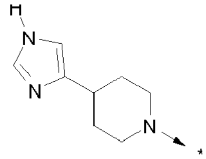 | 5.5 | $C_{30}H_{29}ClN_4O$ | 497/499 $[M+H]^+$ | 0.36 (Cyc/EtOAc 1: 1) |
| 5.50 | 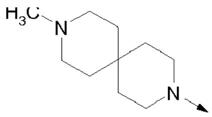 | 8.3 | $C_{32}H_{36}ClN_3O$ | 514/516 $[M+H]^+$ | 4.12 (B) |
| 5.51 | 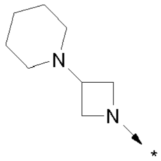 | 3.9 | $C_{30}H_{32}ClN_3O$ | 486/488 $[M+H]^+$ | 4.46 (B) |
| 5.52 | 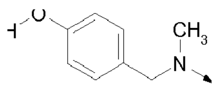 | 36.2 | $C_{30}H_{27}ClN_2O_2$ | 483/485 $[M+H]^+$ | 0.54 (Cyc/EtOAc 1: 1) |

Die folgenden Verbindungen werden wie in Beispiel 5.2 beschrieben hergestellt, wobei das Reaktionsgemisch je nach Bedarf zwischen 3 und 18 h auf 100˚C erwärmt wird. Man engt nach beendeter Reaktion das Reaktionsgemisch i.vac. ein, versetzt den Rückstand mit gesättigter $K_2CO_3$-Lösung, extrahiert die wässrige Phase erschöpfend mit DCM und trocknet die organische Phase über $Na_2SO_4$. Nach Entfernen des Trocken- und Lösungsmittels wird der Rückstand chromatographisch an Alox gereinigt.

| Beispiel | R | Ausbeute (%) | Summenformel | Massen-spektrum | $R_f$-Wert auf Alox (Fliessmittel) oder Retentionszeit HPLC in min (Methode) |
|---|---|---|---|---|---|
| 5.53 | 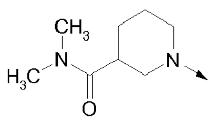 | 75.7 | $C_{30}H_{32}ClN_3O_2$ | 502/504 $[M+H]^+$ | 0.31 (Cyc/EtOAc 1:1) |
| 5.54 | 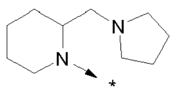 | 23.3 | $C_{32}H_{36}ClN_3O$ | 514/516 $[M+H]^+$ | 4.22 (B) |
| 5.55 | 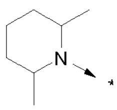 | 15.2 | $C_{29}H_{31}ClN_2O$ | 459/461 $[M+H]^+$ | 5.39 (B) |

Beispiel 5.56

1-(2-{4-[5-(4-Chlor-phenyl)-pyridin-2-ylethinyl]-2-methyl-phenoxy}-ethyl)-4-methylpiperidin-4-ylamin

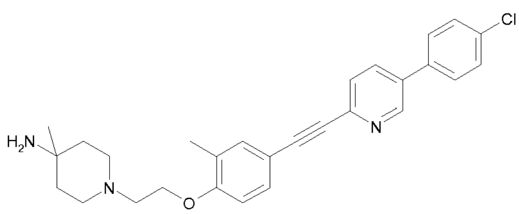

Zu einer Lösung von 130 mg (0.23 mmol) [1-(2-{4-[5-(4-Chlor-phenyl)-pyridin-2-ylethinyl]-2-methyl-phenoxy}-ethyl)-4-methyl-piperidin-4-yl]-carbaminsäure-*tert*-butylester (Beispiel 5.19) in 10 mL DCM wird 1 mL Trifluoressigsäure gegeben und das Reaktionsgemisch 14 h bei RT gerührt. Man engt i.vac. ein (Wasserbadtemperatur max. 30˚C), versetzt den Rückstand mit verdünnter $K_2C0_3$-Lösung, extrahiert erschöpfend mit DCM und trocknet die organische Phase mit $Na_2SO_4$. Nach Entfernen des Trocken- und Lösungsmittels wird der Rückstand mit Diisopropylether verrieben, der Niederschlag abgesaugt und an der Luft getrocknet.
Ausbeute: 65 mg (60.9 % d. Theorie)
$C_{28}H_{30}ClN_3O$ (M= 460,024)
ber.: Molpeak $(M+H)^+$: 460/462 gef.: Molpeak $(M+H)^+$: 460/462
Retentionszeit HPLC: 4.09 min (Methode B)

Mit beschriebenen Verfahren können folgende Verbindungen hergestellt werden:

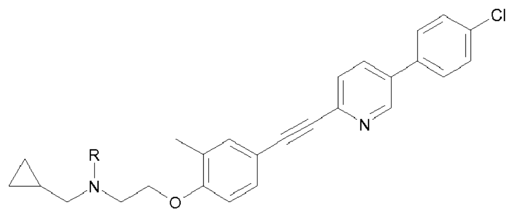

| Beispiel | R |
|---|---|
| 5.57 | 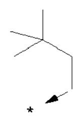 |
| 5.58 | 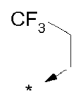 |
| 5.59 | 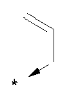 |
| 5.60 | 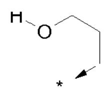 |
| 5.61 | 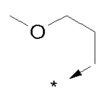 |
| 5.62 | 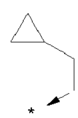 |
| 5.63 | 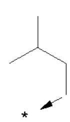 |

Beispiel 6

5-[5-(4-Chlor-phenyl)-pyridin-2-ylethinyl]-1-(2-pyrrolidin-1-yl-ethyl)-1*H*-indol

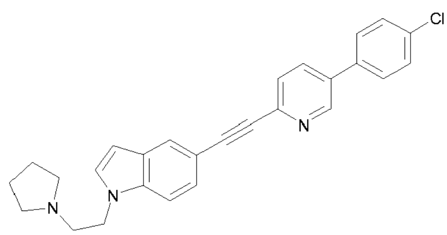

6a 2-(5-lod-indol-1-yl)-ethanol

Unter $N_2$-Atmosphäre werden zu einer Suspension von 27.1 g (484 mmol) KOH in 150 mL DMSO 30 g (121 mmol) 5-lodindol gegeben. Das Reaktionsgemisch wird 1 h bei RT gehalten, mit Eiswasser auf 0˚C gekühlt, 9.7 mL (145 mmol) 2-Chlorethanol in 30 mL DMSO werden langsam zugetropft und 4.5 h bei RT nachgerührt. Das Reaktionsgemisch wird mit 1 L EtOAc versetzt, viermal mit jeweils 800 mL Wasser und einmal mit 400 mL gesättigter NaCl-Lösung gewaschen und die organische Phase über $Na_2SO_4$ getrocknet. Nach Entfernen des Trocken- und Lösungsmittels wird der Rückstand chromatographisch (Kieselgel, Cyc/EtOAc 3:1 ) gereinigt.
Ausbeute: 20.5 g (59.1 % d. Theorie)
$C_{10}H_{10}INO$ (M= 287,102)
ber.: Molpeak $(M+H)^+$: 288 gef.: Molpeak $(M+H)^+$: 288
Retentionszeit HPLC: 7.98 min (Methode A)

6b 2-(5-Trimethylsilanylethinyl-indol-1 -yl)-ethanol

Zu einer auf 0˚C gekühlten Lösung von 30 g (104 mmol) 2-(5-lod-indol-1-yl)-ethanol und 18 mL (125 mmol) Ethinyl-trimethyl-silan in 480 mL Triethylamin und 120 mL THF werden 398 mg (2.1 mmol) CuI und 1.47 g (2.1 mmol) $Pd(PPh_3)_2Cl_2$ gegeben und 30 min bei 0˚C und 2 h bei RT nachgerührt. Man engt i.vac. ein, nimmt den Rückstand in 300 mL EtOAc auf, wäscht die organische Phase mit 150 mL Wasser und trocknet über $Na_2SO_4$. Nach Entfernen des Trocken- und Lösungsmittels wird der Rückstand chromatographisch (Kieselgel, Gradient: Cyc/EtOAc 4:1 nach 2:1) gereinigt.
Ausbeute: 26.85 g (100 % d. Theorie)
$C_{15}H_{19}NOSi$ (M= 257,411)
ber.: Molpeak $(M+H)^+$: 258 gef.: Molpeak $(M+H)^+$: 258
$R_f$-Wert: 0.25 (Kieselgel, Cyc/EtOAc 2:1)

6c 2-(5-Ethinyl-indol-1-yl)-ethanol

Unter $N_2$-Atmosphäre werden zu einer Lösung von 21.5 g (83.5 mmol) 2-(5-Trimethylsilanylethinyl-indol-1-yl)-ethanol in 500 mL THF 29 g (91.8 mmol) TBAF gegeben und das Reaktionsgemisch 1.5 h bei RT gerührt. Man engt i.vac. ein, nimmt den Rückstand in 300 mL EtOAc auf, wäscht die organische Phase zweimal mit jeweils 200 mL Wasser und einmal mit 200 mL gesättigter NaCl-Lösung und trocknet über $Na_2SO_4$. Nach Entfernen des Trocken- und Lösungsmittels erhält man das gewünschte Produkt in Form eines brauen Öls.
Ausbeute: 15.46 g (100 % d. Theorie)
$C_{12}H_{11}$ NO (M= 185,228)
ber.: Molpeak $(M+H)^+$: 186 gef.: Molpeak $(M+H)^+$: 186
Retentionszeit HPLC: 7.04 min (Methode A)

6d 2-[5-(5-Brom-pyridin-2-ylethinyl)-indol-1 -yl]-ethanol

Unter $N_2$-Atmosphäre werden zu einer Lösung von 23.0 g (124 mmol) 2-(5-Ethinyl-indol-1-yl)-ethanol und 35 mL (248 mmol) Diisopropylamin in 1150 mL THF 29.4 g (124 mmol) 2,5-Dibrom-pyridin, 241 mg (1.3 mmol) CuI und 888 mg (1.3 mmol) $Pd(PPh_3)_2Cl_2$ gegeben und das Reaktionsgemisch 3.5 h auf 50˚C erwärmt. Man gibt erneut 241 mg CuI und 888 mg $Pd(PPh_3)_2Cl_2$ und 9 g (38 mmol) 2,5-Dibrom-pyridin zu, rührt weitere 2.5 h bei 50˚C, 64 h bei RT und weitere 8 h bei 60˚C. Man engt i.vac. ein, versetzt den Rückstand mit 500 mL 3% $NH_3$-Lösung und 800 mL EtOAc. Man filtriert den entstandenen Niederschlag ab, wäscht diesen mit Wasser und trocknet ihn bei 50˚C. Die beiden Phasen des Filtrates werden getrennt und die organische Phase i.vac. eingeengt. Der Rückstand wird mit 500 mL PE/Diisopropylether (1:1) kräftig durchgerührt und abgesaugt. Beide Produktfraktionen werden anschliessend vereinigt.

Ausbeute: 24.96 g (58.9 % d. Theorie)
$C_{17}H_{13}BrN_2O$ (M= 341,210)
ber.: Molpeak $(M+H)^+$: 340/342 gef.: Molpeak $(M+H)^+$: 340/342
$R_f$-Wert: 0.39 (Kieselgel, Cyc/EtOAc 1:1)

6e 2-{5-[5-(4-Chlor-phenyl)-pyridin-2-ylethinyl]-indol-1 -yl}-ethanol

Unter $N_2$-Atmosphäre werden zu einer Lösung von 19.0 g (55.7 mmol) 2-[5-(5-Brom-pyridin-2-ylethinyl)-indol-1-yl]-ethanol und 11.55 g (72.4 mmol) 4-Chlorphenyl-boronsäure in 320 mL 1,4-Dioxan und 80 mL MeOH 56 mL 2 M $Na_2CO_3$-Lösung und 1.29 g (1.1 mmol) Tetrakis-triphenylphosphan-Palladium gegeben und das Reaktionsgemisch 16 h auf 110˚C erhitzt. Man engt i.vac. ein, versetzt den Rückstand mit 300 mL Wasser und rührt die Suspension kräftig durch. Man filtriert den Niederschlag ab und wäscht diesen mit 200 mL Wasser nach. Der Niederschlag wird dreimal mit jeweils 600 mL PE/DCM (5:1) aufgeschlämmt, abgesaugt und schliesslich an der Luft bis zur Gewichtskonstanz getrocknet.
Ausbeute: 17.11 g (82.4 % d. Theorie)
$C_{23}H_{17}ClN_2O$ (M= 372,858)
ber.: Molpeak $(M+H)^+$: 373/375 gef.: Molpeak $(M+H)^+$: 373/375
$R_f$-Wert: 0.42 (Kieselgel, DCM/MeOH/$NH_3$ 19:1:0.1)

6f Methansulfonsäure-2-{5-[5-(4-chlor-phenyl)-pyridin-2-ylethinyl]-indol-1 -yl}-ethyl ester

Unter Argon-Atmosphäre werden zu einer Lösung von 16.0 g (42.9 mmol) 2-{5-[5-(4-Chlor-phenyl)-pyridin-2-ylethinyl]-indol-1-yl}-ethanol in 480 mL THF und 30 mL Pyridin 7.1 mL (51.5 mmol) Triethylamin gegeben und diese auf 0˚C gekühlt. Anschliessend tropft man eine Lösung von 4 mL (51.5 mmol) Methansulfonsäure-chlorid in 20 mL THF langsam zu, lässt auf RT erwärmen und rührt weitere 2 h bei RT nach. Die Reaktionslösung wird filtriert und i.vac. eingeengt. Der Rückstand wird mit 1 L DCM versetzt, mit 400 mL Wasser gewaschen und die organische Phase über $Na_2SO_4$ getrocknet. Nach Entfernen des Trocken- und Lösungsmittels wird der Rückstand mit 600 mL PE/DCM (5:1) aufgeschlämmt, abgesaugt und schliesslich an der Luft bis zur Gewichtskonstanz getrocknet.
Ausbeute: 17.10 g (88.4 % d. Theorie)
$C_{24}H_{19}ClN_2O_3S$ (M= 450,948)
ber.: Molpeak $(M+H)^+$: 451/453 gef.: Molpeak $(M+H)^+$: 451/453
$R_f$-Wert: 0.9 (Kieselgel, EtOAc/Me0H/$NH_3$ 19:1:0.1)

6g 5-[5-(4-Chlor-phenyl)-pyridin-2-ylethinyl]-1-(2-pyrrolidin-1-yl-ethyl)-1*H*-indol

Zu einer Lösung von 600 mg (1.33 mmol) Methansulfonsäure-2-{5-[5-(4-chlor-phenyl)-pyridin-2-ylethinyl]-indol-1-yl}-ethyl-ester in 12 mL DMF werden 1.1 mL (13.3 mmol) Pyrrolidin gegeben und das Reaktionsgemisch 24 h bei RT gerührt. Man engt i.vac. ein, nimmt den Rückstand in wenig DCM auf und reinigt das Produkt chromatographisch (Kieselgel, Cyc/EtOAc 2:1)
Ausbeute: 301 mg (53.1 % d. Theorie)
$C_{27}H_{24}ClN_3$ (M= 425,965)
ber.: Molpeak $(M+H)^+$: 426/428 gef.: Molpeak $(M+H)^+$: 426/428
$R_f$-Wert: 0.44 (Kieselgel, Cyc/EtOAc 2:1)

Die folgenden Verbindungen werden wie in Beispiel 6g beschrieben hergestellt, wobei jeweils 5-20 eq. des Amins eingesetzt werden und das Reaktionsgemisch 24 h bei RT (Methode A) oder 24 h bei RT und 24 h bei 60˚C (Methode B) oder 7.5 h bei 80˚C (Methode C) oder 48 h bei 80˚C (Methode D) gerührt wird. Man versetzt mit DCM und Wasser, trennt die Phasen und trocknet die organische Phase über $Na_2SO_4$. Nach Entfernen des Trocken- und Lösungsmittels wird der Rückstand chromatographisch an Alox gereinigt.

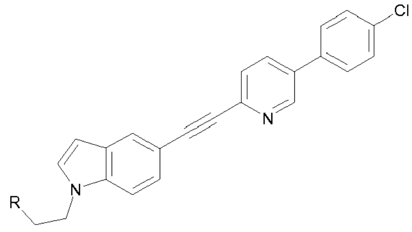

| Beispiel (Methode) | R | Ausbeute (%) | Summenformel | Massen-spektrum | $R_f$-Wert auf Alox (Fliessmittel) oder Retentionszeit HPLC in min (Methode) |
|---|---|---|---|---|---|
| 6.1 (A) | 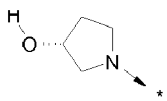 | 33.1 | $C_{27}H_{24}ClN_3O$ | 442/444 $[M+H]^+$ | 0.24 (DCM/ MeOH/$NH_3$ 19: 1:0.1) |
| 6.2 (B) | 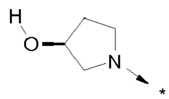 | 35.2 | $C_{27}H_{24}ClN_3O$ | 442/444 $[M+H]^+$ | 0.46 (DCM/ MeOH/$NH_3$ 9:1: 0.1) |
| 6.3 (B) | 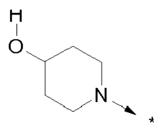 | 34.7 | $C_{28}H_{26}ClN_3O$ | 456/458 $[M+H]^+$ | 0.32 (DCM/ MeOH/$NH_3$ 9:1: 0.1) |
| 6.4 (B) | 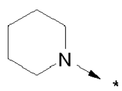 | 22.1 | $C_{28}H_{26}ClN_3$ | 440/442 $[M+H]^+$ | 0.85 (DCM/ MeOH/$NH_3$ 9:1: 0.1) |
| 6.5 (B) | 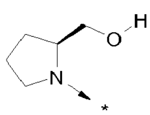 | 26.3 | $C_{28}H_{26}ClN_3O$ | 456/458 $[M+H]^+$ | 0.42 (DCM/ MeOH/$NH_3$ 19: 1:0.1) |
| 6.6 (B) | 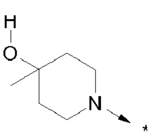 | 21.9 | $C_2sH_{28}ClN_30$ | 470/472 $[M+H]^+$ | 0.21 (DCM/ MeOH/$NH_3$ 19: 1:0.1) |
| 6.7 (B) | 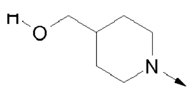 | 27.0 | $C_{29}H_{28}ClN_30$ | 470/472 $[M+H]^+$ | 0.07 (DCM/ MeOH/$NH_3$ 19: 1:0.1) |
| 6.8 (B) | 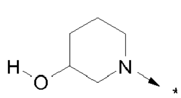 | 21.1 | $C_{28}H_{26}ClN_3O$ | 456/458 $[M+H]^+$ | 0.28 (DCM/ MeOH/$NH_3$ 19: 1:0.1) |
| 6.9 (B) | 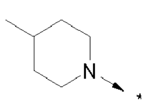 | 17.2 | $C_{29}H_{28}ClN_3$ | 454/456 $[M+H]^+$ | 0.33 (DCM/ MeOH/$NH_3$ 19: 1:0.1) |

(fortgesetzt)

| Beispiel (Methode) | R | Ausbeute (%) | Summenformel | Massen-spektrum | $R_f$-Wert auf Alox (Fliessmittel) oder Retentionszeit HPLC in min (Methode) |
|---|---|---|---|---|---|
| 6.10 (C) | 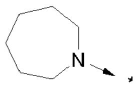 | 55.7 | $C_{29}H_{28}ClN_3$ | 454/456 $[M+H]^+$ | 0.16 (DCM/ MeOH/$NH_3$ 19: 1:0.1) |
| 6.11 (D) | 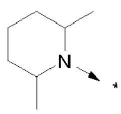 | 11.6 | $C_{30}H_{30}ClN_3$ | 468/470 $[M+H]^+$ | 0.18 (DCM/ MeOH/$NH_3$ 19: 1:0.1) |

Beispiel 6.12

(2-{5-[5-(4-Chlor-phenyl)-pyridin-2-ylethinyl]-indol-1 -yl}-ethyl)-cydopropylmethyl-propyl-amin

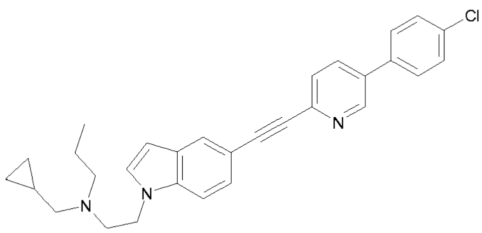

Zu einer Lösung von 100 mg (0.22 mmol) Methansulfonsäure-2-{5-[5-(4-chlor-phenyl)-pyridin-2-ylethinyl]-indol-1-yl}-ethyl-ester in 2 mL DMF werden 63 µL (0.44 mmol) Cyclopropylmethyl-propyl-amin gegeben und das Reaktionsgemisch 16 h bei 60˚C gerührt. Man engt i.vac. ein, nimmt den Rückstand in DCM auf, wäscht die organische Phase mit Wasser und verdünnter $K_2CO_3$-Lösung und trocknet über $Na_2SO_4$. Nach Entfernen des Trocken- und Lösungsmittels wird der Rückstand zweimal chromatographisch (Alox, Cyc/EtOAc 8:2 und Cyc/DCM 1:1) gereinigt.
Ausbeute: 21 mg (20.2 % d. Theorie)
$C_{30}H_{30}ClN_3$ (M= 468,047)
ber.: Molpeak $(M+H)^+$: 468/470 gef.: Molpeak $(M+H)^+$: 468/470
$R_f$-Wert: 0.37 (Alox, Cyc/EtOAc 8:2)

Beispiel 6.13

(2-{5-[5-(4-Chlor-phenyl)-pyridin-2-ylethinyl]-indol-1-yl}-ethyl)-cydopropylmethyl-amin

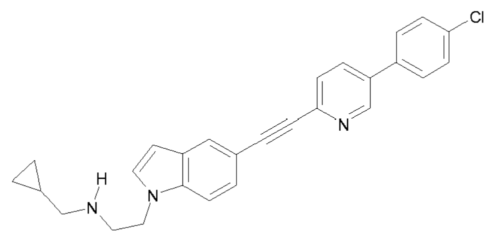

Zu einer Lösung von 2.03 g (4.5 mmol) Methansulfonsäure-2-{5-[5-(4-chlor-phenyl)-pyridin-2-ylethinyl]-indol-1-yl}-ethyl-ester in 20 mL DMF werden 0.46 mL (5.4 mmol) C-Cyclopropyl-methylamin gegeben und das Reaktionsgemisch 4 h bei 60˚C gerührt. Man engt i.vac. ein, verrührt den Rückstand mit DCM, saugt den Niederschlag ab und trocknet diesen an der Luft. Das Produkt fällt als Methansulfonsäure-Salz an.
Ausbeute: 600 mg (25.5 % d. Theorie)
$C_{27}H_{24}ClN_3$*$CH_4O_3S$ (M= 522,07)
ber.: Molpeak $(M+H)^+$: 426/428 gef.: Molpeak $(M+H)^+$: 426/428
Retentionszeit HPLC: 5.23 min (Methode B)

Beispiel 6.14

(2-{5-[5-(4-Chlor-phenyl)-pyridin-2-ylethinyl]-indol-1-yl}-ethyl)-bis-cydopropylmethyl-amin

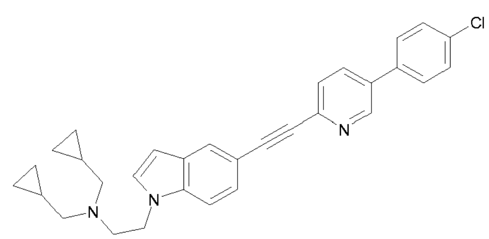

Zu einer Lösung von 100 mg (0.24 mmol) (2-{5-[5-(4-Chlor-phenyl)-pyridin-2-ylethinyl]-indol-1-yl}-ethyl)-cyclopropylmethyl-amin in 20 mL MeOH werden bei RT 36 μL (0.47 mmol) Cyclopropancarbaldehyd gegeben und 15 min später 59 mg (0.94 mmol) $NaBH_4$ und ein Tropfen Eisessig. Man rührt 1 h bei RT, engt i.vac. ein, nimmt den Rückstand in verdünnter $K_2CO_3$-Lösung auf, extrahiert erschöpfend mit EtOAc und trocknet über $Na_2SO_4$. Nach Entfernen des Trocken- und Lösungsmittels wird der Rückstand mit PE verrieben, abgesaugt und getrocknet.
Ausbeute: 105 mg (93.1 % d. Theorie)
$C_{31}H_{30}ClN_3$ (M= 480,058)
ber.: Molpeak $(M+H)^+$: 480/482 gef.: Molpeak $(M+H)^+$: 480/482
Retentionszeit HPLC: 5.53 min (Methode B)

**Beispiel 6.15**

(2-{5-[5-(4-Chlor-phenyl)-pyridin-2-ylethinyl]-indol-1-yl}-ethyl)-cydopropylmethyl-isobutyl-amin

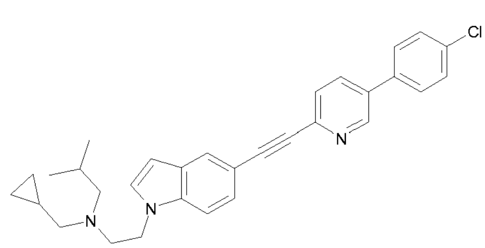

Hergestellt analog Beispiel 6.14, nach der beschriebenen Aufarbeitung wird das Rohprodukt chromatographisch (Alox, Cyc/EtOAc 4:1) gereinigt.
Ausbeute: 35 mg (41.5 % d. Theorie)
$C_{31}H_{32}ClN_3$ (M= 482,074)
ber.: Molpeak $(M+H)^+$: 482/484 gef.: Molpeak $(M+H)^+$: 482/484
$R_f$-Wert: 0.83 (Alox, Cyc/EtOAc 4:1)
Retentionszeit HPLC: 5.7 min (Methode B)

**Beispiel 6.16**

(2-{5-[5-(4-Chlor-phenyl)-pyridin-2-ylethinyl]-indol-1-yl}-ethyl)-cydopropylmethyl-prop-2-inyl-amin

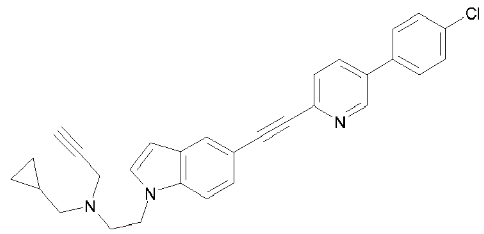

Zu einer Lösung von 70 mg (0.16 mmol) (2-{5-[5-(4-Chlor-phenyl)-pyridin-2-ylethinyl]-indol-1-yl]-ethyl)-cyclopropylmethyl-amin in 2 mL DMF werden bei RT 45 mg (0.33 mmol) $K_2CO_s$ und 13 µL (0.18 mmol) 3-Brom-propin zugegeben und das Reaktionsgemisch 4 h bei RT gerührt. Man engt i.vac. ein, nimmt den Rückstand in Wasser auf, extrahiert erschöpfend mit DCM und trocknet die organische Phase über $Na_2SO_4$. Nach Entfernen des Trocken- und Lösungsmittels wird der Rückstand chromatographisch (Alox, Cyc/EtOAc 4:1) gereinigt.
Ausbeute: 32 mg (42.1 % d. Theorie)
$C_{30}H_{26}ClN_3$ (M= 464,015)
ber.: Molpeak $(M+H)^+$: 464/466 gef.: Molpeak $(M+H)^+$: 464/466
$R_f$-Wert: 0.35 (Alox, Cyc/EtOAc 4:1)
Retentionszeit HPLC: 5.86 min (Methode B)

Mit den in Beispiel 6.14 und 6.16 beschriebenen Verfahren können folgende Verbindungen hergestellt werden:

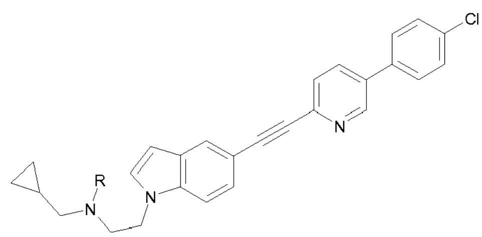

| Beispiel | R | Ausbeute (%) | Summenformel | Massen-spektrum | Retentionszeit HPLC in min (Methode) |
|---|---|---|---|---|---|
| 6.17 | 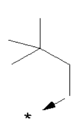 | | | | |

(fortgesetzt)

| Beispiel | R | Ausbeute (%) | Summenformel | Massen-spektrum | Retentionszeit HPLC in min (Methode) |
|---|---|---|---|---|---|
| 6.18 | 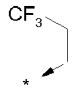 | | | | |
| 6.19 | 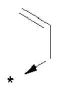 | 30.1 | $C_{30}H_{28}ClN_3$ | 466/468 $[M+H]^+$ | 5.5 (B) |
| 6.20 | 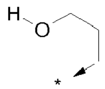 | | | | |
| 6.21 | 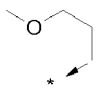 | | | | |

**Beispiel 7**

1 -{3-[5-(4-Chlor-phenyl)-pyridin-2-yl]-prop-2-inyl}-5-pyrrolidin-1-ylmethyl-1*H*-indol

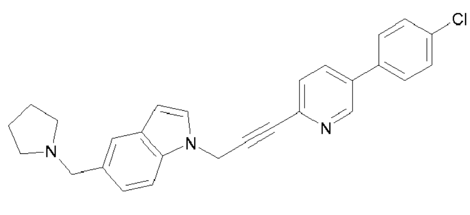

7a 1-Prop-2-inyl-1*H*-indol-5-carbaldehyd

Zu einer auf 0˚C gekühlten Lösung von 2.0 g (13.5 mmol) 1*H*-Indol-5-carbaldehyd in 80 mL THF werden 0.65 g (50% in Mineralöl, 13.5 mmol) NaH portionenweise zugegeben und nach Erwärmen auf RT 15 min nachgerührt. Anschliessend wird eine Lösung von 1.6 mL (80% in Toluol, 15 mmol) Propargylbromid in 20 mL THF langsam zugetropft und das Reaktionsgemisch über Nacht bei RT nachgerührt. Man engt i.vac. ein, versetzt den Rückstand mit Wasser, extrahiert die wässrige Phase erschöpfend mit EtOAc und trocknet die organische Phase über $Na_2SO_4$. Nach Entfernen des Trocken- und Lösungsmittels wird der Rückstand chromatographisch (Kieselgel, Gradient: Cyc/EtOAc 4:1 nach 2: 1) gereinigt.
Ausbeute: 0.65 g (26.4 % d. Theorie)
$C_{12}H_9NO$ (M= 183,212)
ber.: Molpeak $(M+H)^+$: 184 gef.: Molpeak $(M+H)^+$: 184
$R_f$-Wert: 0.34 (Kieselgel, Cyc/EtOAc 3:1)

7b 1-Prop-2-inyl-5-pyrrolidin-1-ylmethyl-1*H*-indol

Eine Lösung von 250 mg (1.37 mmol) 1-Prop-2-inyl-1H-indol-5-carbaldehyd und 200 µL (2.37 mmol) Pyrrolidin in 50 mL THF wird mit Eisessig auf pH 5 eingestellt, mit 550 mg (2.47 mmol) $NaBH(OAc)_3$ versetzt und 24 h bei RT

gerührt. Man gibt 20 mL gesättigte $K_2CO_3$-Lösung zu, extrahiert mit 50 mL EtOAc und trocknet die organische Phase über $Na_2SO_4$. Nach Entfernen des Trocken- und Lösungsmittels wird der Rückstand chromatographisch (Kieselgel, EtOAc/MeOH/$NH_3$ 95:5:0.5) gereinigt.
Ausbeute: 325 mg (100 % d. Theorie)
$C_{16}H_{18}N_2$ (M= 238,335)
ber.: Molpeak $(M+H)^+$: 239 gef.: Molpeak $(M+H)^+$: 239
$R_f$-Wert: 0.38 (Kieselgel, EtOAc/MeOH/$NH_3$ 95:5:0.5)

7c 1-[3-(5-Brom-pyridin-2-yl)-prop-2-inyl]-5-pyrrolidin-1-ylmethyl-1*H*-indol

Unter Argon-Atmosphäre werden zu einer Lösung von 337 mg (1.41 mmol) 1-Prop-2-inyl-5-pyrrolidin-1-ylmethyl-1H-indol und 345 mg (1.41 mmol) 2,5-Dibrom-pyridin in 50 mL THF und 0.4 mL Diisopropylamin 5 mg (0.03 mmol) CuI und 18 mg (0.03 mmol) $Pd(PPh_3)_2Cl_2$ gegeben und das Reaktionsgemisch 17 h bei RT gerührt. Man engt i.vac. ein, nimmt den Rückstand in 30 mL EtOAc auf, wäscht die organische Phase mit 30 mL Wasser und 30 mL gesättigter NaCl-Lösung und trocknet über $Na_2SO_4$. Nach Entfernen des Trocken- und Lösungsmittels wird der Rückstand chromatographisch (Kieselgel, EtOAc/MeOH/$NH_3$ 95:5:0.5) gereinigt.
Ausbeute: 145 mg (26.0 % d. Theorie)
$C_{21}H_{20}BrN_3$ (M= 394,318)
ber.: Molpeak $(M+H)^+$: 394/396 gef.: Molpeak $(M+H)^+$: 394/396
$R_f$-Wert: 0.62 (Kieselgel, EtOAc/MeOH/$NH_3$ 90:10:1)

7d 1-{3-[5-(4-Chlor-phenyl)-pyridin-2-yl]-prop-2-inyl}-5-pyrrolidin-1-ylmethyl-1*H*-indol

Unter Argon-Atmosphäre werden zu einer Lösung von 59 mg (0.15 mmol)1-[3-(5-Brom-pyridin-2-yl)-prop-2-inyl]-5-pyrrolidin-1-ylmethyl-1*H*-indol und 50 mg (0.32 mmol) 4-Chlorphenyl-boronsäure in 5 mL 1,4-Dioxan 0.5 mL 2 M $Na_2CO_3$-Lösung und 10 mg (0.01 mmol) Tetrakis-triphenylphosphan-Palladium gegeben und das Reaktionsgemisch 2.5 h bei 110°C gerührt. Man engt i.vac. ein, versetzt den Rückstand mit 3 mL Wasser, extrahiert mit 5 mL EtOAc, wäscht die organische Phase mit gesättigter NaCl-Lösung und trocknet über $Na_2SO_4$. Nach Entfernen des Trocken- und Lösungsmittels wird der Rückstand via HPLC gereinigt.
Ausbeute: 21.8 mg (34.1 % d. Theorie)
$C_{27}H_{24}ClN_3$ (M= 425,965)
ber.: Molpeak $(M+H)^+$: 426/428 gef.: Molpeak $(M+H)^+$: 426/428
Retentionszeit HPLC: 6.75 min (Methode A).

**Beispiel 8**

5-(4-Chlor-phenyl )-2-[3-(4-pyrrol id in-1-yl methyl-phenoxy)-prop-1-inyl]-pyrid in

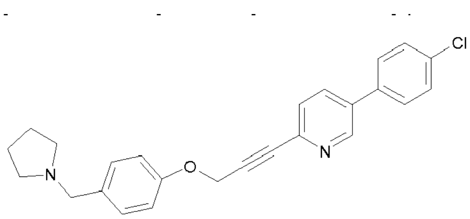

8a 3-[5-(4-Chlor-phenyl)-pyridin-2-yl]-prop-2-in-1-ol

Unter $N_2$-Atmosphäre werden zu einer Lösung von 500 mg (2.36 mmol) 3-(5-Brom-pyridin-2-yl)-prop-2-in-1-ol und 600 mg (3.72 mmol) 4-Chlorphenyl-boronsäure in 10 mL DMF und 2.5 mL Wasser 1 mL (7.22 mmol) Triethylamin, 23 mg (0.1 mmol) $Pd(OAc)_2$ und 57.5 mg (0.19 mmol) Biphenyl-2-yl-di-*tert*-butyl-phosphan gegeben und das Reaktionsgemisch für 8 h bei 60°C gerührt. Dann werden 400 mg (2.48 mmol) 4-Chlorphenyl-boronsäure zugegeben und weitere 19 h bei 60°C gerührt. Man engt i.vac. ein, versetzt den Rückstand mit 10 mL Wasser und 10 mL EtOAc, sättigt die wässrige Phase mit NaCl, trennt die organische Phase ab und trocknet diese über $Na_2SO_4$. Nach Entfernen des Trocken- und Lösungsmittels wird der Rückstand chromatographisch (Kieselgel, Cyc/$EtOAc_3$ 2:1) gereinigt.
Ausbeute: 228 mg (39.6 % d. Theorie)

$C_{14}H_{10}ClNO$(M= 243,695)
ber.: Molpeak $(M+H)^+$: 244/246 gef.: Molpeak $(M+H)^+$: 244/246
$R_f$-Wert: 0.23 (Kieselgel, Cyc/EtOAc 1:1)

8b 5-(4-Chlor-phenyl)-2-[3-(4-pyrrolidin-1 -ylmethyl-phenoxy)-prop-1-inyl]-pyridin

Zu einer Lösung von 100 mg (0.41 mmol) 3-[5-(4-Chlor-phenyl)-pyridin-2-yl]-prop-2-in-1-ol und 88 mg (0.5 mmol) 4-Pyrrolidin-1-ylmethyl-phenol in 4 mL THF werden 131 mg (0.5 mmol) Triphenylphosphan gegeben. Anschliessend wird 0.1 mL (0.5 mmol) Azo-dicarbonsäure-diisopropylester langsam zugetropft und das Reaktionsgemisch für 3 h bei RT gerührt. Man engt i.vac. ein, nimmt den Rückstand in 1 mL DMF auf und reinigt via HPLC. Das erhaltene Produkt, das noch Triphenylphosphanoxid enthält, wird erneut chromatographisch (Kieselgel, EtOAc nach EtOAc/Me0H/$NH_3$ 9:1:0.1) gereinigt.
Ausbeute: 6.5 mg (3.9 % d. Theorie)
$C_{25}H_{23}ClN_2O$(M= 402,928)
ber.: Molpeak $(M+H)^+$: 403/405 gef.: Molpeak $(M+H)^+$: 403/405
$R_f$-Wert: 0.72 (Kieselgel, EtOAc/MeOH/$NH_3$ 9:1:0.1)

**Beispiel 9**

5-(4-Chlor-phenyl )-2-[3-methyl-3-(4-pyrrol id in-1-yl methyl-phenoxy)-but-1-inyl]-pyrid in

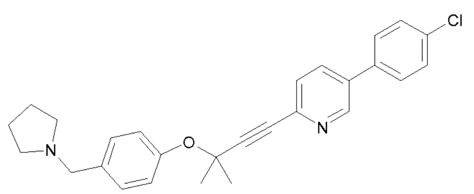

9a 4-(5-Brom-pyridin-2-yl)-2-methyl-but-3-in-2-ol

Unter Argon-Atmosphäre werden zu einer Lösung von 0.99 mL (10.0 mmol) 2-Methyl-but-3-in-2-ol und 2.44 g (10.0 mmol) 2,5-Dibrom-pyridin in 50 mL THF und 2.8 mL (20 mmol) Diisopropylamin 38 mg (0.2 mmol) CuI und 143 mg (0.2 mmol) $Pd(PPh_3)_2Cl_2$ gegeben und das Reaktionsgemisch 15 min bei RT gerührt. Das Reaktionsgemisch wird mit Wasser versetzt, erschöpfend mit EtOAc extrahiert und die organische Phase über $Na_2SO_4$ getrocknet. Nach Entfernen des Trocken- und Lösungsmittels wird der Rückstand chromatographisch (Kieselgel, Cyc/EtOAc 2:1) gereinigt.
Ausbeute: 2.0 g (83.3 % d. Theorie)
$C_{10}H_{10}BrNO$ (M= 240,101 )
ber.: Molpeak $(M+H)^+$: 240/242 gef.: Molpeak $(M+H)^+$: 240/242
$R_f$-Wert: 0.29 (Kieselgel, Cyc/EtOAc 2:1)

9b 4-[5-(4-Chlor-phenyl)-pyridin-2-yl]-2-methyl-but-3-in-2-ol

Unter Argon-Atmosphäre werden zu einer Lösung von 720 mg (3.0 mmol) 4-(5-Brom-pyridin-2-yl)-2-methylbut-3-in-2-ol und 593 mg (3.6 mmol) 4-Chlorphenyl-boronsäure in 60 mL 1,4-Dioxan 3 mL 2 M $Na_2C0_3$-Lösung und 173 mg (0.15 mmol) Tetrakis-triphenylphosphan-Palladium gegeben und das Reaktionsgemisch 18 h bei 85˚C gerührt. Man engt i.vac. ein, versetzt den Rückstand mit Wasser, extrahiert erschöpfend mit EtOAc, wäscht die organische Phase mit Wasser und trocknet über $Na_2SO_4$. Nach Entfernen des Trocken- und Lösungsmittels wird der Rückstand chromatographisch (Kieselgel, PE/EtOAc 1:1) gereinigt.
Ausbeute: 420 mg (51.5 % d. Theorie)
$C_{16}H_{14}ClNO$ (M= 271,749)
ber.: Molpeak $(M+H)^+$: 272/274 gef.: Molpeak $(M+H)^+$: 272/274
$R_f$-Wert: 0.42 (Kieselgel, PE/EtOAc 1:1)

9c 5-(4-Chlor-phenyl)-2-[3-methyl-3-(4-pyrrolidin-1-ylmethyl-phenoxy)-but-1-inyl]-pyridin

Zu einer Lösung von 136 mg (0.5 mmol) 4-[5-(4-Chlor-phenyl)-pyridin-2-yl]-2-methyl-but-3-in-2-ol und 88 mg (0.5 mmol) 4-Pyrrolidin-1-ylmethyl-phenol in 20 ml THF werden 131 mg (0.5 mmol) Triphenylphosphan gegeben. Anschliessend wird 0.1 mL (0.5 mmol) Azo-dicarbonsäure-diisopropylester langsam zugetropft und das Reaktionsgemisch 24 h bei RT gerührt. Man engt i.vac. ein, nimmt den Rückstand in Wasser auf, extrahiert erschöpfend mit EtOAc, wäscht die organische Phase mit gesättigter NaCl-Lösung und trocknet über $Na_2SO_4$. Nach Entfernen des Trocken- und Lösungsmittels wird der Rückstand via HPLC gereinigt.
Ausbeute: 3 mg (1.4 % d. Theorie)
$C_{27}H_{27}ClN_2O$ (M= 430,982)
ber.: Molpeak $(M+H)^+$: 431/433 gef.: Molpeak $(M+H)^+$: 431/433
Retentionszeit HPLC: 7.78 min (Methode A)

**Beispiel 10**

5-(4-Chlor-phenyl)-2-[3-(4-pyrrolidin-1-ylmethyl-phenyl)-prop-2-inyloxy]-pyridin

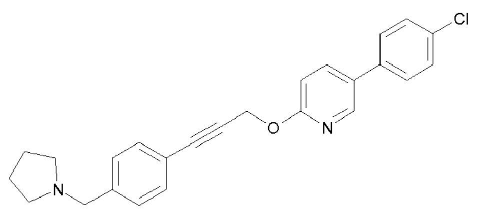

10a 5-(4-Chlor-phenyl)-pyridin-2-ol

Unter $N_2$-Atmosphäre werden zu einer Lösung von 8.0 g (21.7 mmol) 5-lod-pyridin-2-ol und 3.81 g (23.9 mmol) 4-Chlorphenyl-boronsäure in 120 mL 1,4-Dioxan und 30 mL trockenem MeOH 21.7 mL 2 M $Na_2CO_3$-Lösung und 250 mg (0.22 mmol) Tetrakis-triphenylphosphan-Palladium gegeben und das Reaktionsgemisch 19 h bei 110°C gerührt. Man engt i.vac. ein, versetzt den Rückstand mit Wasser, filtriert den Niederschlag ab, wäscht diesen mit Wasser nach und trocknet ihn bei 40°C im Umlufttrockenschrank bis zur Gewichtskonstanz.
Ausbeute: 3.8 g (85.1 % d. Theorie)
$C_{11}H_8ClN0$ (M= 205,646)
ber.: Molpeak $(M+H)^+$: 206/208 gef.: Molpeak $(M+H)^+$: 206/208
$R_f$-Wert: 0.56 (Kieselgel, EtOAc/MeOH/$NH_3$ 9:1:0.1)

10b 5-(4-Chlor-phenyl)-2-prop-2-inyloxy-pyridin

Zu einer Suspension von 3.8 g (18.5 mmol) 5-(4-Chlor-phenyl)-pyridin-2-ol und 5.1 g (37 mmol) $K_2CO_3$ in 50 mL DMF werden 2 mL (80% in Toluol, 18.5 mmol) 3-Brom-propin gegeben und das Reaktionsgemisch 64 h bei RT gerührt. Man engt i.vac. ein, versetzt den Rückstand mit 80 mL Wasser, extrahiert mit 150 mL EtOAc und trocknet die organische Phase über $Na_2SO_4$. Nach Entfernen des Trocken- und Lösungsmittels wird der Rückstand chromatographisch (Kieselgel, Cyc/EtOAc 2:1) gereinigt.
Ausbeute: 216 mg (4.8 % d. Theorie)
$C_{14}H_{10}ClNO$ (M= 243,695)
ber.: Molpeak $(M+H)^+$: 244/246 gef.: Molpeak $(M+H)^+$: 244/246
$R_f$-Wert: 0.16 (Kieselgel, Cyc/EtOAc 2:1)

10c 5-(4-Chlor-phenyl)-2-[3-(4-pyrrolidin-1 -ylmethyl-phenyl)-prop-2-inyloxy]-pyridin

Unter $N_2$-Atmosphäre werden zu einer Lösung von 110 mg (0.45 mmol) 5-(4-Chlor-phenyl)-2-prop-2-inyloxy-pyridin und 129 mg (0.45 mmol) 1-(4-lod-benzyl)-pyrrolidin in 9 mL THF 221 mg (0.68 mmol) $Cs_2CO_3$, 4 mg (0.02 mmol) CuI und 23 mg (0.02 mmol) Tetrakis-triphenylphosphan-Palladium gegeben und das Reaktionsgemisch 4.5 h bei RT

gerührt. Man engt i.vac. ein, versetzt den Rückstand mit 20 mL 3% $NH_3$-Lösung und 40 mL EtOAc, trennt die organische Phase ab und trocknet diese über $Na_2SO_4$. Nach Entfernen des Trocken- und Lösungsmittels wird der Rückstand zuerst via HPLC und dann chromatographisch (Kieselgel, DCM/MeOH/$NH_3$ 19:1:0.1) gereinigt.
Ausbeute: 28 mg (15.5 % d. Theorie)
$C_{25}H_{23}ClN_2O$ (M= 402,928)
ber.: Molpeak $(M+H)^+$: 403/405 gef.: Molpeak $(M+H)^+$: 403/405
$R_f$-Wert: 0.33 (Kieselgel, DCM/MeOH/$NH_3$ 19:1:0.1)
Retentionszeit HPLC: 6.43 min (Methode A)

**Beispiel 11**

1-(2-{4-[4-(4-Chlor-phenyl)-thiophen-2-ylethinyl]-phenoxy}-ethyl)-pyrrolidin

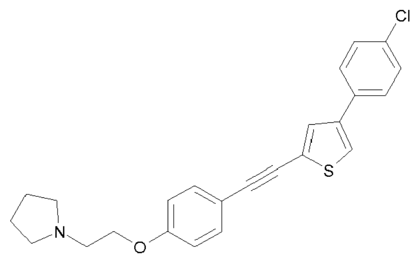

11a (4-Brom-thiophen-2-ylethinyl)-trimethyl-silan

Unter Argon-Atmosphäre werden zu einer Lösung von 10.0 g (38.85 mmol) 2,4-Dibromthiophen in 300 mL THF 0.37 g (1.94 mmol) CuI, 2.24 g (1.94 mmol) Tetrakis-triphenylphosphan-Palladium und 16.2 mL Triethylamin gegeben, das Reaktionsgemisch auf -78°C gekühlt und dann bei dieser Temperatur eine Lösung von 5.6 mL (38.85 mmol) ) Ethinyl-trimethyl-silan in 250 mL THF langsam zugetropft. Nach beendeter Zugabe lässt man langsam auf RT erwärmen und rührt über Nacht nach. Man engt i.vac. ein, versetzt den Rückstand mit Wasser, extrahiert erschöpfend mit DCM und trocknet die vereinigten organischen Phasen über $MgSO_4$. Nach Entfernen des Trocken- und Lösungsmittels wird der Rückstand mit PE verrieben, von unlöslichen Bestandteilen filtriert und das Lösungsmittel eingeengt. Der Rückstand wird chromatographisch (Kieselgel, PE) gereinigt.
Ausbeute: 9.5 g (56.6 % d. Theorie)
$C_9H_{11}$ BrSSi (M= 259,242)
$R_f$-Wert: 0.77 (Kieselgel, PE)

11 b 4-Brom-2-ethinyl-thiophen

Zu einer auf 0°C gekühlten Lösung von 6.3 g (14.58 mmol) (4-Brom-thiophen-2-ylethinyl)-trimethyl-silan in 60 mL THF werden 4.6 g (14.58 mmol) TBAF gegeben. Man entfernt das Kältebad und rührt 30 min nach. Man versetzt das Reaktionsgemisch mit EtOAc, wäscht mit Wasser und trocknet die organische Phase über $MgSO_4$. Nach Entfernen des Trocken- und Lösungsmittels wird der Rückstand chromatographisch (Kieselgel, PE) gereinigt.
Ausbeute: 1.9 g (69.7 % d. Theorie)
$C_6H_3BrS$ (M= 187,059)
ber.: Molpeak $(M+H)^+$: 186/188 gef.: Molpeak $(M+H)^+$: 186/188

11c 1-{2-[4-(4-Brom-thiophen-2-ylethinyl)-phenoxy]-ethyl}-pyrrolidin

Unter Argon-Atmosphäre werden zu einer Lösung von 293 mg (1.56 mmol) 4-Brom-2-ethinyl-thiophen und 620 mg (1.56 mmol) 1-[2-(4-Iod-phenoxy)-ethyl]-pyrrolidin in 10 mL THF 0.3 mL (3.13 mmol) Piperidin, 14.9 mg (0.08 mmol) CuI und 90.3 mg (0.08 mmol) Tetrakis-triphenylphosphan-Palladium gegeben und das Reaktionsgemisch über Nacht bei RT gerührt. Zur Vervollständigung der Reaktion werden erneut 150 mg (0.8 mmol) 4-Brom-2-ethinyl-thiophen zugegeben und weitere 24 h bei RT gerührt. Man engt i.vac. ein, verreibt den Rückstand mit EtOAc und filtriert die unlöslichen Bestandteile ab. Nach Entfernen des Lösungsmittels wird der Rückstand chromatographisch (Kieselgel, EtOAc/MeOH/$NH_3$ 8:2:0.2) gereinigt.

Ausbeute: 300 mg (51.0 % d. Theorie)
$C_{18}H_{18}BrNOS$ (M= 376,318)
ber.: Molpeak $(M+H)^+$: 376/378 gef.: Molpeak $(M+H)^+$: 376/378
$R_f$-Wert: 0.52 (Kieselgel, EtOAc/MeOH/$NH_3$ 9:1:0.1)

11d 1-(2-{4-[4-(4-Chlor-phenyl)-thiophen-2-ylethinyl]-phenoxyl-ethyl)-pyrrolidin

Unter $N_2$-Atmosphäre werden zu einer Lösung von 310 mg (0.82 mmol) 1-{2-[4-(4-Brom-thiophen-2-ylethinyl)-phenoxy]-ethyl}-pyrrolidin und 129 mg (0.82 mmol) 4-Chlorphenyl-boronsäure in 10 mL 1,4-Dioxan 5 mL 2 M $Na_2CO_3$-Lösung und 47 mg (0.41 mmol) Tetrakis-triphenylphosphan-Palladium gegeben und das Reaktionsgemisch 1 h unter Rückfluss erhitzt. Die heisse Lösung wird über einen Glasfaserfilter filtriert, das Filtrat mit EtOAc extrahiert, die organische Phase mit Wasser gewaschen und über $MgSO_4$ getrocknet. Nach Entfernen des Trocken- und Lösungsmittels wird der Rückstand chromatographisch (Kieselgel, EtOAc/MeOH/$NH_3$ 8:2:0.2) gereinigt.
Ausbeute: 23 mg (6.8 % d. Theorie)
$C_{24}H_{22}ClNOS$ (M= 407,966)
ber.: Molpeak $(M+H)^+$: 408/410 gef.: Molpeak $(M+H)^+$: 408/410
Retentionszeit HPLC: 5.35 min (Methode B)

**Beispiel 12**

2-(4-Chlor-phenyl)-5-[4-(2-pyrrolidin-1-yl-ethoxy)-phenylethinyl]-pyrazin

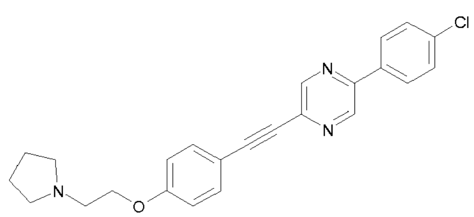

12a 5-(4-Chlor-phenyl)-pyrazin-2-ylamin

Unter Argon-Atmosphäre werden zu einer Lösung von 8.7 g (50.0 mmol) 5-Brom-pyrazin-2-ylamin und 8.0 g (50.0 mmol) 4-Chlorphenyl-boronsäure in 150 mL 1,4-Dioxan und 50 mL MeOH 50 mL 2 M $Na_2CO_3$-Lösung und 1.2 g (1.0 mmol) Tetrakis-triphenylphosphan-Palladium gegeben und das Reaktionsgemisch 2.5 h auf 110˚C erhitzt. Man engt i.vac. ein, versetzt den Rückstand mit Wasser, extrahiert erschöpfend mit EtOAc und trocknet die organische Phase über $Na_2SO_4$. Nach Entfernen des Trocken- und Lösungsmittels wird der Rückstand chromatographisch (Kieselgel, Gradient: DCM nach DCM/MeOH 20:1) gereinigt.
Ausbeute: 8.3 g (80.7 % d. Theorie)
$C_{10}H_8ClN_3$ (M= 205,648)
ber.: Molpeak $(M+H)^+$: 206/208 gef.: Molpeak $(M+H)^+$: 206/208
Retentionszeit HPLC: 7.15 min (Methode A)

12b 2-(4-Chlor-phenyl)-5-iod-pyrazin

Unter Lichtausschluss werden zu einer Lösung von 4.8 g (23.3 mmol) 5-(4-Chlor-phenyl)-pyrazin-2-ylamin in 100 mL $CCl_4$ und 50 mL DCM 4.9 mL (40.0 mmol) *tert*-Butylnitrit und 7.6 g (30 mmol) Iod gegeben und das Reaktionsgemisch über Nacht bei RT gerührt. Man versetzt mit 100 mL Wasser und 50 mL 10% $Na_2S_2O_3$-Lösung, trennt die organische Phase ab, wäscht diese nochmals mit 50 mL 10% $Na_2S_2O_3$-Lösung und zweimal mit jeweils 50 mL Wasser und trocknet über $MgSO_4$. Man filtriert über Aktivkohle, engt i.vac. ein und reinigt den Rückstand chromatographisch (Kieselgel, Gradient: PE nach PE/EtOAc 8:2).
Ausbeute: 3.4 g (46.0 % d. Theorie)
$C_{10}H_6ClIN_2$ (M= 316,530)
ber.: Molpeak $(M+H)^+$: 317/319 gef.: Molpeak $(M+H)^+$: 317/319
$R_f$-Wert: 0.55 (Kieselgel, PE/EtOAc 9:1)

12c2-(4-Chlor-phenyl)-5-[4-(2-pyrrolidin-1-yl-ethoxy)-phenylethinyl]-pyrazin

Unter $N_2$-Atmosphäre werden zu einer Lösung von 316 mg (1.0 mmol) 2-(4-Chlor-phenyl)-5-iod-pyrazin und 215 mg (1.0 mmol) 1-[2-(4-Ethinyl-phenoxy)-ethyl]-pyrrolidin in 50 mL THF und 0.4 mL (3 mmol) Triethylamin 19 mg (0.1 mmol) CuI, 82 mg (0.1 mmol) [1,1'-Bis-(diphenylphosphin)-ferrocen]-palladium(II)-chlorid gegeben und das Reaktionsgemisch über Nacht bei RT gerührt. Zur Vervollständigung der Reaktion werden erneut 100 mg (0.32 mmol) 2-(4-Chlor-phenyl)-5-iod-pyrazin zugegeben und erneut über Nacht gerührt. Man engt i.vac. ein, versetzt den Rückstand mit 10% $Na_2CO_3$-Lösung, extrahiert erschöpfend mit DCM, wäscht die vereinigten organischen Phasen dreimal mit Wasser und trocknet über $MgSO_4$. Man filtriert über Aktivkohle ab, engt i.vac. ein und reinigt den Rückstand chromatographisch (Kieselgel, Gradient: EtOAc nach EtOAc/MeOH/$NH_3$ 9:9:1).
Ausbeute: 170 mg (42.1 % d. Theorie)
$C_{24}H_{22}ClN_3O$ (M= 403,915)
ber.: Molpeak $(M+H)^+$: 404/406 gef.: Molpeak $(M+H)^+$: 404/406
$R_f$-Wert: 0.58 (Kieselgel, DCM/MeOH/$NH_3$ 9:1:0.1)

**Beispiel 13**

2-(4-Chlor-phenyl)-5-[4-(2-pyrrolidin-1-yl-ethoxy)-phenylethinyl]-pyridin

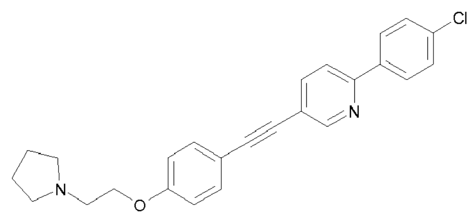

13a 5-Brom-2-(4-chlor-phenyl)-pyridin

Unter $N_2$-Atmosphäre werden zu einer Lösung von 3.00 g (10.6 mmol) 5-Brom-2-iodpyridin und 3.37 g (21.1 mmol) 4-Chlorphenyl-boronsäure in 60 mL 1,4-Dioxan und 15 mL MeOH 11 mL 2 M $Na_2CO_3$-Lösung und 240 mg (0.21 mmol) Tetrakis-triphenylphosphan-Palladium gegeben und das Reaktionsgemisch 3 h auf 110°C erhitzt. Man engt i.vac. ein, versetzt den Rückstand mit 50 mL Wasser,1 0 mL 3% $NH_3$-Lösung und 150 mL EtOAc, trennt die organische Phase ab und trocknet diese über $Na_2SO_4$. Nach Entfernen des Trocken- und Lösungsmittels wird der Rückstand chromatographisch (Kieselgel, Cyc) gereinigt.
Ausbeute: 1.52 g (53.6 % d. Theorie)
$C_{11}H_7BrClN$ (M= 268,542)
ber.: Molpeak $(M+H)^+$: 268/270/272 gef.: Molpeak $(M+H)^+$: 268/270/272
$R_f$-Wert: 0.1 (Kieselgel, Cyc)

13b 2-(4-Chlor-phenyl)-5-[4-(2-pyrrolidin-1-yl-ethoxy)-phenylethinyl]-pyridin

Unter $N_2$-Atmosphäre werden zu einer Lösung von 403 mg (1.5 mmol) 5-Brom-2-(4-chlor-phenyl)-pyridin und 322 mg (1.5 mmol) 1-[2-(4-Ethinyl-phenoxy)-ethyl]-pyrrolidin in 10 mL THF 773 mg (2.25 mmol) $CS_2CO_3$, 14 mg (0.08 mmol) CuI und 87 mg (0.08 mmol) Tetrakis-triphenylphosphan-Palladium gegeben und das Reaktionsgemisch 4 h bei RT gerührt. Anschliessend wird 16 h auf 60°C erwärmt. Man engt i.vac. ein, versetzt den Rückstand mit 30 mL Wasser, 5 mL 3% $NH_3$-Lösung und 60 mL EtOAc, trennt die organische Phase ab und trocknet diese über $Na_2SO_4$. Nach Entfernen des Trocken- und Lösungsmittels wird der Rückstand zuerst chromatographisch (Kieselgel, EtOAc/MeOH/$NH_3$ 19:1:0.1) und dann via HPLC gereinigt.
Ausbeute: 9 mg (2.2 % d. Theorie)
$C_{25}H_{23}C1N_2O$ (M= 402,928)
ber.: Molpeak $(M+H)^+$: 403/405 gef.: Molpeak $(M+H)^+$: 403/405
Retentionszeit HPLC: 8.11 min (Methode A)

**Beispiel 14**

5-[5-(4-Chlor-phenyl)-pyridin-2-ylethinyl]-3-methyl-1-(2-pyrrolidin-1-yl-ethyl)-1*H*-indol-2-carbonsäureethylester

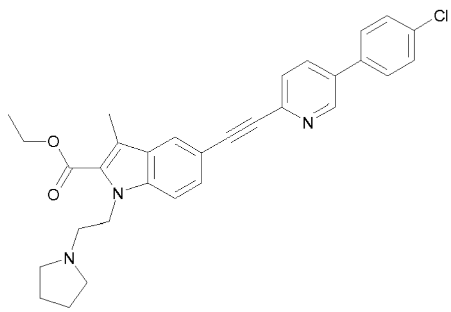

14a 3-Methyl-1-(2-pyrrolidin-1-yl-ethyl)-5-trimethylsilanylethinyl-1H-indol-2-carbonsäureethylester

Unter Argonatmosphäre werden zu einer Lösung von 2.82 g (10 mmol) 5-Brom-3-methyl-1*H*-indol-2-carbonsäureethylester und 1.52 mL (11 mmol) Ethinyl-trimethyl-silan in 3 mL (30 mmol) Piperidin und 30 mL THF 577 mg (0.5 mmol) Tetrakis-triphenylphosphan-Palladium und 95 mg (0.5 mmol) CuI gegeben und das Reaktionsgemisch 14 h bei 60˚C gerührt. Man verdünnt mit Wasser, extrahiert erschöpfend mit EtOAc, wäscht die vereinigten organischen Phasen mit gesättigter NaCI-Lösung und trocknet über $Na_2SO_4$. Nach Entfernen des Trocken- und Lösungsmittels wird der Rückstand chromatographisch (Kieselgel, Cyc/EtOAc 9:1) gereinigt.
Ausbeute: 1.3 g (43.4 % d. Theorie)
$C_{17}H_{21}NO_2Si$ (M= 299,448)
ber.: Molpeak $(M+H)^+$: 300 gef.: Molpeak $(M+H)^+$: 300
$R_f$-Wert: 0.61 (Kieselgel, Cyc/EtOAc 7:3)

14b 5-Ethinyl-3-methyl-1-(2-pyrrolidin-1-yl-ethyl)-1*H*-indol-2-carbonsäureethylester

Unter Argonatmosphäre werden zu einer Suspension von 900 mg (3.0 mmol) 3-Methyl-1-(2-pyrrolidin-1-yl-ethyl)-5-trimethylsilanylethinyl-1*H*-indol-2-carbonsäure-ethylester und 457 mg (3.3 mmol) $K_2CO_3$ in 10 mL DMF 562 mg (3.3 mmol) 1-(2-Chlor-ethyl)-pyrrolidin (eingesetzt als Hydrochlorid) gegeben und das Reaktionsgemisch 42 h bei 60˚C gerührt. Man verdünnt mit Wasser, extrahiert erschöpfend mit EtOAc, wäscht die vereinigten organischen Phasen mit Wasser und trocknet über $Na_2SO_4$. Nach Entfernen des Trocken- und Lösungsmittels wird der Rückstand chromatographisch (Kieselgel, EtOAc/MeOH/$NH_3$ 90:10:1) gereinigt.
Ausbeute: 250 mg (25.6 % d. Theorie)
$C_{20}H_{24}N_2O_2$ (M= 324,426)
ber.: Molpeak $(M+H)^+$: 325 gef.: Molpeak $(M+H)^+$: 325
Retentionszeit HPLC: 4.59 min (Methode B)

14c 5-[5-(4-Chlor-phenyl)-pyridin-2-ylethinyl]-3-methyl-1-(2-pyrrolidin-1-yl-ethyl)-1*H*-indol-2-carbonsäureethylester

Unter Argonatmosphäre werden zu einer Lösung von 250 mg (0.77 mmol) 5-Ethinyl-3-methyl-1-(2-pyrrolidin-1-yl-ethyl)-1*H*-indol-2-carbonsäureethylester und 243 mg (0.77 mmol) 5-(4-Chlor-phenyl)-2-iod-pyridin in 1.52 mL (15.4 mmol) Piperidin und 25 mL THF 11 mg (0.02 mmol) $Pd(PPh_3)_2Cl_2$ und 3 mg (0.02 mmol) CuI gegeben und das Reaktionsgemisch 4 h bei RT gerührt. Man verdünnt mit Wasser, extrahiert erschöpfend mit EtOAc, wäscht die vereinigten organischen Phasen mit Wasser und gesättigter NaCI-Lösung und trocknet über $Na_2SO_4$. Nach Entfernen des Trocken- und Lösungsmittels wird der Rückstand zuerst chromatographisch (Kieselgel, Cyc/EtOAc 2:1) und dann via HPLC gereinigt.
Ausbeute: 7 mg (1.8 % d. Theorie)
$C_{31}H_{30}ClN_3O_2$ (M= 512,057)
ber.: Molpeak $(M+H)^+$: 512/514 gef.: Molpeak $(M+H)^+$: 512/514
$R_f$-Wert: 0.67 (Alox, Cyc/EtOAc 2:1)

Retentionszeit HPLC: 5.96 min (Methode B)

**Beispiel 15**

*N*-{5-[5-(4-Chlor-phenyl)-pyridin-2-ylethinyl]-3-methyl-pyridin-2-yl}-2-pyrrolidin-1-yl-acetamid

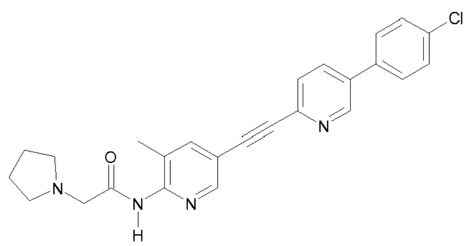

15a *N*-(5-Brom-3-methyl-pyridin-2-yl)-2-chlor-acetamid

Zu einer auf 0˚C gekühlten Lösung von 3.74 g (20 mmol) 2-Amino-5-brom-3-methylpyridin in 50 mL DCM werden 1.75 mL (22 mmol) Chloracetylchlorid gegeben und dann langsam 6.1 mL (44 mmol) Triethylamin zugetropft. Nach beendeter Zugabe wird das Eisbad entfernt und das Reaktionsgemisch 4 h bei RT gerührt. Man giesst auf Wasser, extrahiert erschöpfend mit DCM, wäscht die vereinigten organischen Phasen mit Wasser und gesättigter NaCl-Lösung und trocknet über $Na_2SO_4$. Nach Entfernen des Trocken- und Lösungsmittels wird der Rückstand chromatographisch (Kieselgel, Cyc/EtOAc 2:1) gereinigt.
Ausbeute: 2.7 g (51.2 % d. Theorie)
$C_8H_8BrClN_2O$ (M= 263,523)
ber.: Molpeak $(M+H)^+$: 263/265/267 gef.: Molpeak $(M+H)^+$: 263/265/267
$R_f$-Wert: 0.48 (Kieselgel, Cyc/EtOAc 1:1)

15b *N*-(5-Brom-3-methyl-pyridin-2-yl)-2-pyrrolidin-1-yl-acetamid

Zu einer Suspension von 2.37 g (9.0 mmol) N-(5-Brom-3-methyl-pyridin-2-yl)-2-chlor-acetamid und 2.49 g (18 mmol) $K_2CO_3$ in 22.5 mL DMF werden 0.81 mL (9.9 mmol) Pyrrolidin gegeben und das Reaktionsgemisch 20 h bei RT gerührt. Man verdünnt mit Wasser, extrahiert erschöpfend mit EtOAc, wäscht die vereinigten organischen Phasen mit Wasser und trocknet über $Na_2SO_4$. Nach Entfernen des Trocken- und Lösungsmittels wird der Rückstand in wenig Diisopropylether aufgenommen, auf 0˚C gekühlt, die ausgefallenen Kristalle abgesaugt und an der Luft getrocknet.
Ausbeute: 1.4 g (52.2 % d. Theorie)
$C_{12}H_{16}BrN_3O$ (M= 298,185)
ber.: Molpeak $(M+H)^+$: 298/300 gef.: Molpeak $(M+H)^+$: 298/300
$R_f$-Wert: 0.48 (Kieselgel, EtOAc/MeOH/$NH_3$ 90:10:1)

15c *N*-(3-Methyl-5-trimethylsilanylethinyl-pyridin-2-yl)-2-pyrrolidin-1-yl-acetamid

Unter Argonatmosphäre werden zu einer Lösung von 447 mg (1.5 mmol) *N*-(5-Brom-3-methyl-pyridin-2-yl)-2-pyrrolidin-1-yl-acetamid und 0.23 mL (1.65 mmol) Ethinyl-trimethyl-silan in 0.45 mL (4.5 mmol) Piperidin und 10 mL THF 35 mg (0.03 mmol) Tetrakis-triphenylphosphan-Palladium und 5.7 mg (0.03 mmol) CuI gegeben und das Reaktionsgemisch 14 h bei RT gerührt. Zur Vervollständigung der Reaktion werden erneut 35 mg Tetrakis-triphenylphosphan-Palladium zugegeben und das Reaktionsgemisch 4 h auf 50˚C erwärmt. Man verdünnt mit Wasser, extrahiert erschöpfend mit EtOAc, wäscht die vereinigten organischen Phasen mit gesättigter NaCl-Lösung und trocknet über $Na_2SO_4$. Nach Entfernen des Trocken- und Lösungsmittels wird der Rückstand chromatographisch (Kieselgel, EtOAc/MeOH/$NH_3$ 90:10:1) gereinigt.
Ausbeute: 210 mg (44.4 % d. Theorie)
$C_{17}H_{25}N_3OSi$ (M= 315,494)
ber.: Molpeak $(M+H)^+$: 316 gef.: Molpeak $(M+H)^+$: 316
$R_f$-Wert: 0.65 (Kieselgel, EtOAc/MeOH/$NH_3$ 90:10:1)

15d N-(5-Ethinyl-3-methyl-pyridin-2-yl)-2-pyrrolidin-1-yl-acetamid

Unter Argon-Atmosphäre werden zu einer Lösung von 150 mg (0.48 mmol) N-(3-Methyl-5-trimethylsilanylethinyl-pyridin-2-yl)-2-pyrrolidin-1-yl-acetamid in 10 mL THF 132 mg (0.48 mmol) TBAF gegeben und das Reaktionsgemisch über Nacht bei RT gerührt. Man engt i.vac. ein, nimmt den Rückstand in EtOAc auf, wäscht die organische Phase mit Wasser und gesättigter NaCl-Lösung und trocknet über $Na_2SO_4$. Nach Entfernen des Trocken- und Lösungsmittels erhält man das gewünschte Produkt.
Ausbeute: 110 mg (95.2 % d. Theorie)
$C_{14}H_{17}N_3O$ (M= 243,311)
ber.: Molpeak $(M+H)^+$: 244 gef.: Molpeak $(M+H)^+$: 244
$R_f$-Wert: 0.48 (Kieselgel, EtOAc/MeOH/$NH_3$ 90:10:1)

15e *N*-{5-[5-(4-Chlor-phenyl)-pyridin-2-ylethinyl]-3-methyl-pyridin-2-yl}-2-pyrrolidin-1-yl-acetamid

Unter Argonatmosphäre werden zu einer Lösung von 73 mg (0.3 mmol) N-(5-Ethinyl-3-methyl-pyridin-2-yl)-2-pyrrolidin-1-yl-acetamid und 95 mg (0.3 mmol) 5-(4-Chlor-phenyl)-2-iod-pyridin in 59 μL (0.6 mmol) Piperidin und 10 mL THF 4 mg (0.01 mmol) $Pd(PPh_3)_2Cl_2$ und 1 mg (0.01 mmol) CuI gegeben und das Reaktionsgemisch 30 min bei RT gerührt. Man engt i.vac.ein, nimmt den Rückstand in EtOAc auf, wäscht die organische Phase mit Wasser und gesättigter NaCl-Lösung und trocknet über $Na_2SO_4$. Nach Entfernen des Trocken- und Lösungsmittels wird der Rückstand zuerst chromatographisch (Kieselgel, EtOAc/MeOH/$NH_3$ 95:5:0.5) und dann via HPLC gereinigt.
Ausbeute: 22 mg (17.0 % d. Theorie)
$C_{25}H_{23}ClN_40$ (M= 430,941)
ber.: Molpeak $(M+H)^+$: 431/433 gef.: Molpeak $(M+H)^+$: 431/433
$R_f$-Wert: 0.39 (Kieselgel, EtOAc/MeOH/$NH_3$ 90:10:1)

**Beispiel 16**

{5-[5-(4-Chlor-phenyl)-pyridin-2-ylethinyl]-3-methyl-pyridin-2-yl}-(2-pyrrolidin-1-yl-ethyl)-amin

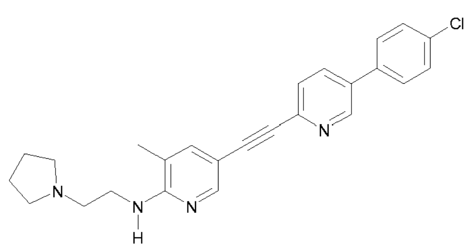

16a (5-Brom-3-methyl-pyridin-2-yl)-(2-pyrrolidin-1-yl-ethyl)-amin

Unter Argonatmosphäre werden zu einer auf 0˚C gekühlten Lösung von 800 mg (2.68 mmol) *N*-(5-Brom-3-methyl-pyridin-2-yl)-2-pyrrolidin-1-yl-acetamid (Beispiel 15b) in 10 mL THF 2 mL einer 1 M Lösung von $LiAlH_4$ in THF langsam zugetropft und das Reaktionsgemisch 3 h bei dieser Temperatur gerührt. Man tropft langsam 20% NaOH zu, gibt zur Suspension festes $K_2CO_3$ und rührt diese kräftig durch. Man filtriert den Niederschlag ab, engt das Filtrat ein und reinigt den Rückstand chromatographisch (Kieselgel, EtOAc/MeOH/$NH_3$ 80:20:2).
Ausbeute: 500 mg (65.6 % d. Theorie)
$C_{12}H_{18}BrN_3$ (M= 284,201)
ber.: Molpeak $(M+H)^+$: 284/286 gef.: Molpeak $(M+H)^+$: 284/286
$R_f$-Wert: 0.25 (Kieselgel, EtOAc/MeOH/$NH_3$ 80:20:2)

16b (3-Methyl-5-trimethylsilanylethinyl-pyridin-2-yl)-(2-pyrrolidin-1-yl-ethyl)-amin

Hergestellt analog Beispiel 15c aus 500 mg (1.76 mmol) (5-Brom-3-methyl-pyridin-2-yl)-(2-pyrrolidin-1-yl-ethyl)-amin und 0.29 mL (2.11 mmol) Ethinyl-trimethyl-silan, wobei das Reaktionsgemisch 12 h auf 50˚C erwärmt wird.
Ausbeute: 400 mg (75.4 % d. Theorie)

$C_{17}H_{27}N_3Si$ (M= 301,511)
ber.: Molpeak $(M+H)^+$: 302 gef.: Molpeak $(M+H)^+$: 302
$R_f$-Wert: 0.27 (Kieselgel, EtOAc/MeOH/$NH_3$ 90:10:1)

16c (5-Ethinyl-3-methyl-pyridin-2-yl)-(2-pyrrolidin-1-yl-ethyl)-amin

Hergestellt analog Beispiel 15d aus 400 mg (1.33 mmol) (3-Methyl-5-trimethylsilanyl-ethinyl-pyridin-2-yl)-(2-pyrrolidin-1-yl-ethyl)-amin.
Ausbeute: 250 mg (82.2 % d. Theorie)
$C_{14}H_{19}N_3$ (M= 229,328)
$R_f$-Wert: 0.51 (Kieselgel, EtOAc/MeOH/$NH_3$ 80:20:2)

16d {5-[5-(4-Chlor-phenyl)-pyridin-2-ylethinyl]-3-methyl-pyridin-2-yl}-(2-pyrrolidin-1-yl-ethyl)-amin

Hergestellt analog Beispiel 15e aus 69 mg (0.3 mmol) (5-Ethinyl-3-methyl-pyridin-2-yl)-(2-pyrrolidin-1-yl-ethyl)-amin und 95 mg (0.3 mmol) 5-(4-Chlor-phenyl)-2-iod-pyridin, wobei das Rohprodukt nach der Aufarbeitung chromatographisch an Alox (Cyc/EtOAc 6:4) gereinigt wird.
Ausbeute: 12 mg (9.6 % d. Theorie)
$C_{25}H_{25}ClN_4$ (M= 416,958)
ber.: Molpeak $(M+H)^+$: 417/419 gef.: Molpeak $(M+H)^+$: 417/419
$R_f$-Wert: 0.42 (Alox, Cyc/EtOAc 1:1)

**Beipiel 17:**

6-(4-Chlor-phenyl)-2-(4-pyrrolidin-1-ylmethyl-phenylethinyl)-chinolin

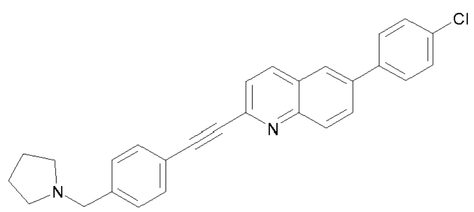

17a (*E*)-3-Ethoxy-acrylsäurechlorid

Zu einer Lösung von 14.99 mL (0.206 mol) Thionylchlorid in 300 ml Toluol werden bei RT 20 g (0.172 mol) (*E*)-3-Ethoxy-acrylsäure portionenweise gegeben und 2 h auf 90°C erhitzt. Die Reaktionsmischung wird bis zur Trockene eingeengt und das verbleibende gelbe Öl ohne weitere Reinigung weiter umgesetzt.

17b (*E*)-N-(4-Brom-phenyl)-3-ethoxy-acrylamid

26.63 g (0.155 mol) 4-Bromanilin werden in 120 ml Pyridin gelöst und tropfenweise bei einer Temperatur zwischen 0°C und 5°C mit 23.14 g (0.172 mol) (*E*)-3-Ethoxy-acrylsäurechlorid versetzt und eine Stunde bei 0°C gerührt. Im Anschluss lässt man auf RT erwärmen und rührt die Reaktionsmischung 14 h. Die Reaktionsmischung wird mit Wasser versetzt, der ausgefallene Niederschlag abfiltriert und mit Wasser gewaschen. Der Feststoff wird bei 65°C im Trockenschrank getrocknet.
Ausbeute: 37.84 g (90.4 % d. Theorie)
$C_{11}H_{12}BrNO_2$ (M= 270,12)
ber.: Molpeak $(M+H)^+$: 270/272 gef.: Molpeak $(M+H)^+$: 270/272
$R_f$-Wert: 0.7 (Kieselgel, Cyc/EtOAc 1:1)

17c 6-Brom-1*H*-chinolin-2-on

37.8 g (0.14 mol) (*E*)-N-(4-Brom-phenyl)-3-ethoxy-acrylamid werden portionenweise in 200 mL konzentrierte Schwefelsäure eingetragen und 2 h bei RT gerührt. Anschließend wird die Reaktionsmischung in Eiswasser gegossen, der Niederschlag abfiltriert und mit Wasser gewaschen. Der Feststoff wird bei 70˚C im Trockenschrank getrocknet.
Ausbeute: 28.6 g (91.2 % d. Theorie)
$C_9H_6BrNO$ (M= 224,05)
ber.: Molpeak $(M+H)^+$: 224/226 gef.: Molpeak $(M+H)^+$: 224/226
$R_f$-Wert: 0.6 (Kieselgel, EtOAc)

17d 6-(4-Chlor-phenyl)-1*H*-chinolin-2-on

Eine Lösung von 22.7 g (0.101 mol) 6-Brom-1H-chinolin-2-on in 380 mL 1,4-Dioxan und 380 mL MeOH wird mit 141.5 ml (0.283 ml) 2 M $Na_2CO_3$-Lösung versetzt und mit Argon gesättigt. Anschließend werden 3.735 g (3.23 mmol) Tetrakis-triphenyl-phosphanpalladium und 4-Chlorphenylboronsäure nacheinander zugefügt. Die Reaktionsmischung wird vier Stunden auf 110˚C erhitzt und anschließend auf ein Volumen von 300 mL eingeengt. Es werden 1.2 L Wasser zugesetzt und der Niederschlag abfiltriert. Der Feststoff wird bei 55˚C im Trockenschrank getrocknet, mit Diisopropylether gewaschen und nochmals getrocknet.
Ausbeute: 25.4 (89.5 % d. Theorie)
$C_{15}H_{10}ClNO$ (M= 255,70)
ber.: Molpeak $(M+H)^+$: 256/258 gef.: Molpeak $(M+H)^+$: 256/258
$R_f$-Wert: 0.6 (Kieselgel, EtOAc/PE 3:1)

17e 2-Brom-6-(4-chlor-phenyl)-chinolin

50 g (0.174 mol) Phosphoroxybromid werden auf 65˚C erhitzt, mit 10 g (0.039 mol) 6-(4-Chlor-phenyl)-1*H*-chinolin-2-on versetzt und 3 h auf 110˚C erhitzt. Das Reaktionsgemisch wird anschließend auf Eiswasser gegossen und mit Ammoniaklösung alkalisch gestellt. Der Niederschlag wird abfiltriert und bei 60˚C im Trockenschrank getrocknet.
Ausbeute: 12.28 g (98.8 % d. Theorie)
$C_{15}H_9BrClN$ (M= 318,60)
ber.: Molpeak $(M+H)^+$: 318/320/322 gef.: Molpeak $(M+H)^+$: 318/320/322
$R_f$-Wert: 0.8 (Kieselgel, Cyc/EtOAc 3:1)

17f 4-[6-(4-Chlor-phenyl)-chinolin-2-ylethinyl]-benzaldehyd

Unter Stickstoffatmosphäre werden zu einer Lösung von 85 mg (0.66 mmol) 4-Ethinylbenzaldehyd und 2.6 mL (18.67 mmol) Triethylamin in 5 mL absolutem DMF und 10 mL Acetonitril nacheinander 0.6 mg CuI, 9.3 mg $Pd(PPh_3)_2Cl_2$ und 318 mg (1 mmol) 2-Brom-6-(4-chlor-phenyl)-chinolin gegeben. Die Reaktionsmischung wird 14 Stunden bei RT gerührt und eingeengt. Die Reinigung erfolgt durch Säulenchromatographie an Kieselgel (PE/EtOAc 1:1).
Ausbeute: 290 mg (78.8 % d. Theorie)
$C_{24}H_{14}ClNO$ (M= 367,83)
ber.: Molpeak $(M+H)^+$: 368/370 gef.: Molpeak $(M+H)^+$: 368/370
$R_f$-Wert: 0.84 (Kieselgel, DCM/MeOH/$NH_3$ 90:10:1)

17g 6-(4-Chlor-phenyl)-2-(4-pyrrolidin-1-ylmethyl-phenylethinyl)-chinolin

Zu einer Lösung von 290 mg (0.78 mmol) 4-[6-(4-Chlor-phenyl)-chinolin-2-ylethinyl]-benzaldehyd und 56 mg (0.78 mmol) Pyrrolidin in 10 mL THF werden bei RT 3 mg p-Toluolsulfonsäure und 100 $\mu$L Eisessig zugefügt und 30 Minuten gerührt. Anschließend werden portionenweise 334 mg (1.57 mmol) $NaBH(OAc)_3$ zugesetzt und die Reaktionsmischung 14 h gerührt. Es werden einige Tropfen Wasser in die Reaktionsmischung getropft und 15 Minuten gerührt. Nachfolgend wird die Reaktionsmischung mit $K_2CO_3$ versetzt und filtriert. Das Filtrat wird eingeengt. Die Reinigung erfolgt durch Säulenchromatographie an Kieselgel (DCM/MeOH/$NH_3$ 90:10:1).
Ausbeute: 150 mg (45 % d. Theorie)
Schmelzpunkt: 170-193˚C
$C_{28}H_{23}ClN_2$ (M= 422,96)
ber.: Molpeak $(M+H)^+$: 423/425 gef.: Molpeak $(M+H)^+$: 423/425
$R_f$-Wert: 0.49 (Kieselgel, DCM/MeOH/$NH_3$ 90:10:1)
Analog Beispiel 17g werden mit 17f als Edukt folgende Verbindungen hergestellt:

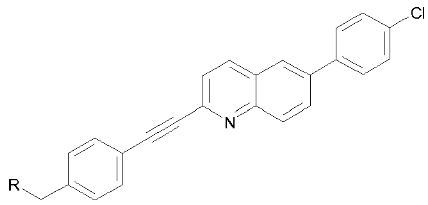

| Beispiel | R | Summenformel | Massen-spektrum | Fp [˚C] | $R_f$-Wert |
|---|---|---|---|---|---|
| 17.1 | 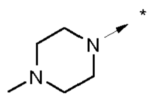 | $C_{29}H_{26}ClN_3$ | 452/54 $[M+H]^+$ | 176-182 | 0.33 |
| 17.2 | 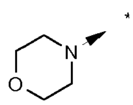 | $C_{28}H_{23}ClN_2O$ | 439/41 $[M+H]^+$ | 170-174 | 0.45 |
| 17.3 | 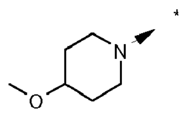 | $C_{30}H_{27}ClN_2O$ | 467/69 $[M+H]^+$ | 159-165 | 0.52 |
| 17.4 | 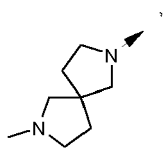 | $C_{32}H_{30}ClN_3$ | 492/94 $[M+H]^+$ | 140-148 | 0.21 |
| 17.5 | 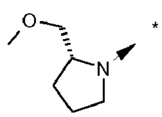 | $C_{30}H_{27}ClN_2O$ | 467/69 $[M+H]^+$ | 139-147 | 0.61 |
| 17.6 | 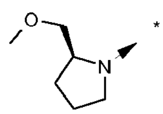 | $C_{30}H_{27}C1IN_2O$ | 467/69 $[M+H]^+$ | 143-173 | 0.61 |
| 17.7 | 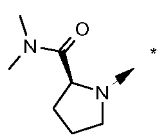 | $C_{31}H_{28}ClN_3O$ | 494/96 $[M+H]^+$ | 142-145 | 0.31 |
| 17.8 | 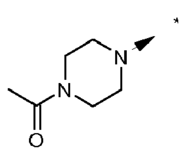 | $C_{30}H_{26}ClN_3O$ | 480/82 $[M+H]^+$ | 165-170 | 0.30 |
| 17.9 | 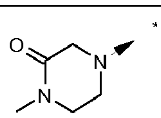 | $C_{29}H_{24}ClN_3O$ | 466/68 $[M+H]^+$ | 152-157 | 0.45 |

(fortgesetzt)

| Beispiel | R | Summenformel | Massen-spektrum | Fp [°C] | $R_f$-Wert |
|---|---|---|---|---|---|
| 17.10 | 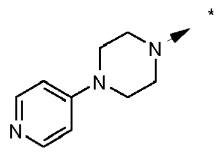 | $C_{33}H_{27}ClN_4$ | 515/17 $[M+H]^+$ | 214-219 | 0.47 |
| 17.11 | 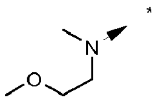 | $C_{28}H_{25}ClN_2O$ | 441/43 $[M+H]^+$ | 153 | 0.38 |
| 17.12 | 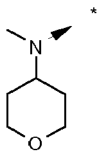 | $C_{30}H_{27}ClN_2O$ | 467/69 $[M+H]^+$ | 130-136 | 0.32 |
| 17.13 | 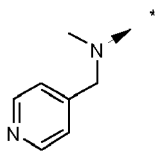 | $C_{31}H_{24}ClN_3$ | 474/476 $[M+H]^+$ | 140-149 | 0.43 |
| 17.14 | 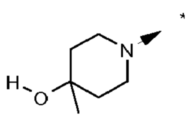 | $C_{30}H_{27}ClN_2O$ | 467/69 $[M+H]^+$ | 190-192 | 0.30 |
| 17.15 | 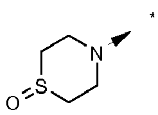 | $C_{28}H_{23}ClN_2OS$ | 471/73 $[M+H]^+$ | 170-174 | 0.38 |
| 17.16 | 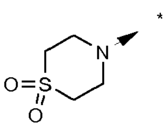 | $C_{28}H_{23}ClN_2O_2S$ | 487/89 $[M+H]^+$ | 216-220 | 0.61 |
| 17.17 | 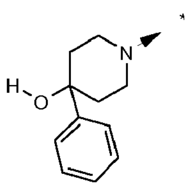 | $C_{35}H_{29}ClN_2O$ | 529/31 $[M+H]^+$ | 208-219 | 0.34 |

Die angegebenen $R_f$-Werte werden auf Kieselgel mit DCM/MeOH/$NH_3$ 90:10:1 als mobile Phase erhalten.

**Beipiel 18:**

6-(4-Chlor-phenyl)-2-[4-(2-pyrrolidin-1-yl-ethoxy)-phenylethinyl]-chinolin

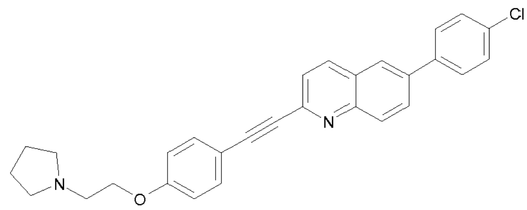

18a 1-[2-(4-lod-phenoxy)-ethyl]-pyrrolidin

Eine Reaktionsmischung aus 44 g (0.2 mol) 4-lodphenol, 34 g (0.2 mol) 1-(2-Chlorethyl)-pyrrolidin-hydrochlorid, 110.56 g (0,8 mol) $K_2CO_3$ und 800 mL DMF wird 48 h bei RT gerührt. Die Reaktionsmischung wird filtriert und das Filtrat eingeengt. Der Rückstand wird in Wasser aufgenommen und mit EtOAc extrahiert. Die organische Phase wird mit gesättigter NaCl-Lösung extrahiert und über $Na_2SO_4$ getrocknet. Die Reinigung erfolgt durch Säulenchromatographie an Kieselgel (EtOAc/MeOH/$NH_3$ 85:15:1.5).
Ausbeute: 34.8 g (54.9 % d. Theorie)
$C_{12}H_{16}INO$ (M= 317,17)
$R_f$-Wert: 0.49 (Kieselgel, EtOAc/MeOH/$NH_3$ 90:10:1)

18b 1-[2-(4-Trimethylsilanylethinyl-phenoxy)-ethyl]-pyrrolidin

Unter Stickstoffatmospäre und Eiskühlung wird eine Reaktionsmischung aus 1.5 g (4.72 mmol) 1-[2-(4-lod-phenoxy)-ethyl]-pyrrolidin, 0.735 ml (5.2 mmol) Ethinyl-trimethyl-silan, 15 mL Piperidin, 115.5 mg (0.1 mmol) Tetrakis-triphenylphosphan-Palladium und 19 mg (0.1 mmol) Cul 1 h gerührt. Anschließend wird die Reaktionsmischung eingeengt, der Rückstand in 20 mL Wasser aufgenommen und mit EtOAc extrahiert. Die organische Phase wird über $Na_2SO_4$ getrocknet. Die Reinigung erfolgt durch Säulenchromatographie an Kieselgel (EtOAc/MeOH/$NH_3$ 95:5:0.5).
Ausbeute: 1.244 g (91.5 % d. Theorie)
$C_{17}H_{25}NOSi$ (M= 287,48)
ber.: Molpeak $(M+H)^+$: 288 gef.: Molpeak $(M+H)^+$: 288
$R_f$-Wert: 0.45 (Kieselgel, EtOAc/MeOH/$NH_3$ 90:10:1)

18c 1-[2-(4-Ethinyl-phenoxy)-ethyl]-pyrrolidin

Eine Reaktionsmischung aus 1.22 g (4.24 mmol) 1-[2-(4-Trimethylsilanylethinylphenoxy)-ethyl]-pyrrolidin, 1.47 g (4.67 mmol) TBAF und 25 mL THF wird 3 h bei RT gerührt. Im Anschluss wird die Reaktionsmischung eingeengt und der Rückstand mit 20 mL gesättigter NaCl-Lösung und 50 mL EtOAc versetzt. Die organische Phase wird über $Na_2SO_4$ getrocknet. Die Reinigung erfolgt durch Säulenchromatographie an Kieselgel (EtOAc/MeOH/$NH_3$ 90:10:1).
Ausbeute: 0.91 g (100 % d. Theorie)
$C_{14}H_{17}NO$ (M= 215,29)
ber.: Molpeak $(M+H)^+$: 216 gef.: Molpeak $(M+H)^+$: 216
$R_f$-Wert: 0.33 (Kieselgel, EtOAc/MeOH/$NH_3$ 90:10:1)

18d 6-(4-Chlor-phenyl)-2-[4-(2-pyrrolidin-1-yl-ethoxy)-phenylethinyl]-chinolin

Zu einer Lösung von 142 mg (0.66 mmol) 1-[2-(4-Ethinyl-phenoxy)-ethyl]-pyrrolidin und 2.6 mL (18.67 mmol) Triethylamin in 5 mL absolutem DMF und 10 mL Acetonitril werden in einer Stickstoffatmosphäre nacheinander 0.6 mg Cul, 9.3 mg $Pd(PPh_3)_2Cl_2$ und 318 mg (1 mmol) 2-Brom-6-(4-chlor-phenyl)-chinolin gegeben Die Reaktionsmischung wird 14 h bei RT gerührt und eingeengt. Die Reinigung erfolgt durch Säulenchromatographie an Kieselgel (DCM/MeOH/$NH_3$ 90:10:1).
Ausbeute: 148 mg (32.7 % d. Theorie)
Schmelzpunkt: 176-185˚C
$C_{29}H_{25}ClN_2O$ (M= 452,98)
ber.: Molpeak $(M+H)^+$: 453/455 gef.: Molpeak $(M+H)^+$: 453/455
$R_f$-Wert: 0.71 (Kieselgel, DCM/MeOH/$NH_3$ 80:20:1)

**Beipiel 19:**

6-(4-Chlor-phenyl)-2-[3-(2-pyrrolidin-1-yl-ethoxy)-phenylethinyl]-chinolin

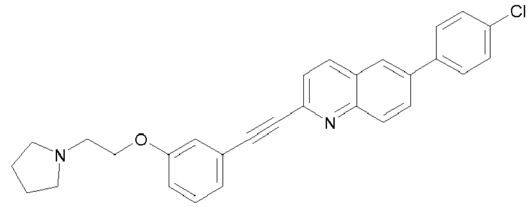

19a 1-[2-(3-Ethinyl-phenoxy)-ethyl]-pyrrolidin

Hergestellt analog Beispiel 18a aus 1-(2-Chlor-ethyl)-pyrrolidin-hydrochlorid und 3-Ethinyl-phenol.
Ausbeute: 1.44 g (79 % d. Theorie)
$C_{14}H_{17}NO$ (M= 215,29)
ber.: Molpeak $(M+H)^+$: 216 gef.: Molpeak $(M+H)^+$: 216
$R_f$-Wert: 0.37 (Kieselgel, DCM/MeOH/$NH_3$ 90:10:1)

19b 6-(4-Chlor-phenyl)-2-[3-(2-pyrrolidin-1-yl-ethoxy)-phenylethinyl]-chinolin

Hergestellt analog Beispiel 18d aus 1-[2-(3-Ethinyl-phenoxy)-ethyl]-pyrrolidin und 2-Brom-6-(4-chlor-phenyl)-chinolin.
Ausbeute: 135 mg (29.8 % d. Theorie)
Schmelzpunkt: 114-117˚C
$C_{29}H_{25}ClN_2O$ (M= 452,98)
ber.: Molpeak $(M+H)^+$: 453/455 gef.: Molpeak $(M+H)^+$: 453/455
$R_f$-Wert: 0.61 (Kieselgel, DCM/MeOH/$NH_3$ 80:20:1)

**Beipiel 20:**

6-(4-Chlor-phenyl)-2-(2-pyrrolidin-1-ylmethyl-benzoxazol-5-ylethinyl)-chinolin

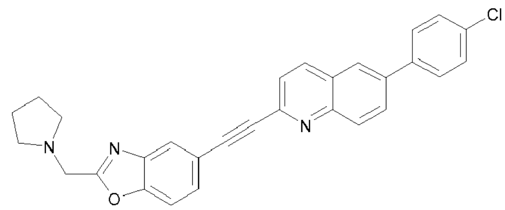

20a 5-Brom-2-chlormethyl-benzoxazol

Zu einer Lösung von 2.5 g (13.29 mmol) 2-Amino-4-bromphenol in 20 mL Ethanol werden bei RT 1.79 mL (13.3 mmol) 2-Chlor-1,1,1-trimethoxy-ethan getropft und 48 h gerührt. Im Anschluss werden 0.4 mL 2-Chlor-1,1,1-trimethoxy-ethan zugesetzt und 20 h gerührt. Die Reaktionsmischung wird eingeengt. Die Reinigung erfolgt durch Säulenchromatographie an Kieselgel (DCM/Ethanol 80:1).
Ausbeute: 2 g (60.9 % d. Theorie)
$C_8H_5BrClNO$ (M= 246,49)
ber.: Molpeak $(M+H)^+$: 246/248/250 gef.: Molpeak $(M+H)^+$: 246/248/250
$R_f$-Wert: 0.95 (Kieselgel, DCM/Ethanol 20:1)

20b 5-Brom-2-pyrrolidin-1-ylmethyl-benzoxazol

Eine Lösung von 2 g (8.11 mmol) 5-Brom-2-chlormethyl-benzoxazol in 30 mL DMF wird mit 2.24 g (16,22 mmol) $K_2CO_3$ und 0.9 mL (10.78 mmol) Pyrrolidin versetzt und 24 Stunden bei RT gerührt. Die Reaktionsmischung wird mit Wasser verdünnt und mit EtOAc extrahiert. Die organische Phase wird über $Na_2SO_4$ getrocknet, das Trockenmittel abfiltriert und das Filtrat eingeengt.
Ausbeute: 2.2 g (96.4 % d. Theorie)
$C_{12}H_{13}BrN_2O$ (M= 281,15)
ber.: Molpeak $(M+H)^+$: 281/283 gef.: Molpeak $(M+H)^+$: 281/283
$R_f$-Wert: 0.15 (Kieselgel, DCM/Ethanol 50:1)

20c 5-lod-2-pyrrolidin-1-ylmethyl-benzoxazol

71 mg (0.36 mmol) Cul, 1 g (3.55 mmol) 5-Brom-2-pyrrolidin-1-ylmethyl-benoxazol und 1.07 g (7.15 mmol) Nal werden in einer Argonatmosphäre nacheinander in einen Kolben gegeben. Im Anschluss werden 0.08 mL (0.73 mmol) N,N'-Dimethylethylendiamin und 3.5 mL 1,4-Dioxan zugesetzt und die Reaktionsmischung 14 h zum Rückfluss erhitzt. Nachfolgend wird die Reaktionsmischung bei RT mit 20 mL konzentrierter Ammoniaklösung versetzt, mit 100 mL Wasser verdünnt und mit DCM extrahiert. Die organische Phase wird dreimal mit Wasser extrahiert und über $Na_2SO_4$ getrocknet.
Ausbeute: 1 g (72.8 % d. Theorie)
$C_{12}H_{13}IN_2O$ 328,15)
ber.: Molpeak $(M+H)^+$: 329 gef.: Molpeak $(M+H)^+$: 329
$R_f$-Wert: 0,35 (Kieselgel, Cyc/EtOAc 1:1)

20d 2-Pyrrolidin-1-ylmethyl-5-trimethylsilanylethinyl-benzoxazol

Hergestellt analog Beispiel 18b aus 5-lod-2-pyrrolidin-1-ylmethyl-benzoxazol und Ethinyl-trimethyl-silan.
Ausbeute: 0.5 g (91.6 % d. Theorie)
$C_{17}H_{22}N_2OSi$ (M= 298,46)
ber.: Molpeak $(M+H)^+$: 299 gef.: Molpeak $(M+H)^+$: 299
$R_f$-Wert: 0.5 (Kieselgel, DCM/MeOH/$NH_3$ 90:10:1)

20e 5-Ethinyl-2-pyrrolidin-1-ylmethyl-benzoxazol

Hergestellt analog Beispiel 18c aus 2-Pyrrolidin-1-ylmethyl-5-trimethylsilanylethinylbenzoxazol.
Ausbeute: 0.265 g (69.9 % d. Theorie)
$C_{14}H_{14}N_2O$ (M= 226,28)
ber.: Molpeak $(M+H)^+$: 227 gef.: Molpeak $(M+H)^+$: 227
$R_f$-Wert: 0.79 (Kieselgel, DCM/MeOH/$NH_3$ 80:20:1)

20f 6-(4-Chlor-phenyl)-2-(2-pyrrolidin-1-ylmethyl-benzoxazol-5-ylethinyl)-chinolin

Hergestellt analog Beispiel 18d aus 5-Ethinyl-2-pyrrolidin-1-ylmethyl-benzoxazol und 2-Brom-6-(4-chlor-phenyl)-chinolin.
Ausbeute: 90 mg (13.9 % d. Theorie)
Schmelzpunkt: 151-153˚C
$C_{29}H_{22}ClN_3O$ (M= 463,97)
ber.: Molpeak $(M+H)^+$: 464/466 gef.: Molpeak $(M+H)^+$: 464/466
$R_f$-Wert: 0.53 (Kieselgel, DCM/MeOH/$NH_3$ 90:10:1)

**Beipiel 21:**

5-[5-(4-Chlor-phenyl)-pyridin-2-ylethinyl]-2-pyrrolidin-1-ylmethyl-benzoxazol

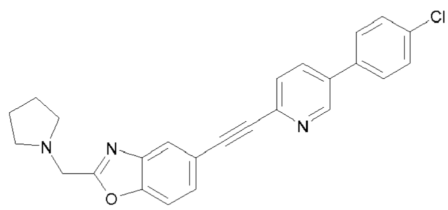

Eine Reaktionsmischung aus 260 mg (0.792 mmol) 5-Iod-2-pyrrolidin-1-ylmethylbenzoxazol, 171 mg (0.8 mmol) 5-(4-Chlor-phenyl)-2-ethinyl-pyridin, 23 mg (0.02 mmol) Tetrakis-triphenylphosphan-Palladium, 3.8 mg (0,02 mmol) CuI und 350 mg (1.075 mmol) $CS_2CO_3$ in 10 mL THF wird 14 h in einer Argonatmosphäre bei RT gerührt. Anschließend wird die Reaktionsmischung eingeengt und der Rückstand durch Säulenchromatographie an Kieselgel (DCM/MeOH 80:1) gereinigt.
Ausbeute: 140 mg (42.7 % d. Theorie)
Schmelzpunkt: 145°C
$C_{25}H_{20}ClN_3O$ (M= 413,91)
ber.: Molpeak $(M+H)^+$: 414/416 gef.: Molpeak $(M+H)^+$: 414/416
$R_f$-Wert: 0.1 (Kieselgel, DCM/MeOH 50:1)

**Beispiel 22**

5-[5-(4-Chlor-phenyl)-pyridin-2-ylethinyl]-2-(2-pyrrolidin-1-yl-ethoxy)-benzoesäure

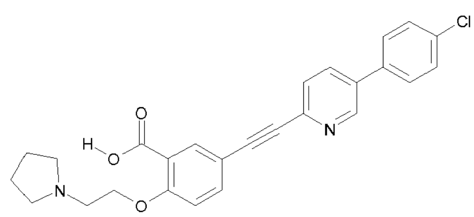

Zu einer Lösung von 369 mg (0.8 mmol) 5-[5-(4-Chlor-phenyl)-pyridin-2-ylethinyl]-2-(2-pyrrolidin-1-yl-ethoxy)-benzoesäuremethylester (Beispiel 3.4) in 20 mL MeOH werden 1.6 mL wässrige 1 M NaOH gegeben und das Reaktionsgemisch 3 h auf 70°C erhitzt. Man versetzt mit 1.6 mL 1 M HCl, engt i.vac. ein und coevaporiert den Rückstand zweimal mit jeweils 20 mL MeOH. Der Rückstand wird in der Wärme mit EtOH verrieben und abgesaugt.
Ausbeute: 340 mg (95.1 % d. Theorie)
$C_{26}H_{23}ClN_2O_3$ (M= 446,938)
ber.: Molpeak $(M+H)^+$: 447/449 gef.: Molpeak $(M+H)^+$: 447/449
Retentionszeit HPLC: 7.0 min (Methode A)

**Beispiel 22.1**

5-[5-(4-Chlor-phenyl)-pyridin-2-ylethinyl]-N-methyl-2-(2-pyrrolidin-1-yl-ethoxy)-benzamid

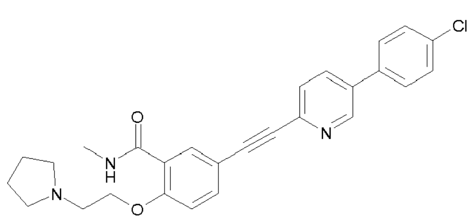

Zu einer Lösung von 112 mg (0.25 mmol) 5-[5-(4-Chlor-phenyl)-pyridin-2-ylethinyl]-2-(2-pyrrolidin-1-yl-ethoxy)-benzoesäure (Beispiel 22) in 5 mL DMF werden 80 mg (0.25 mmol) TBTU und 69 μL Triethylamin zugegeben und das Reaktionsgemisch 2 h bei RT gerührt. Anschliessend gibt man 31 mg (1.0 mmol) Methlyamin zu und rührt weitere 2 h bei RT. Man engt i.vac. ein, versetzt den Rückstand mit verdünnter $Na_2CO_3$-Lösung, extrahiert erschöpfend mit DCM und trocknet die organische Phase über $Na_2SO_4$. Nach Entfernen des Trocken- und Lösungsmittels wird der Rückstand chromatographisch (Kieselgel, DCM nach DCM/MeOH/$NH_3$ 7:3:0.3) gereinigt.
Ausbeute: 45 mg (39.1 % d. Theorie)
$C_{27}H_{26}ClN_3O_2$ (M= 459,980)
ber.: Molpeak $(M+H)^+$: 460/462 gef.: Molpeak $(M+H)^+$: 460/462
Retentionszeit HPLC: 6.8 min (Methode A)

Die folgenden Verbindungen werden wie in Beispiel 22.1 beschrieben hergestellt, wobei für Beispiel 22.5 Ammoniumcarbonat als Ammoniakquelle eingesetzt wird:

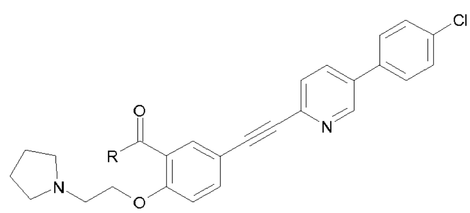

| Beispiel | R | Ausbeute (%) | Summenformel | Massen-spektrum | Retentionszeit HPLC in min (Methode) |
|---|---|---|---|---|---|
| 22.2 | 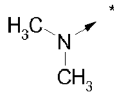 | 42.2 | $C_{28}H_{28}ClN_3O_2$ | 474/476 $[M+H]^+$ | 6.95 (A) |
| 22.3 | 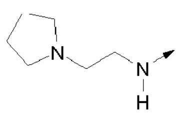 | 9.3 | $C_{32}H_{35}ClN_4O_2$ | 543/545 $[M+H]^+$ | 5.30 (A) |
| 22.4 | 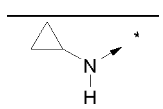 | 6.9 | $C_{29}H_{28}ClN_3O_2$ | 486/488 $[M+H]^+$ | 7.07 (A) |
| 22.5 | 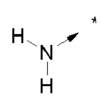 | 4.0 | $C_{26}H_{24}ClN_3O_2$ | 446/448 $[M+H]^+$ | 6.10 (A) |

## Beispiel 23

5-[5-(4-Chlor-phenyl)-pyridin-2-ylethinyl]-2-(2-pyrrolidin-1-yl-ethoxy)-phenylamin

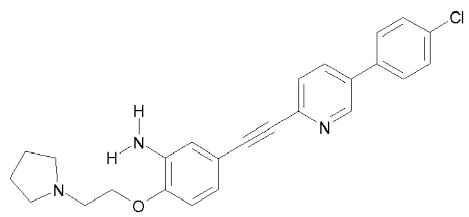

Zu einer Lösung von 100 mg (0.22 mmol) 5-(4-Chlor-phenyl)-2-[3-nitro-4-(2-pyrrolidin-1-yl-ethoxy)-phenylethinyl]-pyridin (Beispiel 3.13) in 10 mL EtOAc werden 200 mg (2.5 mmol) $NaHCO_3$ und 300 mg (1.1 mmol) Zinn-(II)-chlorid Dihydrat gegeben und das Reaktionsgemisch 2 h unter Rückfluss erhitzt. Zur Vervollständigung der Reaktion gibt man erneut 200 mg (2.5 mmol) $NaHCO_3$, 300 mg (1.1 mmol) Zinn-(II)-chlorid Dihydrat und 1 mL MeOH zu und erhitzt weitere 2 h unter Rückfluss. Nach Abkühlen wird mit 4 g Kieselgel versetzt, die Lösemittel i.vac. entfernt und der Rückstand chromatographisch (Kieselgel, DCM/MeOH/$NH_3$ 8:2:0.2) gereinigt.
Ausbeute: 85 mg (39.1 % d. Theorie)
$C_{25}H_{24}ClN_3O$ (M= 417,943)
ber.: Molpeak $(M+H)^+$: 418/420 gef.: Molpeak $(M+H)^+$: 418/420
Retentionszeit HPLC: 7.1 min (Methode A)

**Beispiel 23.1**

N-[5-[5-(4-Chlor-phenyl)-pyridin-2-ylethinyl]-2-(2-pyrrolidin-1-yl-ethoxy)-phenyl]-acetamid

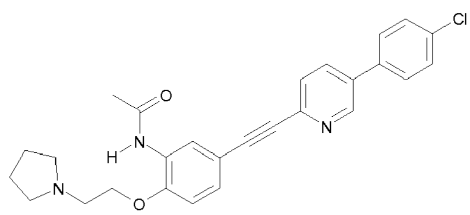

Zu einer Lösung von 35 mg (84 μmol) 5-[5-(4-Chlor-phenyl)-pyridin-2-ylethinyl]-2-(2-pyrrolidin-1-yl-ethoxy)-phenylamin in 2 mL DCM werden 16 μL (170 μmol) Acetanhydrid zugegeben und das Reaktionsgemisch über Nacht bei RT gerührt und anschliessend ohne weitere Aufarbeitung chromatographisch an Kieselgel (Gradient: EtOAc nach EtOAc/Me0H/$NH_3$ 7:3:0.3) gereinigt.
Ausbeute: 10 mg (26.0 % d. Theorie)
$C_{27}H_{26}ClN_3O_2$ (M= 459,980)
ber.: Molpeak $(M+H)^+$: 460/462 gef.: Molpeak $(M+H)^+$: 460/462
$R_f$-Wert: 0.55 (Kieselgel, DCM/MeOH/$NH_3$ 90:10:1)

**Beispiel 23.2**

N-[5-[5-(4-Chlor-phenyl)-pyridin-2-ylethinyl]-2-(2-pyrrolidin-1-yl-ethoxy)-phenyl]-methansulfonamid

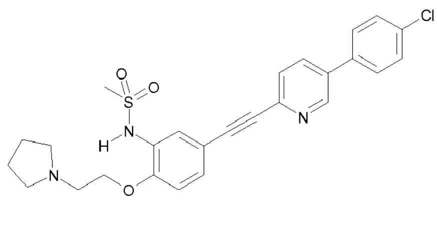

Unter $N_2$-Atmosphäre werden zu einer Lösung von 100 mg (0.24 mmol) 5-[5-(4-Chlor-phenyl)-pyridin-2-ylethinyl]-2-(2-pyrrolidin-1-yl-ethoxy)-phenylamin und 161 µL (2 mmol) Pyridin in 10 mL DCM 39 µL (0.5 mmol) Methansulfonsäurechlorid gegeben und das Reaktionsgemisch 2 h bei RT gerührt. Zur Vervollständigung der Reaktion werden erneut 160 µL Pyridin und 39 µL Methansulfonsäurechlorid zugegeben und über Nacht gerührt. Man versetzt mit 10% $Na_2CO_3$-Lösung, trennt die organische Phase ab und entfernt das Lösungsmittel i.vac.. Der Rückstand wird via HPLC gereinigt.
Ausbeute: 15 mg (13.0 % d. Theorie)
$C_{26}H_{26}ClN_3O_3S$ (M= 496,032)
ber.: Molpeak $(M+H)^+$: 496/498 gef.: Molpeak $(M+H)^+$: 496/498
Retentionszeit HPLC: 6.8 min (Methode A)

**Beispiel 23.3**

[5-[5-(4-Chlor-phenyl)-pyridin-2-ylethinyl]-2-(2-pyrrolidin-1-yl-ethoxy)-phenyl]-dimethylamin

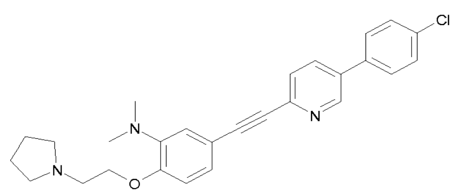

Zu einer Lösung von 100 mg (0.24 mmol) 5-[5-(4-Chlor-phenyl)-pyridin-2-ylethinyl]-2-(2-pyrrolidin-1-yl-ethoxy)-phenylamin in 5 mL Acetonitril werden 180 µL (2.4 mmol) Formalinlösung (37% in Wasser), 63 mg (1.0 mmol) $NaBH_3CN$ und 57 µL (1.0 mmol) Essigsäure gegeben und das Reaktionsgemisch über Nacht bei RT gerührt. Man säuert mit 12% HCl an, rührt eine Stunde kräftig durch, versetzt mit gesättigter $Na_2CO_3$-Lösung bis zur alkalischen Reaktion und extrahiert erschöpfend mit DCM. Nach Entfernen des Lösungsmittels wird der Rückstand via HPLC gereinigt.
Ausbeute: 9 mg (8.4 % d. Theorie)
$C_{27}H_{28}ClN_3O$ (M= 445,997)
ber.: Molpeak $(M+H)^+$: 446/448 gef.: Molpeak $(M+H)^+$: 446/448
Retentionszeit HPLC: 6.7 min (Methode A)

**Beispiel 23.4**

[5-[5-(4-Chlor-phenyl)-pyridin-2-ylethinyl]-2-(2-pyrrolidin-1-yl-ethoxy)-phenyl]-methylamin

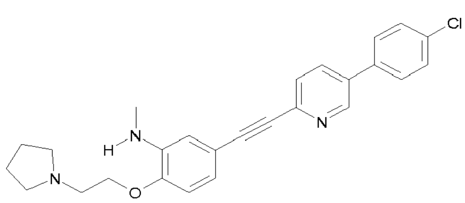

Unter Stickstoffatmosphäre werden zu einer Lösung von 100 mg (0.24 mmol) 5-[5-(4-Chlor-phenyl)-pyridin-2-ylethinyl]-2-(2-pyrrolidin-1-yl-ethoxy)-phenylamin in 2 mL DMF 178 µL (1.35 mmol) N,N-Dimethylformamiddimethylacetal gegeben, das Reaktionsgemisch 5 h bei 60°C gerührt und über Nacht auf RT abgekühlt. Dann erfolgt die Zugabe von 32 mg (0.85 mmol) $NaBH_4$ und erneutes Erhitzen auf 60°C für 1 h. Zur Vervollständigung der Reaktion werden nochmals 32 mg $NaBH_4$ zugegeben und weitere 4 h auf 60°C erwärmt. Nach dem Abkühlen wird mit gesättigter $NaHCO_3$-Lösung versetzt, erschöpfend mit EtOAc extrahiert und die organische Phase über $MgSO_4$ getrocknet. Nach Entfernen des Trocken- und Lösungsmittels wird der Rückstand via HPLC gereinigt.
Ausbeute: 0.5 mg (0.5 % d. Theorie)
$C_{26}H_{26}ClN_3O$ (M= 431,970)
ber.: Molpeak $(M+H)^+$: 432/434 gef.: Molpeak $(M+H)^+$: 432/434
Retentionszeit HPLC: 7.5 min (Methode A)

Mit den im vorangestellten experimentellen Teil beschriebenen Verfahren können auch folgende Verbindungen erhalten werden:

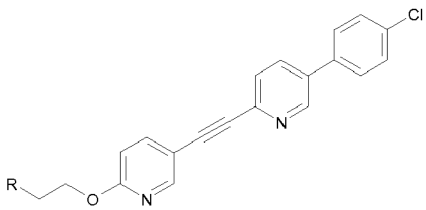

| Beispiel | R |
|---|---|
| 24.1 | 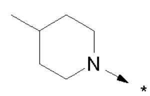 |
| 24.2 | 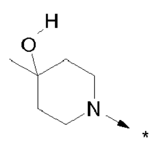 |
| 24.3 | 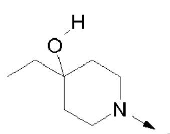 |
| 24.4 | 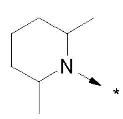 |
| 24.5 | 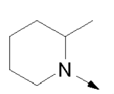 |

(fortgesetzt)

| Beispiel | R |
|---|---|
| 24.6 | 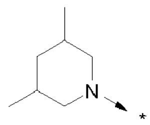 |
| 24.7 | 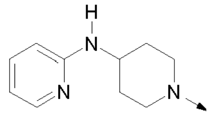 |
| 24.8 | 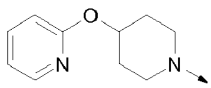 |
| 24.9 | 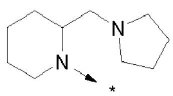 |
| 24.10 | 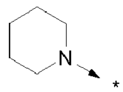 |
| 24.11 | 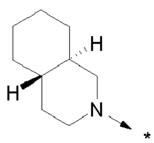 |
| 24.12 | 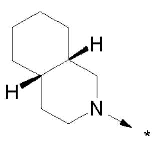 |
| 24.13 | 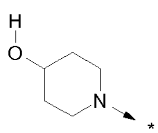 |
| 24.14 | 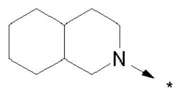 |

(fortgesetzt)

| Beispiel | R |
|---|---|
| 24.15 | 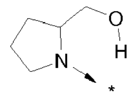 |
| 24.16 | 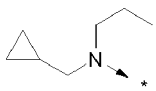 |
| 24.17 | 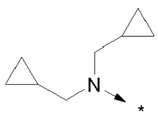 |

Mit den im vorangestellten experimentellen Teil beschriebenen Verfahren können auch folgende Verbindungen erhalten werden:

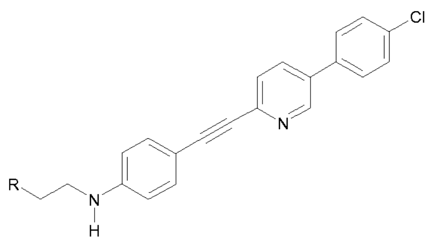

| Beispiel | R |
|---|---|
| 25.1 | 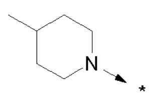 |
| 25.2 | 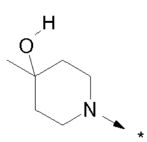 |
| 25.3 | 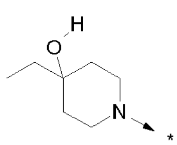 |

(fortgesetzt)

| Beispiel | R |
|---|---|
| 25.4 | 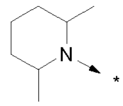 |
| 25.5 | 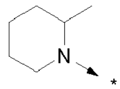 |
| 25.6 | 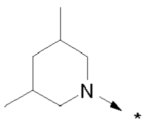 |
| 25.7 | 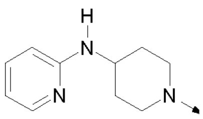 |
| 25.8 | 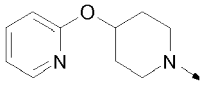 |
| | |
| 25.9 | 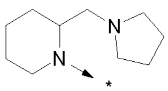 |
| 25.10 | 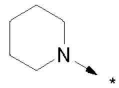 |
| 25.11 | 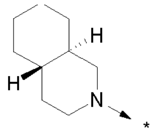 |
| 25.12 | 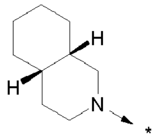 |

(fortgesetzt)

| Beispiel | R |
|---|---|
| 25.13 | 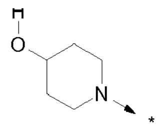 |
| 25.14 | 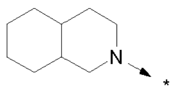 |
| 25.15 | 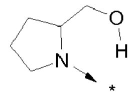 |
| 25.16 | 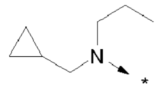 |
| 25.17 | 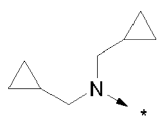 |

Mit den im vorangestellten experimentellen Teil beschriebenen Verfahren können auch folgende Verbindungen erhalten werden:

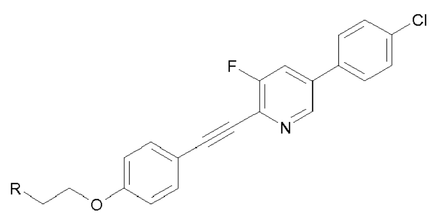

| Beispiel | R |
|---|---|
| 26.1 | 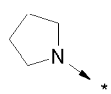 |
| 26.2 | 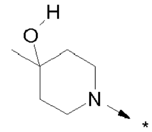 |

(fortgesetzt)

| Beispiel | R |
|---|---|
| 26.3 | 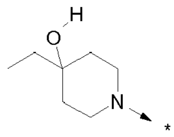 |
| 26.4 | 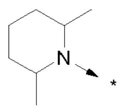 |
| 26.5 | 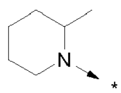 |
| 26.6 | 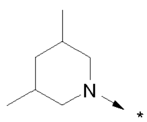 |
| 26.7 | 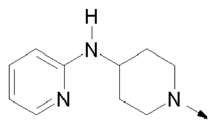 |
| 26.8 | 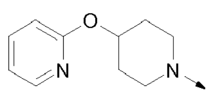 |
| 26.9 | 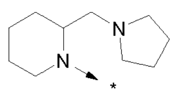 |
| 26.10 | 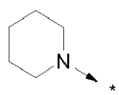 |
| 26.11 | 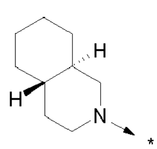 |

(fortgesetzt)

| Beispiel | R |
|---|---|
| 26.12 | 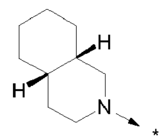 |
| 26.13 | 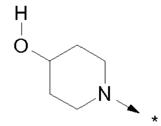 |
| 26.14 | 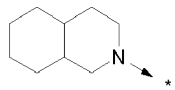 |
| 26.15 | 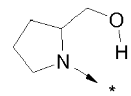 |
| 26.16 | 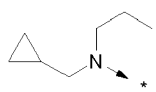 |
| 26.17 | 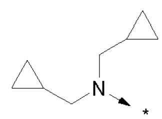 |

Mit den im vorangestellten experimentellen Teil beschriebenen Verfahren können auch folgende Verbindungen erhalten werden:

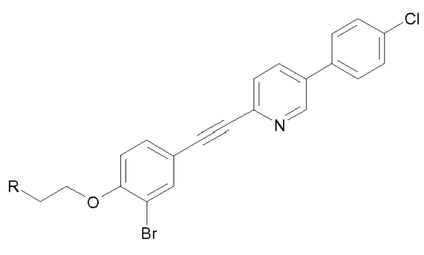

| Beispiel | R |
|---|---|
| 27.1 | 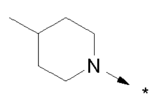 |

(fortgesetzt)

| Beispiel | R |
|---|---|
| 27.2 | 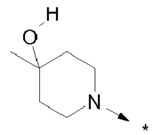 |
| 27.3 | 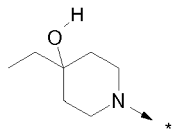 |
| 27.4 | 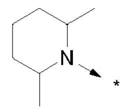 |
| 27.5 | 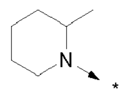 |
| 27.6 | 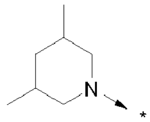 |
| 27.7 | 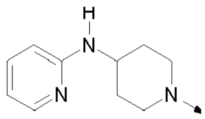 |
| 27.8 | 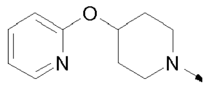 |
| | |
| 27.9 | 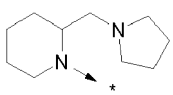 |
| 27.10 | 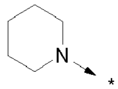 |

(fortgesetzt)

| Beispiel | R |
|---|---|
| 27.11 | 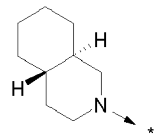 |
| 27.12 | 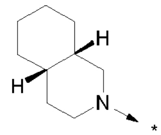 |
| 27.13 | 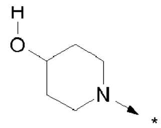 |
| 27.14 | 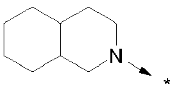 |
| 27.15 | 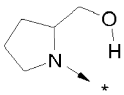 |
| 27.16 | 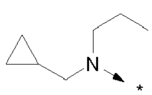 |
| 27.17 | 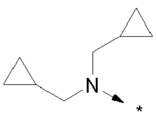 |

Mit den im vorangestellten experimentellen Teil beschriebenen Verfahren können auch folgende Verbindungen erhalten werden:

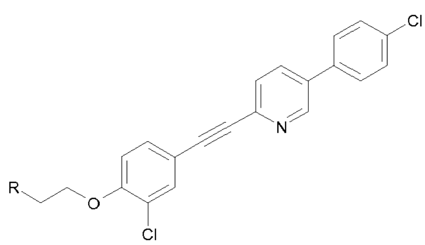

| Beispiel | R |
|---|---|
| 28.1 | 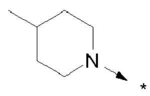 |
| 28.2 | 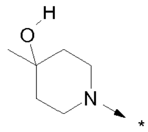 |
| 28.3 | 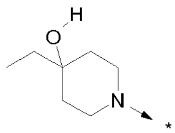 |
| 28.4 | 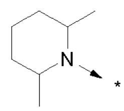 |
| 28.5 | 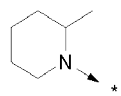 |
| 28.6 | 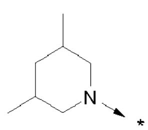 |
| 28.7 | 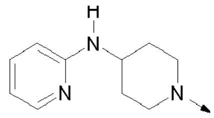 |
| 28.8 | 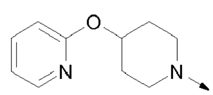 |
| | |
| 28.9 | 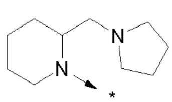 |
| 28.10 | 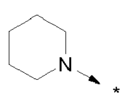 |

(fortgesetzt)

| Beispiel | R |
|---|---|
| 28.11 | 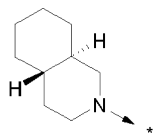 |
| 28.12 | 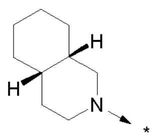 |
| 28.13 | 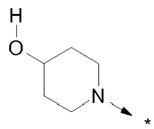 |
| 28.14 | 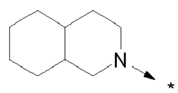 |
| 28.15 | 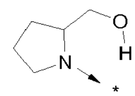 |
| 28.16 | 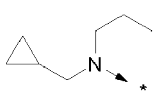 |
| 28.17 | 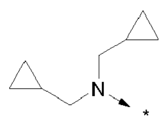 |

Mit den im vorangestellten experimentellen Teil beschriebenen Verfahren können auch folgende Verbindungen erhalten werden:

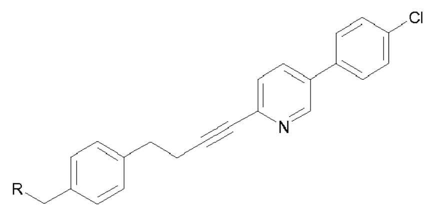

| Beispiel | R |
|---|---|
| 29.1 | 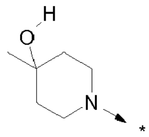 |
| 29.2 | 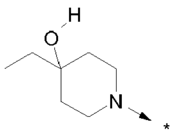 |
| 29.3 | 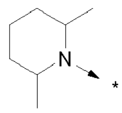 |
| 29.4 | 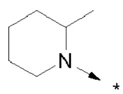 |
| 29.5 | 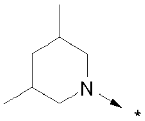 |
| 29.6 | 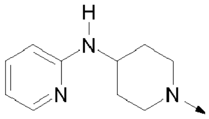 |
| 29.7 | 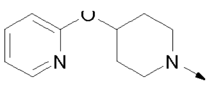 |
| 29.8 | 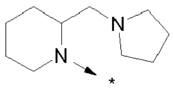 |
| 29.9 | 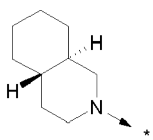 |

(fortgesetzt)

| Beispiel | R |
|---|---|
| 29.10 | 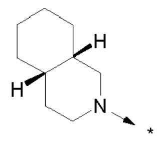 |
| 29.11 | 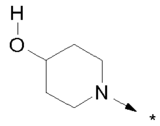 |
| 28.12 | 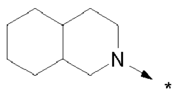 |
| 29.13 | 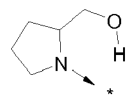 |
| 29.14 | 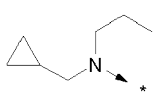 |
| 29.15 | 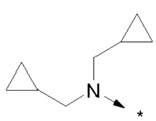 |

Mit den im vorangestellten experimentellen Teil beschriebenen Verfahren können auch folgende Verbindungen erhalten werden:

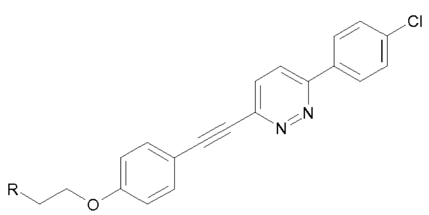

| Beispiel | R |
|---|---|
| 30.1 | 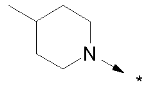 |
| 30.2 | 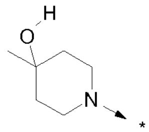 |
| 30.3 | 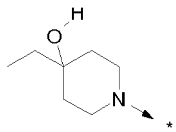 |
| 30.4 | 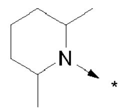 |
| 30.5 | 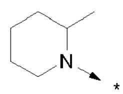 |
| 30.6 | 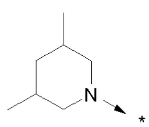 |
| 30.7 | 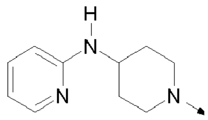 |
| 30.8 | 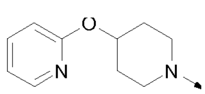 |
| 30.9 | 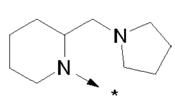 |

(fortgesetzt)

| Beispiel | R |
|---|---|
| 30.10 | 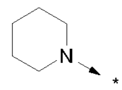 |
| 30.11 | 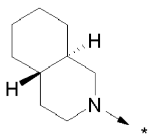 |
| 30.12 | 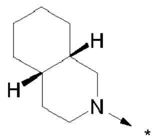 |
| 30.13 | 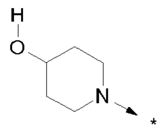 |
| 30.14 | 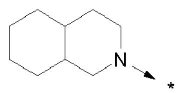 |
| 30.15 | 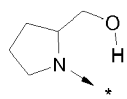 |
| 30.16 | 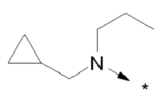 |
| 30.17 | 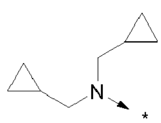 |

Mit den im vorangestellten experimentellen Teil beschriebenen Verfahren können auch folgende Verbindungen erhalten werden:

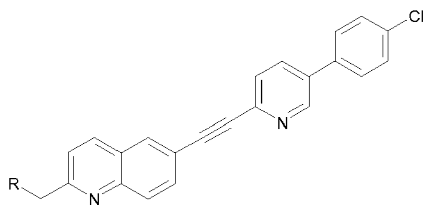

| Beispiel | R |
|---|---|
| 31.1 | 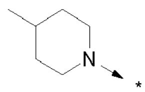 |
| 31.2 | 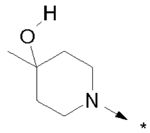 |
| 31.3 | 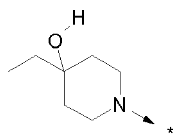 |
| 31.4 | 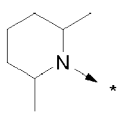 |
| 31.5 | 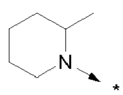 |
| 31.6 | 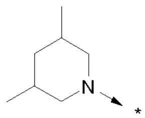 |
| 31.7 | 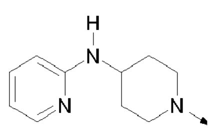 |
| 31.8 | 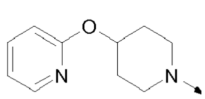 |

(fortgesetzt)

| Beispiel | R |
|---|---|
| 31.9 | 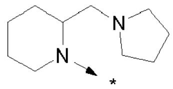 |
| 31.10 | 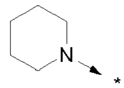 |
| 31.11 | 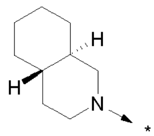 |
| 31.12 | 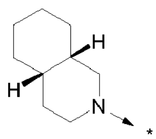 |
| 31.13 | 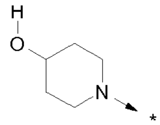 |
| 31.14 | 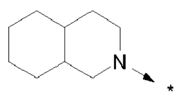 |
| 31.15 | 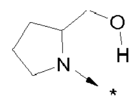 |
| 31.16 | 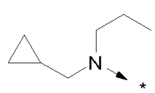 |
| 31.17 | 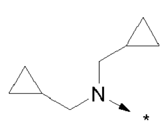 |

Mit den im vorangestellten experimentellen Teil beschriebenen Verfahren können auch die Verbindungen der Beispiele 32 bis 32.12 erhalten werden:

Beispiel 32

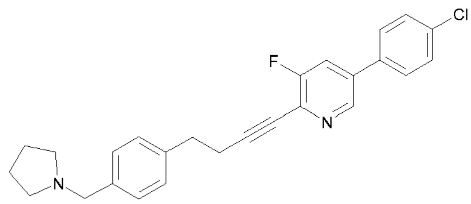

Beispiel 32.1

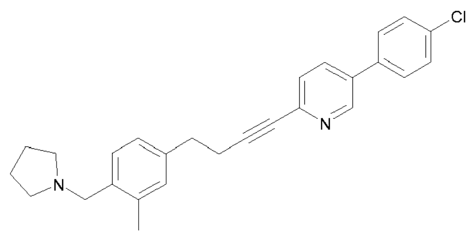

Beispiel 32.2

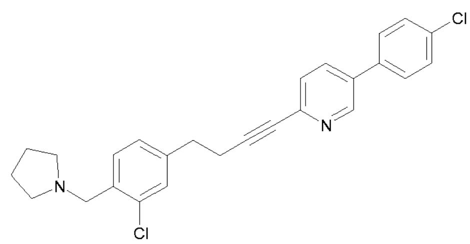

Beispiel 32.3

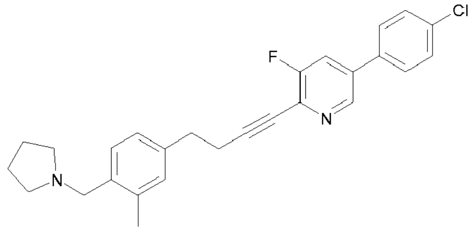

Beispiel 32.4

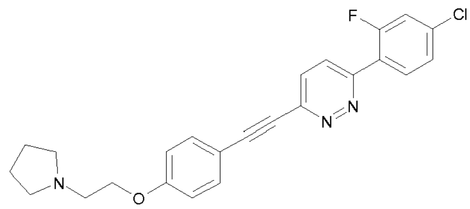

Beispiel 32.5

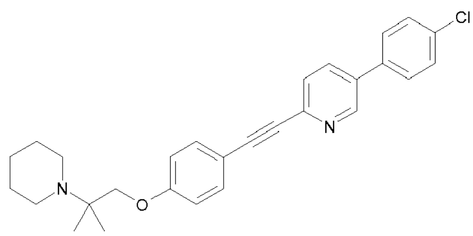

Beispiel 32.6

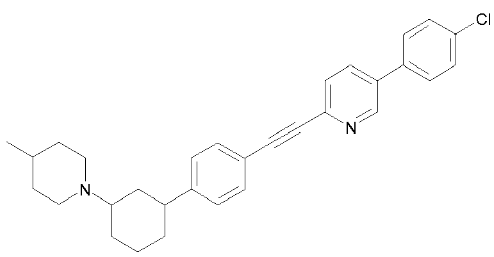

Beispiel 32.7

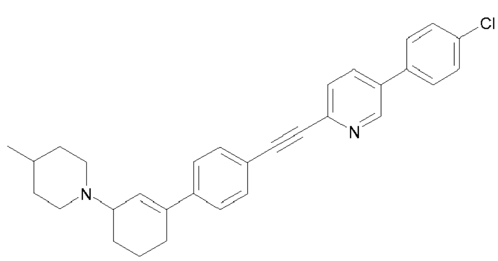

Beispiel 32.8

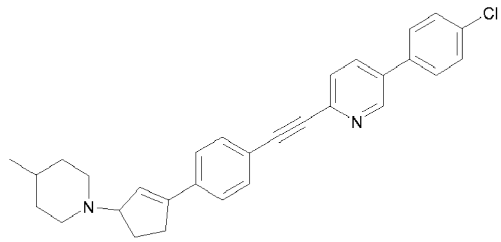

Beispiel 32.9

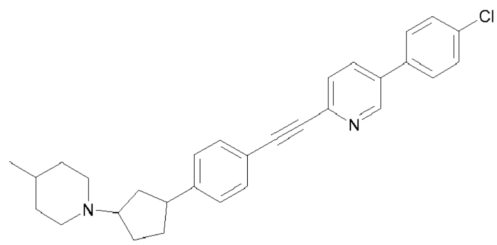

Beispiel 32.10

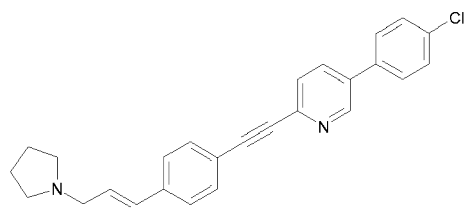

Beispiel 32.11

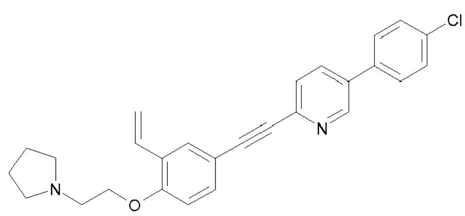

Beispiel 32.12

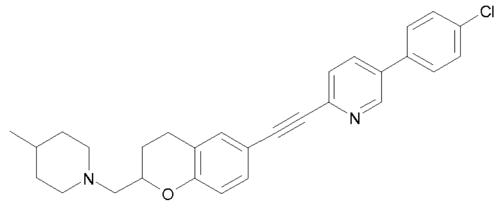

Mit den im vorangestellten experimentellen Teil beschriebenen Verfahren können auch folgende Verbindungen erhalten werden:

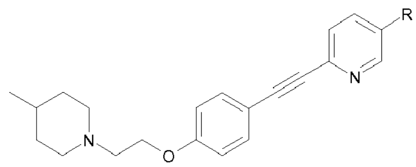

| Beispiel | R |
|---|---|
| 33.1 | 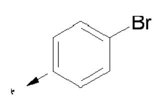 |
| 33.2 | 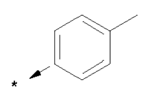 |
| 33.3 | 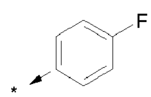 |
| 33.4 | 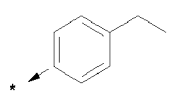 |
| 33.5 | 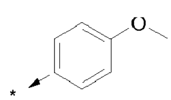 |
| 33.6 | 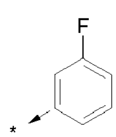 |
| 33.7 | 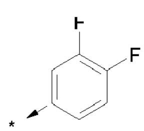 |

(fortgesetzt)

| Beispiel | R |
|---|---|
| 33.8 | 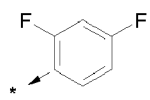 |
| 33.9 | 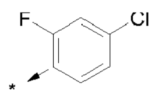 |
| 33.10 | 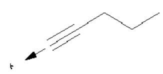 |
| 33.11 | 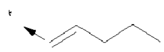 |
| 33.12 | 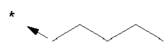 |
| 33.13 | 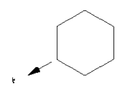 |
| 33.14 | 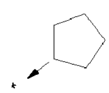 |

Nachfolgend werden Testverfahren zur Bestimmung einer MCH-Rezeptor antagonistischen Aktivität beschrieben. Darüber hinaus können auch weitere dem Fachmann bekannte Testverfahren, beispielsweise über die Inhibition der MCH-Rezeptor vermittelten Hemmung der cAMP-Produktion, wie von Hoogduijn M et al. in "Melanin-concentrating hormone and its receptor are expressed and functional in human skin", Biochem. Biophys. Res Commun. 296 (2002) 698-701 sowie über die biosensorische Messung der Bindung von MCH an den MCH Rezeptor in Gegenwart antagonistischer Substanzen durch Plasmonresonanz, wie von Karlsson OP und Lofas S. in "Flow-Mediated On-Surface Reconstitution of G-Protein Coupled Receptors for Applications in Surface Plasmon Resonance Biosensors", Anal. Biochem. 300 (2002), 132-138 beschrieben, eingesetzt werden. Weitere Testmethoden auf MCH-Rezeptor antagonistische Aktivität sind in den einleitend genannten Literaturstellen und Patentdokumenten enthalten, deren Beschreibung der Testmethoden hiermit in diese Anmeldung aufgenommen wird.

**MCH-1 Rezeptorbindungstest**

| | |
|---|---|
| Methode: | MCH Bindung an hMCH-1 R transfizierten Zellen |
| Spezies: | Human |
| Testzelle: | hMCH-1 R stabil-transfiziert in CHO/Galpha16 Zellen |
| Resultate: | 1C50 Werte |

Membranen aus mit humanem hMCH-1 R stabil-transfizierten CHO/Galpha16 Zellen werden mit Hilfe einer Spritze resuspendiert (Nadel 0.6 x 25 mm) und in Testpuffer (50 mM HEPES, 10 mM $MgCl_2$, 2 mM EGTA, pH 7.00; 0.1

% Rinderserum-Albumin (Protease-frei), 0.021 % Bacitracin, 1 μg/ml Aprotinin, 1 μg/ml Leupeptin and 1 μM Phosphoramidon) auf eine Konzentration von 5 bis 15 μg/ml verdünnt. 200 Mikroliter dieser Membranfraktion (enthält 1 bis 3 μg Protein) werden für 60 Minuten bei Raumtemperatur mit 100 pM $^{125}$ I-tyrosyl melanine concentrating hormone $C^{25}$I-MCH kommerziell erhältlich von NEN) und steigende Konzentrationen der Testverbindung in einem Endvolumen von 250 Mikroliter inkubiert. Nach der Inkubation wird die Reaktion unter Benutzung eines Zellernters durch 0.5% PEI behandelte Glasfiberfilter (GF/B, Unifilter Packard) filtriert. Die membrangebundene auf dem Filter retenierte Radioaktivität wird anschliessend nach Zugabe von Szintillatorsubstanz (Packard Microscint 20) in einem Messgerät bestimmt (TopCount von Packard).

Die nichtspezifische Bindung ist definiert als gebundene Radioaktivität in Gegenwart von 1 Mikromolar MCH während der Inkubationsperiode.

Die Analyse der Konzentration-Bindungskurve erfolgt unter der Annahme einer Rezeptorbindungsstelle.

Standard:

Nichtmarkiertes MCH kompetiert mit markiertem $^{125}$I–MCH um die Rezeptorbindung mit einem IC50 Wert zwischen 0.06 bis 0.15 nM.

Der KD-Wert des Radioliganden beträgt 0.156 nM.

**MCH-1 Rezeptor-gekoppelter $Ca^{2+}$ Mobilisierungstest**

| | |
|---|---|
| Methode: | Calciummobilisierungstest mit humanem MCH (FLIPR$^{384}$) |
| Spezies: | Human |
| Testzellen: | Mit hMCH-R1 stabil-transfizierte CHO/ Galpha 16 Zellen |
| Resultate: : | 1. Messung: % Stimulation der Referenz (MCH $10^{-6}$M) |
| | 2. Messung: pKB Wert |

| | | |
|---|---|---|
| Reagentien: | HBSS (10x) | (GIBCO) |
| | HEPES Puffer (1 M) | (GIBCO) |
| | Pluronic F-127 | (Molecular Probes) |
| | Fluo-4 | (Molecular Probes) |
| | Probenecid | (Sigma) |
| | MCH | (Bachem) |
| | Rinderserum-Albumin (Protease frei) | (Serva) |
| | DMSO | (Serva) |
| | Ham's F12 | (BioWhittaker) |
| | FCS | (BioWhittaker) |
| | L-Glutamine | (GIBCO) |
| | Hygromycin B | (GIBCO) |
| | PENStrep | (BioWhittaker) |
| | Zeocin | (Invitrogen) |

Klonale CHO/Galpha16 hMCH-R1 Zellen werden in Ham's F12 Zellkulturmedium (mit L-Glutamine; BioWhittaker; Cat.Nr.: BE12-615F) kultiviert. Dieses enthält pro 500 ml 10% FCS, 1 % PENStrep, 5 ml L-Glutamine (200 mM Stocklösung), 3 ml Hygromycin B (50 mg/ml in PBS) and 1.25 ml Zeocin (100 μg/ml Stocklösung). Einen Tag vor dem Experiment werden die Zellen auf 384-Well-Mikrotiterplatte (schwarzwandig mit durchsichtigem Boden, Hersteller: Costar) in einer Dichte von 2500 Zellen pro Kavität ausplattiert und in dem obenbeschriebenen Medium über Nacht bei 37˚C, 5% $CO_2$ und 95% relativer Luftfeuchtigkeit kultiviert. Am Tag des Experiments werden die Zellen mit Zellkulturmedium, dem 2 mM Fluo-4 and 4.6 mM Probenicid zugesetzt ist, bei 37˚C für 45 Minuten inkubiert. Nach der Beladung mit Fluoreszenzfarbstoff werden die Zellen viermal mit Hanks Pufferlösung (1 x HBSS, 20 mM HEPES), welche mit 0.07% Probenicid versetzt ist, gewaschen. Die Testsubstanzen werden in Hanks Pufferlösung, versetzt mit 2.5% DMSO, verdünnt. Die Hintergrundsfluoreszenz nicht-stimulierter Zellen wird in Gegenwart von Substanz in der 384-Well-Mikrotiterplatte fünf Minuten nach dem letzten Waschschritt im FLIPR$^{384}$-Gerät (Molecular Devices; Anregungswellenlänge:

488 nm; Emissionwellenlänge: bandpass 510 bis 570 nm) gemessen. Für die Zellstimulation wird MCH in Hanks Puffer mit 0.1% BSA verdünnt, 35 Minuten nach dem letzten Waschschritt zur 384-Well-Zellkulturplatte pipettiert und die MCH-stimulierte Fluoreszenz anschliessend im $FLIPR^{384}$ Gerät gemessen.

**Datenanalyse:**

1. Messung: Die zelluläre $Ca^{2+}$-Mobilisierung wird als Peak der relativen Fluoreszenz abzüglich Hintergrund gemessen und als Prozentanteil des Maximalsignals der Referenz (MCH $10^{-6}$M) ausgedrückt. Diese Messung dient der Identifizierung eines möglichen agonistischen Effektes einer Testsubstanz.
2. Messung: Die zelluläre $Ca^{2+}$-Mobilisierung wird als Peak der relativen Fluoreszenz abzüglich Hintergrund gemessen und als Prozentanteil des Maximalsignals der Referenz (MCH $10^{-6}$M, Signal wird auf 100% normiert) ausgedrückt. Die EC50-Werte der MCH Dosis-Wirkungskurve mit und ohne Testsubstanz (definierte Konzentration) werden durch das GraphPad Prism 2.01 Kurvenprogramm graphisch ermittelt. MCH-Antagonisten bewirken in der erstellten Graphik eine Rechtsverschiebung der MCH-Stimulationskurve.

Die Inhibition wird pKB-Wert ausgedrückt:

$$pKB=\log(EC_{50(Testsubstanz+MCH)} / EC_{50(MCH)} -1) -\log c_{(Testsubstanz)}$$

Die erfindungsgemäßen Verbindungen, einschließlich deren Salze, zeigen in den genannten Tests eine MCH-Rezeptor antagonistische Wirkung. Unter Anwendung des zuvor beschriebenen MCH-1 Rezeptor-Bindungstests wird eine antagonistische Aktivität in einem Dosisbereich von etwa $10^{-10}$ bis $10^{-5}$ M, insbesondere von $10^{-9}$ bis $10^{-6}$ M, erhalten.

Folgende IC50 Werte wurden mit Hilfe des zuvor beschriebenen MCH-1 Rezeptor-Bindungstests bestimmt:

| Verbindung gemäß Beispiel-Nr. | Substanzname | IC50-Wert |
|---|---|---|
| 1.8 | 5-(4-Brom-phenyl)-2-[4-(2-pyrrolidin-1-yl-ethoxy)-phenylethinyl]-pyridin | 8 nM |
| 1.3 | (2-{4-[5-(3,4-Difluor-phenyl)-pyridin-2-ylethinyl]-phenoxy}-ethyl)-diethyl-amin | 74 nM |

Nachfolgend werden Beispiele zu Darreichungsformen beschrieben, worin die Angabe "Wirkstoff" eine oder mehrere erfindungsgemäße Verbindungen, einschließlich deren Salze bedeutet. Im Falle einer der beschriebenen Kombinationen mit einem oder mehreren weiteren Wirksubstanzen umfasst der Begriff "Wirkstoff" auch die weiteren Wirksubstanzen.

<u>Beispiel A</u>

Kapseln zur Pulverinhalation mit 1 mg Wirkstoff

Zusammensetzung:

1 Kapsel zur Pulverinhalation enthält:

| | |
|---|---|
| Wirkstoff | 1.0 mg |
| Milchzucker | 20.0 mg |
| Hartgelatinekapseln | 50.0 mg |
| | 71.0 mg |

Herstellunqsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

Beispiel B

Inhalationslösunq für Respimat® mit 1 mg Wirkstoff

Zusammensetzung:

1 Hub enthält:

| | |
|---|---|
| Wirkstoff | 1.0 mg |
| Benzalkoniumchlorid | 0.002 mg |
| Dinatriumedetat | 0.0075 mg |
| Wasser gereinigt ad | 15.0 µl |

Herstellungsverfahren:

Der Wirkstoff und Benzalkoniumchlorid werden in Wasser gelöst und in Respimat®-Kartuschen abgefüllt.

Beispiel C

Inhalationslösunq für Vernebler mit 1 mq Wirkstoff

Zusammensetzung:

1 Fläschchen enthält:

| | |
|---|---|
| Wirkstoff | 0.1 g |
| Natriumchlorid | 0.18 g |
| Benzalkoniumchlorid | 0.002 g |
| Wasser gereinigt ad | 20.0 ml |

Herstellungsverfahren:

Wirkstoff, Natriumchlorid und Benzalkoniumchlorid werden in Wasser gelöst.

Beispiel D

Treibgas-Dosieraerosol mit 1 mg Wirkstoff

Zusammensetzung:

1 Hub enthält:

| | |
|---|---|
| Wirkstoff | 1.0 mg |
| Lecithin | 0.1 % |
| Treibgas ad | 50.0 µl |

Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

Beispiel E

Nasalspray mit 1 mg Wirkstoff

Zusammensetzung:

| | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

Herstellungsverfahren:

Der Wirkstoff und die Hilfsstoffe werden in Wasser gelöst und in ein entsprechendes Behältnis abgefüllt.

Beispiel F

Iniektionslösung mit 5 mq Wirksubstanz pro 5 ml

Zusammensetzung:

| | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

Beispiel G

Iniektionslösung mit 100 mq Wirksubstanz pro 20 ml

Zusammensetzung:

| | |
|---|---|
| Wirksubstanz | 100 mg |
| Monokaliumdihydrogenphosphat = $KH_2PO_4$ | 12 mg |
| Dinatriumhydrogenphosphat = $Na_2HPO_4.2H_2O$ | 2 mg |
| Natriumchlorid | 180 mg |
| Human-Serum-Albumin | 50 mg |

(fortgesetzt)

| | |
|---|---|
| Polysorbat 80 | 20 mg |
| Wasser für Injektionszwecke ad | 20 ml |

Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen.

Beispiel H

Lyophilisat mit 10 mg Wirksubstanz

Zusammensetzung:

| | |
|---|---|
| Wirksubstanz | 10 mg |
| Mannit | 300 mg |
| Human-Serum-Albumin | 20 mg |

Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.

Lösungsmittel für Lyophilisat:

| | |
|---|---|
| Polysorbat 80 = Tween 80 | 20 mg |
| Mannit | 200 mg |
| Wasser für Injektionszwecke ad | 10 ml |

Herstellung:

Polysorbat 80 und Mannit in Wasser für Injektionszwecke (Wfl) auflösen, in Ampullen abfüllen.

Beispiel I

Tabletten mit 20 mg Wirksubstanz

Zusammensetzung:

| | |
|---|---|
| Wirksubstanz | 20 mg |
| Lactose | 120 mg |
| Maisstärke | 40 mg |
| Magnesiumstearat | 2 mg |
| Povidon K 25 | 18 mg |

Herstellung:

Wirksubstanz, Lactose und Maisstärke homogen mischen; mit einer wässerigen Lösung von Povidon granulieren; mit Magnesiumstearat mischen; auf einer Tablettenpresse abpressen; Tablettengewicht 200 mg.

Beispiel J

Kapseln mit 20 mg Wirksubstanz

Zusammensetzung:

| | |
|---|---|
| Wirksubstanz | 20 mg |
| Maisstärke | 80 mg |
| Kieselsäure. hochdispers | 5 mg |
| Magnesiumstearat | 2.5 mg |

Herstellung:

Wirksubstanz, Maisstärke und Kieselsäure homogen mischen; mit Magnesiumstearat mischen; Mischung auf einer Kapselfüllmaschine in Hartgelatine-Kapseln Grösse 3 abfüllen.

Beispiel K

Zäpfchen mit 50 mgWirksubstanz

Zusammensetzung:

| | |
|---|---|
| Wirksubstanz | 50 mg |
| Hartfett (Adeps solidus) q.s. ad | 1700 mg |

Herstellung:

Hartfett bei ca. 38˚C aufschmelzen; gemahlene Wirksubstanz im geschmolzenen Hartfett homogen dispergieren; nach Abkühlen auf ca. 35˚C in vorgekühlte Formen ausgiessen.

Beispiel L

Injektionslösung mit 10 mg Wirksubstanz pro 1 ml

Zusammensetzung:

| | |
|---|---|
| Wirksubstanz | 10 mg |
| Mannitol | 50 mg |
| Human-Serum-Albumin | 10 mg |
| Wasser für Injektionszwecke ad | 1 ml |

Herstellung:

Mannitol in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

**Patentansprüche**

1. Alkin-Verbindungen der allgemeinen Formel I

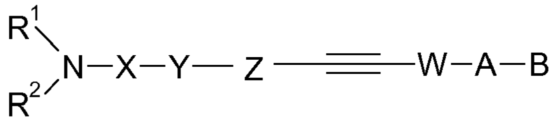

I

in der

$R^1$, $R^2$ unabhängig voneinander H, eine gegebenenfalls mit dem Rest $R^{11}$ substituierte $C_{1-8}$-Alkyl- oder $C_{3-7}$-Cycloalkyl-Gruppe, wobei eine $-CH_2$-Gruppe in Position 3 oder 4 einer 5, 6 oder 7-gliedrigen Cycloalkylgruppe durch -O-, -S- oder $-NR^{13}$- ersetzt sein kann, oder ein gegebenenfalls mit dem Rest $R^{12}$ ein- oder mehrfach und/oder mit Nitro einfach substituierter Phenyl- oder Pyridinylrest, oder $R^1$ und $R^2$ bilden eine $C_{2-8}$-Alkylen-Brücke, in der

- ein oder zwei $-CH_2$-Gruppen unabhängig voneinander durch -CH=N- oder -CH=CH- ersetzt sein können und/oder
- ein oder zwei $-CH_2$-Gruppen unabhängig voneinander durch -O-, -S-, -SO, $-(S0_2)-$, $-C=N-R^{18}$, $-C=N-O-R^{18}$, -CO-, $-C(=CH_2)-$ oder $-NR^{13}$ - derart ersetzt sein können, dass Heteroatome nicht unmittelbar miteinander verbunden sind,

wobei in der zuvor definierten Alkylen-Brücke ein oder mehrere H- Atome durch $R^{14}$ ersetzt sein können, und wobei die zuvor definierte Alkylen-Brücke mit einer oder zwei gleichen oder verschiedenen carbo- oder heterocyclischen Gruppen Cy derart substituiert sein kann, dass die Bindung zwischen der Alkylenbrücke und der Gruppe Cy

- über eine Einfach- oder Doppelbindung,
- über ein gemeinsames C-Atom unter Ausbildung eines spirocyclischen Ringsystems,
- über zwei gemeinsame, benachbarte C- und/oder N-Atome unter Ausbildung eines kondensierten bicyclischen Ringsystems oder
- über drei oder mehrere C- und/oder N-Atome unter Ausbildung eines verbrückten Ringsystems erfolgt,

X eine Einfachbindung oder eine $C_{1-6}$-Alkylen-Brücke, in der

- eine $-CH_2$-Gruppe durch -CH=CH- oder $-C\equiv C-$ ersetzt sein kann und/oder
- ein oder zwei $-CH_2$-Gruppen unabhängig voneinander durch -O-, -S-, -(SO)-, $-(SO_2)-$, -CO- oder $-NR^4$- derart ersetzt sein können, dass jeweils zwei O-, S- oder N-Atome oder ein O- mit einem S- Atom nicht unmittelbar miteinander verbunden sind,

wobei die Brücke X mit $R^1$ unter Einschluss des mit $R^1$ und X verbundenen N-Atoms unter Ausbildung einer heterocyclischen Gruppe verbunden sein kann, wobei die Brücke X zusätzlich auch mit $R^2$ unter Einschluss des mit $R^2$ und X verbundenen N-Atoms unter Ausbildung einer heterocyclischen Gruppe verbunden sein kann, und

wobei zwei C-Atome oder ein C- und ein N-Atom der Alkylenbrücke durch eine zusätzliche $C_{1-4}$-Alkylen-Brücke miteinander verbunden sein können, und

wobei ein C-Atom mit $R^{10}$ und/oder ein oder zwei C-Atome jeweils mit einem oder zwei gleichen oder verschiedenen Substituenten ausgewählt aus $C_{1-6}$-Alkyl-, $C_{2-6}$-Alkenyl-, $C_{2-6}$-Alkinyl-, $C_{3-7}$- Cycloalkyl, $C_{3-7}$-Cycloalkyl-$C_{1-3}$-alkyl, $C_{4-7}$-Cycloalkenyl und $C_{4-7}$- Cycloalkenyl-$C_{1-3}$-alkyl substituiert sein können, wobei zwei Alkyl- und/oder Alkenyl-Substituenten unter Ausbildung eines carbocyclischen Ringsystems miteinander verbunden sein können,

und
W, Z unabhängig voneinander eine Einfachbindung oder eine $C_{1-4}$-Alkylen- Brücke,
wobei in der Gruppe W und/oder Z eine nicht mit der -C≡C-Gruppe benachbarte -$CH_2$-Gruppe durch -O- oder -$NR^5$- ersetzt sein kann, und
wobei zwei benachbarte C-Atome oder ein C-Atom und ein benachbartes N-Atom mit einer zusätzlichen $C_1$-$_4$-Alkylen-Brücke miteinander verbunden sein können, und
wobei in der Alkylen-Brücke und/oder in der zusätzlichen Alkylen- Brücke ein C-Atom mit $R^{10}$ und/oder ein oder zwei C-Atome unabhängig voneinander mit einem oder zwei gleichen oder verschiedenen $C_{1-6}$-Alkyl-Resten substituiert sein können, wobei zwei Alkylreste unter Ausbildung eines carbocyclischen Rings miteinander verbunden sein können, und
Y eine der für Cy angegebenen Bedeutungen,
wobei $R^1$ mit Y unter Einschluss der Gruppe X und des mit $R^1$ und X verbundenen N-Atoms unter Ausbildung einer an Y kondensierten heterocyclischen Gruppe verbunden sein kann, und/oder
wobei X mit Y unter Ausbildung einer an Y kondensierten carbo- oder heterocyclischen Gruppe verbunden sein kann, und
A eine der für Cy angegebenen Bedeutungen und
B eine der für Cy angegebenen Bedeutungen oder $C_{1-6}$-Alkyl, $C_{1-6}$-Alkenyl, $C_{1-6}$-Alkinyl, $C_{3-7}$-Cycloalkyl-$C_{1-3}$-alkyl-, $C_{3-7}$- Cycloalkenyl-$C_{1-3}$-alkyl-, $C_{3-7}$-Cycloalkyl-$C_{1-3}$-alkenyl- oder $C_{3-7}$- Cycloalkyl-$C_{1-3}$-alkinyl-, worin ein oder mehrere C-Atome ein- oder mehrfach mit Halogen und/ oder einfach mit Hydroxy oder Cyano und/ oder cyclische Gruppen ein- oder mehrfach mit $R^{20}$ substituiert sein können,
Cy eine carbo- oder heterocyclische Gruppe ausgewählt aus einer der folgenden Bedeutungen

- eine gesättigte 3- bis 7-gliedrige carbocyclische Gruppe,
- eine ungesättigte 4- bis 7-gliedrige carbocyclische Gruppe,
- eine Phenyl-Gruppe,
- eine gesättigte 4- bis 7-gliedrige oder ungesättigte 5- bis 7- gliedrige heterocyclische Gruppe mit einem N-, O- oder S-Atom als Heteroatom,
- eine gesättigte oder ungesättigte 5- bis 7-gliedrige heterocyclische Gruppe mit zwei oder mehreren N-Atomen oder mit einem oder zwei N-Atomen und einem O- oder S-Atom als Heteroatome,
- eine aromatische heterocyclische 5- oder 6-gliedrige Gruppe mit einem oder mehreren gleichen oder verschiedenen Heteroatomen ausgewählt aus N, O und/oder S,

wobei die zuvor angeführten 4-, 5-, 6- oder 7-gliedrigen Gruppen über zwei gemeinsame, benachbarte C-Atome mit einem Phenyl- oder Pyridin-Ring kondensiert verbunden sein können, und
wobei in den zuvor genannten 5-, 6- oder 7-gliedrigen Gruppen eine oder zwei nicht benachbarte -$CH_2$-Gruppen unabhängig voneinander durch eine -CO-, -C(=$CH_2$)-, -(SO)- oder -($SO_2$)-Gruppe ersetzt sein können, und
wobei die zuvor angeführten gesättigten 6- oder 7-gliedrigen Gruppen auch als verbrückte Ringsysteme mit einer Imino-, ($C_{1-4}$- alkyl)-imino-, Methylen-, ($C_{1-4}$-Alkyl)-methylen- oder Di-($C_{1-4}$-alkyl)- methylen-Brücke vorliegen können, und
wobei die zuvor genannten cyclischen Gruppen ein- oder mehrfach an ein oder mehreren C-Atomen mit $R^{20}$, im Falle einer Phenylgruppe auch zusätzlich einfach mit Nitro, und/oder ein oder mehrere NH- Gruppen mit $R^{21}$ substituiert sein können,
$R^4$ $R^5$ unabhängig voneinander eine der für $R^{17}$ angegebenen Bedeutungen,
$R^{10}$ Hydroxy, ω-Hydroxy-$C_{1-3}$-alkyl, $C_{1-4}$-Alkoxy, ω($C_{1-4}$-Alkoxy)-$C_{1-3}$-alkyl- , Carboxy, $C_{1-4}$-Alkoxycarbonyl, Amino, $C_{1-4}$-Alkyl-amino, Di-($C_{1-4}$- alkyl)-amino, Cyclo-$C_{3-6}$-alkylenimino-, Amino-$C_{1-3}$-alkyl, $C_{1-4}$-Alkyl- amino-$C_{1-3}$-alkyl, Di-($C_{1-4}$-alkyl)-amino-$C_{1-3}$-alkyl, Cyclo-$C_{3-6}$- alkylenimino-$C_3$-alkyl, Amino-$C_2$-$_3$-alkoxy, $C_{1-4}$-Alkyl-amino-$C_{2-3}$- alkoxy, Di-($C_{1-4}$-alkyl)-amino-$C_{2-3}$-alkoxy, Cyclo-$C_{3-6}$-alkylenimino-$C_{2-3}$-alkoxy, Aminocarbonyl-, $C_{1-4}$-Alkyl-aminocarbonyl-, Di-($C_{1-4}$-alkyl)- aminocarbonyl- oder Cyclo-$C_{3-6}$-alkylenimino-carbonyl-,
$R^{11}$ $C_{2-6}$-Alkenyl, $C_{2-6}$-Alkinyl, $R^{15}$-O-, $R^{15}$-O-CO-, $R^{15}$-CO-O-, $_R{}^{16}{}_R{}^{17}$ N-, $R^{18}R^{19}$N-CO- oder Cy-,
$R^{12}$ eine der für $R^{20}$ angegebenen Bedeutungen,
$R^{13}$ eine der für $R^{17}$ angegebenen Bedeutungen, ausgenommen Carboxy,
$R^{14}$ Halogen, $C_{1-6}$-Alkyl, $C_{2-6}$-Alkenyl, $C_{2-6}$-Alkinyl, $R^{15}$-O-, $R^{15}$-O-CO-, $R^{15}$-CO-, $R^{15}$-CO-O-, $R^{16}R^{17}$ N-, $R^{18}R^{19}$N-CO-, $R^{15}$-O-$C_{1-3}$-alkyl , $R^{15}$- O-CO-$C_{1-3}$-alkyl, $R^{15}$-O-CO-NH-, $R^{15}$-$SO_2$-NH-, $R^{15}$-O-CO-NH-$C_{1-3}$- alkyl-, $R^{15}$-$SO_2$-NH-$C_{1-3}$-alkyl-, $R^{15}$-CO-$C_{1-3}$-alkyl, $R^{15}$-CO-O-$C_{1-3}$- alkyl, $R^{16}R^{17}$N-$C_{1-3}$-alkyl, $R^{18}R^{19}$N-CO-$C_{1-3}$-alkyl oder Cy-$C_{1-3}$-alkyl,
$R^{15}$ H, $C_{1-4}$-Alkyl, $C_{3-7}$-Cycloalkyl, $C_{3-7}$-Cycloalkyl-$C_{1-3}$-alkyl, Phenyl, Phenyl-$C_1$-$_3$-alkyl, Pyridinyl oder Pyridinyl-$C_{1-3}$-alkyl,
$R^{16}$ H, $C_{1-6}$-Alkyl, $C_{3-7}$-Cycloalkyl, $C_{3-7}$-Cycloalkyl-$C_{1-3}$-alkyl, $C_{4-7}$- Cycloalkenyl, $C_{4-7}$-Cycloalkenyl-$C_{1-3}$-alkyl,

ω-Hydroxy-$C_{2-3}$-alkyl, $_{\omega}$-($C_{1-4}$-Alkoxy)-$C_{2-3}$-alkyl, Amino-$C_{2-6}$-alkyl, $C_{1-4}$-Alkyl-amino-$C_{2}$-$_{6}$- alkyl, Di-($C_{1-4}$-alkyl)-amino-$C_{2-6}$-alkyl oder Cyclo-$C_{3-6}$-alkylenimino- $C_{2-6}$-alkyl-,
$R^{17}$ eine der für R $^{16}$ angegebenen Bedeutungen oder Phenyl, Phenyl-$C_{1-3}$-alkyl, Pyridinyl, Dioxolan-2-yl, -CHO, $C_{1-4}$-Alkylcarbonyl, Carboxy, Hydroxycarbonyl-$C_{1-3}$-alkyl, $C_{1-4}$-Alkoxycarbonyl-, $C_{1-4}$-Alkoxycarbonyl-$C_{1-3}$-alkyl, $C_{1-4}$-Alkyl- carbonylam ino-$C_{2-3}$-alkyl, N-($C_{1--4}$-Alkylcarbonyl)-N-($C_{1-4}$-Alkyl)- amino-$C_{2-3}$-alkyl, $C_{1-4}$-Alkylsulfonyl, $C_{1-4}$-Alkylsulfonylamino-$C_{2-3}$-alkyl oder N-($C_{1}$-$_{4}$-Alkylsulfonyl)-N(-$C_{1}$-$_{4}$-Alkyl)-amino-$C_{2}$-$_{3}$-alkyl
$R^{18}$, $R^{19}$ unabhängig voneinander H oder $C_{l-6}$-Alkyl,
$R^{20}$ Halogen, Hydroxy, Cyano, $C_{1-6}$-Alkyl, $C_{2-6}$-Alkenyl, $C_{2}$-$_{6}$-Alkinyl, $C_{3-7}$- Cycloalkyl, $C_{3-7}$-Cycloalkyl-$C_{1}$-$_{3}$-alkyl, Hydroxy-$C_{1-3}$-alkyl, $R^{22}$-$C_{1-3}$- alkyl oder eine der für $R^{22}$ angegebenen Bedeutungen,
$R^{21}$ $C_{1-4}$-Alkyl, ω-Hydroxy-$C_{2-6}$-alkyl, ω-$C_{1-4}$-Alkoxy-$C_{2-6}$-alkyl, ω-$C_{1-4}$-Alkyl- amino-$C_{2-6}$-alkyl, ω-Di-($C_{1-4}$-alkyl)-amino-$C_{2-6}$-alkyl, ωCyclo-$C_{3-6}$- alkylenimino-$C_{2-6}$-alkyl, Phenyl, Phenyl-$C_{1-3}$-alkyl, $C_{1}$-$_{4}$-Alkyl-carbonyl, $C_{1-4}$-Alkoxy-carbonyl, $C_{1-4}$-Alkylsulfonyl, Phenylcarbonyl oder Phenyl-$C_{1-3}$-alkyl-carbonyl,
$R^{22}$ Pyridinyl, Phenyl, Phenyl-$C_{1-3}$-alkoxy, OHC-, HO-N=HC-, $C_{1-4}$-Alkoxy- N=HC-, $C_{1-4}$-Alkoxy, $C_{1-4}$-Alkylthio, Carboxy, $C_{1-4}$-Alkylcarbonyl, $C_{1-4}$-Alkoxycarbonyl, Aminocarbonyl, $C_{1-4}$-Alkylaminocarbonyl, Di-($C_{1-4}$-alkyl)-aminocarbonyl, Cyclo-$C_{3-6}$-alkyl-amino-carbonyl-, Cyclo-$C_{3-6}$- alkylenimino-carbonyl, $C_{YC}l_{O}$-$C_{3}$-$_{6}$-alkylenimino-$C_{2}$-$_{4}$-alkyl- aminocarbonyl, $C_{1}$-$_{4}$-Alkyl-sulfonyl, $C_{1-4}$-Alkyl-sulfinyl, $C_{1-4}$-Alkylsulfonylamino, Amino, $C_{1-4}$-alkylamino, Di-($C_{1-4}$-alkyl)-amino, $C_{1-4}$- Alkyl-carbonyl-amino, Cyclo-$C_{3-6}$-alkylenimino, Phe- nyl-$C_{1-3}$-alkylamino, N-($C_{1-4}$-Alkyl)-phenyl-$C_{1-3}$-alkylamino, Acetylamino-, Propionylamino, Phenylcarbonyl, Phenylcarbonylamino, Phenylcarbonylmethylamino, Hydroxy-$C_{2-3}$-alkylaminocarbonyl, (4-Morpholinyl)carbonyl, (1-Pyrrolidinyl)carbonyl, (1-Piperidinyl)carbonyl, (Hexahydro-1-azepinyl)carbonyl, (4-Methyl-1- piperazinyl)carbonyl, Methylendioxy, Aminocarbonylamino oder Alkylaminocarbonylamino bedeuten,

wobei in den zuvor genannten Gruppen und Resten, insbesondere in A, B, W, X, Y, Z, $R^1$ bis $R^5$ und $R^{10}$ bis $R^{22}$, jeweils ein oder mehrere C-Atome zusätzlich ein- oder mehrfach mit F und/oder jeweils ein oder zwei C-Atome unabhängig voneinander zusätzlich einfach mit Cl oder Br und/oder jeweils ein oder mehrere Phenyl-Ringe unabhängig voneinander zusätzlich ein, zwei oder drei Substituenten ausgewählt aus der Gruppe F, Cl, Br, I, Cyano, $C_{1-4}$-alkyl, $C_{1-4}$-alkoxy-, Difluormethyl-, Trifluormethyl-, Hydroxy-, Amino-, $C_{1-3}$-alkylamino-, Di-($C_{1-3}$-alkyl)-amino-, Acetylamino-, Aminocarbonyl-, Difluormethoxy-, Trifluormethoxy-, Amino-$C_{1-3}$-alkyl-, $C_{1-3}$-alkylamino-$C_{1-3}$-alkyl- und Di-($C_{1-3}$-Alkyl)-amino-$C_{1-3}$-alkyl- aufweisen und/oder einfach mit Nitro substituiert sein können, und
das H-Atom einer vorhandenen Carboxygruppe oder ein an ein N-Atom gebundenes H-Atom jeweils durch einen in-vivo abspaltbaren Rest ersetzt sein kann, deren Tautomere, deren Diastereomere, deren Enantiomere, deren Gemische und deren Salze.

2. Alkin-Verbindungen gemäß Anspruch 1, **dadurch gekennzeichnet, dass** $R^1$, $R^2$ unabhängig voneinander H, $C_{1-6}$-Alkyl, $C_{3-7}$-Cycloalkyl, $C_{3-7}$-Cycloalkyl-$C_{1-3}$-alkyl, ω-Hydroxy-$C_{2-3}$-alkyl, $_{\omega}$-($C_{1-4}$-Alkoxy)-$C_{2-3}$-alkyl, $C_{1}$-$_{4}$-Alkoxy-carbonyl-$C_{1-4}$-alkyl, Carboxy-$C_{1}$-$_{4}$-alkyl, Amino-$C_{2}$-$_{4}$-alkyl, $C_{l}$-$_{4}$-Alkyl-amino-$C_{2}$-$_{4}$-alkyl, Di-($C_{1-4}$-alkyl)-amino-$C_{2-4}$-alkyl, Cyclo-$C_{3-6}$-alkylenimino-$C_{2-4}$-alkyl, Pyrrolidinyl, N-($C_{1}$-$_{4}$-alkyl)-pyrrolidinyl, Pyrrolidinyl-$C_{1-3}$-alkyl, N-($C_{1-4}$-alkyl)-pyrrolidinyl-$C_{1-3}$-alkyl, Piperidinyl, N-($C_{1}$-$_{4}$-alkyl)-piperidinyl, Piperidinyl-$C_{1}$-$_{3}$-alkyl, N-($C_{1}$-$_{4}$-alkyl)-piperidinyl-$C_{1}$-$_{3}$-alkyl, Phenyl, Phenyl-$C_{1}$-$_{3}$-alkyl, Pyridyl oder Pyridyl-$C_{1}$-$_{3}$-alkyl bedeuten,
wobei in den zuvor angegebenen Gruppen und Resten ein oder mehrere C-Atome ein- oder mehrfach mit F und/ oder ein oder zwei C-Atome unabhängig voneinander einfach mit Cl oder Br substituiert sein können und
wobei der Phenyl- oder Pyridylrest ein- oder mehrfach mit dem in Anspruch 1 definierten Rest $R^{12}$ und/oder einfach mit Nitro substituiert sein kann.

3. Alkin-Verbindungen gemäß einem oder mehreren der Ansprüche 1 oder 2,
**dadurch gekennzeichnet, dass** $R^1$ und $R^2$ derart eine Alkylen-Brücke gemäß Anspruch 1 bilden, dass $R^1R^2N$-eine Gruppe ausgewählt aus Azetidin, Pyrrolidin, Piperidin, Azepan, 2,5-Dihydro-1H-pyrrol, 1,2,3,6-Tetrahydropyridin, 2,3,4,7-Tetrahydro-1H-azepin, 2,3,6,7-Tetrahydro-1H-azepin, Piperazin, worin die freie Imin-Funktion mit $R^{13}$ substituiert sein kann, Piperidin-4-on-oxim, Piperidin-4-on-O-$C_{1-4}$-alkyl-oxim, Morpholin und Thiomorpholin bildet,
wobei gemäß Anspruch 1 ein- oder mehrere H-Atome durch R $^{14}$ ersetzt sein können, und/ oder wobei die Alkylen-Brücke in der in Anspruch 1 angegebenen Weise mit einer oder zwei gleichen oder verschiedenen carbo- oder heterocyclischen Gruppen Cy substituiert sein kann,
wobei R $^{13}$ , $R^{14}$ und Cy die in Anspruch 1 angegebene Bedeutung besitzen.

4. Alkin-Verbindungen nach einem der vorherigen Ansprüchen, **dadurch gekennzeichnet, dass** X -$CH_2$-, -$CH_2$-$CH_2$- oder -$CH_2$-$CH_2$-$CH_2$- und

für den Fall, dass die Gruppe Y über ein C-Atom mit X verbundenen ist, auch $-CH_2-C\equiv C-$, $-CH_2-CH_2-O-$, $-CH_2-CH_2-NR^4-$ oder 1,3-Pyrrolidinylen bedeutet, wobei die Pyrrolidinylen-Gruppe über die Imino-Gruppe mit Y verbunden ist, und

wobei die Brücke X mit $R^1$ unter Einschluss des mit $R^1$ und X verbundenen N-Atoms unter Ausbildung einer heterocyclischen Gruppe verbunden sein kann, und wobei die Brücke X zusätzlich auch mit $R^2$ unter Einschluss des mit $R^2$ und X verbundenen N-Atoms unter Ausbildung einer heterocyclischen Gruppe verbunden sein kann, und

wobei in X ein C-Atom mit $R^{1O}$, vorzugsweise einem Hydroxy-, ω-Hydroxy-$C_{1-3}$-alkyl-, ω($C_{1-4}$-Alkoxy)-$C_{1-3}$-alkyl- und/oder $C_{1-4}$-Alkoxy-Rest, und/oder ein oder zwei C-Atome jeweils mit einem oder zwei gleichen oder verschiedenen Substituenten ausgewählt aus $C_{1-6}$-Alkyl-,$C_{2-6}$-Alkenyl-, $C_{2-6}$-Alkinyl-, $C_{3-7}$-Cycloalkyl, $C_{3-7}$-Cycloalkyl-$C_{1-3}$-alkyl, $C_{4-7}$-Cycloalkenyl und $C_{4-7}$-Cycloalkenyl-$C_{1-3}$-alkyl substituiert sein können, wobei zwei Alkyl- und/oder Alkenyl-Substituenten unter Ausbildung eines carbocyclischen Ringsystems miteinander verbunden sein können, und

wobei jeweils ein oder mehrere C-Atome ein- oder mehrfach mit F und/oder jeweils ein oder zwei C-Atome unabhängig voneinander einfach mit Cl oder Br substituiert sein können und

worin $R^1$, $R^4$ und $R^{10}$ eine der in Anspruch 1 angegebenen Bedeutungen besitzt.

**5.** Alkin-Verbindungen nach einem der vorherigen Ansprüchen, **dadurch gekennzeichnet, dass** W und/oder Z unabhängig voneinander eine Einfachbindung oder ausgewählt sind aus der Gruppe der Brücken $-CH_2-$, $-CH_2-CH_2-$, $-CH_2-CH(CH_3)-$, $-CH_2-C(CH_3)_2-$, $-CH(CH_3)-CH_2-$, $-C(CH_3)_2-CH_2-$, Cyclopropylen, $-CH_2-CH(R^{10})-$, $-CH(R^{10})-CH_2-$ und

W zusätzlich auch $-CH_2-O-$ oder $-CH_2-NR^4-$ sowie

Z zusätzlich auch $-O-CH_2-$ oder $-NR^4-CH_2-$ bedeuten können,

worin $R^4$ die in Anspruch 1 angegebene Bedeutung, vorzugsweise -H, Methyl, Ethyl oder Propyl, besitzt und

worin $R^{10}$ die in Anspruch 1 angegebene Bedeutung, vorzugsweise -OH, N-Pyrrolidinyl, Amino-ethoxy, $C_{1-4}$-Alkylamino-ethoxy, Di-($C_{1-4}$-Alkyl)-amino-ethoxy, besitzt und

wobei jeweils ein oder mehrere C-Atome ein- oder mehrfach mit F und/oder jeweils ein oder zwei C-Atome unabhängig voneinander einfach mit Cl oder Br substituiert sein können.

**6.** Alkin-Verbindungen nach einem oder mehreren der vorherigen Ansprüche, **dadurch gekennzeichnet, dass** die Gruppe Y ausgewählt ist aus der Gruppe der bivalenten cyclischen Gruppen Phenyl, Naphthyl, Thienyl, Benzothienyl, Tetrahydronaphthyl, Pyridinyl, Pyrimidinyl, Pyrazinyl, Pyridazinyl, Indolyl, Dihydroindolyl, Dihydroindolon-yl, Chinolinyl, Tetrahydrochinolinyl, Isochinolinyl, Tetrahydro-isochinolinyl, Indazolyl, Benzimidazolyl, Benzfuranyl oder Benzoxazolyl,

wobei die zuvor genannten cyclischen Gruppen ein- oder mehrfach an ein oder mehreren C-Atomen mit $R^{20}$, im Falle einer Phenylgruppe auch zusätzlich einfach mit Nitro, und/oder an ein oder mehreren N-Atomen mit $R^{21}$ substituiert sein können,

wobei $R^1$ mit Y und/oder X mit Y wie in Anspruch 1 angegeben verbunden sein können, und

X, $R^1$, $R^{20}$ und $R^{21}$ die in Anspruch 1 angegebenen Bedeutungen aufweisen.

**7.** Alkin-Verbindungen nach einem oder mehreren der vorherigen Ansprüche, **dadurch gekennzeichnet, dass** die Gruppe A ausgewählt ist aus der Gruppe der bivalenten cyclischen Gruppen Phenyl, Pyridinyl, Pyrimidinyl, Pyrazinyl oder Pyridazinyl,

wobei die zuvor genannten cyclischen Gruppen ein- oder mehrfach an ein oder mehreren C-Atomen mit $R^{20}$, im Falle eines Phenylrings auch zusätzlich einfach mit Nitro, und/oder eine oder mehrere NH-Gruppen mit $R^{21}$ substituiert sein können, und

$R^{20}$ und $R^{21}$ die in Anspruch 1 angegebenen Bedeutungen aufweisen.

**8.** Alkin-Verbindungen nach einem oder mehreren der vorherigen Ansprüche, **dadurch gekennzeichnet, dass** die Gruppe B aus der ersten Gruppe Phenyl, Thienyl und Furanyl oder

aus der zweiten Gruppe $C_{1-6}$-Alkyl, $C_{1-6}$-Alkenyl, $C_{1-6}$-Alkinyl, $C_{3-7}$-Cycloalkyl-$C_{1-3}$-alkyl-, $C_{3-7}$-Cycloalkenyl-$C_{1-3}$-alkyl-, $C_{3-7}$-Cycloalkyl-$C_{1-3}$-alkenyl-, $C_{3-7}$-Cycloalkyl-$C_{1-3}$-alkinyl-, worin ein oder mehrere C-Atome ein- oder mehrfach mit Fluor substituiert sein können, ausgewählt ist, und

wobei die zuvor genannten cyclischen Gruppen ein- oder mehrfach an ein oder mehreren C-Atomen mit $R^{20}$, im Falle einer Phenylgruppe auch zusätzlich einfach mit Nitro substituiert sein können, und

$R^{20}$ die in Anspruch 1 angegebenen Bedeutungen aufweist.

**9.** Alkin-Verbindungen nach einem oder mehreren der vorherigen Ansprüche, **dadurch gekennzeichnet, dass**

Y eine Gruppe ist ausgewählt aus

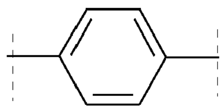 , 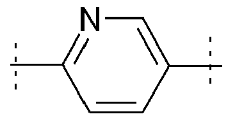 ,

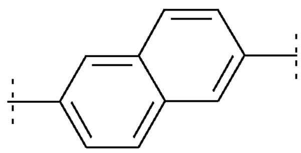 , 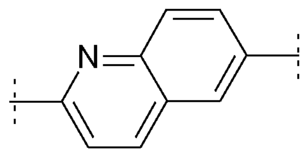 ,

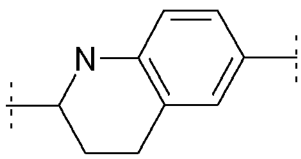 , 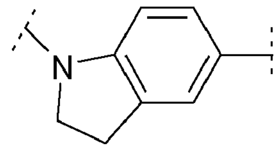 ,

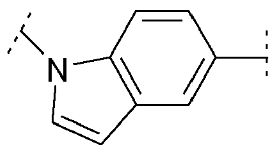 , 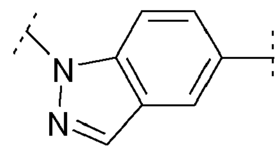 ,

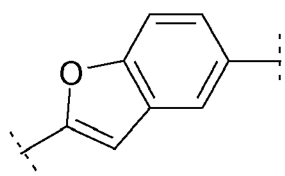 , 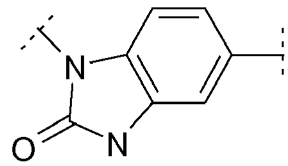

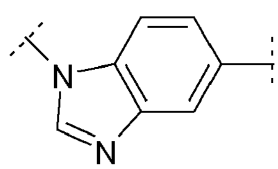 ,

bedeutet, und/oder
A

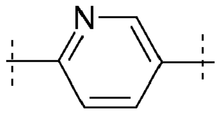

oder

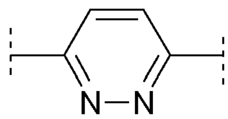

bedeutet, und/oder
B Phenyl bedeutet,

wobei A, B und/oder Y ein- oder zweifach, B auch dreifach, an ein oder mehreren C-Atomen mit $R^{20}$, im Falle eines Phenyl-Rings auch zusätzlich einfach mit Nitro, substituiert sein können, und wobei einie -NH-Gruppe mit $R^{21}$ substituiert sein kann, und worin $R^{20}$ und $R^{21}$ die in Anspruch 1 angegebene Bedeutung aufweisen.

**10.** Alkin-Verbindungen nach einem oder mehreren der vorherigen Ansprüche,
**dadurch gekennzeichnet, dass**

$R^{20}$ F, Cl, Br, I, OH, Cyano, Methyl, Difluormethyl, Trifluormethyl, Ethyl, n-Propyl, iso-Propyl, Methoxy, Difluormethoxy, Trifluormethoxy, Ethoxy, n-Propoxy oder iso-Propoxy bedeutet, wobei mehrfach vorkommende Substituenten $R^{20}$ gleiche oder verschiedene Bedeutungen aufweisen können.

**11.** Alkin-Verbindungen nach Anspruch 1 ausgewählt aus der Gruppe der Formeln

(1) 5-(4-Chlor-phenyl)-2-[5-(2-pyrrolidin-1-yl-ethoxy)- pyrid-2-yl-ethinyl]-pyridin
(2) [(R)-1-(2-{4-[5-(4-Chlor-phenyl)-pyridin-2-ylethinyl]-phenoxy}-ethyl)-pyrrolidin-2-yl]-methanol
(3) 5-(4-Chlor-phenyl)-2-[2-(4-methyl-piperidin-1-ylmethyl)-benzofuran-5-ylethinyl]-pyridin
(4) 5-[5-(4-Chlor-phenyl)-pyridin-2-ylethinyl]-1-(2-pyrrolidin-1 -yl-ethyl)-1,3-dihydro-benzimidazol-2-on
(5) [1 -(2-{4-[5-(4-Chlor-phenyl)-pyridin-2-ylethinyl]-phenoxy}-ethyl)-piperid in-4-yl]-methanol
(6) 1 -(2-{4-[5-(4-Chlor-phenyl)-pyridin-2-ylethinyl]-2-methyl-phenoxy}-ethyl)-piperidin-3-ol
(7) N-{4-[5-(4-Chlor-phenyl)-pyridin-2-ylethinyl]-phenyl}-2-pyrrolid in-1-yl-propionamid
(8) 1 -{3-[5-(4-Chlor-phenyl)-pyridin-2-yl]-prop-2-inyl}-5-pyrrolidin-1 - ylmethyl-1H-indol
(9) 2-[4-(4-Azetidin-1-ylmethyl-phenyl)-but-1-inyl]-5-(4-chlor-phenyl)-pyridin
(10) 5-(4-Chlor-phenyl)-2-[4-(4-piperidin-1-ylmethyl-phenyl)-but-1-inyl]-pyridin
(11) 5-(4-Brom-phenyl)-2-[4-(4-pyrrolidin-1-ylmethyl-phenyl)-but-1-inyl]-pyridin
(12) 2-[(4{4-[5-(4-Chlor-phenyl)-pyridin-2-yl]-but-3-inyl}-benzyl)-methyl-amino]-ethanol
(13) 5-(4-Chlor-phenyl)-2-{4-[4-((S)-2-methoxymethyl-pyrrolid in-1-ylmethyl)-phenyl]-but-1-inyl}-pyridin
(14) 5-(4-Chlor-phenyl)-2-{4-[2-(4-propyl-piperidin-1-yl)-ethoxy]-phenylethinyl}-pyridin
(15) 5'-[5-(4-Chlor-phenyl)-pyridin-2-ylethinyl]-3-pyrrolidin-1-yl-3,4,5,6-tetrahydro-2H-[1,2']bipyridinyl
(16) 5-(4-Chlor-phenyl)-2-{4-[4-(2-methyl-pyrrolidin-1-ylmethyl)-phenyl]-but-1-inyl}-pyridin
(17) 3-(4-Chlor-phenyl)-6-[4-(4-pyrrolidin-1-ylmethyl-phenyl)-but-1-inyl]-pyridazin
(18) 5-(4-Chlor-phenyl)-2-[4-(4-pyrrolidin-1-ylmethyl-phenyl)-but-1-inyl]-pyridin
(19) 5-(4-Chlor-phenyl)-2-{4-[2-(2,6-dimethyl-piperidin-1-yl)-ethoxy]-3-methyl-phenylethinyl}-pyridin
(20) 5-[5-(4-Chlor-phenyl)-pyridin-2-ylethinyl]-2-(2-pyrrolidin-1-yl-ethoxy)-benzoesäuremethylester
(21) 5-(4-Chlor-phenyl)-2-[3-methyl-4-(2-piperidin-1-yl-ethoxy)-phenylethinyl]-pyridin
(22) 5-(4-Chlor-phenyl)-2-[3-methyl-4-(2-pyrrolidin-1-yl-ethoxy)-phenylethinyl]-pyridin
(23) 5-(4-Chlor-phenyl)-2-{4-[4-(4-methyl-piperidin-1 -ylmethyl)-phenyl]-but-1-inyl}-pyridin
(24) 1-(2-{4-[5-(4-Chlor-phenyl)-pyridin-2-ylethinyl]-2-methyl-phenoxy}-ethyl)-piperidin-4-ol

(25) 5-(4-Chlor-phenyl)-2-{3-methyl-4-[2-(2-pyrrolidin-1-ylmethyl-piperidin-1-yl)-ethoxy]-phenylethinyl}-pyridin
(26) (5-[5-(4-Chlor-phenyl)-pyridin-2-ylethinyl]-pyridin-2-yl)-(2-piperidin-1-yl-ethyl)-amin
(27) 4-(4-{4-[5-(4-Chlor-phenyl)-pyridin-2-yl]-but-3-inyl}-benzyl)-morpholin
(28) (4-{4-[5-(4-Chlor-phenyl)-pyridin-2-yl]-but-3-inyl}-benzyl)-methylpiperidin-4-yl-amin
(29) 5-(4-Chlor-phenyl)-2-[3-(4-pyrrolidin-1-ylmethyl-phenoxy)-prop-1-inyl]-pyridin
(30) 6-[5-(4-Chlor-phenyl)-pyridin-2-ylethinyl]-2-pyrrolidin-1-ylmethyl-1,2,3,4-tetrahydro-chinolin
(31) (1-{5-[5-(4-Chlor-phenyl )-pyridin-2-ylethinyl]-pyridin-2-yl}-pyrrolid in-3-yl)-dimethyl-amin
(32) [(S)-1-(2-{4-[5-(4-Chlor-phenyl)-pyridin-2-ylethinyl]-2-methyl-phenoxy}-ethyl)-pyrrolidin-2-yl]-methanol
(33) 5-[5-(4-Chlor-phenyl)-pyridin-2-ylethinyl]-2-(2-pyrrolidin-1-yl-ethoxy)-phenylamin
(34) {5-[5-(4-Chlor-phenyl)-pyridin-2-ylethinyl]-pyridin-2-yl}-(2-pyrrolidin-1-yl-propyl)-amin
(35) 1-(4-{4-[5-(4-Chlor-phenyl)-pyridin-2-yl]-but-3-inyl}-benzyl)-pyrrolidin-3-ylamin
(36) 2-[3-Brom-4-(2-pyrrolidin-1-yl-ethoxy)-phenylethynyl]-5-(4-chlor-phenyl)-pyridin
(37) 1-(2-{4-[5-(4-Chlor-phenyl)-pyridin-2-ylethinyl]-2-methyl-phenoxy}-ethyl)-azepan
(38) 5-(4-Chlor-phenyl)-2-(6-pyrrolidin-1-ylmethyl-naphthalen-2-ylethinyl)-pyridin
(39) 5-[5-(4-Chlor-phenyl)-pyridin-2-ylethinyl]-**N**-methyl-2-(2-pyrrolidin-1-yl-ethoxy)-benzamid
(40) (2-{4-[5-(4-Chlor-phenyl)-pyridin-2-ylethinyl]-2-methyl -phenoxy}-ethyl)-cyclopropylmethyl-propyl-amin
(41) 1-(2-{4-[5-(4-Chlor-phenyl)-pyridin-2-ylethinyl]-phenoxy}-ethyl)-4-methyl-piperidin-4-ol
(42) 5-(4-Chlor-phenyl)-2-{3-methyl-4-[2-(4-methyl-piperidin-1-yl)-ethoxy]-phenylethinyl}-pyridin
(43) 5-(4-Chlor-phenyl)-3-fluor-2-{4-[2-(4-methyl-piperidin-1-yl)-ethoxy]-phenylethinyl}-pyridin
(44) 5-[5-(4-Chlor-phenyl)-pyridin-2-ylethinyl]-1-(2-pyrrolidin-1-yl-ethyl)-1H-indol
(45) {4-[5-(4-Chlor-phenyl)-pyridin-2-ylethinyl]-phenyl}-(2-pyrrolidin-1-yl-ethyl)-amin
(46)[1-(2-(4-[5-(4-Chlor-phenyl)-pyridin-2-ylethinyl]-2-methyl-phenoxy}-ethyl)-piperidin-4-yl]-essigsäuremethylester
(47) {5-[5-(4-Chlor-phenyl)-pyridin-2-ylethinyl]-pyrid in-2-yl}-methyl-(2-pyrrolidin-1-yl-ethyl)-amin
(48) [1-(2-{4-[5-(4-Chlor-phenyl )-pyridin-2-ylethinyl]-phenoxy}-ethyl)-pyrrolidin-3-yl]-carbaminsäure-tert-butylester
(49) 5-(4-Chlor-phenyl)-2-[3-methoxy-4-(2-pyrrolidin-1-yl-ethoxy)-phenylethinyl]-pyridin
(50) 5-(4-Chlor-phenyl)-2-[4-(2-piperidin-1-yl-ethoxy)-phenylethinyl]-pyridin
(51) 5-[5-(4-Chlor-phenyl)-pyridin-2-ylethinyl]-1-(2-pyrrolidin-1-yl-ethyl)-1H-indazol
(52) 2-[4-(2-Azetidin-1-yl-ethoxy)-phenylethinyl]-5-(4-chlor-phenyl)-pyridin
(53) 5-[5-(4-Chlor-phenyl)-pyridin-2-ylethinyl]-2-(2-pyrrolidin-1-yl-ethoxy)-benzaldehyd O-methyl-oxim
(54) 1'-{5-[5-(4-Chlor-phenyl)-pyridin-2-ylethinyl]-pyridin-2-yl}-[1,3']bipyrrolidinyl
(55) (4-{4-[5-(4-Chlor-phenyl)-pyridin-2-yl]-but-3-inyl}-benzyl)-methyl-(1-methyl-piperidin-4-yl)-amin
(56) 5-(4-Chlor-phenyl)-2-[3-chlor-4-(2-pyrrolidin-1-yl-ethoxy)-phenylethynyl]-pyridin
(57) (S)-1-(2-{4-[5-(4-Chlor-phenyl)-pyridin-2-ylethinyl]-2-methyl-phenoxy}-ethyl)-pyrrolidin-3-ol
(58) [1-(2-{4-[5-(4-Chlor-phenyl)-pyridin-2-ylethinyl]-2-methyl-phenoxy}-ethyl)-piperidin-4-yl]-pyridin-2-yl-amin
(59) 5-(4-Brom-phenyl)-2-[4-(2-pyrrolidin-1-yl-ethoxy)-phenylethinyl]-pyridin
(60) N-[1-(2-(4-[5-(4-Chlor-phenyl)-pyridin-2-ylethinyl]-phenoxy}-ethyl)-piperidin-4-ylmethyl]-N-methyl-acetamid
(61) 5-(2,4-Dichlor-phenyl)-2-[4-(4-pyrrolidin-1-ylmethyl-phenyl)-but-1 -inyl]-pyridin
(62) 5-(4-Chlor-phenyl)-2-{4-[2-(4-ethyl-piperidin-1-yl)-ethoxy]-phenylethinyl}-pyridin
(63) [1-(2-{4-[5-(4-Chlor-phenyl)-pyridin-2-ylethinyl]-2-methyl-phenoxy}-ethyl)-piperidin-4-yl]-methanol
(64) 5-(4-Chlor-phenyl)-2-[4-(2-pyrrolidin-1-yl-ethoxy)-phenylethinyl]-pyridin
(65) 5-(4-Chlor-phenyl)-2-{4-[2-(3,6-dihydro-2H-pyridin-1-yl)-ethoxy]-phenylethinyl)-pyridin
(66) 5-(4-Chlor-phenyl)-2-{4-[2-(2-methyl-pyrrolidin-1-yl)-ethoxy]-phenylethinyl}-pyridin
(67) (4-{4-[5-(4-Chlor-phenyl)-pyridin-2-yl]-but-3-inyl}-benzyl)-cyclopropylmethyl-amin
(68) 5-(4-Chlor-phenyl)-2-{4-[4-(4-pyrrolidin-1-yl-piperidin-1-ylmethyl)-phenyl]-but-1-inyl}-pyridin
(69) 5-(4-Methoxy-phenyl)-2-[4-(4-pyrrolidin-1-ylmethyl-phenyl)-but-1-inyl]-pyridin
(70) 5-(3,4-Difluor-phenyl)-2-[4-(4-pyrrolidin-1-ylmethyl-phenyl)-but-1-inyl]-pyridin
(71) 1 -(2-{4-[5-(4-Chlor-phenyl)-pyridin-2-ylethinyl]-2-methyl-phenoxy}-ethyl)-4-methyl-piperidin-4-ol
(72) 5-( 4-Chlor-phenyl )-2-{4-[4-((R)-2-methoxymethyl-pyrrolidin-1-ylmethyl)-phenyl]-but-1-inyl}-pyridin
(73) 6-[5-(4-Chlor-phenyl)-pyridin-2-ylethinyl]-2-pyrrolidin-1-ylmethyl-chinolin
(74) 1-(4-{4-[5-(4-Chlor-phenyl)-pyridin-2-yl]-but-3-inyl}-benzyl)-4-methylpiperazin
(75) (5-[5-(4-Chlor-phenyl)-pyridin-2-ylethinyl]-pyridin-2-yl)-(2-pyrrolidin-1-yl-ethyl)-amin
(76) 5-(4-Chlor-phenyl)-2-(3-methyl-4-{2-[4-(pyridin-2-yloxy)-piperidin-1-yl]-ethoxy}-phenylethinyl)-pyridin
(77) 5-(4-Chlor-phenyl)-2-{4-[2-(3,6-dihydro-**2**H-pyridin-1-yl)-ethoxy]-3-methyl-phenylethinyl}-pyridin
(78) (R)-1-(2-{4-[5-(4-Chlor-phenyl)-pyridin-2-ylethinyl]-2-methyl-phenoxy}-ethyl)-pyrrolidin-3-ol
(79) 1-(2-{5-[5-(4-Chlor-phenyl)-pyridin-2-ylethinyl]-indol-1-yl}-ethyl)-piperidin-4-ol

(80) 1-(2-{4-[5-(4-Chlor-phenyl)-pyridin-2-ylethinyl]-phenoxy}-ethyl)-piperidin-4-ol
(81) 1-(2-{4-[5-(4-Chlor-phenyl)-pyridin-2-ylethinyl]-2-methyl-phenoxy}-ethyl)-4-phenyl-piperidin-4-ol
(82) 1-(2-{4-[5-(4-Chlor-phenyl)-pyridin-2-ylethinyl]-phenoxy}-ethyl)-[4,4']bipiperidinyl
(83) 5-(4-Chlor-phenyl)-2-[3-ethinyl-4-(2-pyrrolidin-1-yl-ethoxy)-phenylethinyl]-pyridin
(84) 5-(3,4-Dichlor-phenyl)-2-[4-(4-pyrrolidin-1-ylmethyl-phenyl)-but-1-inyl]-pyridin
(85) 1-(2-{4-[5-(4-Chlor-phenyl)-pyridin-2-ylethinyl]-2-methyl-phenoxy}-ethyl)-4-methyl-piperidin-4-ylamin
(86) 5-[5-(4-Chlor-phenyl)-pyridin-2-ylethinyl]-2-(2-pyrrolidin-1-yl-ethoxy)-benzaldehyd-oxim
(87) 5-(4-Chlor-phenyl)-2-{4-[2-(2,6-dimethyl-piperidin-1-yl)-ethoxy]-phenylethinyl)-pyridin
(88) 5-(4-Chlor-phenyl)-2-(4-(2-{4-(1H-imidazol-4-yl)-piperidin-1-yl]-ethoxy}-3-methyl-phenylethinyl)-pyridin
(89) [1-(2-{4-[5-(4-Chlor-phenyl)-pyridin-2-ylethinyl]-2-methyl-phenoxy}-ethyl)-piperidin-2-yl]-methanol
(90) (4-{4-[5-(4-Chlor-phenyl)-pyridin-2-yl]-but-3-inyl}-benzyl)-methyl-pyridin-2-ylmethyl-amin
(91) 1-(2-{4-[5-(4-Chlor-phenyl)-pyridin-2-ylethinyl]-phenoxy}-ethyl)-piperidin-4-carbonsäureamid
(92) 2-[(2-{4-[5-(4-Chlor-phenyl)-pyridin-2-ylethinyl]-phenoxy}-ethyl)-methyl-amino]-ethanol
(93) 5-(4-Chlor-phenyl)-2-{4-[2-(4-methyl-piperidin-1-yl)-ethoxy]-phenylethinyl}-pyridin
(94) {2-[1-(2-{4-[5-(4-Chlor-phenyl)-pyridin-2-ylethinyl]-2-methyl-phenoxy}-ethyl)-piperidin-4-yl]-ethyl)-diethylamin
(95) 5-(4-Chlor-phenyl)-2-{4-[2-(2,4,6-trimethyl-piperidin-1-yl)-ethoxy]-phenylethinyl}-pyridin
(96) 5-(4-Chlor-phenyl)-2-{4-[2-(3,5-dimethyl-piperidin-1-yl)-ethoxy]-3-methyl-phenylethinyl}-pyridin
(97) cis-2-(2-{4-[5-(4-Chlor-phenyl )-pyridin-2-ylethinyl]-phenoxy}-ethyl)-decahydro-isochinolin
(98) 6-(2-{4-[5-(4-Chlor-phenyl)-pyridin-2-ylethinyl]-2-methyl-phenoxy}-ethyl)-2-methyl-2,6-diaza-spiro[3.4]octan
(99) 1-(2-{5-[5-(4-Chlor-phenyl)-pyridin-2-ylethinyl]-indol-1 -yl}-ethyl)-4-methyl-piperid in-4-ol
(100) [1-(2-{4-[5-(4-Chlor-phenyl)-pyridin-2-ylethinyl]-2-methyl-phenoxy}-ethyl)-pyrrolidin-3-yl]-dimethyl-amin
(101) 5-(4-Chlor-phenyl)-2-[3-fluor-4-(2-pyrrolidin-1-yl-ethoxy)-phenylethinyl]-pyridin
(102) [1-(2-{4-[5-(4-Chlor-phenyl)-pyridin-2-ylethinyl]-2-methyl-phenoxy}-ethyl)-piperidin-4-yl]-cyclopentyl-methyl-amin
(103) 5-[5-(4-Chlor-phenyl)-pyridin-2-ylethinyl]-1-(2-pyrrolidin-1-yl-ethyl)-2,3-dihydro-1H-indol
(104) 5-(4-Chlor-phenyl)-2-{4-[2-(4-pyrrolidin-1-yl-piperidin-1-yl)-ethoxy]-phenylethinyl}-pyridin
(105) 5-(4-Chlor-phenyl)-2-{4-[2-(2,5-dihydro-pyrrol-1-yl)-ethoxy]-phenylethinyl}-pyridin
(106) [1-(2-{4-[5-(4-Chlor-phenyl)-pyridin-2-ylethinyl]-phenoxy}-ethyl)-piperidin-4-ylmethyl]-dimethyl-amin
(107) 1-(2-{4-[5-(4-Chlor-phenyl)-pyridin-2-ylethinyl]-phenoxy}-ethyl)-4-methyl-piperazin
(108) (4-{4-[5-(4-Chlor-phenyl)-pyridin-2-yl]-but-3-inyl}-benzyl)-pyridin-2-ylmethyl-amin
(109) 1-(2-{4-[5-(4-Chlor-phenyl)-pyridin-2-ylethinyl]-2-methyl-phenoxy}-ethyl)-spiro[piperidin-4,2'(1H')-chinazolin]-4'(3'H)on
(110) 4-{[(2-(4-[5-(4-Chlor-phenyl)-pyridin-2-ylethinyl]-2-methyl-phenoxy}-ethyl)-methyl-amino]-methyl}-phenol
(111) 5-(4-Chlor-phenyl)-2-[4-(3-piperidin-1 -yl-pyrrolidin-1 -yl)-phenylethinyl]-pyridin
(112) 5-(4-Chlor-phenyl)-2-[2-(2-pyrrolidin-1 -yl-ethoxy)-pyrid-5-yl-ethynyl]-pyridin
(113) 3-(2-{4-[5-(4-Chlor-phenyl)-pyridin-2-ylethinyl]-2-methyl-phenoxy}-ethyl)-9-methyl-3,9-diaza-spiro[5.5]undecan
(114) (2-{4-[5-(4-Chlor-phenyl)-pyrid in-2-ylethinyl]-2-methyl-phenoxy}-ethyl)-diisopropyl-amin
(115) 5-(4-Chlor-phenyl)-2-[4-(3-pyrrolidin-1 -yl-propyl)-phenylethinyl]-pyridin
(116) 2-(2-{4-[5-(4-Chlor-phenyl)-pyridin-2-ylethinyl]-phenoxyl-ethyl}-1,2,3,4-tetrahydro-isochinolin
(117) 3-(4-Chlor-phenyl)-6-[4-(2-pyrrolidin-1 -yl-ethoxy)-phenylethinyl]-pyridazin
(118) (R)-1 -(2-{5-[5-(4-Chlor-phenyl)-pyridin-2-ylethinyl]-indol-1 -yl}-ethyl)-pyrrolidin-3-ol
(119) 5-[5-(4-Chlor-phenyl)-pyridin-2-ylethinyl]-3-methyl-1-(2-pyrrolidin-1-yl-ethyl )-1,3-dihydro-benzimidazol-2-on
(120) 5-[5-(4-Chlor-phenyl)-pyridin-2-ylethinyl]-1-(2-pyrrolidin-1-yl-ethyl)-1H-benzimidazol
(121) 2-[5-(4-Chlor-phenyl)-pyridin-2-ylethinyl]-1-methyl-5-pyrrolidin-1-ylmethyl-1H-benzimidazol
(122) trans-2-(2-{4-[5-(4-Chlor-phenyl)-pyridin-2-ylethinyl]-phenoxy}-ethyl)-decahydro-isochinolin

einschließlich deren Tautomere, deren Diastereomere, deren Enantiomere, deren Gemische und deren Salze.

**12.** Physiologisch verträgliche Salze der Alkin-Verbindungen nach einem oder mehreren der Ansprüche 1 bis 11.

**13.** Arzneimittel, enthaltend mindestens eine Alkin-Verbindung nach einem oder mehreren der Ansprüche 1 bis 11 und/oder ein Salz gemäß Anspruch 12 neben gegebenenfalls einem oder mehreren inerten Trägerstoffen und/oder Verdünnungsmitteln.

**14.** Alkin-Verbindung nach einem oder mehreren der Ansprüche 1 bis 11 und/ oder Salz gemäß Anspruch 12 zur Beeinflussung des Essverhaltens eines Säugetiers, zur Reduzierung des Körpergewichts und/ oder zum Verhindern einer Zunahme des Körpergewichts eines Säugetiers.

**15.** Arzneimittel, enthaltend
einen ersten Wirkstoff, der aus den Alkin-Verbindungen nach einem oder mehreren der Ansprüche 1 bis 11 und/ oder den Salzen gemäß Anspruch 12 ausgewählt ist, sowie
einen zweiten Wirkstoff, der aus der Gruppe ausgewählt ist bestehend aus Wirkstoffen zur Behandlung von Diabetes, Wirkstoffen zur Behandlung diabetischer Komplikationen, Wirkstoffen zur Behandlung von Obesitas, vorzugsweise anderen als MCH-Antagonisten, Wirkstoffen zur Behandlung von Bluthochdruck, Wirkstoffen zur Behandlung von Hyperlipidemia, einschließlich Arteriosklerose, Wirkstoffen zur Behandlung von Arthritis, Wirkstoffen zur Behandlung von Angstzuständen und Wirkstoffen zur Behandlung von Depressionen,
neben gegebenenfalls einem oder mehreren inerten Trägerstoffen und/oder Verdünnungsmitteln.

Europäisches Patentamt
European Patent Office
Office européen des brevets

# EUROPÄISCHER RECHERCHENBERICHT

Nummer der Anmeldung
EP 10 17 9237

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 99/02497 A (NOVARTIS A.-G., SWITZ.;NOVARTIS-ERFINDUNGEN VERWALTUNGSGESELLSCHAFT M.) 21. Januar 1999 (1999-01-21)<br>* Beispiele Seiten 21 und 23-26Ansprüche 1,9 *<br>----- | 1 | INV.<br>C07D213/30<br>A61K31/4375<br>A61P3/04<br>C07D401/12<br>C07D405/06<br>C07D401/06<br>C07D213/40<br>C07D401/10<br>C07D213/38<br>C07D401/14<br>C07D213/55<br>C07D213/56<br>C07D213/61<br>C07D213/53 |
| X,P | WO 03/018579 A (MERCK FROSST CANADA & CO., CAN.) 6. März 2003 (2003-03-06)<br>* Anspruch 1; Beispiele 24,26-28,30-32 *<br>----- | 1 | |
| X,P | WO 03/014111 A (ASTRAZENECA AB, SWED.) 20. Februar 2003 (2003-02-20)<br>* Seite 22, Zeilen 24/25; Seite 23, Zeilen 13/14 *<br>----- | 1 | |
| Y | WO 02/04433 A (BAKTHAVATCHALAM RAJAGOPAL ;NEUROGEN CORP (US); HUTCHISON ALAN (US)) 17. Januar 2002 (2002-01-17)<br>* Anspruch 1 *<br>----- | 1-15 | |
| | | | RECHERCHIERTE SACHGEBIETE (IPC) |
| Y,D | WO 01/21577 A (ISHIHARA YUJI ;KATO KANEYOSHI (JP); MORI MASAAKI (JP); SHIMOMURA Y) 29. März 2001 (2001-03-29)<br>* Ansprüche 1-9 *<br>----- | 1-15 | C07D<br>A61K |
| Y,P | EP 1 285 651 A1 (TAKEDA CHEMICAL INDUSTRIES LTD [JP] TAKEDA PHARMACEUTICAL [JP]) 26. Februar 2003 (2003-02-26)<br>* Ansprüche 1-4 * | 1-15 | |
| Y,D | & WO 01/82925 A (ISHIHARA YUJI ;SUZUKI NOBUHIRO (JP); TAKEKAWA SHIRO (JP); TAKEDA C) 8. November 2001 (2001-11-08)<br>----- | 1-15 | |

Der vorliegende Recherchenbericht wurde für alle Patentansprüche erstellt

1

| Recherchenort | Abschlußdatum der Recherche | Prüfer |
|---|---|---|
| München | 9. März 2011 | Fink, Dieter |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR. EP 10 17 9237

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-03-2011

| Im Recherchenbericht angeführtes Patentdokument | Datum der Veröffentlichung | Mitglied(er) der Patentfamilie | Datum der Veröffentlichung |
|---|---|---|---|
| WO 9902497 A | 21-01-1999 | AT 393145 T | 15-05-2008 |
| | | AU 738973 B2 | 04-10-2001 |
| | | AU 8974398 A | 08-02-1999 |
| | | BR 9811685 A | 19-09-2000 |
| | | CA 2295678 A1 | 21-01-1999 |
| | | CN 1262676 A | 09-08-2000 |
| | | DE 69839398 T2 | 28-05-2009 |
| | | EP 0998459 A2 | 10-05-2000 |
| | | ES 2308811 T3 | 01-12-2008 |
| | | HU 0004225 A2 | 28-05-2001 |
| | | ID 23889 A | 25-05-2000 |
| | | JP 3481208 B2 | 22-12-2003 |
| | | JP 2001509504 T | 24-07-2001 |
| | | NO 20000124 A | 02-03-2000 |
| | | NZ 502210 A | 26-07-2002 |
| | | PL 343865 A1 | 10-09-2001 |
| | | PT 998459 E | 31-07-2008 |
| | | RU 2203889 C2 | 10-05-2003 |
| | | SK 232000 A3 | 12-06-2000 |
| | | TR 200000059 T2 | 21-06-2000 |
| | | TW 544448 B | 01-08-2003 |
| | | US 6656957 B1 | 02-12-2003 |
| WO 03018579 A | 06-03-2003 | AR 036365 A1 | 01-09-2004 |
| | | BR 0212042 A | 17-08-2004 |
| | | CA 2456817 A1 | 06-03-2003 |
| | | CN 1639161 A | 13-07-2005 |
| | | EC SP044992 A | 28-04-2004 |
| | | EP 1436290 A1 | 14-07-2004 |
| | | HK 1080073 A1 | 08-08-2008 |
| | | HR 20040151 A2 | 31-05-2006 |
| | | HU 0401729 A2 | 28-12-2004 |
| | | IS 7138 A | 30-01-2004 |
| | | JP 4157035 B2 | 24-09-2008 |
| | | JP 2005508313 T | 31-03-2005 |
| | | MX PA04001889 A | 15-06-2004 |
| | | NZ 530931 A | 23-12-2005 |
| | | PE 04162003 A1 | 15-05-2003 |
| | | UA 75957 C2 | 15-06-2004 |
| | | ZA 200400952 A | 22-10-2004 |
| WO 03014111 A | 20-02-2003 | EP 1417201 A1 | 12-05-2004 |
| | | JP 2005501091 T | 13-01-2005 |
| | | US 2006229313 A1 | 12-10-2006 |
| | | US 2004220185 A1 | 04-11-2004 |

EPO FORM P0461

Für nähere Einzelheiten zu diesem Anhang : siehe Amtsblatt des Europäischen Patentamts, Nr.12/82

## ANHANG ZUM EUROPÄISCHEN RECHERCHENBERICHT ÜBER DIE EUROPÄISCHE PATENTANMELDUNG NR.

EP 10 17 9237

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

09-03-2011

| Im Recherchenbericht angeführtes Patentdokument | Datum der Veröffentlichung | Mitglied(er) der Patentfamilie | Datum der Veröffentlichung |
|---|---|---|---|
| WO 0204433 A | 17-01-2002 | AU 7929401 A | 21-01-2002 |
| | | CA 2414198 A1 | 17-01-2002 |
| | | EP 1366030 A2 | 03-12-2003 |
| | | JP 2004516239 T | 03-06-2004 |
| | | MX PA03000174 A | 22-09-2003 |
| WO 0121577 A | 29-03-2001 | AU 7315700 A | 24-04-2001 |
| | | CA 2386474 A1 | 29-03-2001 |
| | | CO 5251437 A1 | 28-02-2003 |
| | | EP 1218336 A2 | 03-07-2002 |
| | | US 7115750 B1 | 03-10-2006 |
| | | US 2007173498 A1 | 26-07-2007 |
| EP 1285651 A1 | 26-02-2003 | AT 479429 T | 15-09-2010 |
| | | AU 5259601 A | 12-11-2001 |
| | | CA 2407149 A1 | 08-11-2001 |
| | | DK 1285651 T3 | 13-12-2010 |
| | | WO 0182925 A1 | 08-11-2001 |
| | | PT 1285651 E | 21-10-2010 |
| | | US 2004077628 A1 | 22-04-2004 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 0121577 A
- WO 0182925 A


### In der Beschreibung aufgeführte Nicht-Patentliteratur


- **Qu, D. et al.** A role for melanin-concentrating hormone in the central regulation of feeding behaviour. *Nature,* 1996, vol. 380 (6571), 243-7
- **Shimada, M. et al.** Mice lacking melanin-concentrating hormone are hypophagic and lean. *Nature,* 1998, vol. 396 (6712), 670-4
- **Borowsky, B. et al.** Antidepressant, anxiolytic and anorectic effects of a melanin-concentrating hormone-1 receptor antagonist. *Nat Med,* 2002, vol. 8 (8), 825-30
- **Chen, Y. et al.** Targeted disruption of the melanin-concentrating hormone receptor-1 results in hyperphagia and resistance to diet-induced obesity. *Endocrinology,* 2002, vol. 143 (7), 2469-77
- **Marsh, D.J. et al.** Melanin-concentrating hormone 1 receptor-deficient mice are lean, hyperactive, an+d hyperphagic and have altered metabolism. *Proc Natl Acad Sci U S A,* 2002, vol. 99 (5), 3240-5
- **Takekawa, S. et al.** T-226296: a novel, orally active and selective melanin-concentrating hormone receptor antagonist. *Eur J Pharmacol,* 2002, vol. 438 (3), 129-35
- **Klapars, Artis ; Buchwald, Stephen L.** Copper-Catalyzed Halogen Exchange in Aryl Halides: An Aromatic Finkelstein Reaction. *Journal of the American Chemical Society,* 2002, vol. 124 (50), 14844-14845
- **von Hoogduijn M et al.** Melanin-concentrating hormone and its receptor are expressed and functional in human skin. *Biochem. Biophys. Res Commun.,* 2002, vol. 296, 698-701
- **Karlsson OP ; Lofas S.** Flow-Mediated On-Surface Reconstitution of G-Protein Coupled Receptors for Applications in Surface Plasmon Resonance Biosensors. *Biochem.,* 2002, vol. 300, 132-138